(19) Europäisches Patentamt
European Patent Office
Office européen des brevets

(11) EP 4 570 804 A1

(12) # EUROPEAN PATENT APPLICATION
published in accordance with Art. 153(4) EPC

(43) Date of publication:
**18.06.2025 Bulletin 2025/25**

(21) Application number: **23851894.8**

(22) Date of filing: **09.08.2023**

(51) International Patent Classification (IPC):
***C07D 495/04*** *(2006.01)* ***C07D 519/00*** *(2006.01)*
***A61K 31/519*** *(2006.01)* ***A61K 31/498*** *(2006.01)*

(52) Cooperative Patent Classification (CPC):
**A61K 31/498; A61K 31/519; C07D 495/04; C07D 519/00**

(86) International application number:
**PCT/CN2023/112061**

(87) International publication number:
**WO 2024/032673 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.08.2022 CN 202210950666**
**02.09.2022 CN 202211075118**
**27.10.2022 CN 202211323499**
**24.11.2022 CN 202211744316**
**12.01.2023 CN 202310041300**
**24.02.2023 CN 202310161801**
**20.03.2023 CN 202310269232**
**07.04.2023 CN 202310366104**
**23.04.2023 CN 202310441682**
**16.05.2023 CN 202310550529**
**07.06.2023 CN 202310667178**
**25.07.2023 CN 202310911699**

(71) Applicant: **Xizang Haisco Pharmaceutical Co., Ltd.**
**Lhoka, Tibet 856099 (CN)**

(72) Inventors:
- **LI, Yao**
  **Lhoka, Tibet 856099 (CN)**
- **CHEN, Lei**
  **Lhoka, Tibet 856099 (CN)**
- **SHI, Zongjun**
  **Lhoka, Tibet 856099 (CN)**
- **REN, Lei**
  **Lhoka, Tibet 856099 (CN)**
- **GENG, Pengxin**
  **Lhoka, Tibet 856099 (CN)**
- **WANG, Jie**
  **Lhoka, Tibet 856099 (CN)**
- **CHENG, Fengkai**
  **Lhoka, Tibet 856099 (CN)**
- **LIN, Xiao**
  **Lhoka, Tibet 856099 (CN)**
- **HUANG, Shuai**
  **Lhoka, Tibet 856099 (CN)**
- **ZHANG, Xiaohai**
  **Lhoka, Tibet 856099 (CN)**
- **YANG, Shuang**
  **Lhoka, Tibet 856099 (CN)**
- **TANG, Pingming**
  **Lhoka, Tibet 856099 (CN)**
- **ZHANG, Chen**
  **Lhoka, Tibet 856099 (CN)**
- **YAN, Pangke**
  **Lhoka, Tibet 856099 (CN)**

(74) Representative: **IP TRUST SERVICES**
**Europole - Le Grenat**
**3, avenue Doyen Louis Weil**
**38000 Grenoble (FR)**

(54) **PDE4B INHIBITOR AND USE THEREOF**

(57) A compound represented by formula I, stereoisomers or pharmaceutically acceptable salts thereof, or a pharmaceutical composition containing same, and the use thereof as a PDE4B inhibitor in the preparation of a drug for treatment of related diseases. Each group in formula (I) is as defined in the description.

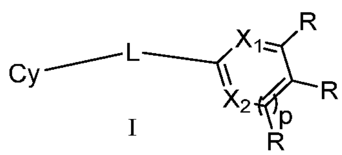

I

EP 4 570 804 A1

## Description

Technical Field

The present disclosure belongs to the field of medicine and in particular relates to a small molecule compound and a stereoisomer or pharmaceutically acceptable salt thereof, which have a selective inhibitory activity against PDE4B, and the use thereof in the preparation of a drug for treating a related disease.

Background Art

PDE4 inhibitors produce antidepressant effects in humans and animals by enhancing cAMP signaling in the brain. PDE4 inhibitors also play an important role in the treatment of other central nervous system diseases, including diseases such as Alzheimer's disease, Parkinson's disease, schizophrenia, stroke, and Huntington's chorea. In addition, the research and development of PDE4 inhibitors have also made great progress in the treatment of respiratory diseases such as asthma and chronic obstructive pulmonary disease. The principle of research and development of such drugs stems from the role of PDE4 in inhibiting the function of a range of inflammatory cells and resident cells, which is believed to be associated with the pathogenesis of such diseases. A large number of clinical studies have shown that cyclic adenosine monophosphate (cAMP) can block the proliferation and chemotaxis of inflammatory cells and inhibit the release of inflammatory and cytotoxic mediators in lungs. In addition, PDE4 is especially abundant in immune cells, inflammatory cells, and smooth muscle cells.

PDE4 inhibitors play an anti-inflammatory role mainly by inhibiting the hydrolysis function of PDE4 so as to increase the cAMP level *in vivo,* thereby inhibiting the release of inflammatory factors and at the same time promoting the production of anti-inflammatory mediators. Roflumilast is used clinically for the treatment of COPD with a significant anti-inflammatory effect and can inhibit the release of inflammatory mediators such as TNF-$\alpha$, interleukins, and chemokines from monocytes, macrophages, T cells, etc. However, such inhibitors generally have serious side effects such as nausea and vomiting, which limits the clinical application of PDE4 inhibitors. A large number of studies have shown that phosphodiesterase 4 subtype B (PDE4B), which is associated with inflammatory responses in the human body, participates in the release of various inflammatory mediators *in vivo,* while subtype D is closely related to the production of side effects such as nausea and vomiting, which provides a new idea for finding PDE4 inhibitors with low side effects, i.e., designing PDE4B inhibitors that may have reduced side effects, thereby promoting further clinical application.

Phosphodiesterase 4 is highly selective for cAMP and has four subtypes, namely PDE4A, 4B, 4C, and 4D, with at least 25 splice variants. The protein sequences of the catalytic domains of the four subtypes of PDE4 are highly homologous; therefore, inhibitors that act on the catalytic domain do not exhibit subtype selectivity. Most of the classical PDE4 inhibitors have been reported to act on the catalytic domain. A new mode of action of PDE4 inhibitors has been reported in recent years, in which the inhibitor acts on both the catalytic domain and a regulatory sequence, so that the regulatory sequence can stabilize the closed conformation of the protein, thereby preventing cAMP from entering, thus exerting an inhibitory effect. However, it has been found from research that there are amino acid differences between PDE4B and 4D in this regulatory sequence, so designing inhibitors based on such differences can produce subtype selectivity. Therefore, based on the difference of the two amino acids PDE4B Leu674/PDE4D Gln594 in CR3 (Conserved Region 3) of the downstream regulatory sequence, it is expected to achieve the selectivity to subtype B and reduce the side effects of the inhibitors while maintaining the activity.

Selective PDE4B inhibitors with good activity, high safety and minor side effects are found, which have a good clinical development prospect and can be used for treating COPD or cancers or other proliferative diseases or conditions.

Summary of the Invention

The present disclosure provides a small molecule compound with PDE4B inhibitory activity, or a stereoisomer or pharmaceutically acceptable salt thereof, wherein the compound is as represented by formula (I), and has a high activity, relatively low toxic and side effects, excellent pharmacokinetic characteristics, and bioavailability;

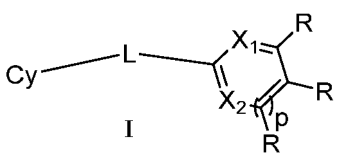
I

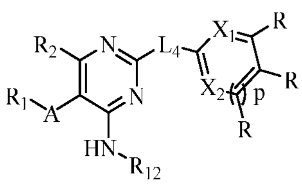
II

,

wherein when Cy is selected from Cy1, Cy2, Cy3, Cy8, and Cy10, L is $-(L_1)n-(L_2)m-$;

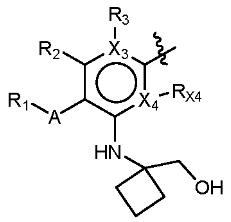
Cy1 ,
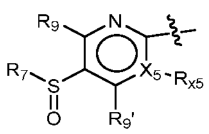
Cy2 ,
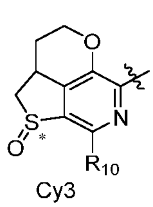
Cy3 ,
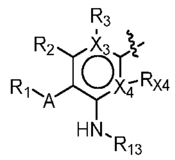
Cy8 , and
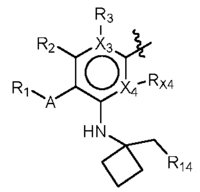
Cy10 ;

when Cy is selected from Cy4, Cy5, and Cy6, L is $-L_3-$;

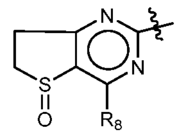
Cy4 ,
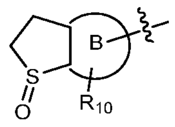
Cy5 ,
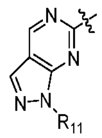
Cy6 or
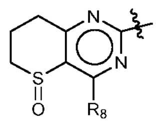
Cy11 ;

when Cy is selected from Cy7 and Cy9, L is $-L_4-$;

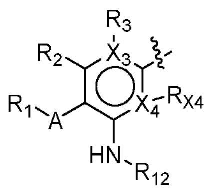
Cy7 and
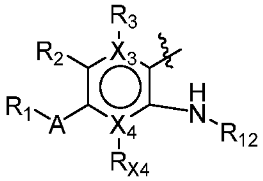
Cy9 ;

$L_1$ is $-NR_4-$, $-CR_5=N-$, $-CR_5=CR_5-$, or $-CR_5R_5-$; in all the solutions of the present disclosure, unless otherwise specially specified, the site of attachment of $L_1$ is arbitrary, for example, as for $L_1(-CR_2=N-)$ that is connected to $L_2$, the N atom and C atom thereof can both be used as the site of attachment to $L_2$;

$L_2$ is 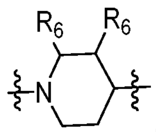 , 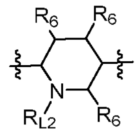 , 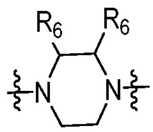 , 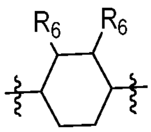 , 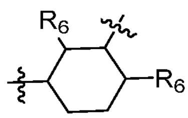 , 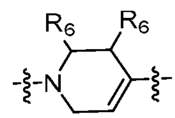 ,

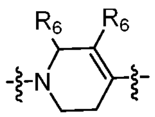 , 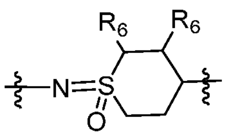 , 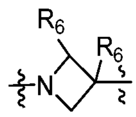 ,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; in some embodiments, each $L_2$ is

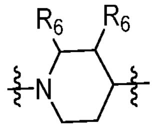, 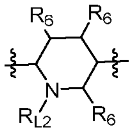, 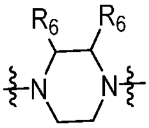,

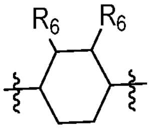, 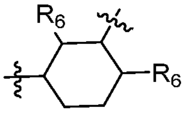, 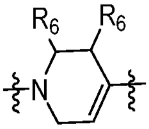, 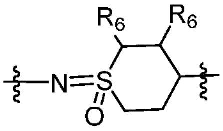, 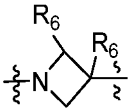,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; in some embodiments, each $L_2$ is independently selected from

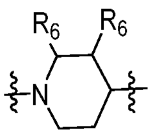, 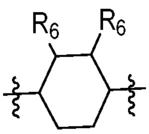, 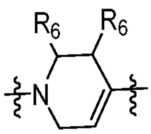,

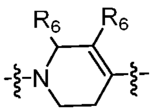, 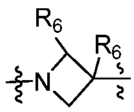,

CO, O, $NR_{L2}$, $C_{1-4}$ alkylene, $C_{2-4}$ alkenylene, $C_{2-4}$ alkynylene,

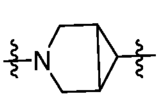 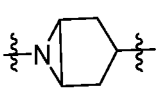 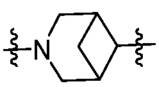 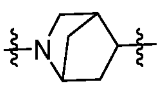 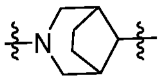 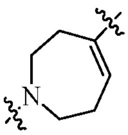

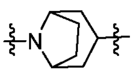 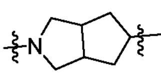 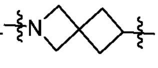 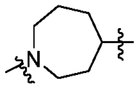 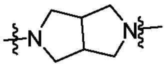

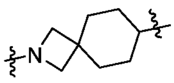 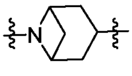 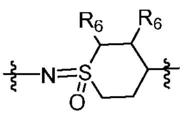 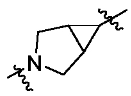 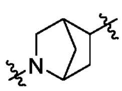 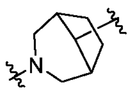

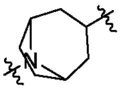 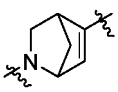 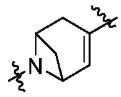 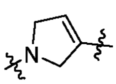 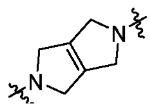 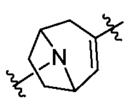 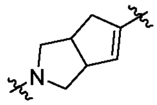

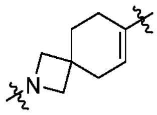 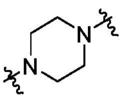 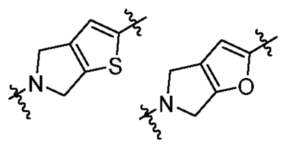 ;

in some embodiments, each $L_2$ is independently selected from

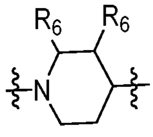, 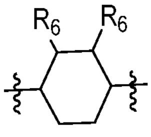, 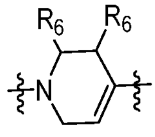,

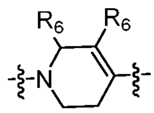, 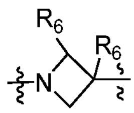,

CO, O, $NR_{L2}$, $C_{1-4}$ alkylene, $C_{2-4}$ alkenylene, $C_{2-4}$ alkynylene,

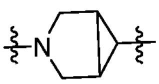 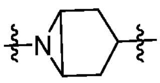 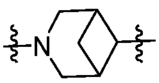 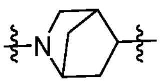 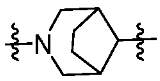 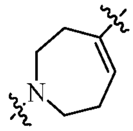

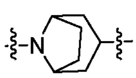 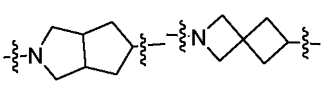 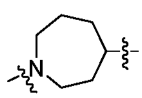 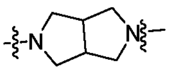

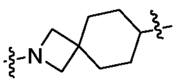 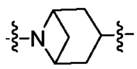 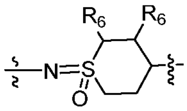 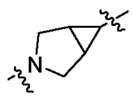 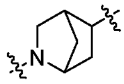 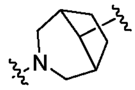

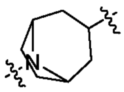 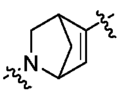 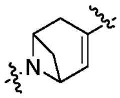 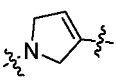 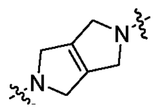 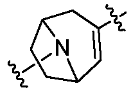 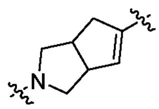

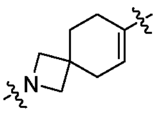 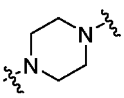 ;

in some embodiments, each $L_2$ is independently selected from

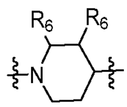, 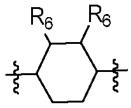,

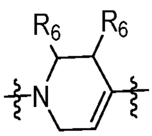, 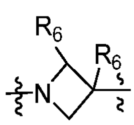,

CO, O, $NR_{L2}$, $C_{1-4}$ alkylene, $C_{2-4}$ alkenylene, $C_{2-4}$ alkynylene,

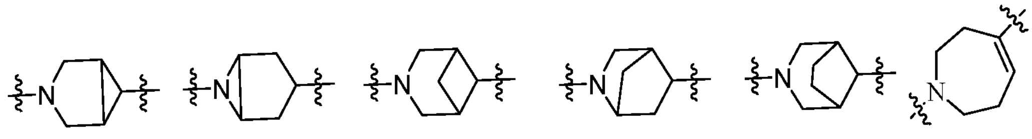
;

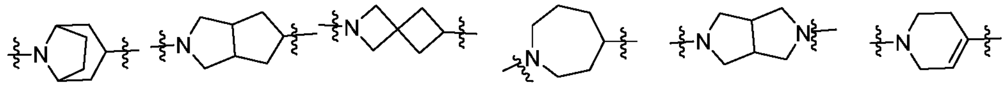

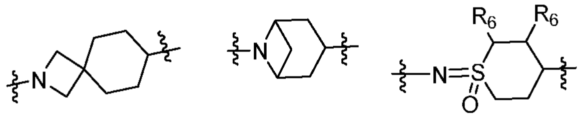

in all the solutions of the present disclosure, unless otherwise specially specified, the sites of attachment of $L_2$ to the left and right groups are arbitrary, for example, in $L_2$

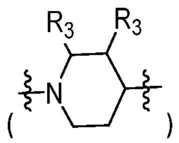

as one end, the N atom and C atom thereof can both be used as the site of attachment to Cy;

$R_{L2}$ is H, $C_{1-4}$ alkyl, or $C_{3-6}$ cycloalkyl; in some embodiments, $R_{L2}$ is H, $C_{1-4}$ alkyl, or $C_{3-6}$ cycloalkyl; in some embodiments, $R_{L2}$ is H, methyl, ethyl, propyl, or isopropyl;

$L_3$ is heterocycloalkylene or heterocycloalkylene-$(CH_2)$,-, wherein the heterocycloalkylene is a 4-to 10-membered heterocycle containing 1-3 heteroatoms selected from N, S, and O and is optionally substituted with 1-3 $R_{L3}$; in some embodiments, $L_3$ is selected from

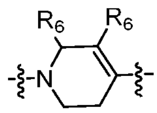
,

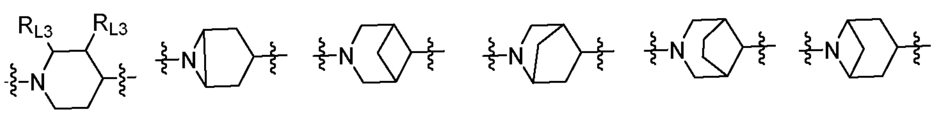

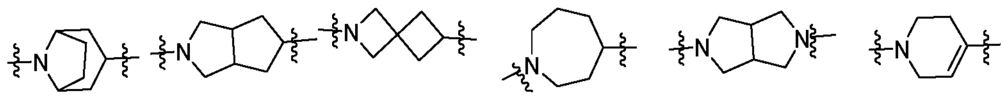

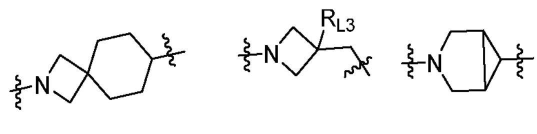

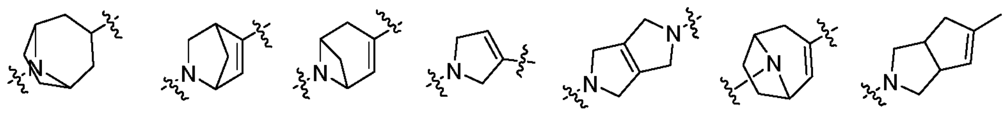

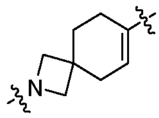 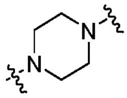 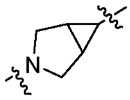 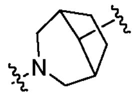;

in some embodiments, $L_3$ is selected from

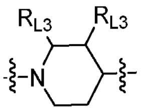 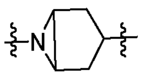 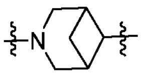 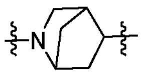 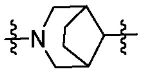 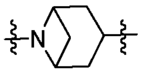

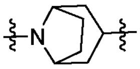 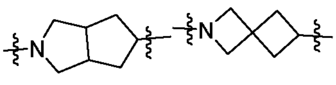 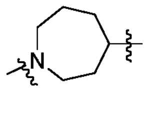 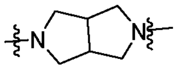 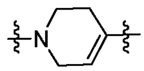

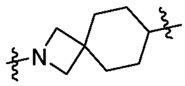 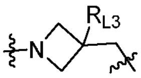 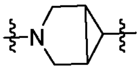;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O; in some embodiments, each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form a $C_{3-6}$ cycloalkyl or a 5- to 6-membered heterocycloalkyl containing 1-3 heteroatoms selected from N, S, and O;

$L_4$ is

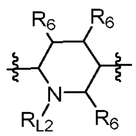, 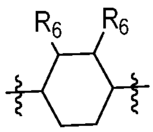, 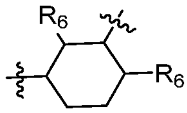, 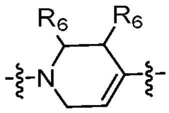, 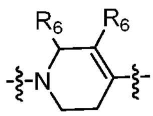,

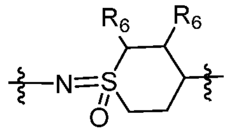, 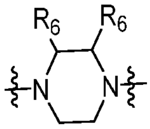,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; in some embodiments, $L_4$ is

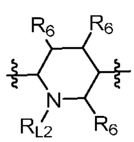, 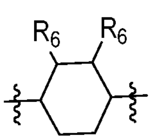, 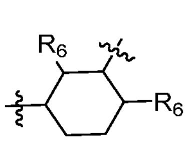, 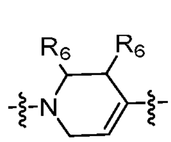,

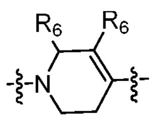, 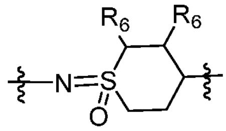,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; in some embodiments, $L_4$ is

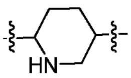, 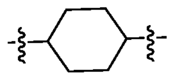, 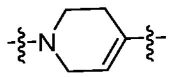, 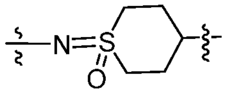,

a 5- to 6-membered heterocycloalkyl fused 5- to 6-membered heterocycloalkyl containing 1-3 heteroatoms selected from N, S, and O, or a 5- to 6-membered heterocycloalkyl fused 5- to 6-membered cycloalkyl containing 1-3 heteroatoms selected from N, S, and O, wherein the 5- to 6-membered heterocycloalkyl fused 5- to 6-membered heterocycloalkyl and 5- to 6-membered heterocycloalkyl fused 5- to 6-membered cycloalkyl are optionally substituted with 1-3 groups selected from halogen, =O, CN, $C_{1-4}$ alkyl, $C_{1-4}$ alkoxy, OH, and $NH_2$; in some embodiments, $L_4$ is selected from

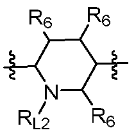, 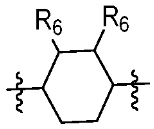, 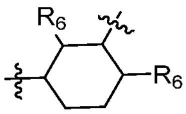, 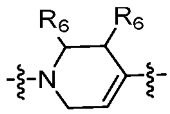, 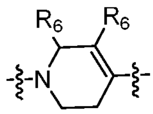,

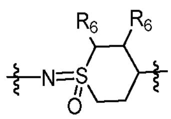, 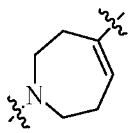, 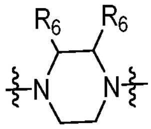,

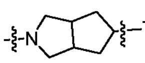 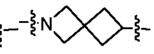 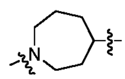 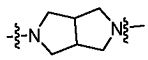 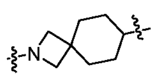 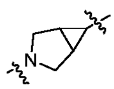 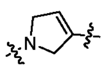

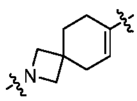 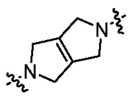 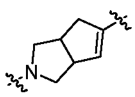 ;

in some embodiments, $L_4$ is selected from

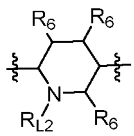, 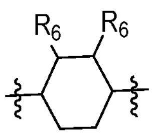, 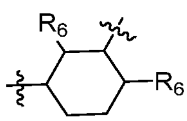, 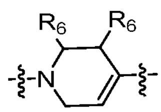,

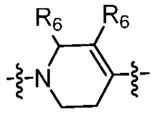, 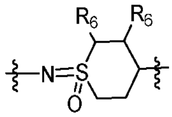,

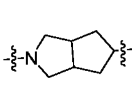 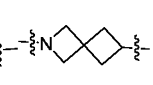 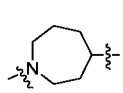 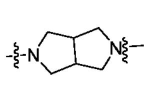 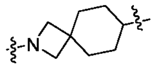 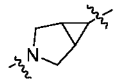 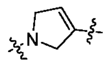

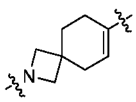 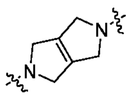 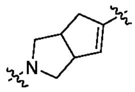;

in some embodiments, $L_4$ is

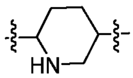, 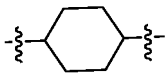, 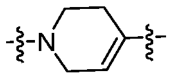, 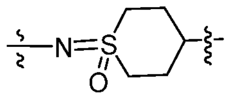, 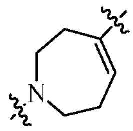,

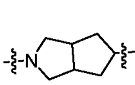 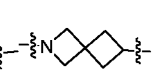 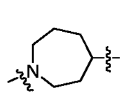 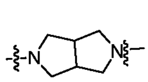 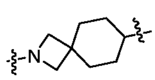 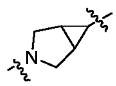 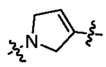

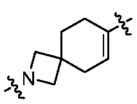 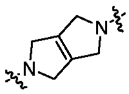 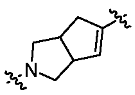;

in some embodiments, $L_4$ is

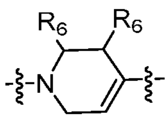, 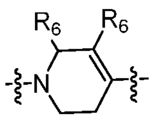, 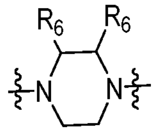,

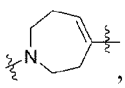, 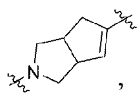, 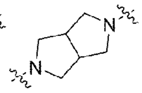;

in some embodiments, $L_4$ is

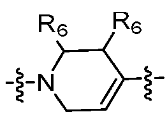, 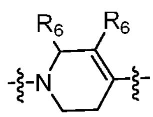,

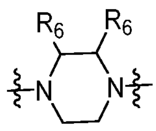;

in some embodiments, $L_4$ is

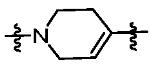, 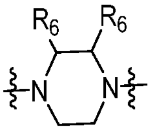;

A is -S(O)-, -C(O)-, -B(OH)-, -S-, -S(=N)-CN, or -C(=N)-CN; in some embodiments, A is -S(O)-, -C(O)-, -B(OH)-, -S-, or -S(=N)-CN; in some embodiments, A is -S(O)-;

$X_1$ and $X_2$ are independently $CR_{X1}$, O, S, or N; in some embodiments, $X_1$ and $X_2$ are independently $CR_{X1}$, S, or N; in some embodiments, $X_1$ and $X_2$ are independently S, or N; in some embodiments, $X_1$ and $X_2$ are independently $CR_{X1}$ or N; in some embodiments, $X_1$ and $X_2$ are $CR_{X1}$; in some embodiments, $X_1$ and $X_2$ are independently N;

$X_3$, $X_4$, and $X_5$ are independently C or N; in some embodiments, $X_3$ and $X_4$ are independently N; ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O; in some embodiments, Cy5 is selected from

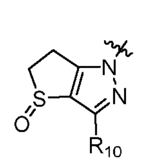 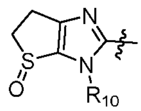 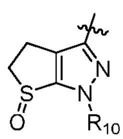 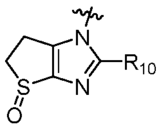 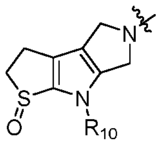;

each $R_{X1}$ and R are independently H, halogen, $C_{1-4}$ alkyl, $C_{1-4}$ alkoxy, $-N(CH_3)_2$, $-NH(CH_3)$, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, $C_{3-6}$ cycloalkyl, $C_{2-6}$ alkynyl, and $NH_2$; in some embodiments, each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, $C_{3-6}$ cycloalkyl, $C_{2-6}$ alkynyl, and $NH_2$; in some embodiments, each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $R_{X1}$ and R are independently 3- to 5-membered cycloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen,

=O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, and O, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or two R on adjacent ring atoms, R and $R_{X1}$, or $R_{X1}$ and $R_6$, together with the atom to which they are attached, form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; in some embodiments, each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, $R_1$ is $C_{1-4}$ alkyl, or $C_{1-4}$ haloalkyl, or $R_1$ and $R_2$, together with the atom to which they are attached, form a 5- to 7-membered monocyclic heterocycloalkyl , a 6- to 8-membered fused heterocycloalkyl, a 7- to 9-membered spiro heterocycloalkyl, or a 5- to 6-membered heteroaryl containing 1-3 heteroatoms selected from N, S, B, and O, wherein the monocyclic heterocycloalkyl, fused heterocycloalkyl, spiro heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, $R_1$ is $C_{1-4}$ alkyl, or $C_{1-4}$ haloalkyl; or $R_1$ and $R_2$, together with the atom to which they are attached, form a ring selected from:

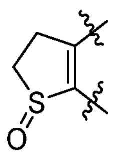
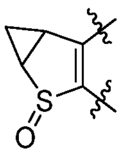
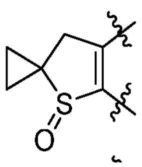
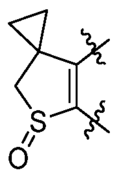
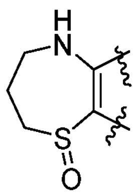
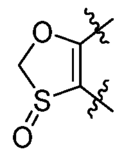
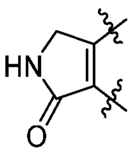
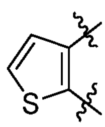
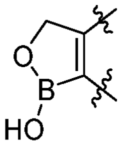
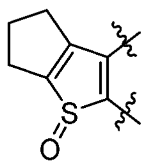
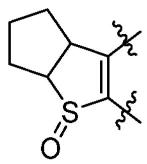
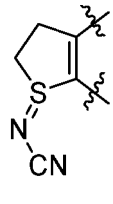
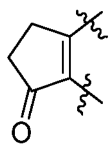
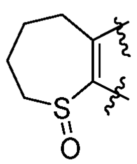
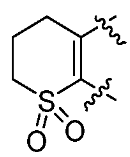
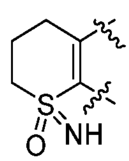
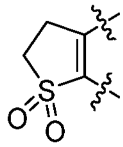
,

optionally substituted with 1-3 groups selected from halogen, =O, CN, $C_{1-4}$ alkyl, $C_{1-4}$ alkoxy, OH, and $NH_2$; or $R_1$ and $R_2$, together with the atom to which they are attached, form a ring selected from:

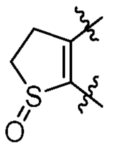 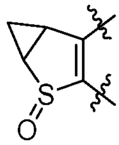 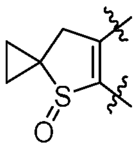 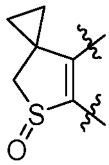 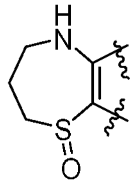 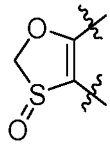

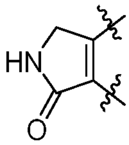 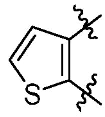 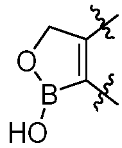 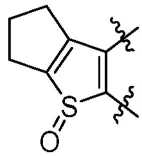 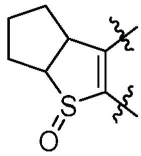 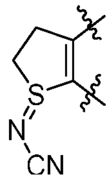

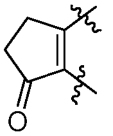 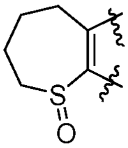 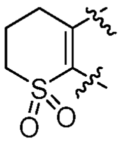 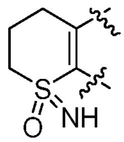 ,

optionally substituted with 1-3 groups selected from halogen, =O, CN, $C_{1-4}$ alkyl, $C_{1-4}$ alkoxy, OH, and $NH_2$; or $R_1$ and $R_2$, together with the atom to which they are attached, form a ring selected from:

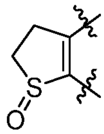 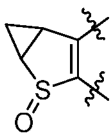 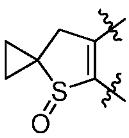 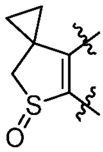 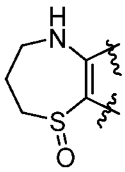 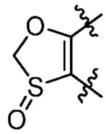

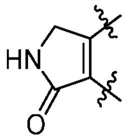 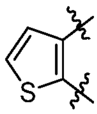 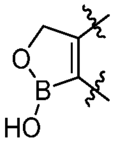 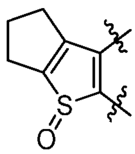 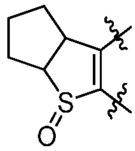 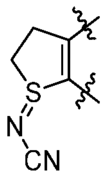

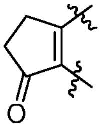 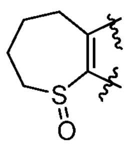 ,

optionally substituted with 1-3 groups selected from halogen, =O, CN, $C_{1-4}$ alkyl, $C_{1-4}$ alkoxy, OH, and $NH_2$; or $R_1$ and $R_2$, together with the atom to which they are attached, form a ring selected from:

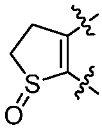 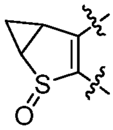 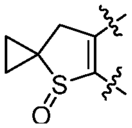 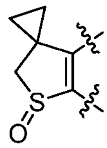 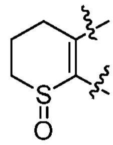 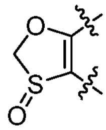

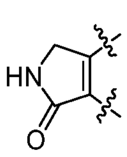 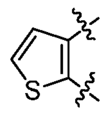 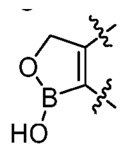 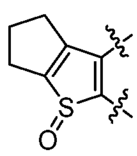 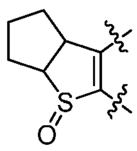 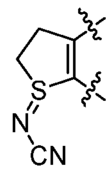

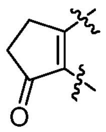 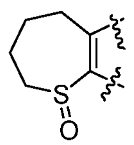 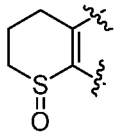 ,

optionally substituted with 1-3 groups selected from halogen, =O, CN, $C_{1-4}$ alkyl, $C_{1-4}$ alkoxy, OH, and $NH_2$; in some embodiments, $R_1$ and $R_2$, together with the atom to which they are attached, form a ring selected from:

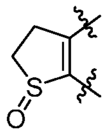 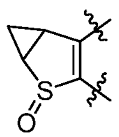 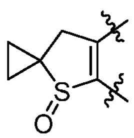 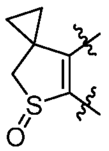 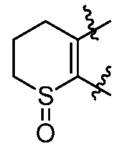 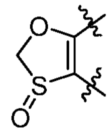

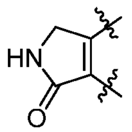 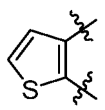 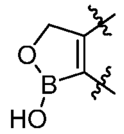 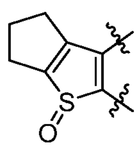 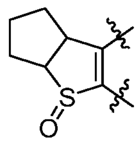 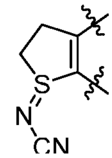

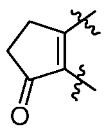 ,

optionally substituted with 1-3 groups selected from halogen, =O, CN, $C_{1-4}$ alkyl, $C_{1-4}$ alkoxy, OH, and $NH_2$; $R_2$ is H, $C_{1-4}$ alkyl, or $C_{1-4}$ haloalkyl; in some embodiments, $R_2$ is H, $C_{1-4}$ alkyl, or $C_{1-4}$ haloalkyl; in some embodiments, $R_3$ and $R_2$, together with the atom to which they are attached, form a ring selected from:

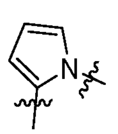 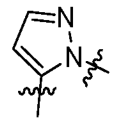 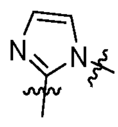 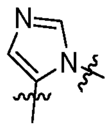 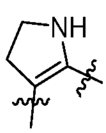 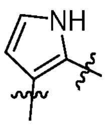 ,

optionally substituted with 1-3 groups selected from halogen, =O, CN, $C_{1-4}$ alkyl, $C_{1-4}$ alkoxy, OH, and $NH_2$; $R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; in some embodiments, $R_3$ is H, halogen, $C_{1-4}$ alkyl, CN, OH, or absent; or $R_3$ and $R_2$, together with the atom to which they are attached, form a 5- to 6-membered heterocycloalkyl or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or $R_3$ and $R_6$, together with the atom to which they are attached, form $C_{5-7}$ cycloalkyl, a 5- to 6-membered

heterocycloalkyl, or a 5- to 6-membered heteroaryl containing 1-3 heteroatoms selected from N, S, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

in some embodiments, $R_3$ is H, halogen, $C_{1-4}$ alkyl, CN, OH, or absent;

or $R_3$ and $R_2$, together with the atom to which they are attached, form a ring selected from:

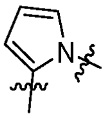 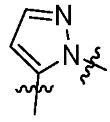 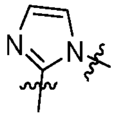 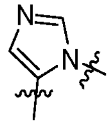 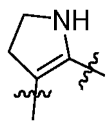 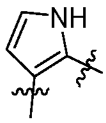 ,

optionally substituted with 1-3 groups selected from halogen, =O, CN, $C_{1-4}$ alkyl, $C_{1-4}$ alkoxy, OH, and $NH_2$;

or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$; $R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; in some embodiments, $R_{x4}$ is H, halogen, $C_{1-4}$ alkyl, CN, OH, or absent, or $R_{x4}$ and $R_4$ or $R_{x4}$ and $R_5$, together with the atom to which they are attached, form a 5- to 6-membered heteroaryl containing 1-3 heteroatoms selected from N, S, and O, wherein the heteroaryl is optionally substituted with 1-3 groups selected from halogen, =O, CN, $C_{1-4}$ alkyl, $C_{1-4}$ alkoxy, OH, and $NH_2$; in some embodiments, $R_{x4}$ is H, halogen, $C_{1-4}$ alkyl, CN, OH, or absent; or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl; in some embodiments, $R_4$ is H, $C_{1-4}$ alkyl, or $C_{1-4}$ haloalkyl; in some embodiments, $R_4$ is H, methyl, ethyl, propyl, or isopropyl, or halogenated methyl, ethyl, propyl, or isopropyl, with the halogen including F, Cl, Br, and I;

$R_5$ is H, $C_{1-4}$ alkyl, $C_{1-4}$ haloalkyl, $C_{1-4}$ alkoxy, $-NHC_{1-4}$ alkyl, or $C_{3-6}$ cycloalkyl; in some embodiments, $R_5$ is H, $C_{1-4}$ alkyl, $C_{1-4}$ haloalkyl, $C_{1-4}$ alkoxy, or $-NHC_{1-4}$ alkyl;

$R_6$ is H, halogen, =O, CN, $C_{1-4}$ alkyl, or $C_{1-4}$ alkoxy; in some embodiments, $R_6$ is H, halogen, =O, CN, methyl, ethyl, propyl, isopropyl, methoxy, ethoxy, propoxy, or isopropoxy;

$R_{x5}$ is $C_{1-4}$ alkyl, CN, OH, or absent; in some embodiments, $R_{x5}$ is methyl, ethyl, propyl, isopropyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$; in some embodiments, $R_7$ is $C_{1-4}$ alkyl, or $C_{1-4}$ haloalkyl, or $R_7$ and $R_9$, together with the atom to which they are attached, form a 5- to 6-membered heterocycloalkyl containing 1-3 heteroatoms selected from N, S, and O, wherein the heterocycloalkyl is optionally substituted with 1-3 groups selected from halogen, CN, $C_{1-4}$ alkyl, $C_{1-4}$ alkoxy, OH, and $NH_2$; in some embodiments, $R_7$ is $C_{1-4}$ alkyl, or $C_{1-4}$ haloalkyl, or $R_7$ and $R_9$, together with the atom to which they are attached, form a ring selected from:

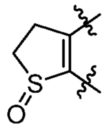 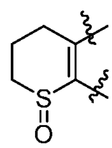 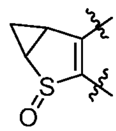 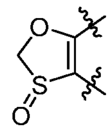 ,

wherein the ring is optionally substituted with 1-3 groups selected from halogen, CN, $C_{1-4}$ alkyl, $C_{1-4}$ alkoxy, OH, and $NH_2$;

in the present disclosure, at least one group of $R_{9'}$ and $R_7$ or $R_{9'}$ and $R_{x5}$, together with the atom to which they are

attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; in some embodiments, at least one group of $R_{9'}$ and $R_7$ or $R_{9'}$ and $R_{x5}$, together with the atom to which they are attached, form a 5- to 7-membered monocyclic heterocycloalkyl containing 1-3 heteroatoms selected from N, S, and O, a 7- to 10-membered spiro heterocycloalkyl, or a 5- to 6-membered heteroaryl, wherein the monocyclic heterocycloalkyl, spiro heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; and in some embodiments, $R_{9'}$ and $R_7$, together with the atom to which they are attached, form a ring selected from:

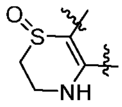 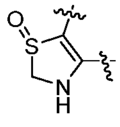 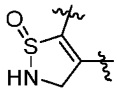 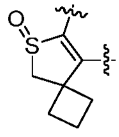 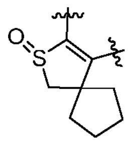,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

or $R_{9'}$ and $R_{x5}$, together with the atom to which they are attached, form

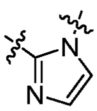 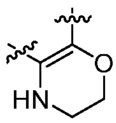 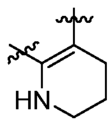,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; provided that at least one group of $R_{9'}$ and $R_7$ or $R_{9'}$ and $R_{x5}$ form a ring;

$R_8$ is $R_{8'}$, $-OR_{8'}$, or $-(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, or $-N=S(=O)(C_{1-4}\ alkyl)_2$, or -N(C3-6 cycloalkyl)C(O)NH(C1-4 alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN; in some embodiments, $R_{8'}$ is $C_{3-6}$ cycloalkyl, a 4- to 8-membered heterocycloalkyl containing 1-3 heteroatoms selected from N, S, and O, or a 5- to 6-membered heteroaryl containing 1-3 heteroatoms selected from N, S, and O, wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN; in some embodiments, $R_{8'}$ is $C_{3-6}$ cycloalkyl, wherein the cycloalkyl is optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl; in some embodiments, $R_9$ is H or $C_{1-4}$ alkyl; in some embodiments, $R_9$ is H, methyl, ethyl, propyl, or isopropyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl; in some embodiments, the cycloalkyl is optionally substituted with 1-3 F, Cl, Br, I, CN, =O, OH, methyl, ethyl, propyl, isopropyl, hydroxymethyl, hydroxyethyl, hydroxypropyl, or hydroxyisopropyl, or halogenated methyl, ethyl, propyl, or isopropyl, etc., with the halogen including F, Cl, Br, and I;

$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, $C_{1-4}$ alkyl-CN, -CRaRb=N-O-$C_{1-4}$ alkyl, -C(NRaRb)=N-CN, -C($C_{1-4}$ alkyl)=N-CN, -C(NRaRb)=CRa-$NO_2$, $-C(CH_3)_2-CH_2-O-C(O)NH_2$, $-C(CH_3)_2-CH_2-NH-C(O)O(C_{1-4}\ alkyl)$, $-C(CH_3)_2-CH_2-O-C(O)NHCH_3$, $-CH(CH_3)-CH_2-O-C(O)NH_2$, $-CH(CH_3)-CH_2-NH-C(O)O(C_{1-4}\ alkyl)$, $-CH(CH_3)-CH_2-O-C(O)NHCH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from halogen, CN, =O, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t-NRa-C(O)NH_2$, $-(CH_2)_t-NRa-C(=NH)NH_2$, $-(CH_2)_t-NRa-C(O)-O-(C_{1-4}\ alkyl)$, $-(CH_2)_t-NRa-C(C_{1-4}\ alkyl)=N-CN$, $-(CH_2)_t-NRa-C(NRaRb)=N-CN$, $-(CH_2)_t-NRa-C(NRaRb)=CRa-NO_2$, $-(CH_2)_t-C(NRaRb)=N-CN$, $-(CH_2)_t-C(C_{1-4}\ alkyl)=N-CN$, $-(CH_2)_t-C(C_{1-4}\ alkyl)=N-O-(C_{1-4}\ alkyl)$, =N-O-$C_{1-4}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cy-

cloalkyl, $-(CH_2)_t$-COOH, $-(CH_2)_t$-NRaRb, $-(CH_2)_t$-C(O)NRaRb, and $-(CH_2)_t$-NRa-C(O)$CH_3$;

in some embodiments, $R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, -CRaRb=N-O-C1-4 alkyl, -C(NRaRb)=N-CN, -C($C_{1-4}$ alkyl)=N-CN, -C(NRaRb)=CRa-$NO_2$, -C$(CH_3)_2$-$CH_2$-O-C(O)$NH_2$, -C$(CH_3)_2$-$CH_2$-NH-C(O)O($C_{1-4}$ alkyl), -C$(CH_3)_2$-$CH_2$-O-C(O)NH$CH_3$, - CH($CH_3$)-$CH_2$-O-C(O)$NH_2$, -CH($CH_3$)-$CH_2$-NH-C(O)O($C_{1-4}$ alkyl), -CH($CH_3$)-$CH_2$-O-C(O)NH$CH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from halogen, CN, =O, OH, -$SF_5$, $C_{1-4}$ alkyl, $C_{1-4}$ alkyl-CN, hydroxy $C_{1-4}$ alkyl, $C_{1-4}$ haloalkyl, $-(CH_2)_t$-O-C(O)$NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, a 5- to 6-membered heteroaryl, $-(CH_2)_t$-O-$C_{1-4}$ alkyl, $-(CH_2)_t$-O-C(O)NH$CH_3$, $-(CH_2)_t$-O-$C_{1-2}$ haloalkyl, - $(CH_2)_t$-O-$C_{3-6}$ cycloalkyl, $-(CH_2)_t$-O-$C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$;

in some embodiments, $R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, -CRaRb=N-O-$C_{1-4}$ alkyl, -C(NRaRb)=N-CN, -C($C_{1-4}$ alkyl)=N-CN, -C(NRaRb)=CRa-$NO_2$, -C$(CH_3)_2$-$CH_2$-O-C(O)$NH_2$, -CH($CH_3$)-$CH_2$-O-C(O)$NH_2$, -C(O)-Ra, -CH($CH_3$)-$CH_2$-O-C(O)NH$CH_3$, or -C$(CH_3)_2$-$CH_2$-O-C(O)NH$CH_3$, wherein the heterocycloalkylene, cycloalkylene, arylene, and heteroarylene are optionally further substituted with 1-3 groups from halogen, CN, =O, OH, -$SF_5$, $C_{1-4}$ alkyl, hydroxy $C_{1-4}$ alkyl, $C_{1-4}$ haloalkyl, $-(CH_2)_t$-O-C(O)$NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, a 5- to 6-membered heteroaryl, $-(CH_2)_t$-O-$C_{1-4}$ alkyl, $-(CH_2)_t$-O-C(O)NH$CH_3$, $-(CH_2)_t$-O-$C_{1-2}$ haloalkyl, $-(CH_2)_t$-O-$C_{3-6}$ cycloalkyl, $-(CH_2)_t$-O-$C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$; in some embodiments, $R_{12}$ is selected from a 4- to 10-membered heterocycloalkylene, a 3- to 10-membered cycloalkylene, a 6- to 10-membered arylene, a 5- to 10-membered heteroarylene, hydroxy $C_{1-6}$ alkyl, -CRaRb=N-O-$C_{1-4}$ alkyl, -C(NRaRb)=N-CN, -C($C_{1-4}$ alkyl)=N-CN, -C(NRaRb)=CRa-$NO_2$, or -C$(CH_3)_t$-$CH_2$-O-C(O)NH$CH_3$, wherein the heterocycloalkylene, cycloalkylene, arylene, and heteroarylene are optionally further substituted with 1-3 groups from halogen, CN, =O, OH, -$SF_5$, $C_{1-4}$ alkyl, hydroxy $C_{1-4}$ alkyl, $C_{1-4}$ haloalkyl, $-(CH_2)_t$-O-C(O)$NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, and a 5- to 6-membered heteroaryl; in some embodiments, $R_{12}$ is selected from a 4- to 10-membered heterocycloalkylene, a 3- to 10-membered cycloalkylene, a 6- to 10-membered arylene, or a 5- to 10-membered heteroarylene, wherein the heterocycloalkylene, cycloalkylene, arylene, and heteroarylene are optionally further substituted with 1-3 groups from halogen, CN, =O, OH, $C_{1-4}$ alkyl, hydroxy $C_{1-4}$ alkyl, and $C_{1-4}$ haloalkyl; in some embodiments, $R_{12}$ is selected from a 4- to 10-membered heterocycloalkylene and a 3- to 10-membered cycloalkylene, wherein the heterocycloalkylene and cycloalkylene are optionally further substituted with 1-3 groups from halogen, CN, =O, OH, $C_{1-4}$ alkyl, hydroxy $C_{1-4}$ alkyl, and $C_{1-4}$ haloalkyl; in some embodiments, $R_{12}$ is selected from a 4- to 6-membered monocyclic heterocycloalkylene, a 6- to 10-membered fused heterocarbocyclylene, a 7- to 8-membered bridged heterocarbocyclylene, a 7- to 10-membered spiro heterocarbocyclylene, a 3- to 6-membered monocyclic cycloalkylene, a 6- to 10-membered fused carbocyclylene, a 7- to 8-membered bridged carbocyclylene, and a 7- to 10-membered spiro carbocyclic ring, wherein the monocyclic heterocycloalkylene, fused heterocarbocyclylene, bridged heterocarbocyclylene, spiro heterocarbocyclylene, monocyclic cycloalkylene, fused carbocyclylene, bridged carbocyclylene, and spiro carbocyclic ring are optionally further substituted with 1-3 groups from halogen, CN, =O, OH, $C_{1-4}$ alkyl, hydroxy $C_{1-4}$ alkyl, and $C_{1-4}$ haloalkyl; in some embodiments, $R_{12}$ is selected from cyclobutyl, cyclopentyl, cyclohexyl, phenyl, cyclopropyl, adamantyl, tetrahydropyranyl, piperidinyl, oxolanyl, oxanyl,

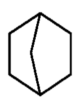,

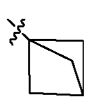 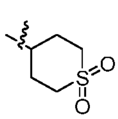 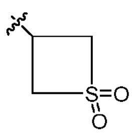 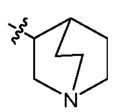 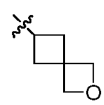 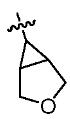 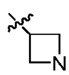, 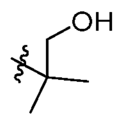,

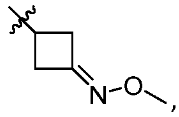, 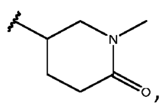,

$C_{1-4}$ alkyl-CN, -C(NHC$_{1-2}$ alkyl)=N-CN, -C(C$_{1-2}$ alkyl)=N-CN, -C(NHC$_{1-2}$ alkyl)=CH-NO$_2$, -CH(CH$_3$)-CH$_2$-NH-C(O)O(CH$_3$), -C(CH$_3$)$_2$-CH$_2$-NH-C(O)O(CH$_3$), -CH(CH$_3$)-CH$_2$-O-C(O)NH$_2$, -C(CH$_3$)$_2$-CH$_2$-O-C(O)NH$_2$, -C(O)-Ra, -CH(CH$_3$)-CH$_2$-O-C(O)NHCH$_3$, or -C(CH$_3$)$_2$-CH$_2$-O-C(O)NHCH$_3$; wherein the ring is optionally substituted with 1-3 groups selected from halogen, CN, OH, -SF$_5$, $C_{1-4}$ alkyl, $C_{1-4}$ alkyl-CN, hydroxy $C_{1-4}$ alkyl, $C_{1-4}$ haloalkyl, -(CH$_2$)$_t$-O-C(O)NH$_2$, -(CH$_2$)$_t$-NH-C(O)NH$_2$, -(CH$_2$)$_t$-NH-C(=NH)NH$_2$, -(CH$_2$)$_t$-NH-C(O)-O-(C$_{1-2}$ alkyl), -(CH$_2$)$_t$-NH-C(C$_{1-2}$ alkyl)=N-CN, - (CH$_2$)$_t$-NH-C(NHC$_{1-2}$ alkyl)=N-CN, -(CH$_2$)$_t$-NH-C(NHC$_{1-2}$ alkyl)=CH-NO$_2$, -(CH$_2$)$_t$-C(NHC$_{1-2}$ alkyl)=N-CN, -(CH$_2$)$_t$-C(C$_{1-2}$ alkyl)=N-CN, -(CH$_2$)$_t$-C(C$_{1-2}$ alkyl)=N-O-(C$_{1-2}$ alkyl), =N-O-C$_{1-2}$ alkyl, a 5- to 6-membered heteroaryl, -(CH$_2$)$_t$-O-C$_{1-4}$ alkyl, -(CH$_2$)$_t$-O-C(O)NHCH$_3$, -(CH$_2$)$_t$-O-C$_{1-2}$ haloalkyl, -(CH$_2$)$_t$-O-C$_{3-6}$ cycloalkyl, -(CH$_2$)$_t$-O-C$_{1-4}$ alkyl-O-C$_{3-6}$ cycloalkyl, -(CH$_2$)$_t$-COOH, -(CH$_2$)$_t$-NRaRb, -(CH$_2$)$_t$-C(O)NRaRb, and -(CH$_2$)$_t$-NRa-C(O)CH$_3$; in some embodiments, $R_{12}$ is selected from cyclobutyl, cyclopentyl, cyclohexyl, phenyl, cyclopropyl, adamantyl, tetrahydropyranyl, piperidinyl, oxolanyl, oxanyl,

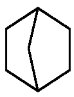, 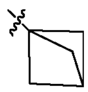 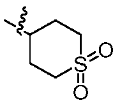 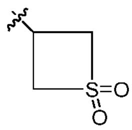 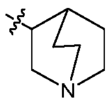 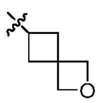 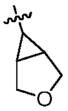 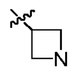,

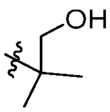, 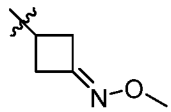, 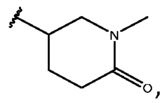,

-C(NHC$_{1-2}$ alkyl)=N-CN, -C(C$_{1-2}$ alkyl)=N-CN, -C(NHC$_{1-2}$ alkyl)=CH-NO$_2$, -C(CH$_3$)$_2$-CH$_2$-NH-C(O)O(CH$_3$), -C(CH$_3$)$_2$-CH$_2$-O-C(O)NH$_2$, -CH(CH$_3$)-CH$_2$-NH-C(O)O(CH$_3$), -CH(CH$_3$)$_2$-CH$_2$-O-C(O)NH$_2$, -C(O)-Ra, -C(CH$_3$)$_2$-CH$_2$-O-C(O)NHCH$_3$, or -CH(CH$_3$)-CH$_2$-O-C(O)NHCH$_3$; wherein the ring is optionally substituted with 1-3 groups selected from halogen, CN, OH, -SF$_5$, $C_{1-4}$ alkyl, $C_{1-4}$ alkyl-CN, hydroxy $C_{1-4}$ alkyl, $C_{1-4}$ haloalkyl, -(CH$_2$)$_t$-O-C(O)NH$_2$, -(CH$_2$)$_t$-NH-C(O)NH$_2$, -(CH$_2$)$_t$-NH-C(=NH)NH$_2$, -(CH$_2$)$_t$-NH-C(O)-O-(C$_{1-2}$ alkyl), -(CH$_2$)$_t$-NH-C(C$_{1-2}$ alkyl)=N-CN, - (CH$_2$)$_t$-NH-C(NHC$_{1-2}$ alkyl)=N-CN, -(CH$_2$)$_t$-NH-C(NHC$_{1-2}$ alkyl)=CH-NO$_2$, -(CH$_2$)$_t$-C(NHC$_{1-2}$ alkyl)=N-CN, -(CH$_2$)$_t$-C(C$_{1-2}$ alkyl)=N-CN, -(CH$_2$)$_t$-C(C$_{1-2}$ alkyl)=N-O-(C$_{1-2}$ alkyl), =N-O-C$_{1-2}$ alkyl, a 5- to 6-membered heteroaryl, -(CH$_2$)$_t$-O-C$_{1-4}$ alkyl, -(CH$_2$)$_t$-O-C(O)NHCH$_3$, -(CH$_2$)$_t$-O-C$_{1-2}$ haloalkyl, -(CH$_2$)$_t$-O-C$_{3-6}$ cycloalkyl, -(CH$_2$)$_t$-O-C$_{1-4}$ alkyl-O-C$_{3-6}$ cycloalkyl, -(CH$_2$)$_t$-COOH, -(CH$_2$)$_t$-NRaRb, -(CH$_2$)$_t$-C(O)NRaRb, and -(CH$_2$)$_t$-NRa-C(O)CH$_3$; in some embodiments, $R_{12}$ is selected from cyclobutyl, cyclopentyl, cyclohexyl, phenyl, cyclopropyl, adamantyl, tetrahydropyranyl, piperidinyl, oxolanyl, oxanyl,

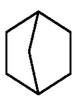, 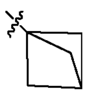 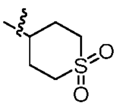 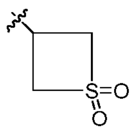 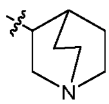 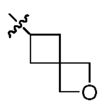 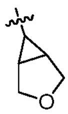 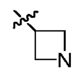,

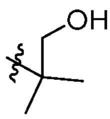, 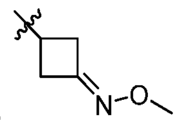,

-C(NHC$_{1-2}$ alkyl)=N-CN, -C(C$_{1-2}$ alkyl)=N-CN, -C(NHC$_{1-2}$ alkyl)=CH-NO$_2$, - C(CH$_3$)$_2$-CH$_2$-NH-C(O)O(CH$_3$), -C(CH$_3$)$_2$-CH$_2$-O-C(O)NH$_2$, -CH(CH$_3$)-CH$_2$-NH-C(O)O(CH$_3$), -CH(CH$_3$)-CH$_2$-O-C(O)NH$_2$, -C(O)-Ra, -C(CH$_3$)$_2$-CH$_2$-O-C(O)NHCH$_3$, or -CH(CH$_3$)-CH$_2$-O-C(O)NHCH$_3$; wherein the ring is optionally substituted with 1-3 groups selected from halogen, CN, OH, -SF$_5$, $C_{1-4}$ alkyl, $C_{1-4}$ alkyl-CN, hydroxy $C_{1-4}$ alkyl, $C_{1-4}$ haloalkyl, -(CH$_2$)$_t$-O-C(O)NH$_2$, -(CH$_2$)$_t$-NH-C(O)NH$_2$, -(CH$_2$)$_t$-NH-C(=NH)NH$_2$, -(CH$_2$)$_t$-NH-C(O)-O-(C$_{1-2}$ alkyl), -(CH$_2$)$_t$-NH-C(C$_{1-2}$ alkyl)=N-CN, -(CH$_2$)$_t$-NH-C(NHC$_{1-2}$ alkyl)=N-CN, -(CH$_2$)$_t$-NH-C(NHC$_{1-2}$ alkyl)=CH-NO$_2$, -(CH$_2$)$_t$-C(NHC$_{1-2}$

alkyl)=N-CN, $-(CH_2)_t-C(C_{1-2}$ alkyl)=N-CN, $-(CH_2)_t-C(C_{1-2}$ alkyl)=N-O-($C_{1-2}$ alkyl), =N-O-$C_{1-2}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$; in some embodiments, $R_{12}$ is selected from cyclobutyl, cyclopentyl, cyclohexyl, phenyl, cyclopropyl, adamantyl, tetrahydropyranyl, piperidinyl, oxolanyl, oxanyl,

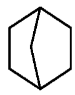,

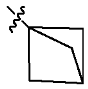 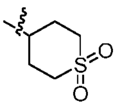 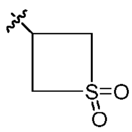 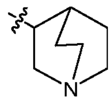 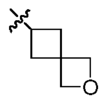 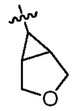 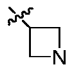, 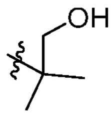,

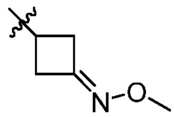,

-C(NH$C_{1-2}$ alkyl)=N-CN, -C($C_{1-2}$ alkyl)=N-CN, or -C(NH$C_{1-2}$ alkyl)=CH-$NO_2$; the ring is optionally substituted with 1-3 groups selected from halogen, CN, OH, $-SF_5$, $C_{1-4}$ alkyl, hydroxy $C_{1-4}$ alkyl, $C_{1-4}$ haloalkyl, $-(CH_2)_t-O-C(O)NH_2$, $-(CH_2)_t-NH-C(O)NH_2$, $-(CH_2)_t-NH-C(=NH)NH_2$, $-(CH_2)_t$-NH-C(O)-O-($C_{1-2}$ alkyl), $-(CH_2)_t$-NH-C($C_{1-2}$ alkyl)=N-CN, $-(CH_2)_t$-NH-C(NH$C_{1-2}$ alkyl)=N-CN, $-(CH_2)_t$-NH-C(NH$C_{1-2}$ alkyl)=CH-$NO_2$, $-(CH_2)_t$-C(NH$C_{1-2}$ alkyl)=N-CN, $-(CH_2)_t$-C($C_{1-2}$ alkyl)=N-CN, $-(CH_2)_t$-C($C_{1-2}$ alkyl)=N-O-($C_{1-2}$ alkyl), =N-O-$C_{1-2}$ alkyl, and a 5- to 6-membered heteroaryl; in some embodiments, $R_{12}$ is selected from cycloalkyl, cyclohexyl, phenyl, cyclopropyl, adamantyl, tetrahydropyranyl, piperidinyl, oxolanyl,

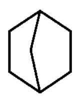,

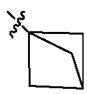 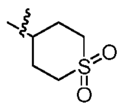 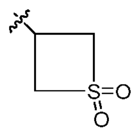 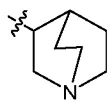 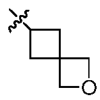 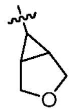 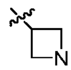,

wherein the ring is optionally substituted with 1-3 groups selected from halogen, CN, OH, $C_{1-4}$ alkyl, hydroxy $C_{1-4}$ alkyl, and $C_{1-4}$ haloalkyl; in some embodiments, $R_{12}$ is selected from $-C(CH_3)_2-CH_2-O-C(O)NH_2$, - $C(CH_3)_2-CH_2$-NH-C(O)O($C_{1-4}$ alkyl), $-C(CH_3)_2-CH_2-O-C(O)NHCH_3$, $-CH(CH_3)-CH_2-O-C(O)NH_2$, $-CH(CH_3)-CH_2$-NH-C(O)O($C_{1-4}$ alkyl), and $-CH(CH_3)-CH_2-O-C(O)NHCH_3$;

Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O; or Ra and Rb are each independently selected from H, halogen, $C_{1-4}$ alkyl, $C_{1-4}$ haloalkyl, $C_{1-4}$ alkoxy, or halo $C_{1-4}$ alkoxy;

or alternatively, Ra and Rb, together with the atom to which they are attached, form $C_{3-6}$ cycloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl; in some embodiments, $R_{13}$ is selected from

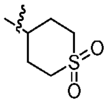 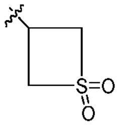 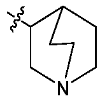 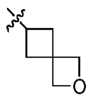 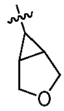 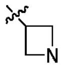 ;

wherein the ring is optionally substituted with 1-3 groups selected from halogen, CN, OH, $C_{1-4}$ alkyl, hydroxy $C_{1-4}$ alkyl, and $C_{1-4}$ haloalkyl; $R_{14}$ is selected from $-(CH_2)_t-O-C(O)NH_2$, $-(CH_2)_t-O-C(O)NHCH_3$, and a 5- to 6-membered monocyclic heterocycloalkyl containing 1-3 heteroatoms selected from N, S, and O, wherein the monocyclic heterocycloalkyl is optionally substituted with 1-3 groups from halogen, CN, =O, OH, $C_{1-4}$ alkyl, and $C_{1-4}$ haloalkyl; in some embodiments, $R_{14}$ is selected from $-(CH_2)_t-O-C(O)NH_2$ and a 5- to 6-membered monocyclic heterocycloalkyl containing 1-3 heteroatoms selected from N, S, and O; in some embodiments, $R_{14}$ is selected from $-(CH_2)_t-O-C(O)NH_2$,

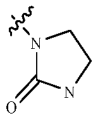 , 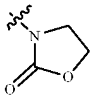 ;

n and m are independently 0, 1, 2, or 3; in some embodiments, n is 0, 1, 2, or 3; in some embodiments, m is 0, 1, or 2;
r is 1, 2, 3, or 4; in some embodiments, r is 1 or 2;
t is selected from 0, 1, 2, 3, or 4; in some embodiments, t is selected from 0, 1, or 2; and
p is selected from 0 or 1;
provided that Cy1 is not

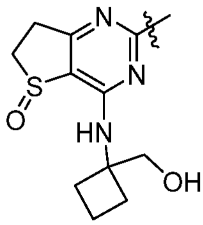 or 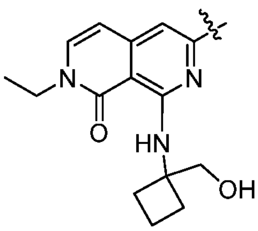 .

The present disclosure provides more specific technical solution 1, which relates to a compound represented by formula I, or a stereoisomer or a pharmaceutically acceptable salt thereof, wherein

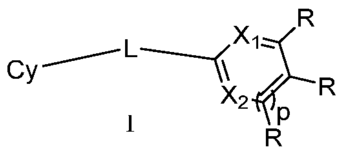

I

wherein when Cy is selected from Cy1, Cy2, Cy3, Cy8, and Cy10, L is $-(L_1)n-(L_2)m-$;

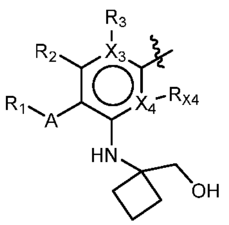
Cy1 ,
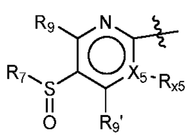
Cy2 ,
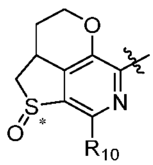
Cy3 ,
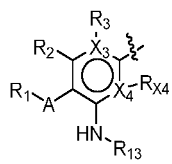
Cy8 , and
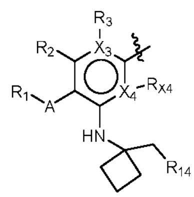
Cy10 ;

when Cy is selected from Cy4, Cy5, and Cy6, L is $-L_3-$;

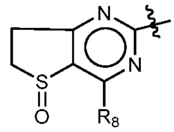
Cy4 ,
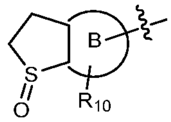
Cy5 ,
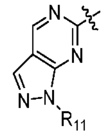
Cy6 or
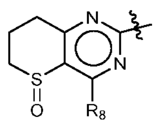
Cy11 ;

and

when Cy is selected from Cy7 and Cy9, L is $-L_4-$;

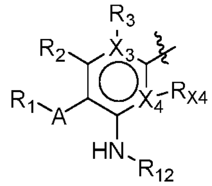
Cy7 and
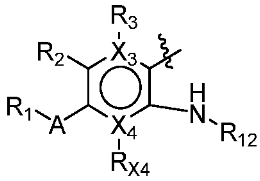
Cy9 ;

$L_1$ is $-NR_4-$, $-CR_5=N-$, $-CR_5=CR_5-$, or $-CR_5R_5-$;

$L_2$ is

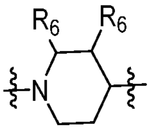 , 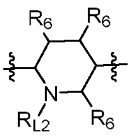 , 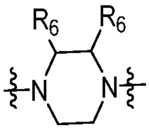 , 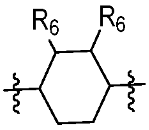 , 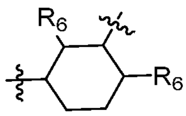 , 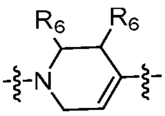 ,

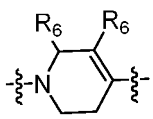 , 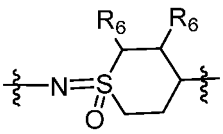 , 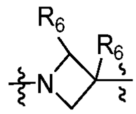 ,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

$L_4$ is

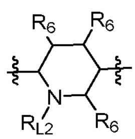 , 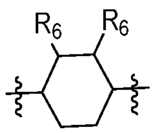 , 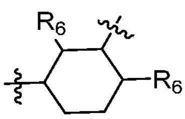 , 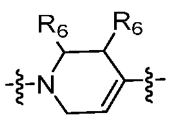 , 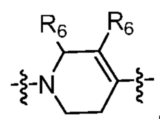 ,

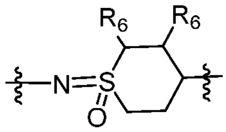, 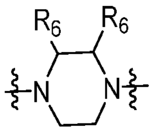,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

in some embodiments, $L_4$ is

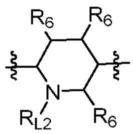, 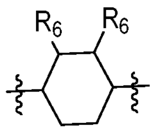, 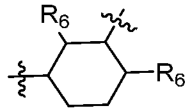, 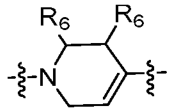,

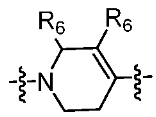, 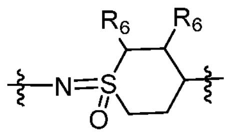,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; A is -S(O)-, -C(O)-, -B(OH)-, -S-, -S(=N)-CN, or -C(=N)-CN;

$X_1$ and $X_2$ are independently $CR_{X1}$, O, S, or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $C_{1-4}$ alkoxy, $-N(CH_3)_2$, $-NH(CH_3)$, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; or each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, $C_{3-6}$ cycloalkyl, $C_{2-6}$ alkynyl,

and $NH_2$;

or each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, or $C_{1-4}$ haloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, $-NHC_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, $-OR_{8'}$, or $-(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, $-N=S(=O)(C_{1-4}\ alkyl)_2$, or $-N(C_{3-6}\ cycloalkyl)C(O)NH(C_{1-4}\ alkyl)$, wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, $C_{1-4}$ alkyl-CN, $-CRaRb=N-O-C_{1-4}$ alkyl,

-C(NRaRb)=N-CN, -C($C_{1-4}$ alkyl)=N-CN, -C(NRaRb)=CRa-$NO_2$, -C$(CH_3)_2$-$CH_2$-O-C(O)$NH_2$, -C$(CH_3)_2$-$CH_2$-NH-C(O)O($C_{1-4}$ alkyl), -C$(CH_3)_2$-$CH_2$-O-C(O)NH$CH_3$, - CH($CH_3$)-$CH_2$-O-C(O)$NH_2$, -CH($CH_3$)-$CH_2$-NH-C(O)O($C_{1-4}$ alkyl), -CH($CH_3$)-$CH_2$-O-C(O)NH$CH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from halogen, CN, =O, OH, -$SF_5$, $C_{1-4}$ alkyl, $C_{1-4}$ alkyl-CN, hydroxy $C_{1-4}$ alkyl, $C_{1-4}$ haloalkyl, -$(CH_2)_t$-O-C(O)$NH_2$, -$(CH_2)_t$-NRa-C(O)$NH_2$, -$(CH_2)_t$-NRa-C(=NH)$NH_2$, -$(CH_2)_t$-NRa-C(O)-O-($C_{1-4}$ alkyl), -$(CH_2)_t$-NRa-C($C_{1-4}$ alkyl)=N-CN, -$(CH_2)_t$-NRa-C(NRaRb)=N-CN, -$(CH_2)_t$-NRa-C(NRaRb)=CRa-$NO_2$, -$(CH_2)_t$-C(NRaRb)=N-CN, -$(CH_2)_t$-C($C_{1-4}$ alkyl)=N-CN, -$(CH_2)_t$-C($C_{1-4}$ alkyl)=N-O-($C_{1-4}$ alkyl), =N-O-$C_{1-4}$ alkyl, a 5- to 6-membered heteroaryl, -$(CH_2)_t$-O-$C_{1-4}$ alkyl, -$(CH_2)_t$-O-C(O)NH$CH_3$, -$(CH_2)_t$-O-$C_{1-2}$ haloalkyl, - $(CH_2)_t$-O-$C_{3-6}$ cycloalkyl, -$(CH_2)_t$-O-$C_{1-4}$ alkyl-O-$C_{3-6}$ cycloalkyl, -$(CH_2)_t$-COOH, -$(CH_2)_t$-NRaRb, -$(CH_2)_t$-C(O)NRaRb, and -$(CH_2)_t$-NRa-C(O)$CH_3$;

or $R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, -CRaRb=N-O-$C_{1-4}$ alkyl, -C(NRaRb)=N-CN, -C($C_{1-4}$ alkyl)=N-CN, -C(NRaRb)=CRa-$NO_2$, -C$(CH_3)_t$-$CH_2$-O-C(O)$NH_2$, -C$(CH_3)_t$-$CH_2$-NH-C(O)O($C_{1-4}$ alkyl), -C$(CH_3)_t$-$CH_2$-O-C(O)NH$CH_3$, and -C(O)-Ra, or - C(O)-(4- to 6-membered heterocycloalkyl), wherein the heterocycloalkylene, cycloalkylene, arylene, and heteroarylene are optionally further substituted with 1-3 groups from halogen, CN, =O, OH, -$SF_5$, $C_{1-4}$ alkyl, $C_{1-4}$ alkyl-CN, hydroxy $C_{1-4}$ alkyl, $C_{1-4}$ haloalkyl, -$(CH_2)_t$-O-C(O)$NH_2$, -$(CH_2)_t$-NRa-C(O)$NH_2$, -$(CH_2)_t$-NRa-C(=NH)$NH_2$, -$(CH_2)_t$-NRa-C(O)-O-($C_{1-4}$ alkyl), -$(CH_2)_t$-NRa-C($C_{1-4}$ alkyl)=N-CN, -$(CH_2)_t$-NRa-C(NRaRb)=N-CN, -$(CH_2)_t$-NRa-C(NRaRb)=CRa-$NO_2$, -$(CH_2)_t$-C(NRaRb)=N-CN, - $(CH_2)_t$-C($C_{1-4}$ alkyl)=N-CN, -$(CH_2)_t$-C($C_{1-4}$ alkyl)=N-O-($C_{1-4}$ alkyl), =N-O-$C_{1-4}$ alkyl, a 5- to 6-membered heteroaryl, -$(CH_2)_t$-O-$C_{1-4}$ alkyl, -$(CH_2)_t$-O-C(O)NH$CH_3$, -$(CH_2)_t$-O-$C_{1-2}$ haloalkyl, -$(CH_2)_t$-O-$C_{3-6}$ cycloalkyl, -$(CH_2)_t$-O-$C_{1-4}$ alkyl-O-$C_{3-6}$ cycloalkyl, -$(CH_2)_t$-COOH, -$(CH_2)_t$-NRaRb, -$(CH_2)_t$-C(O)NRaRb, and -$(CH_2)_t$-NRa-C(O)$CH_3$;

Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O;

or alternatively, Ra and Rb, together with the atom to which they are attached, form $C_{3-6}$ cycloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl;

$R_{14}$ is selected from -$(CH_2)_t$-O-C(O)$NH_2$, -$(CH_2)_t$-O-C(O)NH$CH_3$, and a 5- to 6-membered monocyclic heterocycloalkyl containing 1-3 heteroatoms selected from N, S, and O, wherein the monocyclic heterocycloalkyl is optionally substituted with 1-3 groups from halogen, CN, =O, OH, $C_{1-4}$ alkyl, and $C_{1-4}$ haloalkyl;

n and m are independently 0, 1, 2, or 3;

r is selected from 1, 2, 3, or 4;

t is selected from 0, 1, 2, 3, or 4; and

p is selected from 0 or 1;

provided that

Cy1 is not

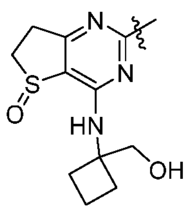

:

or

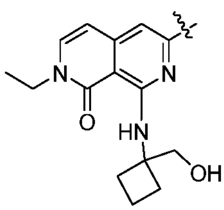

.

The present disclosure provides more specific technical solution 2, which relates to a compound represented by formula I, or a stereoisomer or a pharmaceutically acceptable salt thereof, wherein when Cy is selected from Cy1, Cy2, Cy3, Cy8, and Cy10, L is -$(L_1)$n-$(L_2)$m-;

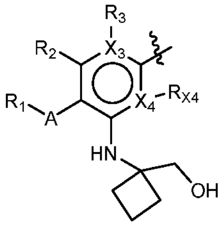
Cy1 ,
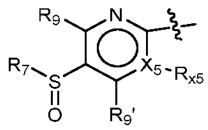
Cy2 ,
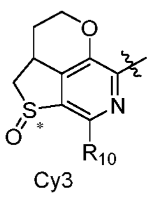
Cy3 ,
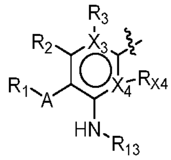
Cy8 , and
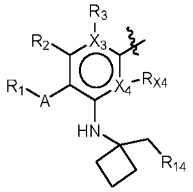
Cy10 ;

when Cy is selected from Cy4, Cy5, and Cy6, L is $-L_3-$;

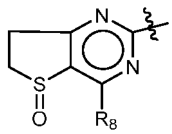
Cy4 ,
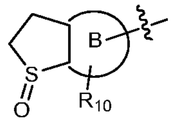
Cy5 , and
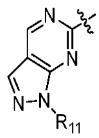
Cy6 ;

and
when Cy is selected from Cy7 and Cy9, L is $-L_4-$;

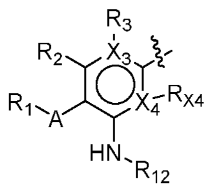
Cy7 and
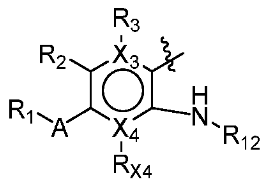
Cy9 ;

$L_1$ is $-NR_4-$, $-CR_5=N-$, $-CR_5=CR_5-$, or $-CR_5R_5-$;
$L_2$ is

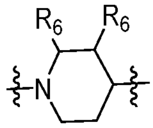,
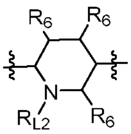,
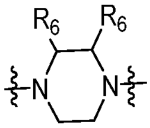,
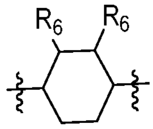,
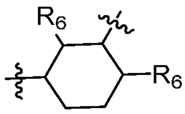,
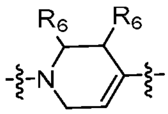,

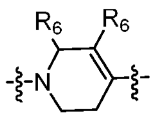,
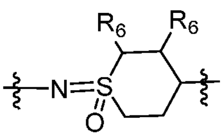,
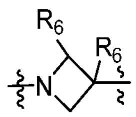,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;
$R_{L2}$ is H, $C_{1-4}$ alkyl, or $C_{3-6}$ cycloalkyl;
$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

$L_4$ is

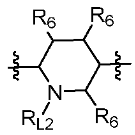, 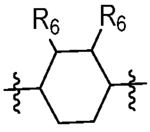, 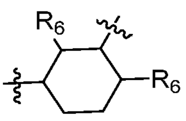, 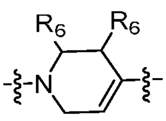, 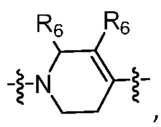,

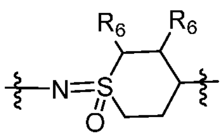,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

A is -S(O)-, -C(O)-, -B(OH)-, -S-, -S(=N)-CN, or -C(=N)-CN;

$X_1$ and $X_2$ are independently $CR_{X1}$, O, S, or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, $C_{1-4}$ haloalkyl, or a 3- to 5-membered cycloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, $-NHC_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, $-OR_{8'}$, or $-(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, or $-N=S(=O)(C_{1-4}\ alkyl)_2$, or -N(C3-6 cycloalkyl)C(O)NH(C1-4 alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, -CRaRb=N-O-$C_{1-4}$ alkyl, -C(NRaRb)=N-CN, -C($C_{1-4}$ alkyl)=N-CN, -C(NRaRb)=CRa-$NO_2$, $-C(CH_3)_t-CH_2-O-C(O)NH_2$, -C(O)-Ra, or $-C(CH_3)_t-CH_2-O-C(O)NHCH_3$, wherein the heterocycloalkylene, cycloalkylene, arylene, and heteroarylene are optionally further substituted with 1-3 groups from halogen, CN, =O, OH, $-SF_5$, $C_{1-4}$ alkyl, hydroxy $C_{1-4}$ alkyl, $C_{1-4}$ haloalkyl, $-(CH_2)_t-O-C(O)NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$; or $R_{12}$ is selected from a 4- to 10-membered heterocycloalkylene, a 3- to 10-membered cycloalkylene, a 6- to 10-membered arylene, a 5- to 10-membered heteroarylene, hydroxy $C_{1-6}$ alkyl, -CRaRb=N-O-$C_{1-4}$ alkyl, -C(NRaRb)=N-CN, -C($C_{1-4}$ alkyl)=N-CN, or -C(NRaRb)=CRa-$NO_2$, wherein the heterocycloalkylene, cycloalkylene, arylene, and heteroarylene are optionally further substituted with 1-3 groups from halogen, CN, =O, OH, $-SF_5$, $C_{1-4}$ alkyl, hydroxy $C_{1-4}$ alkyl, $C_{1-4}$ haloalkyl, $-(CH_2)_t-O-C(O)NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, and a 5- to 6-membered heteroaryl;

Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O;

or alternatively, Ra and Rb, together with the atom to which they are attached, form $C_{3-6}$ cycloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl;

$R_{14}$ is selected from $-(CH_2)_t-O-C(O)NH_2$, $-(CH_2)_t-O-C(O)NHCH_3$, and a 5- to 6-membered monocyclic heterocycloalkyl containing 1-3 heteroatoms selected from N, S, and O, wherein the monocyclic heterocycloalkyl is optionally substituted with 1-3 groups from halogen, CN, =O, OH, $C_{1-4}$ alkyl, and $C_{1-4}$ haloalkyl;

n and m are independently 0, 1, 2, or 3;

r is selected from 1, 2, 3, or 4;

t is selected from 0, 1, 2, 3, or 4; and

P is selected from 0 or 1;

provided that

Cy1 is not

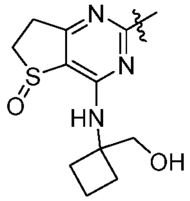 or 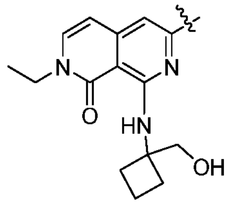 .

The present disclosure provides more specific technical solution 3, which relates to a compound represented by formula I, or a stereoisomer or a pharmaceutically acceptable salt thereof, wherein

when Cy is selected from Cy1, Cy2, Cy3, and Cy8, L is $-(L_1)n-(L_2)m-$;

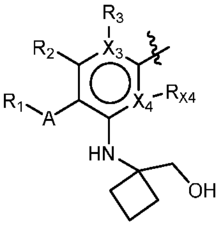
Cy1 ,
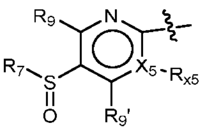
Cy2 ,
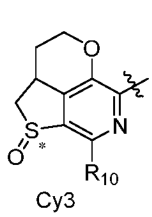
Cy3 , and
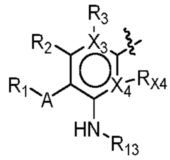
Cy8 ;

when Cy is selected from Cy4, Cy5, and Cy6, L is $-L_3-$;

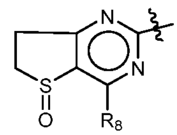
Cy4 ,
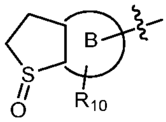
Cy5 , and
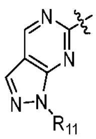
Cy6 ;

and

when Cy is selected from Cy7 and Cy9, L is $-L_4-$;

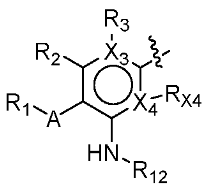
Cy7 and
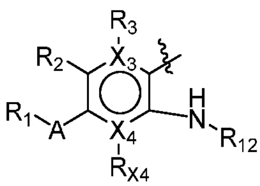
Cy9 ;

$L_1$ is $-NR_4-$, $-CR_5=N-$, $-CR_5=CR_5-$, or $-CR_5R_5-$;

$L_2$ is

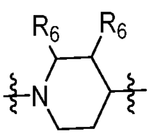 , 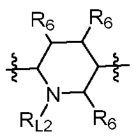 , 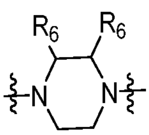 , 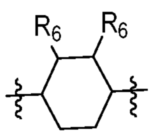 , 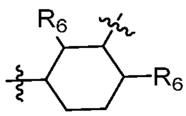 , 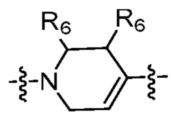 ,

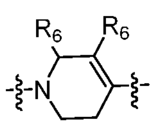 , 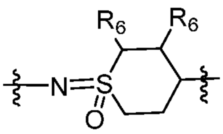 , 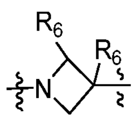 ,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

$L_4$ is

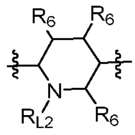 , 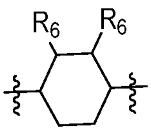 , 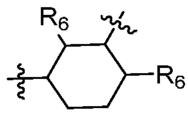 , 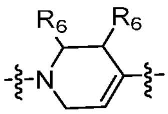 , 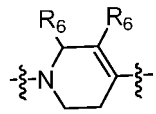 ,

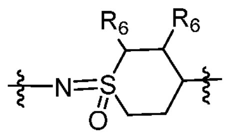 ,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

A is -S(O)-, -C(O)-, -B(OH)-, -S-, -S(=N)-CN, or -C(=N)-CN;

$X_1$ and $X_2$ are independently $CR_{X1}$ or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the

atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, -NH$C_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_9$, and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, -O$R_{8'}$, or -$(CH_2)_r R_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, or -N=S(=O)($C_{1-4}$ alkyl$)_2$, or -N(C3-6 cycloalkyl)C(O)NH(C1-4 alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl;

each $R_{10}$ is independently $R_{8'}$, -O$R_{8'}$, -NH$R_{8'}$, or -$(CH_2)_r R_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from a 4- to 10-membered heterocycloalkylene, a 3- to 10-membered cycloalkylene, a 6- to 10-membered arylene, a 5- to 10-membered heteroarylene, hydroxy $C_{1-6}$ alkyl, -CRaRb=N-O-$C_{1-4}$ alkyl, -C(NRaRb)=N-CN, -C($C_{1-4}$ alkyl)=N-CN, or -C(NRaRb)=CRa-$NO_2$, wherein the heterocycloalkylene, cycloalkylene, arylene, and heteroarylene are optionally further substituted with 1-3 groups from halogen, CN, =O, OH, -$SF_5$, $C_{1-4}$ alkyl, hydroxy $C_{1-4}$ alkyl, $C_{1-4}$ haloalkyl, -$(CH_2)_t$-O-C(O)$NH_2$, -$(CH_2)_t$-NRa-C(O)$NH_2$, -$(CH_2)_t$-NRa-C(=NH)$NH_2$, -$(CH_2)_t$-NRa-C(O)-O-($C_{1-4}$ alkyl), -$(CH_2)_t$-NRa-C($C_{1-4}$ alkyl)=N-CN, -$(CH_2)_t$-NRa-C(NRaRb)=N-CN, -$(CH_2)_t$-NRa-C(NRaRb)=CRa-$NO_2$, -$(CH_2)_t$-C(NRaRb)=N-CN, -$(CH_2)_t$-C($C_{1-4}$ alkyl)=N-CN, -$(CH_2)_t$-C($C_{1-4}$ alkyl)=N-O-($C_{1-4}$ alkyl), =N-O-$C_{1-4}$ alkyl, and a 5- to 6-membered heteroaryl;

Ra and Rb are each independently selected from H, halogen, $C_{1-4}$ alkyl, $C_{1-4}$ haloalkyl, $C_{1-4}$ alkoxy, or halo $C_{1-4}$ alkoxy; or alternatively, Ra and Rb, together with the atom to which they are attached, form $C_{3-6}$ cycloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl;

n and m are independently 0, 1, 2, or 3;

r is selected from 1, 2, 3, or 4; and

t is selected from 0, 1, 2, 3, or 4;

provided that

Cy1 is not

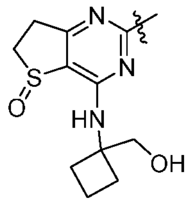

or

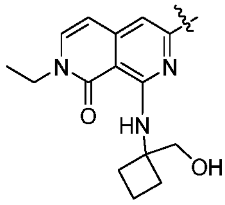

.

The present disclosure provides more specific technical solution 4, which relates to a compound represented by formula I, or a stereoisomer or a pharmaceutically acceptable salt thereof,

wherein when Cy is selected from Cy1, Cy2, Cy3, and Cy8, L is -$(L_1)$n-$(L_2)$m-;

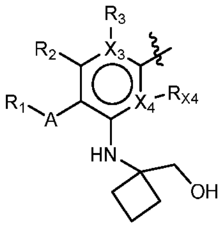

Cy1 ,

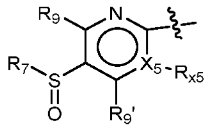

Cy2 ,

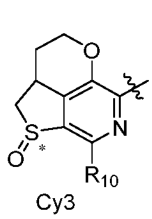

Cy3 , and

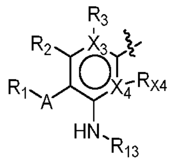

Cy8 ;

and

when Cy is selected from Cy4, Cy5, and Cy6, L is -$L_3$-;

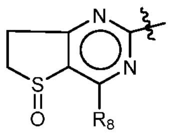

Cy4 ,

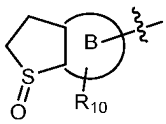

Cy5 , and

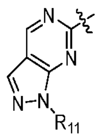

Cy6 ;

and

when Cy is Cy7, L is -$L_4$-;

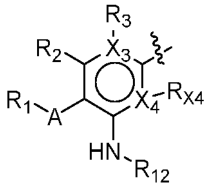

Cy7 ;

$L_1$ is -$NR_4$-, -$CR_5$=N-, -$CR_5$=$CR_5$-, or -$CR_5R_5$-;

$L_2$ is

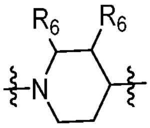 , 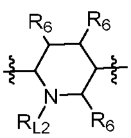 , 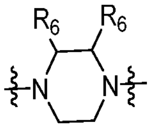 , 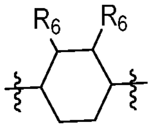 , 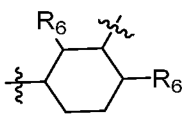 , 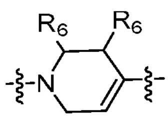 ,

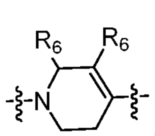 , 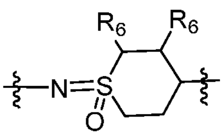 , 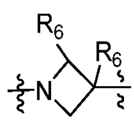 ,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

$L_4$ is

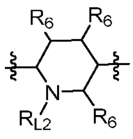, 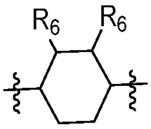, 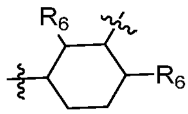, 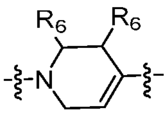, 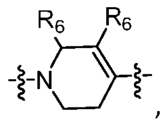,

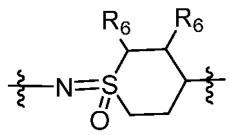,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered cyclic heterocycloalkyl fused 5- to 6-membered cyclic heterocycloalkyl containing 1-3 heteroatoms selected from N, S, and O, a 5- to 6-membered cyclic heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered cyclic heterocycloalkyl fused 5- to 6-membered cyclic heterocycloalkyl, 5- to 6-membered cyclic heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

A is -S(O)-, -C(O)-, -B(OH)-, -S-, -S(=N)-CN, or -C(=N)-CN;

$X_1$ and $X_2$ are independently $CR_{X1}$ or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $R_{X1}$ and R are independently 3- to 5-membered cycloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; $R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the

atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, -NH$C_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, -O$R_{8'}$, or -$(CH_2)_r R_{8'}$;

$R_{8'}$ is $C_{3-6}$ cycloalkyl, a 4- to 8-membered heterocycloalkyl containing 1-3 heteroatoms selected from N, S, and O, or a 5- to 6-membered heteroaryl containing 1-3 heteroatoms selected from N, S, and O, or $R_{8'}$ is -N(C3-6 cycloalkyl)C(O)NH(C1-4 alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl;

each $R_{10}$ is independently $R_{8'}$, -O$R_{8'}$, -NH$R_{8'}$, or -$(CH_2)_r R_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from a 4- to 10-membered heterocycloalkylene, a 3- to 10-membered cycloalkylene, a 6- to 10-membered arylene, or a 5- to 10-membered heteroarylene, wherein the heterocycloalkylene, cycloalkylene, arylene, and heteroarylene are optionally further substituted with 1-3 groups from halogen, CN, =O, OH, $C_{1-4}$ alkyl, hydroxy $C_{1-4}$ alkyl, and $C_{1-4}$ haloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl;

n and m are independently 0, 1, 2, or 3; and

r is 1, 2, 3, or 4;

provided that

Cy1 is not

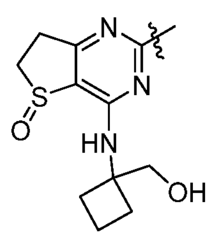

or

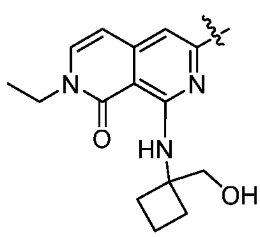

.

The present disclosure provides more specific technical solution 5, which relates to a compound represented by formula I, or a stereoisomer or a pharmaceutically acceptable salt thereof,

wherein when Cy is selected from Cy1, Cy2, and Cy3, L is $-(L_1)n-(L_2)m-$;

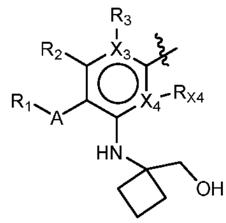
Cy1 ,
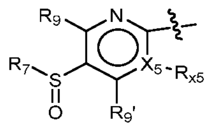
Cy2 , and
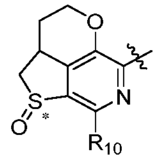
Cy3 ;

and

when Cy is selected from Cy4, Cy5, and Cy6, L is $-L_3-$;

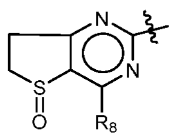
Cy4 ,
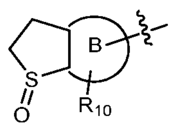
Cy5 , and
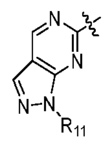
Cy6 ;

$L_1$ is $-NR_4-$, $-CR_5=N-$, $-CR_5=CR_5-$, or $-CR_5R_5-$;

$L_2$ is

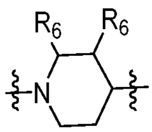 , 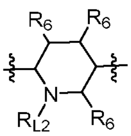 , 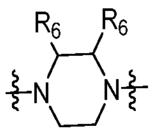 , 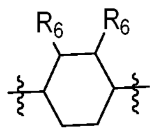 , 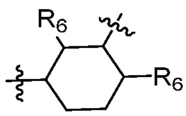 , 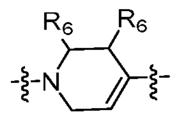 ,

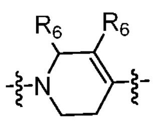 , 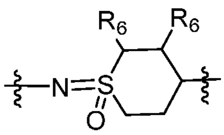 , 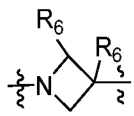 ,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; or $L_2$ is

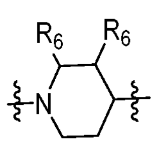 , 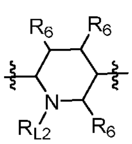 , 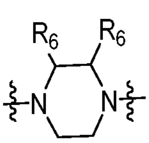 , 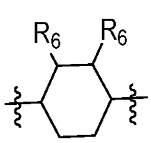 , 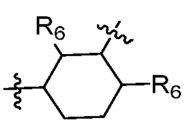 ,

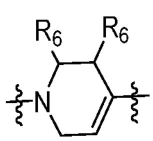 , 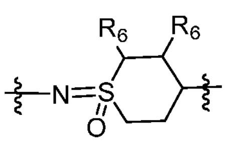 , 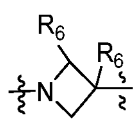 ,

$C_{1-4}$ alkylene, $C_{2-4}$ alkenylene, $C_{2-4}$ alkynylene, CO, O, $NR_{L2}$, a 5- to 6-membered heteroaryl containing 1-3

heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

A is -S(O)-, -C(O)-, -B(OH)-, -S-, -S(=N)-CN, or -C(=N)-CN;

$X_1$ and $X_2$ are independently $CR_{X1}$ or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $C_{1-4}$ haloalkyl, or $R_{X1}$ and R are independently 3- to 5-membered cycloalkyl, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, and O, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; $R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, -$NHC_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, -$OR_{8'}$, or -$(CH_2)_rR_{8'}$;

$R_{8'}$ is $C_{3-6}$ cycloalkyl, a 4- to 8-membered heterocycloalkyl containing 1-3 heteroatoms selected from N, S, and O, or a 5- to 6-membered heteroaryl containing 1-3 heteroatoms selected from N, S, and O, or $R_{8'}$ is -N(C3-6 cycloalkyl)C(O) NH(C1-4 alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;
$R_9$ is H, $C_{1-4}$ alkyl, or $C_{1-4}$ haloalkyl;
each $R_{10}$ is independently $R_{8'}$, $-OR_{8'}$, $-NHR_{8'}$, or $-(CH_2)_rR_{8'}$;
$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;
n and m are independently 0, 1, 2, or 3; and
r is 1, 2, 3, or 4;
provided that
Cy1 is not

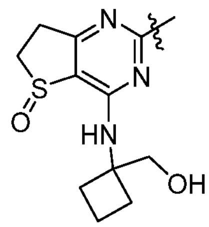
or
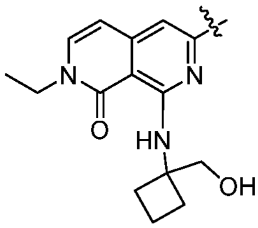
.

Solution 6 of the present disclosure relates to the compounds, or the stereoisomers or pharmaceutically acceptable salts thereof, as described above in solutions 1, 2, 3, 4, and 5, wherein ring B is pyrazolyl, imidazolyl, pyrrolyl, tetrahydropyrrolopyrrolyl, tetrahydropyrroloimidazolyl, or thienopyrrolyl;

$R_1$ is $C_{1-4}$ alkyl, or $C_{1-4}$ haloalkyl, or $R_1$ and $R_2$, together with the atom to which they are attached, form a 5- to 7-membered monocyclic heterocycloalkyl, a 6- to 8-membered fused heterocycloalkyl, a 7- to 9-membered spiro heterocycloalkyl, or a 5- to 6-membered heteroaryl containing 1-3 heteroatoms selected from N, S, B, and O, wherein the monocyclic heterocycloalkyl, fused heterocycloalkyl, spiro heterocycloalkyl, and heteroaryl are optionally substituted with 1-3 groups selected from halogen, =O, CN, $C_{1-4}$ alkyl, $C_{1-4}$ alkoxy, OH, and $NH_2$; $R_2$ is H, $C_{1-4}$ alkyl, or $C_{1-4}$ haloalkyl;
$R_3$ is H, halogen, $C_{1-4}$ alkyl, CN, OH, or absent;
or $R_3$ and $R_2$, together with the atom to which they are attached, form a 5- to 6-membered heterocycloalkyl or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$; or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
or $R_3$ and $R_6$, together with the atom to which they are attached, form $C_{5-7}$ cycloalkyl, a 5- to 6-membered heterocycloalkyl, or a 5- to 6-membered heteroaryl containing 1-3 heteroatoms selected from N, S, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; $R_{x4}$ is H, halogen, $C_{1-4}$ alkyl, CN, OH, or absent, or $R_{x4}$ and $R_4$ or $R_{x4}$ and $R_5$, together with the atom to which they are attached, form a 5- to 6-membered heteroaryl containing 1-3 heteroatoms selected from N, S, and O, wherein the heteroaryl is optionally substituted with 1-3 groups selected from halogen, =O, CN, $C_{1-4}$ alkyl, $C_{1-4}$ alkoxy, OH, and $NH_2$;
$R_7$ is $C_{1-4}$ alkyl, or $C_{1-4}$ haloalkyl, or $R_7$ and $R_9$, together with the atom to which they are attached, form a 5- to 6-membered heterocycloalkyl containing 1-3 heteroatoms selected from N, S, and O, wherein the heterocycloalkyl is optionally substituted with 1-3 groups selected from halogen, CN, $C_{1-4}$ alkyl, $C_{1-4}$ alkoxy, OH, and $NH_2$;
at least one group of $R_{9'}$ and $R_7$ or $R_{9'}$ and $R_{x5}$, together with the atom to which they are attached, form a 5- to 7-membered monocyclic heterocycloalkyl, a 7- to 10-membered spiro heterocycloalkyl, or a 5- to 6-membered heteroaryl containing 1-3 heteroatoms selected from N, S, and O, wherein the monocyclic heterocycloalkyl, spiro heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; and
r is 1 or 2.

Solution 7 of the present disclosure relates to the compounds as described in solutions 1-6, or stereoisomers or

pharmaceutically acceptable salts thereof, wherein each $L_2$ is independently selected from

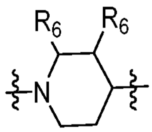, 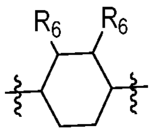, 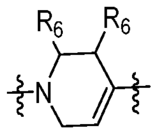, 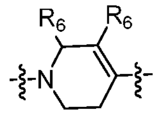, 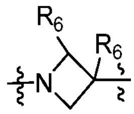,

CO, O, $NR_{L2}$, $C_{1-4}$ alkylene, $C_{2-4}$ alkenylene, $C_{2-4}$ alkynylene,

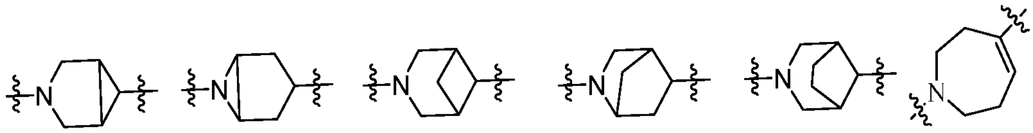

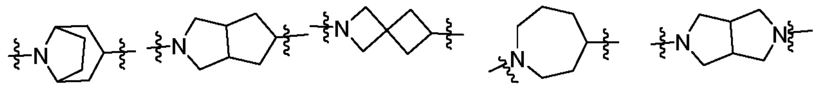

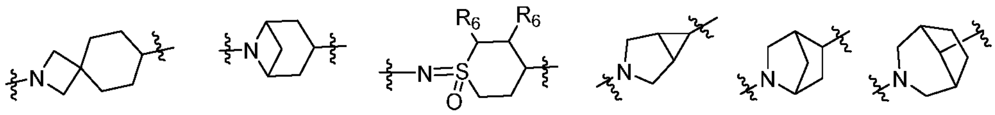

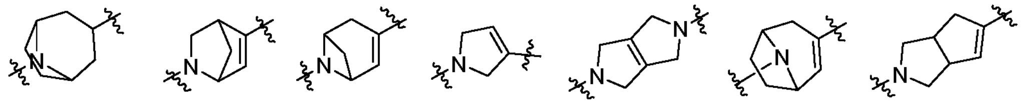

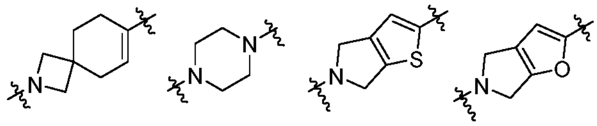

;

$R_{L2}$ is H, $C_{1-4}$ alkyl, or $C_{3-6}$ cycloalkyl;
$L_3$ is selected from

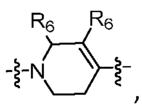,

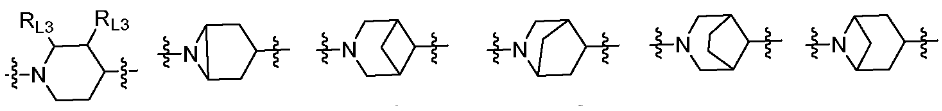

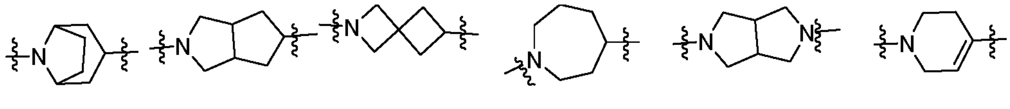

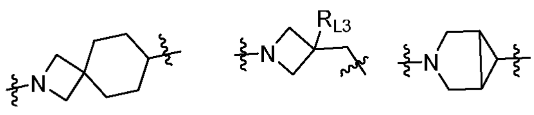

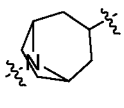 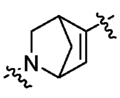 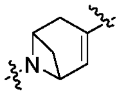 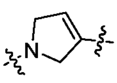 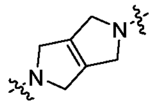 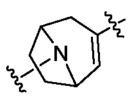 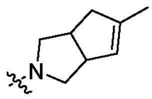

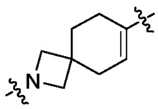 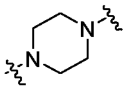 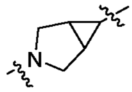 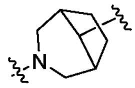 ;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 6-membered heterocycloalkyl containing 1-3 heteroatoms selected from N, S, and O;

$L_4$ is selected from

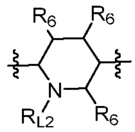, 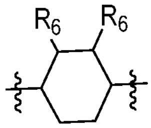, 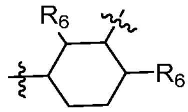, 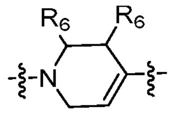, 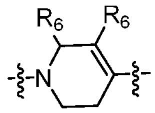,

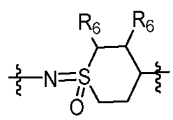, 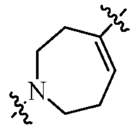, 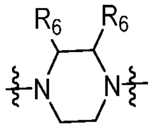,

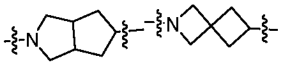 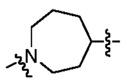 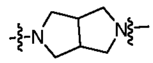 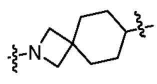 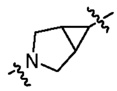 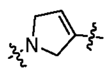

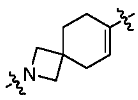 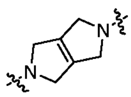 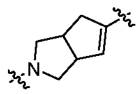 ;

in some embodiments, $L_4$ is selected from

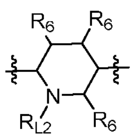, 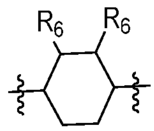, 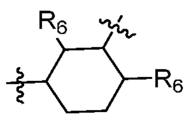,

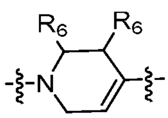, 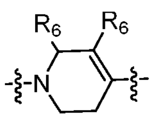, 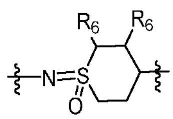, 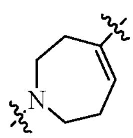,

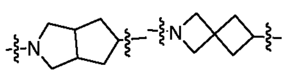 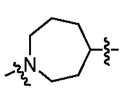 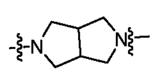 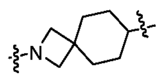 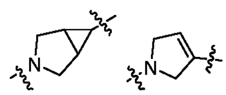

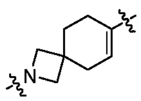 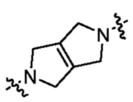 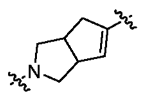

;

A is -S(O)-, -C(O)-, -B(OH)-, -S-, or -S(=N)-CN;
Cy5 is selected from

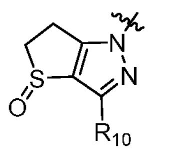 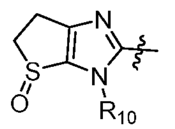 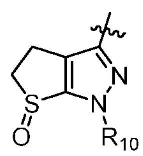 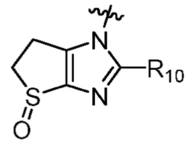 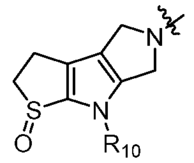

;

each $R_{X1}$ and R are independently H, halogen, $C_{1-4}$ alkyl, $C_{1-4}$ alkoxy, $-N(CH_3)_2$, $-NH(CH_3)$, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl, or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;
or each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, $C_{3-6}$ cycloalkyl, $C_{2-6}$ alkynyl, and $NH_2$;
or each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;
or each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, or $C_{1-4}$ haloalkyl, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, and O, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;
$R_1$ is $C_{1-4}$ alkyl or $C_{1-4}$ haloalkyl;
or $R_1$ and $R_2$, together with the atom to which they are attached, form a ring selected from:

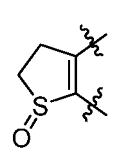 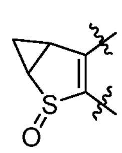 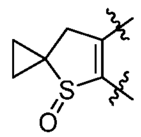 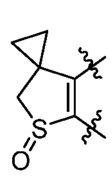 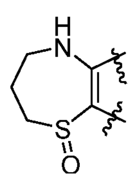 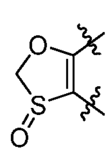

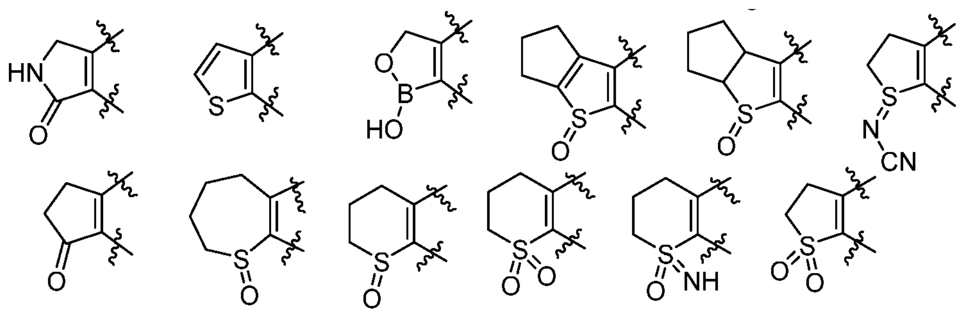

,

optionally substituted with 1-3 groups selected from halogen, =O, CN, $C_{1-4}$ alkyl, $C_{1-4}$ alkoxy, OH, and $NH_2$;
$R_3$ is H, halogen, $C_{1-4}$ alkyl, CN, OH, or absent;
or $R_3$ and $R_2$, together with the atom to which they are attached, form a ring selected from:

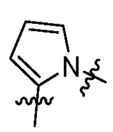 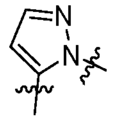 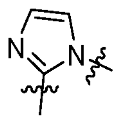 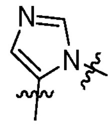 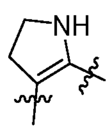 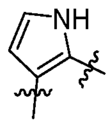

,

optionally substituted with 1-3 groups selected from halogen, =O, CN, $C_{1-4}$ alkyl, $C_{1-4}$ alkoxy, OH, and $NH_2$;
or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$; $R_{x4}$ is H, halogen, $C_{1-4}$ alkyl, CN, OH, or absent;
or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$; $R_4$ is H, $C_{1-4}$ alkyl, or $C_{1-4}$ haloalkyl;
$R_5$ is H, $C_{1-4}$ alkyl, $C_{1-4}$ haloalkyl, $C_{1-4}$ alkoxy, or $-NHC_{1-4}$ alkyl;
$R_6$ is H, halogen, =O, CN, $C_{1-4}$ alkyl, or $C_{1-4}$ alkoxy;
$R_7$ is $C_{1-4}$ alkyl, or $C_{1-4}$ haloalkyl, or $R_7$ and $R_9$, together with the atom to which they are attached, form a ring selected from:

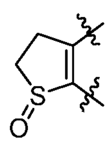 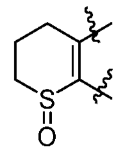 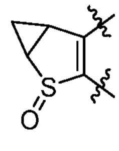 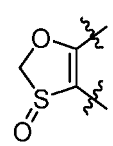

,

wherein the ring is optionally substituted with 1-3 groups selected from halogen, CN, $C_{1-4}$ alkyl, $C_{1-4}$ alkoxy, OH, and $NH_2$;
or $R_{9'}$ and $R_7$, together with the atom to which they are attached, form a ring selected from:

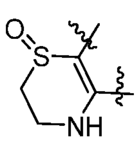 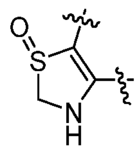 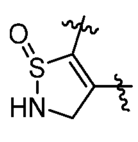 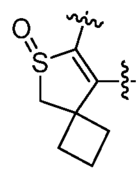 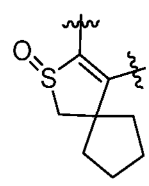

,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;
or $R_{9'}$ and $R_{x5}$, together with the atom to which they are attached, form

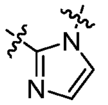 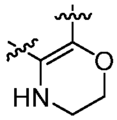 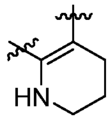,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

provided that at least one group of $R_{9'}$ and $R_7$ or $R_{9'}$ and $R_{x5}$ form a ring;

$R_8$ is $R_{8'}$, $-OR_{8'}$, or $-(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, $-N{=}S(=O)(C_{1-4}\ alkyl)_2$, or $-N(C_{3-6}$ cycloalkyl)C(O)NH($C_{1-4}$ alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H or $C_{1-4}$ alkyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from cyclobutyl, cyclopentyl, cyclohexyl, phenyl, cyclopropyl, adamantyl, tetrahydropyranyl, piperidinyl, oxolanyl, oxanyl,

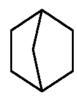,

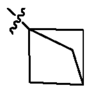 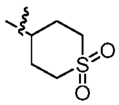 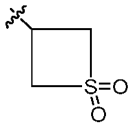 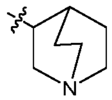 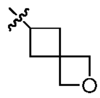 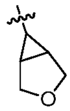 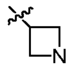, 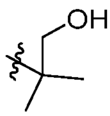,

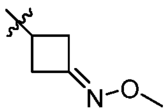, 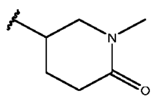,

-C(NH$C_{1-2}$ alkyl)=N-CN, -C($C_{1-2}$ alkyl)=N-CN, -C(NH$C_{1-2}$ alkyl)=CH-$NO_2$, $-C(CH_3)_t-CH_2-NH-C(O)O(CH_3)$, $-C(CH_3)_t-CH_2-O-C(O)NH_2$, -C(O)-Ra, or $-C(CH_3)_t-CH_2-O-C(O)NHCH_3$; wherein the ring is optionally substituted with 1-3 groups selected from halogen, CN, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t-NH-C(O)NH_2$, - $(CH_2)_t-NH-C(=NH)NH_2$, $-(CH_2)_t-NH-C(O)-O-(C_{1-2}$ alkyl), $-(CH_2)_t-NH-C(C_{1-2}$ alkyl)=N-CN, $-(CH_2)_t-NH-C(NHC_{1-2}$ alkyl)=N-CN, $-(CH_2)_t-NH-C(NHC_{1-2}$ alkyl)=CH-$NO_2$, $-(CH_2)_t-C(NHC_{1-2}$ alkyl)=N-CN, $-(CH_2)_t-C(C_{1-2}$ alkyl)=N-CN, $-(CH_2)_t-C(C_{1-2}$ alkyl)=N-O-($C_{1-2}$ alkyl), =N-O-$C_{1-2}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cycloalkyl, $-(CH_2)_t-COOH$, $-(CH_2)_t$-NRaRb, $-(CH_2)_t$-C(O)NRaRb, and $-(CH_2)_t$-NRa-C(O) $CH_3$;

in some embodiments, $R_{12}$ is selected from cyclobutyl, cyclopentyl, cyclohexyl, phenyl, cyclopropyl, adamantyl, tetrahydropyranyl, piperidinyl, oxolanyl, oxanyl,

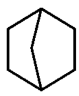,

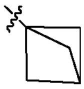 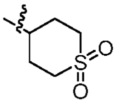 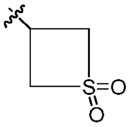 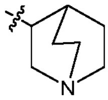 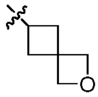 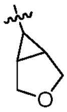 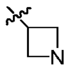 , 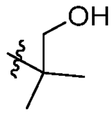 ,

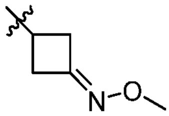 ,

$-C(NHC_{1-2}$ alkyl)=N-CN, $-C(C_{1-2}$ alkyl)=N-CN, $-C(NHC_{1-2}$ alkyl)=CH-$NO_2$, $-C(CH_3)_t-CH_2-NH-C(O)O(CH_3)$, $-C(CH_3)_t-CH_2-O-C(O)NH_2$, -C(O)-Ra, or $-C(CH_3)_t-CH_2-O-C(O)NHCH_3$; wherein the ring is optionally substituted with 1-3 groups selected from halogen, CN, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t-NH-C(O)NH_2$, $-(CH_2)_t-NH-C(=NH)NH_2$, $-(CH_2)_t-NH-C(O)-O-(C_{1-2}$ alkyl), $-(CH_2)_t-NH-C(C_{1-2}$ alkyl)=N-CN, $-(CH_2)_t-NH-C(NHC_{1-2}$ alkyl)=N-CN, $-(CH_2)_t-NH-C(NHC_{1-2}$ alkyl)=CH-$NO_2$, $-(CH_2)_t-C(NHC_{1-2}$ alkyl)=N-CN, $-(CH_2)_t-C(C_{1-2}$ alkyl)=N-CN, $-(CH_2)_t-C(C_{1-2}$ alkyl)=N-O-($C_{1-2}$ alkyl), =N-O-$C_{1-2}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$;

$R_{13}$ is selected from

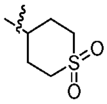 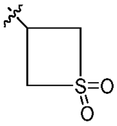 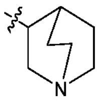 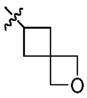 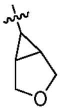 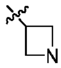 ;

wherein the ring is optionally substituted with 1-3 groups selected from halogen, CN, OH, $C_{1-4}$ alkyl, hydroxy $C_{1-4}$ alkyl, and $C_{1-4}$ haloalkyl;

n is 0, 1, 2, or 3;

m is 0, 1, or 2;

r is 1 or 2; and

t is selected from 0, 1, or 2.

Solution 8 of the present disclosure relates to the compounds as described in solutions 1-6, or stereoisomers or pharmaceutically acceptable salts thereof, wherein each $L_2$ is independently selected from

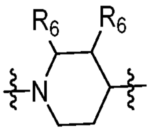 , 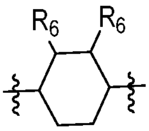 , 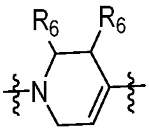 , 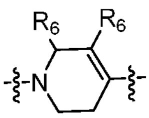 , 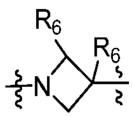 ,

CO, O, $NR_{L2}$, $C_{1-4}$ alkylene, $C_{2-4}$ alkenylene, $C_{2-4}$ alkynylene,

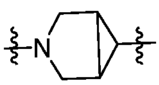 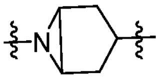 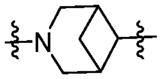 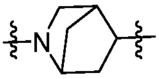 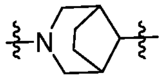 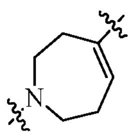

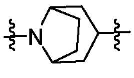 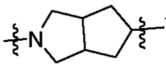 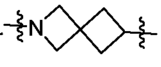 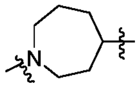 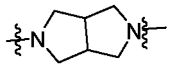

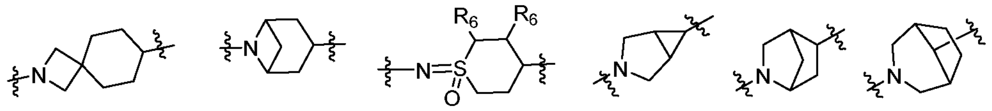

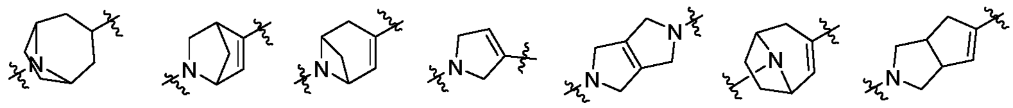

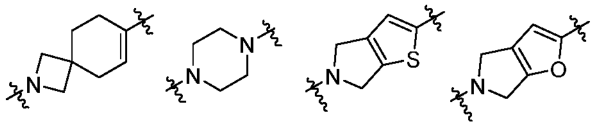

,

$R_{L2}$ is H, $C_{1-4}$ alkyl, or $C_{3-6}$ cycloalkyl;

$L_3$ is selected from

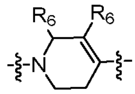

,

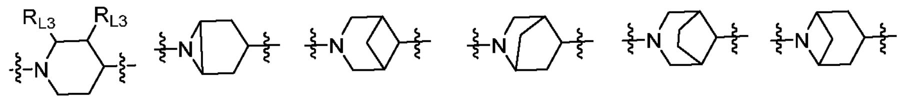

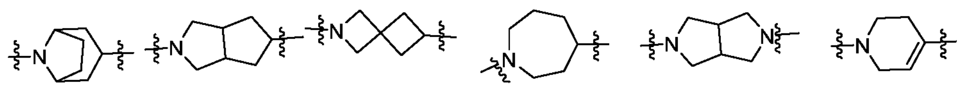

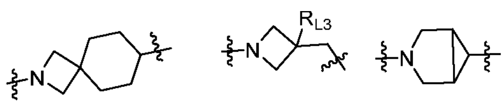

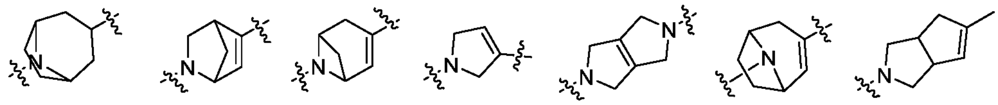

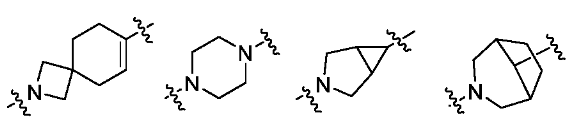

;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 6-membered heterocycloalkyl containing 1-3 heteroatoms selected from N, S, and O;

$L_4$ is selected from

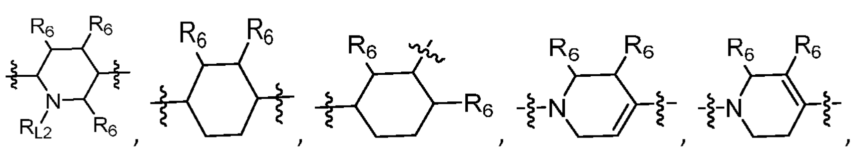

, , , , ,

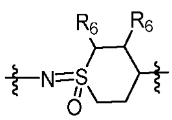, 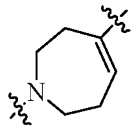,

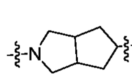 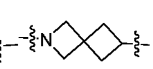 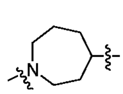 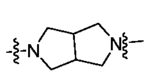 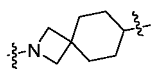 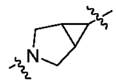 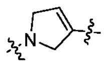

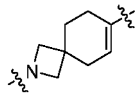 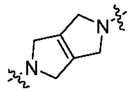 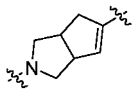;

A is -S(O)-, -C(O)-, -B(OH)-, -S-, or -S(=N)-CN;
Cy5 is selected from

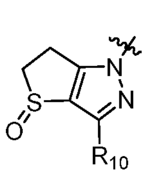 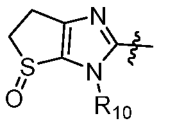 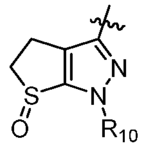 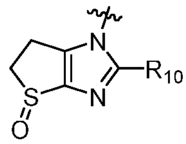 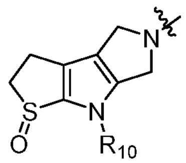;

each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $R_{X1}$ and R are independently a 3- to 5-membered cycloalkyl, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3 heteroatoms selected from N, S, and O, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;
$R_1$ is $C_{1-4}$ alkyl or $C_{1-4}$ haloalkyl;
or $R_1$ and $R_2$, together with the atom to which they are attached, form a ring selected from:

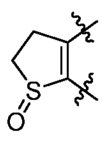 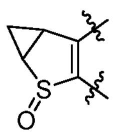 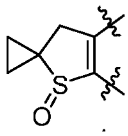 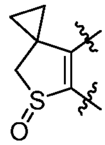 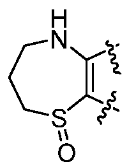 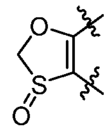

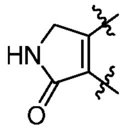 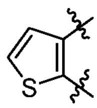 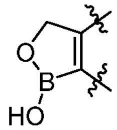 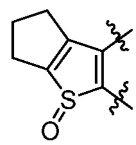 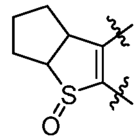 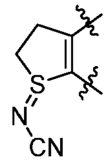

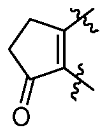 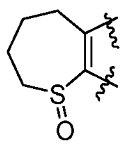 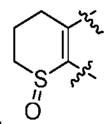, ,

or
$R_1$ and $R_2$, together with the atom to which they are attached, form a ring selected from:

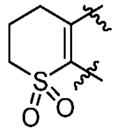 , 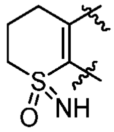 , and 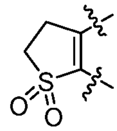 ;

optionally substituted with 1-3 groups selected from halogen, =O, CN, $C_{1-4}$ alkyl, $C_{1-4}$ alkoxy, OH, and $NH_2$;
$R_3$ is H, halogen, $C_{1-4}$ alkyl, CN, OH, or absent;
or $R_3$ and $R_2$, together with the atom to which they are attached, form a ring selected from:

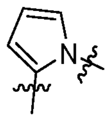 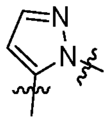 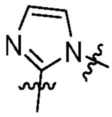 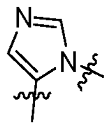 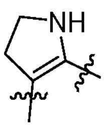 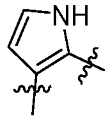 ,

optionally substituted with 1-3 groups selected from halogen, =O, CN, $C_{1-4}$ alkyl, $C_{1-4}$ alkoxy, OH, and $NH_2$;
or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$; $R_{x4}$ is H, halogen, $C_{1-4}$ alkyl, CN, OH, or absent;
or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$; $R_4$ is H, $C_{1-4}$ alkyl, or $C_{1-4}$ haloalkyl;
$R_5$ is H, $C_{1-4}$ alkyl, $C_{1-4}$ haloalkyl, $C_{1-4}$ alkoxy, or -NH$C_{1-4}$ alkyl;
$R_6$ is H, halogen, =O, CN, $C_{1-4}$ alkyl, or $C_{1-4}$ alkoxy;
$R_7$ is $C_{1-4}$ alkyl, or $C_{1-4}$ haloalkyl, or $R_7$ and $R_9$, together with the atom to which they are attached, form a ring selected from:

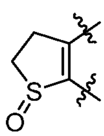 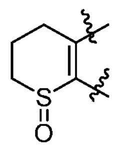 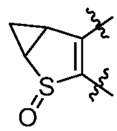 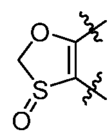 ,

wherein the ring is optionally substituted with 1-3 groups selected from halogen, CN, $C_{1-4}$ alkyl, $C_{1-4}$ alkoxy, OH, and $NH_2$;
or $R_{9'}$ and $R_7$, together with the atom to which they are attached, form a ring selected from:

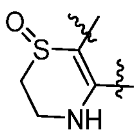 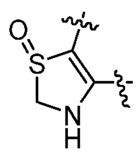 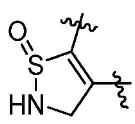 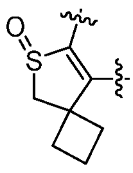 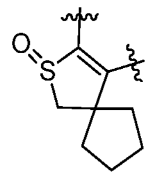 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;
or $R_{9'}$ and $R_{x5}$, together with the atom to which they are attached, form

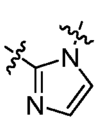 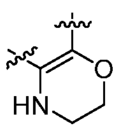 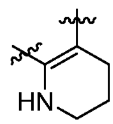 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

provided that at least one group of $R_{9'}$ and $R_7$ or $R_{9'}$ and $R_{x5}$ form a ring;

$R_8$ is $R_{8'}$, -$OR_{8'}$, or -$(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, or -N=S(=O)($C_{1-4}$ alkyl)$_2$, or $R_{8'}$ is -N($C_{3-6}$ cycloalkyl)C(O)NH($C_{1-4}$ alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H or $C_{1-4}$ alkyl;

each $R_{10}$ is independently $R_{8'}$, -$OR_{8'}$, -$NHR_{8'}$, or -$(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from cyclobutyl, cyclopentyl, cyclohexyl, phenyl, cyclopropyl, adamantyl, tetrahydropyranyl, piperidinyl, oxolanyl, oxanyl,

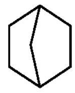,

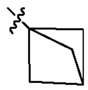 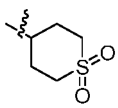 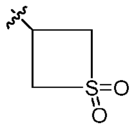 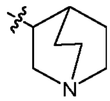 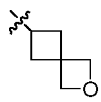 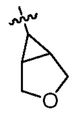 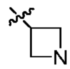, 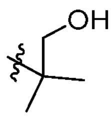,

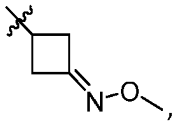, 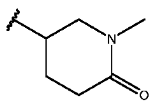,

$C_{1-4}$ alkyl-CN, -C($NHC_{1-2}$ alkyl)=N-CN, -C($C_{1-2}$ alkyl)=N-CN, -C($NHC_{1-2}$ alkyl)=CH-$NO_2$, -CH($CH_3$)-$CH_2$-NH-C(O)O($CH_3$), -C($CH_3$)$_2$-$CH_2$-NH-C(O)O($CH_3$), -CH($CH_3$)-$CH_2$-O-C(O)$NH_2$, -C($CH_3$)$_2$-$CH_2$-O-C(O)$NH_2$, -C(O)-Ra, -CH($CH_3$)-$CH_2$-O-C(O)$NHCH_3$, or -C($CH_3$)$_2$-$CH_2$-O-C(O)$NHCH_3$; wherein the ring is optionally substituted with 1-3 groups selected from halogen, CN, OH, -$SF_5$, $C_{1-4}$ alkyl, $C_{1-4}$ alkyl-CN, hydroxy $C_{1-4}$ alkyl, $C_{1-4}$ haloalkyl, -$(CH_2)_t$-O-C(O)$NH_2$, -$(CH_2)_t$-NH-C(O)$NH_2$, -$(CH_2)_t$-NH-C(=NH)$NH_2$, -$(CH_2)_t$-NH-C(O)-O-($C_{1-2}$ alkyl), -$(CH_2)_t$-NH-C($C_{1-2}$ alkyl)=N-CN, - $(CH_2)_t$-NH-C($NHC_{1-2}$ alkyl)=N-CN, -$(CH_2)_t$-NH-C($NHC_{1-2}$ alkyl)=CH-$NO_2$, -$(CH_2)_t$-C($NHC_{1-2}$ alkyl)=N-CN, -$(CH_2)_t$-C($C_{1-2}$ alkyl)=N-CN, -$(CH_2)_t$-C($C_{1-2}$ alkyl)=N-O-($C_{1-2}$ alkyl), =N-O-$C_{1-2}$ alkyl, a 5- to 6-membered heteroaryl, -$(CH_2)_t$-O-$C_{1-4}$ alkyl, -$(CH_2)_t$-O-C(O)$NHCH_3$, -$(CH_2)_t$-O-$C_{1-2}$ haloalkyl, -$(CH_2)_t$-O-$C_{3-6}$ cycloalkyl, -$(CH_2)_t$-O-$C_{1-4}$ alkyl-O-$C_{3-6}$ cycloalkyl, -$(CH_2)_t$-COOH, -$(CH_2)_t$-NRaRb, -$(CH_2)_t$-C(O)NRaRb, and -$(CH_2)_t$-NRa-C(O)$CH_3$;

or $R_{12}$ is selected from cyclobutyl, cyclopentyl, cyclohexyl, phenyl, cyclopropyl, adamantyl, tetrahydropyranyl, piperidinyl, oxolanyl, oxanyl,

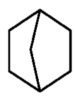,

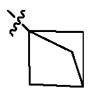 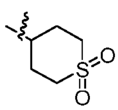 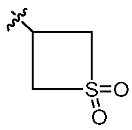 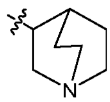 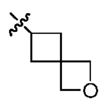 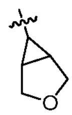 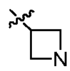, 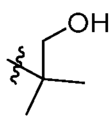,

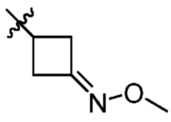,

-C(NHC$_{1-2}$ alkyl)=N-CN, -C(C$_{1-2}$ alkyl)=N-CN, or -C(NHC$_{1-2}$ alkyl)=CH-NO$_2$, or from - C(CH$_3$)$_t$-CH$_2$-O-C(O)NH$_2$ and -C(O)-Ra, or is -C(CH$_3$)$_t$-CH$_2$-O-C(O)NHCH$_3$; the ring is optionally substituted with 1-3 groups selected from halogen, CN, OH, -SF$_5$, C$_{1-4}$ alkyl, hydroxy C$_{1-4}$ alkyl, C$_{1-4}$ haloalkyl, -(CH$_2$)$_t$-O-C(O)NH$_2$, -(CH$_2$)$_t$-NH-C(O)NH$_2$, -(CH$_2$)$_t$-NH-C(=NH)NH$_2$, -(CH$_2$)$_t$-NH-C(O)-O-(C$_{1-2}$ alkyl), -(CH$_2$)$_t$-NH-C(C$_{1-2}$ alkyl)=N-CN, -(CH$_2$)$_t$-NH-C(NHC$_{1-2}$ alkyl)=N-CN, -(CH$_2$)$_t$-NH-C(NHC$_{1-2}$ alkyl)=CH-NO$_2$, -(CH$_2$)$_t$-C(NHC$_{1-2}$ alkyl)=N-CN, -(CH$_2$)$_t$-C(C$_{1-2}$ alkyl)=N-CN, -(CH$_2$)$_t$-C(C$_{1-2}$ alkyl)=N-O-(C$_{1-2}$ alkyl), =N-O-C$_{1-2}$ alkyl, and a 5- to 6-membered heteroaryl, or substituted with -(CH$_2$)$_t$-O-C$_{1-4}$ alkyl, -(CH$_2$)$_t$-O-C(O)NHCH$_3$, -(CH$_2$)$_t$-O-C$_{1-2}$ haloalkyl, -(CH$_2$)$_t$-O-C$_{3-6}$ cycloalkyl, -(CH$_2$)$_t$-O-C$_{1-4}$ alkyl-O-C$_{3-6}$ cycloalkyl, -(CH$_2$)$_t$-COOH, -(CH$_2$)$_t$-NRaRb, -(CH$_2$)$_t$-C(O)NRaRb, or -(CH$_2$)$_t$-NRa-C(O)CH$_3$;

R$_{13}$ is selected from

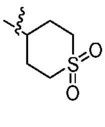 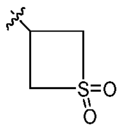 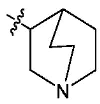 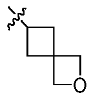 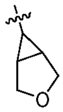 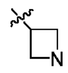

;

wherein the ring is optionally substituted with 1-3 groups selected from halogen, CN, OH, C$_{1-4}$ alkyl, hydroxy C$_{1-4}$ alkyl, and C$_{1-4}$ haloalkyl;

n is 0, 1, 2, or 3;

m is 0, 1, or 2;

r is 1 or 2; and

t is selected from 0, 1, or 2.

Solution 9 of the present disclosure relates to the compounds as described in solutions 1-6, or stereoisomers or pharmaceutically acceptable salts thereof, wherein each L$_2$ is independently selected from

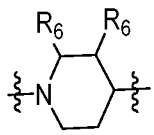, 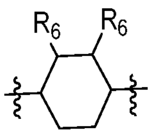, 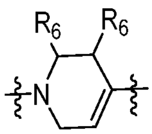, 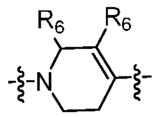, 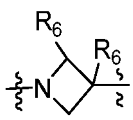,

CO, O, NR$_{L2}$, C$_{1-4}$ alkylene, C$_{2-4}$ alkenylene, C$_{2-4}$ alkynylene,

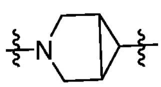 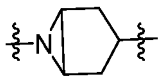 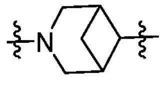 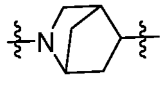 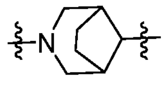 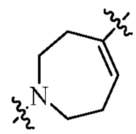

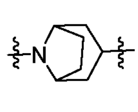 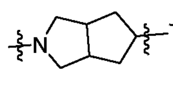 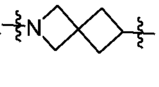 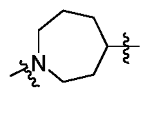 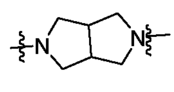

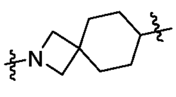 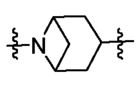 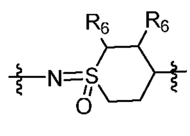 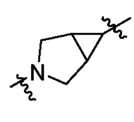 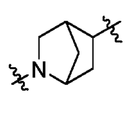 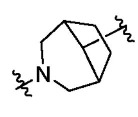

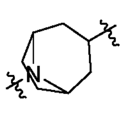 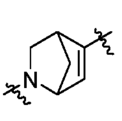 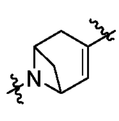 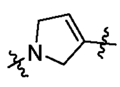 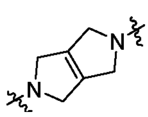 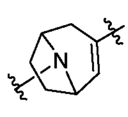 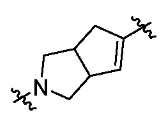

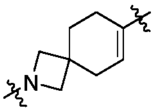 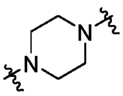 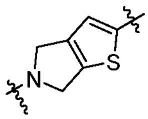 ;

or each

$L_2$ is independently selected from

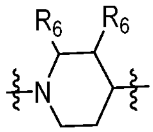, 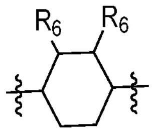, 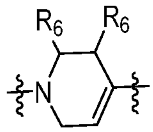, 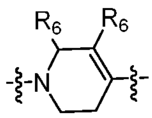,

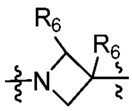,

CO, O, $NR_{L2}$, $C_{1-4}$ alkylene, $C_{2-4}$ alkenylene, $C_{2-4}$ alkynylene,

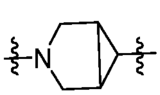 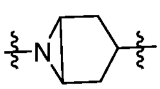 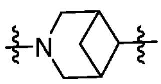 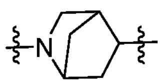 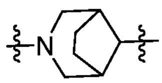 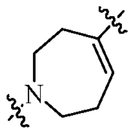

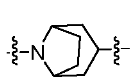 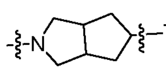 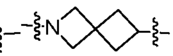 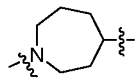 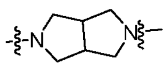

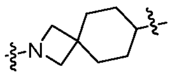 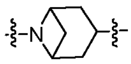 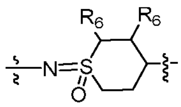 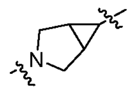 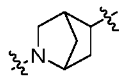 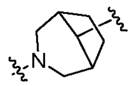

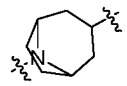 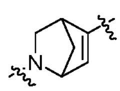 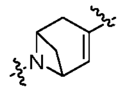 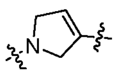 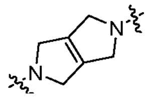 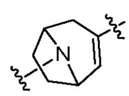 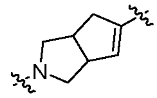

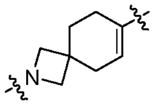 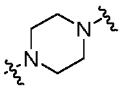 ;

or each $L_2$
is independently selected from

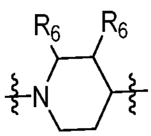, 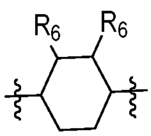, 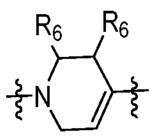, 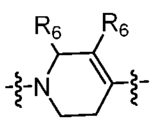,

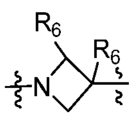,

CO, O, $NR_{L2}$, $C_{1-4}$ alkylene, $C_{2-4}$ alkenylene, $C_{2-4}$ alkynylene,

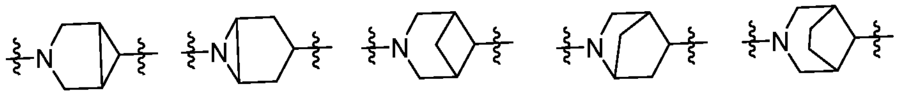

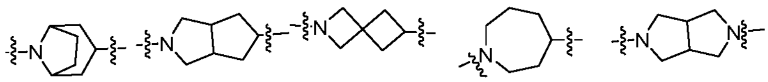

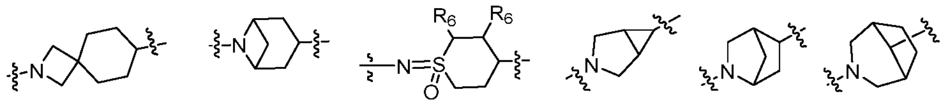

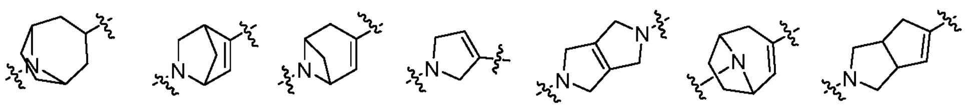

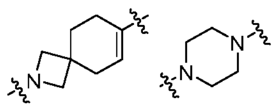

;

or each $L_2$ is independently selected from

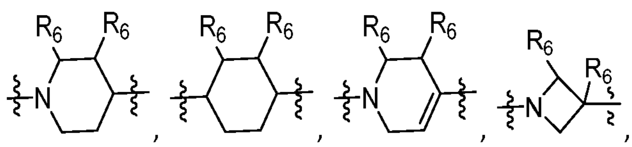

, , , ,

CO, O, $NR_{L2}$, $C_{1-4}$ alkylene, $C_{2-4}$ alkenylene, $C_{2-4}$ alkynylene,

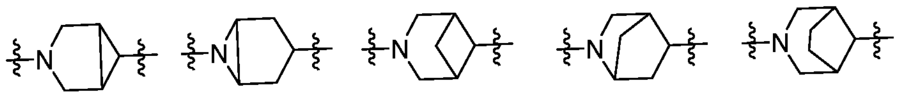

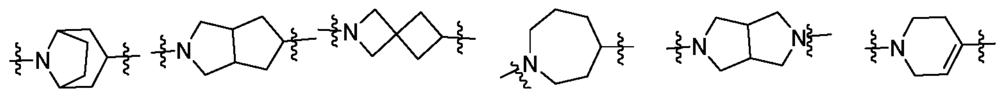

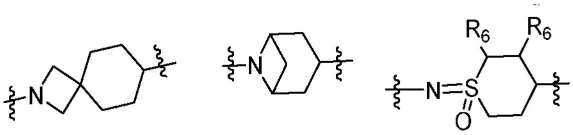

;

$R_{L2}$ is H, $C_{1-4}$ alkyl, or $C_{3-6}$ cycloalkyl;
$L_3$ is selected from

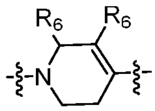

,

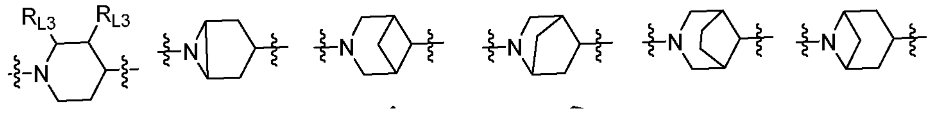

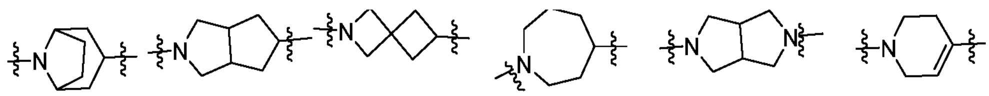

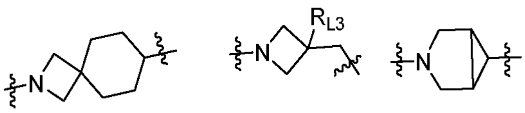

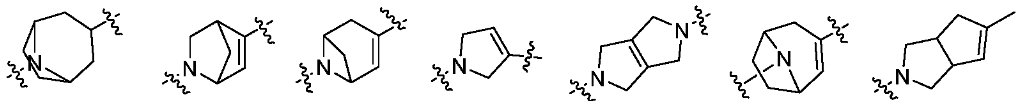

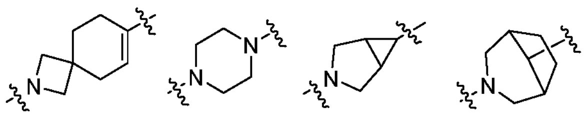

;

or $L_3$ is selected from

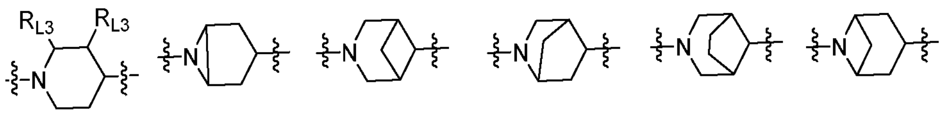

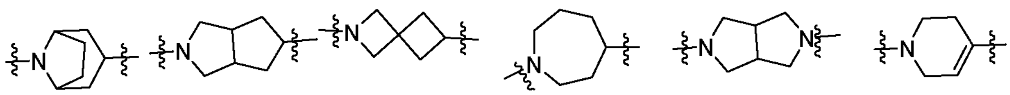

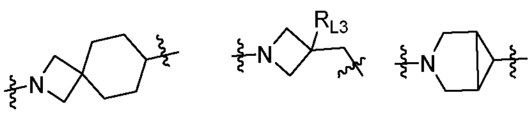

;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form a $C_{3-6}$ cycloalkyl or a 5- to 6-membered heterocycloalkyl containing 1-3 heteroatoms selected from N, S, and O;

A is -S(O)-, -C(O)-, -B(OH)-, -S-, or -S(=N)-CN;

Cy5 is selected from

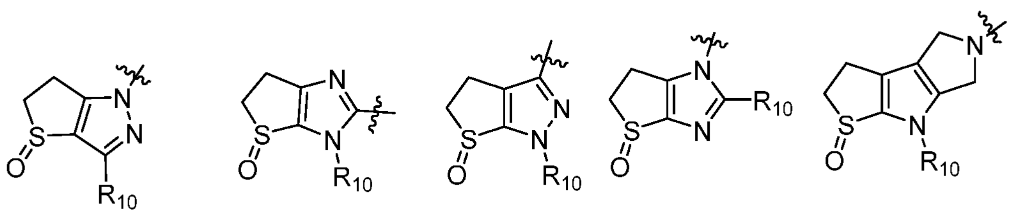

;

each $R_{X1}$ and R are independently H, halogen, $C_{1-4}$ alkyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $R_{X1}$ and R are independently 3- to 5-membered cycloalkyl, or two R on adjacent ring atoms, R and $R_{X1}$, or $R_{X1}$ and $R_6$, together with the atom to which they are attached, form $C_{3-6}$ cycloalkyl, wherein the cycloalkyl is optionally substituted with 1-3 groups selected from

halogen, =O, CN, $C_{1-4}$ alkyl, $C_{1-4}$ alkoxy, OH, and $NH_2$;
$R_1$ is $C_{1-4}$ alkyl or $C_{1-4}$ haloalkyl;
or $R_1$ and $R_2$, together with the atom to which they are attached, form a ring selected from:

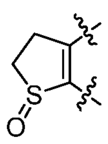 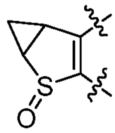 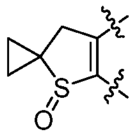 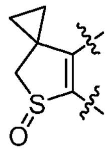 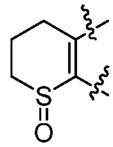 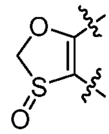

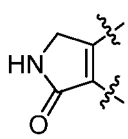 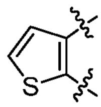 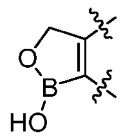 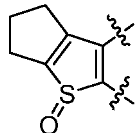 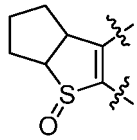 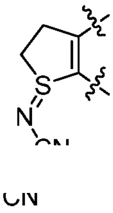

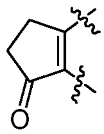 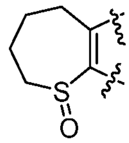 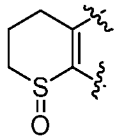 ,

optionally substituted with 1-3 groups selected from halogen, =O, CN, $C_{1-4}$ alkyl, $C_{1-4}$ alkoxy, OH, and $NH_2$;
or $R_1$ and $R_2$, together with the atom to which they are attached, form a ring selected from:

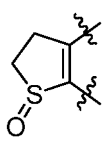 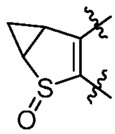 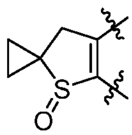 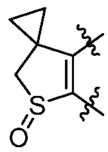 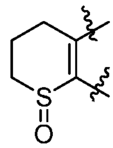 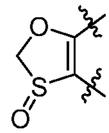

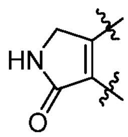 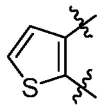 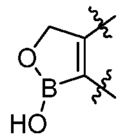 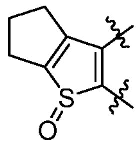 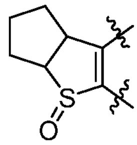 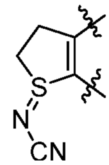

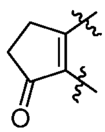 ,

optionally substituted with 1-3 groups selected from halogen, =O, CN, $C_{1-4}$ alkyl, $C_{1-4}$ alkoxy, OH, and $NH_2$;
$R_3$ is H, halogen, $C_{1-4}$ alkyl, CN, OH, or absent;
or $R_3$ and $R_2$, together with the atom to which they are attached, form a ring selected from:

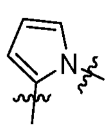 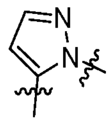 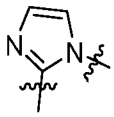 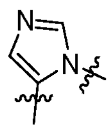 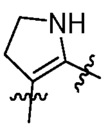 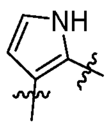 ,

optionally substituted with 1-3 groups selected from halogen, =O, CN, $C_{1-4}$ alkyl, $C_{1-4}$ alkoxy, OH, and $NH_2$;
or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN,

$C_{1-4}$ alkyl, $C_{1-4}$ alkoxy, OH, and $NH_2$;

or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent; or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$; $R_4$ is H, $C_{1-4}$ alkyl, or $C_{1-4}$ haloalkyl;

$R_5$ is H, $C_{1-4}$ alkyl, $C_{1-4}$ haloalkyl, $C_{1-4}$ alkoxy, or $-NHC_{1-4}$ alkyl;

$R_6$ is H, halogen, =O, CN, $C_{1-4}$ alkyl, or $C_{1-4}$ alkoxy;

$R_7$ is $C_{1-4}$ alkyl, or $C_{1-4}$ haloalkyl, or $R_7$ and $R_9$, together with the atom to which they are attached, form a ring selected from:

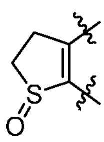 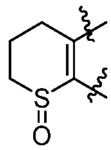 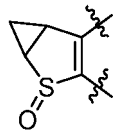 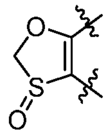 ,

wherein the ring is optionally substituted with 1-3 groups selected from halogen, CN, $C_{1-4}$ alkyl, $C_{1-4}$ alkoxy, OH, and $NH_2$;

or $R_{9'}$ and $R_7$, together with the atom to which they are attached, form a ring selected from:

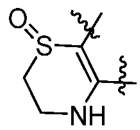 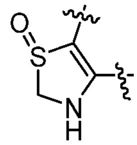 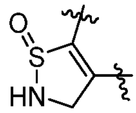 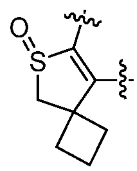 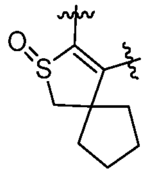 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

or $R_{9'}$ and $R_{x5}$, together with the atom to which they are attached, form

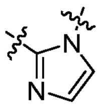 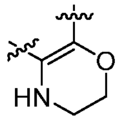 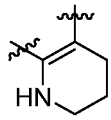 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

provided that at least one group of $R_{9'}$ and $R_7$ or $R_{9'}$ and $R_{x5}$ form a ring;

$R_8$ is $R_{8'}$, $-OR_{8'}$, or $-(CH_2)_rR_{8'}$;

$R_{8'}$ is $C_{3-6}$ cycloalkyl, wherein the cycloalkyl is optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H or $C_{1-4}$ alkyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

n is 0, 1, 2, or 3;

m is 0, 1, or 2; and

r is 1 or 2.

Solution 10 of the present disclosure relates to the compounds or the stereoisomers or pharmaceutically acceptable salts thereof as described above in solutions 1-6, wherein

each $L_2$ is independently selected from

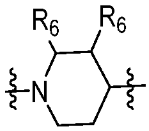, 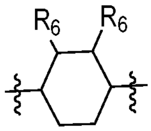, 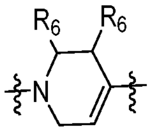, 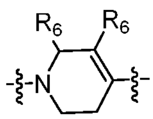,

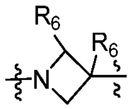,

CO, O, $NR_{L2}$, $C_{1-4}$ alkylene, $C_{2-4}$ alkenylene, $C_{2-4}$ alkynylene,

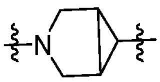 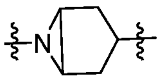 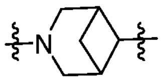 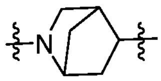 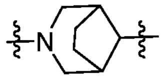 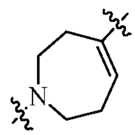

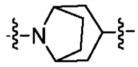 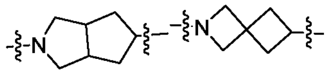 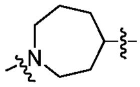 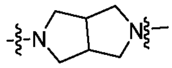

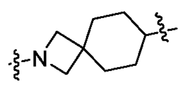 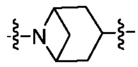 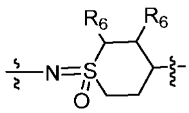 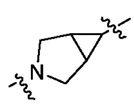 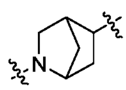 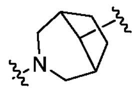

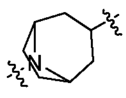 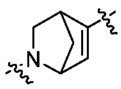 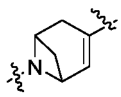 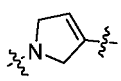 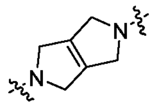 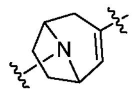 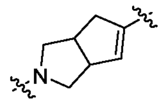

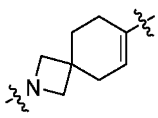 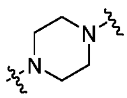 ,

or

each $L_2$ is independently selected from

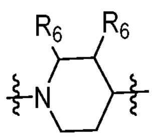, 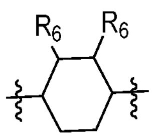, 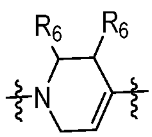, 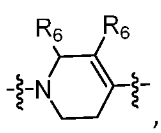,

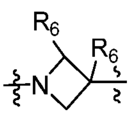,

CO, O, $NR_{L2}$, $C_{1-4}$ alkylene, $C_{2-4}$ alkenylene, $C_{2-4}$ alkynylene,

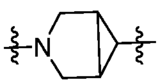 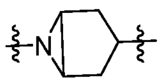 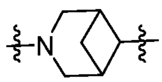 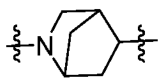 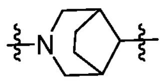

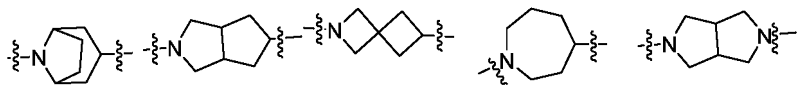

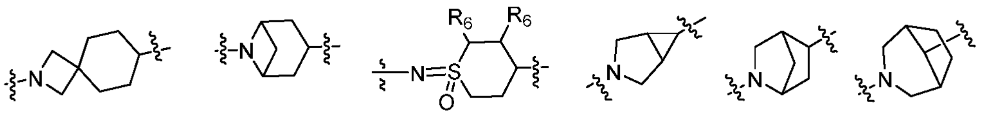

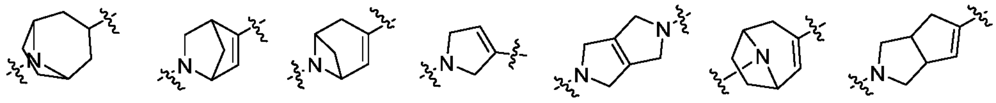

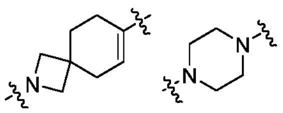

;

$R_{L2}$ is H, $C_{1-4}$ alkyl, or $C_{3-6}$ cycloalkyl;
$L_3$ is selected from

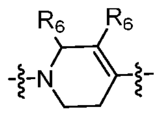

,

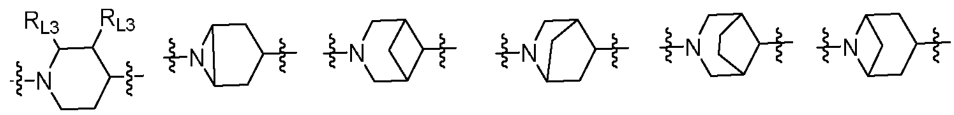

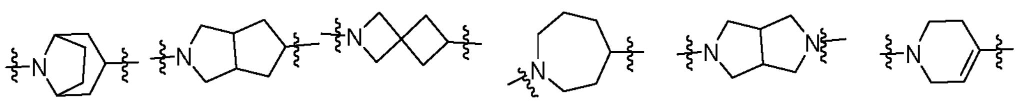

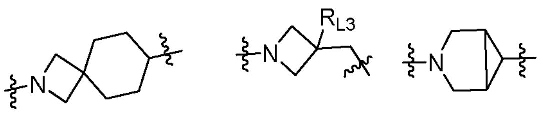

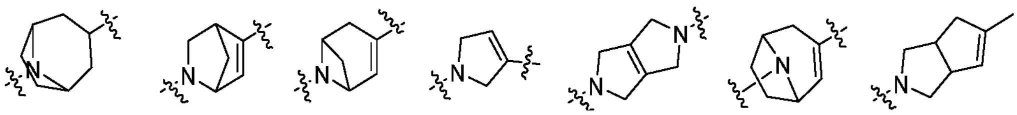

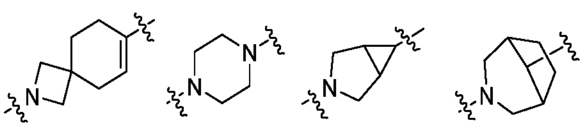

;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 6-membered heterocycloalkyl containing 1-3 heteroatoms selected from N, S, and O;
$L_4$ is selected from

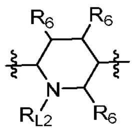, 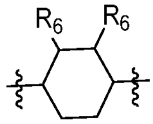, 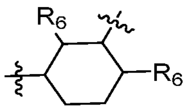, 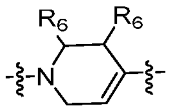, 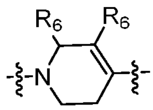,

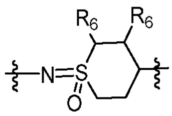, 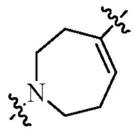,

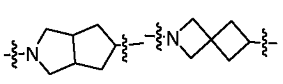 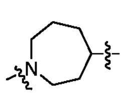 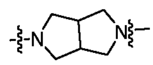 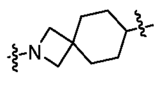 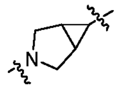 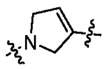

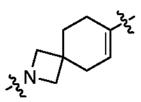 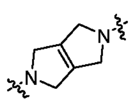 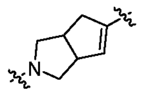,

or $L_4$ is selected from

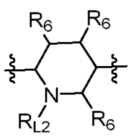, 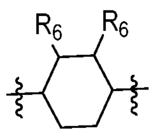, 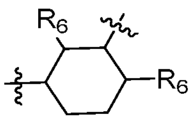, 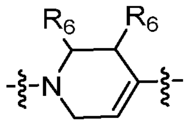, 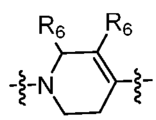,

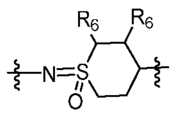,

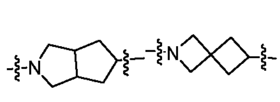 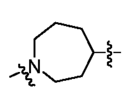 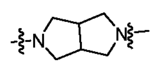 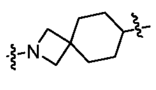 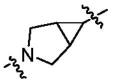 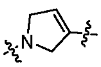

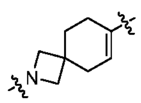 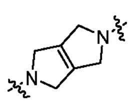 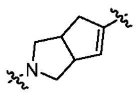;

A is -S(O)-, -C(O)-, -B(OH)-, -S-, or -S(=N)-CN;
Cy5 is selected from

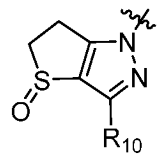 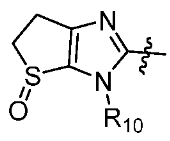 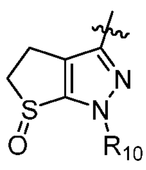 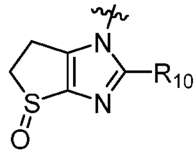 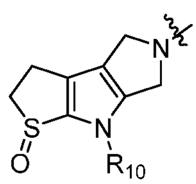;

each $R_{X1}$ and R are independently H, halogen, $C_{1-4}$ alkyl, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, or $C_{1-4}$ haloalkyl, or $R_{X1}$ and R are independently a 3- to 5-membered cycloalkyl, or two R on adjacent ring atoms, R and $R_{X1}$, or $R_{X1}$ and $R_6$, together with the atom to which they are attached, form $C_{3-6}$ cycloalkyl or a 5- to 6-membered heterocycloalkyl containing 1-3

heteroatoms selected from N, S, and O, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;
$R_1$ is $C_{1-4}$ alkyl or $C_{1-4}$ haloalkyl;
or $R_1$ and $R_2$, together with the atom to which they are attached, form a ring selected from:

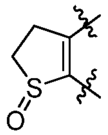 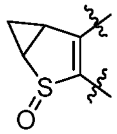 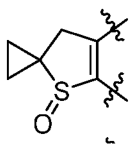 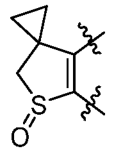 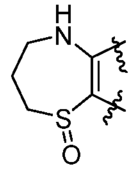 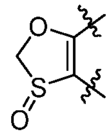

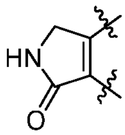 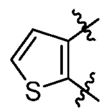 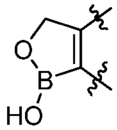 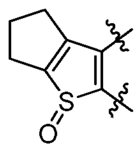 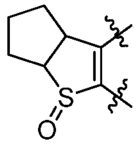 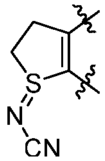

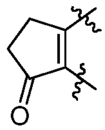 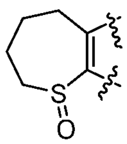 ,

or $R_1$ and $R_2$, together with the atom to which they are attached, form

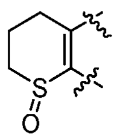 ,

optionally substituted with 1-3 groups selected from halogen, =O, CN, $C_{1-4}$ alkyl, $C_{1-4}$ alkoxy, OH, and $NH_2$;
$R_3$ is H, halogen, $C_{1-4}$ alkyl, CN, OH, or absent;
or $R_3$ and $R_2$, together with the atom to which they are attached, form a ring selected from:

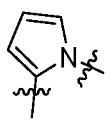 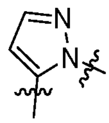 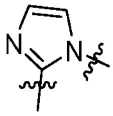 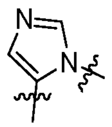 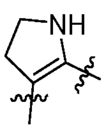 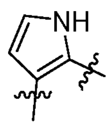 ,

optionally substituted with 1-3 groups selected from halogen, =O, CN, $C_{1-4}$ alkyl, $C_{1-4}$ alkoxy, OH, and $NH_2$;
or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$;
$R_{x4}$ is H, halogen, $C_{1-4}$ alkyl, CN, OH, or absent; or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$;
$R_4$ is H, $C_{1-4}$ alkyl, or $C_{1-4}$ haloalkyl;
$R_5$ is H, $C_{1-4}$ alkyl, $C_{1-4}$ haloalkyl, $C_{1-4}$ alkoxy, or -$NHC_{1-4}$ alkyl;
$R_6$ is H, halogen, =O, CN, $C_{1-4}$ alkyl, or $C_{1-4}$ alkoxy;
$R_7$ is $C_{1-4}$ alkyl, or $C_{1-4}$ haloalkyl, or $R_7$ and $R_9$, together with the atom to which they are attached, form a ring selected from:

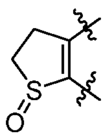 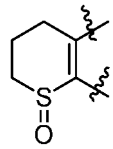 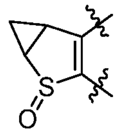 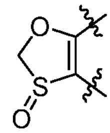 ,

wherein the ring is optionally substituted with 1-3 groups selected from halogen, CN, $C_{1-4}$ alkyl, $C_{1-4}$ alkoxy, OH, and $NH_2$;

or $R_{9'}$ and $R_7$, together with the atom to which they are attached, form a ring selected from:

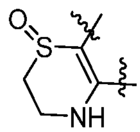 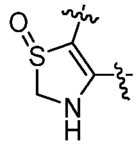 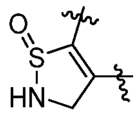 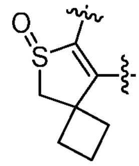 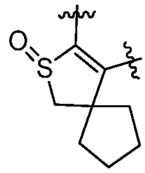 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

or $R_{9'}$ and $R_{x5}$, together with the atom to which they are attached, form

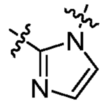 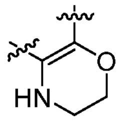 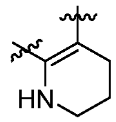 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; provided that at least one group of $R_{9'}$ and $R_7$ or $R_{9'}$ and $R_{x5}$ form a ring;

$R_8$ is $R_{8'}$, $-OR_{8'}$, or $-(CH_2)_rR_{8'}$;

$R_{8'}$ is $C_{3-6}$ cycloalkyl, wherein the cycloalkyl is optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H or $C_{1-4}$ alkyl;

each $R_{10}$ is independently $R_{8'}$, $-OR_{8'}$, $-NHR_{8'}$, or $-(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from cycloalkyl, cyclohexyl, phenyl, cyclopropyl, adamantyl, tetrahydropyranyl, piperidinyl, oxolanyl,

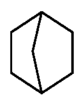 ,

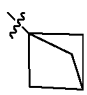 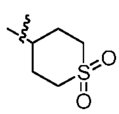 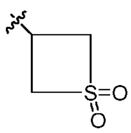 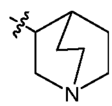 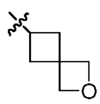 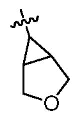 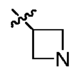 ;

wherein the ring is optionally substituted with 1-3 groups selected from halogen, CN, OH, $C_{1-4}$ alkyl, hydroxy $C_{1-4}$ alkyl, and $C_{1-4}$ haloalkyl;

$R_{13}$ is selected from

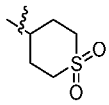 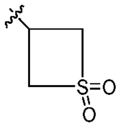 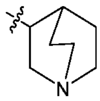 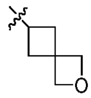 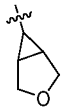 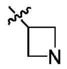 ;

wherein the ring is optionally substituted with 1-3 groups selected from halogen, CN, OH, $C_{1-4}$ alkyl, hydroxy $C_{1-4}$ alkyl, and $C_{1-4}$ haloalkyl;
n is 0, 1, 2, or 3;
m is 0, 1, or 2; and
r is 1 or 2.

Solution 11 of the present disclosure relates to the compounds, or the stereoisomers or pharmaceutically acceptable salts thereof, as described above in solutions 1-7, wherein

Cy1 is selected from

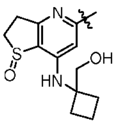 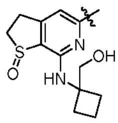 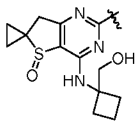 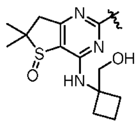 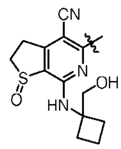 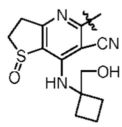 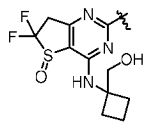

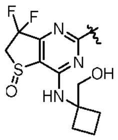 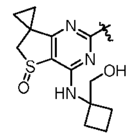 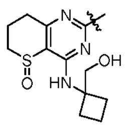 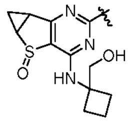 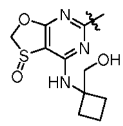 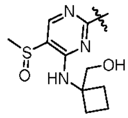

or from

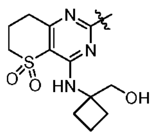 and 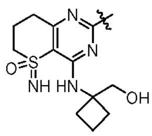 ,

or is

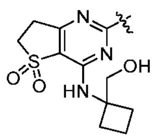 ;

Cy2 is selected from

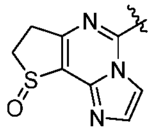 and 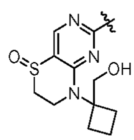 ;

Cy4 is selected from

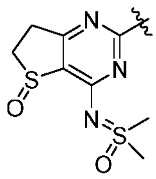 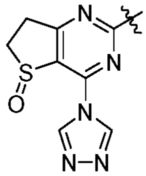 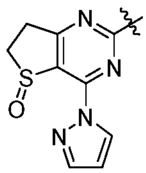 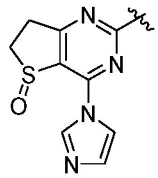

and

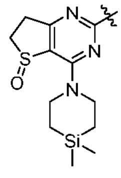 ,

or is

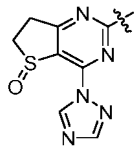 ,

or is

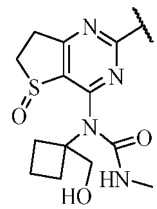 ,

or is

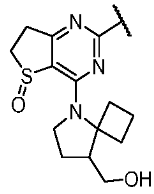 ,

or is

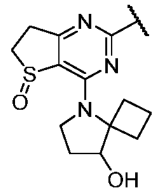 ,

or is

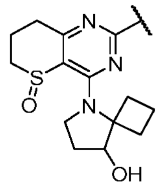 ;

Cy5 is

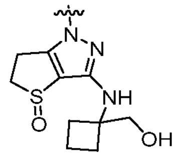 ;

Cy7 is selected from

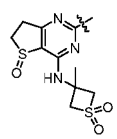
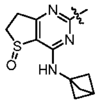
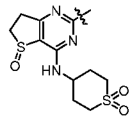
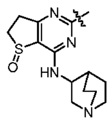
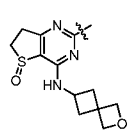
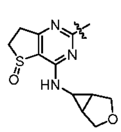
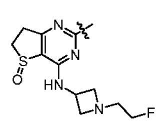

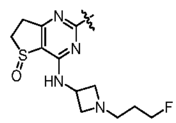

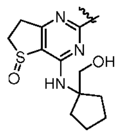
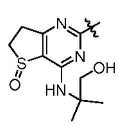
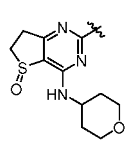
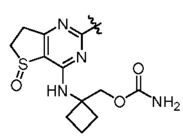
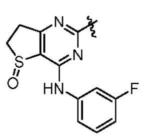
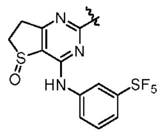

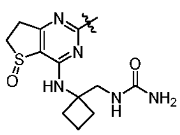
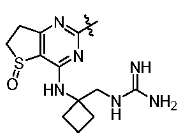
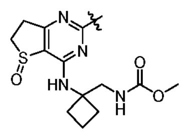
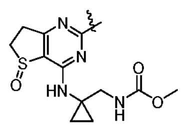

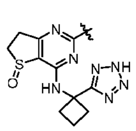
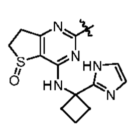
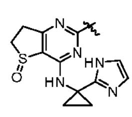
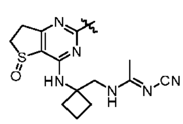
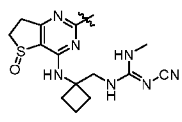
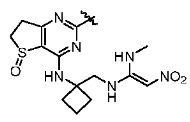

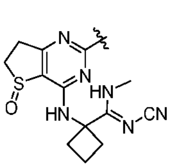
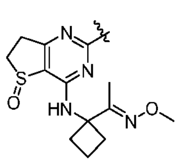
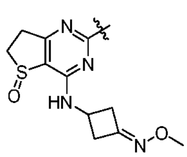
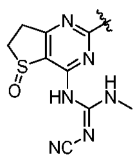
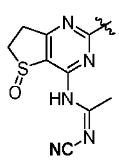
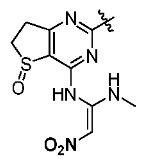 ;

or from

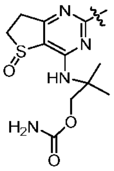 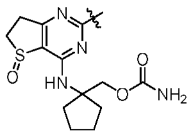 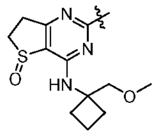 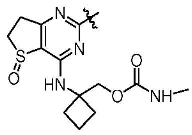 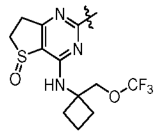 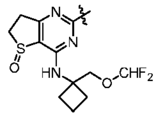

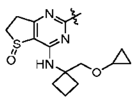 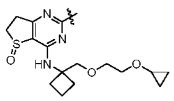 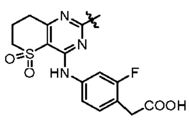 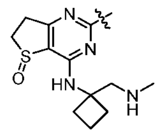 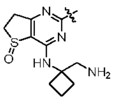 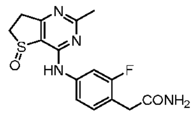

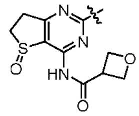 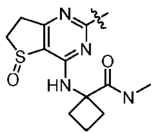 and 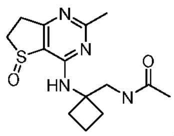 ;

or is

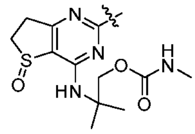 ;

or is selected from

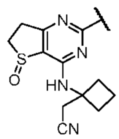 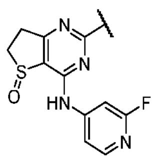 and 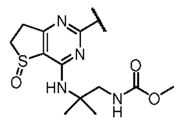 ;

or is

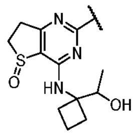 ;

or is

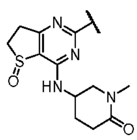 ;

or is selected from

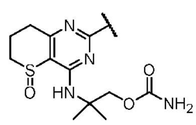 and 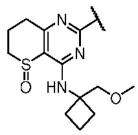 ;

or is selected from

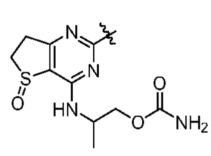 and 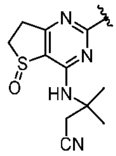 ;

or is selected from

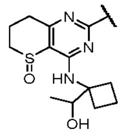 and 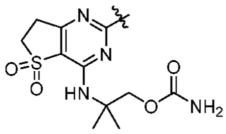 ;

and
Cy9 is selected from

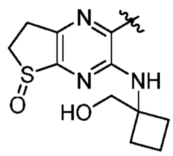 .

Solution 12 of the present disclosure relates to the compounds, or the stereoisomers or pharmaceutically acceptable salts thereof, as described above in solutions 1-7, wherein Cy1 is selected from

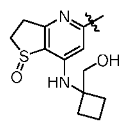 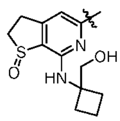 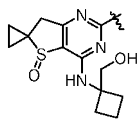 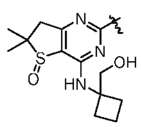 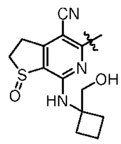 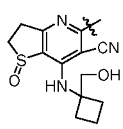 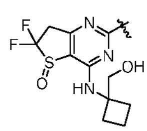

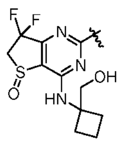 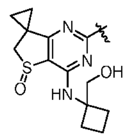 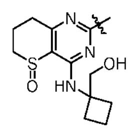 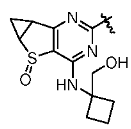 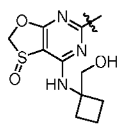 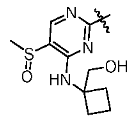

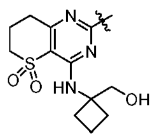 and 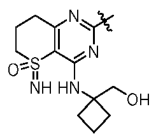 ;

or Cy1 is selected from

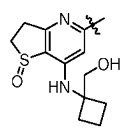 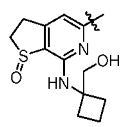 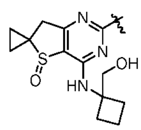 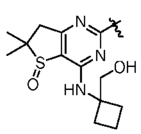 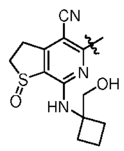 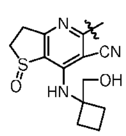 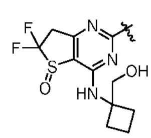

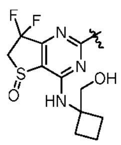 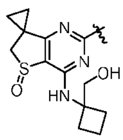 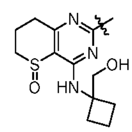 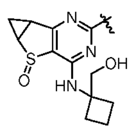 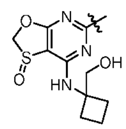 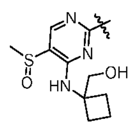 ;

or Cy1 is selected from

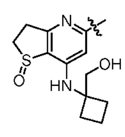 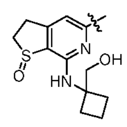 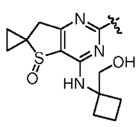 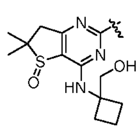 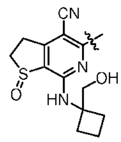 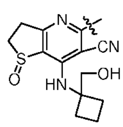 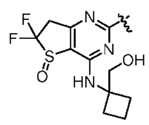

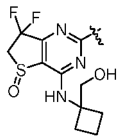 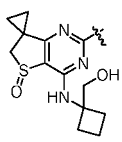 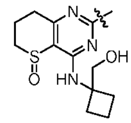 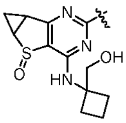 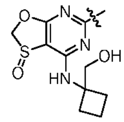 ;

or Cy1 is

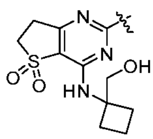 ;

Cy5 is

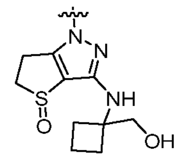 ;

or Cy5 is

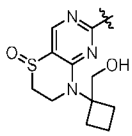 ;

Cy7 is selected from

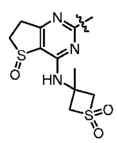 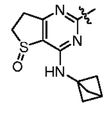 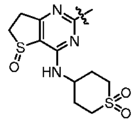 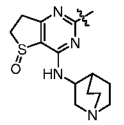 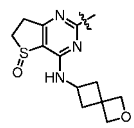 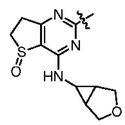 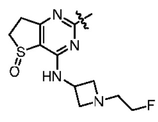 ;

and

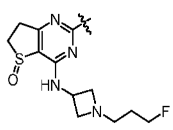 ;

or Cy7 is selected from

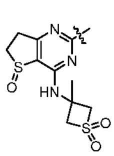 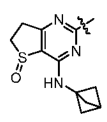 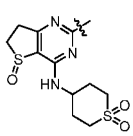 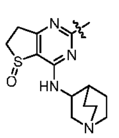 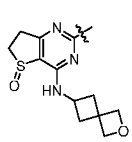 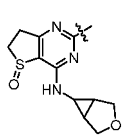 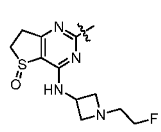 ,

or

Cy7 is

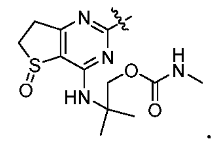.

Solution 13 of the present disclosure relates to the compounds, or the stereoisomers or pharmaceutically acceptable salts thereof, as described above in solutions 1-7, wherein $-(L_1)n-(L_2)m-$ is selected from

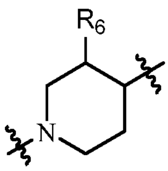, 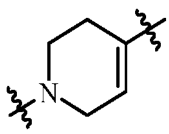, 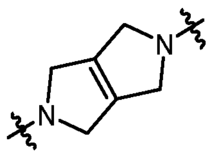, 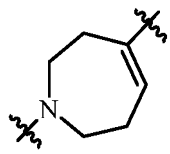,

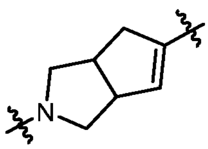, 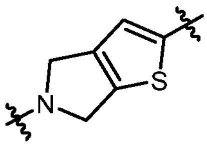, 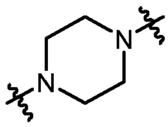, 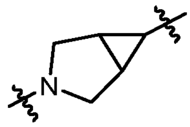, and 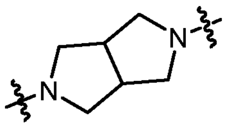,

with $R_6$ being H;

$-L_3-$ is selected from:

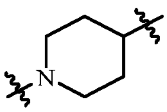, 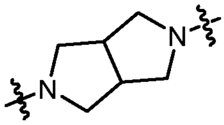, 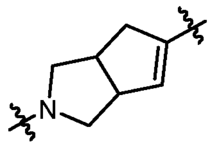, and 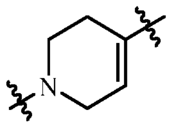;

$-L_4-$ is selected from:

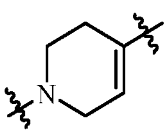, 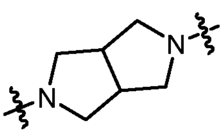, 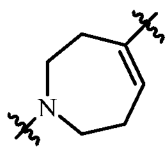, 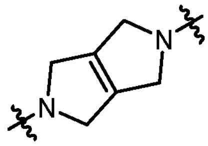,

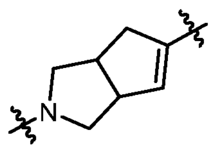, and 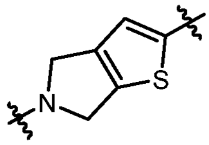,

or is

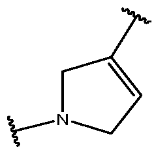;

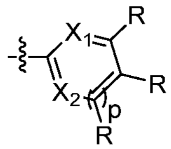

is selected from one of the following structures optionally substituted with 1-3 $R_R$:

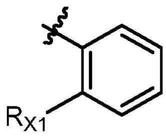, 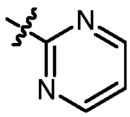, 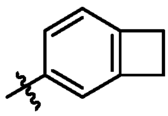, 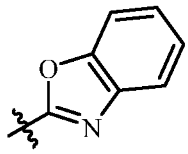, 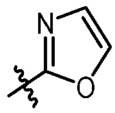, 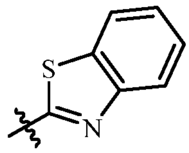,

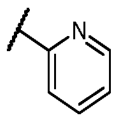, 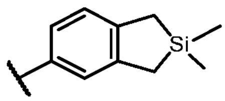, 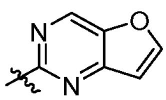, 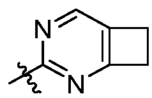, 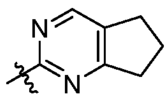,

and each $R_R$ is independently selected from F, Cl, Br, methyl, isopropyl, ethoxy, methoxy, ethynyl, propynyl, cyclopropyl, cyclobutyl, dimethylamino, and

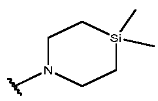;

and in some embodiments,

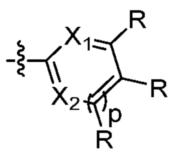

is selected from one of the following structures optionally substituted with 1-3

$R_R$:

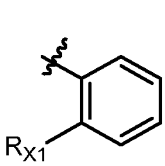, 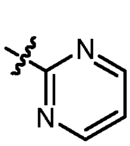, 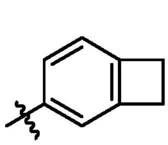, 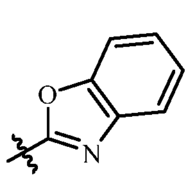, 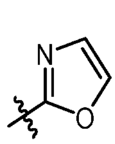, 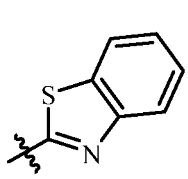,

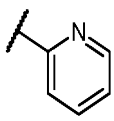, and 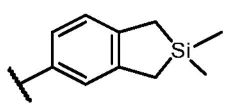,

each $R_R$ is independently selected from F, Cl, Br, methyl, ethynyl, propynyl, cyclopropyl,

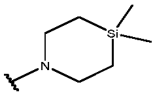;

$R_{X1}$ is H;

in some embodiments,

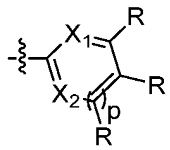

is selected from one of the following structures optionally substituted with 1-3 $R_R$:

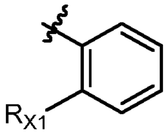, 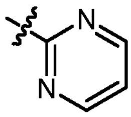, 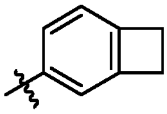, 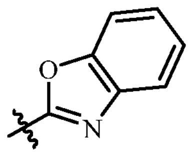,

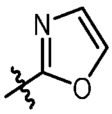, 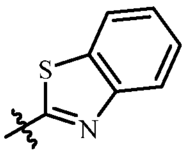, and 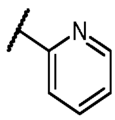,

and each $R_R$ is independently selected from F, Cl, Br, methyl, ethynyl, propynyl, or cyclopropyl; $R_{X1}$ is H;

in some embodiments,

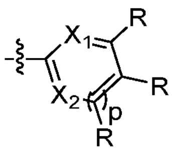

is selected from one of the following structures optionally substituted with 1-3 $R_R$:

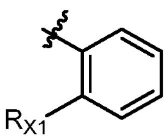, 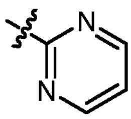, 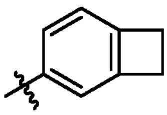, 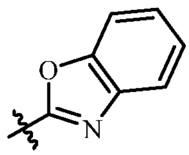,

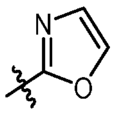, and 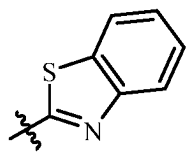,

and each $R_R$ is independently selected from F, Cl, Br, methyl, ethynyl, propynyl, or cyclopropyl; $R_{X1}$ is H;

or $R_{X1}$ and $R_6$, together with the atom to which they are attached, form morpholinyl, furyl, pyrrolyl, or thienyl; in some embodiments, $R_{X1}$ and $R_6$, together with the atom to which they are attached, form morpholinyl, furyl, or thienyl.

Solution 14 of the present disclosure relates to the compounds, or stereoisomers or pharmaceutically acceptable salts thereof, as described above in solutions 1-7, wherein $-(L_1)n-(L_2)m-$ is selected from

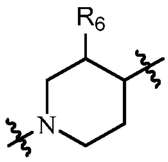, 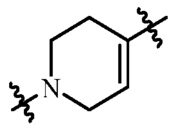, 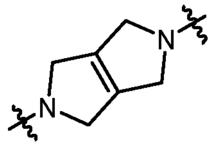, 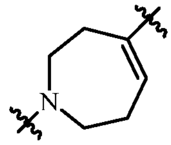,

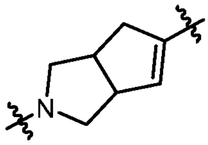, 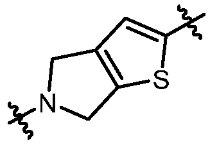, 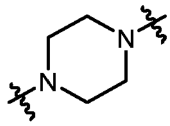, 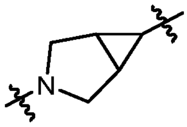, and 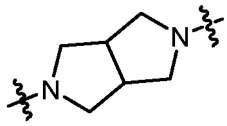,

with $R_6$ being H;
$-L_3-$ is selected from:

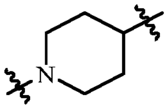, 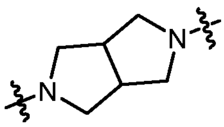, 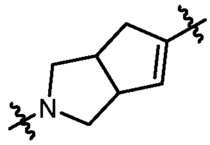, and 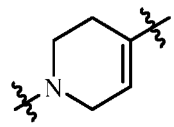;

$-L_4-$ is selected from

: 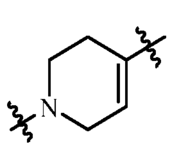, 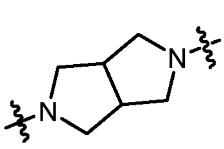, 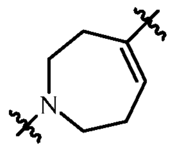, 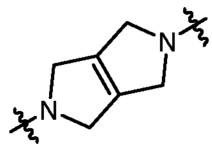,

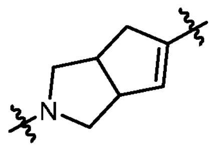, and 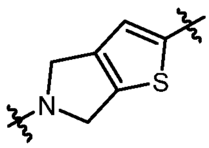;

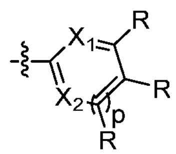

is selected from one of the following structures optionally substituted with 1-3 $R_R$:

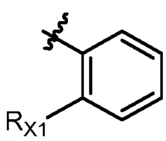, 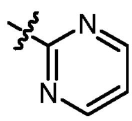, 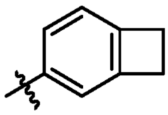, 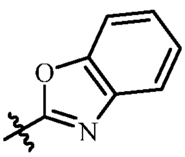, 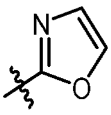, and 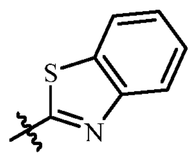,

and each $R_R$ is independently selected from F, Cl, Br, methyl, ethynyl, or cyclopropyl; $R_{X1}$ is H; and or $R_{X1}$ and $R_6$, together with the atom to which they are attached, form morpholinyl, furyl, or thienyl.

Solution 15 of the present disclosure relates to the compounds, or the stereoisomers or pharmaceutically acceptable salts thereof, as described above in solutions 1-5,

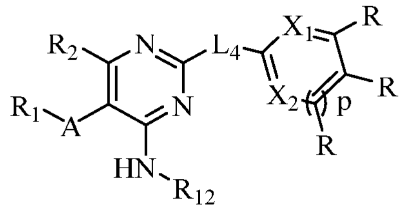

II

,

wherein $L_4$ is

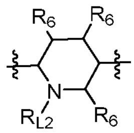 , 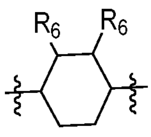 , 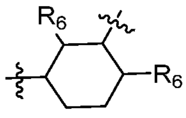 , 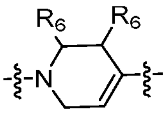 , 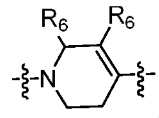 ,

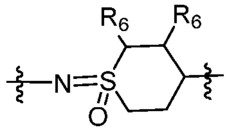 , 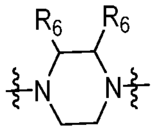 ,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

A is -S(O)-, -C(O)-, -B(OH)-, -S-, -S(=N)-CN, or -C(=N)-CN;
$X_1$ and $X_2$ are independently $CR_{X1}$, S, or N;
$R_{L2}$ is H, $C_{1-4}$ alkyl, or $C_{3-6}$ cycloalkyl;
$R_6$ is H, halogen, =O, CN, $C_{1-4}$ alkyl, or $C_{1-4}$ alkoxy;
each $R_{X1}$ and R are independently H, halogen, $C_{1-4}$ alkyl, $C_{1-4}$ alkoxy, $-N(CH_3)_2$, $-NH(CH_3)$, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
$R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;
$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, $C_{1-4}$ alkyl-CN, -CRaRb=N-O-$C_{1-4}$ alkyl, -C(NRaRb)=N-CN, -C($C_{1-4}$ alkyl)=N-CN, -C(NRaRb)=CRa-$NO_2$, $-C(CH_3)_2-CH_2-O-C(O)NH_2$, $-C(CH_3)_2-CH_2-NH-C(O)O(C_{1-4}$ alkyl), $-C(CH_3)_2-CH_2-O-C(O)NHCH_3$, $- CH(CH_3)-CH_2-O-C(O)NH_2$, $-CH(CH_3)-CH_2-NH-C(O)O(C_{1-4}$ alkyl), $-CH(CH_3)-CH_2-O-C(O)NHCH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from

halogen, CN, =O, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t-NRa-C(O)NH_2$, $-(CH_2)_t-NRa-C(=NH)NH_2$, $-(CH_2)_tNRa-C(O)-O-(C_{1-4}$ alkyl), $-(CH_2)_t-NRa-C(C_{1-4}$ alkyl)=N-CN, $-(CH_2)_t-NRa-C(NRaRb)=N-CN$, $-(CH_2)_t-NRa-C(NRaRb)=CRa-NO_2$, $-(CH_2)_t-C(NRaRb)=N-CN$, $-(CH_2)_t-C(C_{1-4}$ alkyl)=N-CN, $-(CH_2)_t-C(C_{1-4}$ alkyl)=N-O-($C_{1-4}$ alkyl), =N-O-$C_{1-4}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, - $(CH_2)_t-O-C_{3-6}$ cycloalkyl, $-(CH_2)_t-O-C_{1-4}$ alkyl-O-$C_{3-6}$ cycloalkyl, $-(CH_2)_t-COOH$, $-(CH_2)_t-NRaRb$, $-(CH_2)_t-C(O)NRaRb$, and $-(CH_2)_t-NRa-C(O)CH_3$;
Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O; and
p is selected from 0 or 1.

Solution 16 of the present disclosure relates to the compound, or the stereoisomer or pharmaceutically acceptable salt thereof, as described above in solution 15,

wherein $L_4$ is

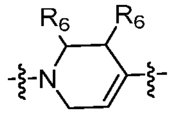 , 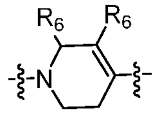 , 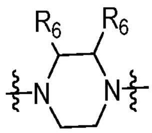 , 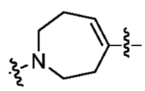 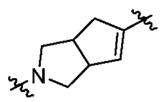 ;

and
$R_{12}$ is selected from $-C(CH_3)_2-CH_2-O-C(O)NH_2$, $-C(CH_3)_2-CH_2-NH-C(O)O(C_{1-4}$ alkyl), $-C(CH_3)_2-CH_2-O-C(O)NHCH_3$, $-CH(CH_3)-CH_2-O-C(O)NH_2$, $-CH(CH_3)-CH_2-NH-C(O)O(C_{1-4}$ alkyl), and $-CH(CH_3)-CH_2-O-C(O)NHCH_3$.

Solution 17 of the present disclosure relates to the compounds as described above in formulas (I) and (II), or stereoisomers or pharmaceutically acceptable salts thereof, wherein

$L_4$ is

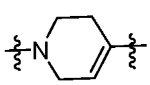 , 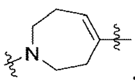 , 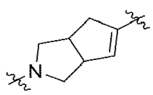 ;

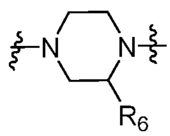 ,

$R_6$ and $R_{X1}$, together with the atom to which they are attached, form a 5- to 7-membered heterocycloalkyl containing 1-3 heteroatoms selected from N, S, O, and Si;
A is -S(O)-;
$R_1$ and $R_2$, together with the atom to which they are attached, form

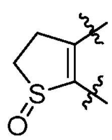 ;

$R_{12}$ is selected from $-C(CH_3)_2-CH_2-O-C(O)NH_2$, $-C(CH_3)_2-CH_2-NH-C(O)O(C_{1-2}$ alkyl), $-C(CH_3)_2-CH_2-O-C(O)NHCH_3$, $-CH(CH_3)-CH_2-O-C(O)NH_2$, $-CH(CH_3)-CH_2-NH-C(O)O(C_{1-2}$ alkyl), and $-CH(CH_3)-CH_2-O-C(O)NHCH_3$;

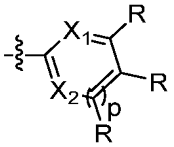

is selected from one of the following structures optionally substituted with 1-3 $R_R$:

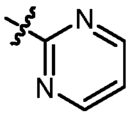, 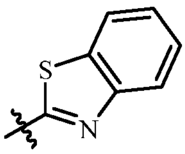, 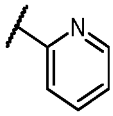, 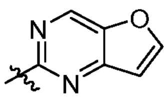, and 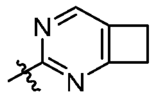,

and each $R_R$ is independently selected from F, Cl, methyl, ethyl, isopropyl, ethoxy, methoxy, ethynyl, propynyl, cyclopropyl, cyclobutyl, and dimethylamino.

Solution 18 of the present disclosure relates to the compound as described above in formula (I), or the stereoisomer or pharmaceutically acceptable salt thereof, wherein

Cy is selected from Cy1, Cy2, Cy4, Cy5, Cy7, and Cy9;

when Cy is selected from Cy1 and Cy2, L is $-(L_1)n-(L_2)m-$; when Cy is selected from Cy4 and Cy5, L is $-L_3-$; and when Cy is selected from Cy7 and Cy9, L is $-L_4-$;

Cy1 is selected from

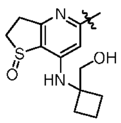 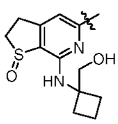 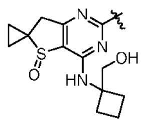 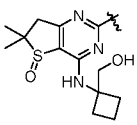 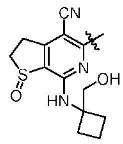 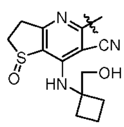 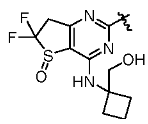

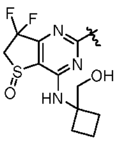 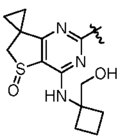 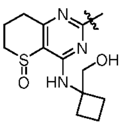 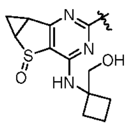 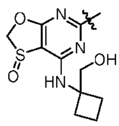 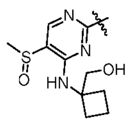

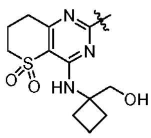 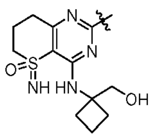 and 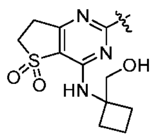 ;

Cy2 is selected from

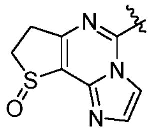 and 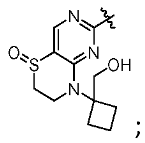 ;

Cy4 is selected from

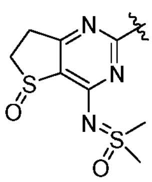 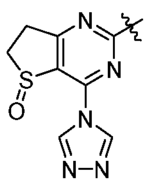 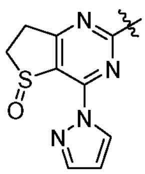 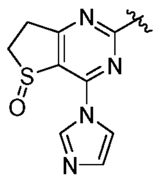 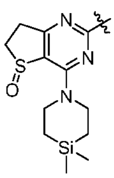 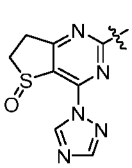

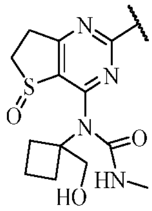 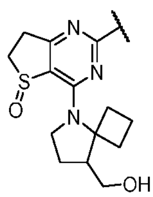 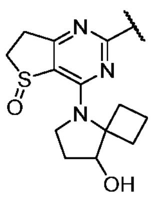 and 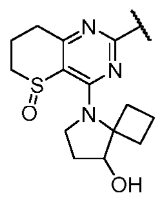 ;

Cy5 is

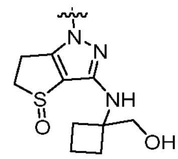 ;

Cy7 is selected from

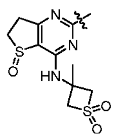 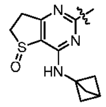 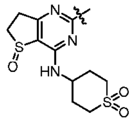 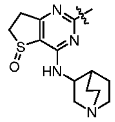 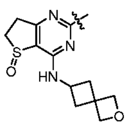 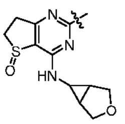 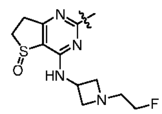

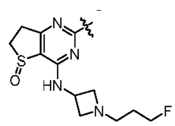

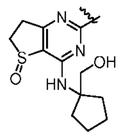 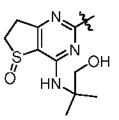 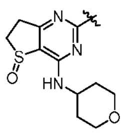 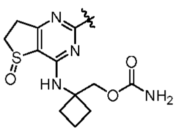 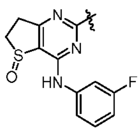 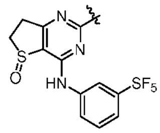

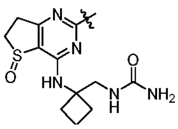 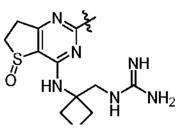 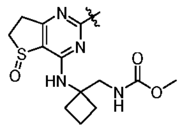 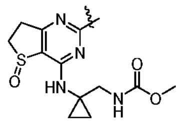

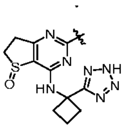 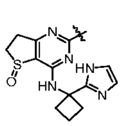 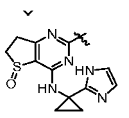 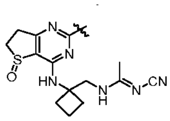 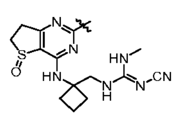 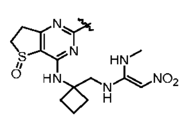

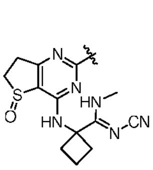 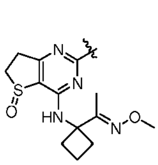 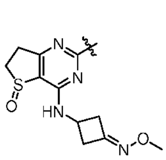 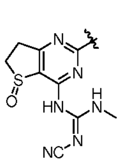 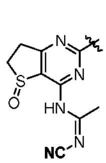 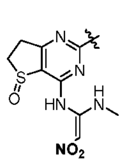 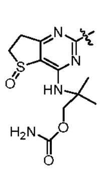

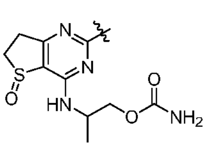 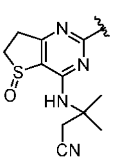 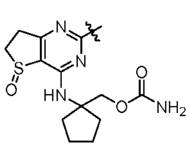 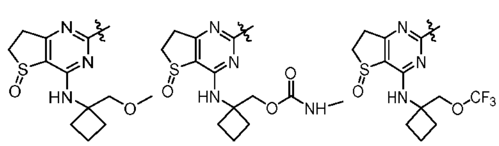

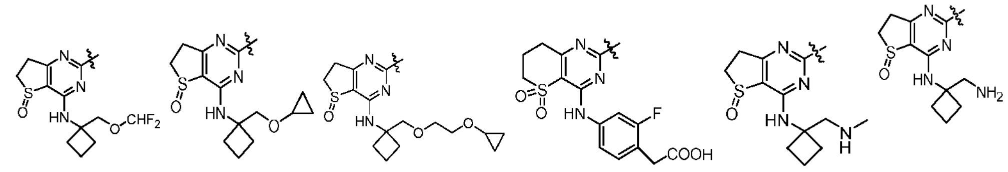

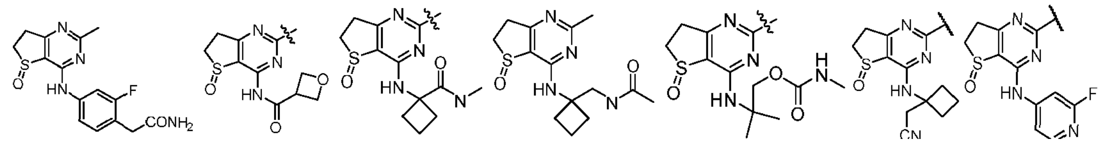

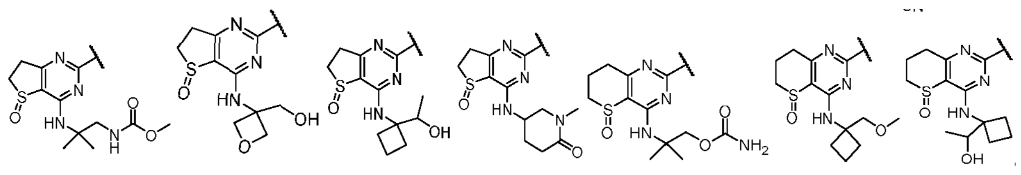

and

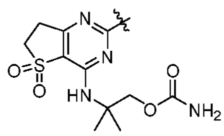
;

Cy9 is

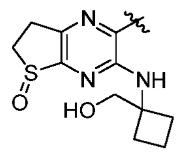
;

-$(L_1)$n-$(L_2)$m- is selected from:

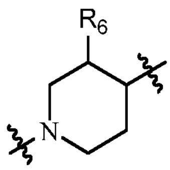
,
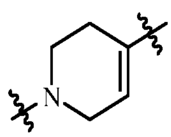
,
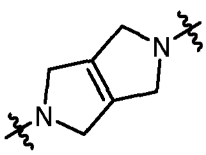
,
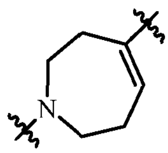
,

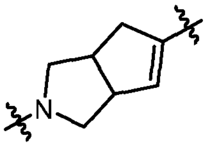
,
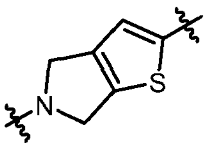
,
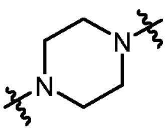
,
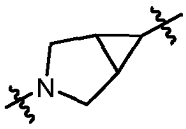
, and
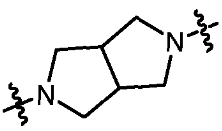
,

with $R_6$ being H;
-$L_3$- is selected from:

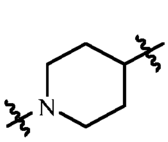, 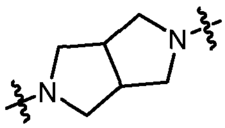, 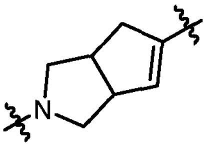, and 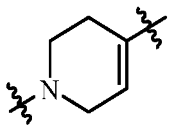;

$-L_4-$ is selected from:

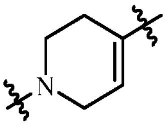, 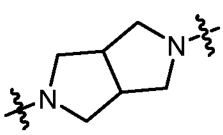, 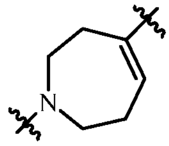, 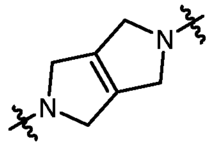,

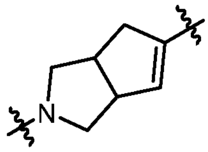, 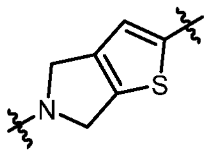, and 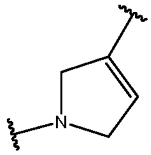;

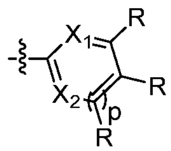

is selected from one of the following structures optionally substituted with 1-3 $R_R$:

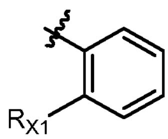, 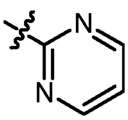, 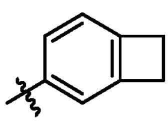, 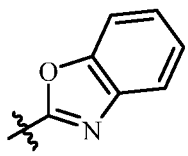, 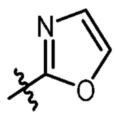, 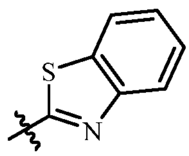,

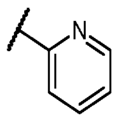, 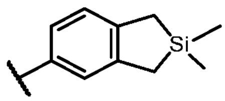, 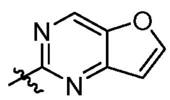, 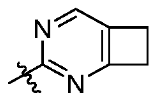, 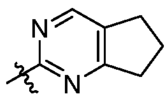,

and each $R_R$ is independently selected from F, Cl, Br, methyl, isopropyl, ethoxy, methoxy, ethynyl, propynyl, cyclopropyl, cyclobutyl, dimethylamino, and

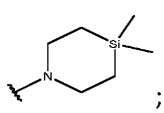;

and $R_{X1}$ is H;
or $R_{X1}$ and $R_6$, together with the atom to which they are attached, form morpholinyl, furyl, pyrrolyl, or thienyl.

Solution 19 of the present disclosure relates to the compound as described above in formula (I), or the stereoisomer or pharmaceutically acceptable salt thereof, wherein

Cy is selected from Cy1 and Cy7;
when Cy is selected from Cy1, L is $-(L_1)n-(L_2)m-$; and when Cy is Cy7, L is $-L_4-$;
Cy1 is selected from

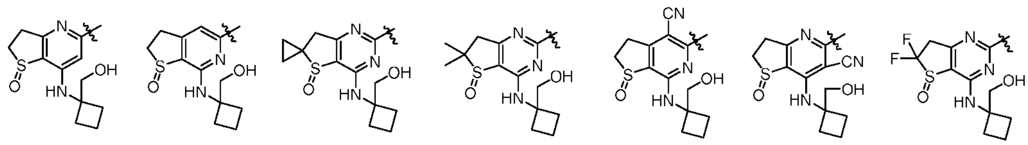

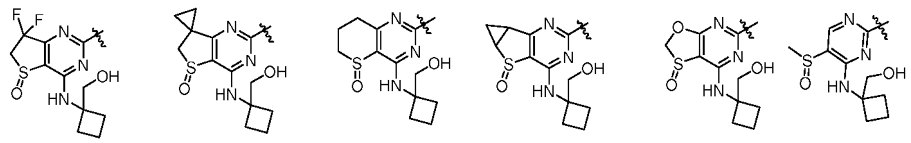

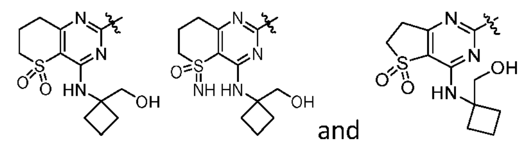 and ;

Cy7 is selected from

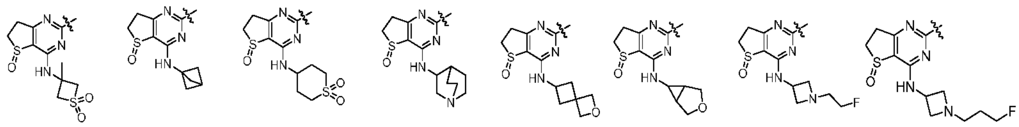

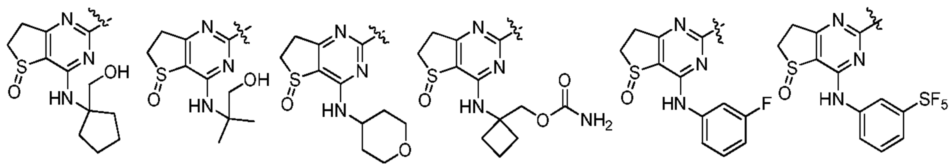

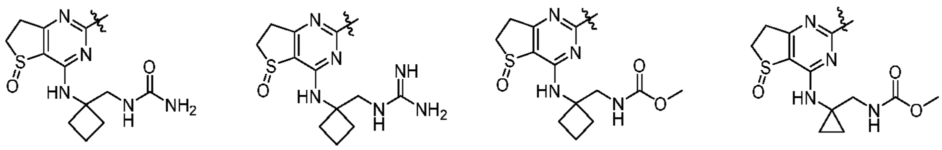

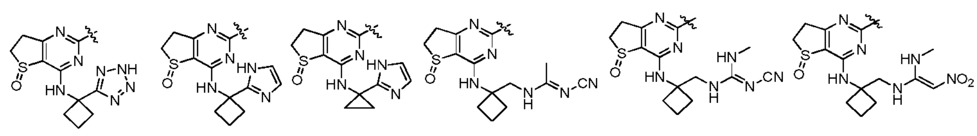

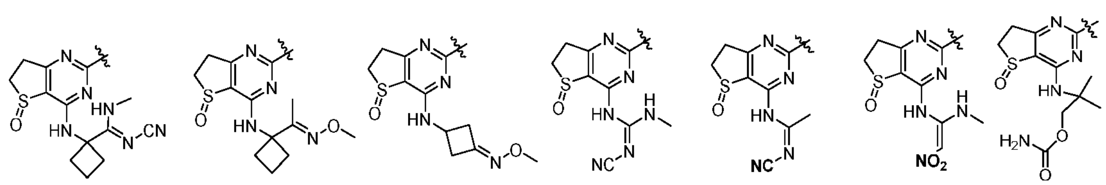

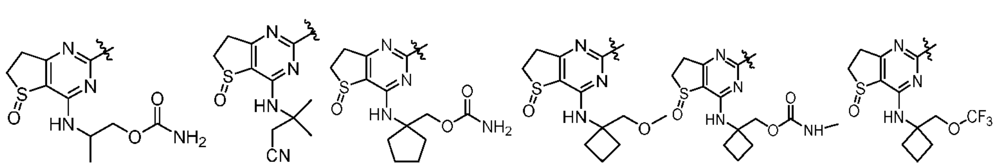

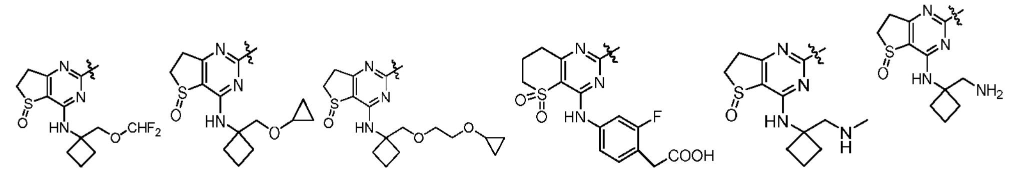

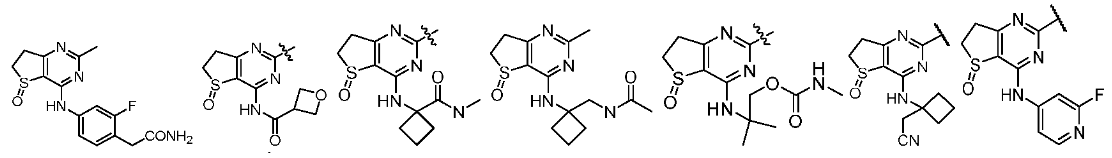

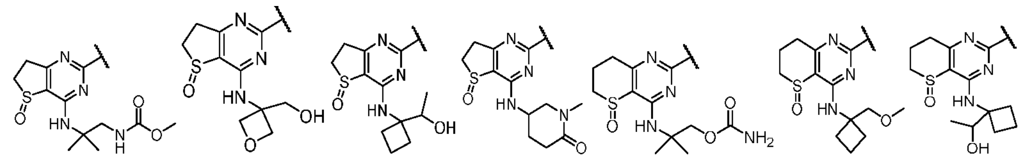

and

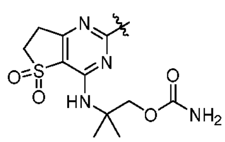;

-$(L_1)n$-$(L_2)m$- is selected from:

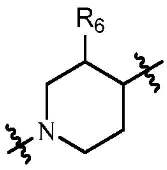, 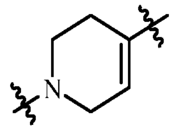, 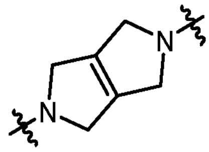, 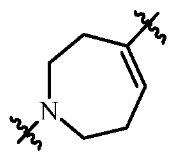,

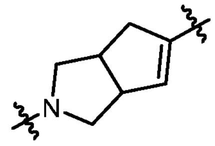, 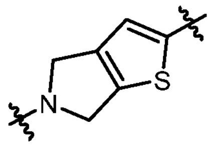, 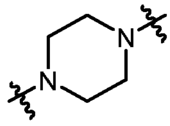, 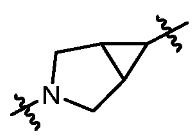, and 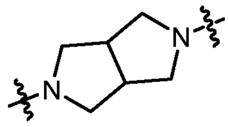,

with $R_6$ being H;
-$L_4$- is selected from:

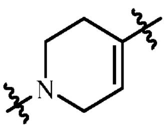, 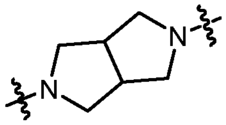, 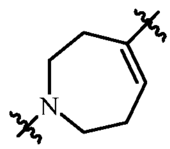, 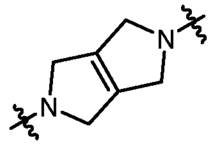,

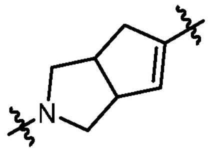, 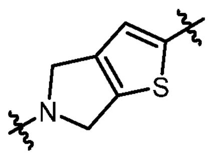, and 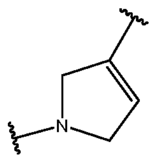;

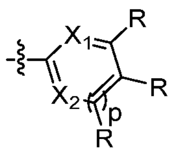

is selected from one of the following structures optionally substituted with 1-3 $R_R$:

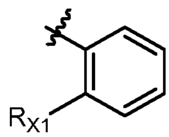, 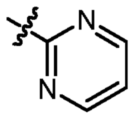, 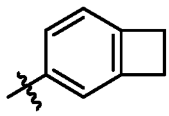, 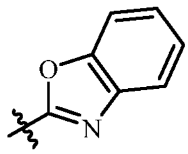, 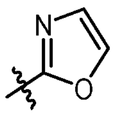, 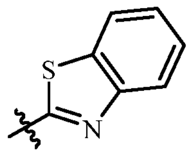,

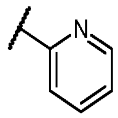, 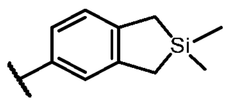, 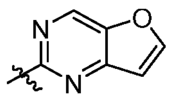, 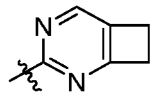, 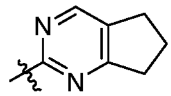,

and each $R_R$ is independently selected from F, Cl, Br, methyl, isopropyl, ethoxy, methoxy, ethynyl, propynyl, cyclopropyl, cyclobutyl, dimethylamino, and

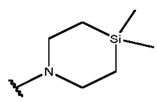;

and $R_{X1}$ is H;

or $R_{X1}$ and $R_6$, together with the atom to which they are attached, form morpholinyl, furyl, pyrrolyl, or thienyl.

Solution 20 of the present disclosure relates to a compound selected from one of the structures in Table 1, or stereoisomers or pharmaceutically acceptable salts thereof:

Table 1:

| | | | | |
|---|---|---|---|---|
| 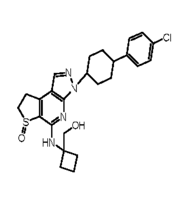 | 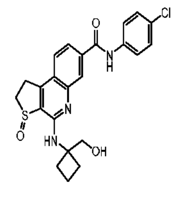 | 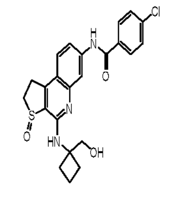 | 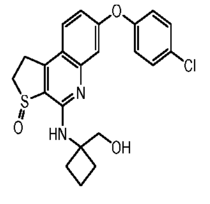 | 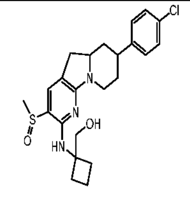 |
| 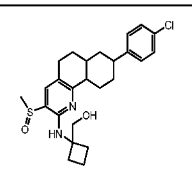 | 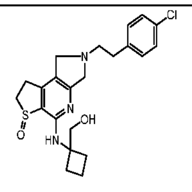 | 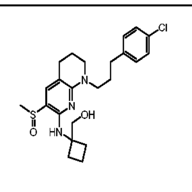 | 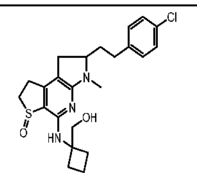 | 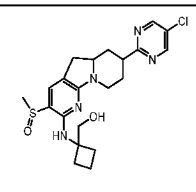 |
| 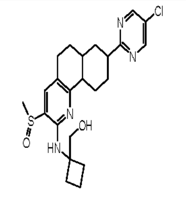 | 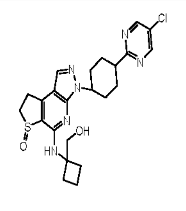 | 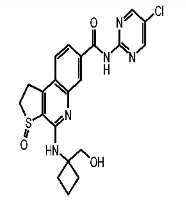 | 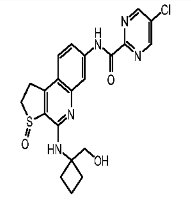 | 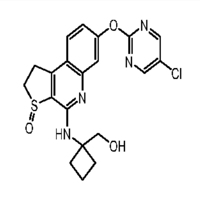 |
| 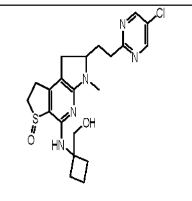 | 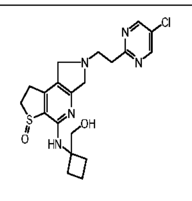 | 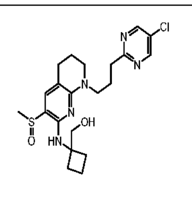 | 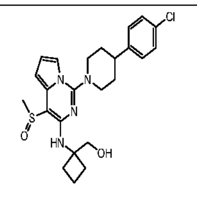 | 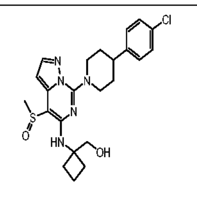 |
| 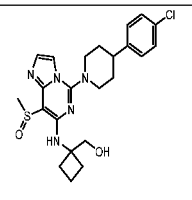 | 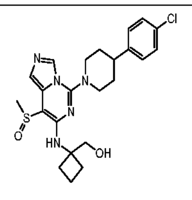 | 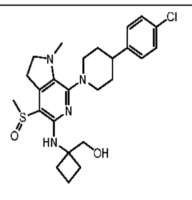 | 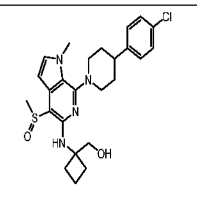 | 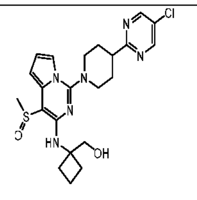 |

| | | | | |
|---|---|---|---|---|
| 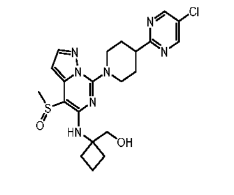 | 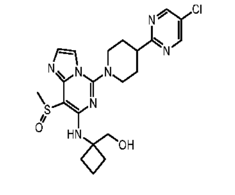 | 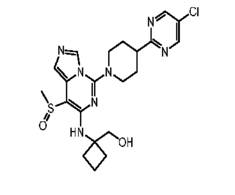 | 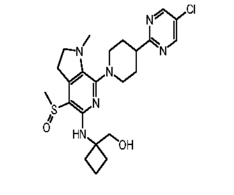 | 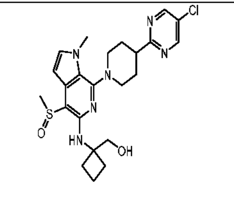 |
| 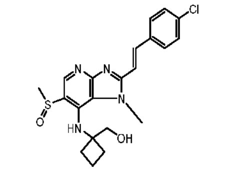 | 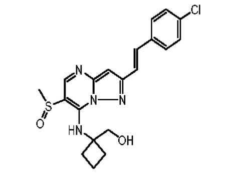 | 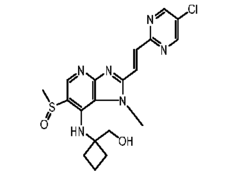 | 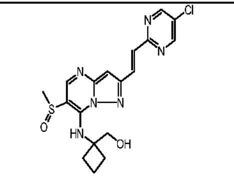 | 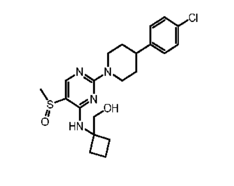 |
| 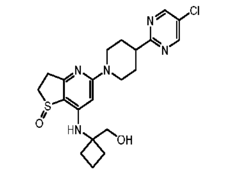 | 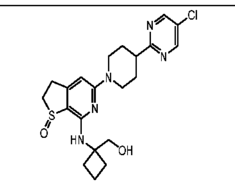 | 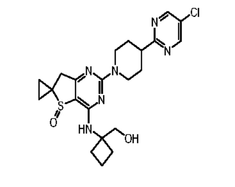 | 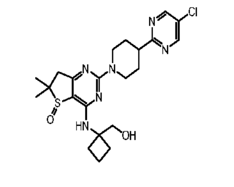 | 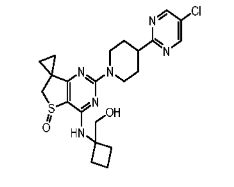 |
| 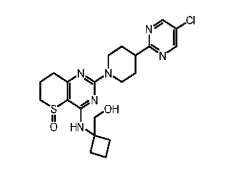 | 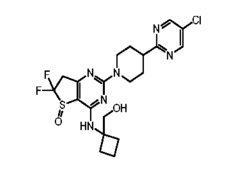 | 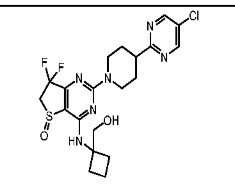 | 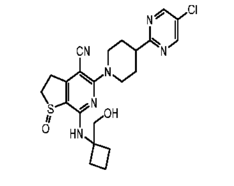 | 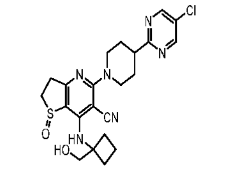 |
| 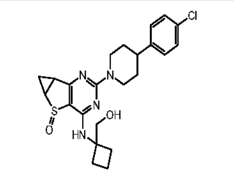 | 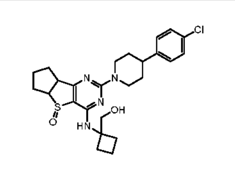 | 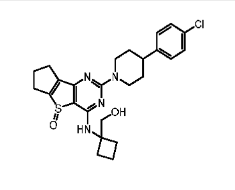 | 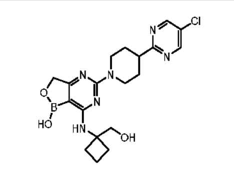 | 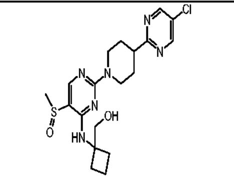 |
| 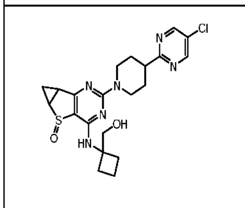 | 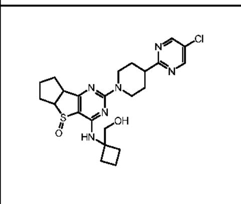 | 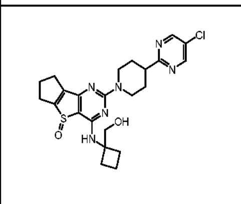 | 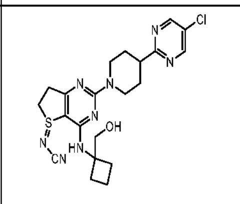 | 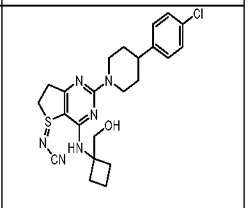 |
| 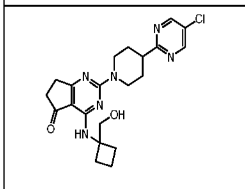 | 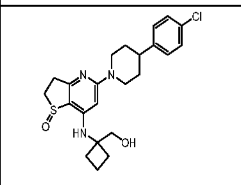 | 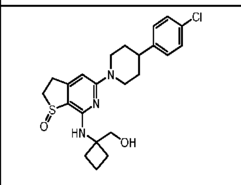 | 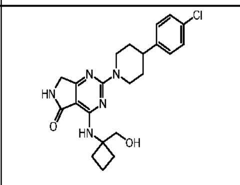 | 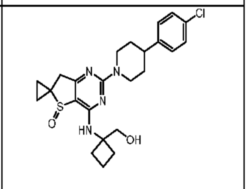 |
| 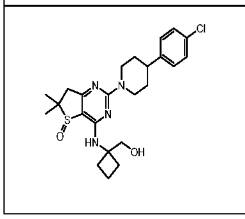 | 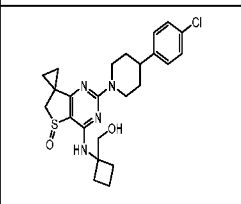 | 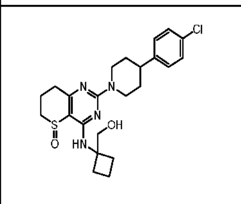 | 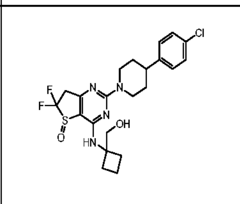 | 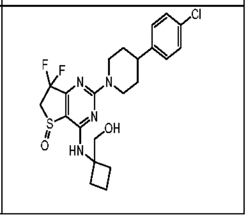 |

| | | | | |
|---|---|---|---|---|
| 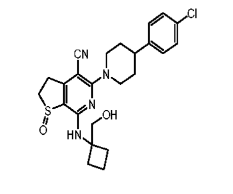 | 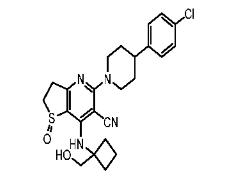 | 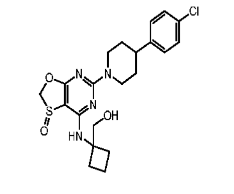 | 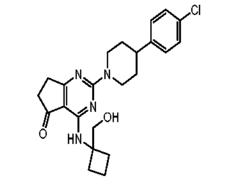 | 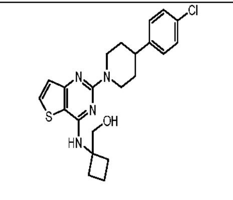 |
| 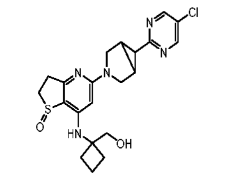 | 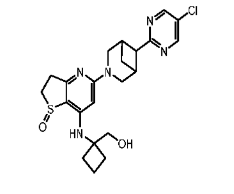 | 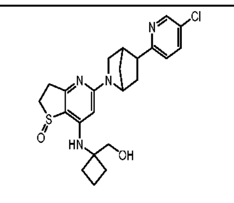 | 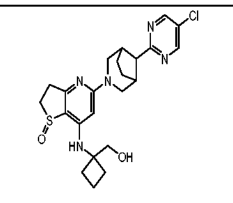 | 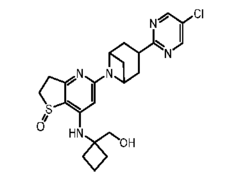 |
| 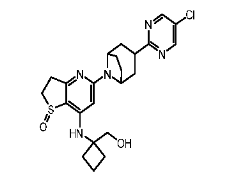 | 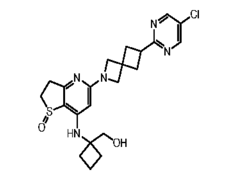 | 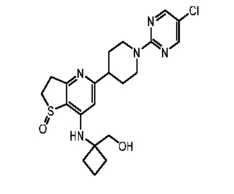 | 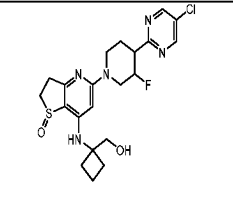 | 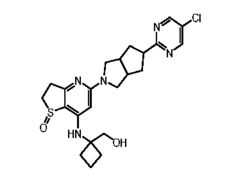 |
| 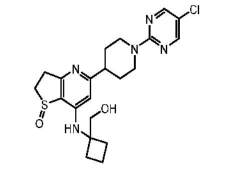 | 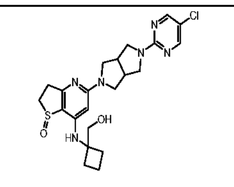 | 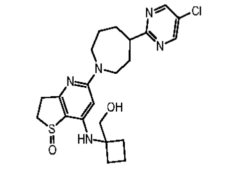 | 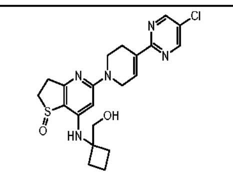 | 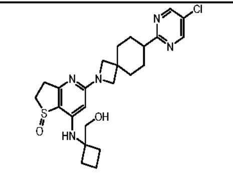 |
| 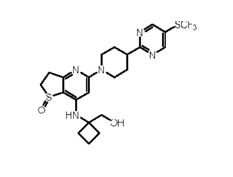 | 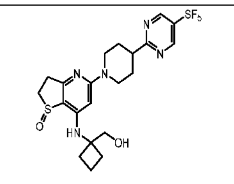 | 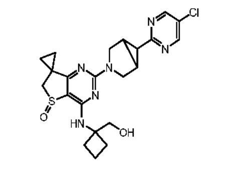 | 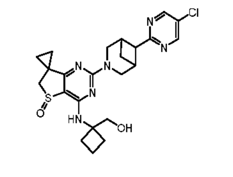 | 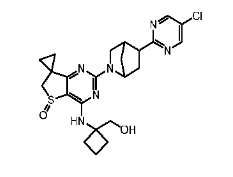 |
| 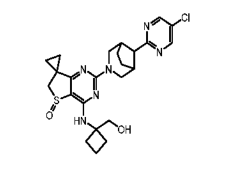 | 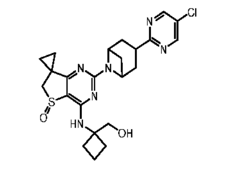 | 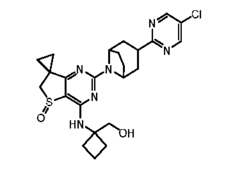 | 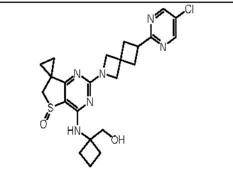 | 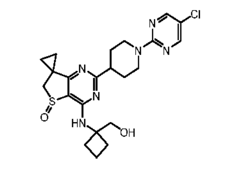 |
| 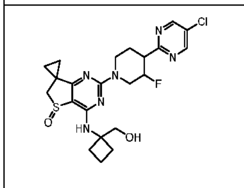 | 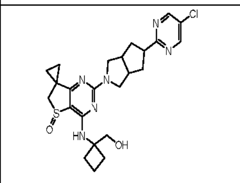 | 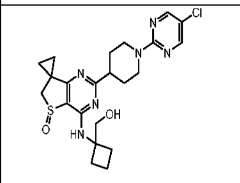 | 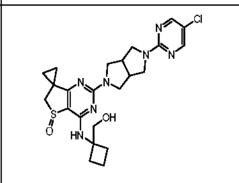 | 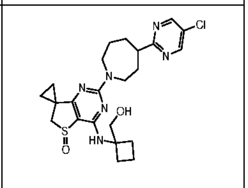 |
| 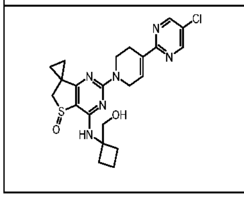 | 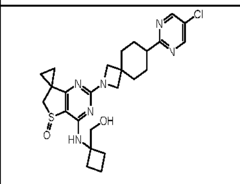 | 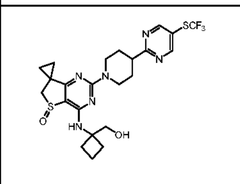 | 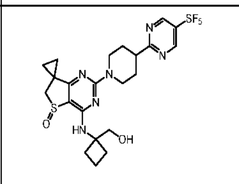 | 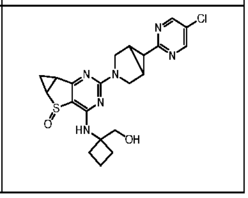 |

| | | | | |
|---|---|---|---|---|
| 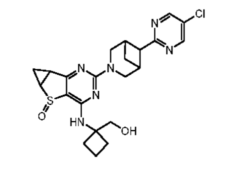 | 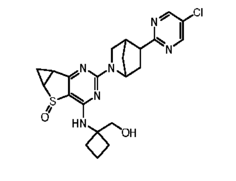 | 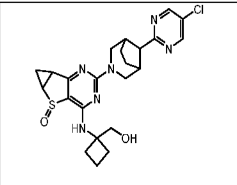 | 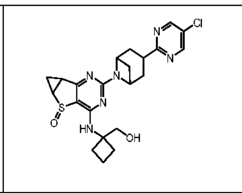 | 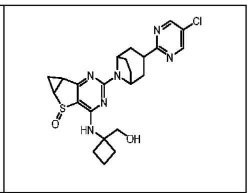 |
| 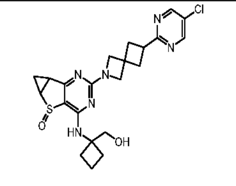 | 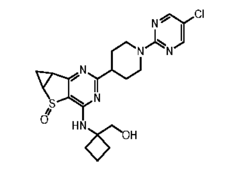 | 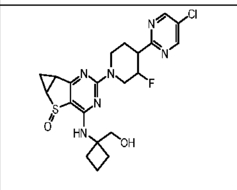 | 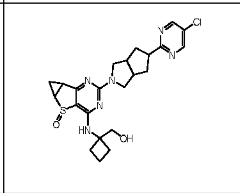 | 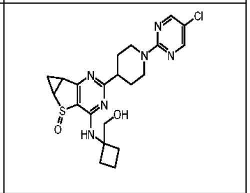 |
| 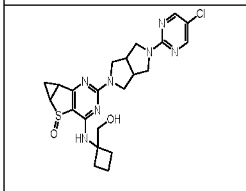 | 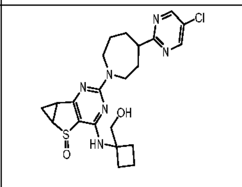 | 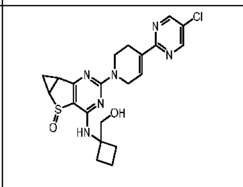 | 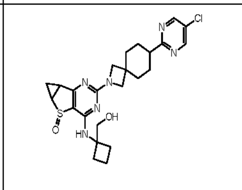 | 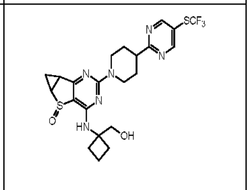 |
| 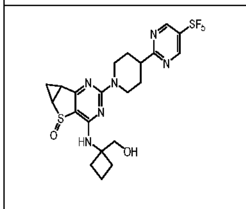 | 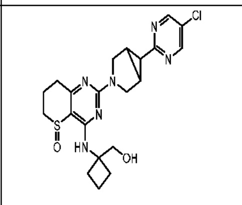 | 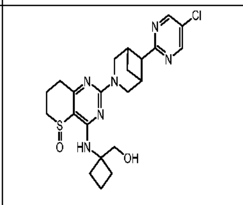 | 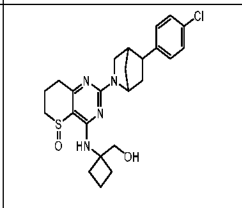 | 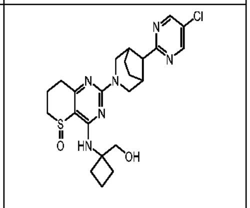 |
| 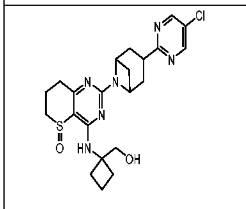 | 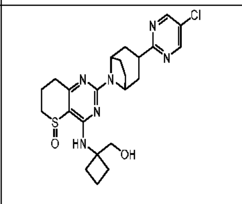 | 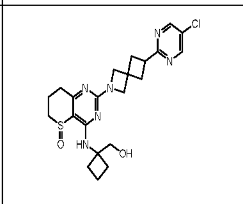 | 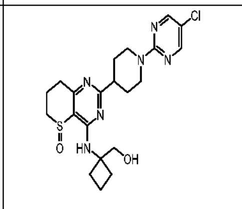 | 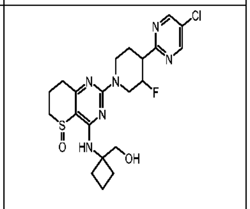 |
| 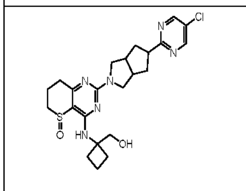 | 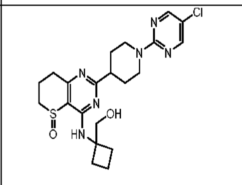 | 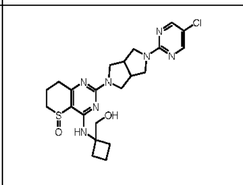 | 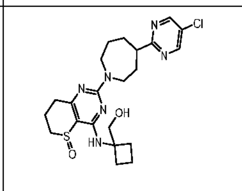 | 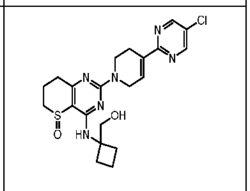 |
| 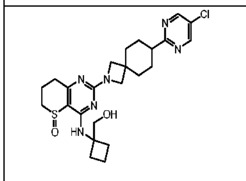 | 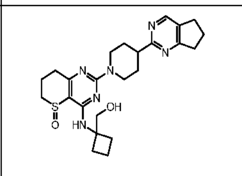 | 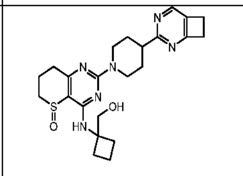 | 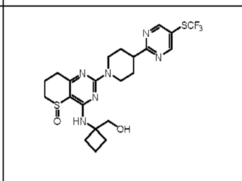 | 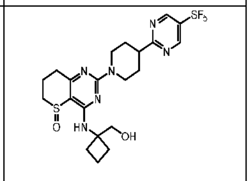 |
| 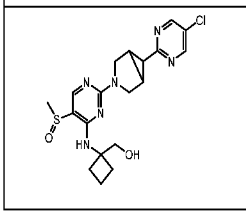 | 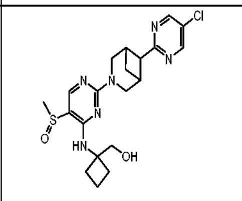 | 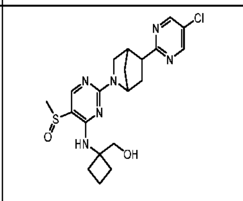 | 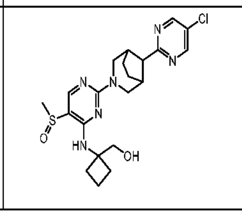 | 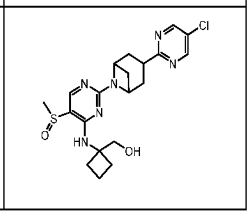 |

| | | | | |
|---|---|---|---|---|
| 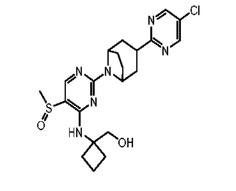 | 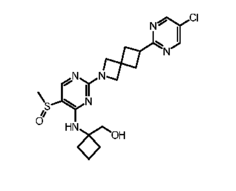 | 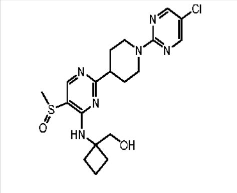 | 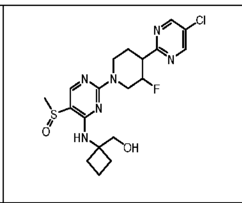 | 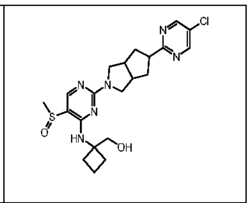 |
| 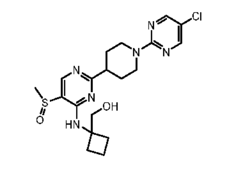 | 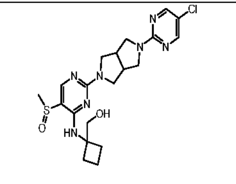 | 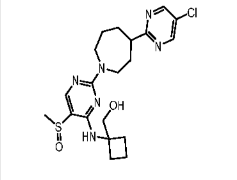 | 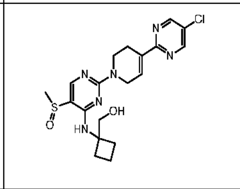 | 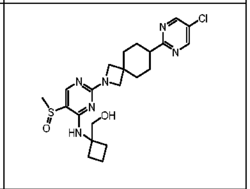 |
| 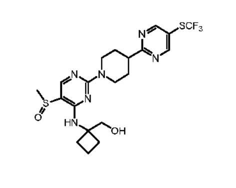 | 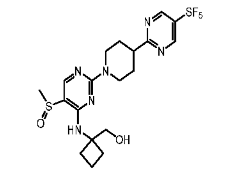 | 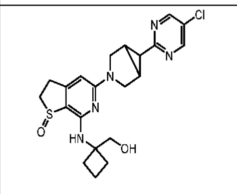 | 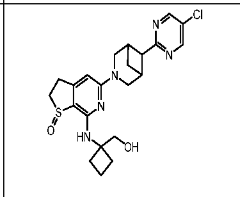 | 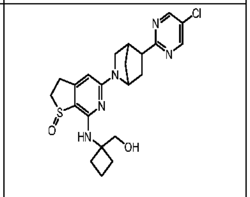 |
| 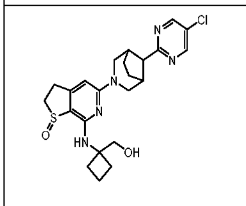 | 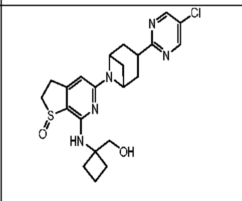 | 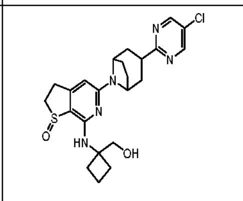 | 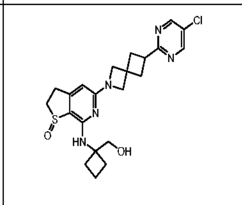 | 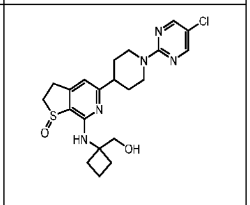 |
| 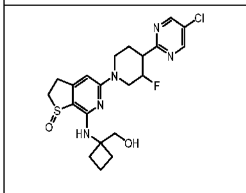 | 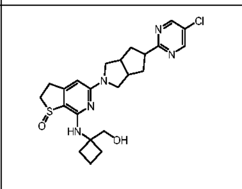 | 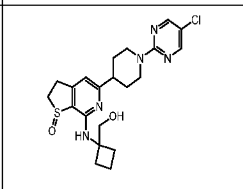 | 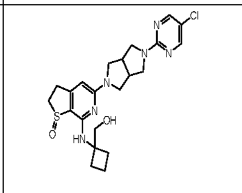 | 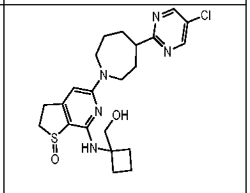 |
| 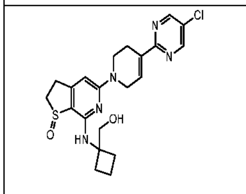 | 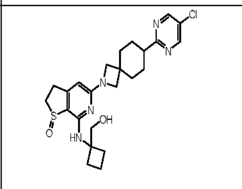 | 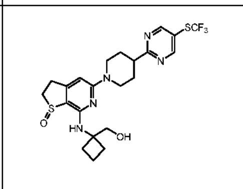 | 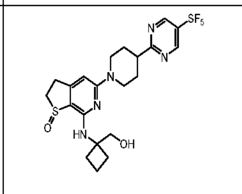 | 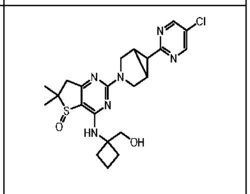 |
| 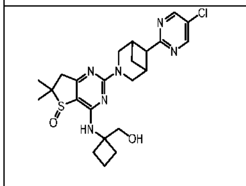 | 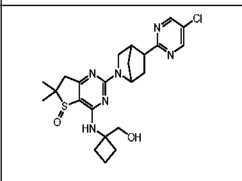 | 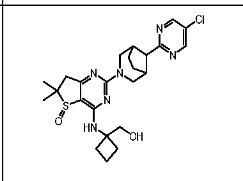 | 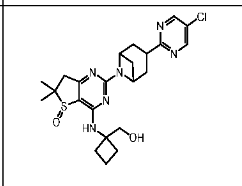 | 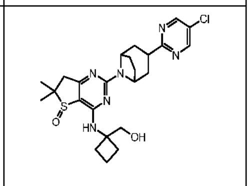 |
| 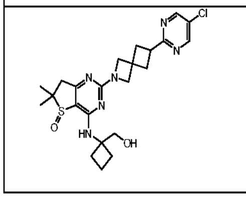 | 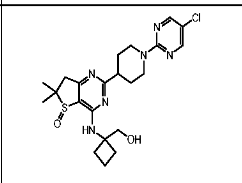 | 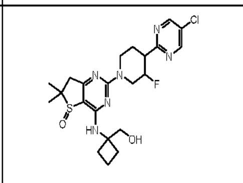 | 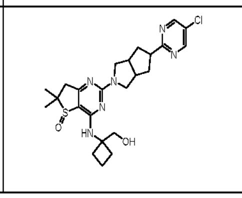 | 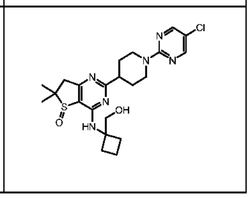 |

| | | | | |
|---|---|---|---|---|
| 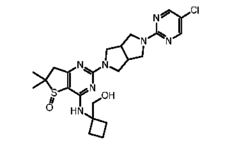 | 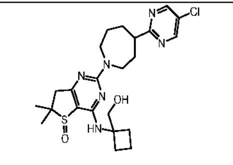 | 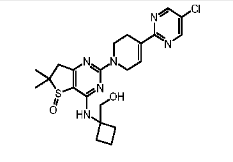 | 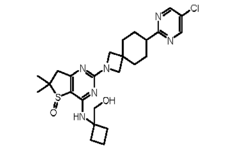 | 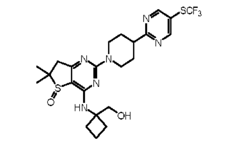 |
| 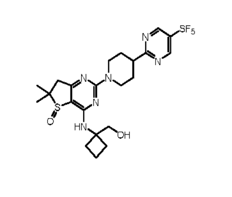 | 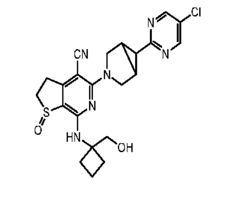 | 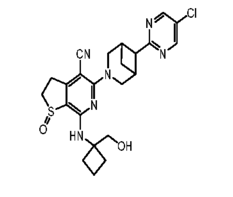 | 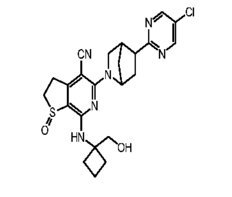 | 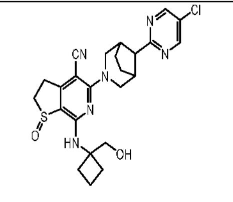 |
| 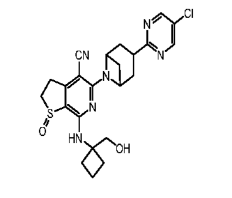 | 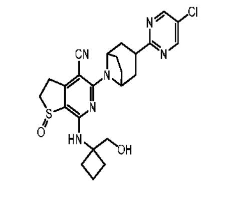 | 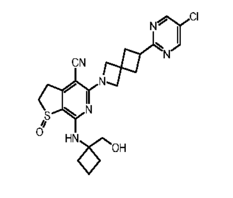 | 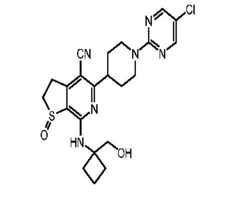 | 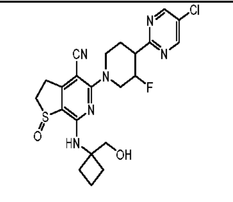 |
| 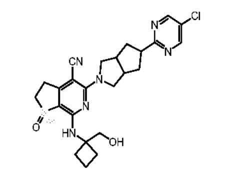 | 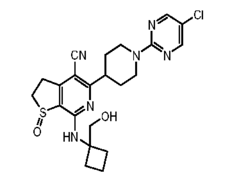 | 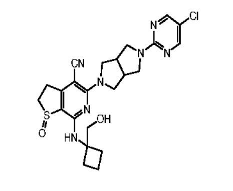 | 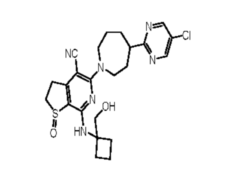 | 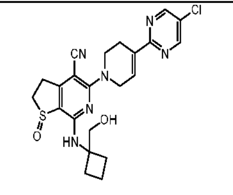 |
| 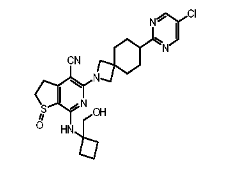 | 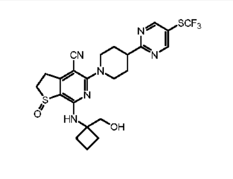 | 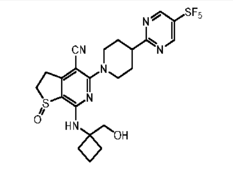 | 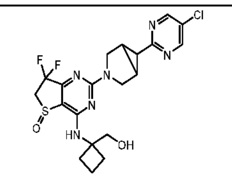 | 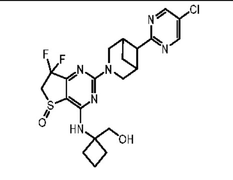 |
| 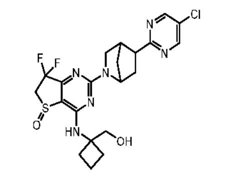 | 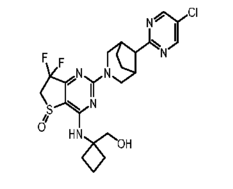 | 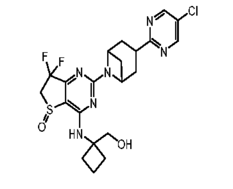 | 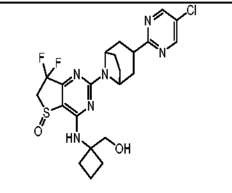 | 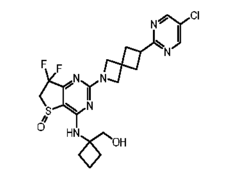 |
| 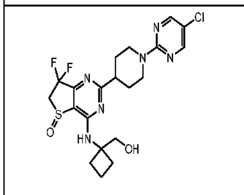 | 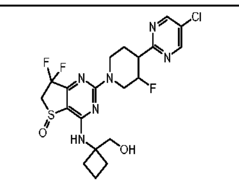 | 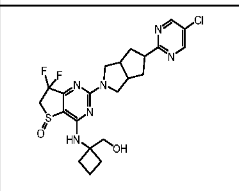 | 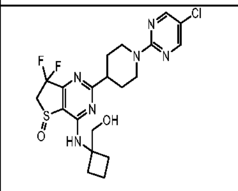 | 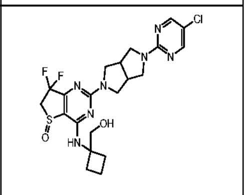 |
| 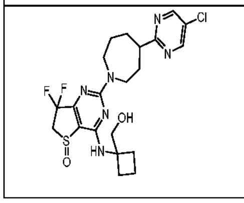 | 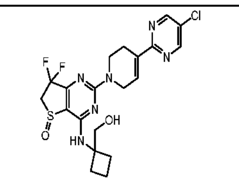 | 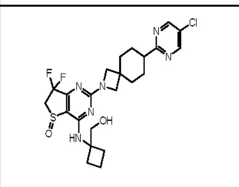 | 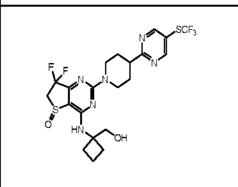 | 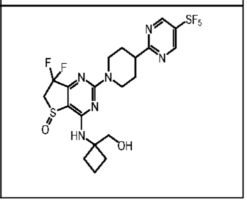 |

| | | | | |
|---|---|---|---|---|
| 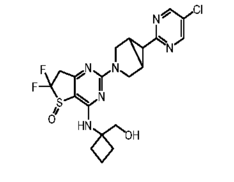 | 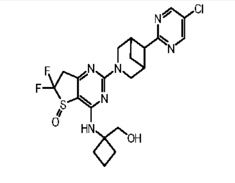 | 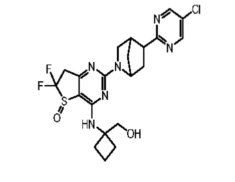 | 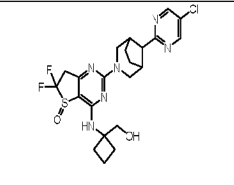 | 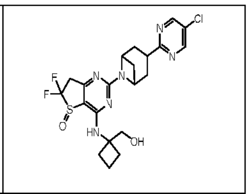 |
| 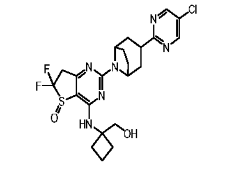 | 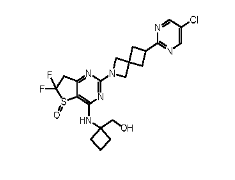 | 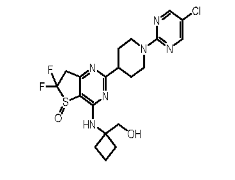 | 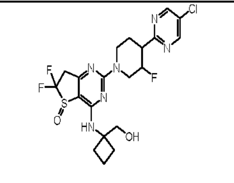 | 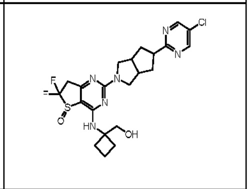 |
| 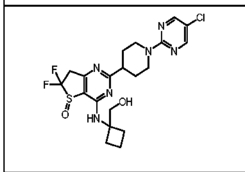 | 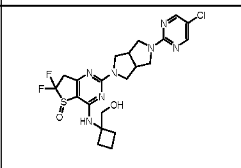 | 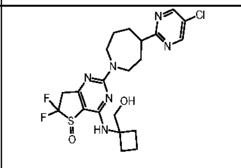 | 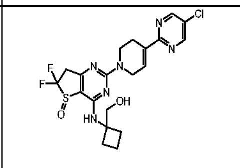 | 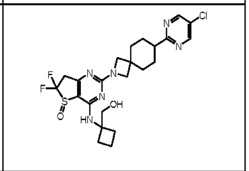 |
| 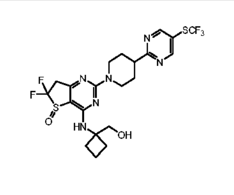 | 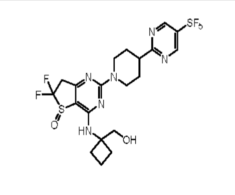 | 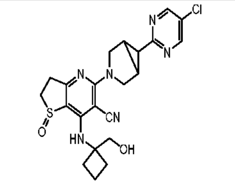 | 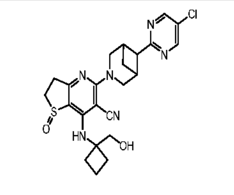 | 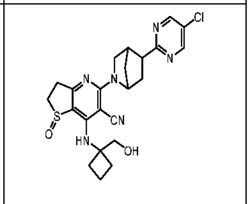 |
| 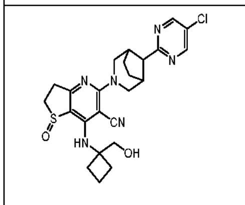 | 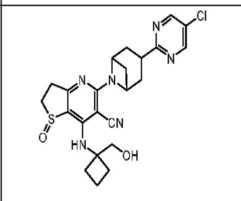 | 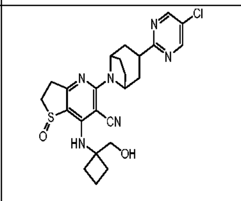 | 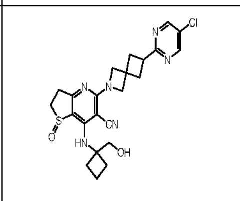 | 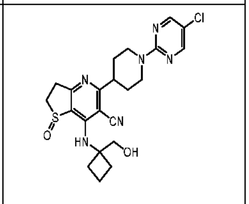 |
| 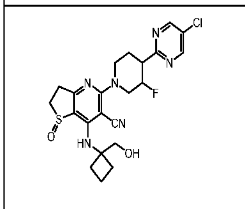 | 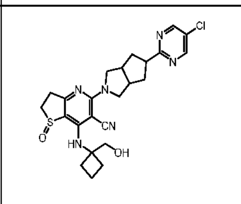 | 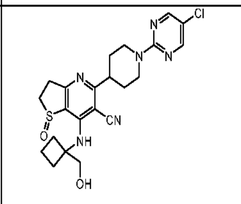 | 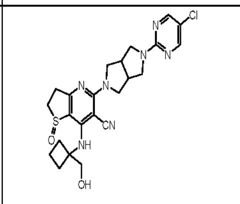 | 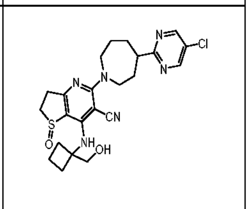 |
| 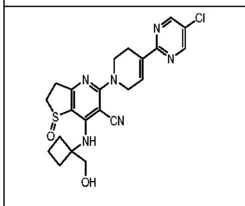 | 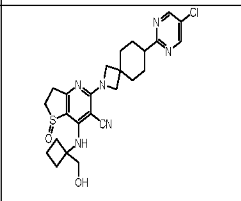 | 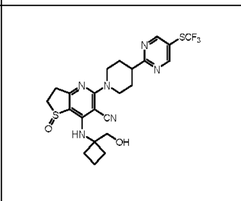 | 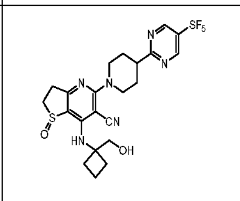 | 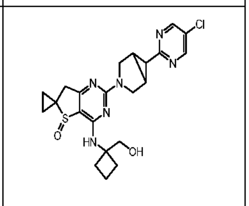 |
| 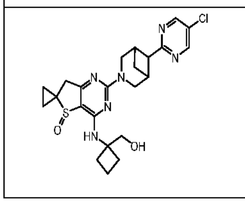 | 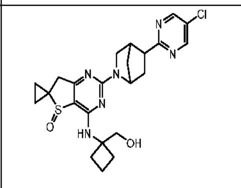 | 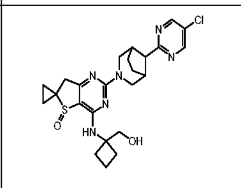 | 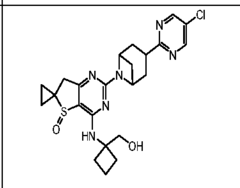 | 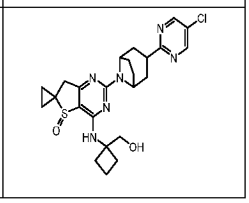 |

| | | | | |
|---|---|---|---|---|
| 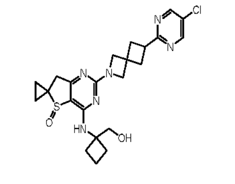 | 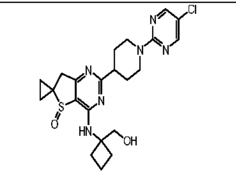 | 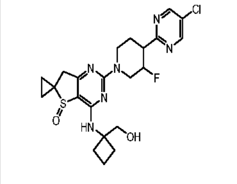 | 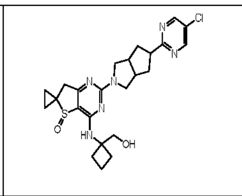 | 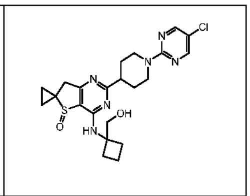 |
| 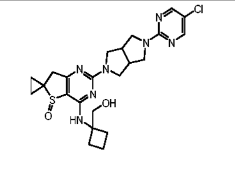 | 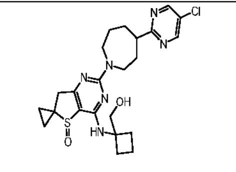 | 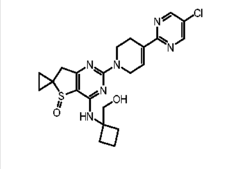 | 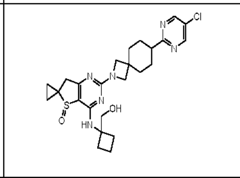 | 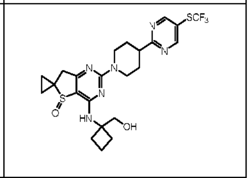 |
| 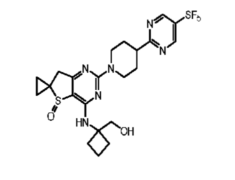 | 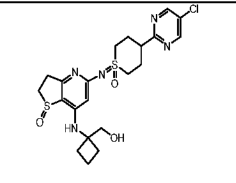 | 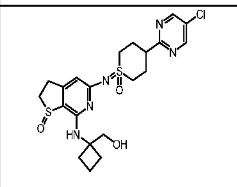 | 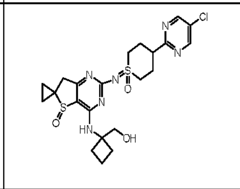 | 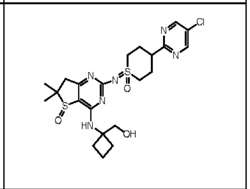 |
| 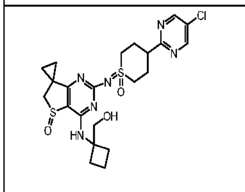 | 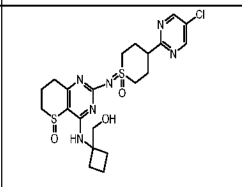 | 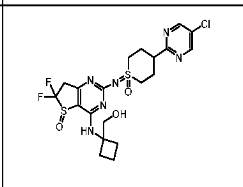 | 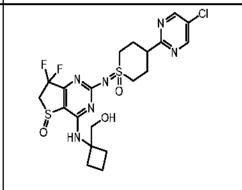 | 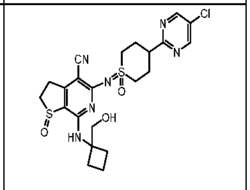 |
| 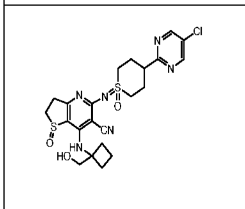 | 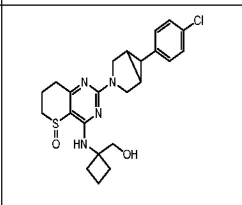 | 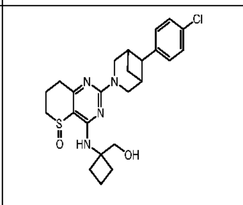 | 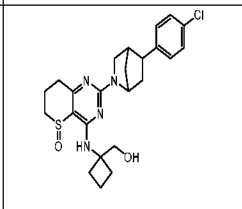 | 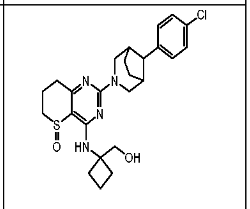 |
| 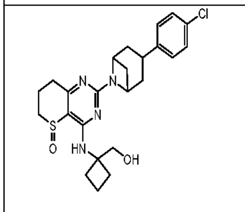 | 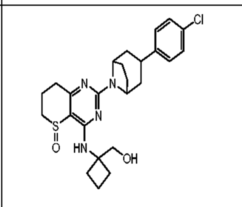 | 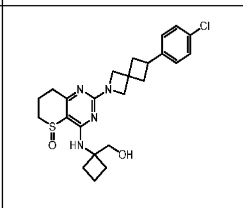 | 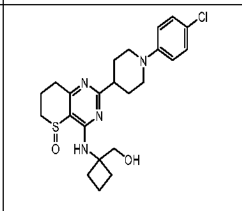 | 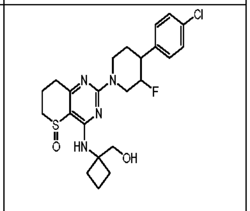 |
| 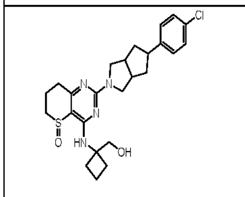 | 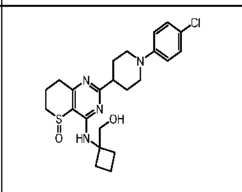 | 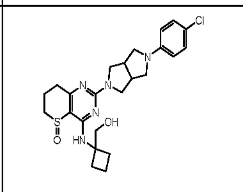 | 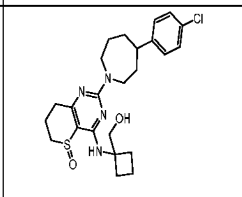 | 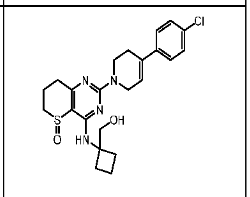 |
| 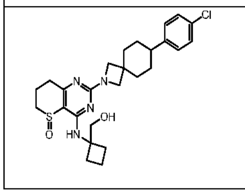 | 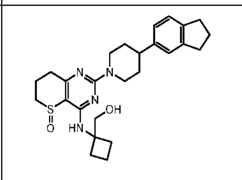 | 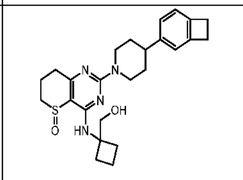 | 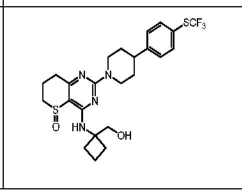 | 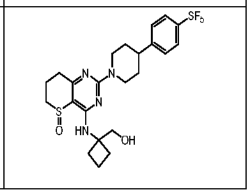 |

| | | | | |
|---|---|---|---|---|
| 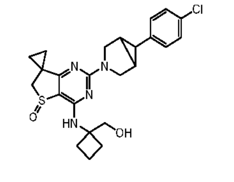 | 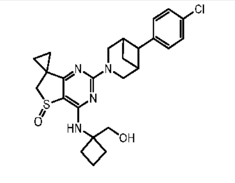 | 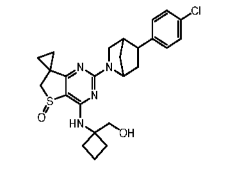 | 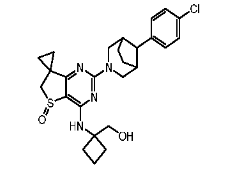 | 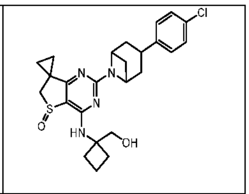 |
| 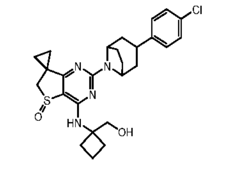 | 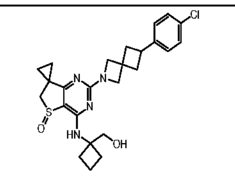 | 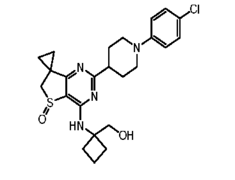 | 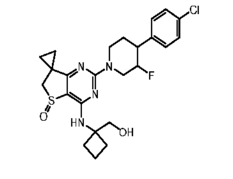 | 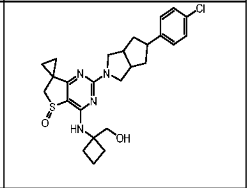 |
| 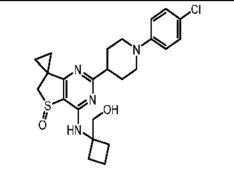 | 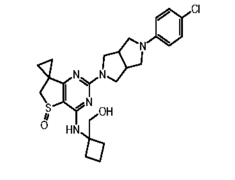 | 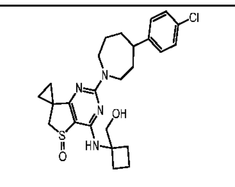 | 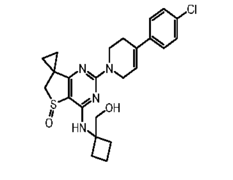 | 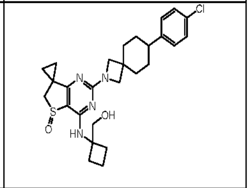 |
| 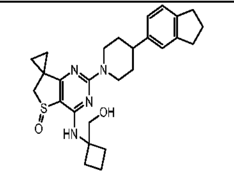 | 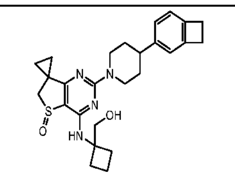 | 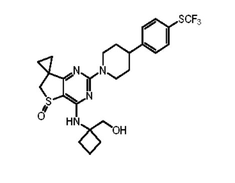 | 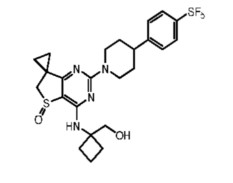 | 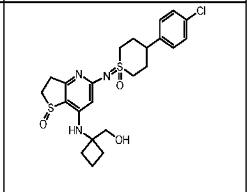 |
| 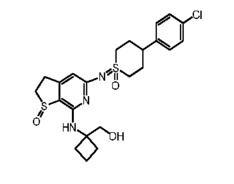 | 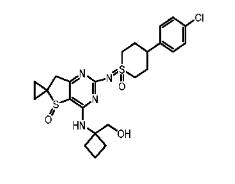 | 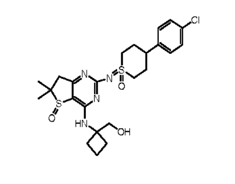 | 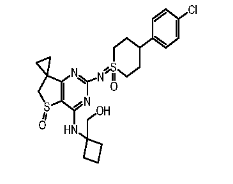 | 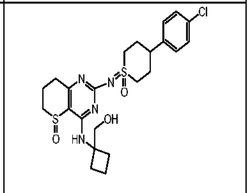 |
| 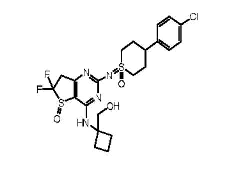 | 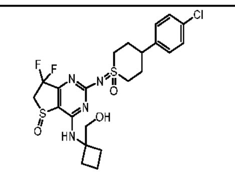 | 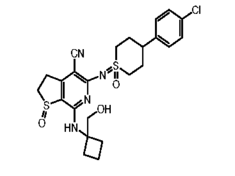 | 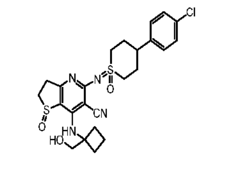 | 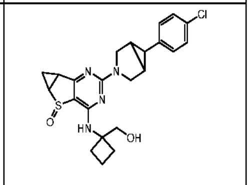 |
| 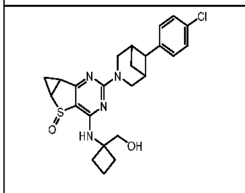 | 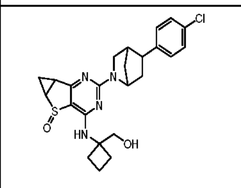 | 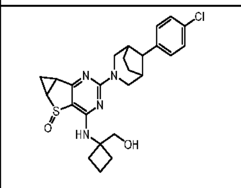 | 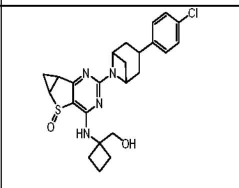 | 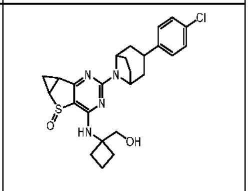 |
| 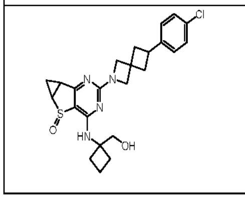 | 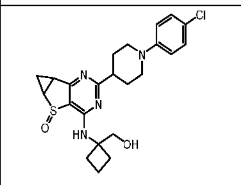 | 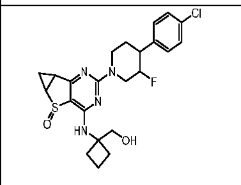 | 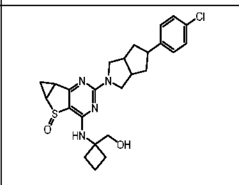 | 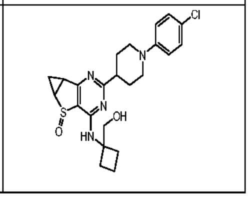 |

| | | | | |
|---|---|---|---|---|
| 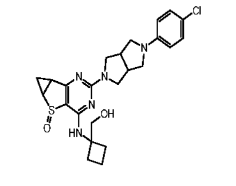 | 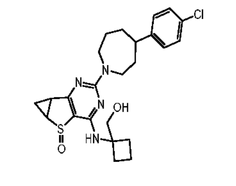 | 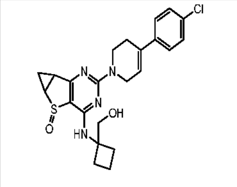 | 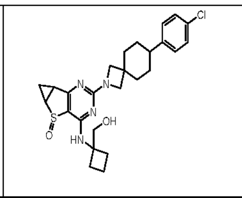 | 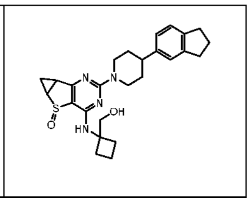 |
| 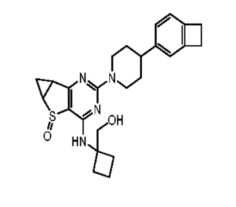 | 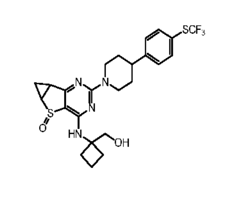 | 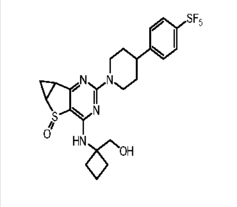 | 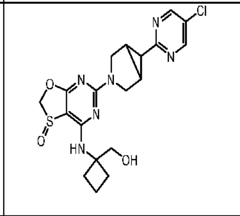 | 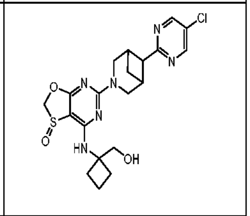 |
| 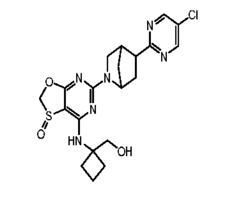 | 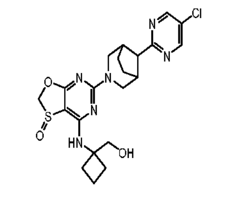 | 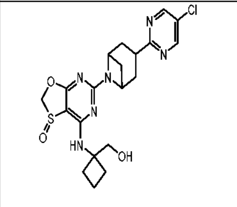 | 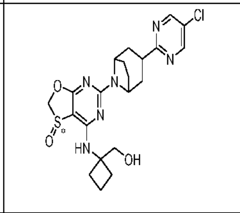 | 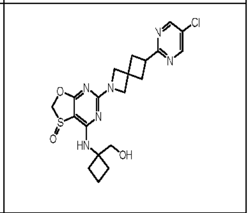 |
| 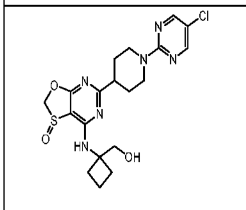 | 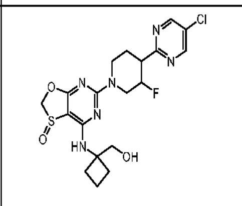 | 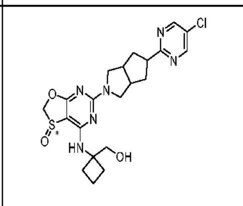 | 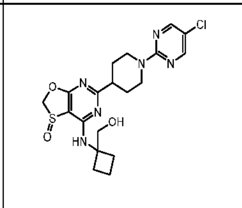 | 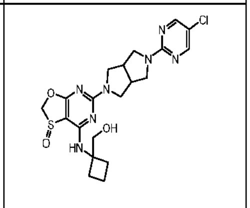 |
| 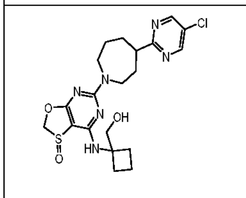 | 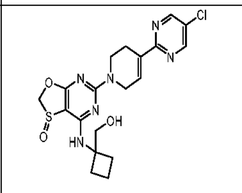 | 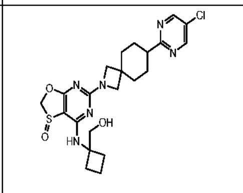 | 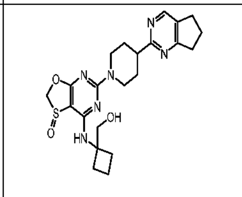 | 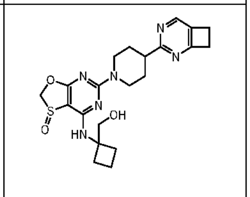 |
| 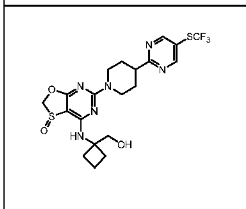 | 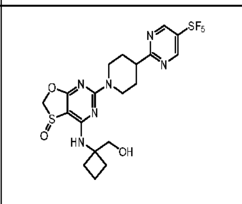 | 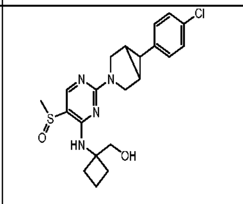 | 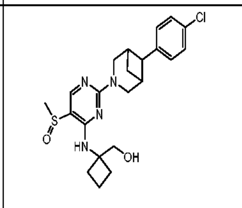 | 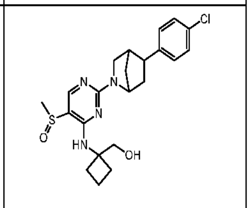 |
| 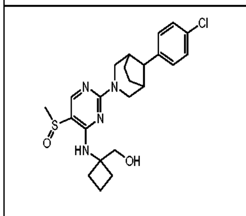 | 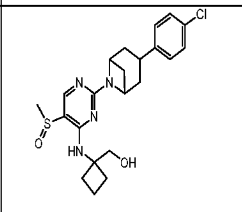 | 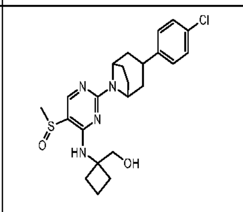 | 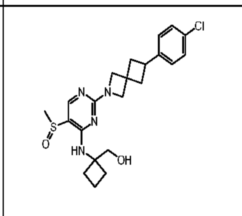 | 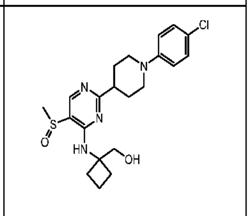 |
| 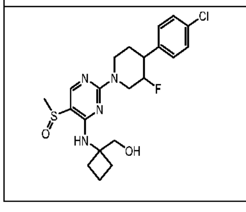 | 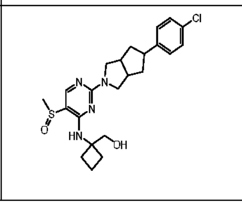 | 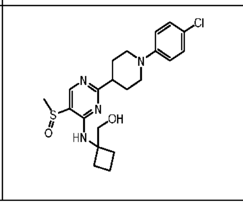 | 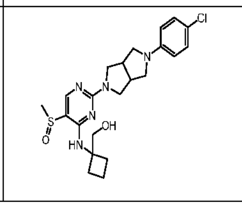 | 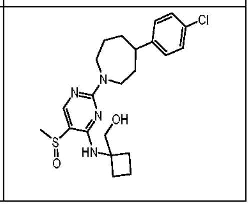 |

| | | | | |
|---|---|---|---|---|
| 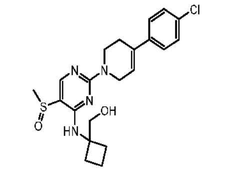 | 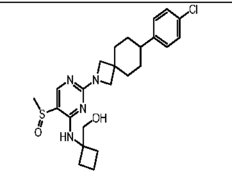 | 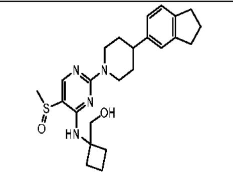 | 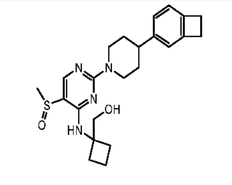 | 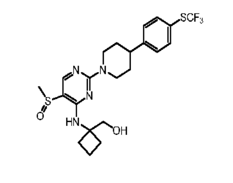 |
| 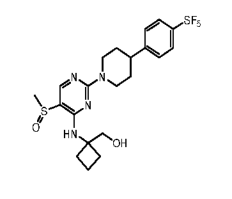 | 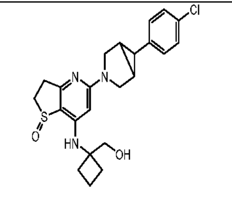 | 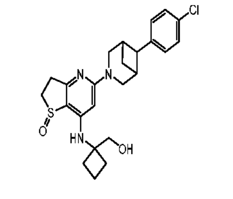 | 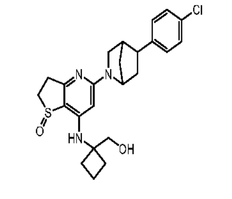 | 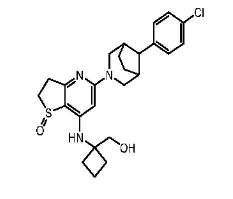 |
| 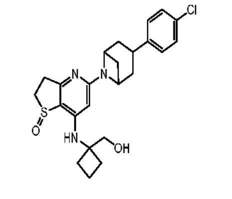 | 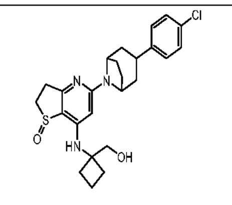 | 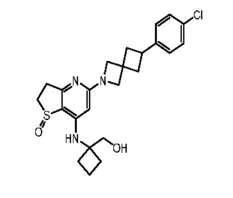 | 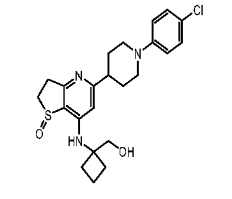 | 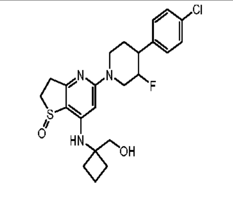 |
| 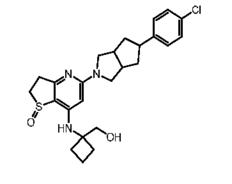 | 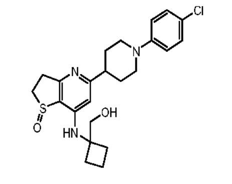 | 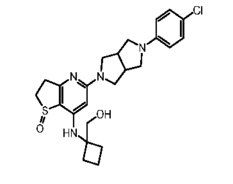 | 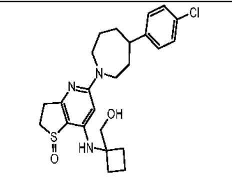 | 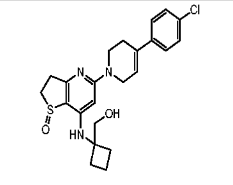 |
| 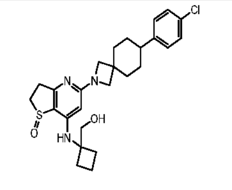 | 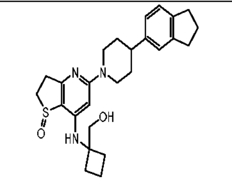 | 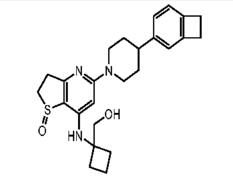 | 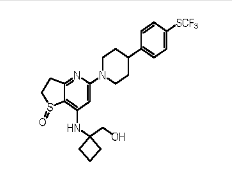 | 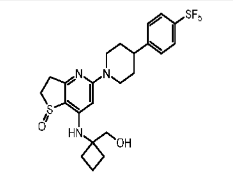 |
| 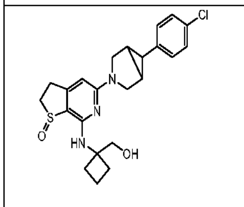 | 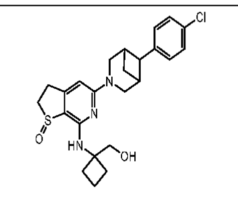 | 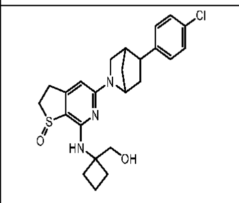 | 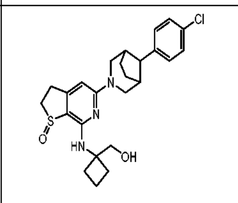 | 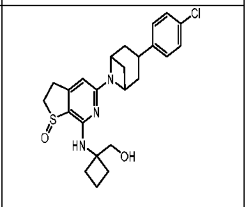 |
| 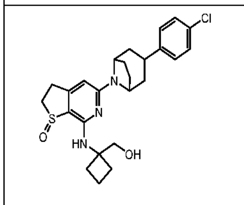 | 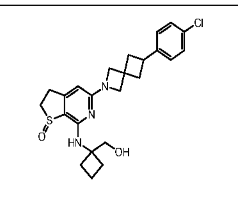 | 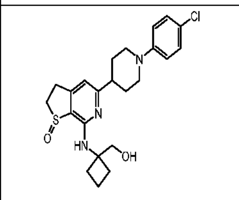 | 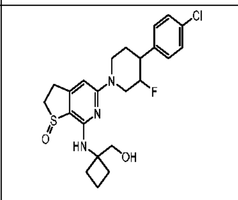 | 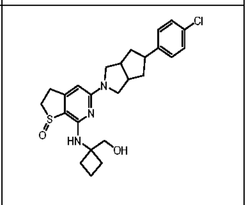 |
| 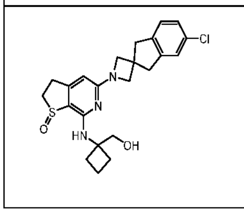 | 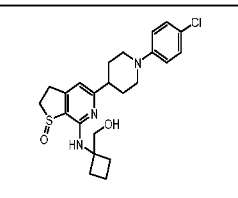 | 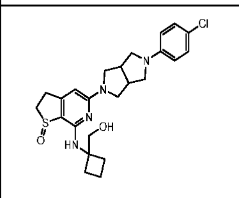 | 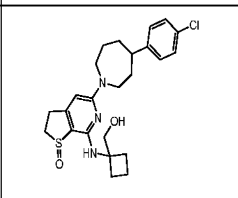 | 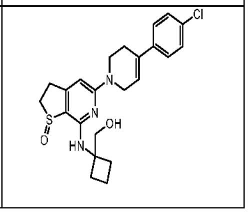 |

| | | | | |
|---|---|---|---|---|
| 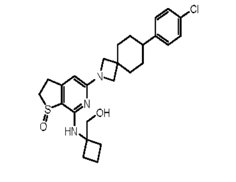 | 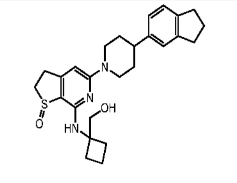 | 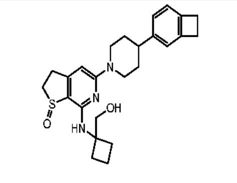 | 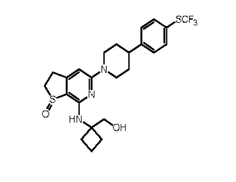 | 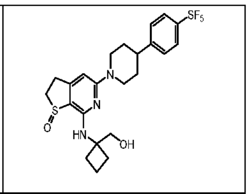 |
| 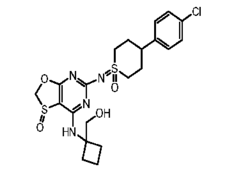 | 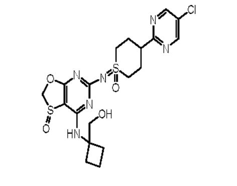 | 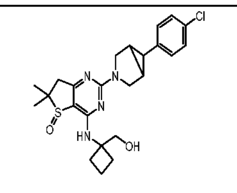 | 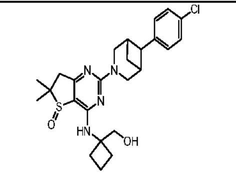 | 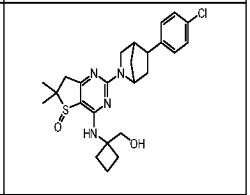 |
| 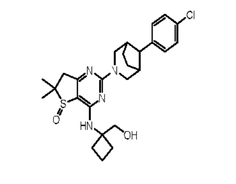 | 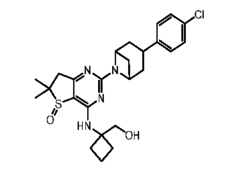 | 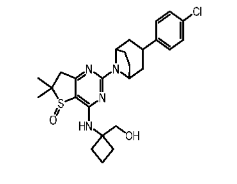 | 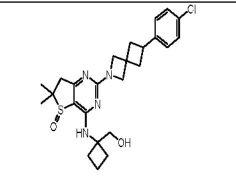 | 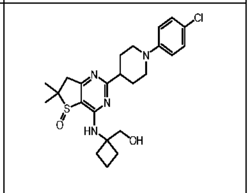 |
| 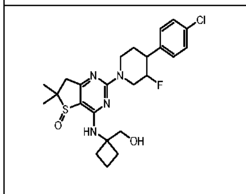 | 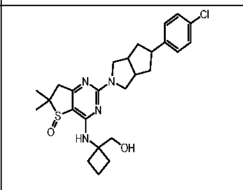 | 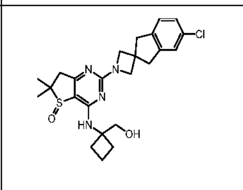 | 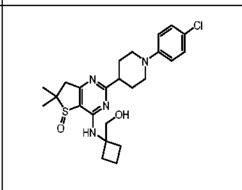 | 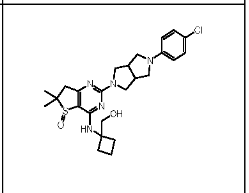 |
| 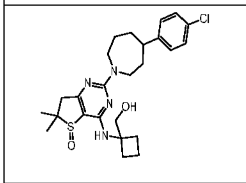 | 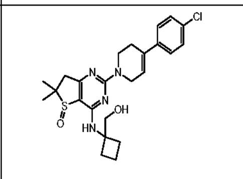 | 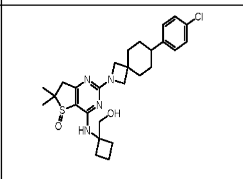 | 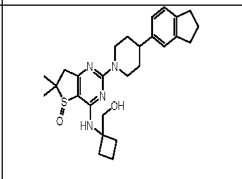 | 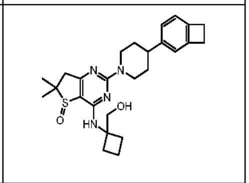 |
| 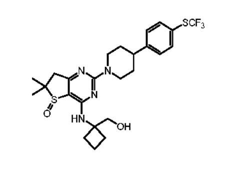 | 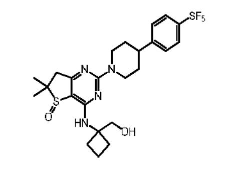 | 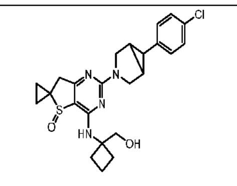 | 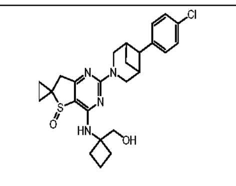 | 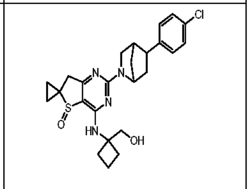 |
| 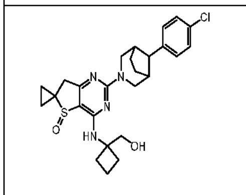 | 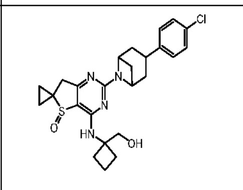 | 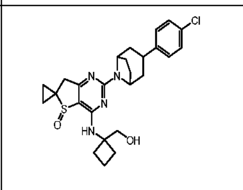 | 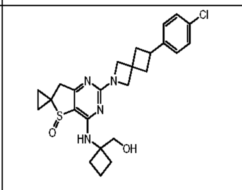 | 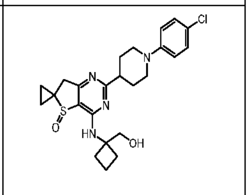 |
| 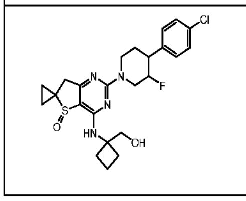 | 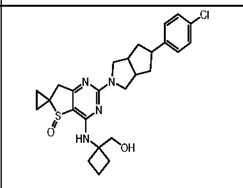 | 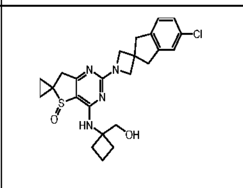 | 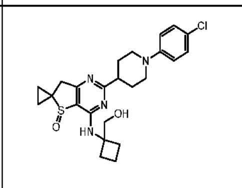 | 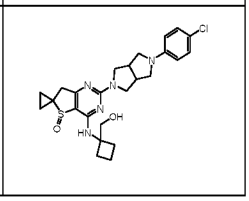 |

| | | | | |
|---|---|---|---|---|
| 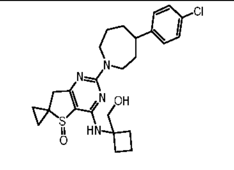 | 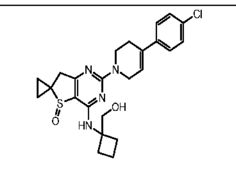 | 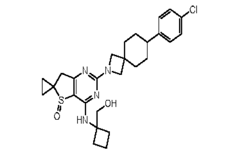 | 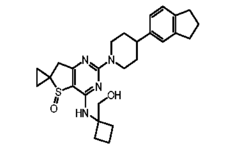 | 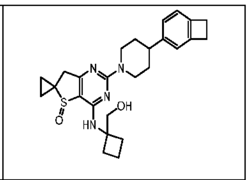 |
| 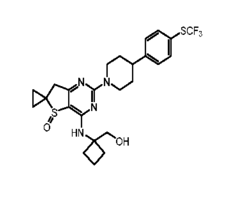 | 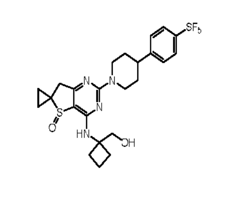 | 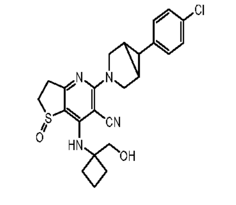 | 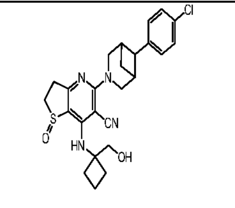 | 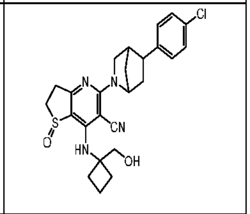 |
| 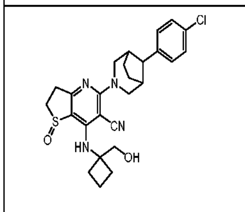 | 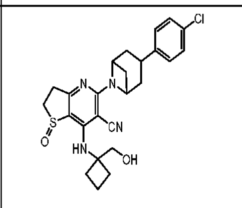 | 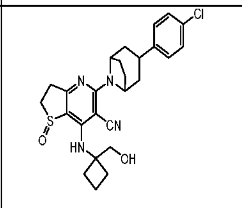 | 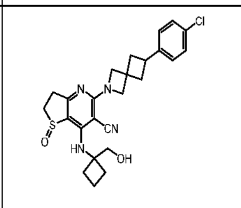 | 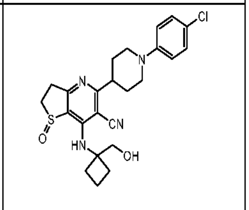 |
| 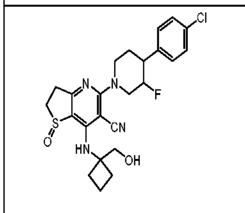 | 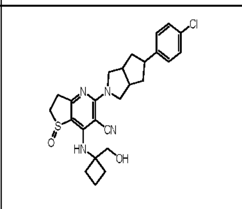 | 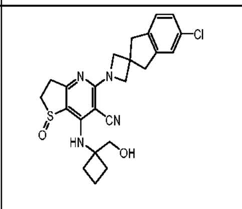 | 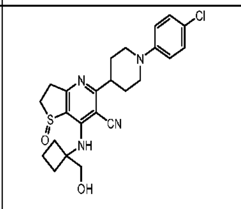 | 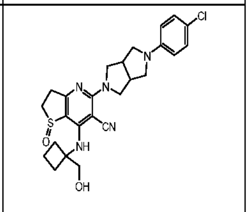 |
| 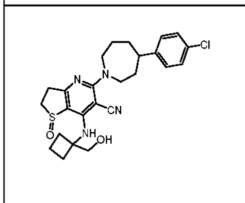 | 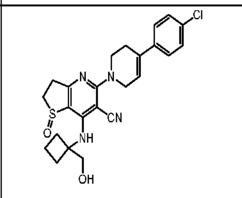 | 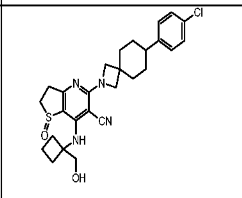 | 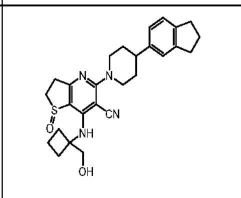 | 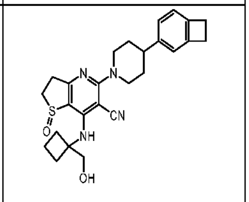 |
| 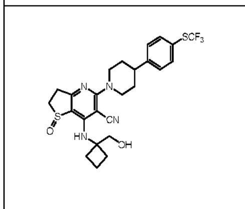 | 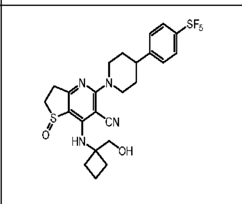 | 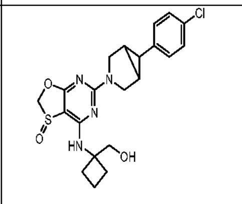 | 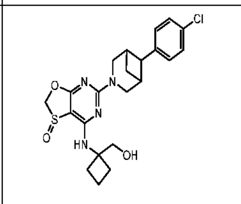 | 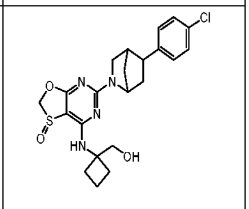 |
| 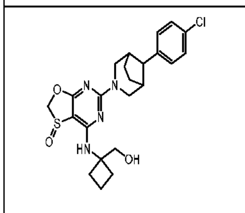 | 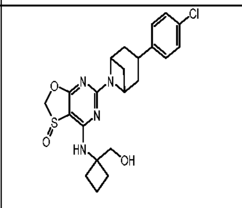 | 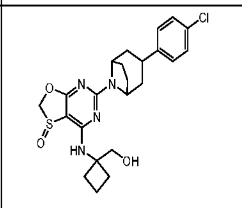 | 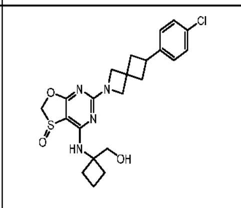 | 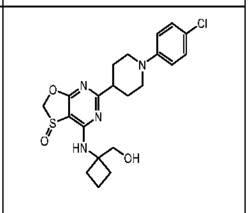 |
| 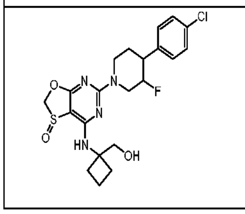 | 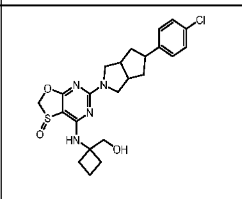 | 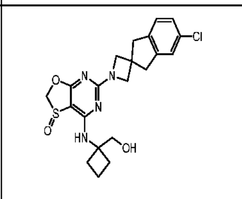 | 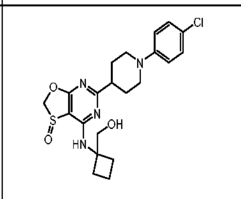 | 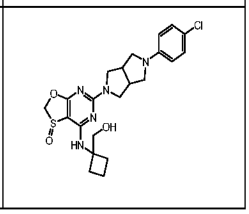 |

| | | | | |
|---|---|---|---|---|
| 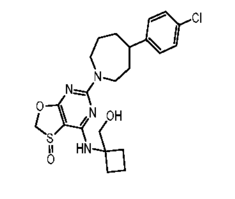 | 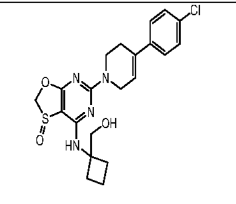 | 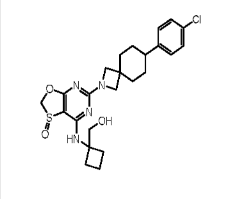 | 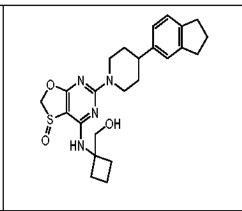 | 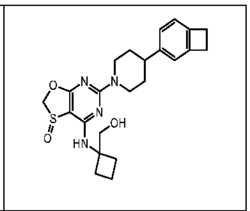 |
| 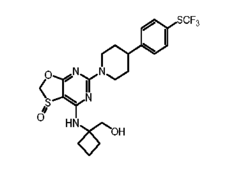 | 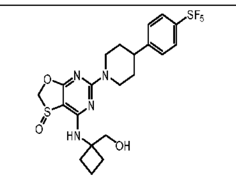 | 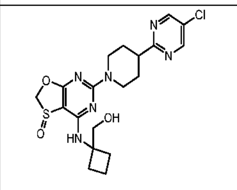 | 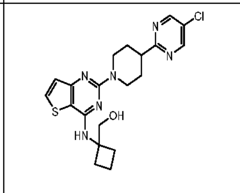 | 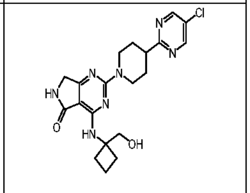 |
| 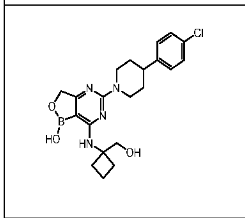 | 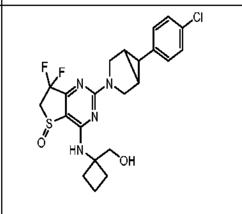 | 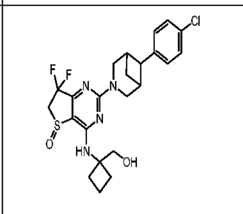 | 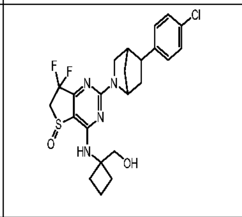 | 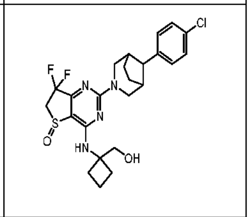 |
| 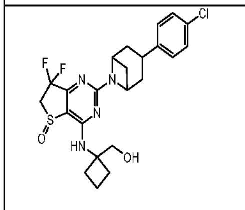 | 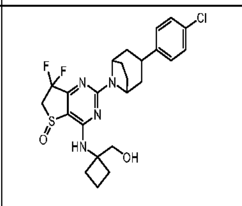 | 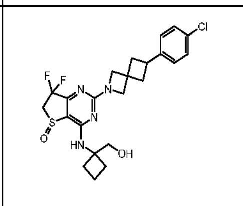 | 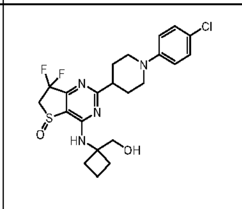 | 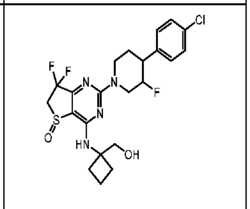 |
| 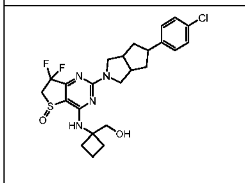 | 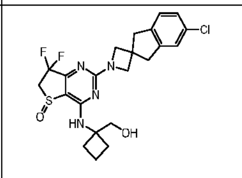 | 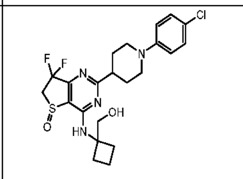 | 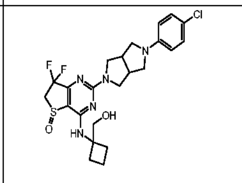 | 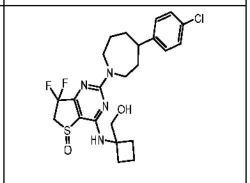 |
| 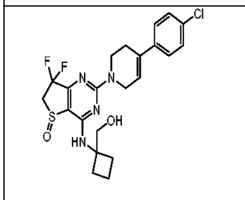 | 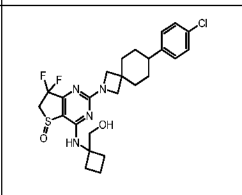 | 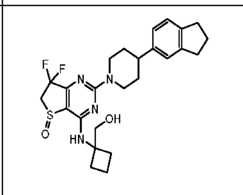 | 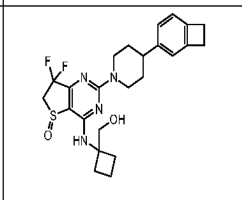 | 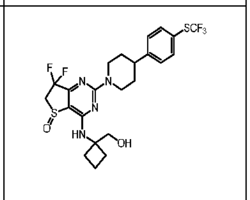 |
| 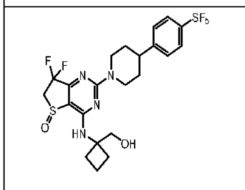 | 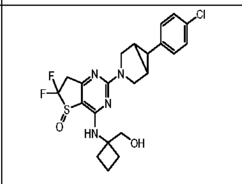 | 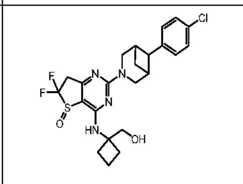 | 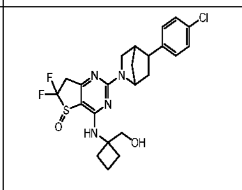 | 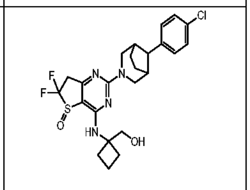 |
| 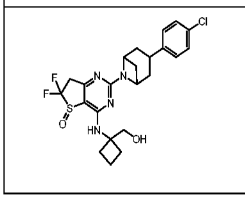 | 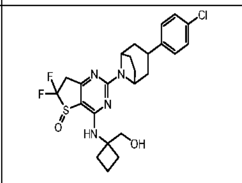 | 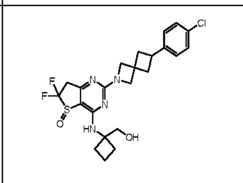 | 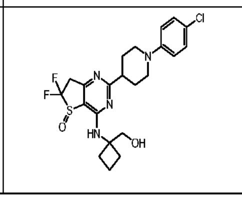 | 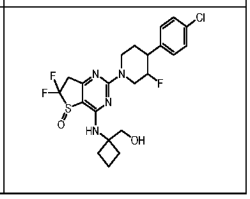 |

| | | | | |
|---|---|---|---|---|
| 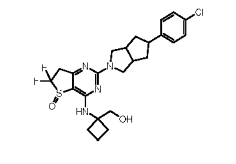 | 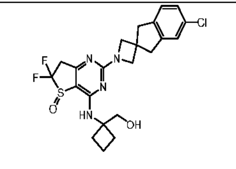 | 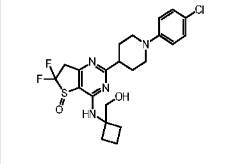 | 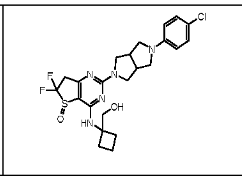 | 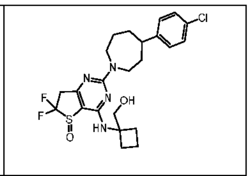 |
| 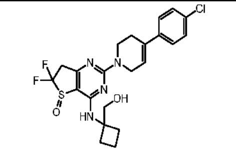 | 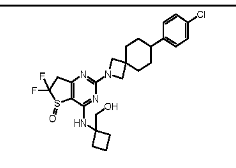 | 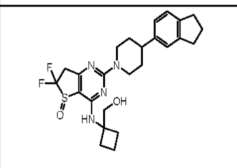 | 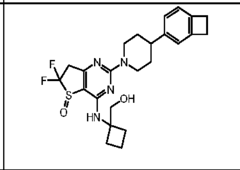 | 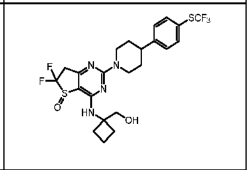 |
| 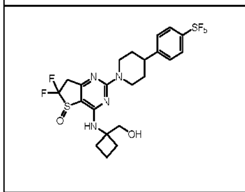 | 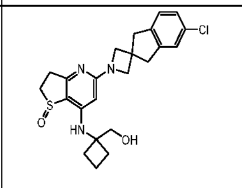 | 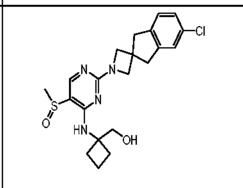 | 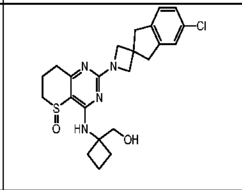 | 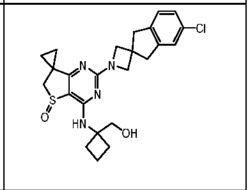 |
| 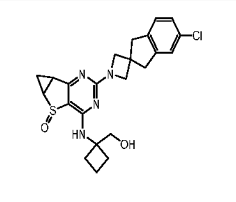 | 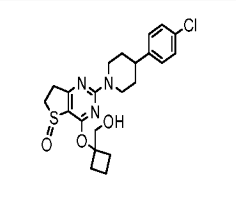 | 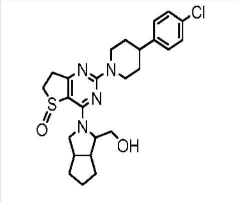 | 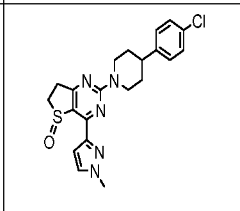 | 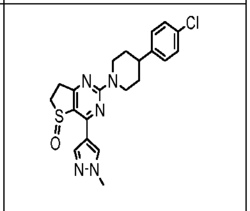 |
| 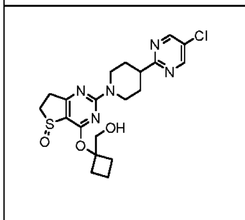 | 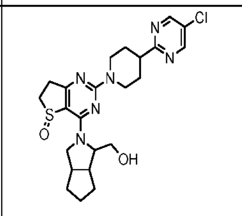 | 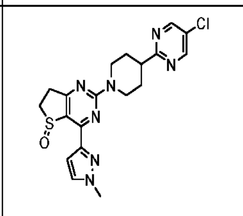 | 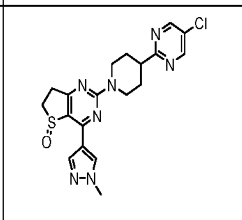 | 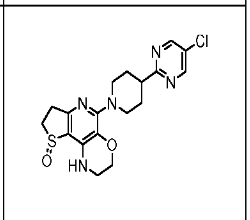 |
| 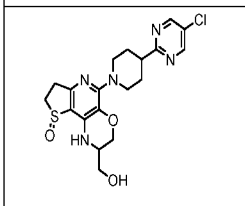 | 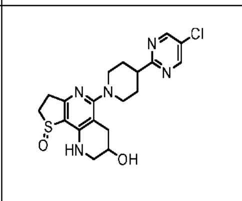 | 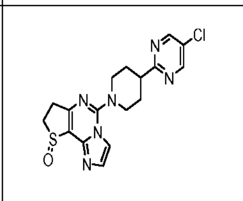 | 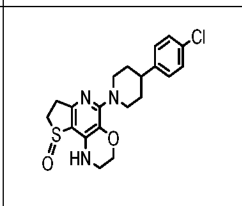 | 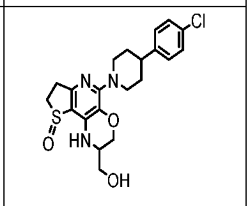 |
| 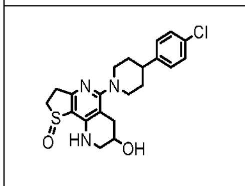 | 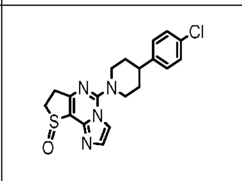 | 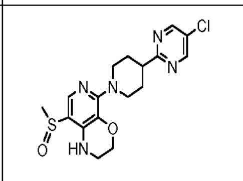 | 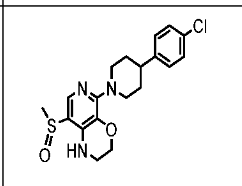 | 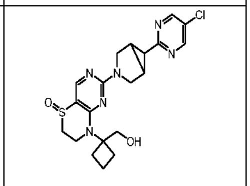 |
| 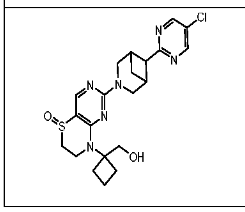 | 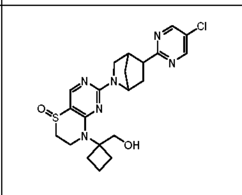 | 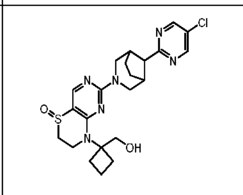 | 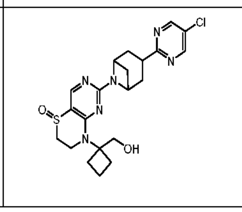 | 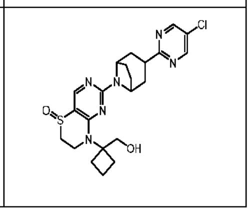 |

| | | | | |
|---|---|---|---|---|
| 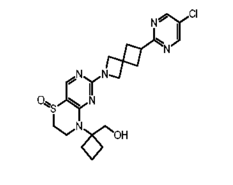 | 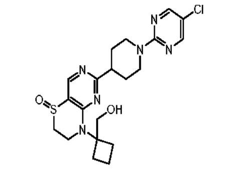 | 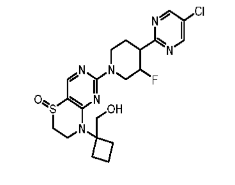 | 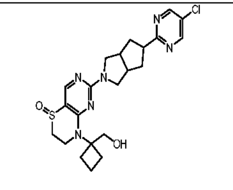 | 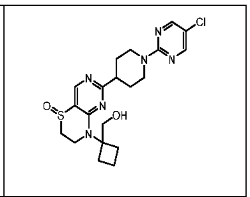 |
| 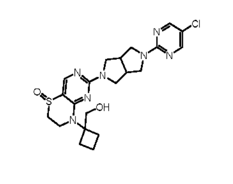 | 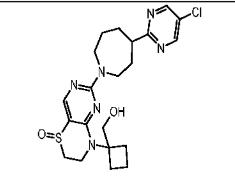 | 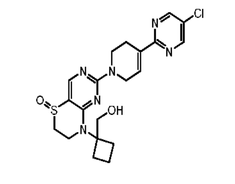 | 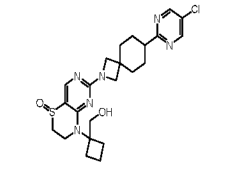 | 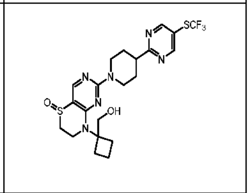 |
| 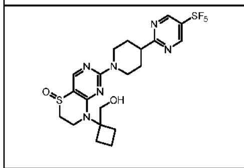 | 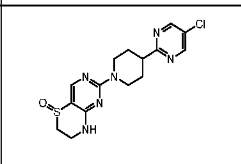 | 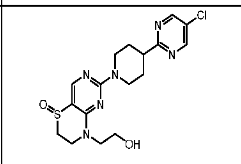 | 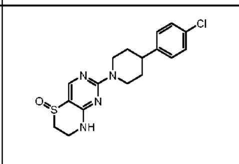 | 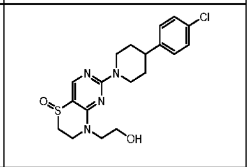 |
| 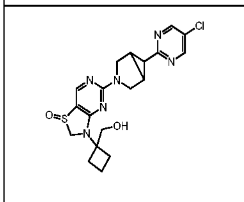 | 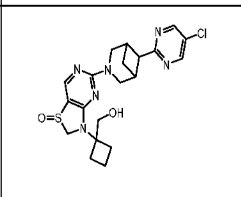 | 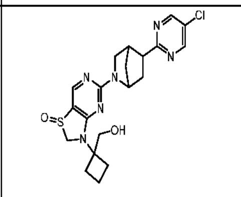 | 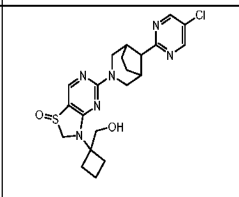 | 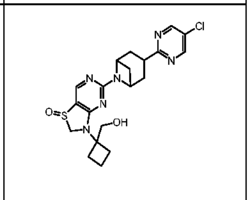 |
| 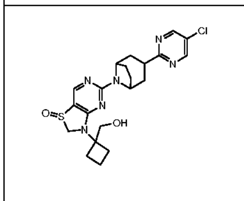 | 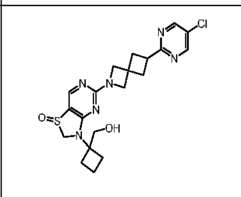 | 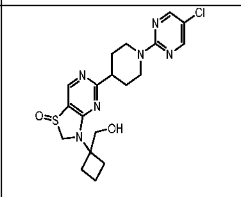 | 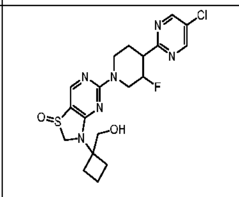 | 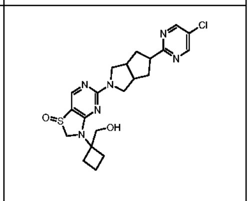 |
| 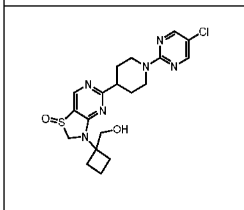 | 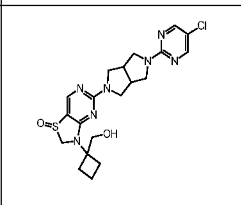 | 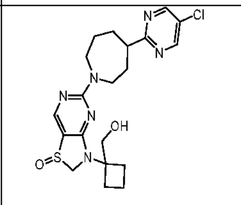 | 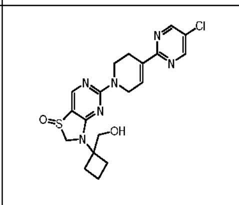 | 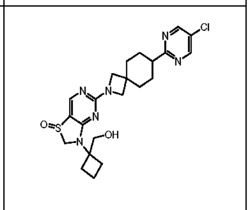 |
| 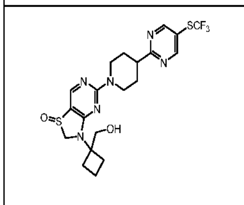 | 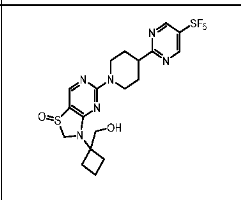 | 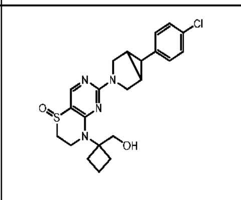 | 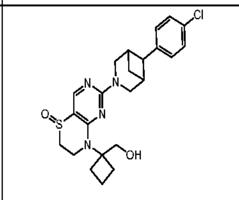 | 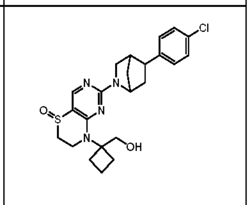 |
| 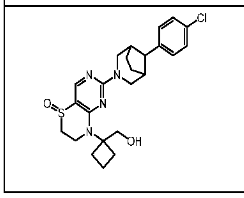 | 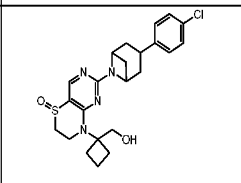 | 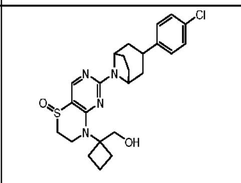 | 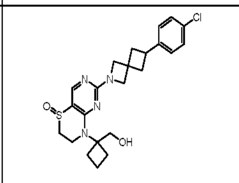 | 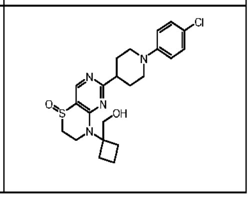 |

| | | | | |
|---|---|---|---|---|
| 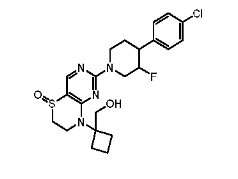 | 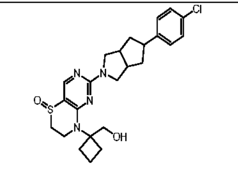 | 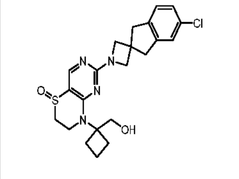 | 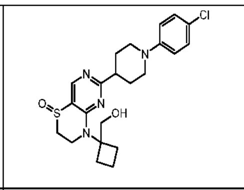 | 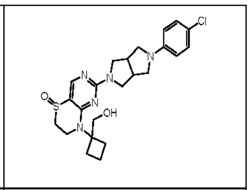 |
| 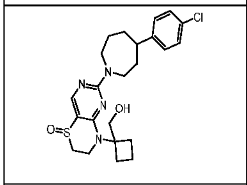 | 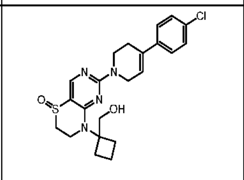 | 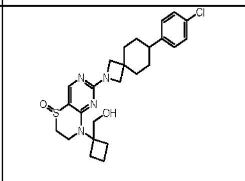 | 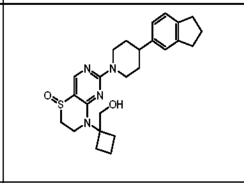 | 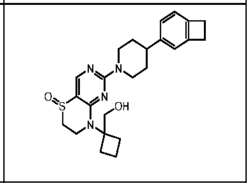 |
| 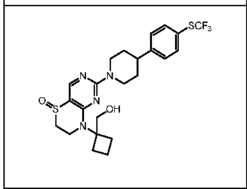 | 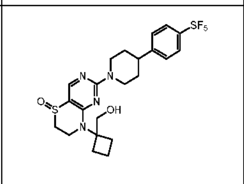 | 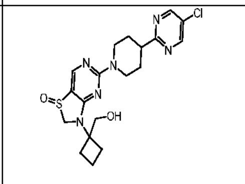 | 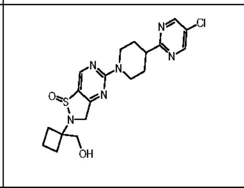 | 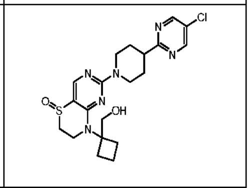 |
| 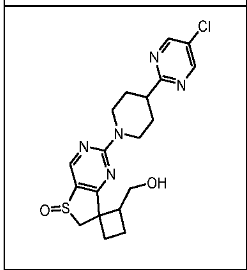 | 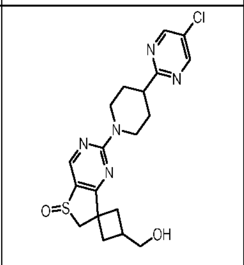 | 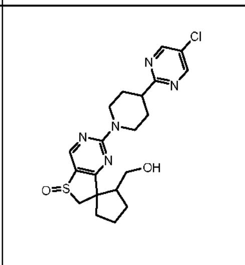 | 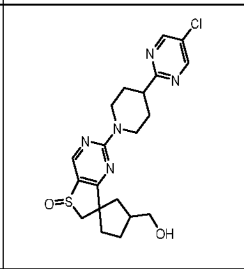 | 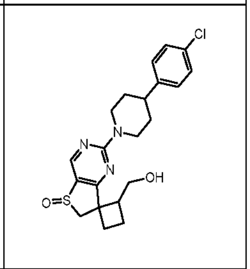 |
| 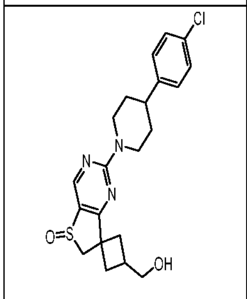 | 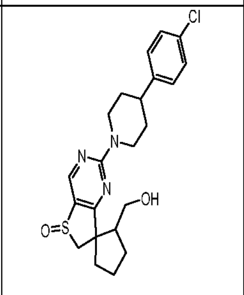 | 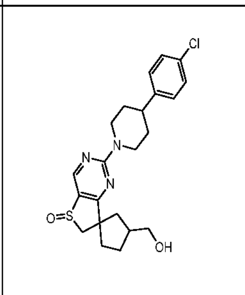 | 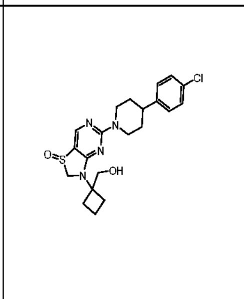 | 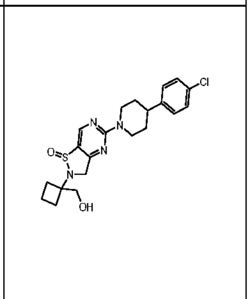 |
| 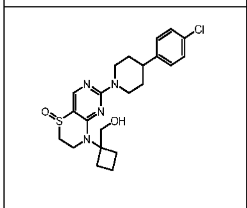 | 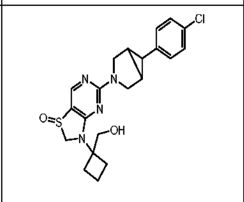 | 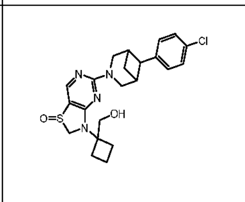 | 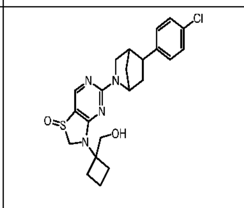 | 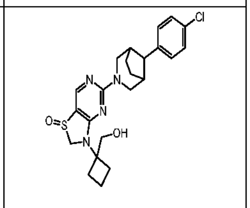 |
| 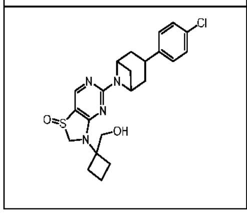 | 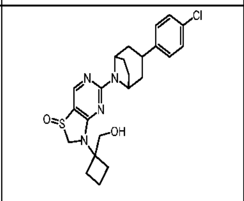 | 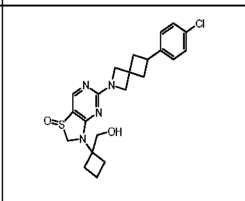 | 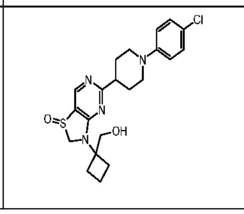 | 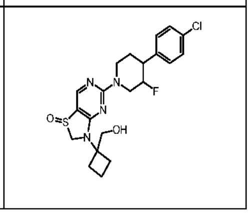 |

| | | | | |
|---|---|---|---|---|
| 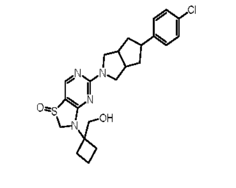 | 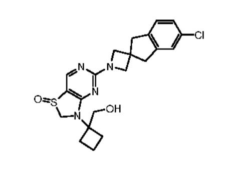 | 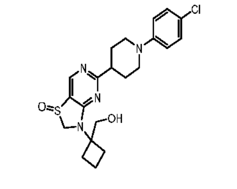 | 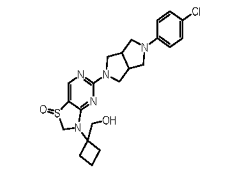 | 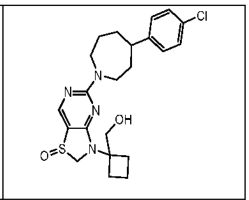 |
| 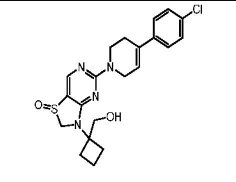 | 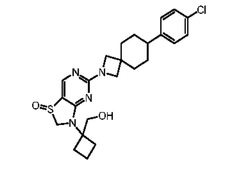 | 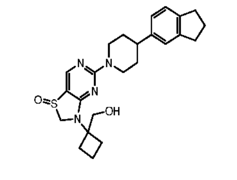 | 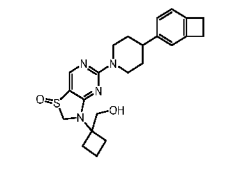 | 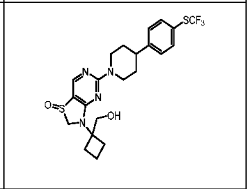 |
| 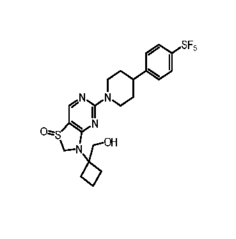 | 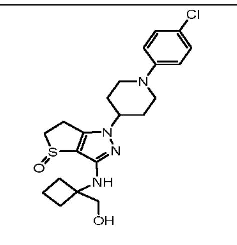 | 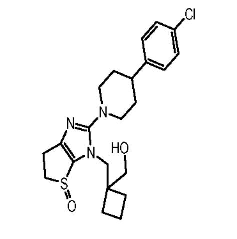 | 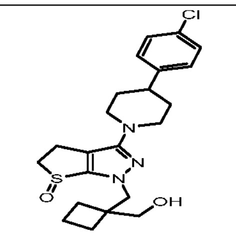 | 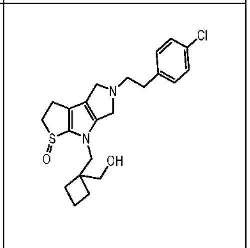 |
| 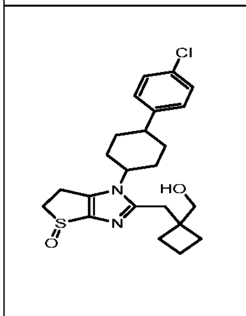 | 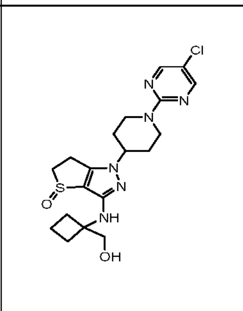 | 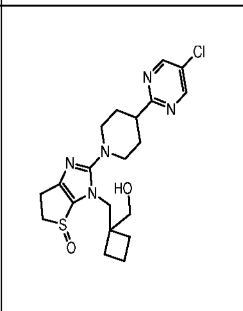 | 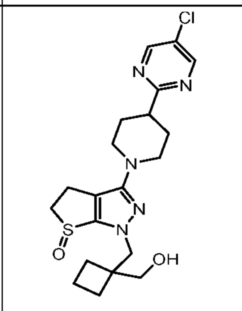 | 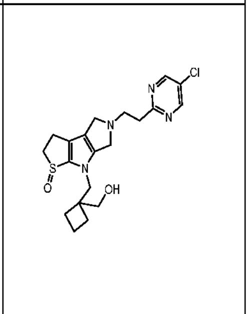 |
| 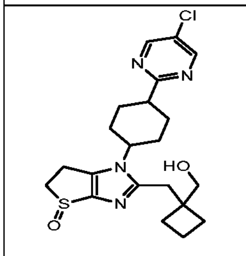 | 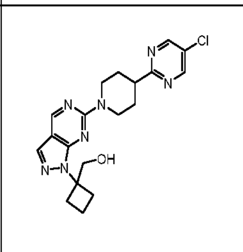 | 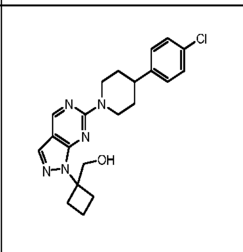 | 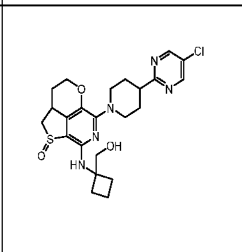 | 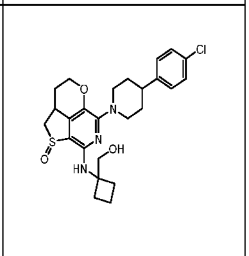 |
| 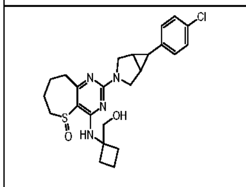 | 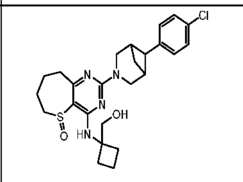 | 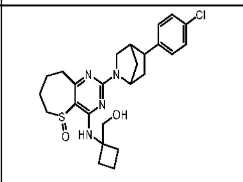 | 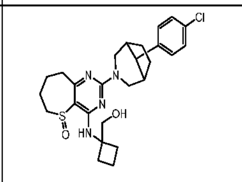 | 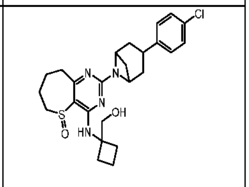 |
| 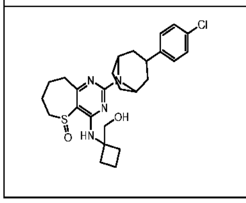 | 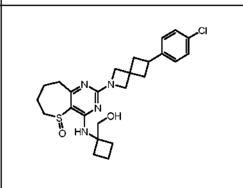 | 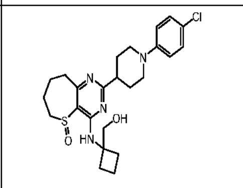 | 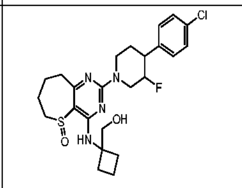 | 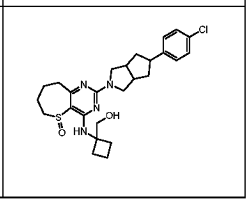 |

| | | | | |
|---|---|---|---|---|
| 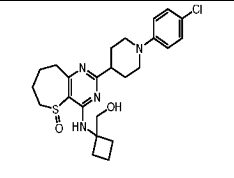 | 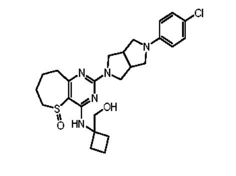 | 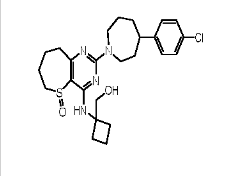 | 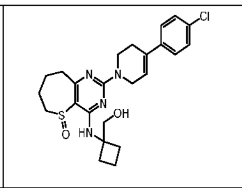 | 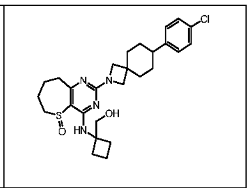 |
| 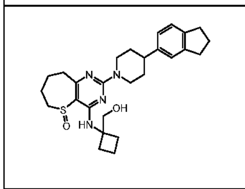 | 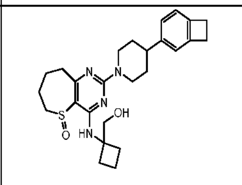 | 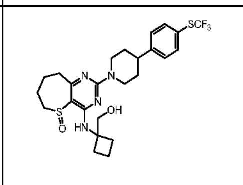 | 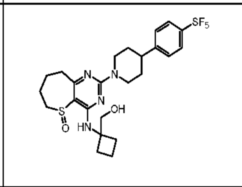 | 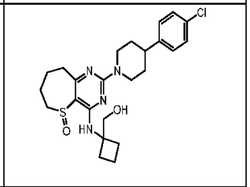 |
| 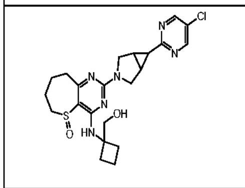 | 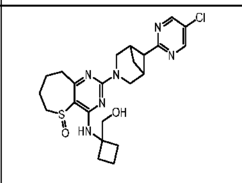 | 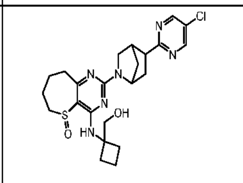 | 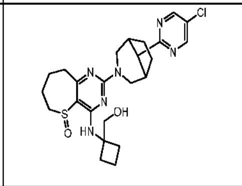 | 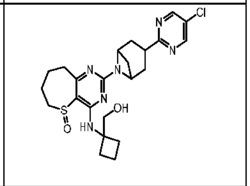 |
| 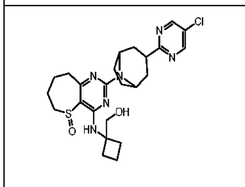 | 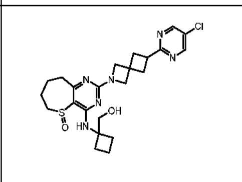 | 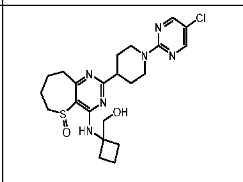 | 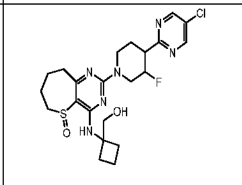 | 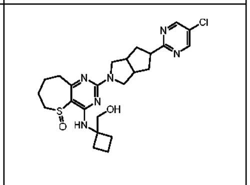 |
| 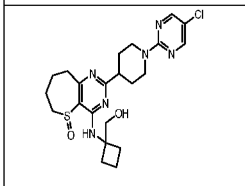 | 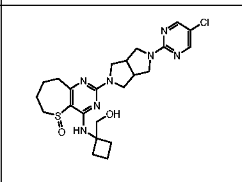 | 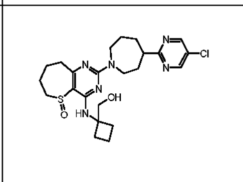 | 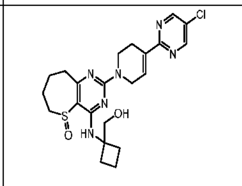 | 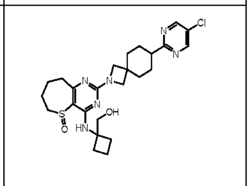 |
| 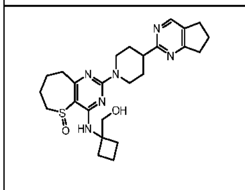 | 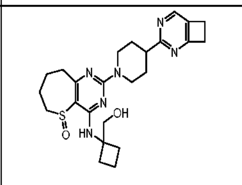 | 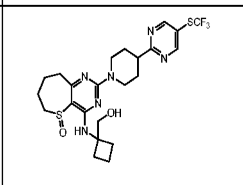 | 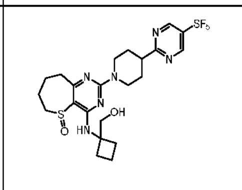 | 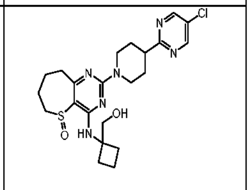 |
| 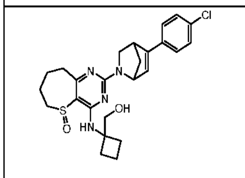 | 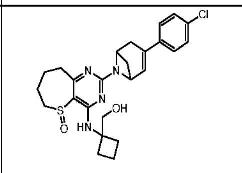 | 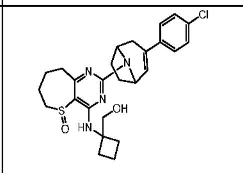 | 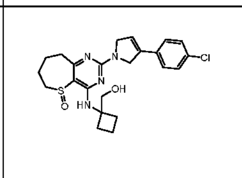 | 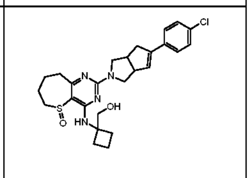 |
| 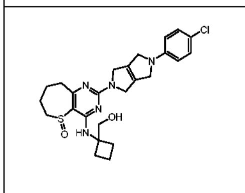 | 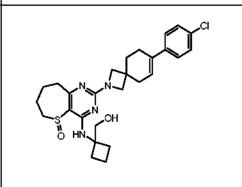 | 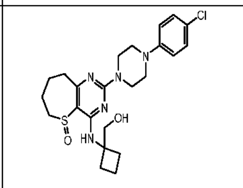 | 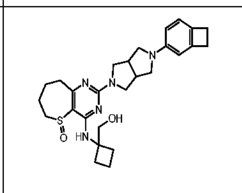 | 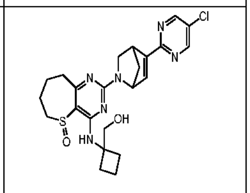 |
| | | | | |

| | | | | |
|---|---|---|---|---|
| 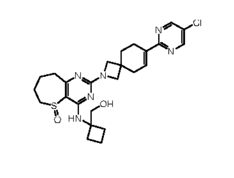 | 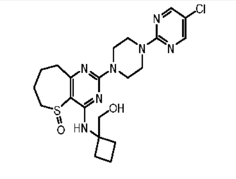 | 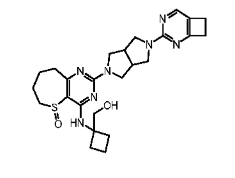 | 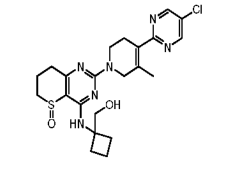 | 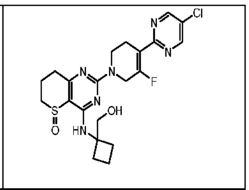 |
| 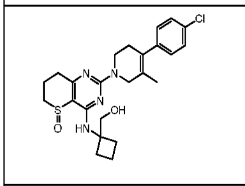 | 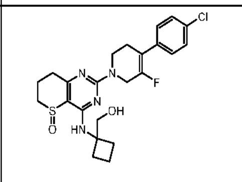 | 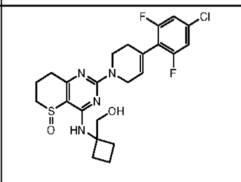 | 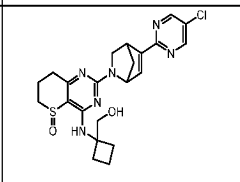 | 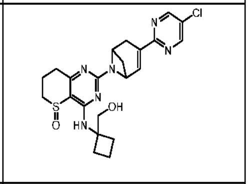 |
| 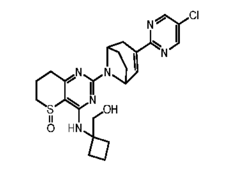 | 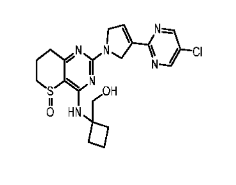 | 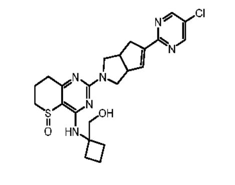 | 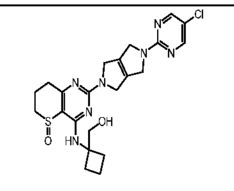 | 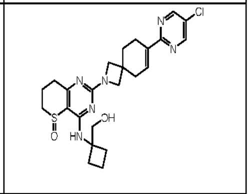 |
| 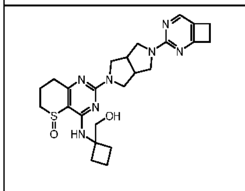 | 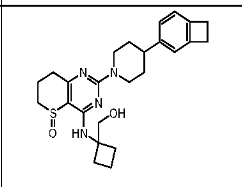 | 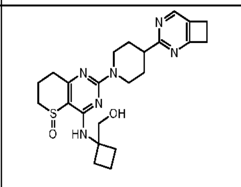 | 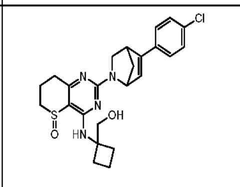 | 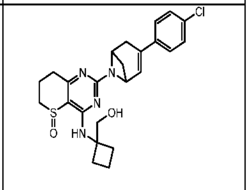 |
| 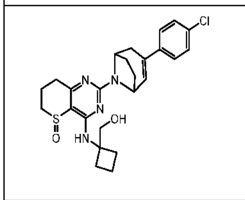 | 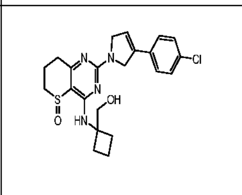 | 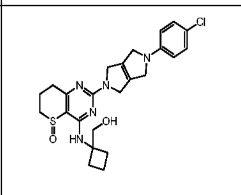 | 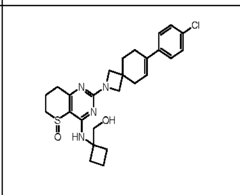 | 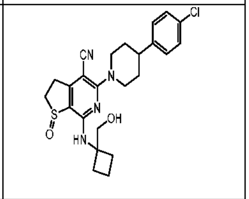 |
| 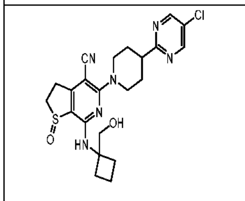 | 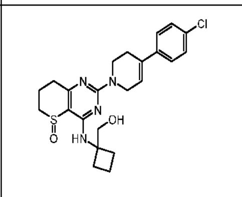 | 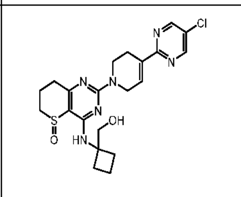 | 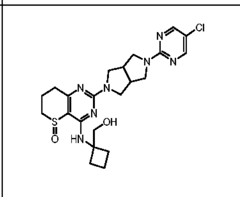 | 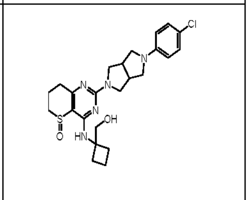 |
| 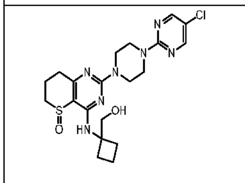 | 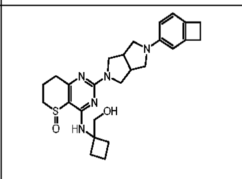 | 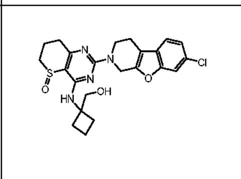 | 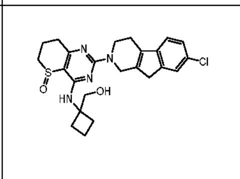 | 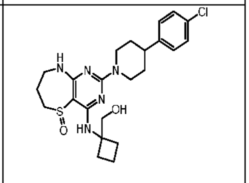 |
| 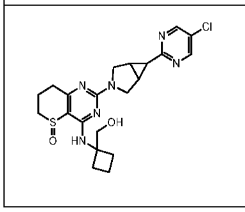 | 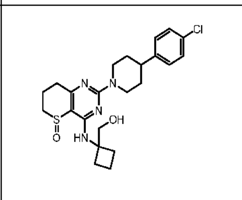 | 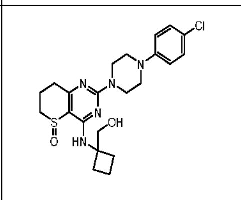 | 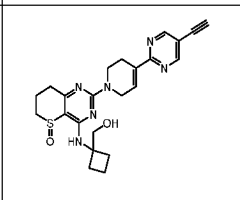 | 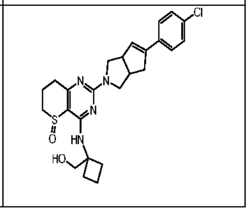 |

| | | | | |
|---|---|---|---|---|
| 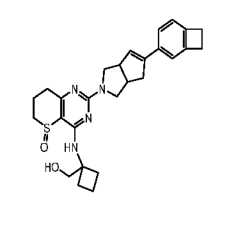 | 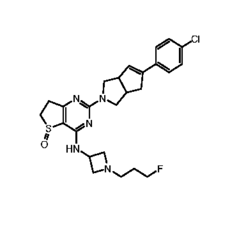 | 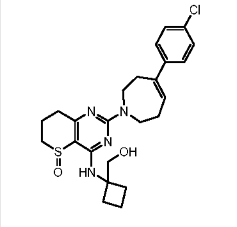 | 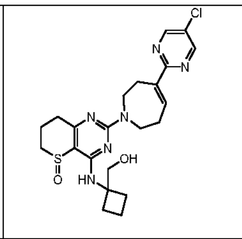 | 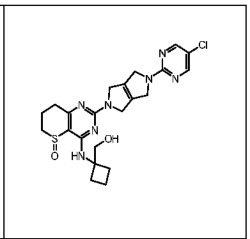 |
| 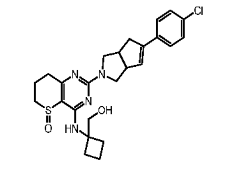 | 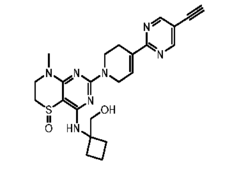 | 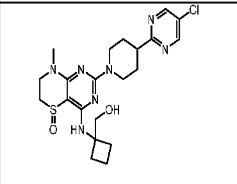 | 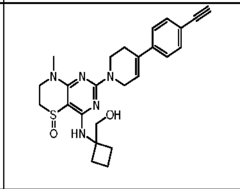 | 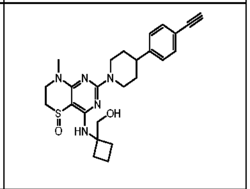 |
| 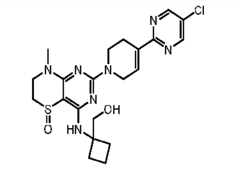 | 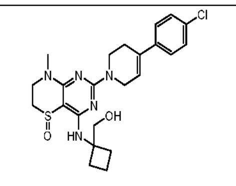 | 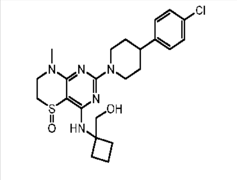 | 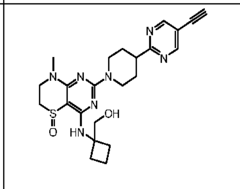 | 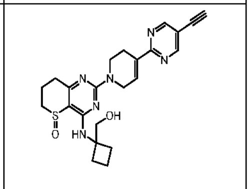 |
| 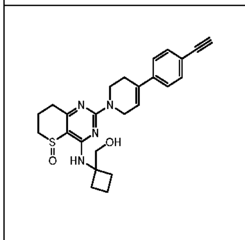 | 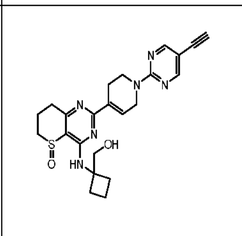 | 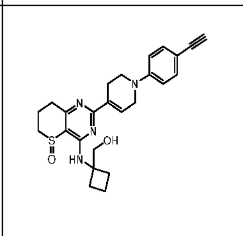 | 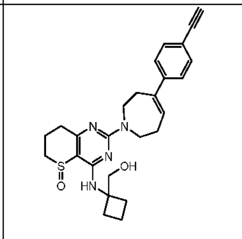 | 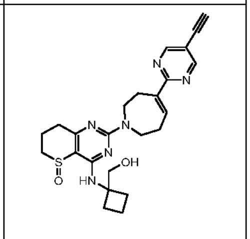 |
| 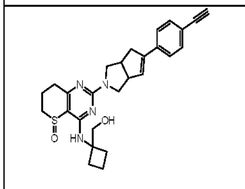 | 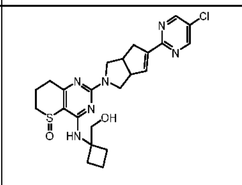 | 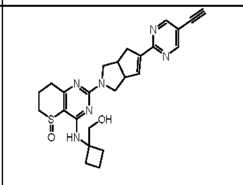 | 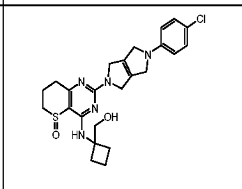 | 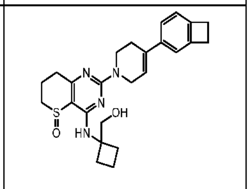 |
| 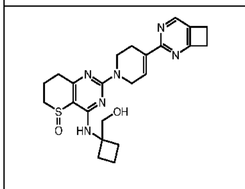 | 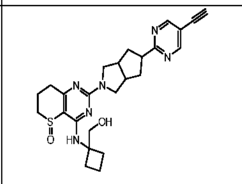 | 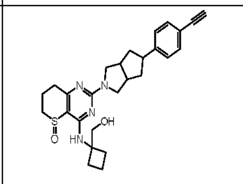 | 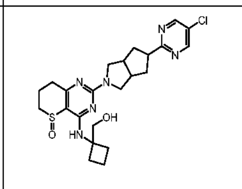 | 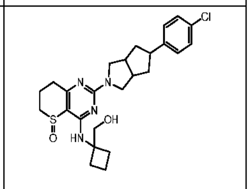 |
| 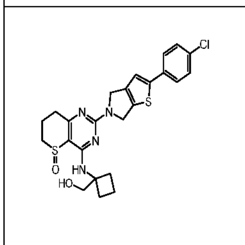 | 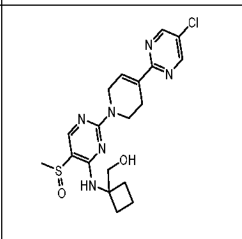 | 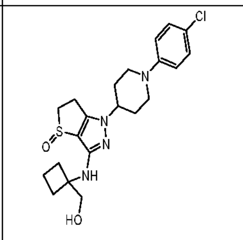 | 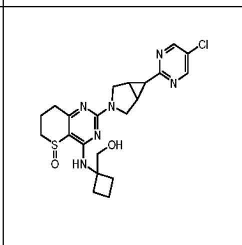 | 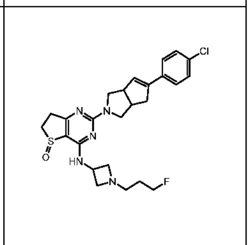 |
| 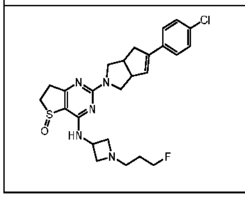 | 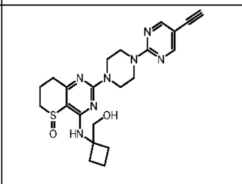 | 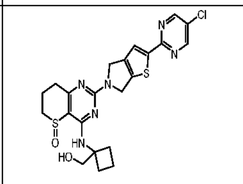 | 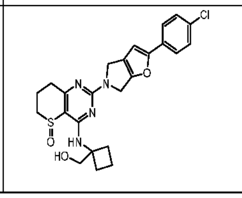 | 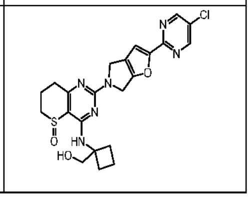 |

| | | | | |
|---|---|---|---|---|
| 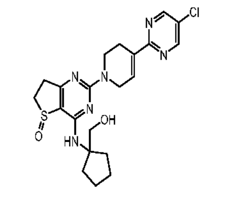 | 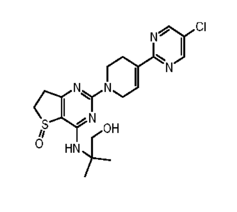 | 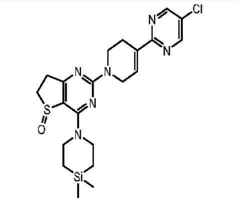 | 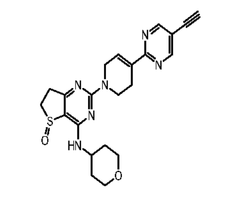 | 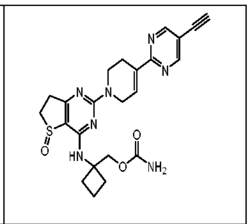 |
| 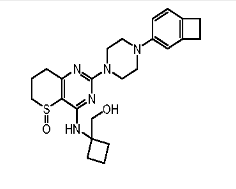 | 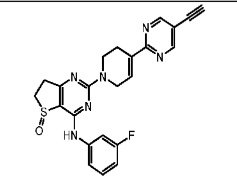 | 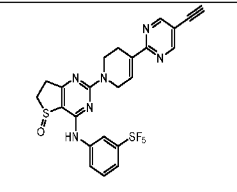 | 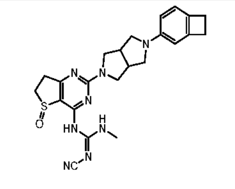 | 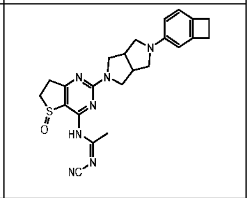 |
| 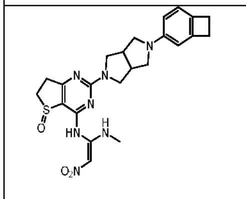 | 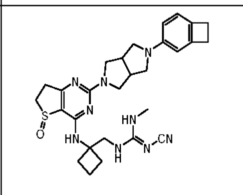 | 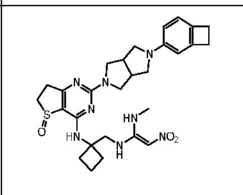 | 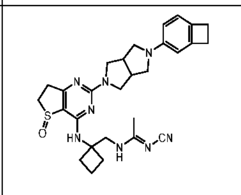 | 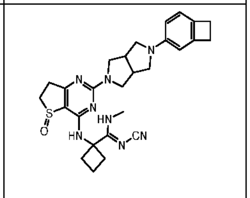 |
| 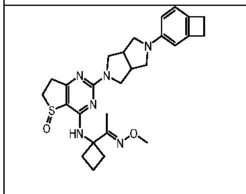 | 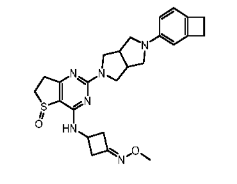 | 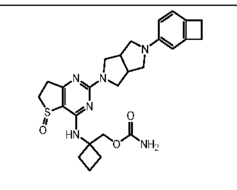 | 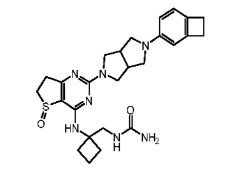 | 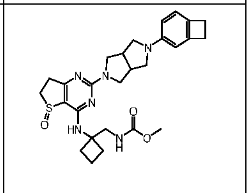 |
| 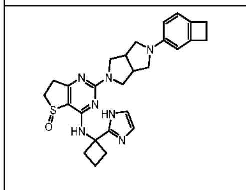 | 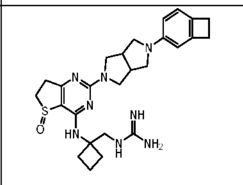 | 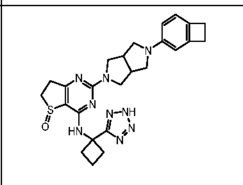 | 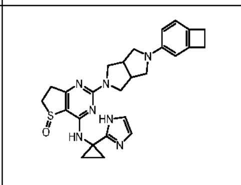 | 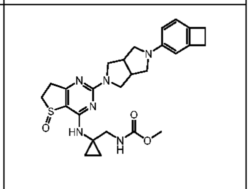 |
| 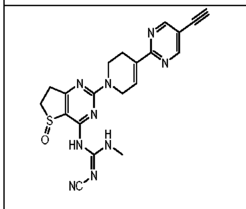 | 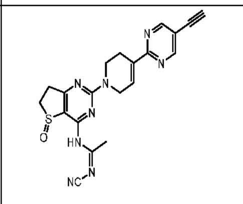 | 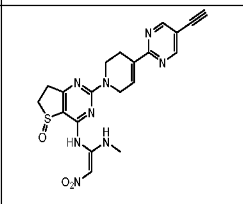 | 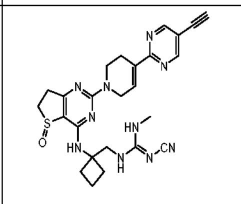 | 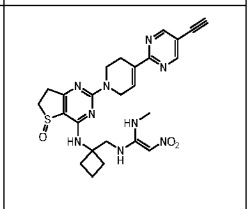 |
| 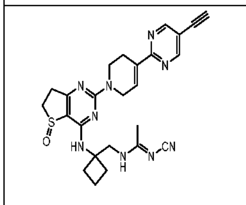 | 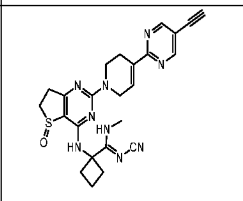 | 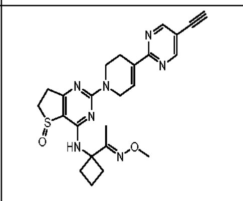 | 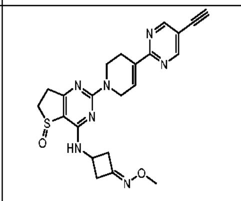 | 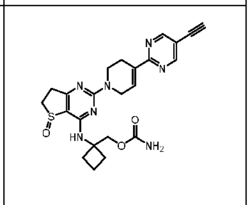 |
| 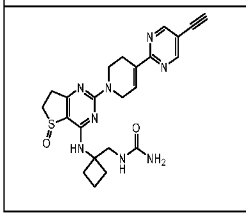 | 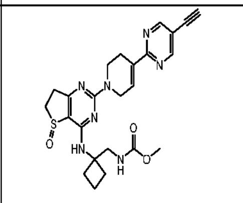 | 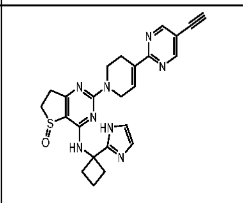 | 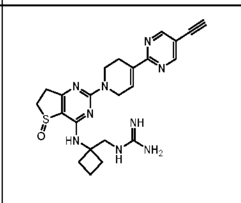 | 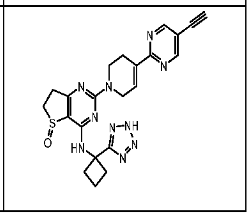 |

| | | | | |
|---|---|---|---|---|
| 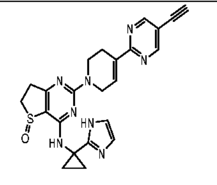 | 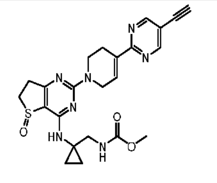 | 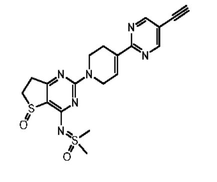 | 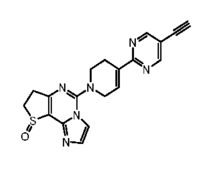 | 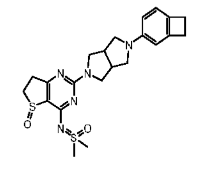 |
| 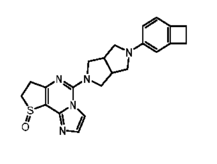 | 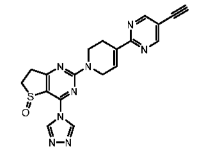 | 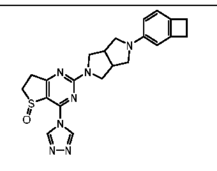 | 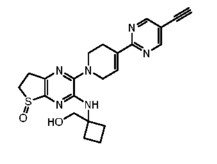 | 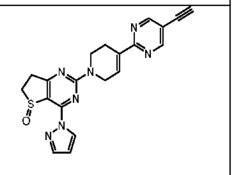 |
| 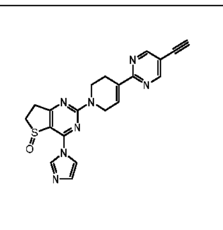 | 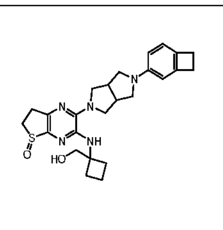 | 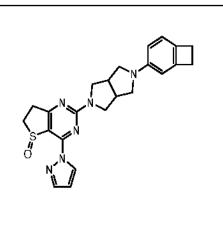 | 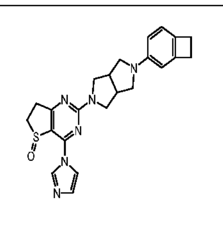 | 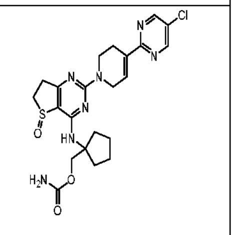 |
| 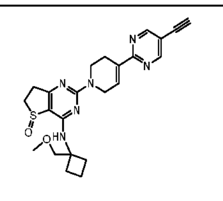 | 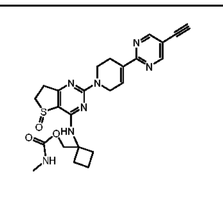 | 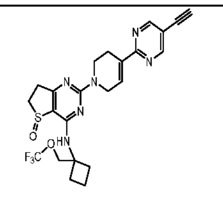 | 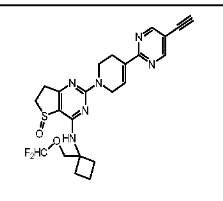 | 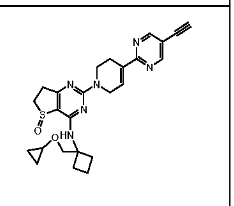 |
| 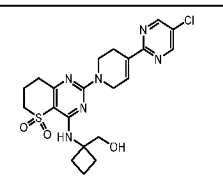 | 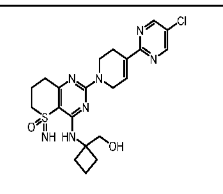 | 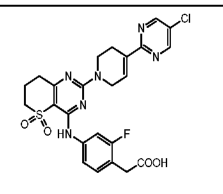 | 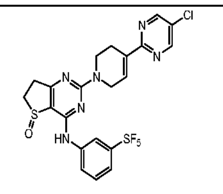 | 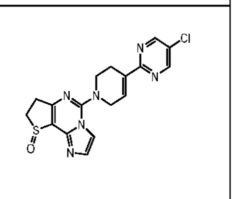 |
| 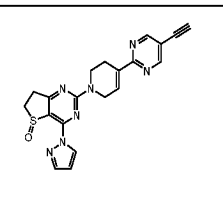 | 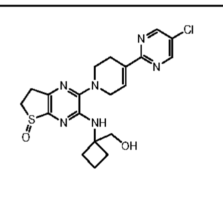 | 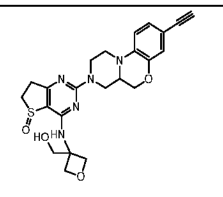 | 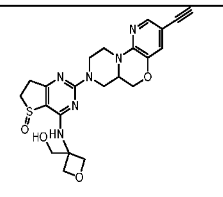 | 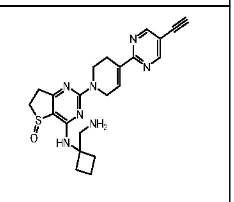 |
| 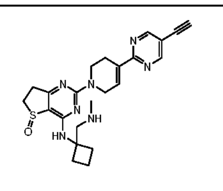 | 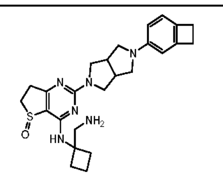 | 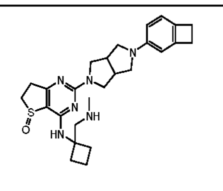 | 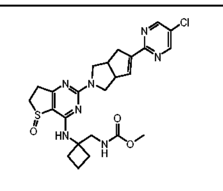 | 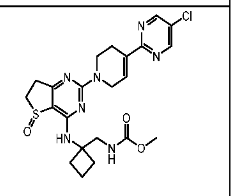 |
| 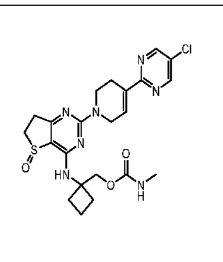 | 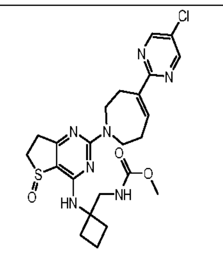 | 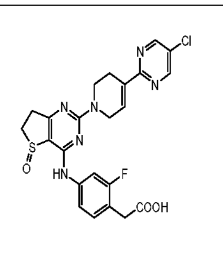 | 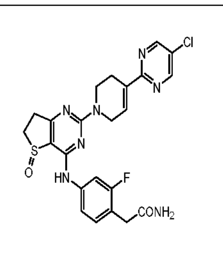 | 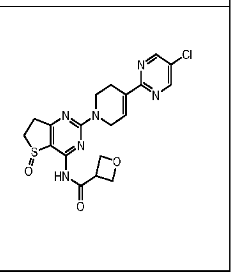 |

| | | | | |
|---|---|---|---|---|
| 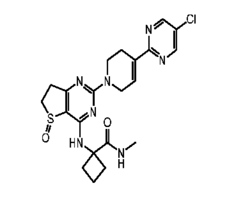 | 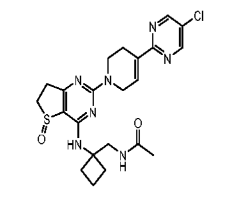 | 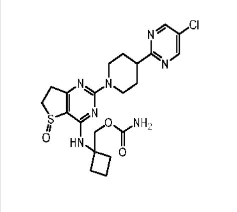 | 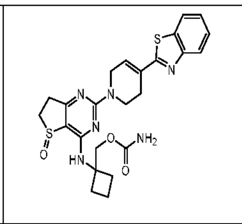 | 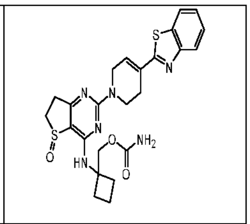 |
| 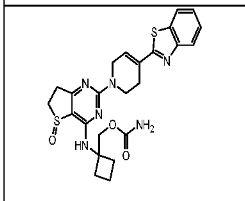 | 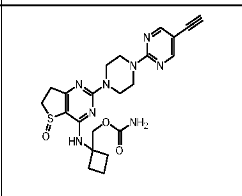 | 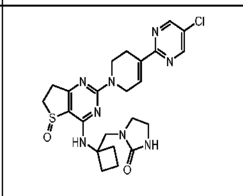 | 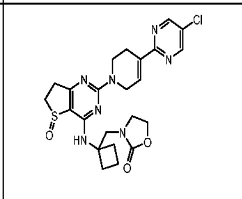 | 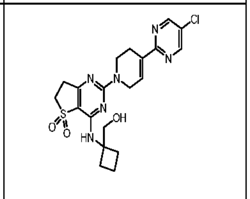 |
| 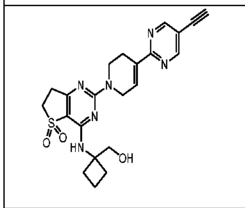 | 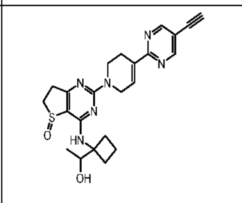 | 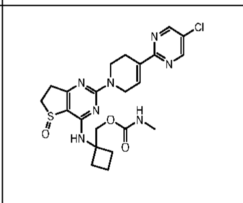 | 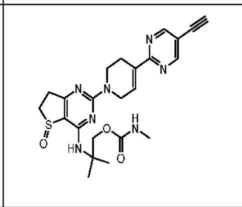 | 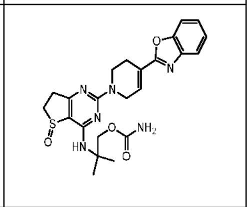 |
| 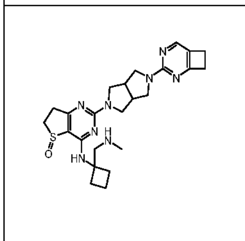 | 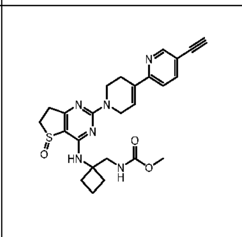 | 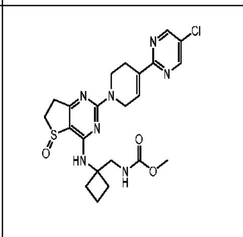 | 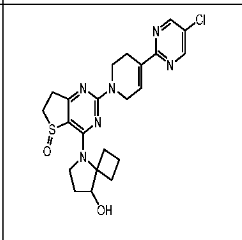 | 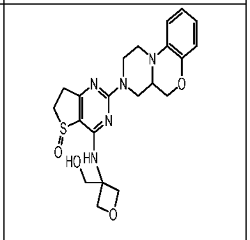 |
| 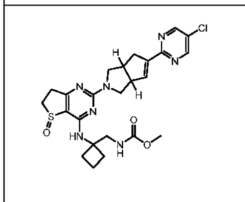 | 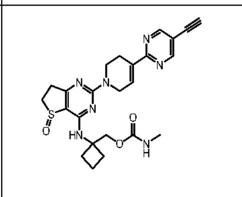 | 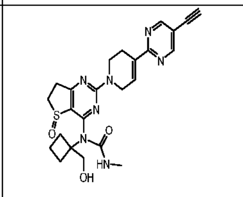 | 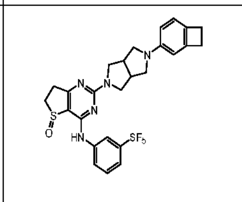 | 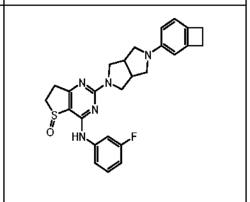 |
| 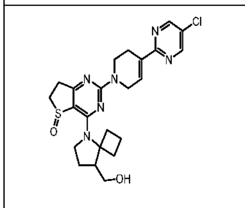 | 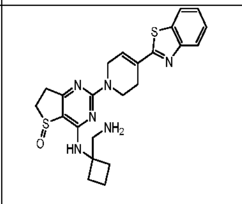 | 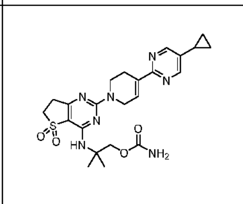 | 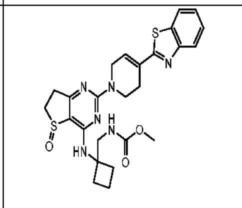 | 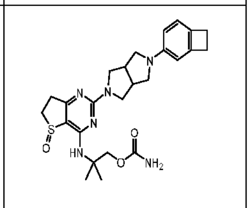 |
| 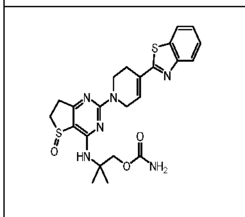 | 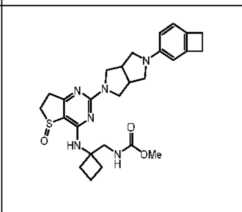 | 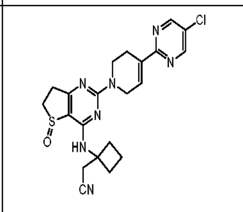 | 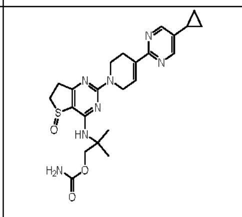 | 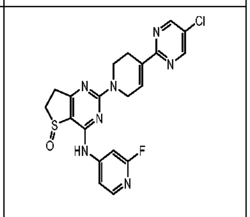 |
| 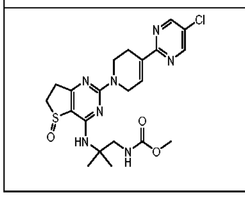 | 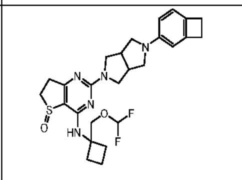 | 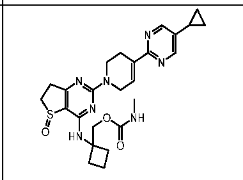 | 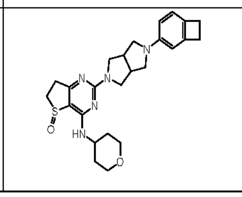 | 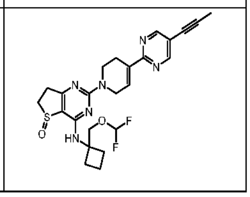 |

| | | | | |
|---|---|---|---|---|
| 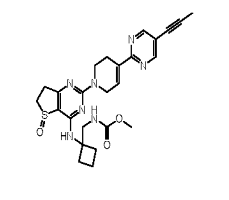 | 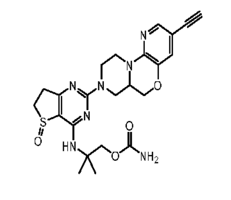 | 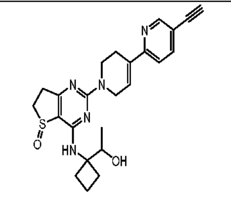 | 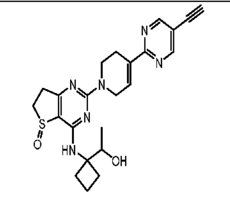 | 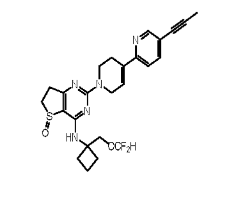 |
| 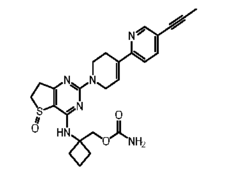 | 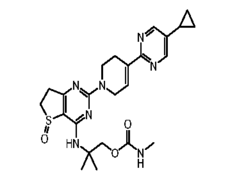 | 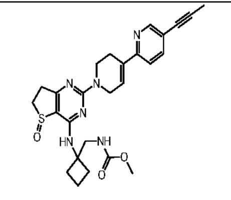 | 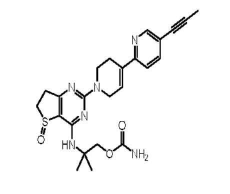 | 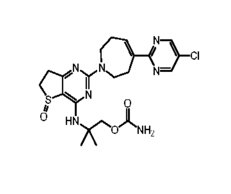 |
| 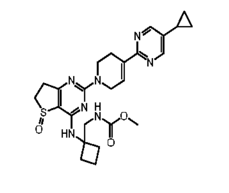 | 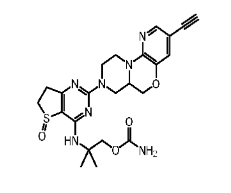 | 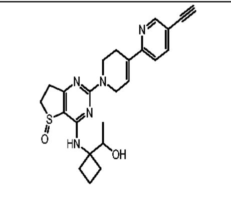 | 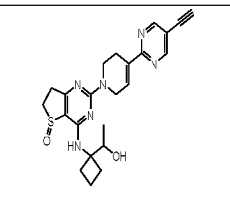 | 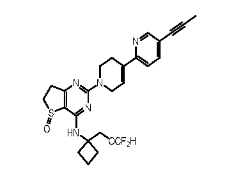 |
| 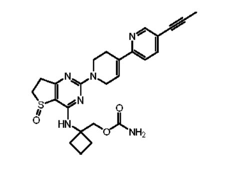 | 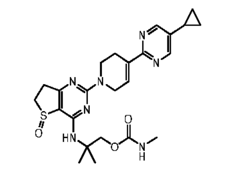 | 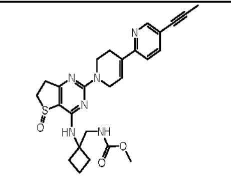 | 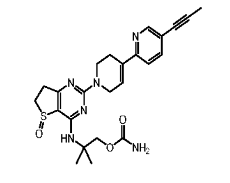 | 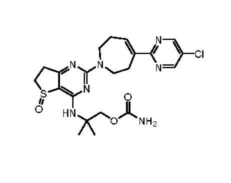 |
| 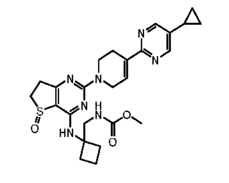 | 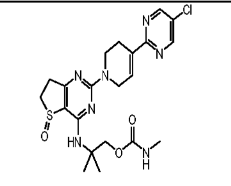 | 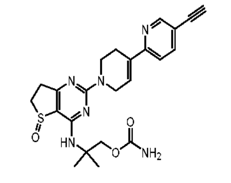 | 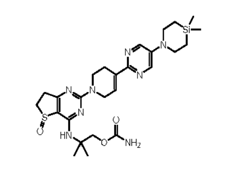 | 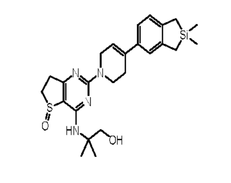 |
| 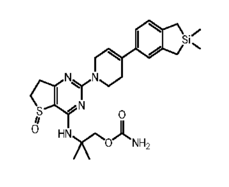 | 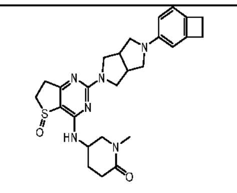 | 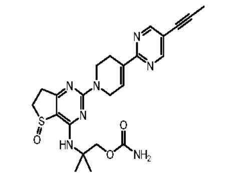 | 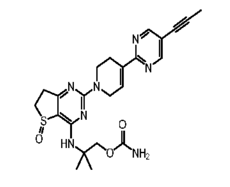 | 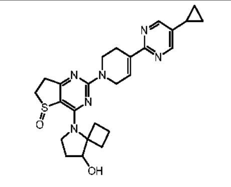 |
| 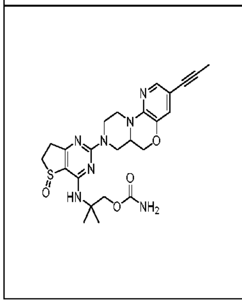 | 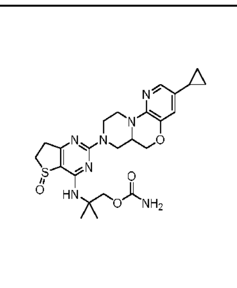 | 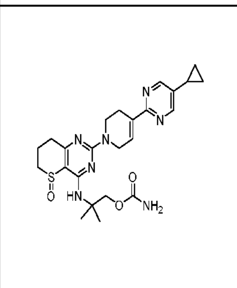 | 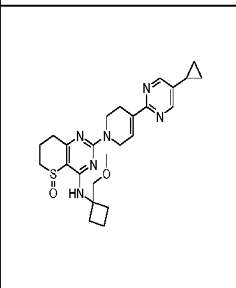 | 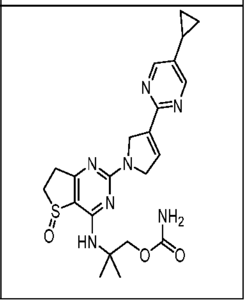 |

| | | | | |
|---|---|---|---|---|
| 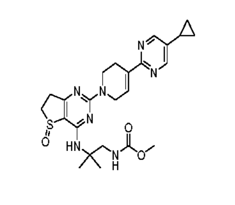 | 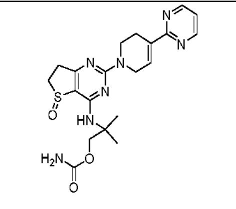 | 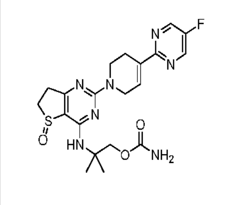 | 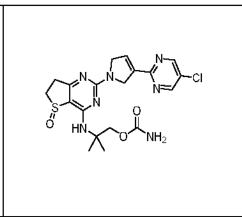 | 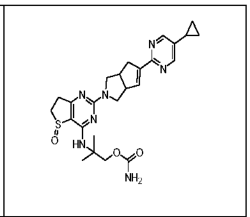 |
| 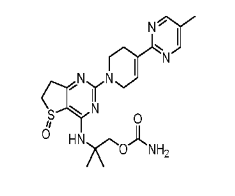 | 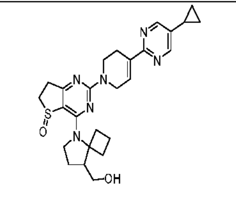 | 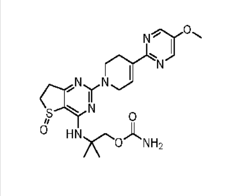 | 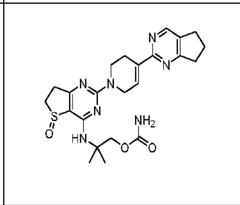 | 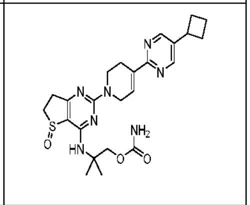 |
| 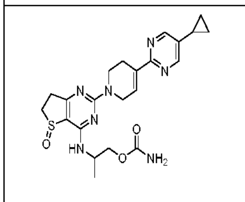 | 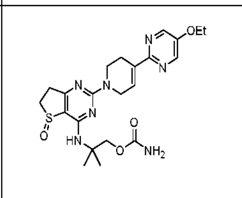 | 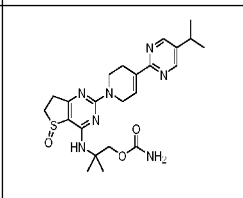 | 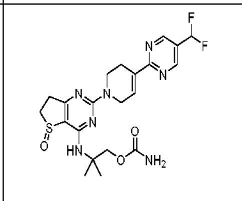 | 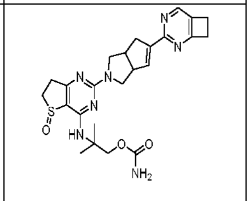 |
| 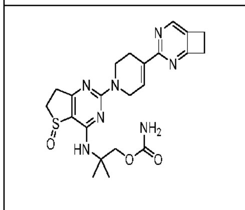 | 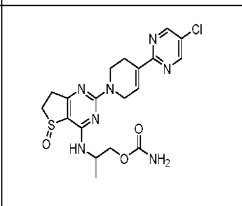 | 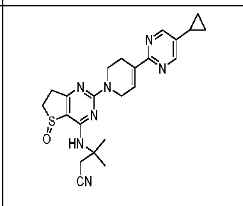 | 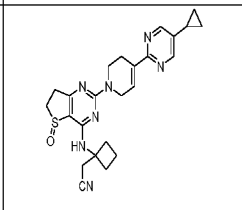 | 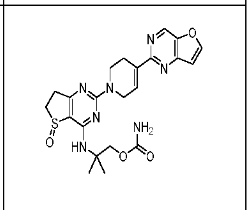 |
| 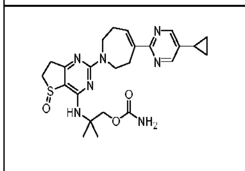 | 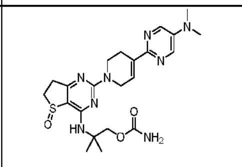 | 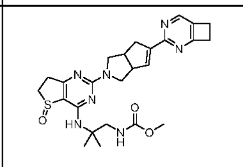 | 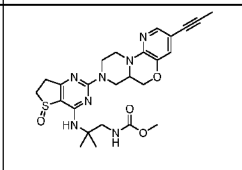 | 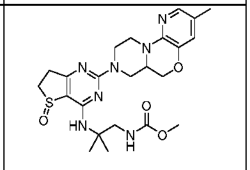 |
| 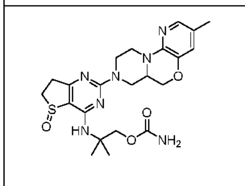 | 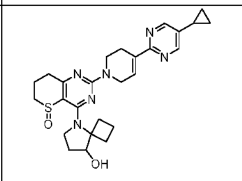 | 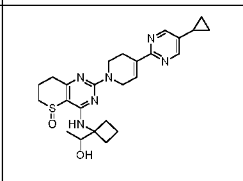 | 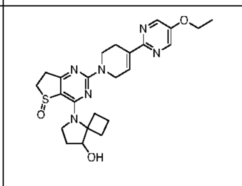 | 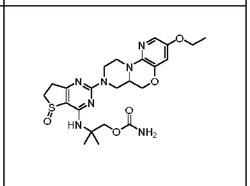 |
| 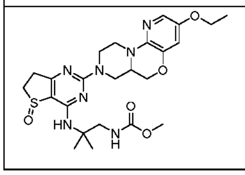 | | | | |

Solution 21 of the present disclosure relates to a compound selected from one of the structures in Table 2 below, or stereoisomers or pharmaceutically acceptable salts thereof:

Table 2:

| | | | | |
|---|---|---|---|---|
| 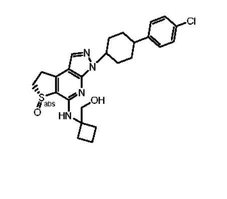 | 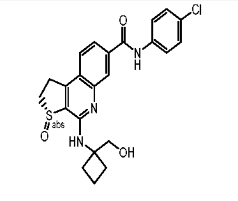 | 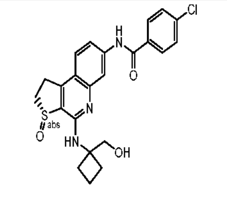 | 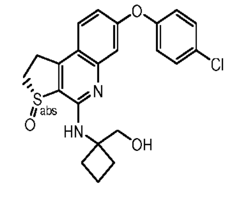 | 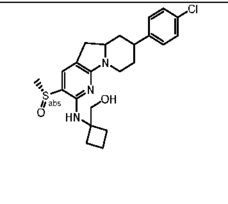 |
| 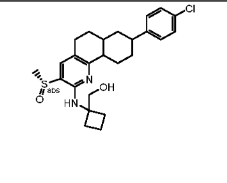 | 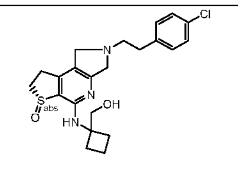 | 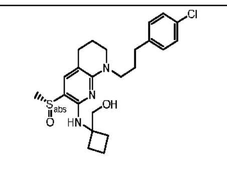 | 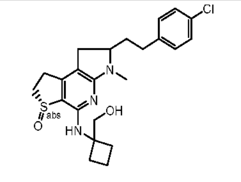 | 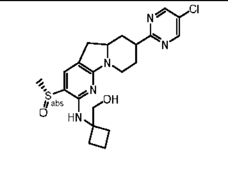 |
| 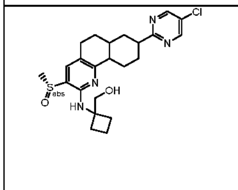 | 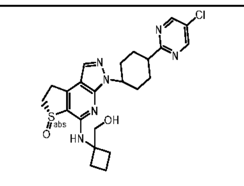 | 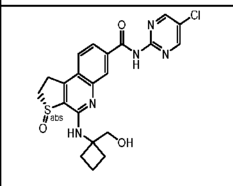 | 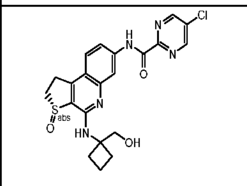 | 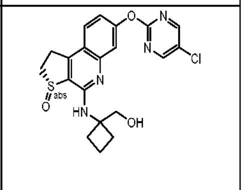 |
| 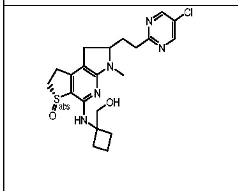 | 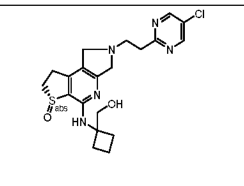 | 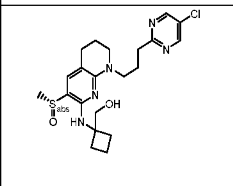 | 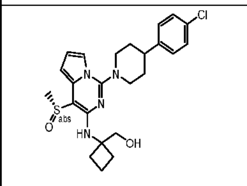 | 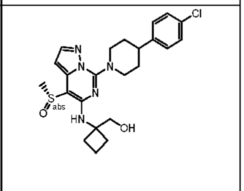 |
| 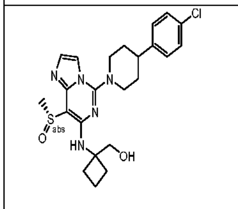 | 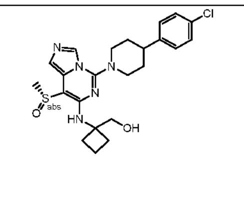 | 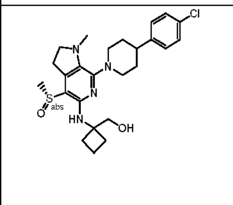 | 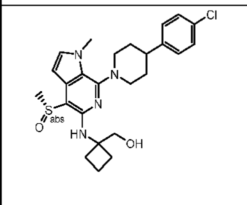 | 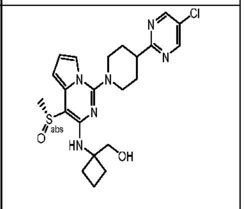 |
| 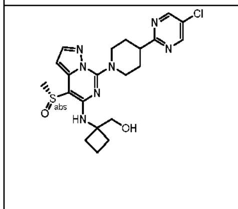 | 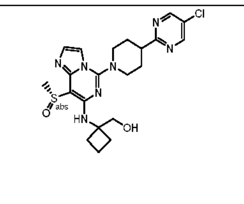 | 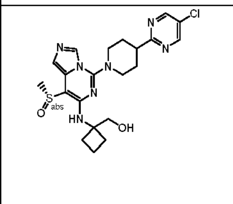 | 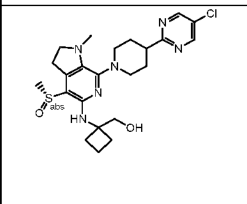 | 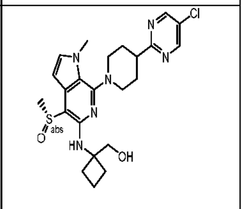 |
| 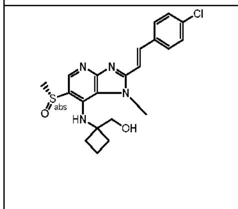 | 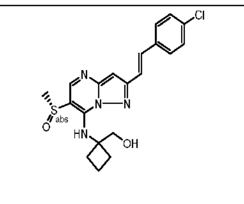 | 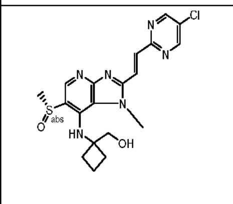 | 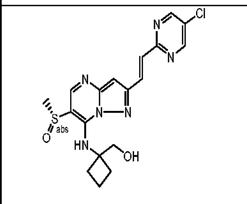 | 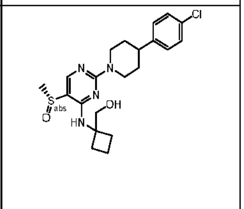 |
| 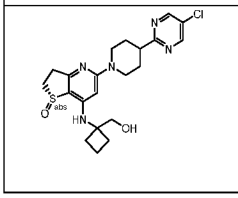 | 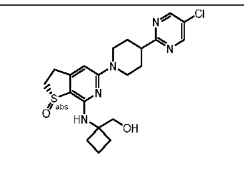 | 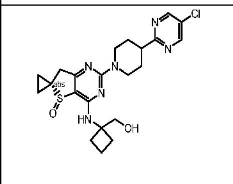 | 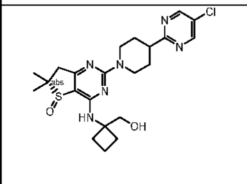 | 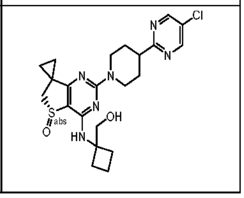 |

| | | | | |
|---|---|---|---|---|
| 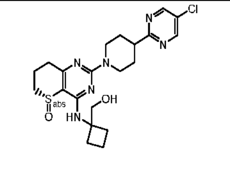 | 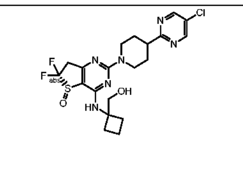 | 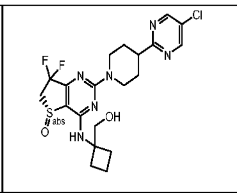 | 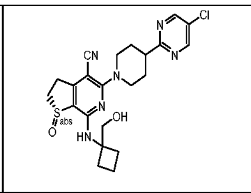 | 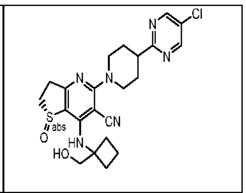 |
| 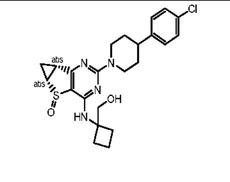 | 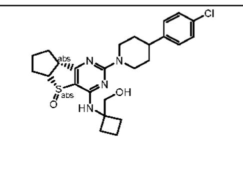 | 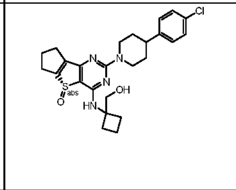 | 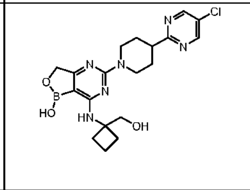 | 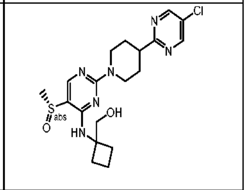 |
| 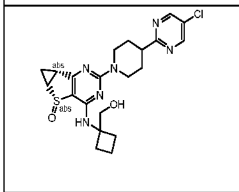 | 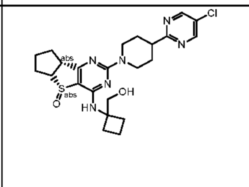 | 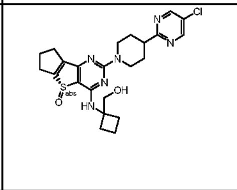 | 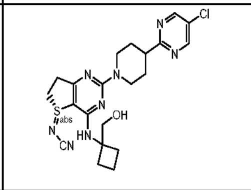 | 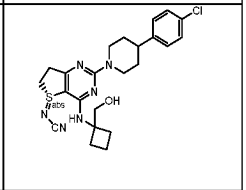 |
| 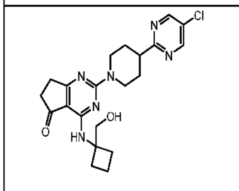 | 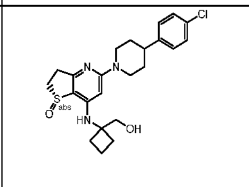 | 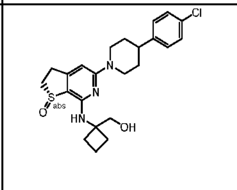 | 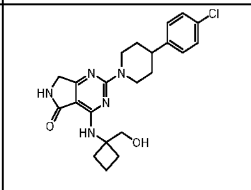 | 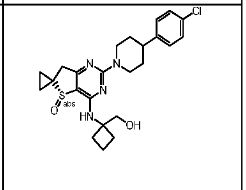 |
| 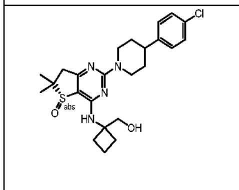 | 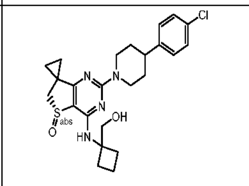 | 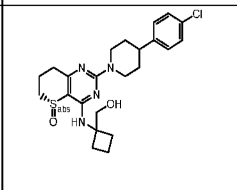 | 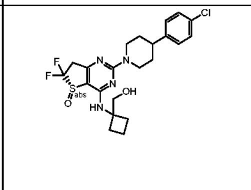 | 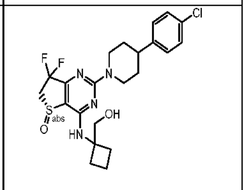 |
| 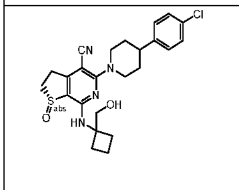 | 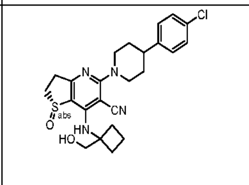 | 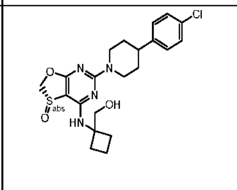 | 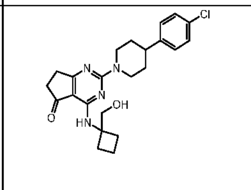 | 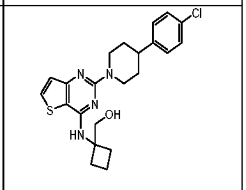 |
| 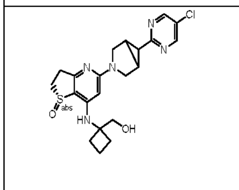 | 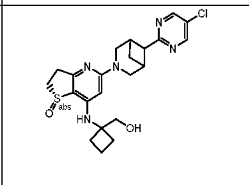 | 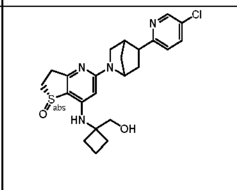 | 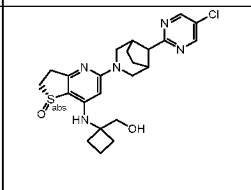 | 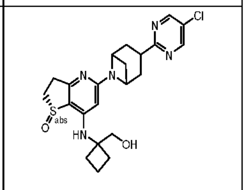 |
| 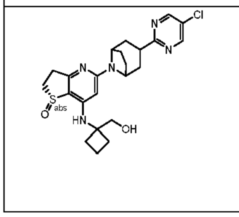 | 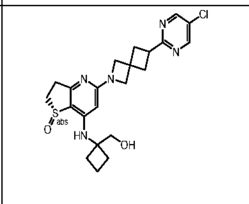 | 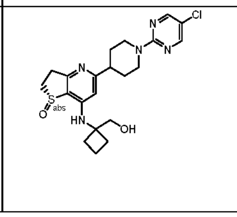 | 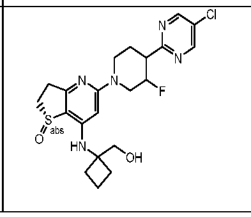 | 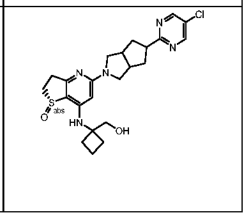 |

| | | | | |
|---|---|---|---|---|
| 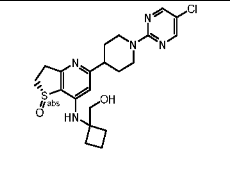 | 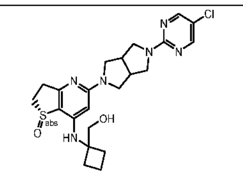 | 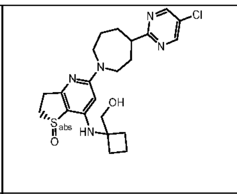 | 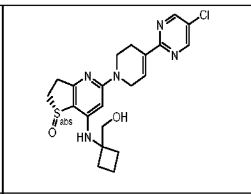 | 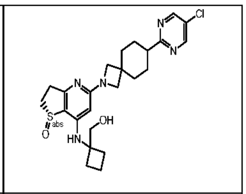 |
| 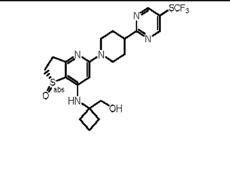 | 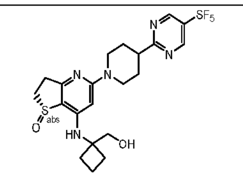 | 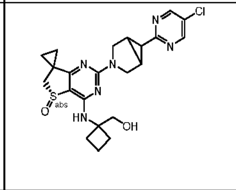 | 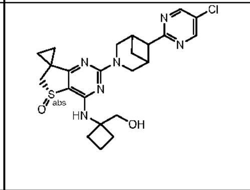 | 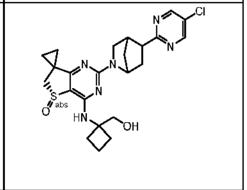 |
| 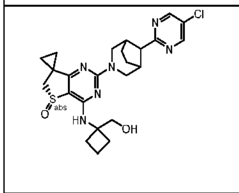 | 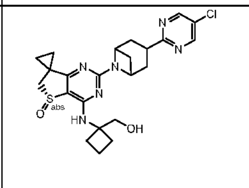 | 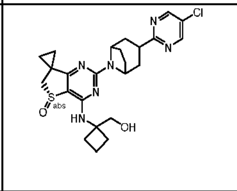 | 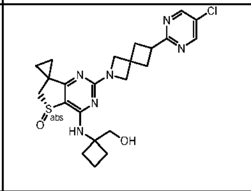 | 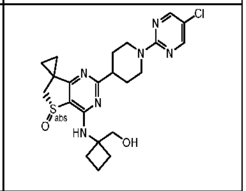 |
| 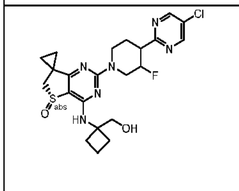 | 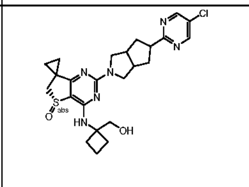 | 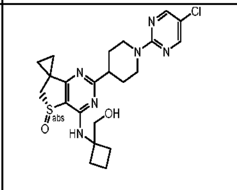 | 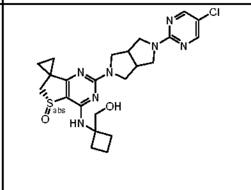 | 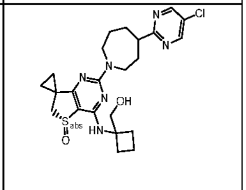 |
| 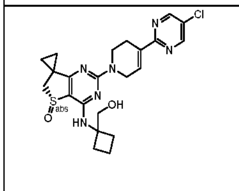 | 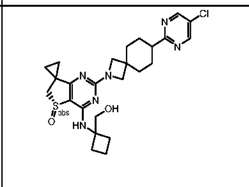 | 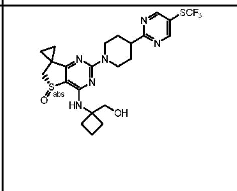 | 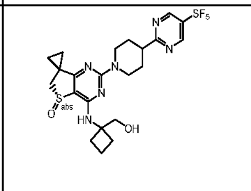 | 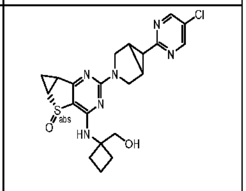 |
| 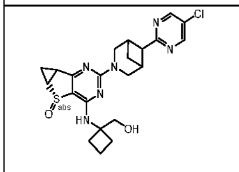 | 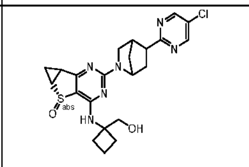 | 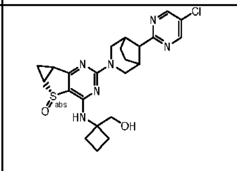 | 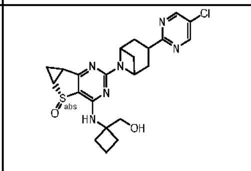 | 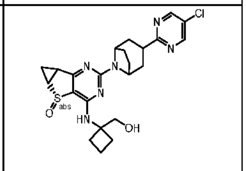 |
| 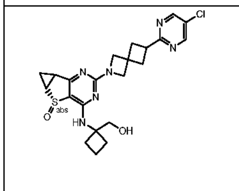 | 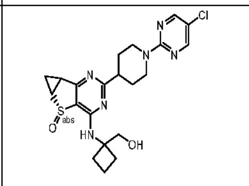 | 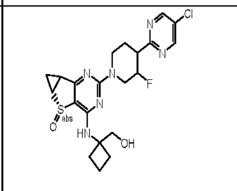 | 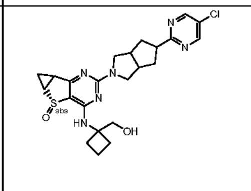 | 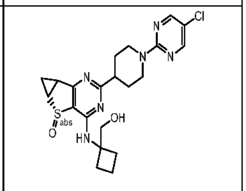 |
| 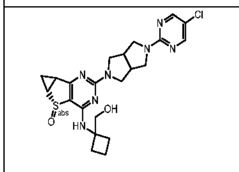 | 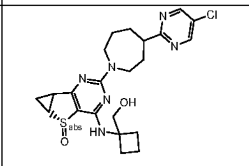 | 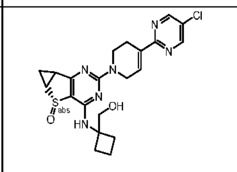 | 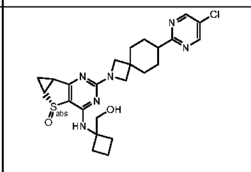 | 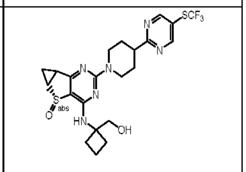 |
| | | | | |

| | | | | |
|---|---|---|---|---|
| 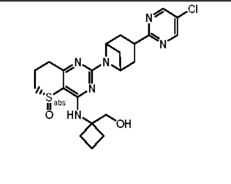 | 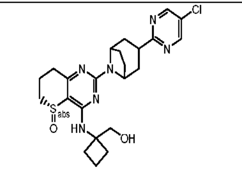 | 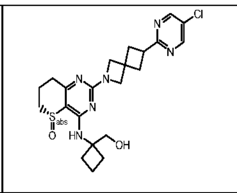 | 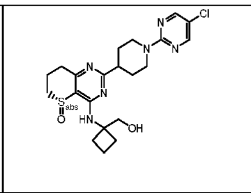 | 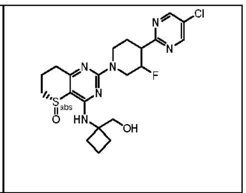 |
| 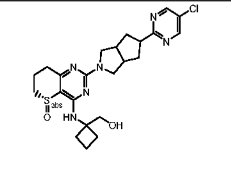 | 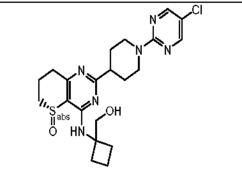 | 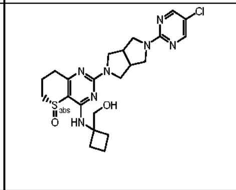 | 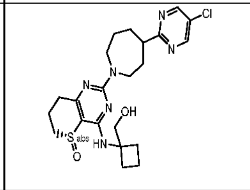 | 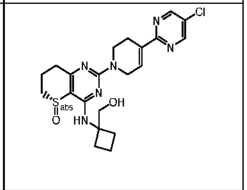 |
| 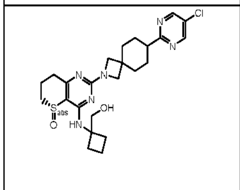 | 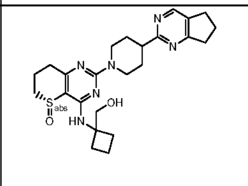 | 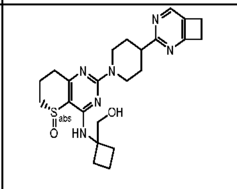 | 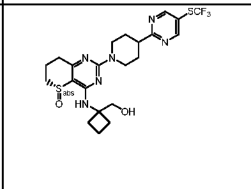 | 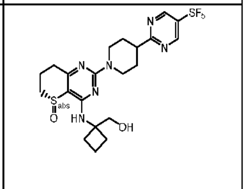 |
| 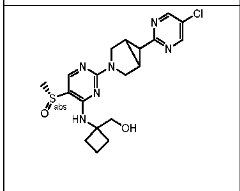 | 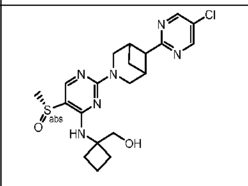 | 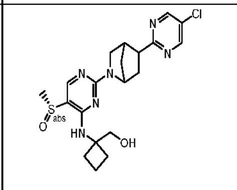 | 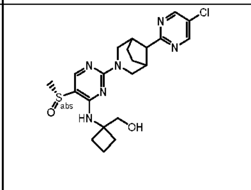 | 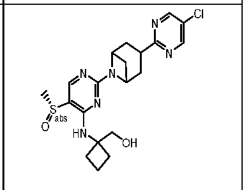 |
| 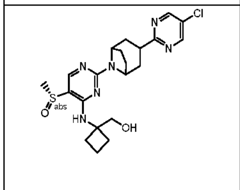 | 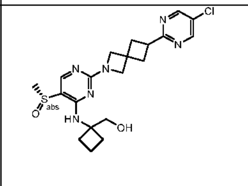 | 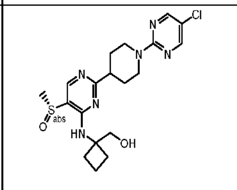 | 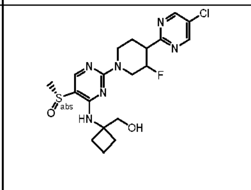 | 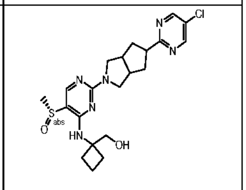 |
| 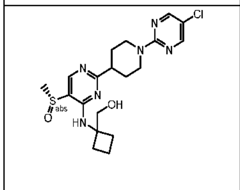 | 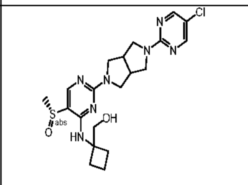 | 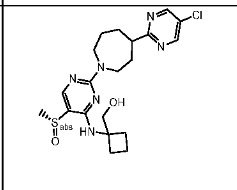 | 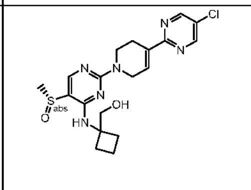 | 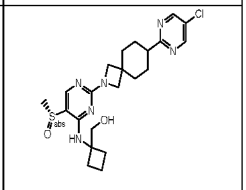 |
| 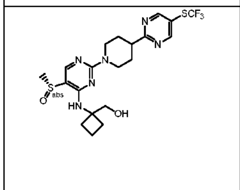 | 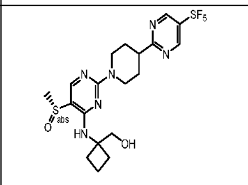 | 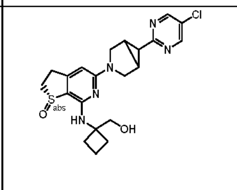 | 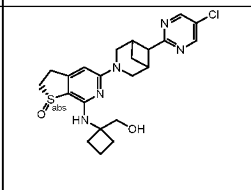 | 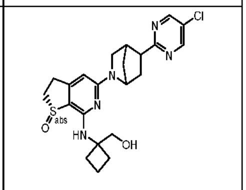 |
| 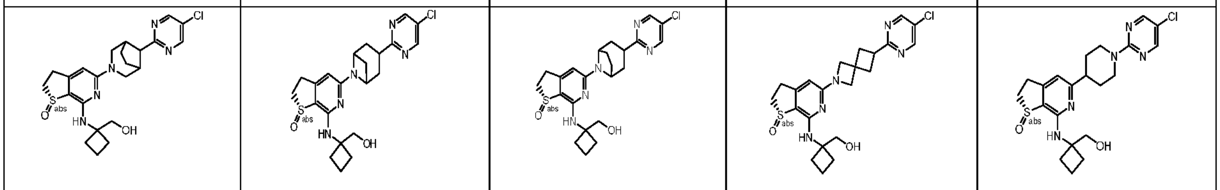 | | | | |
| 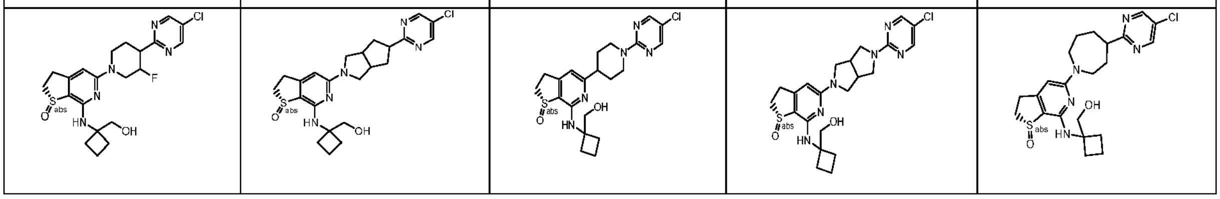 | | | | |

| | | | | |
|---|---|---|---|---|
| 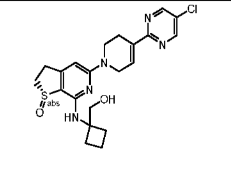 | 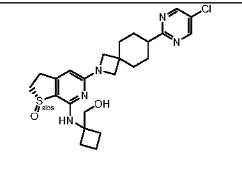 | 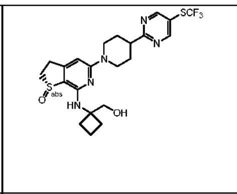 | 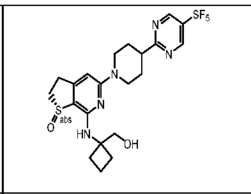 | 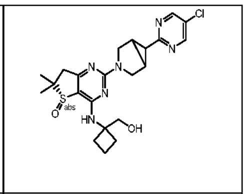 |
| 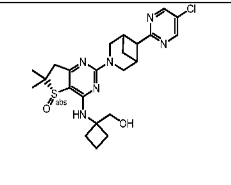 | 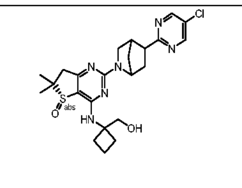 | 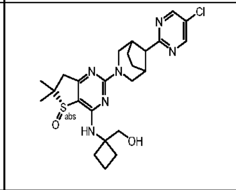 | 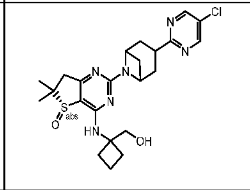 | 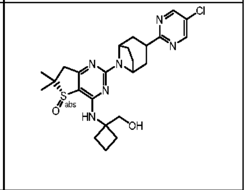 |
| 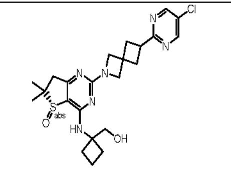 | 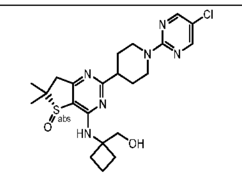 | 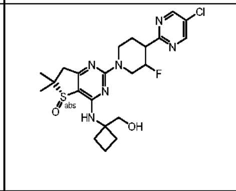 | 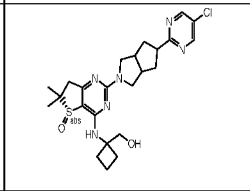 | 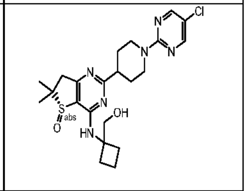 |
| 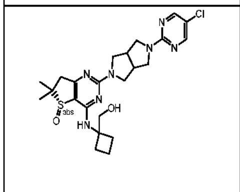 | 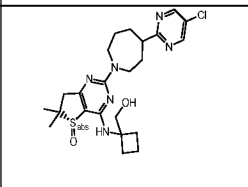 | 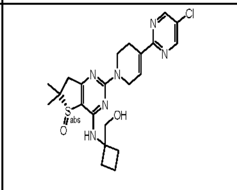 | 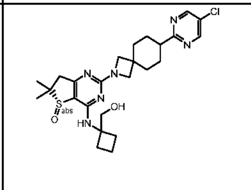 | 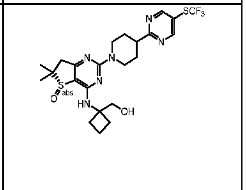 |
| 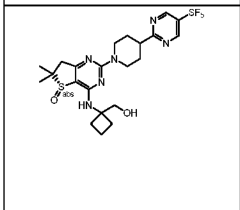 | 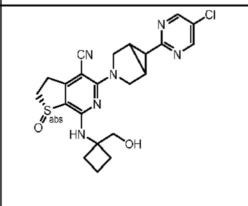 | 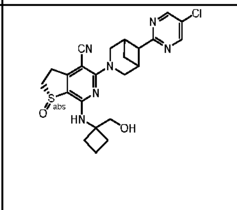 | 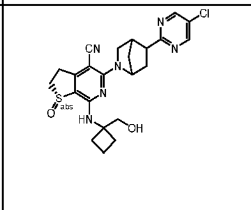 | 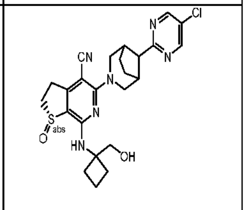 |
| 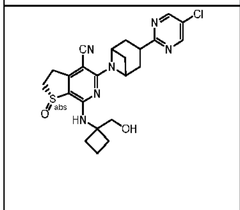 | 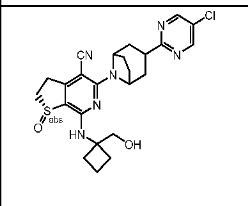 | 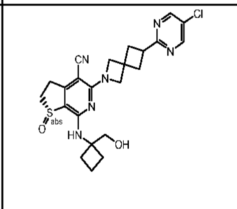 | 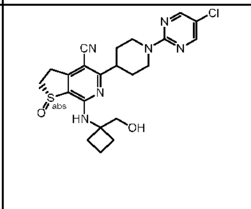 | 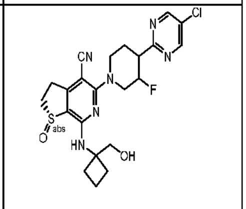 |
| 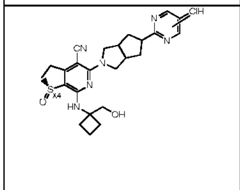 | 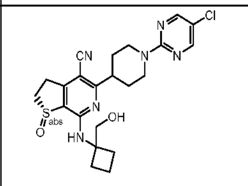 | 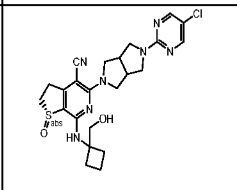 | 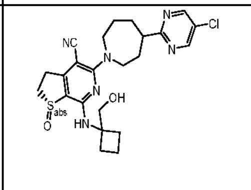 | 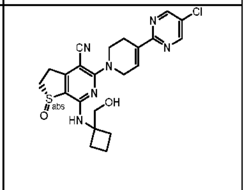 |
| 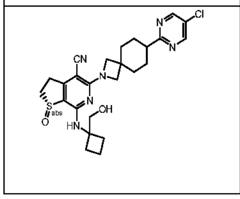 | 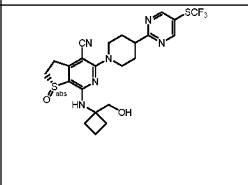 | 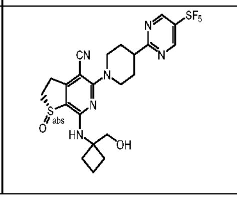 | 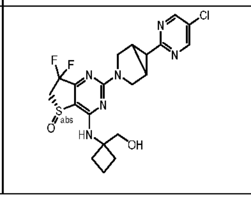 | 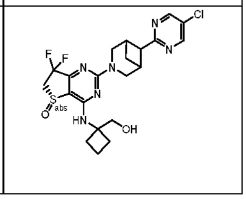 |

| | | | | |
|---|---|---|---|---|
| 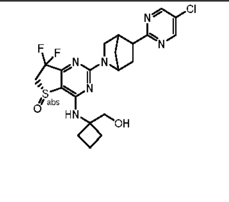 | 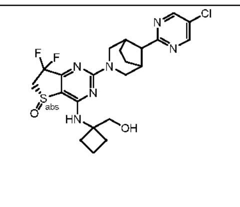 | 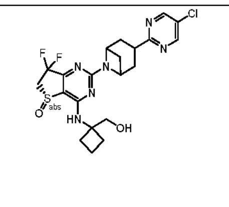 | 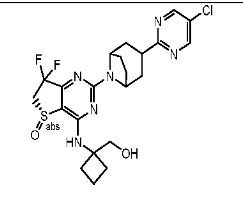 | 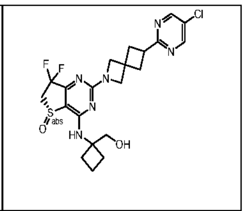 |
| 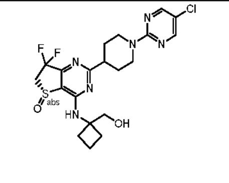 | 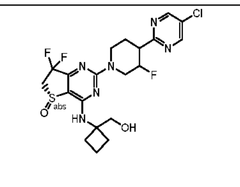 | 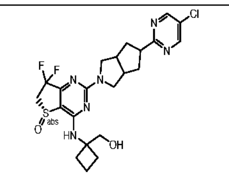 | 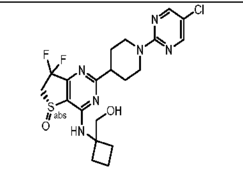 | 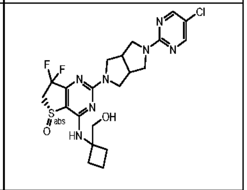 |
| 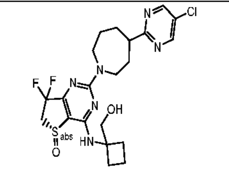 | 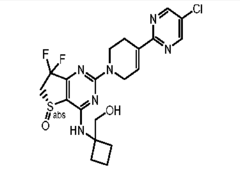 | 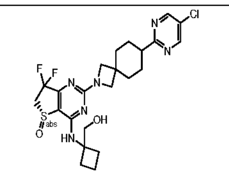 | 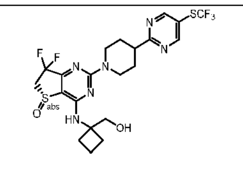 | 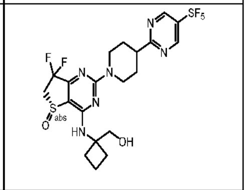 |
| 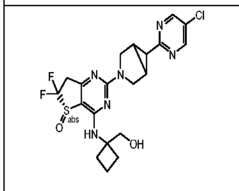 | 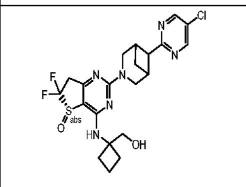 | 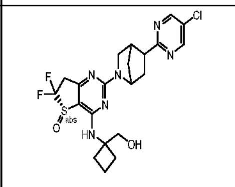 | 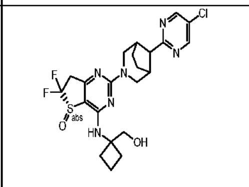 | 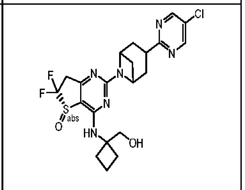 |
| 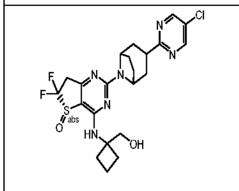 | 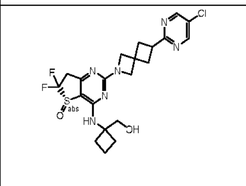 | 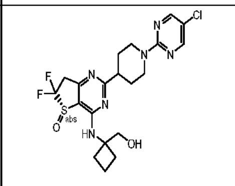 | 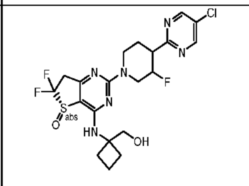 | 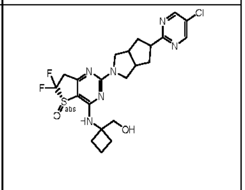 |
| 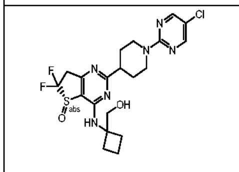 | 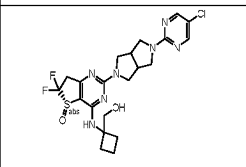 | 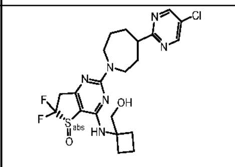 | 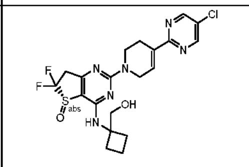 | 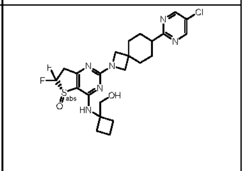 |
| 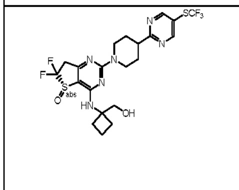 | 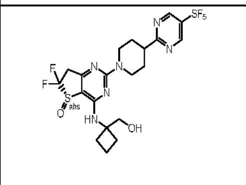 | 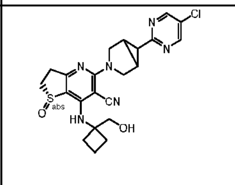 | 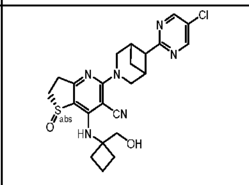 | 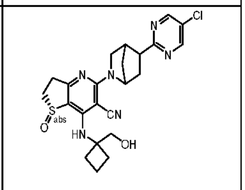 |
| 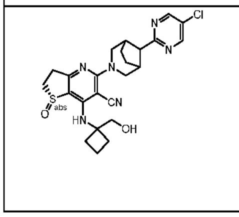 | 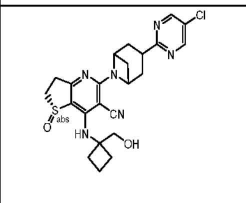 | 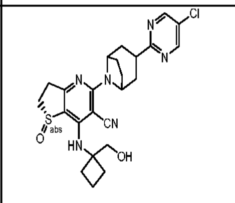 | 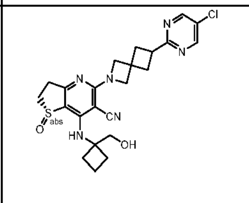 | 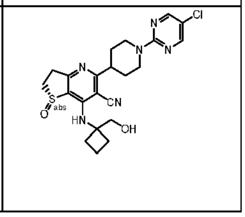 |

| | | | | |
|---|---|---|---|---|
| 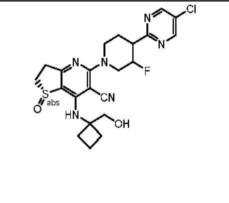 | 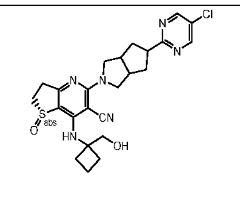 | 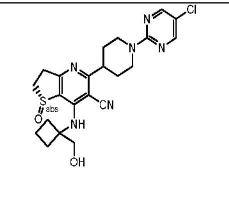 | 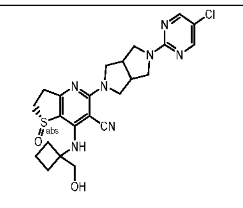 | 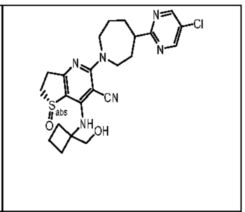 |
| 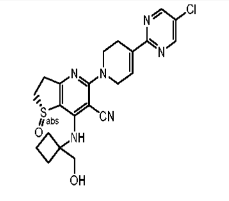 | 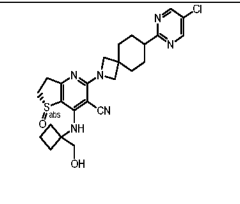 | 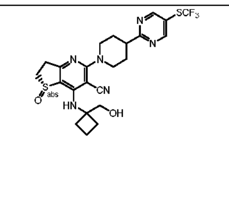 | 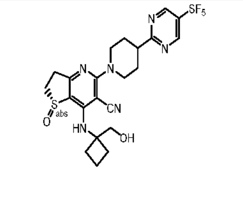 | 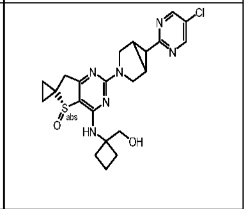 |
| 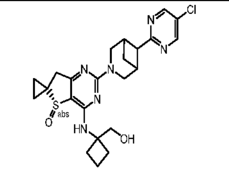 | 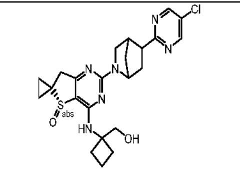 | 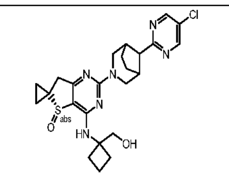 | 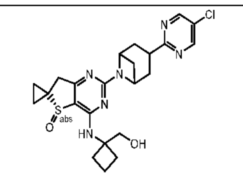 | 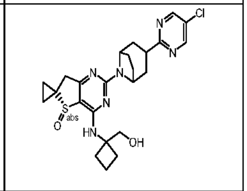 |
| 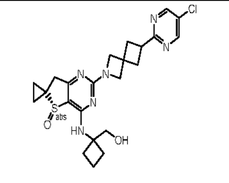 | 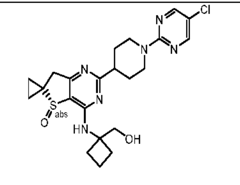 | 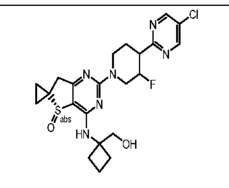 | 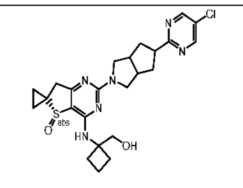 | 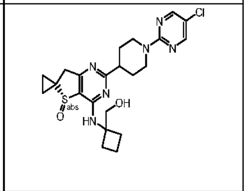 |
| 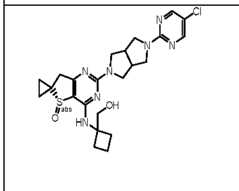 | 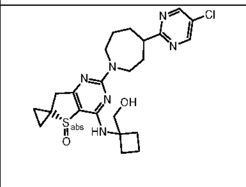 | 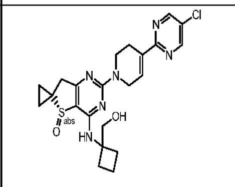 | 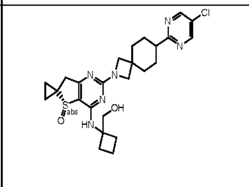 | 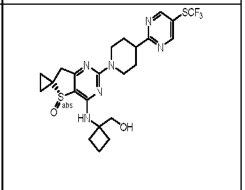 |
| 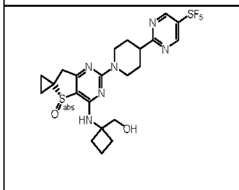 | 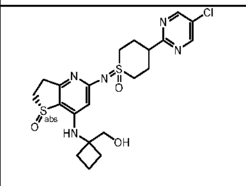 | 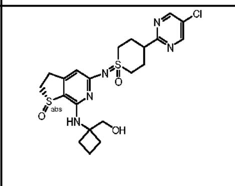 | 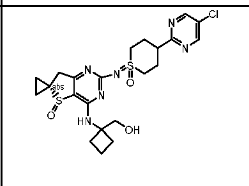 | 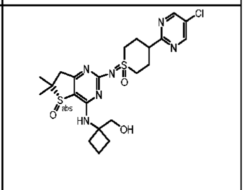 |
| 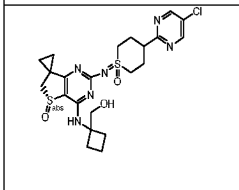 | 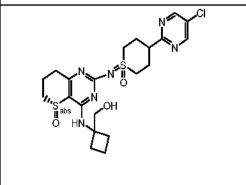 | 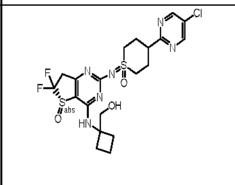 | 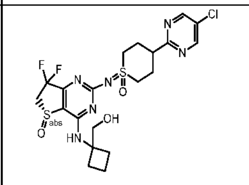 | 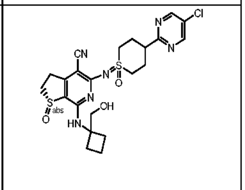 |
| 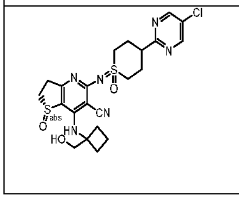 | 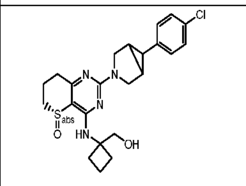 | 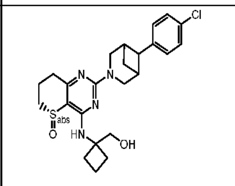 | 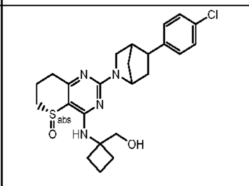 | 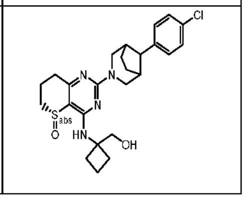 |

| | | | | |
|---|---|---|---|---|
| 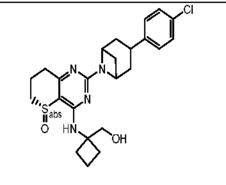 | 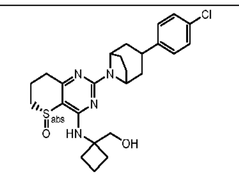 | 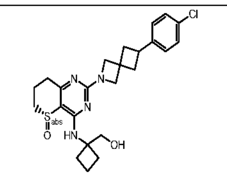 | 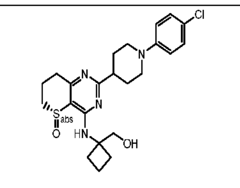 | 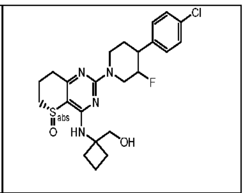 |
| 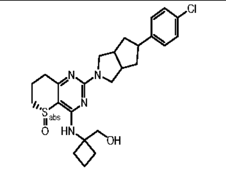 | 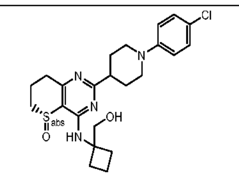 | 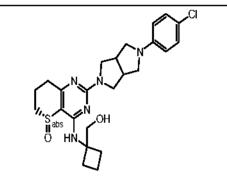 | 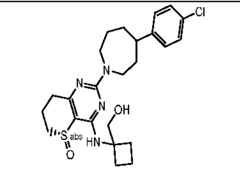 | 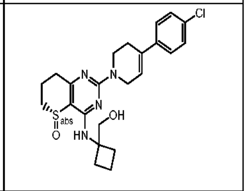 |
| 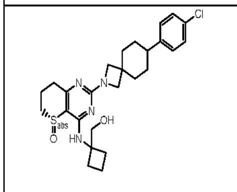 | 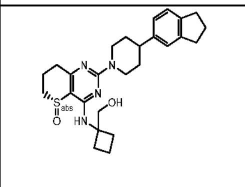 | 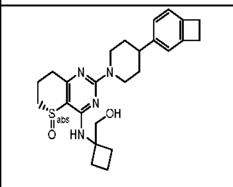 | 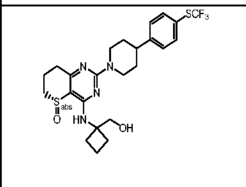 | 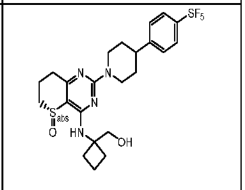 |
| 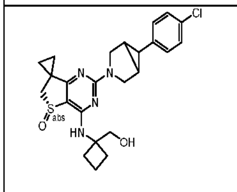 | 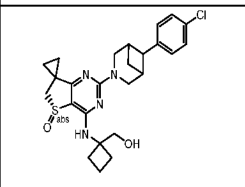 | 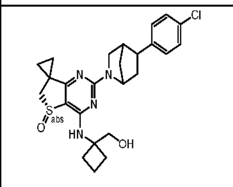 | 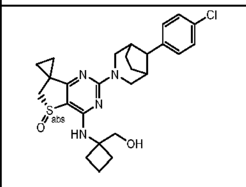 | 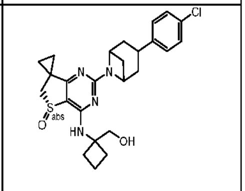 |
| 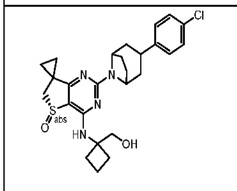 | 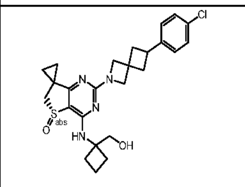 | 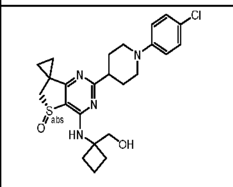 | 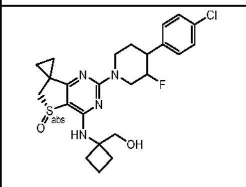 | 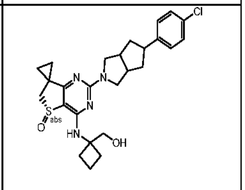 |
| 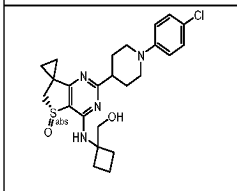 | 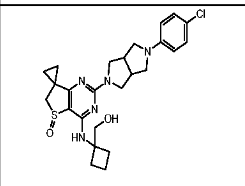 | 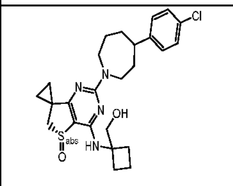 | 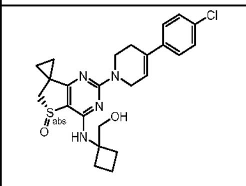 | 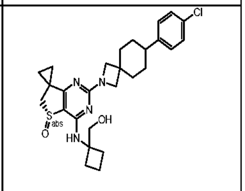 |
| 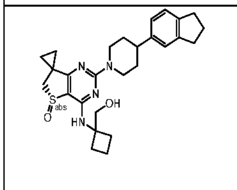 | 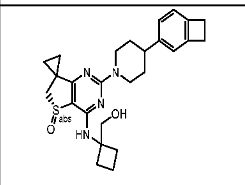 | 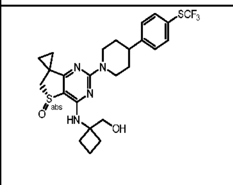 | 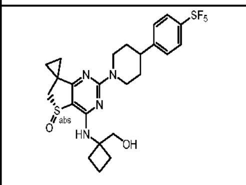 | 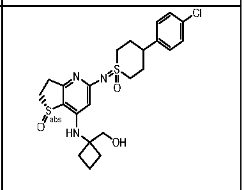 |
| 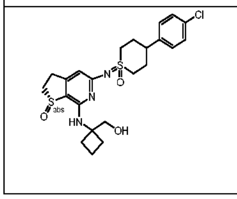 | 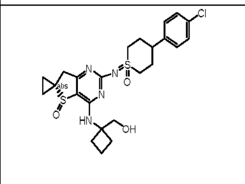 | 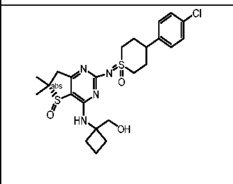 | 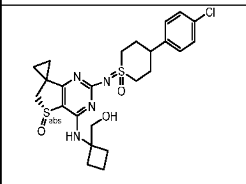 | 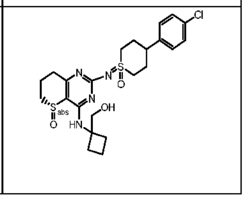 |

| | | | | |
|---|---|---|---|---|
| | | | | |
| | | | | |
| | | | | |
| | | | | |
| | | | | |
| 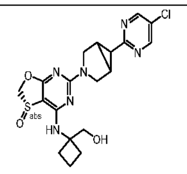 | 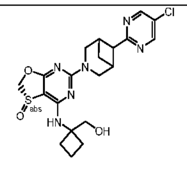 | 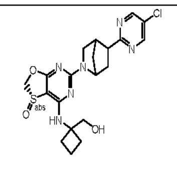 | 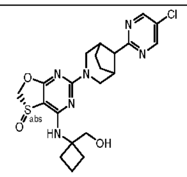 | 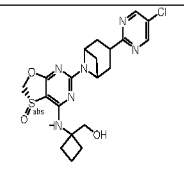 |
| 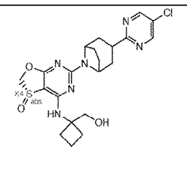 | 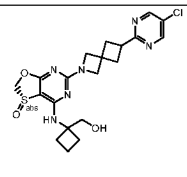 | 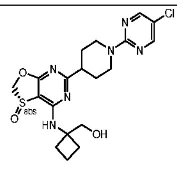 | 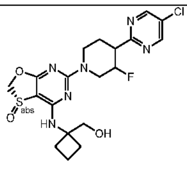 | 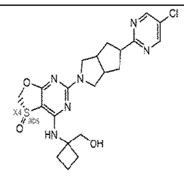 |
| 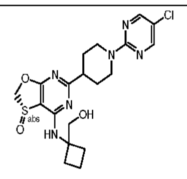 | 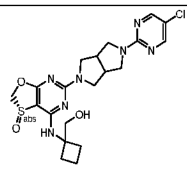 | 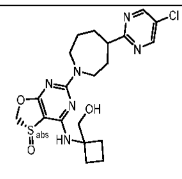 | 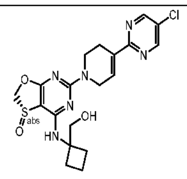 | 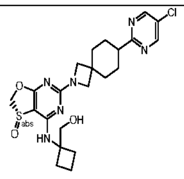 |
| 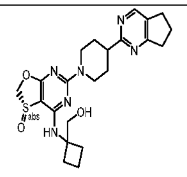 | 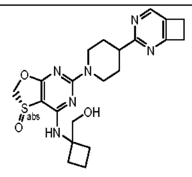 | 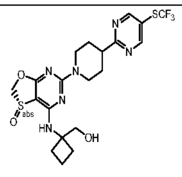 | 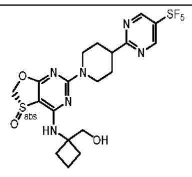 | 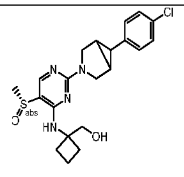 |

| | | | | |
|---|---|---|---|---|
| 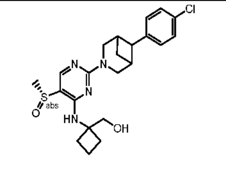 | 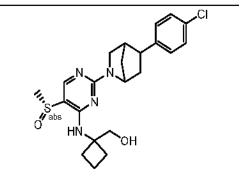 | 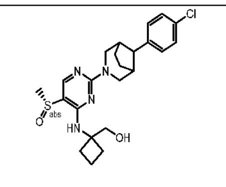 | 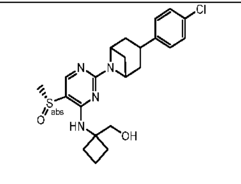 | 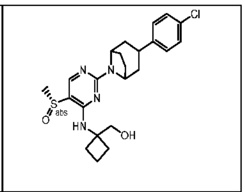 |
| 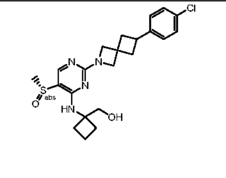 | 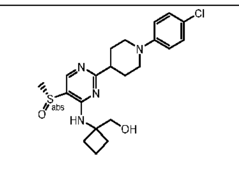 | 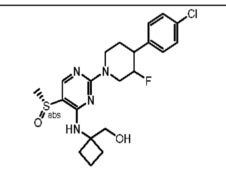 | 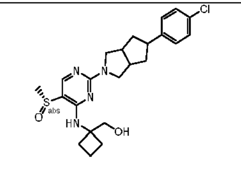 | 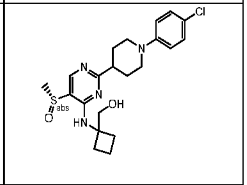 |
| 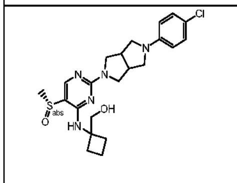 | 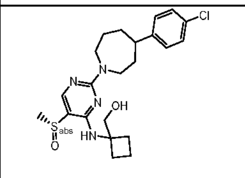 | 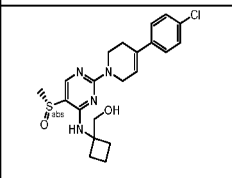 | 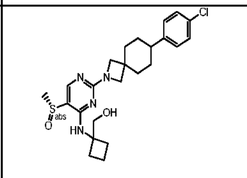 | 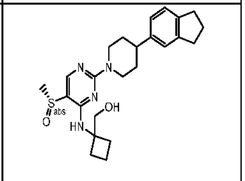 |
| 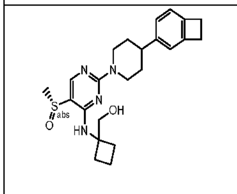 | 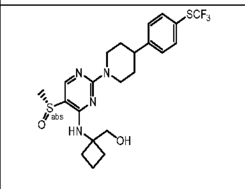 | 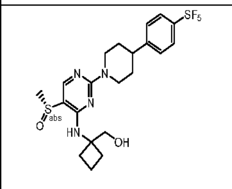 | 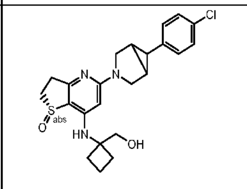 | 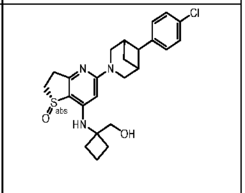 |
| 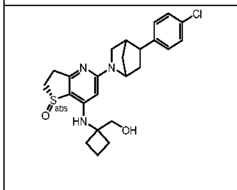 | 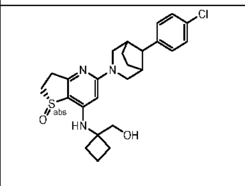 | 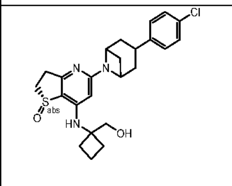 | 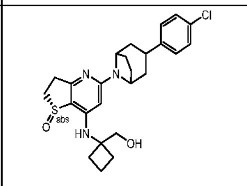 | 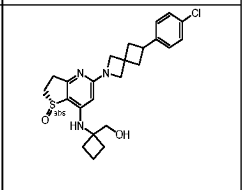 |
| 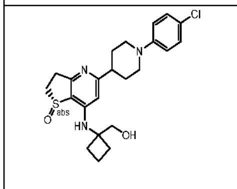 | 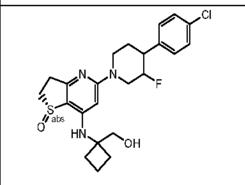 | 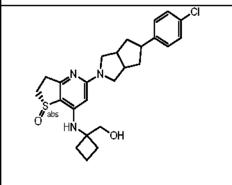 | 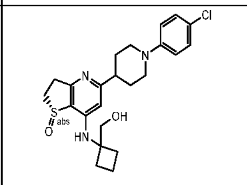 | 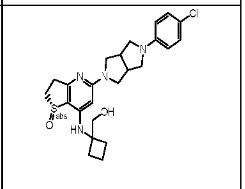 |
| 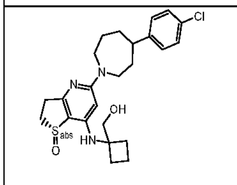 | 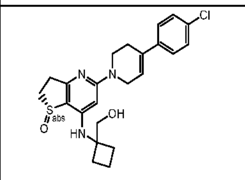 | 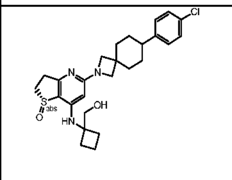 | 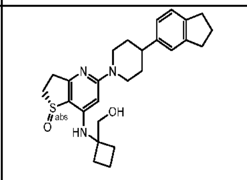 | 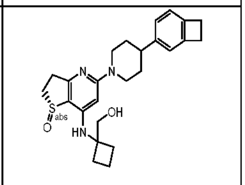 |
| 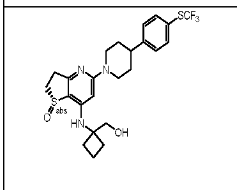 | 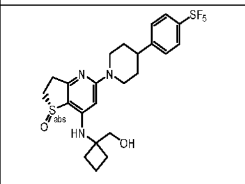 | 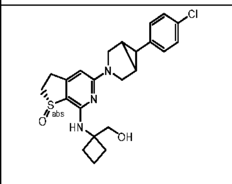 | 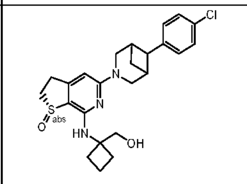 | 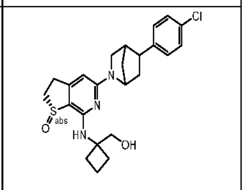 |
| | | | | |

| | | | | |
|---|---|---|---|---|
| 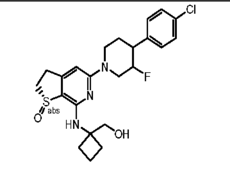 | 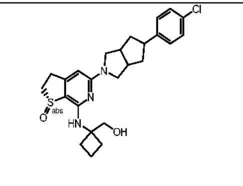 | 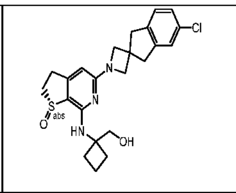 | 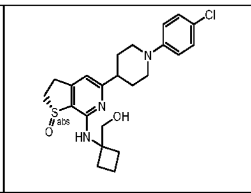 | 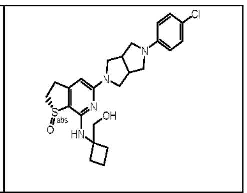 |
| 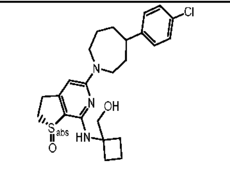 | 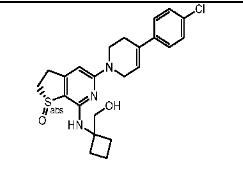 | 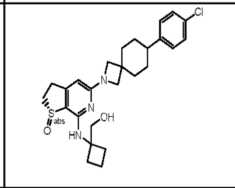 | 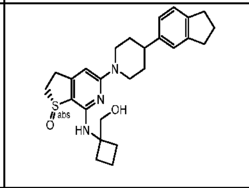 | 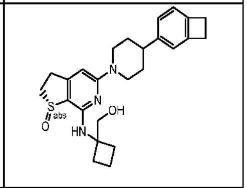 |
| 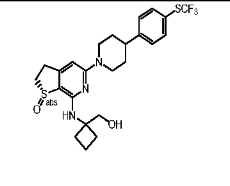 | 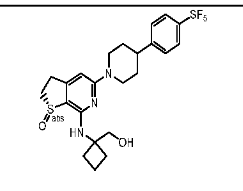 | 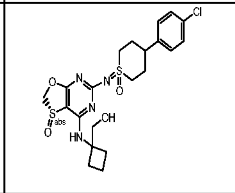 | 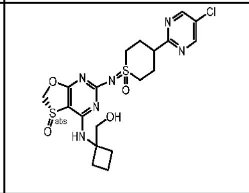 | 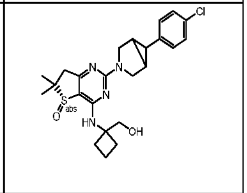 |
| 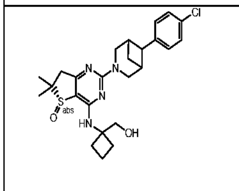 | 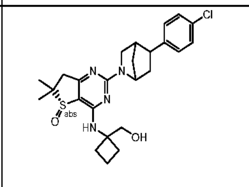 | 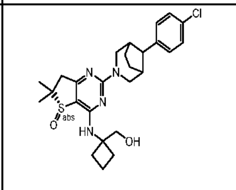 | 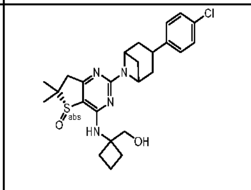 | 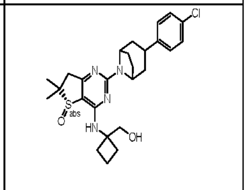 |
| 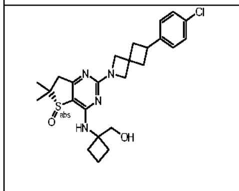 | 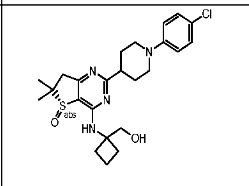 | 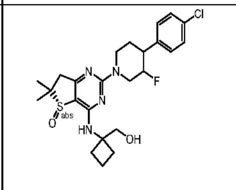 | 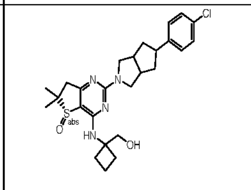 | 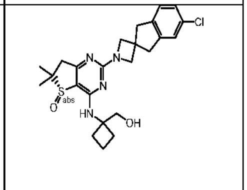 |
| 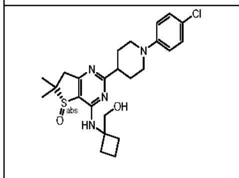 | 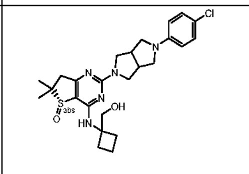 | 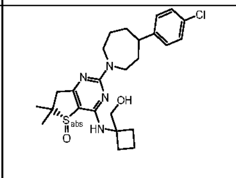 | 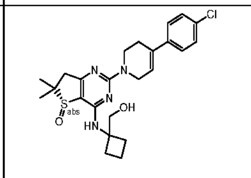 | 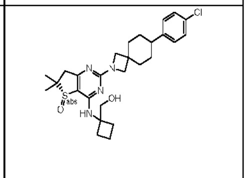 |
| 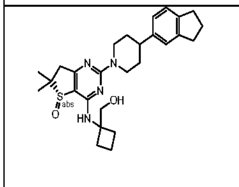 | 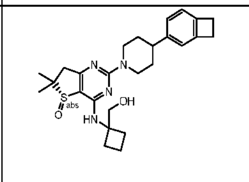 | 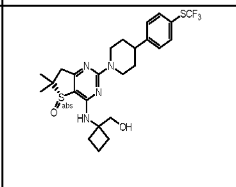 | 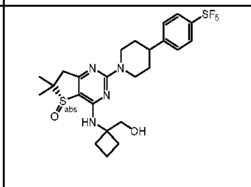 | 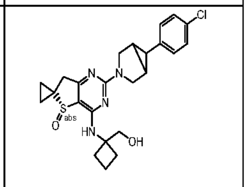 |
| 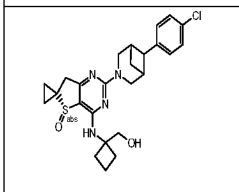 | 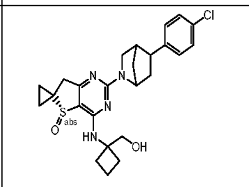 | 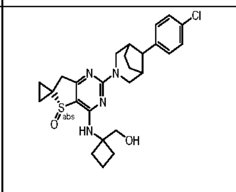 | 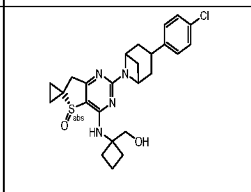 | 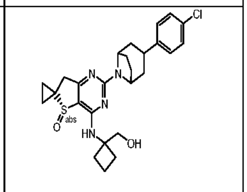 |
| | | | | |

| | | | | |
|---|---|---|---|---|
| 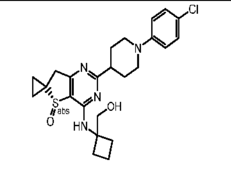 | 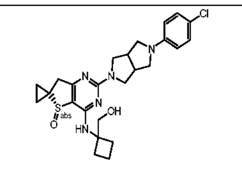 | 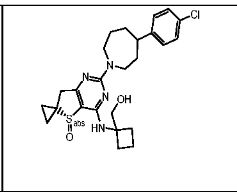 | 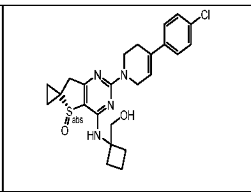 | 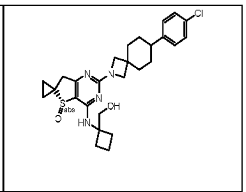 |
| 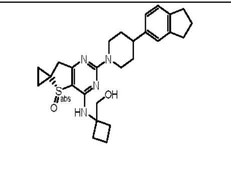 | 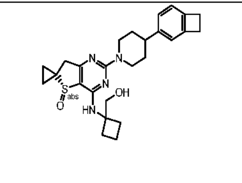 | 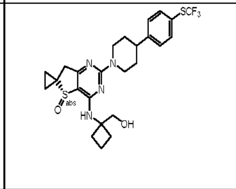 | 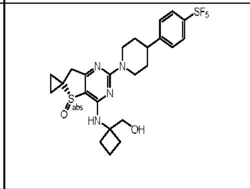 | 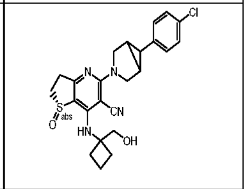 |
| 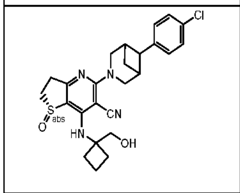 | 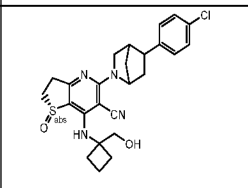 | 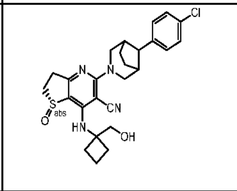 | 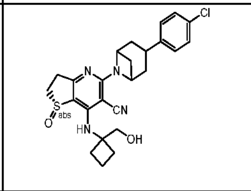 | 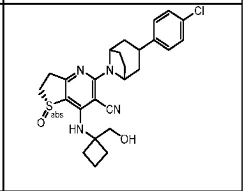 |
| 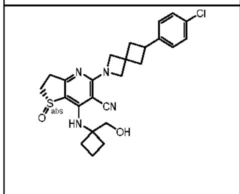 | 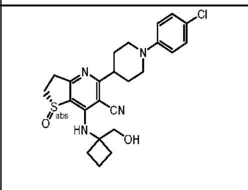 | 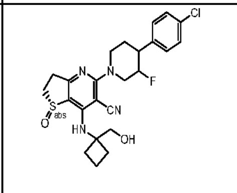 | 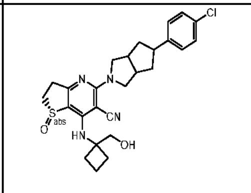 | 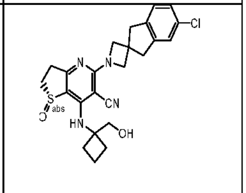 |
| 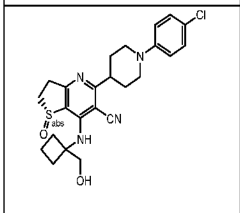 | 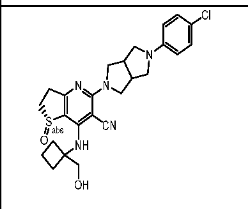 | 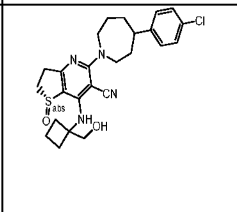 | 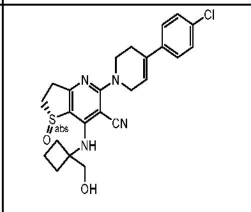 | 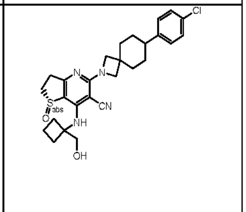 |
| 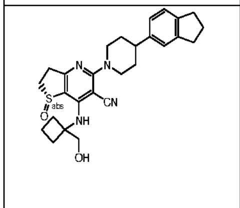 | 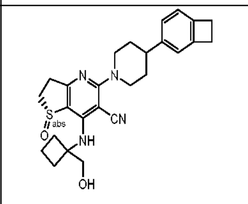 | 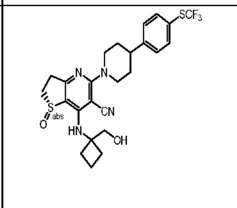 | 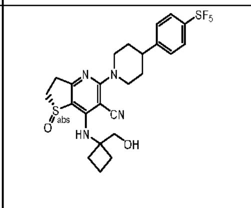 | 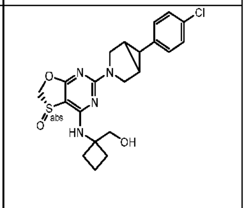 |
| 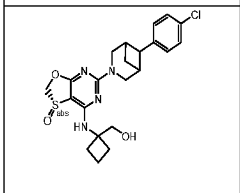 | 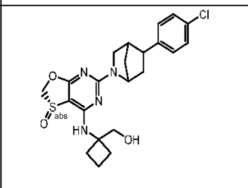 | 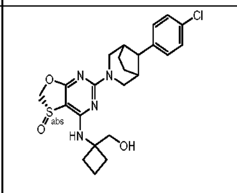 | 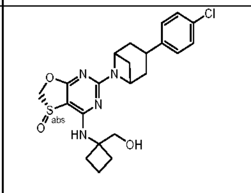 | 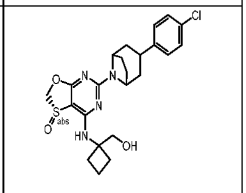 |
| 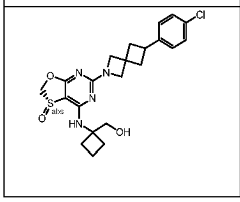 | 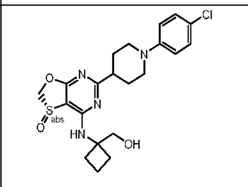 | 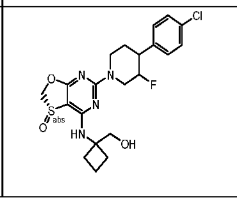 | 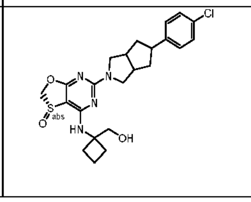 | 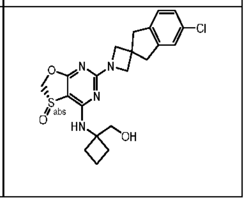 |

| | | | | |
|---|---|---|---|---|
| 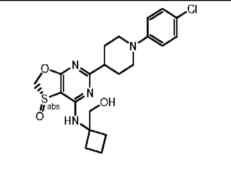 | 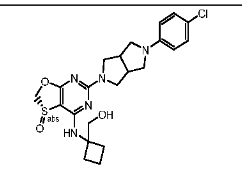 | 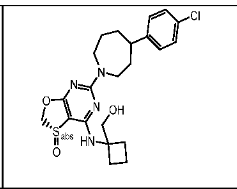 | 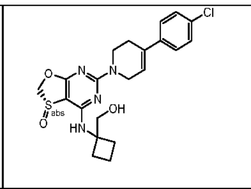 | 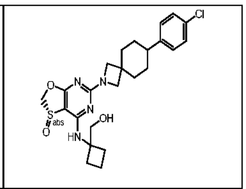 |
| 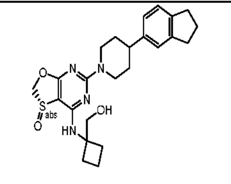 | 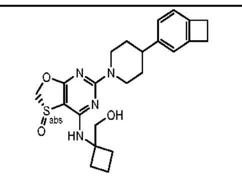 | 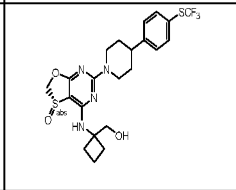 | 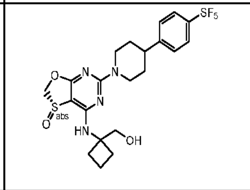 | 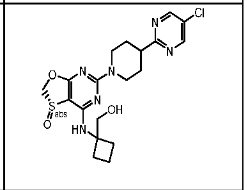 |
| 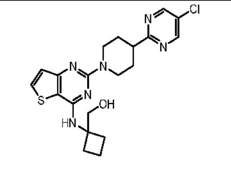 | 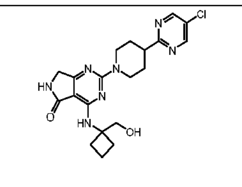 | 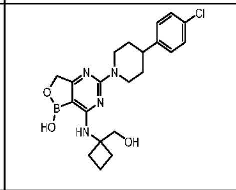 | 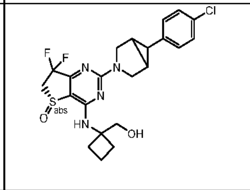 | 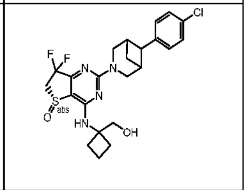 |
| 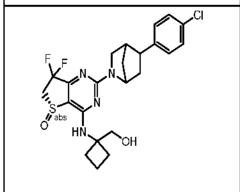 | 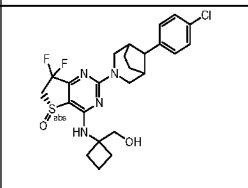 | 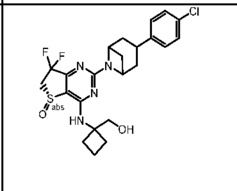 | 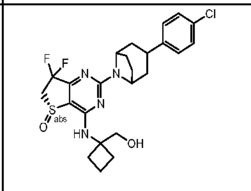 | 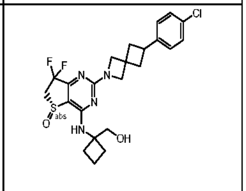 |
| 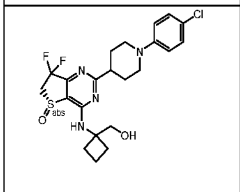 | 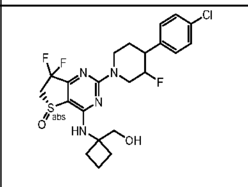 | 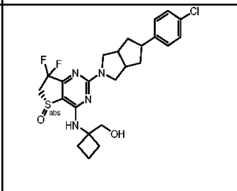 | 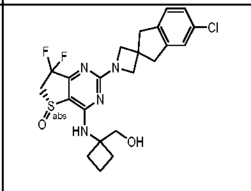 | 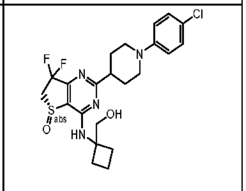 |
| 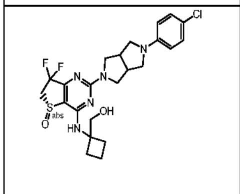 | 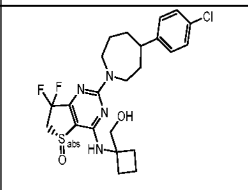 | 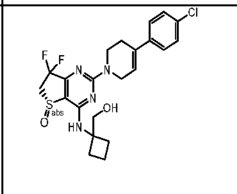 | 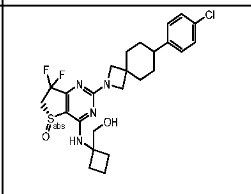 | 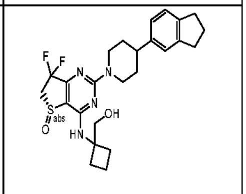 |
| 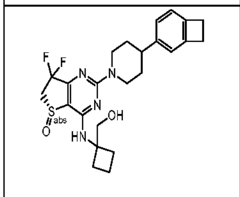 | 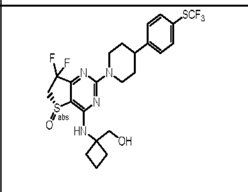 | 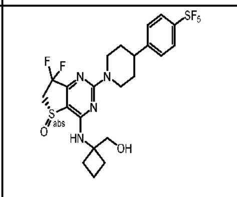 | 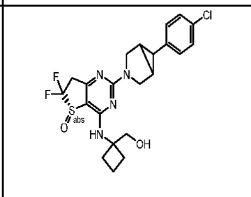 | 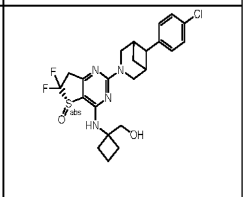 |
| 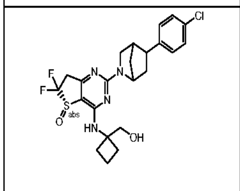 | 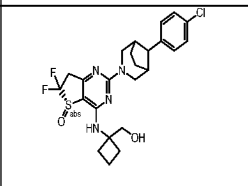 | 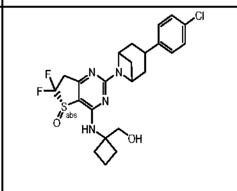 | 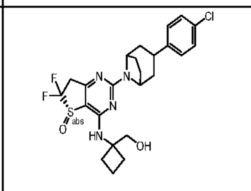 | 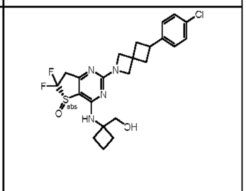 |
| | | | | |

| | | | | |
|---|---|---|---|---|
| 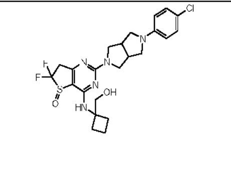 | 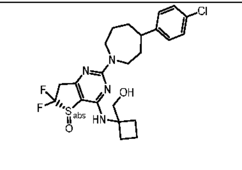 | 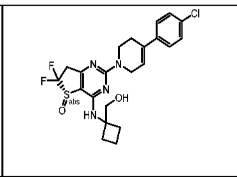 | 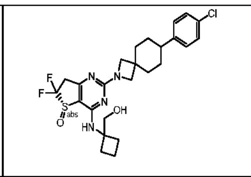 | 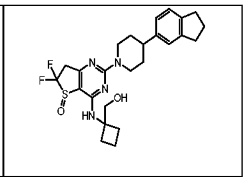 |
| 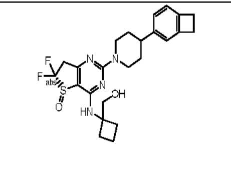 | 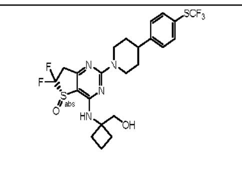 | 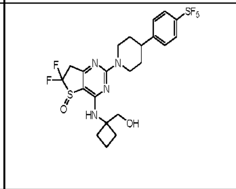 | 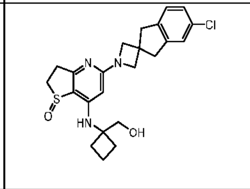 | 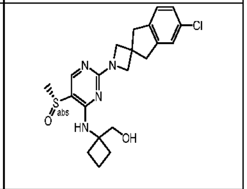 |
| 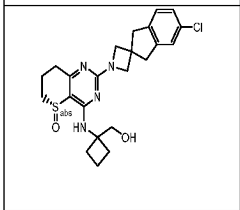 | 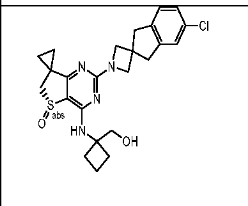 | 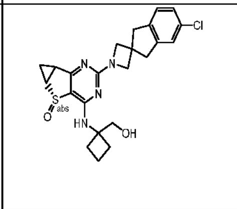 | 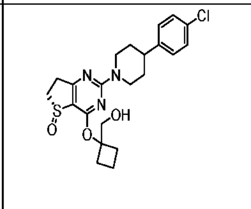 | 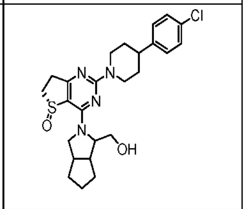 |
| 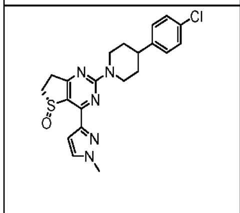 | 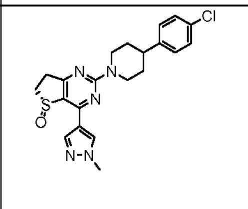 | 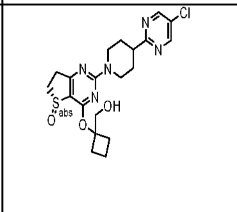 | 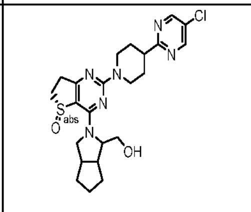 | 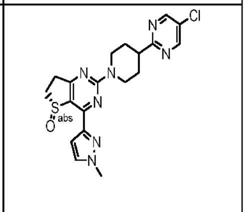 |
| 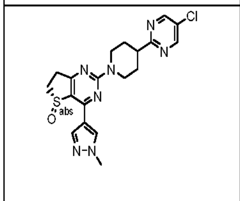 | 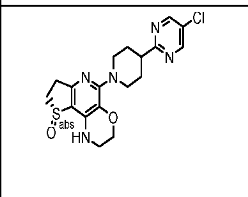 | 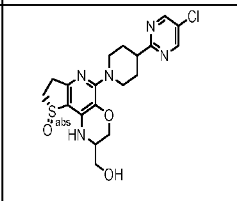 | 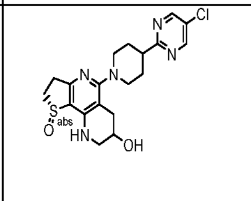 | 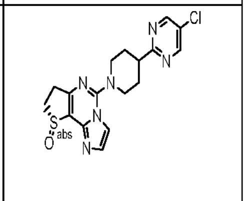 |
| 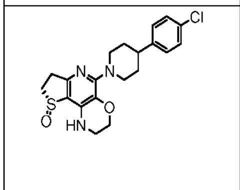 | 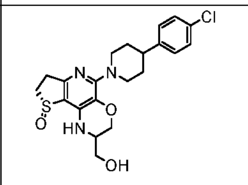 | 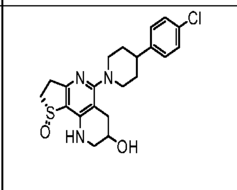 | 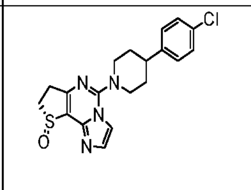 | 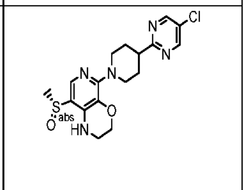 |
| 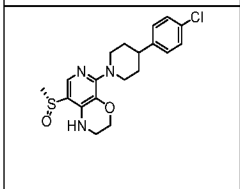 | 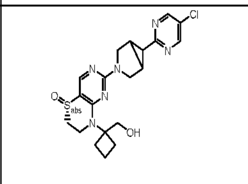 | 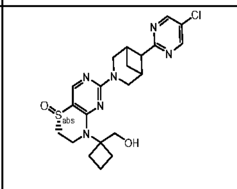 | 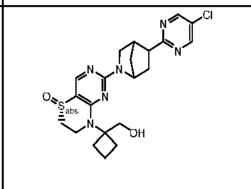 | 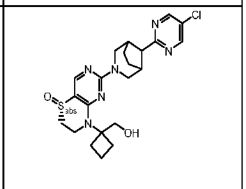 |
| 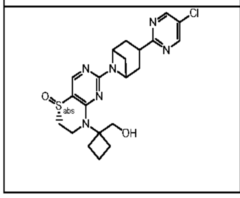 | 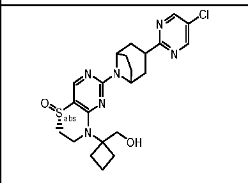 | 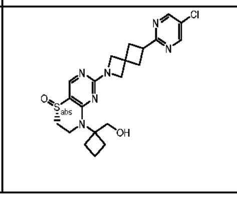 | 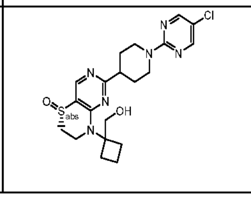 | 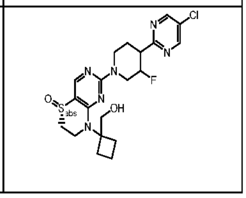 |

| | | | | |
|---|---|---|---|---|
| 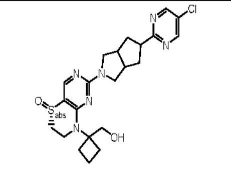 | 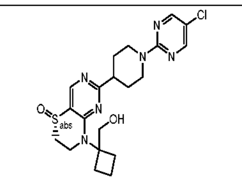 | 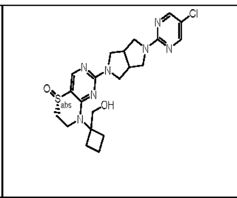 | 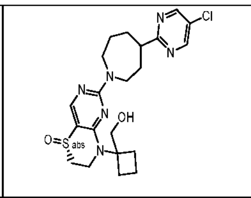 | 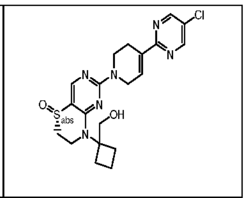 |
| 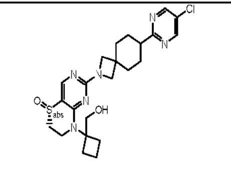 | 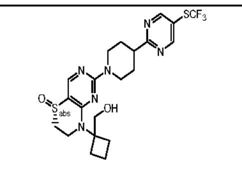 | 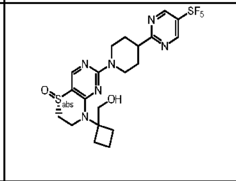 | 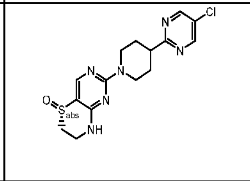 | 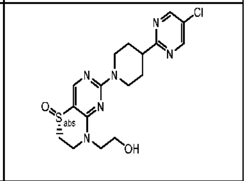 |
| 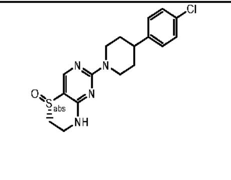 | 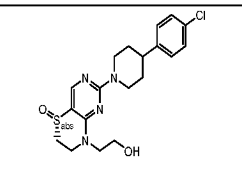 | 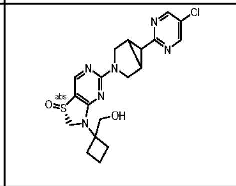 | 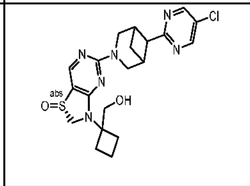 | 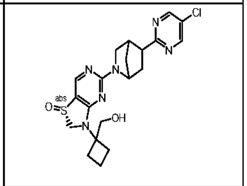 |
| 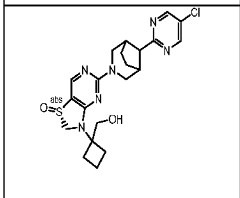 | 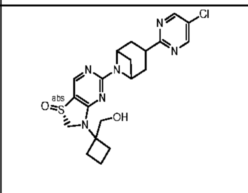 | 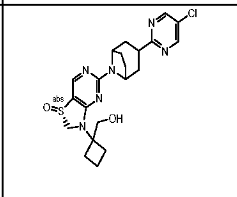 | 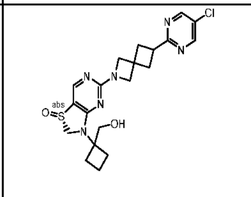 | 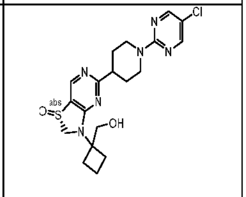 |
| 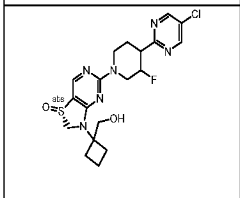 | 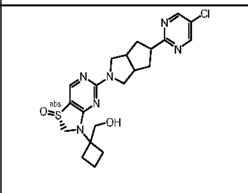 | 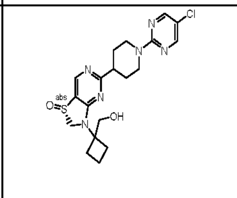 | 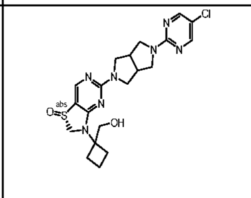 | 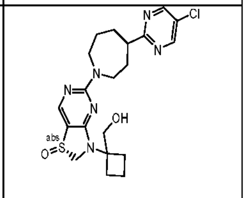 |
| 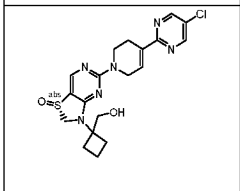 | 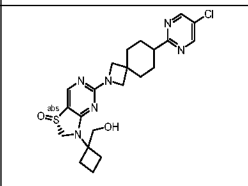 | 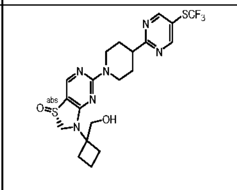 | 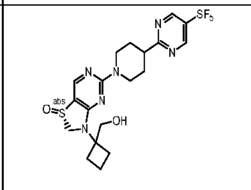 | 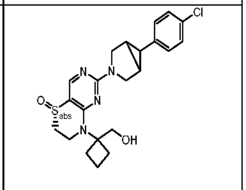 |
| 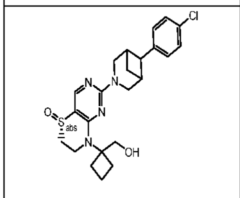 | 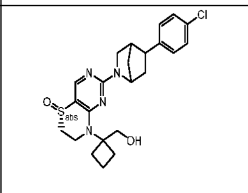 | 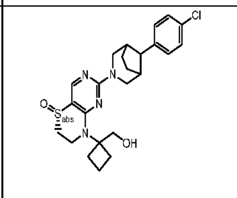 | 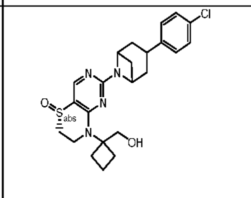 | 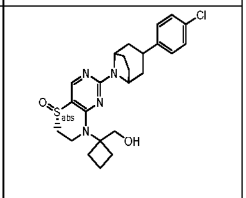 |
| 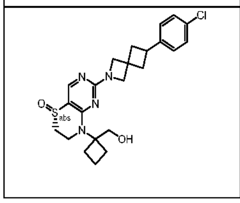 | 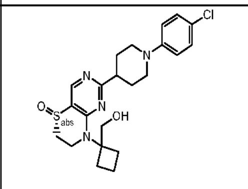 | 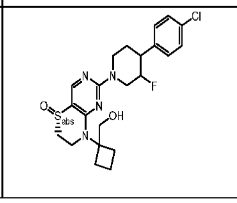 | 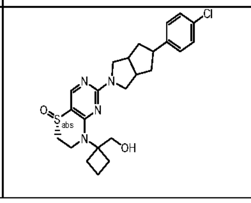 | 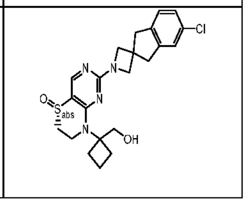 |

| | | | | |
|---|---|---|---|---|
| 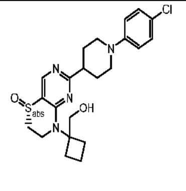 | 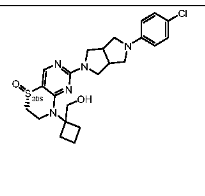 | 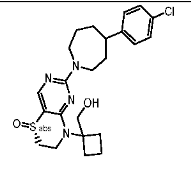 | 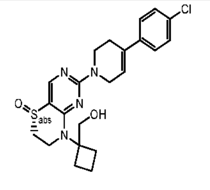 | 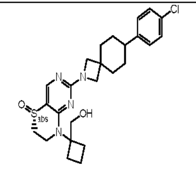 |
| 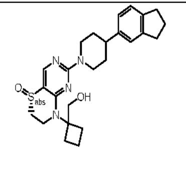 | 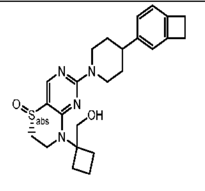 | 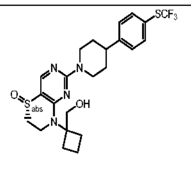 | 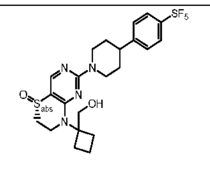 | 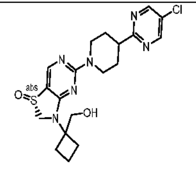 |
| 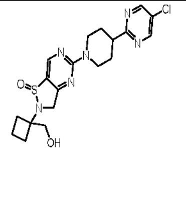 | 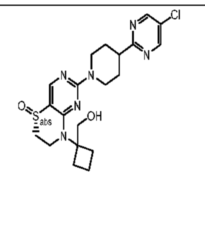 | 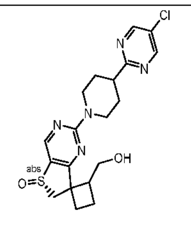 | 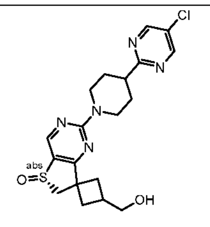 | 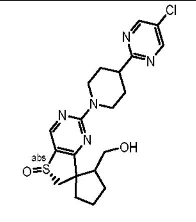 |
| 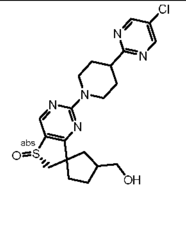 | 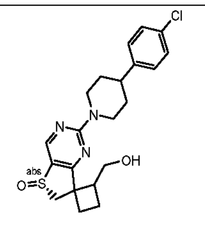 | 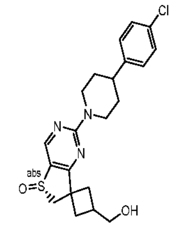 | 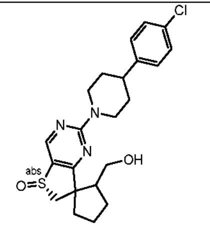 | 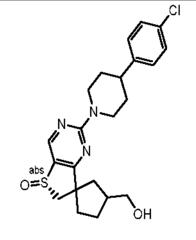 |
| 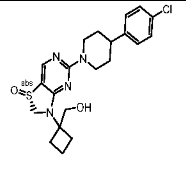 | 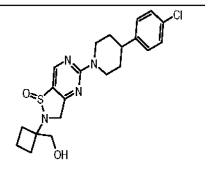 | 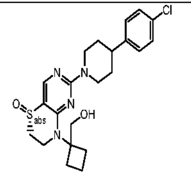 | 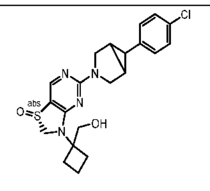 | 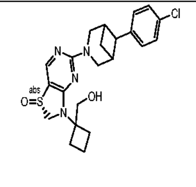 |
| 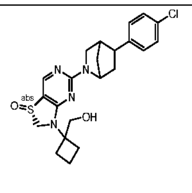 | 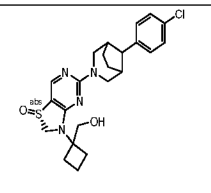 | 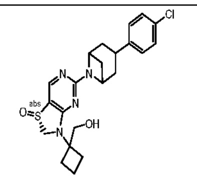 | 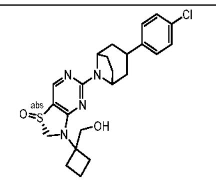 | 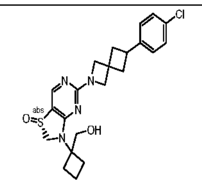 |
| 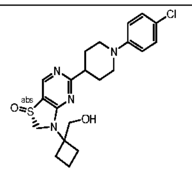 | 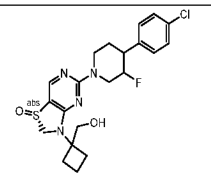 | 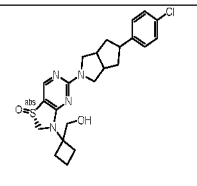 | 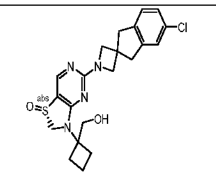 | 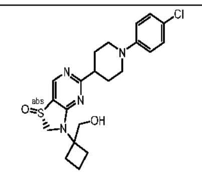 |
| 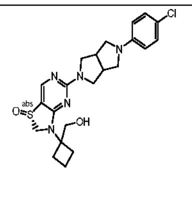 | 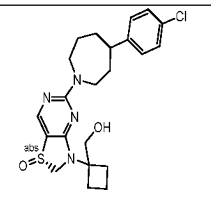 | 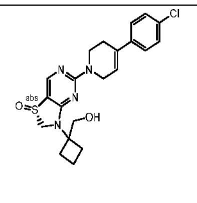 | 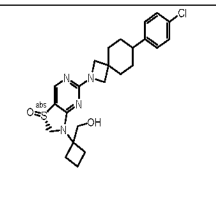 | 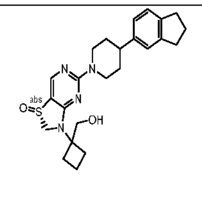 |

| | | | | |
|---|---|---|---|---|
| 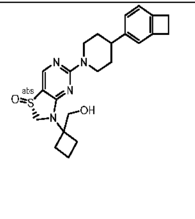 | 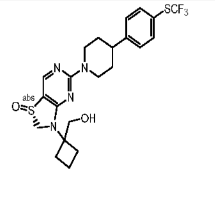 | 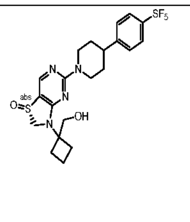 | 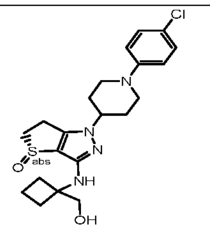 | 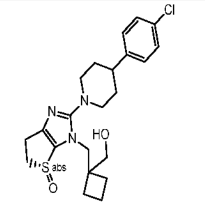 |
| 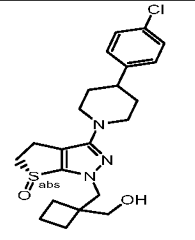 | 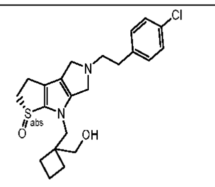 | 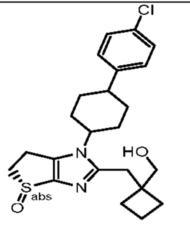 | 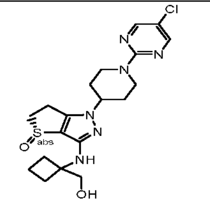 | 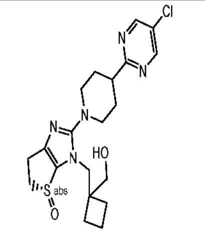 |
| 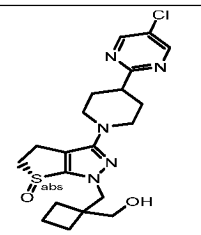 | 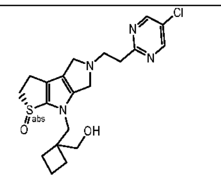 | 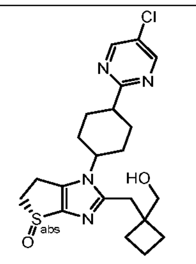 | 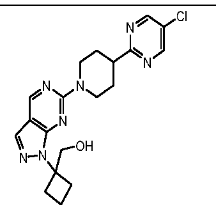 | 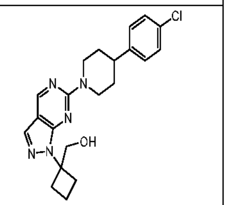 |
| 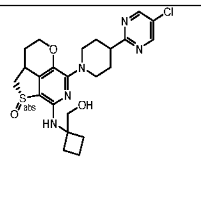 | 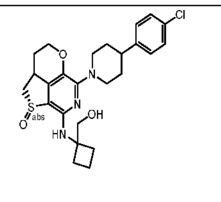 | 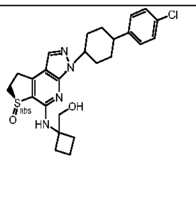 | 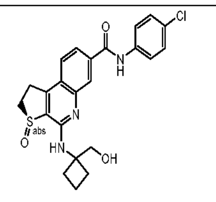 | 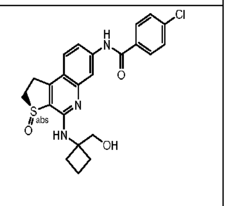 |
| 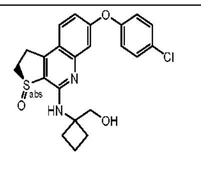 | 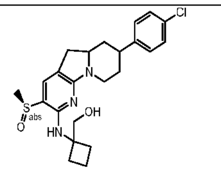 | 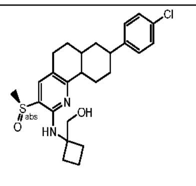 | 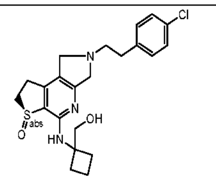 | 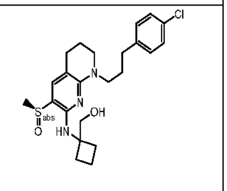 |
| 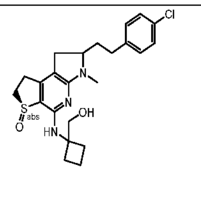 | 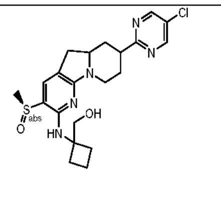 | 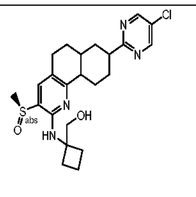 | 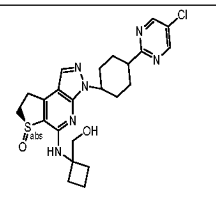 | 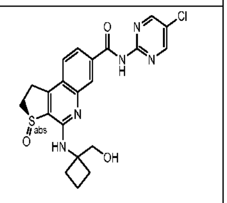 |
| 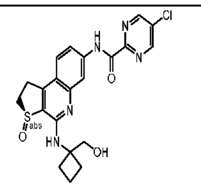 | 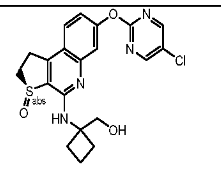 | 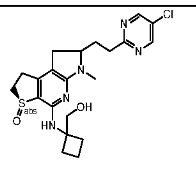 | 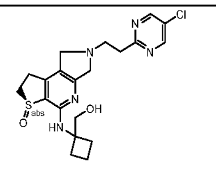 | 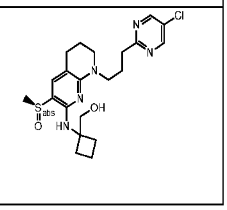 |

| | | | | |
|---|---|---|---|---|
| 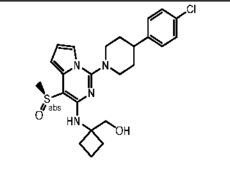 | 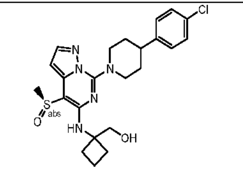 | 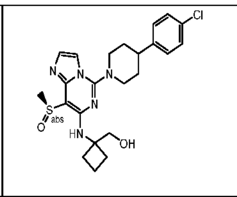 | 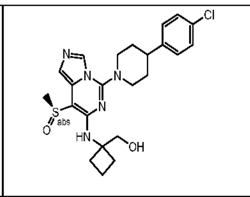 | 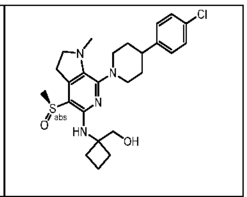 |
| 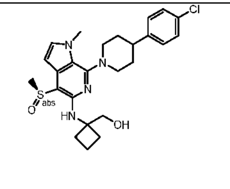 | 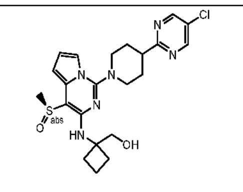 | 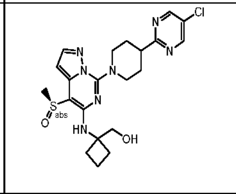 | 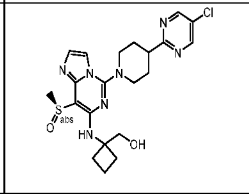 | 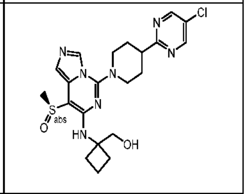 |
| 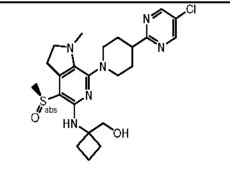 | 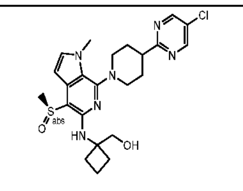 | 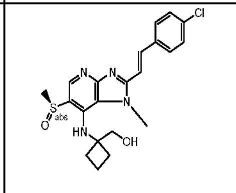 | 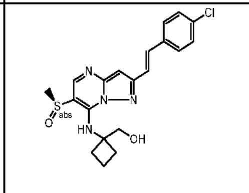 | 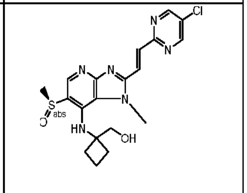 |
| 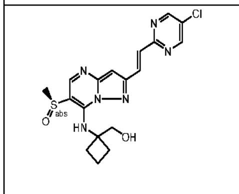 | 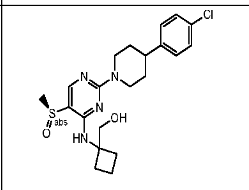 | 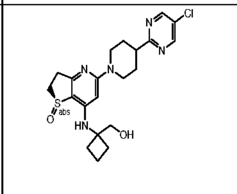 | 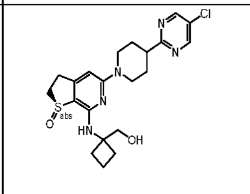 | 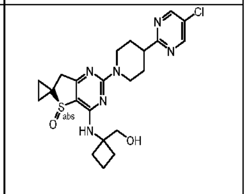 |
| 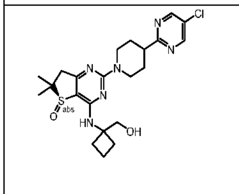 | 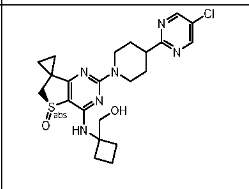 | 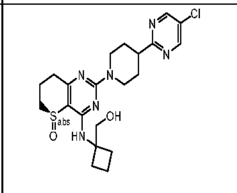 | 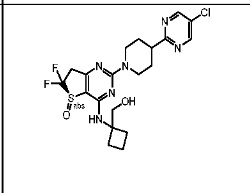 | 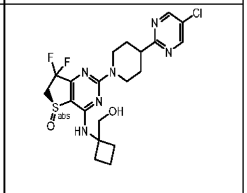 |
| 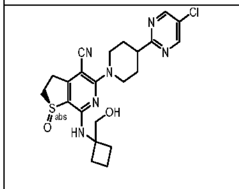 | 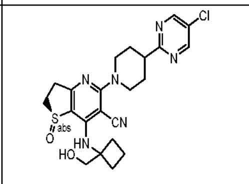 | 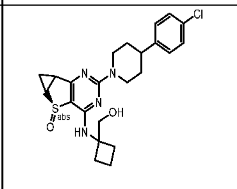 | 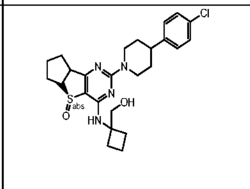 | 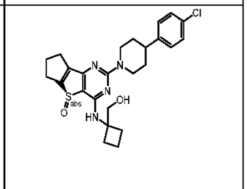 |
| 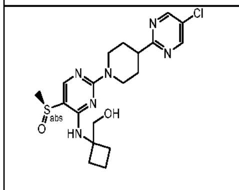 | 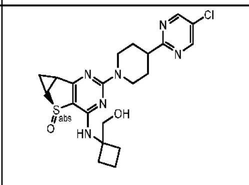 | 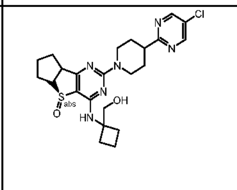 | 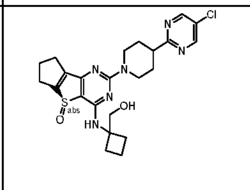 | 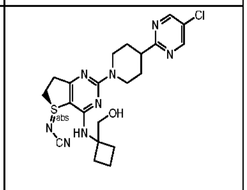 |
| 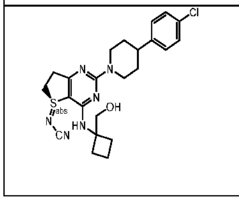 | 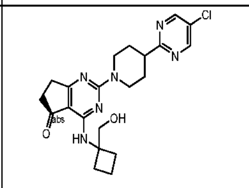 | 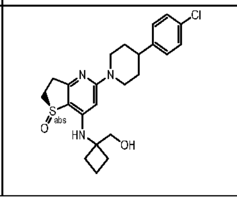 | 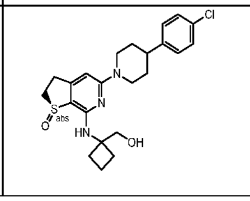 | 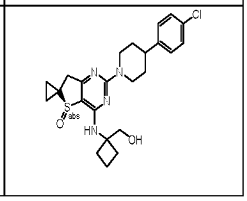 |

| | | | | |
|---|---|---|---|---|
| 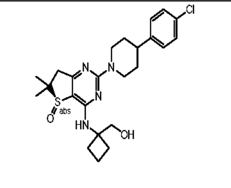 | 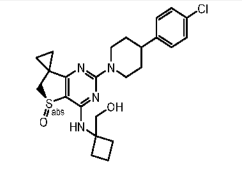 | 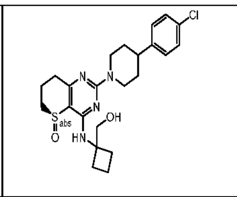 | 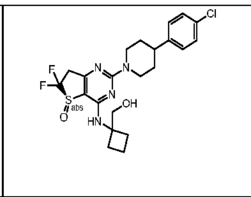 | 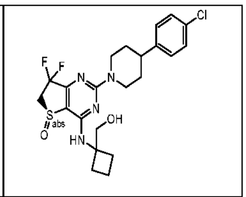 |
| 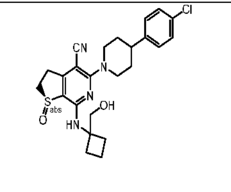 | 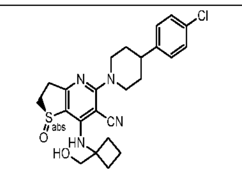 | 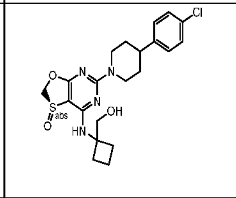 | 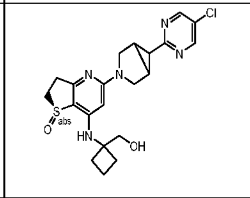 | 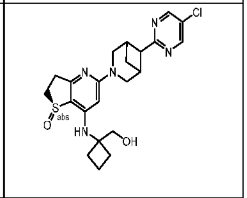 |
| 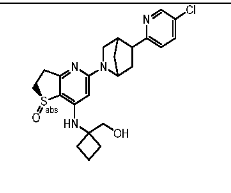 | 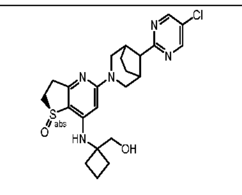 | 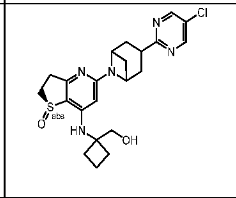 | 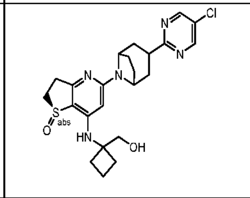 | 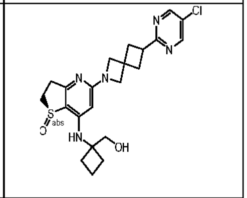 |
| 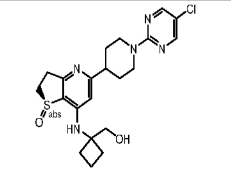 | 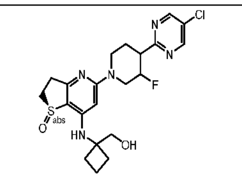 | 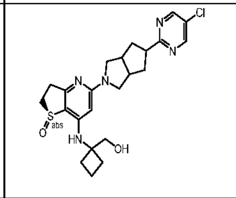 | 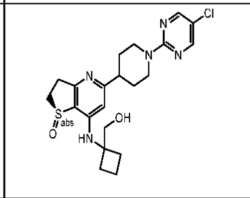 | 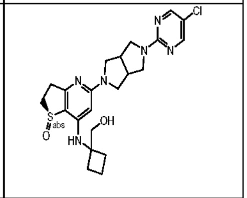 |
| 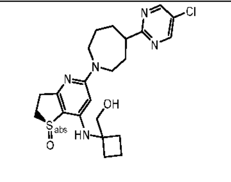 | 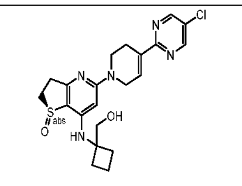 | 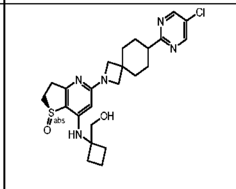 | 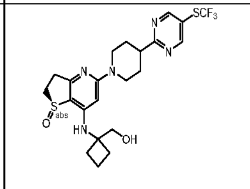 | 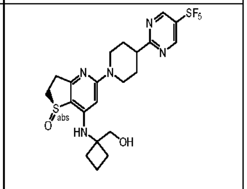 |
| 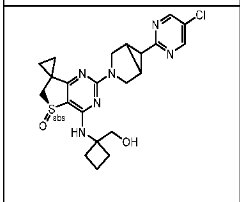 | 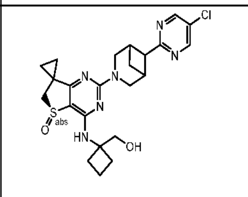 | 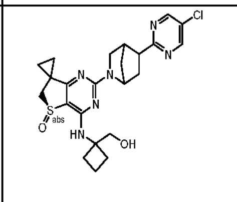 | 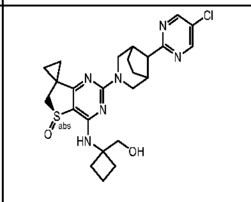 | 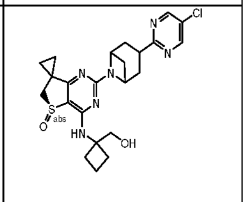 |
| 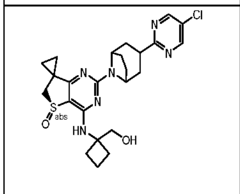 | 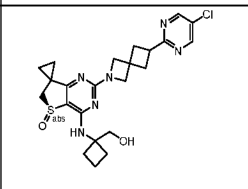 | 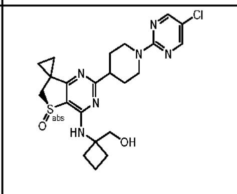 | 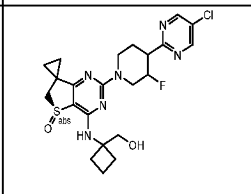 | 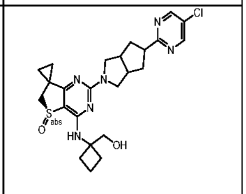 |
| 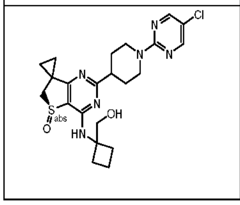 | 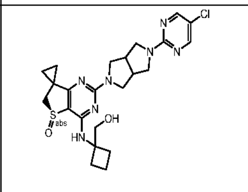 | 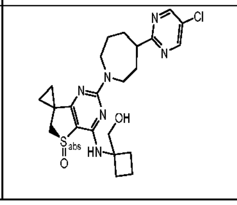 | 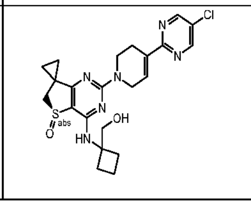 | 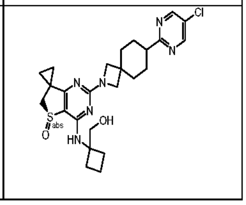 |

| | | | | |
|---|---|---|---|---|
| 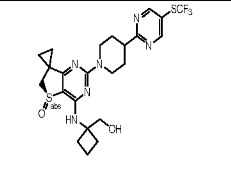 | 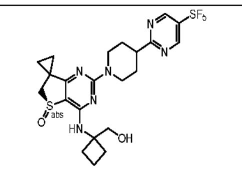 | 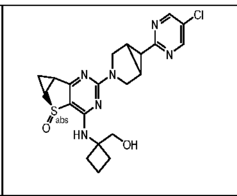 | 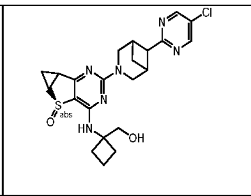 | 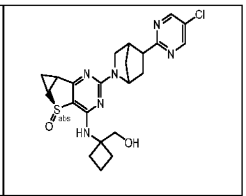 |
| 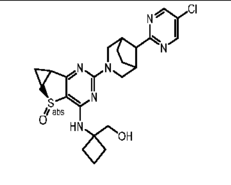 | 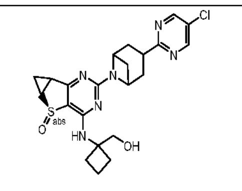 | 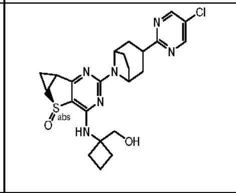 | 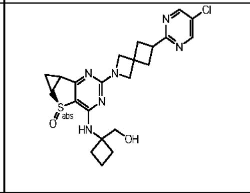 | 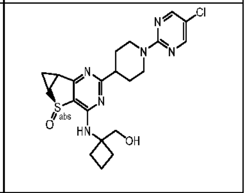 |
| 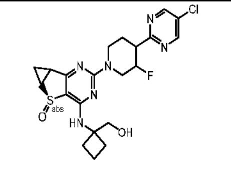 | 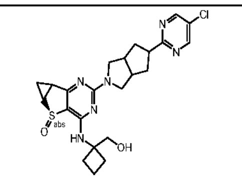 | 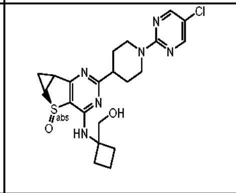 | 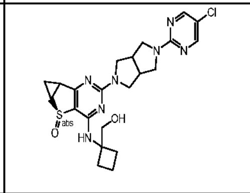 | 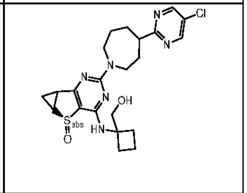 |
| 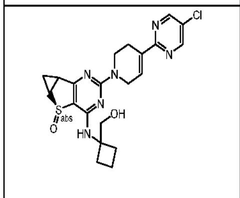 | 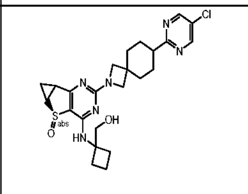 | 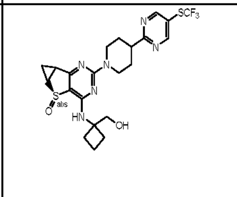 | 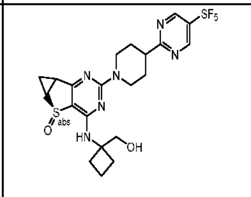 | 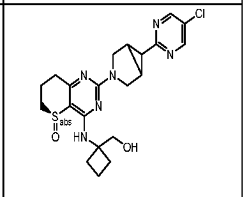 |
| 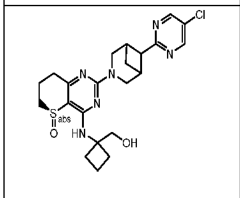 | 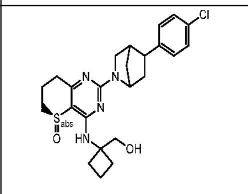 | 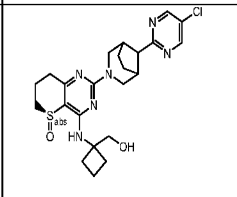 | 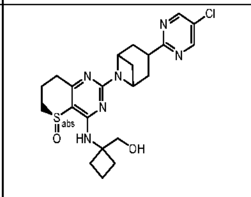 | 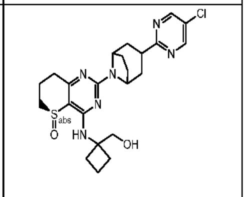 |
| 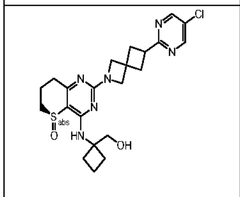 | 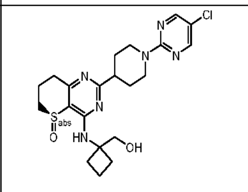 | 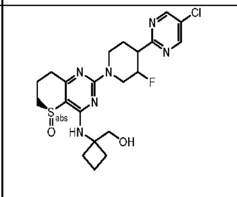 | 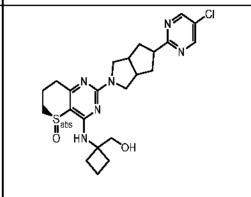 | 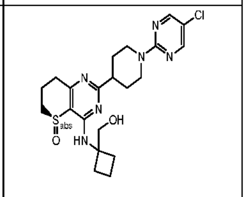 |
| 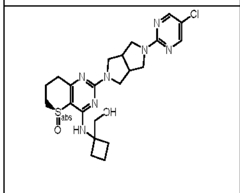 | 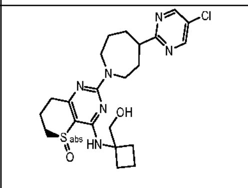 | 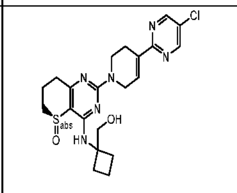 | 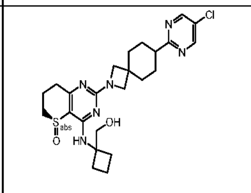 | 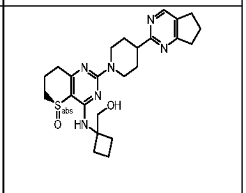 |
| 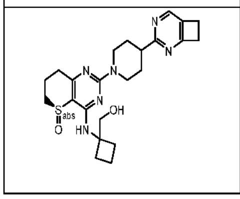 | 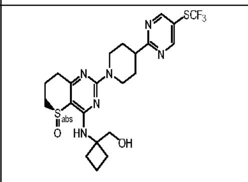 | 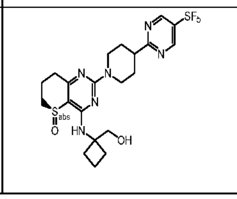 | 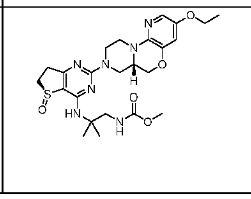 | 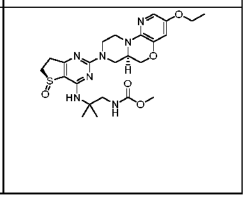 |

| | | | | |
|---|---|---|---|---|
| 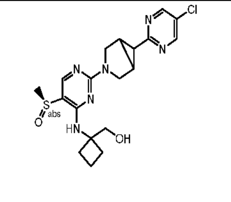 | 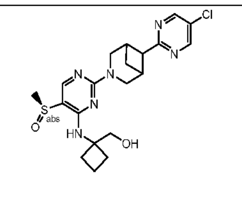 | 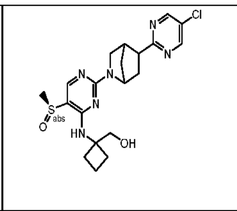 | 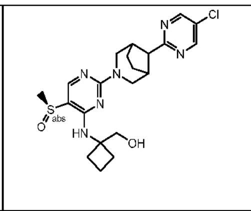 | 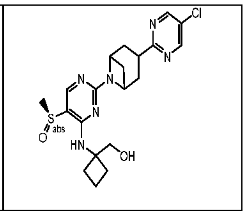 |
| 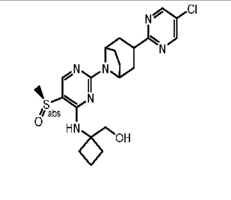 | 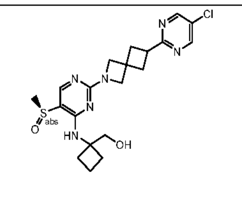 | 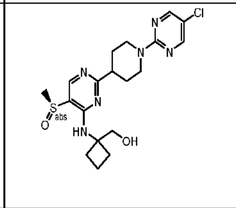 | 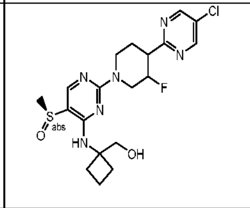 | 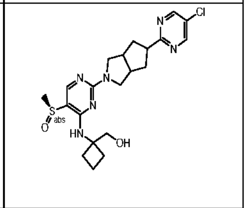 |
| 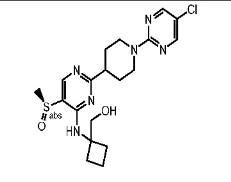 | 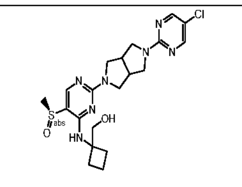 | 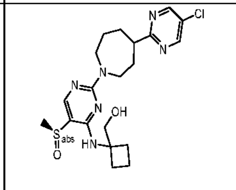 | 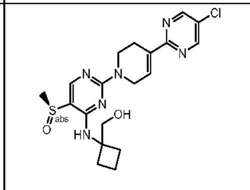 | 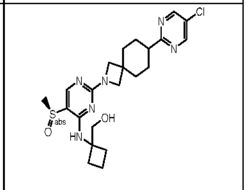 |
| 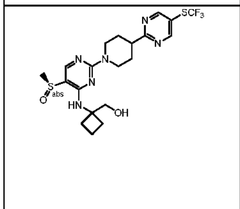 | 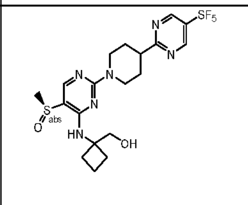 | 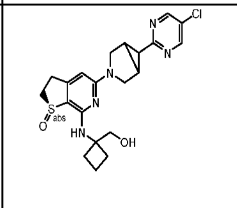 | 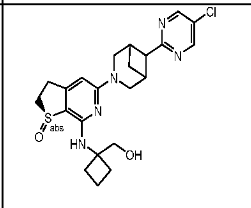 | 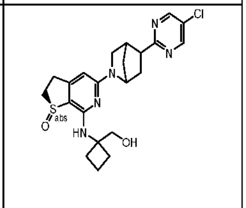 |
| 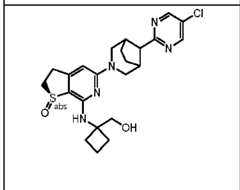 | 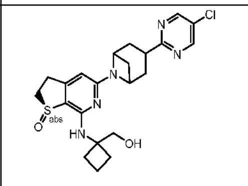 | 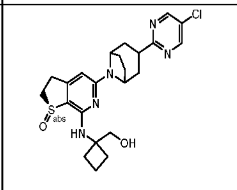 | 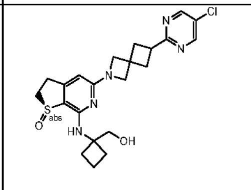 | 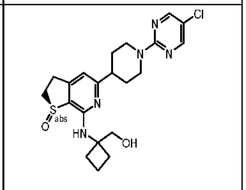 |
| 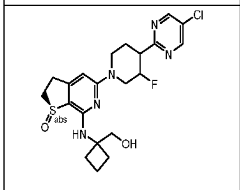 | 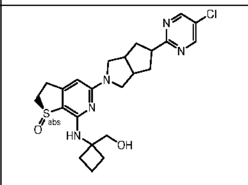 | 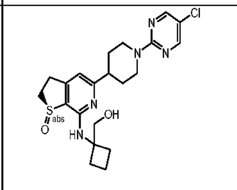 | 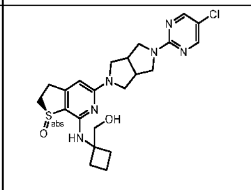 | 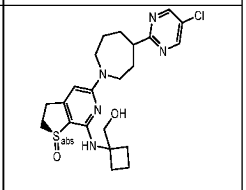 |
| 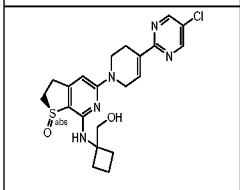 | 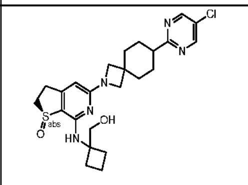 | 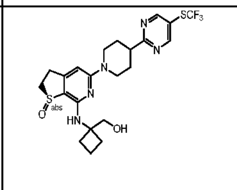 | 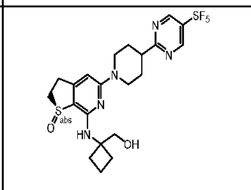 | 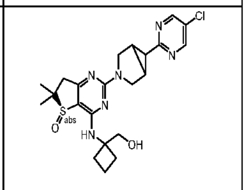 |
| 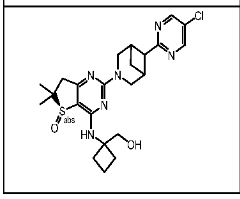 | 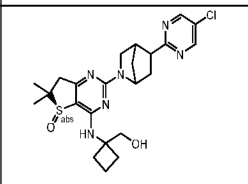 | 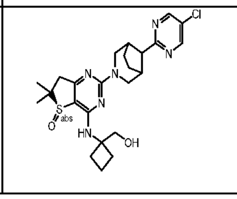 | 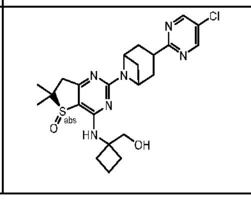 | 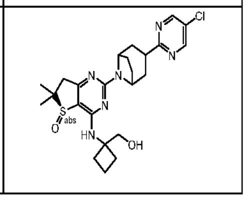 |

| | | | | |
|---|---|---|---|---|
| 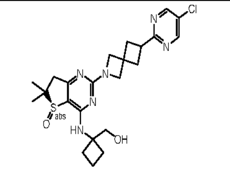 | 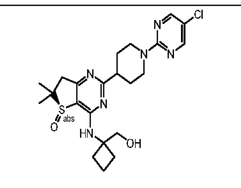 | 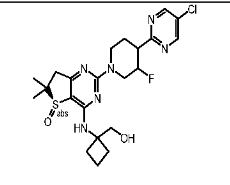 | 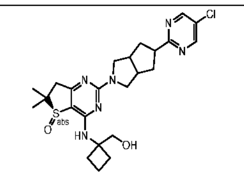 | 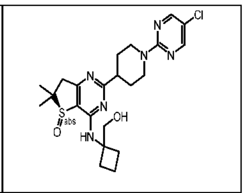 |
| 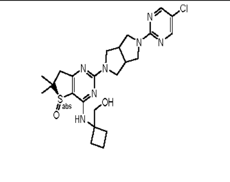 | 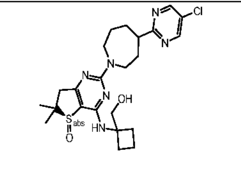 | 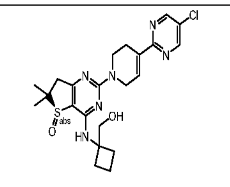 | 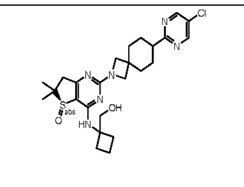 | 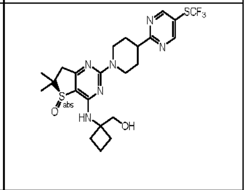 |
| 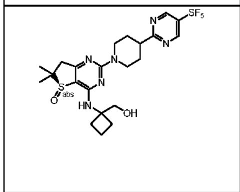 | 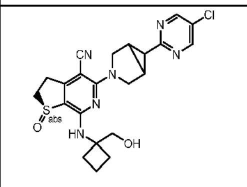 | 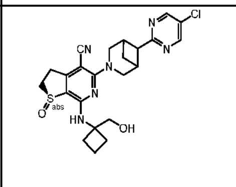 | 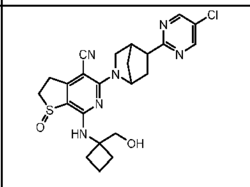 | 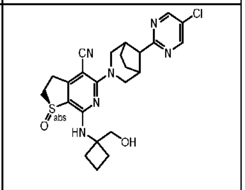 |
| 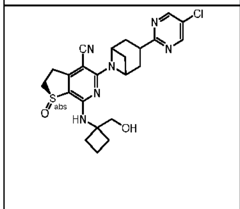 | 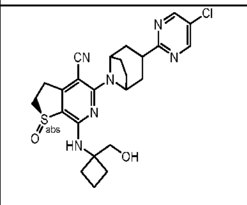 | 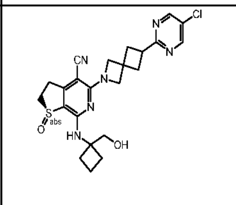 | 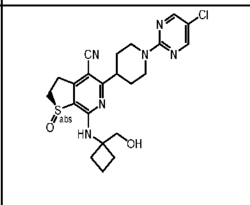 | 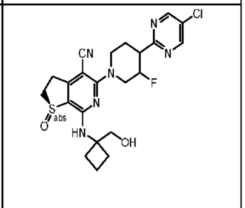 |
| 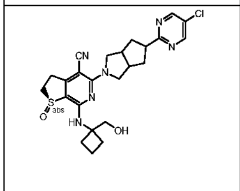 | 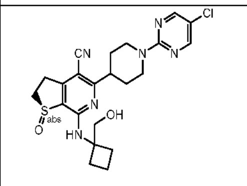 | 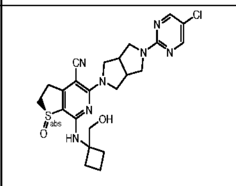 | 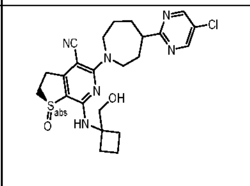 | 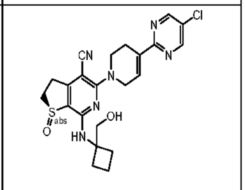 |
| 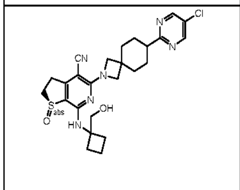 | 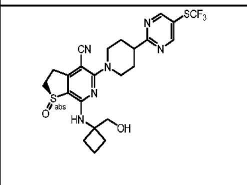 | 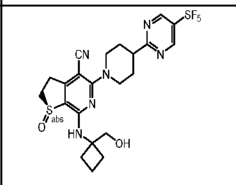 | 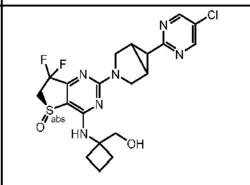 | 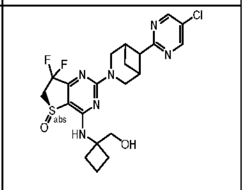 |
| 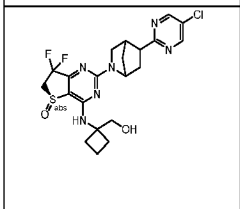 | 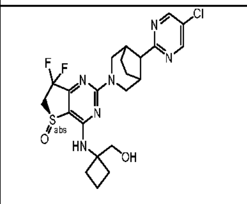 | 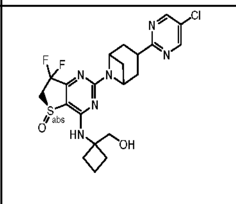 | 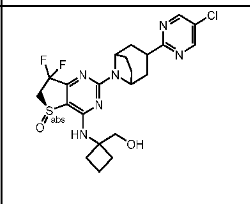 | 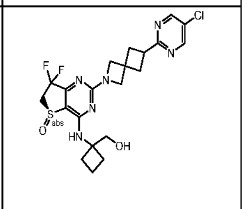 |
| 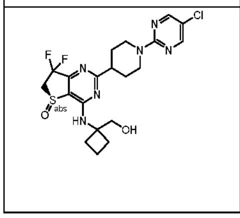 | 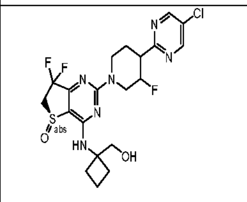 | 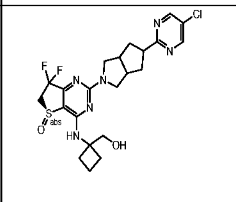 | 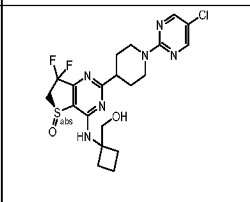 | 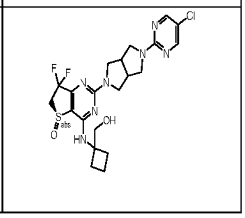 |

| | | | | |
|---|---|---|---|---|
| 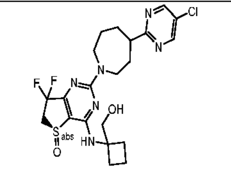 | 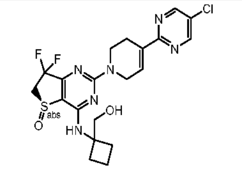 | 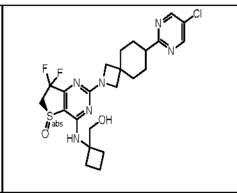 | 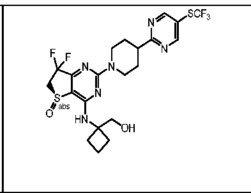 | 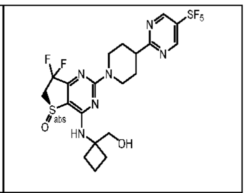 |
| 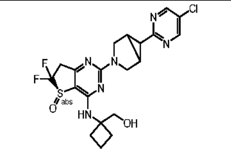 | 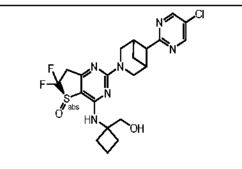 | 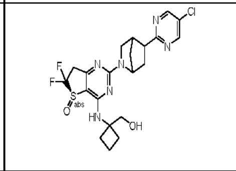 | 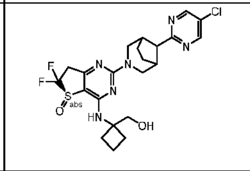 | 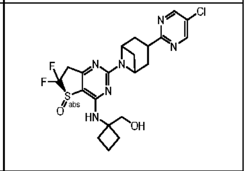 |
| 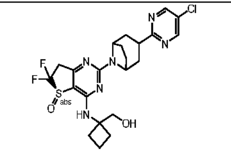 | 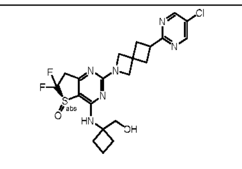 | 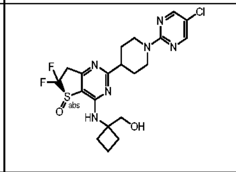 | 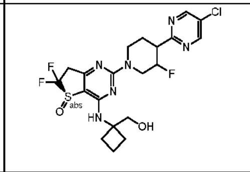 | 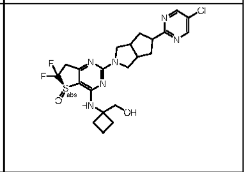 |
| 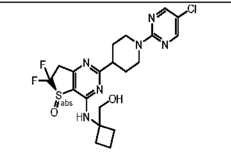 | 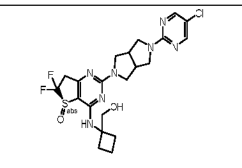 | 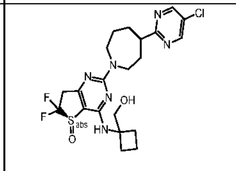 | 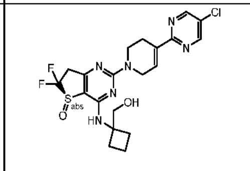 | 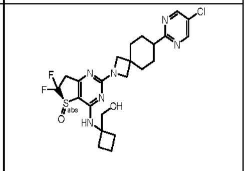 |
| 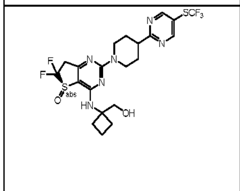 | 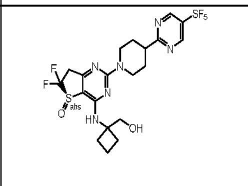 | 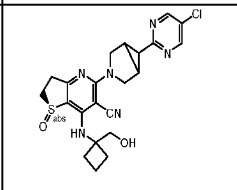 | 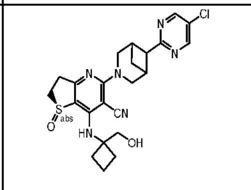 | 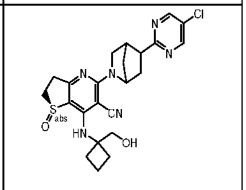 |
| 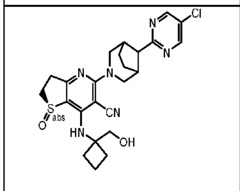 | 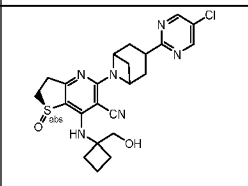 | 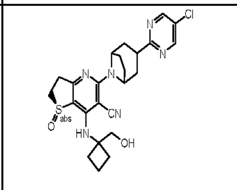 | 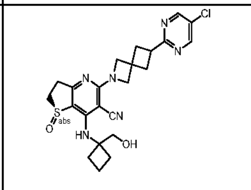 | 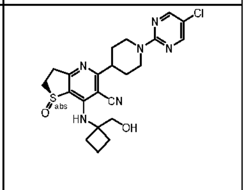 |
| 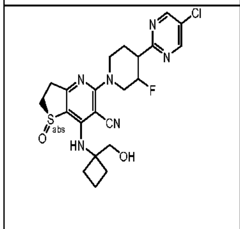 | 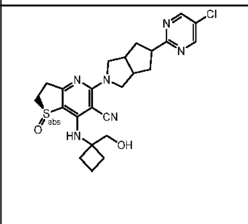 | 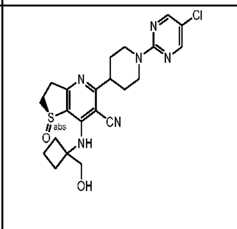 | 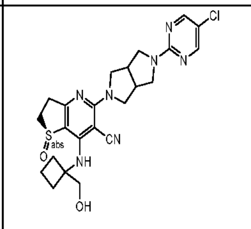 | 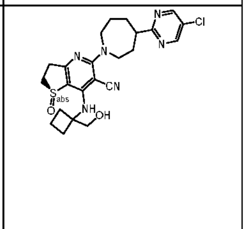 |
| 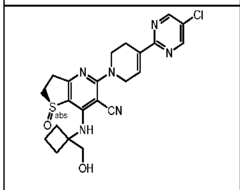 | 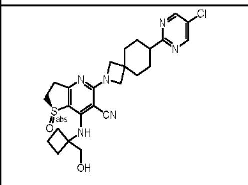 | 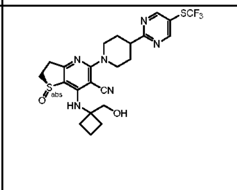 | 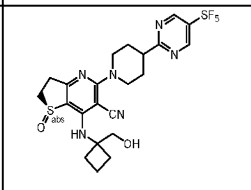 | 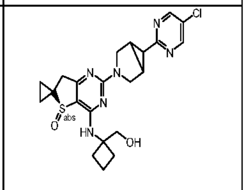 |

| | | | | |
|---|---|---|---|---|
| 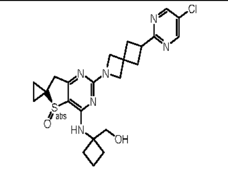 | 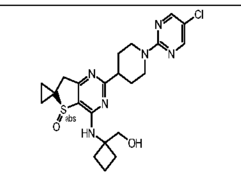 | 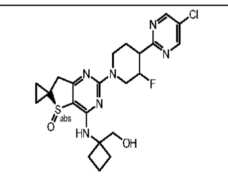 | 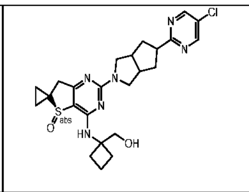 | 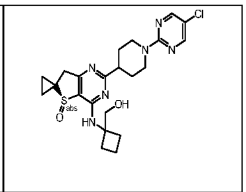 |
| 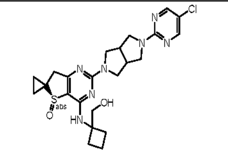 | 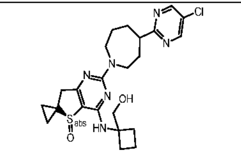 | 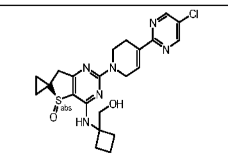 | 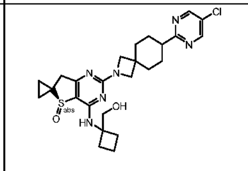 | 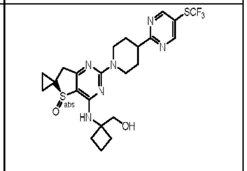 |
| 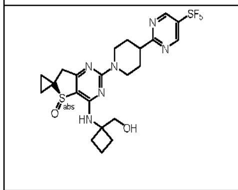 | 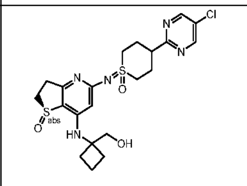 | 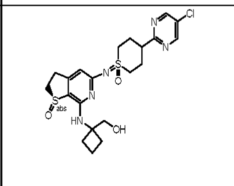 | 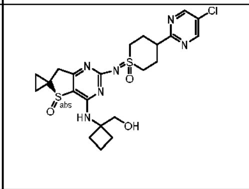 | 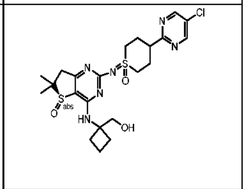 |
| 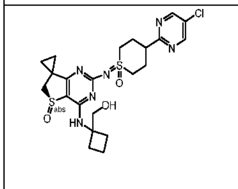 | 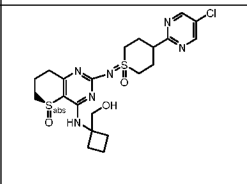 | 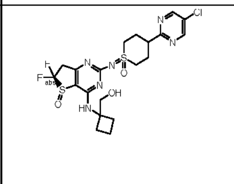 | 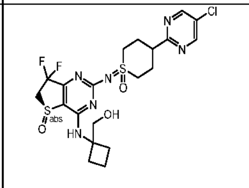 | 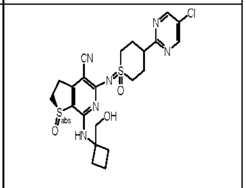 |
| 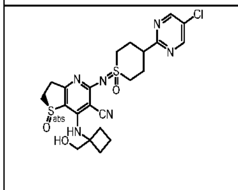 | 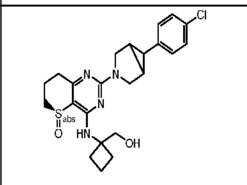 | 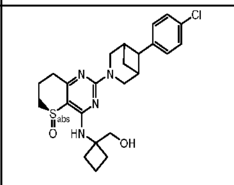 | 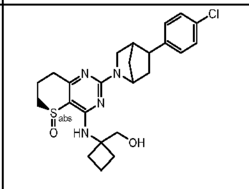 | 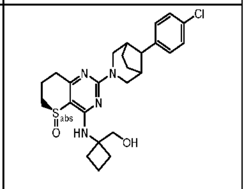 |
| 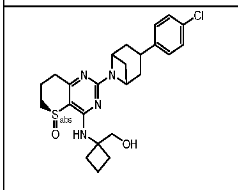 | 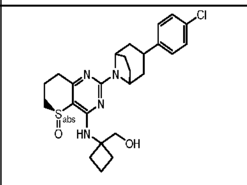 | 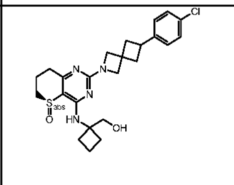 | 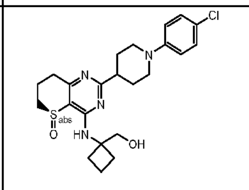 | 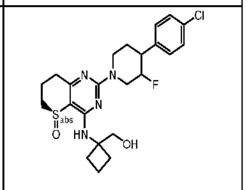 |
| 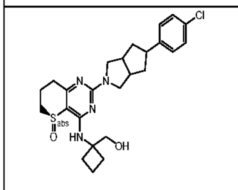 | 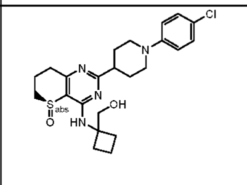 | 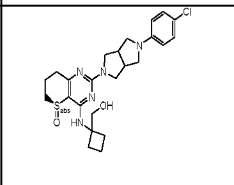 | 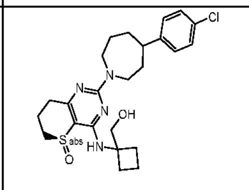 | 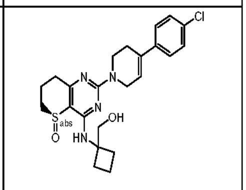 |
| 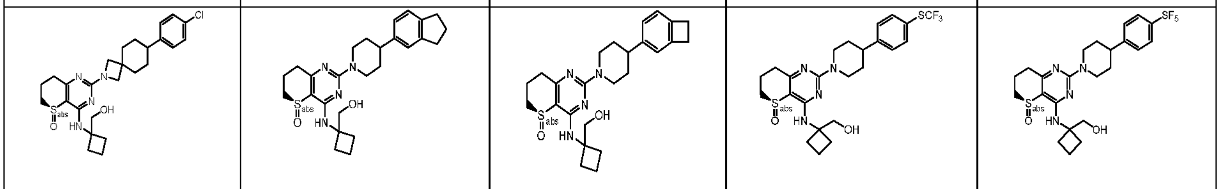 | | | | |
| 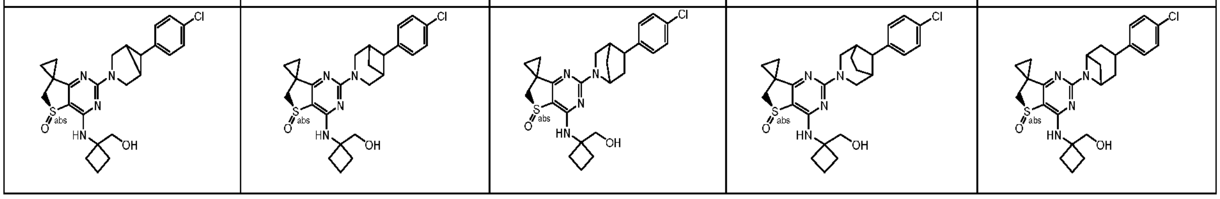 | | | | |

| | | | | |
|---|---|---|---|---|
| 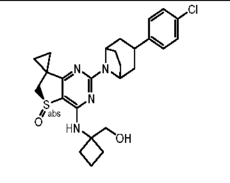 | 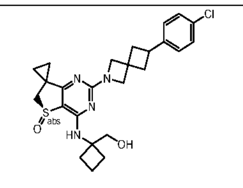 | 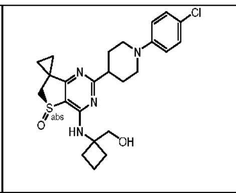 | 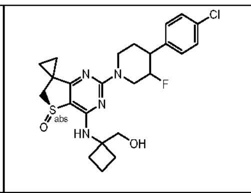 | 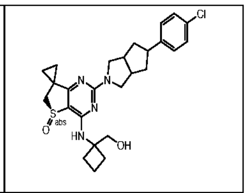 |
| 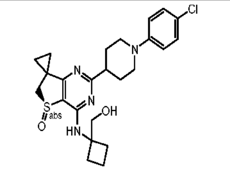 | 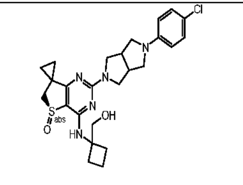 | 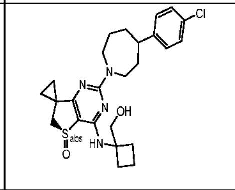 | 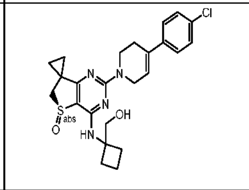 | 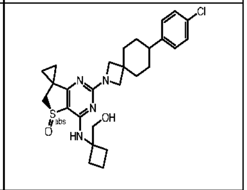 |
| 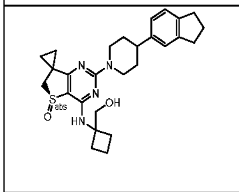 | 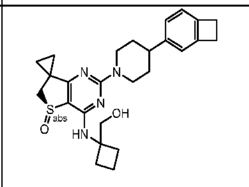 | 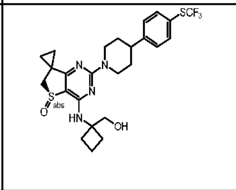 | 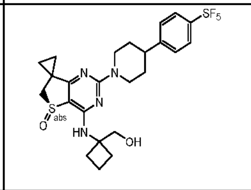 | 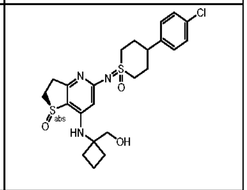 |
| 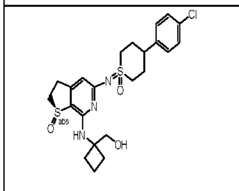 | 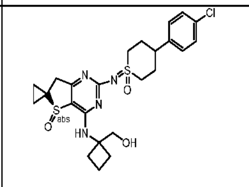 | 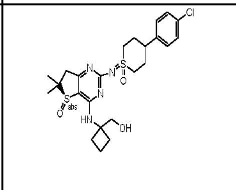 | 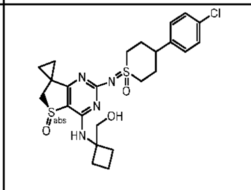 | 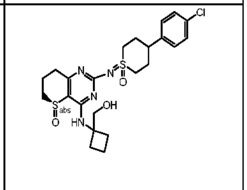 |
| 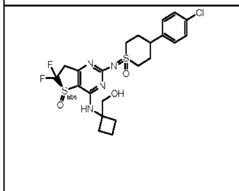 | 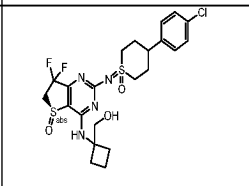 | 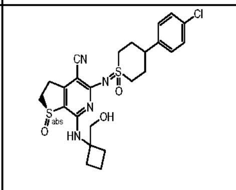 | 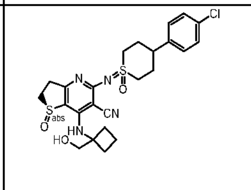 | 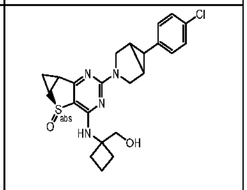 |
| 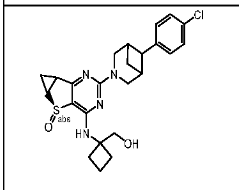 | 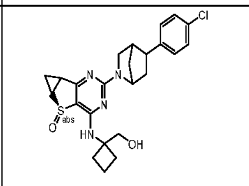 | 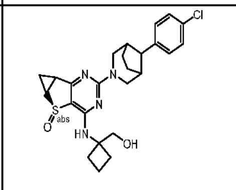 | 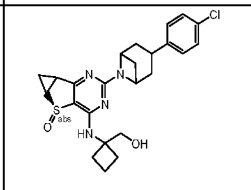 | 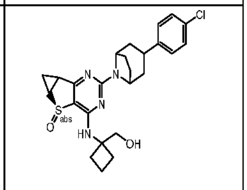 |
| 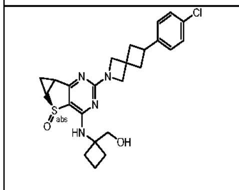 | 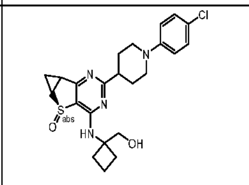 | 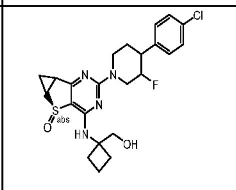 | 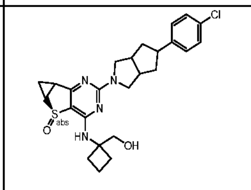 | 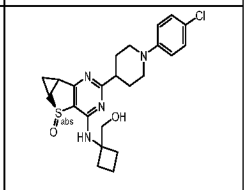 |
| 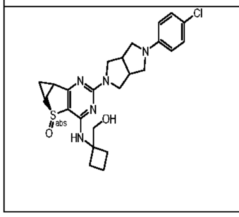 | 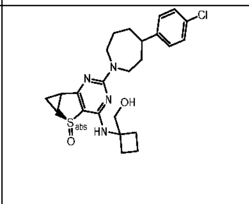 | 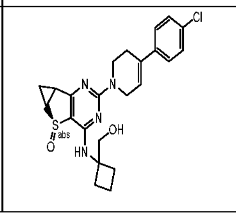 | 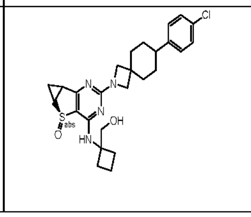 | 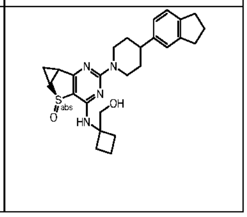 |

| | | | | |
|---|---|---|---|---|
| 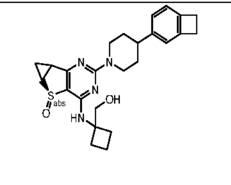 | 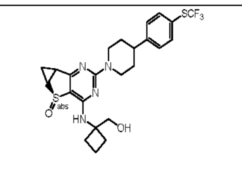 | 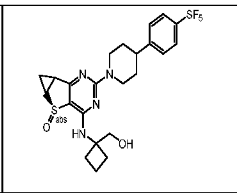 | 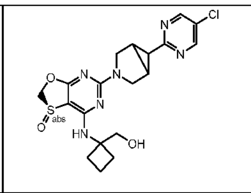 | 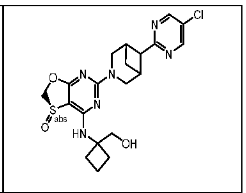 |
| 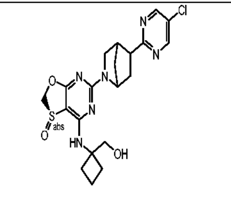 | 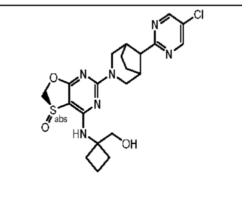 | 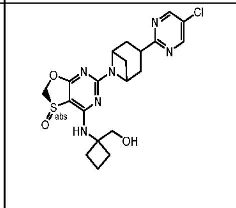 | 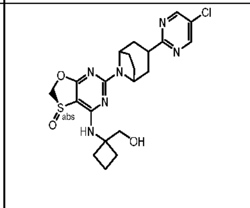 | 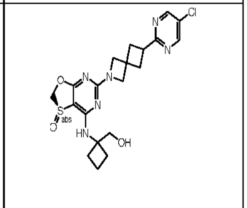 |
| 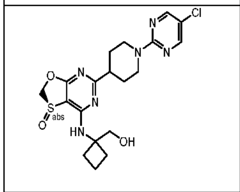 | 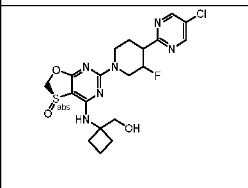 | 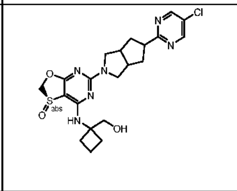 | 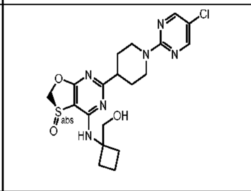 | 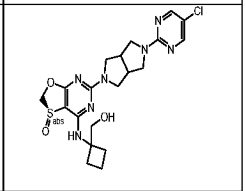 |
| 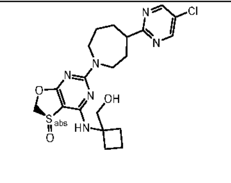 | 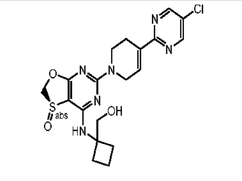 | 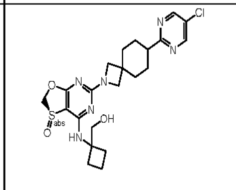 | 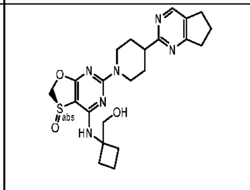 | 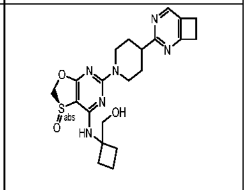 |
| 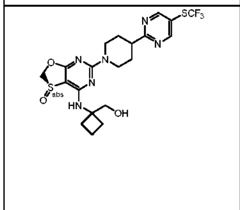 | 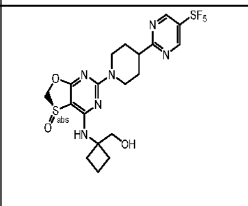 | 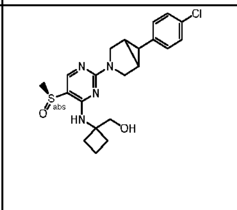 | 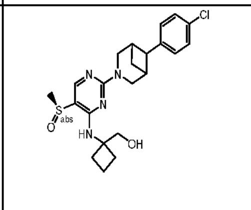 | 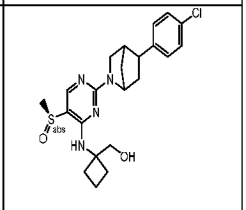 |
| 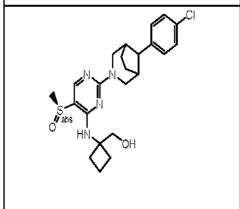 | 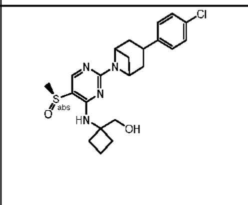 | 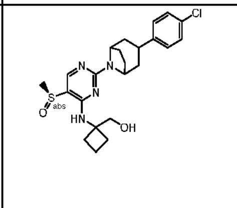 | 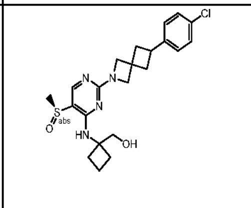 | 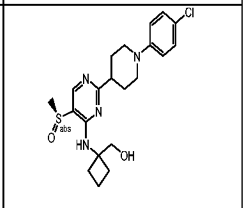 |
| 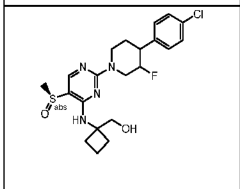 | 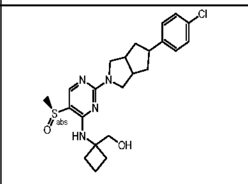 | 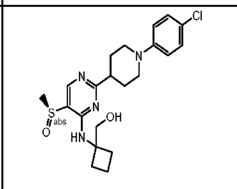 | 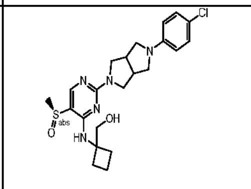 | 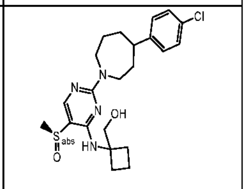 |
| 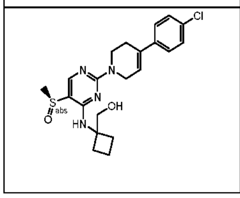 | 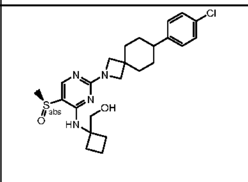 | 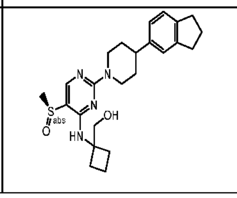 | 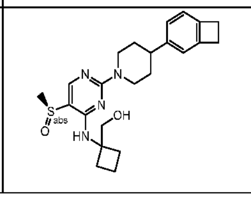 | 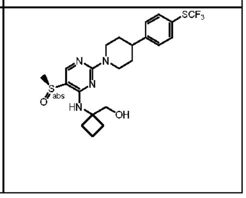 |

| | | | | |
|---|---|---|---|---|
| 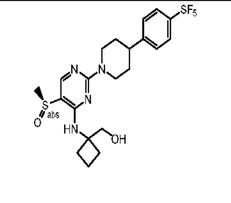 | 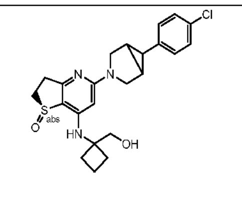 | 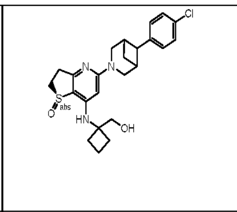 | 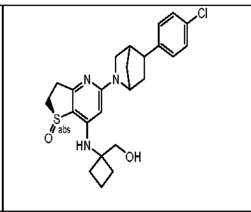 | 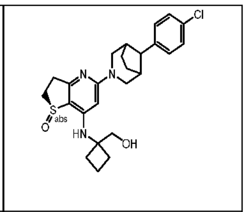 |
| 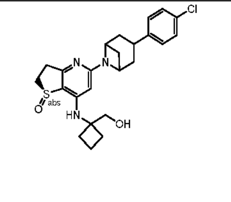 | 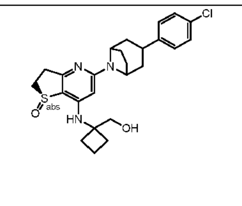 | 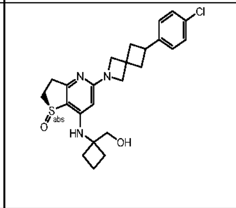 | 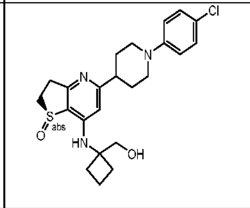 | 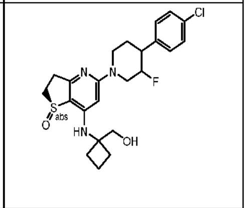 |
| 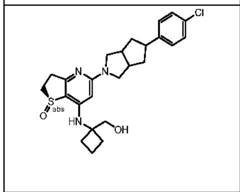 | 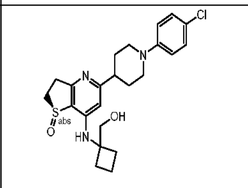 | 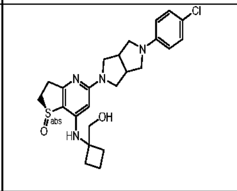 | 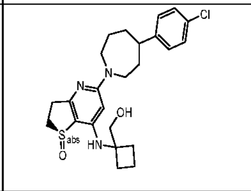 | 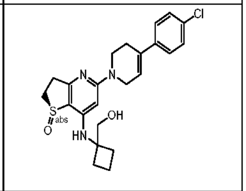 |
| 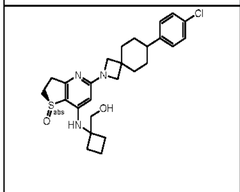 | 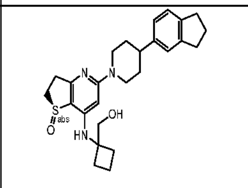 | 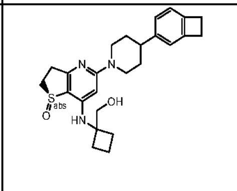 | 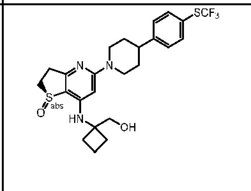 | 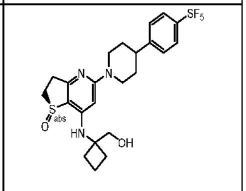 |
| 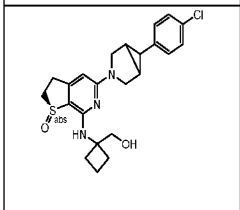 | 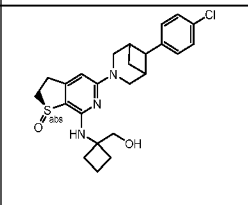 | 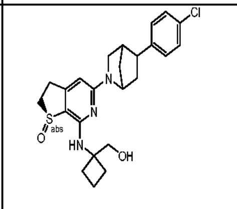 | 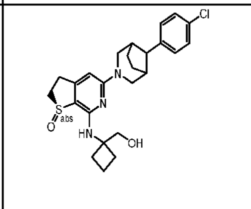 | 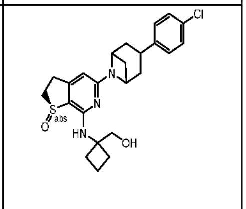 |
| 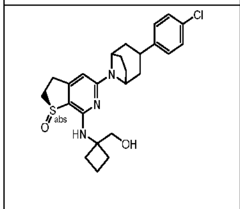 | 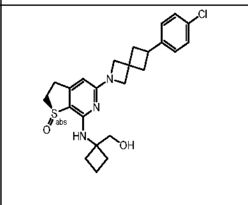 | 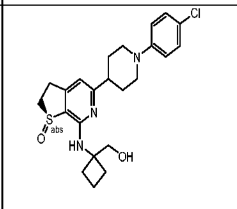 | 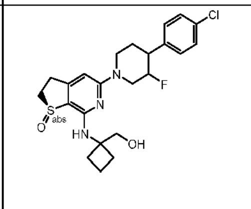 | 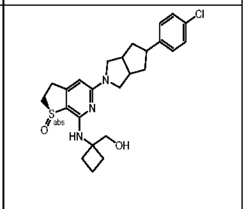 |
| 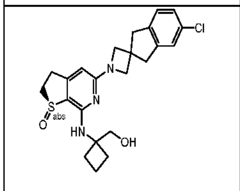 | 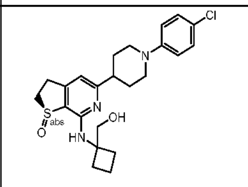 | 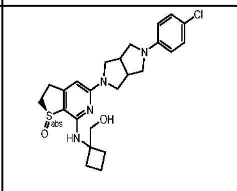 | 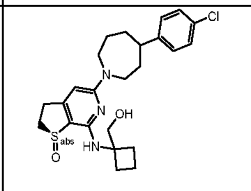 | 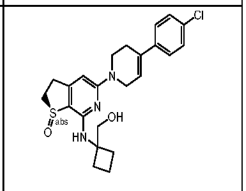 |
| 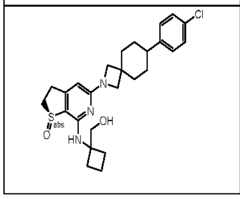 | 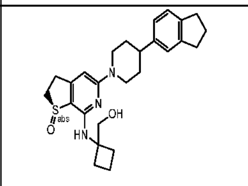 | 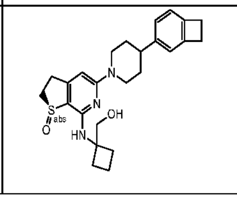 | 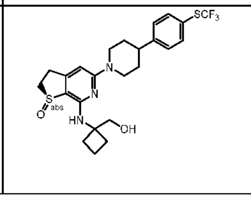 | 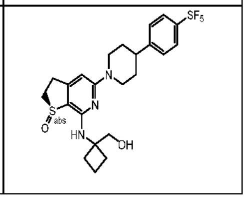 |

| | | | | |
|---|---|---|---|---|
| 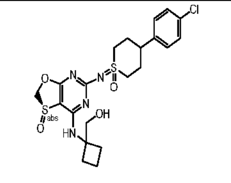 | 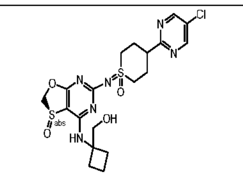 | 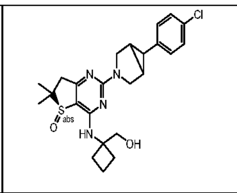 | 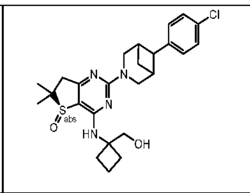 | 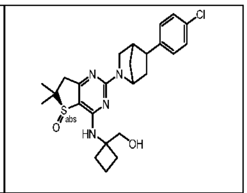 |
| 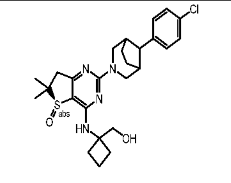 | 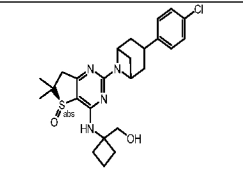 | 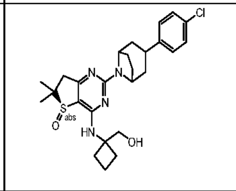 | 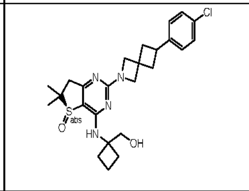 | 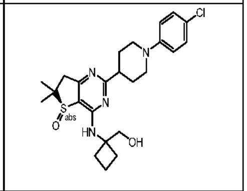 |
| 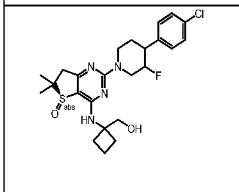 | 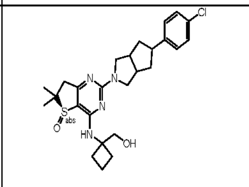 | 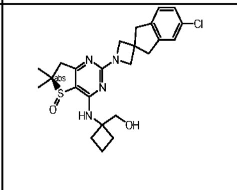 | 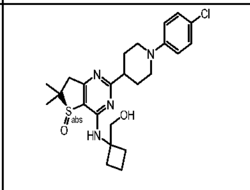 | 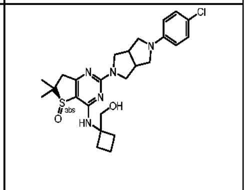 |
| 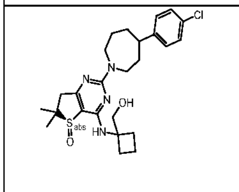 | 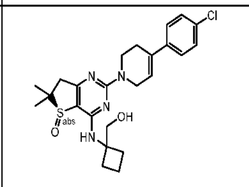 | 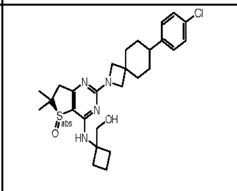 | 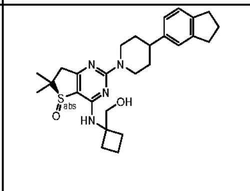 | 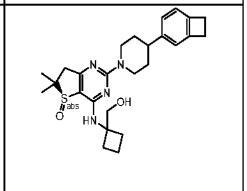 |
| 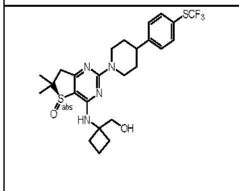 | 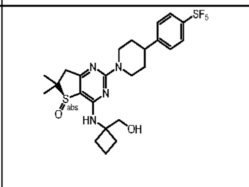 | 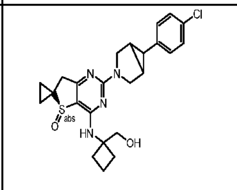 | 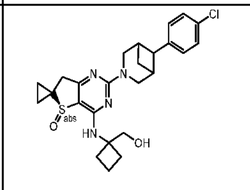 | 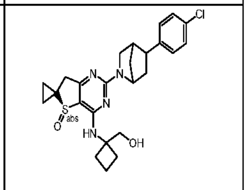 |
| 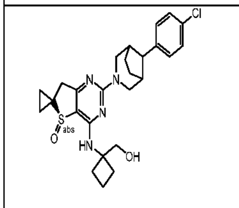 | 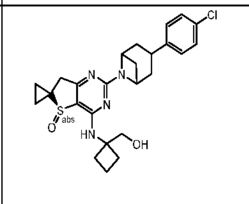 | 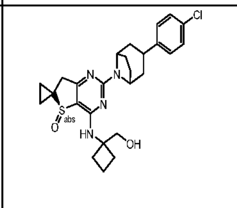 | 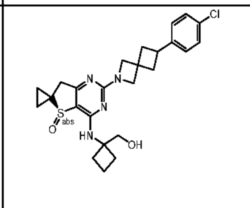 | 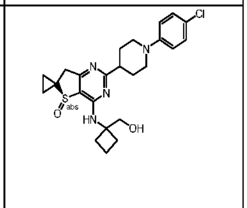 |
| 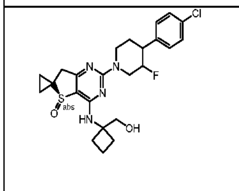 | 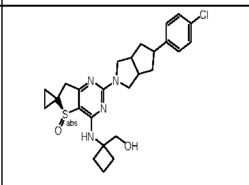 | 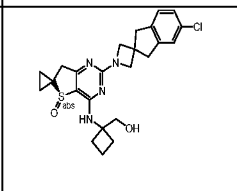 | 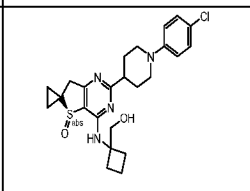 | 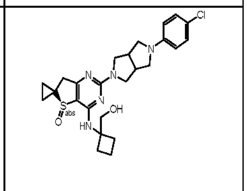 |
| 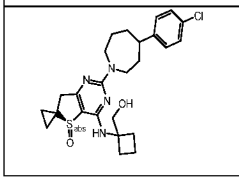 | 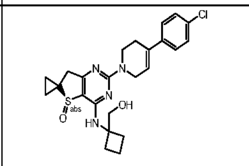 | 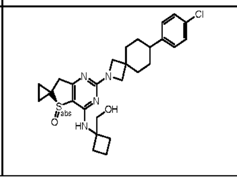 | 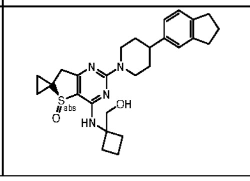 | 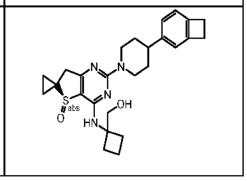 |

| | | | | |
|---|---|---|---|---|
| 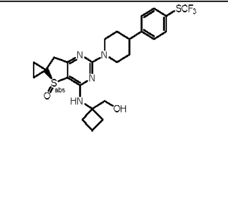 | 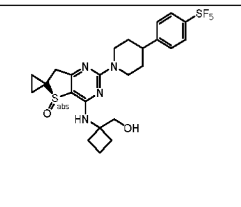 | 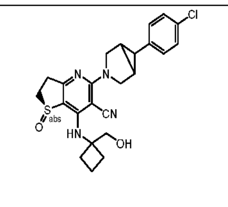 | 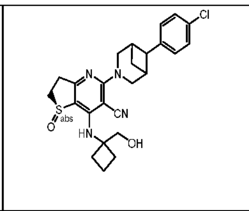 | 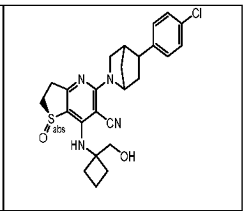 |
| 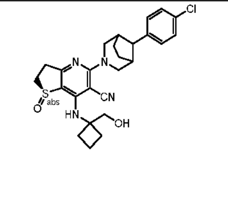 | 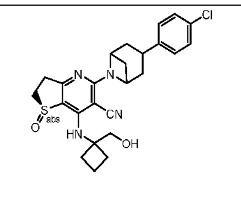 | 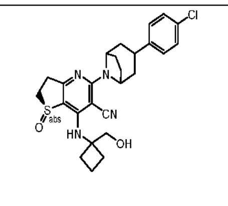 | 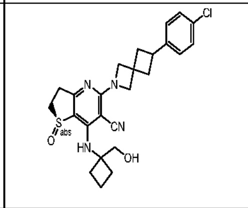 | 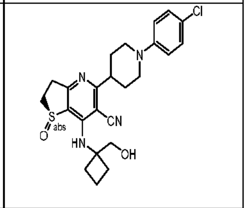 |
| 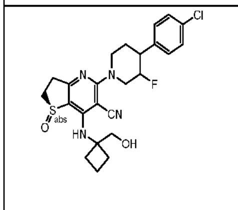 | 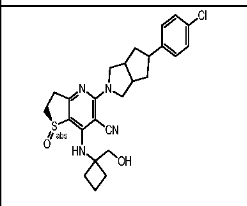 | 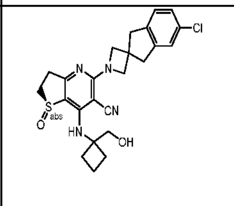 | 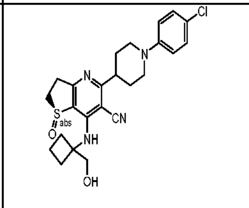 | 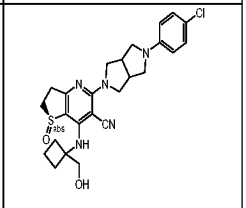 |
| 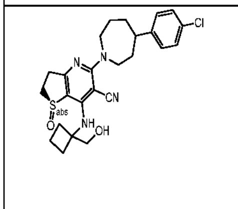 | 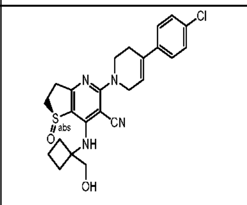 | 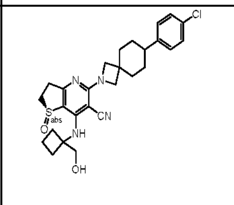 | 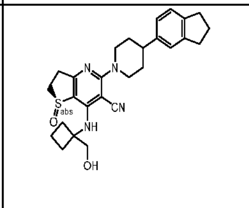 | 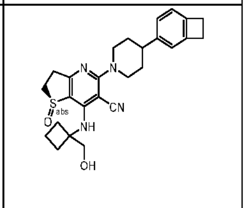 |
| 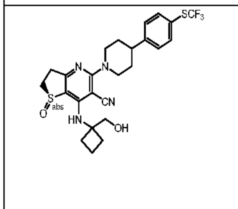 | 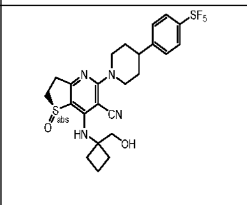 | 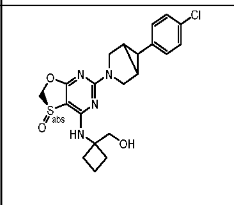 | 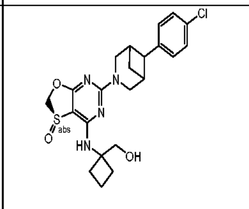 | 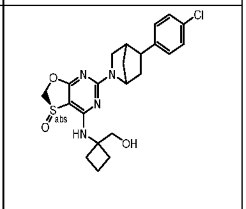 |
| 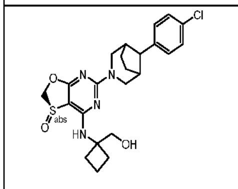 | 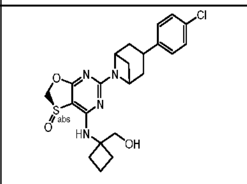 | 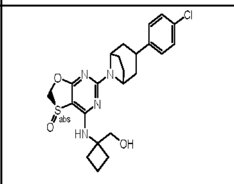 | 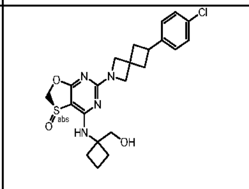 | 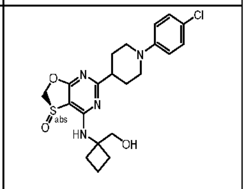 |
| 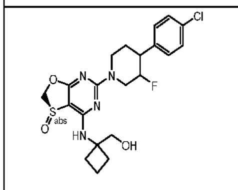 | 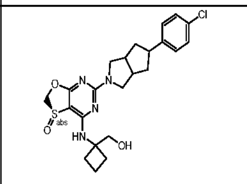 | 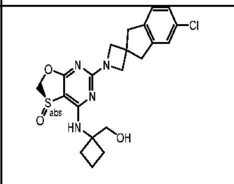 | 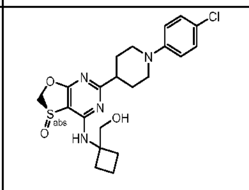 | 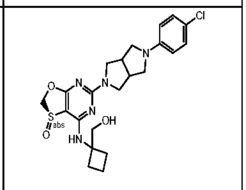 |
| 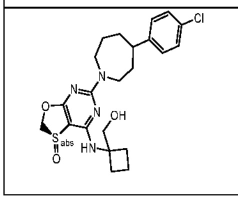 | 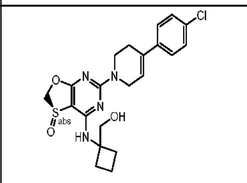 | 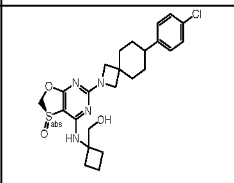 | 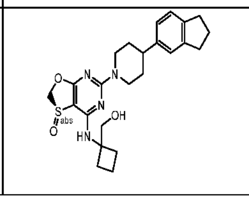 | 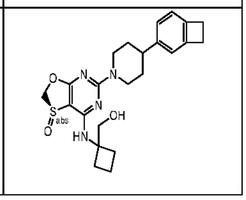 |

| | | | | |
|---|---|---|---|---|
| 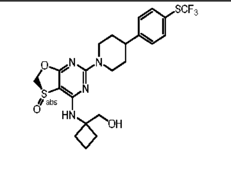 | 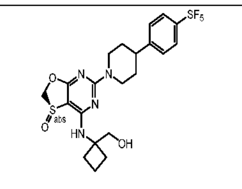 | 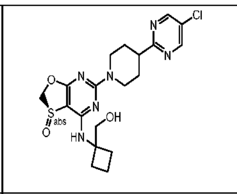 | 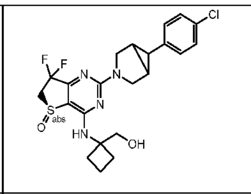 | 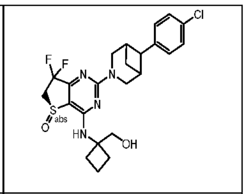 |
| 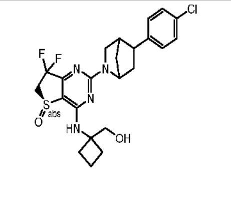 | 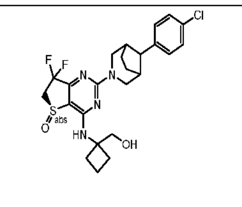 | 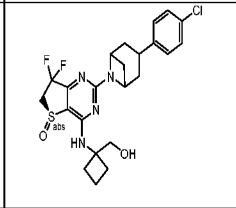 | 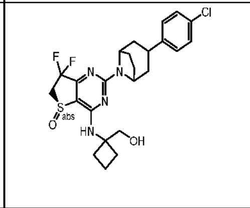 | 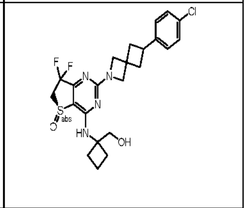 |
| 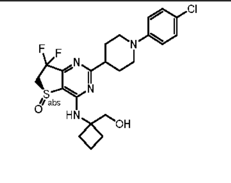 | 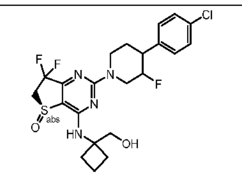 | 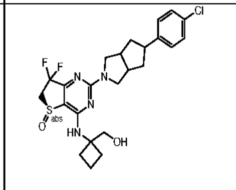 | 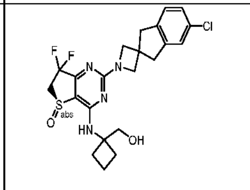 | 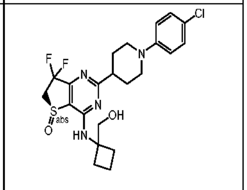 |
| 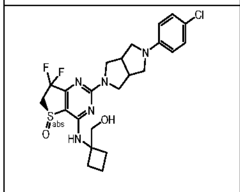 | 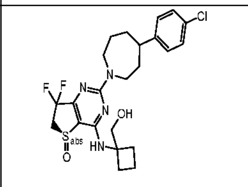 | 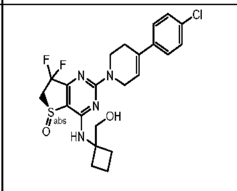 | 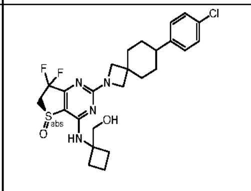 | 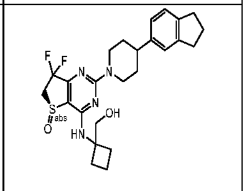 |
| 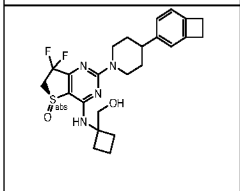 | 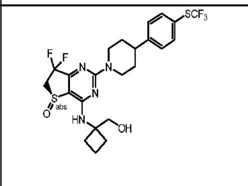 | 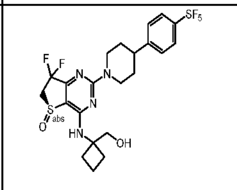 | 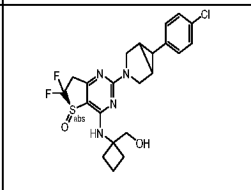 | 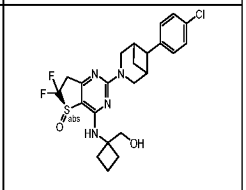 |
| 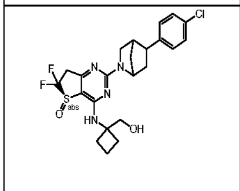 | 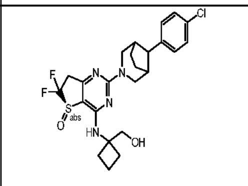 | 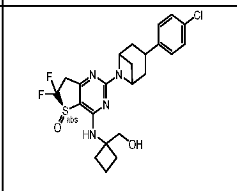 | 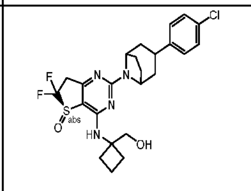 | 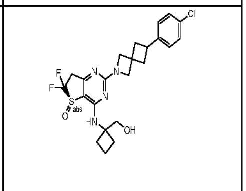 |
| 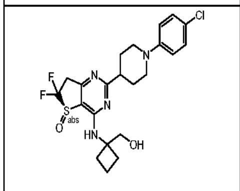 | 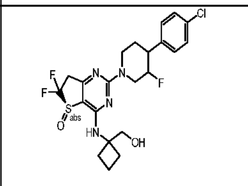 | 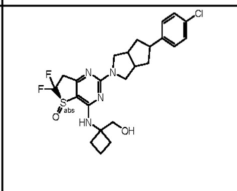 | 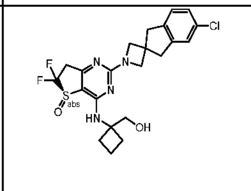 | 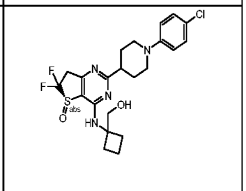 |
| 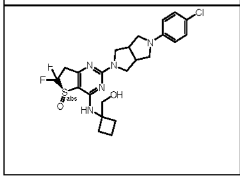 | 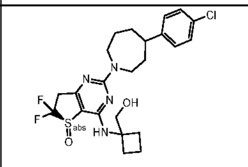 | 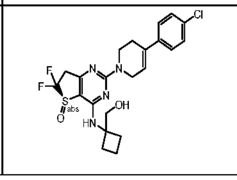 | 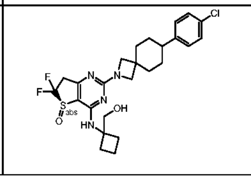 | 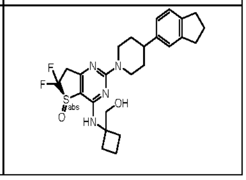 |

| | | | | |
|---|---|---|---|---|
| 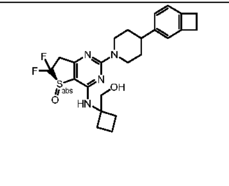 | 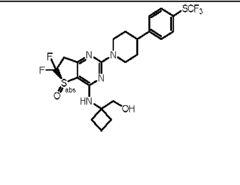 | 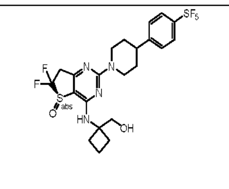 | 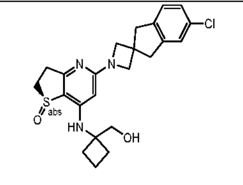 | 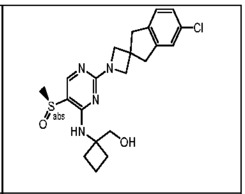 |
| 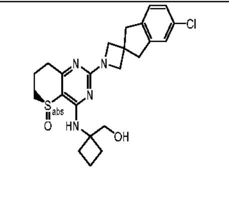 | 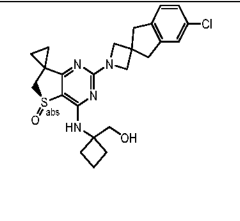 | 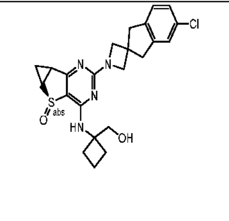 | 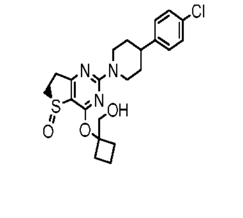 | 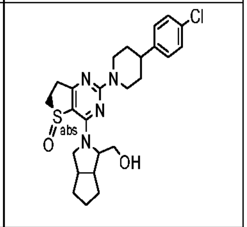 |
| 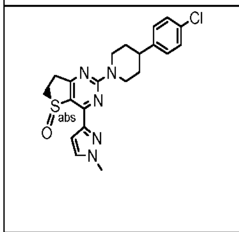 | 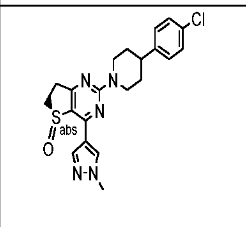 | 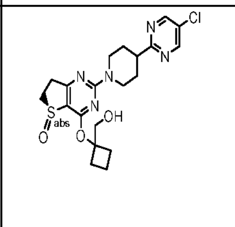 | 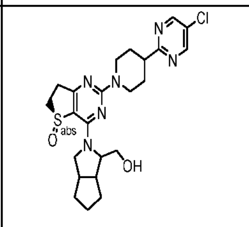 | 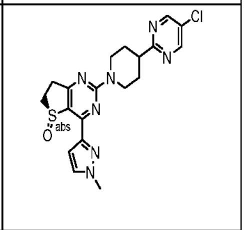 |
| 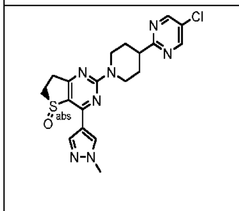 | 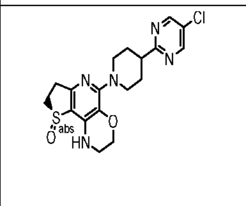 | 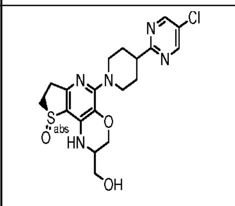 | 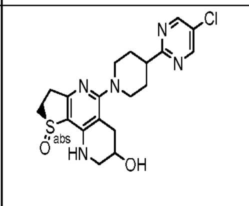 | 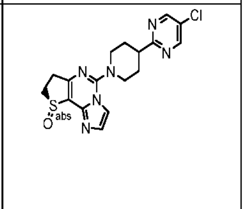 |
| 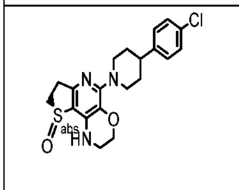 | 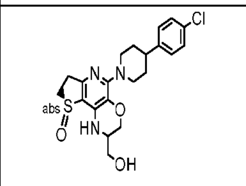 | 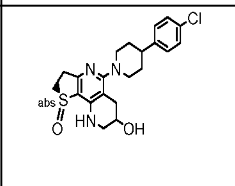 | 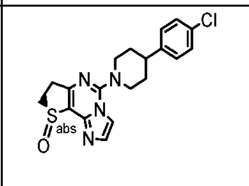 | 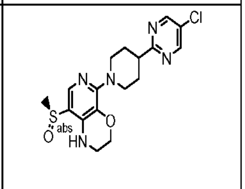 |
| 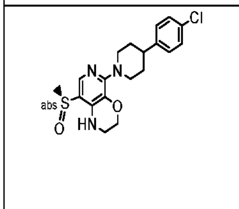 | 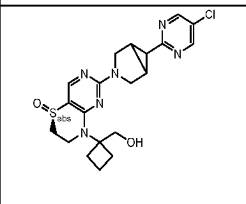 | 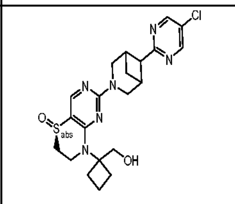 | 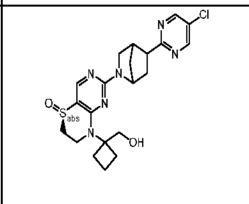 | 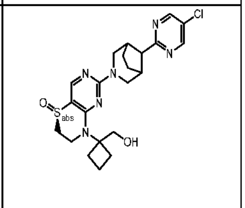 |
| 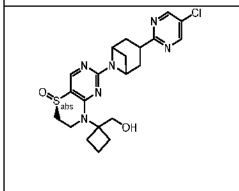 | 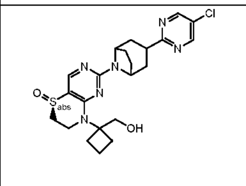 | 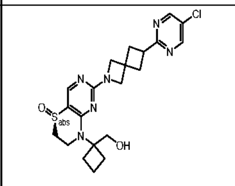 | 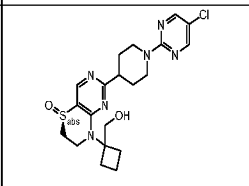 | 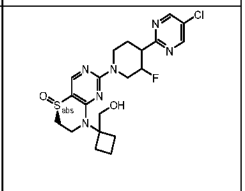 |
| 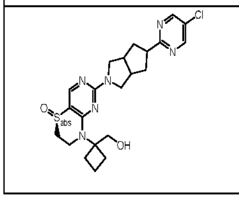 | 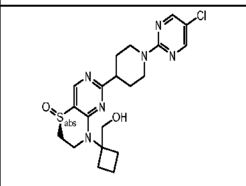 | 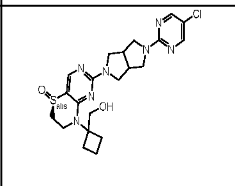 | 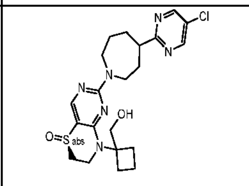 | 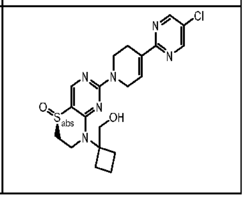 |

| | | | | |
|---|---|---|---|---|
| 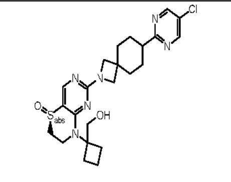 | 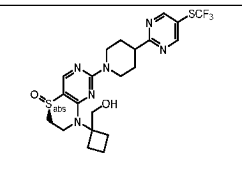 | 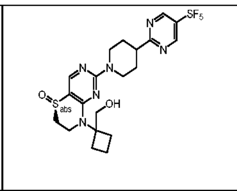 | 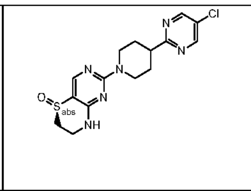 | 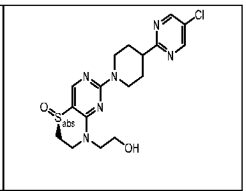 |
| 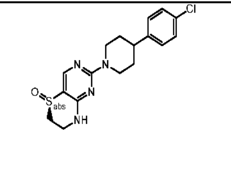 | 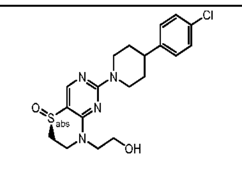 | 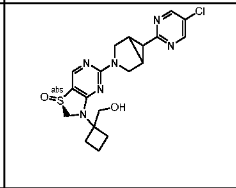 | 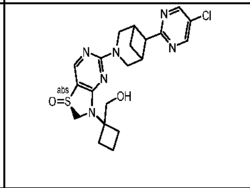 | 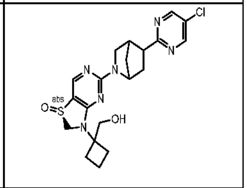 |
| 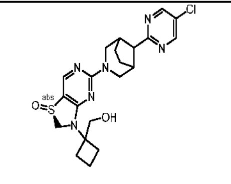 | 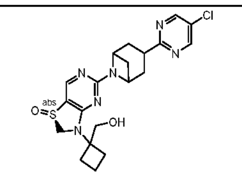 | 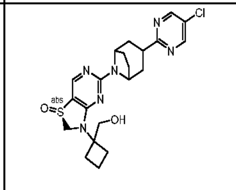 | 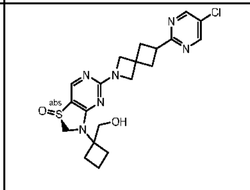 | 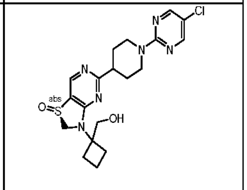 |
| 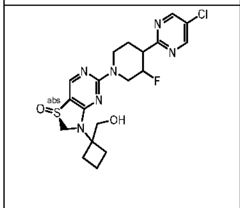 | 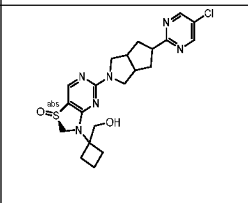 | 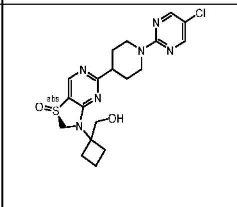 | 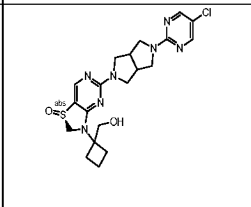 | 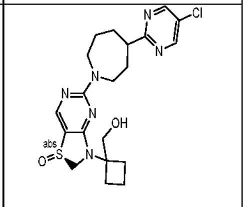 |
| 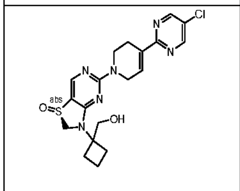 | 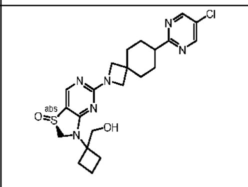 | 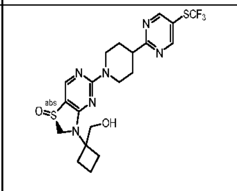 | 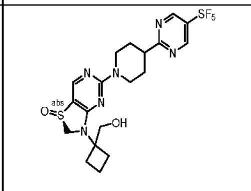 | |
| 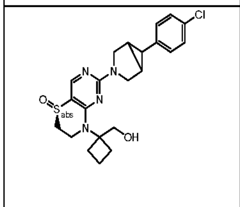 | 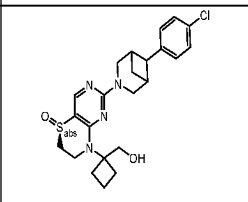 | 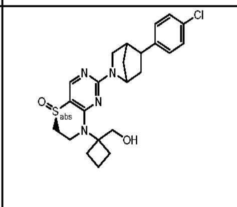 | 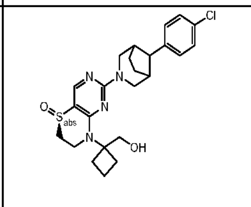 | 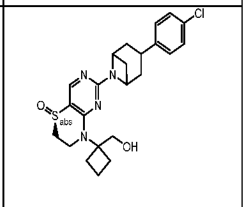 |
| 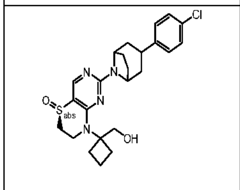 | 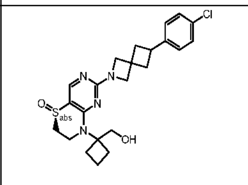 | 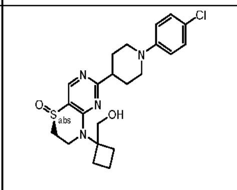 | 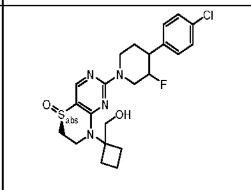 | 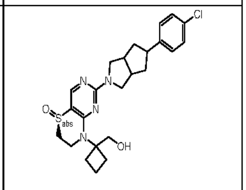 |
| 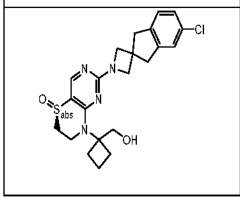 | 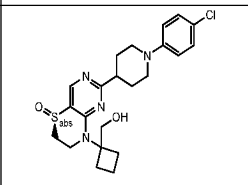 | 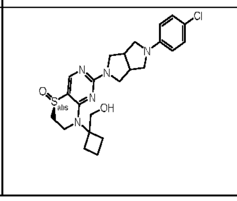 | 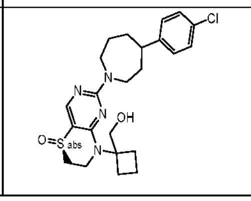 | 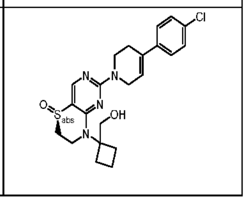 |

| | | | | |
|---|---|---|---|---|
| 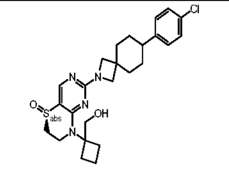 | 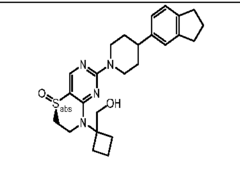 | 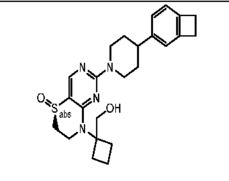 | 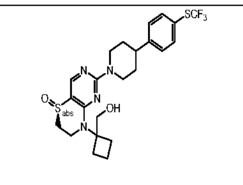 | 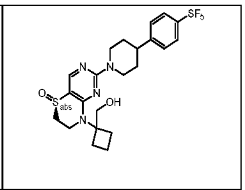 |
| 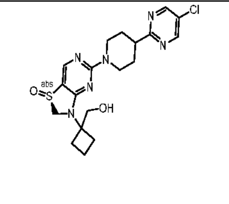 | 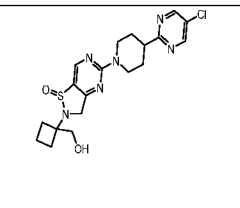 | 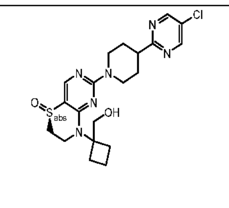 | 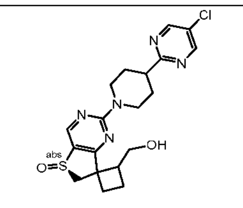 | 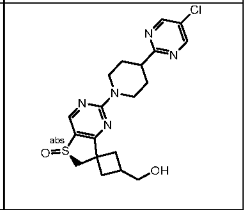 |
| 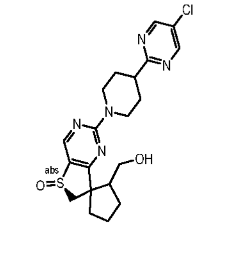 | 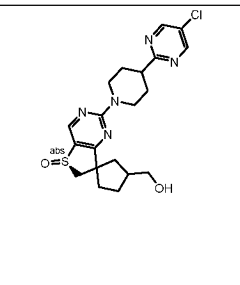 | 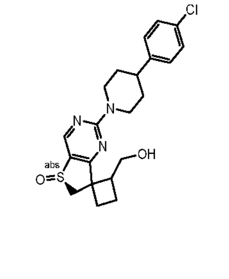 | 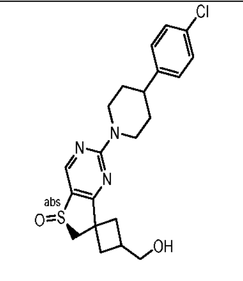 | 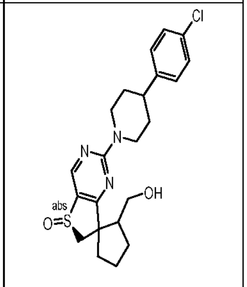 |
| 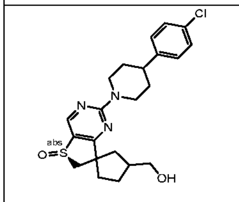 | 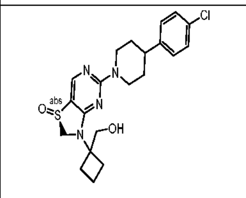 | 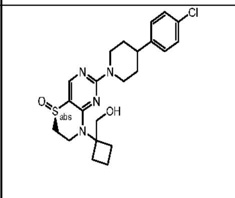 | 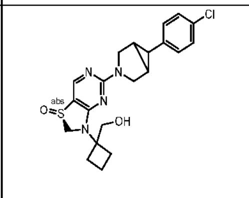 | 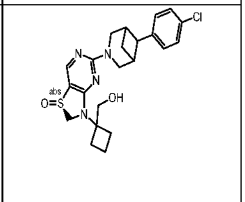 |
| 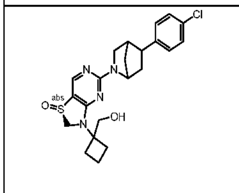 | 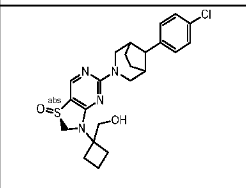 | 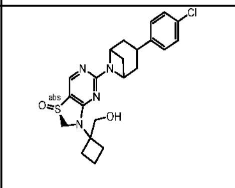 | 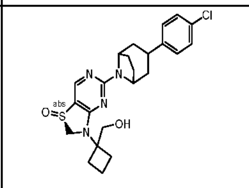 | 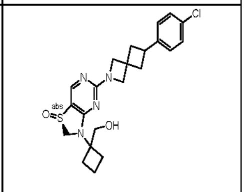 |
| 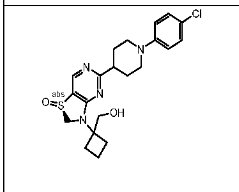 | 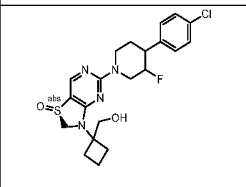 | 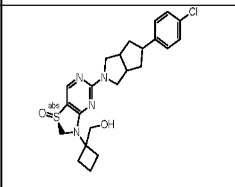 | 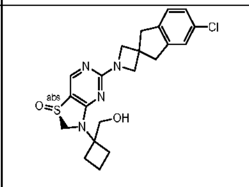 | 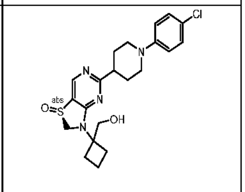 |
| 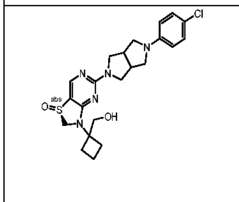 | 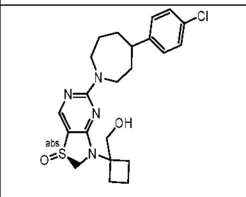 | 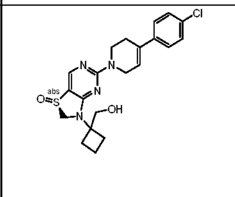 | 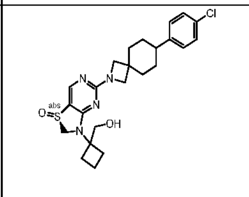 | 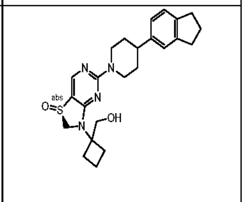 |
| 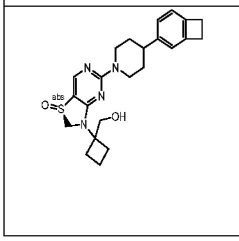 | 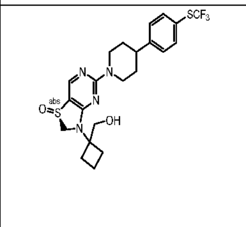 | 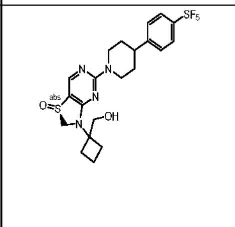 | 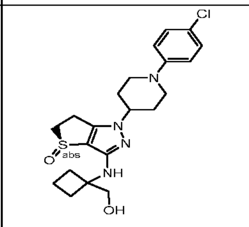 | 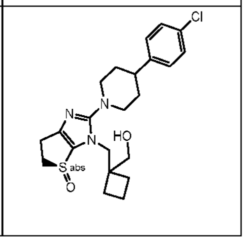 |

| | | | | |
|---|---|---|---|---|
| 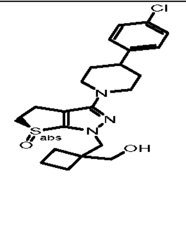 | 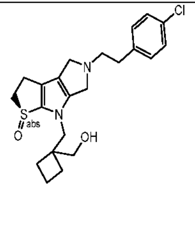 | 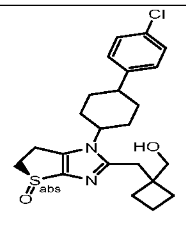 | 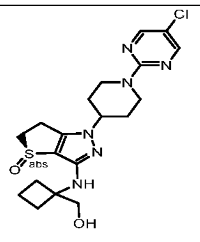 | 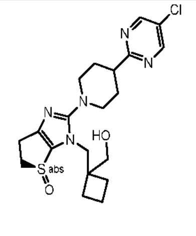 |
| 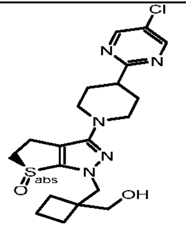 | 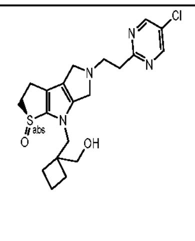 | 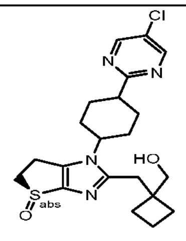 | 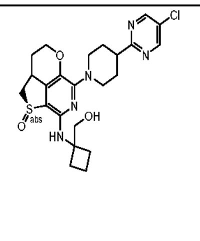 | 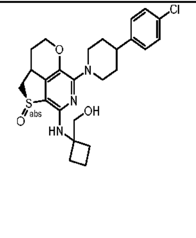 |
| 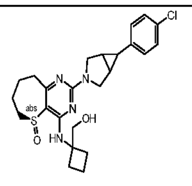 | 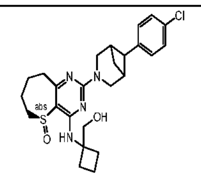 | 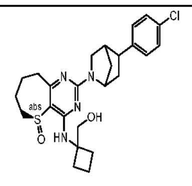 | 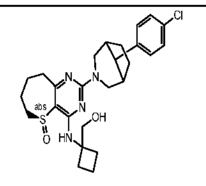 | 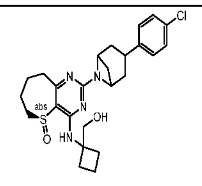 |
| 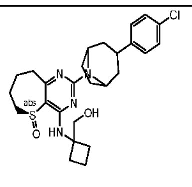 | 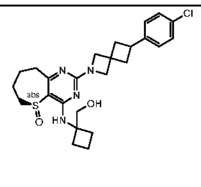 | 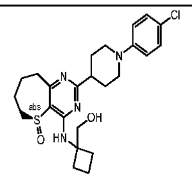 | 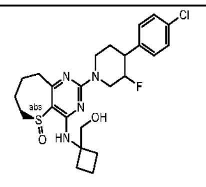 | 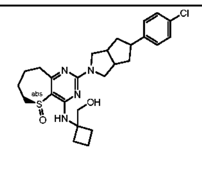 |
| 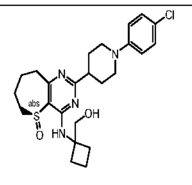 | 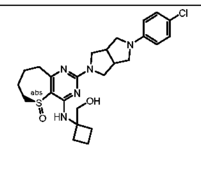 | 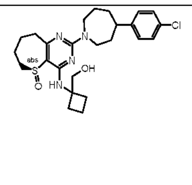 | 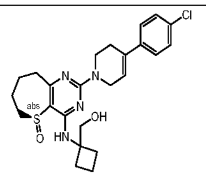 | 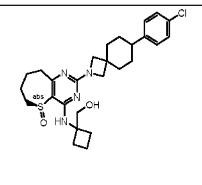 |
| 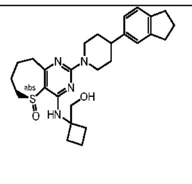 | 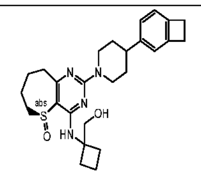 | 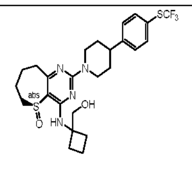 | 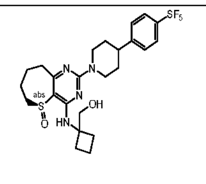 | 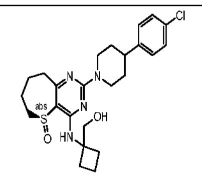 |
| 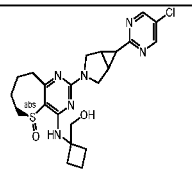 | 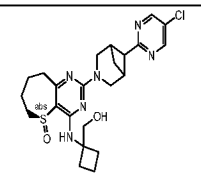 | 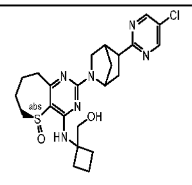 | 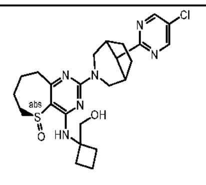 | 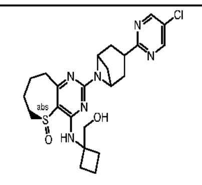 |
| 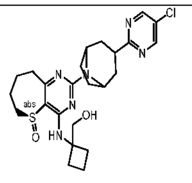 | 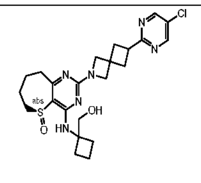 | 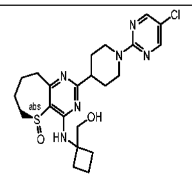 | 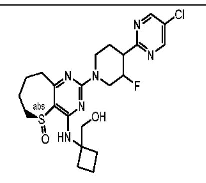 | 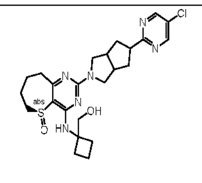 |

| | | | | |
|---|---|---|---|---|
| 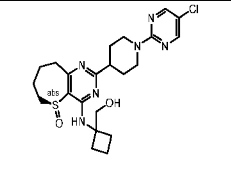 | 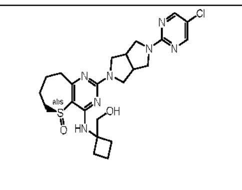 | 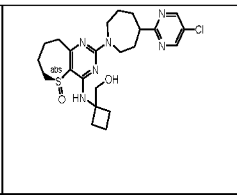 | 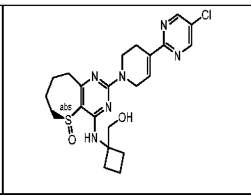 | 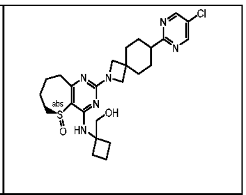 |
| 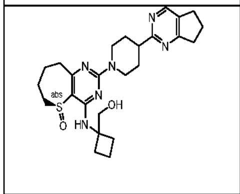 | 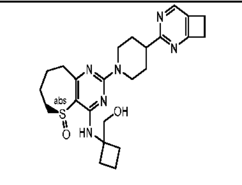 | 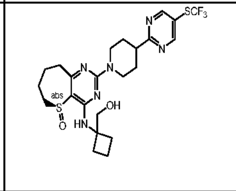 | 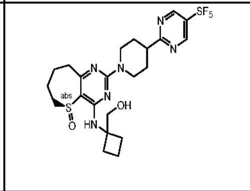 | 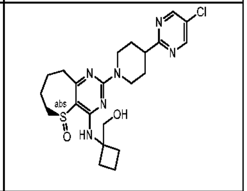 |
| 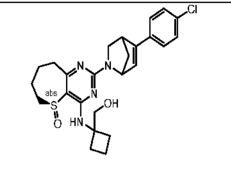 | 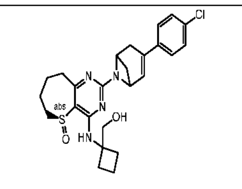 | 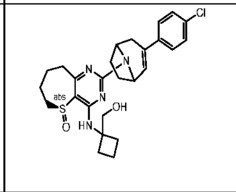 | 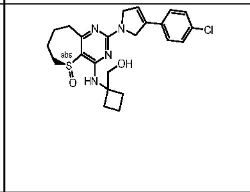 | 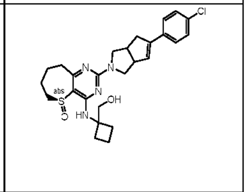 |
| 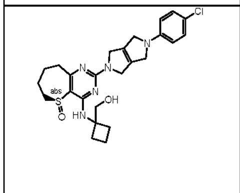 | 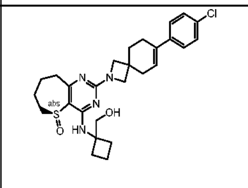 | 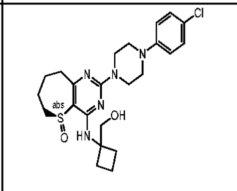 | 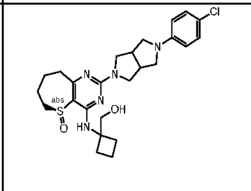 | 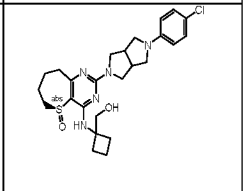 |
| 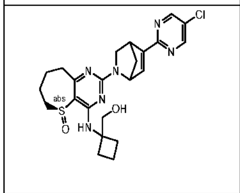 | 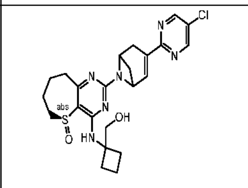 | 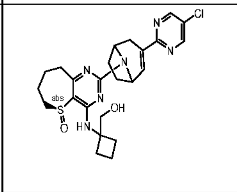 | 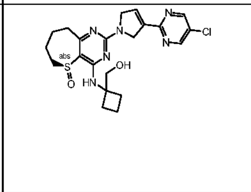 | 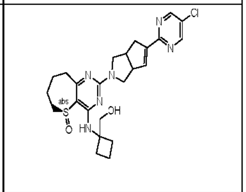 |
| 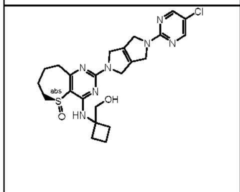 | 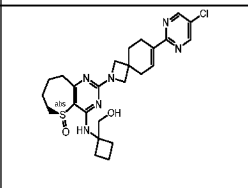 | 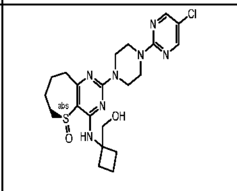 | 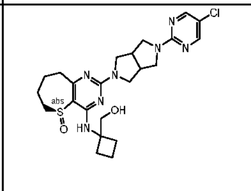 | 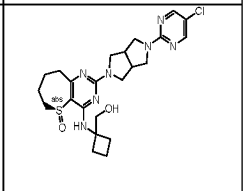 |
| 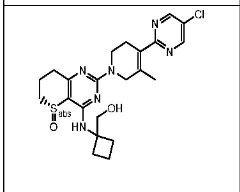 | 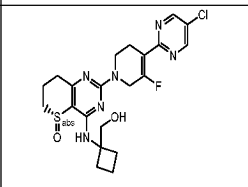 | 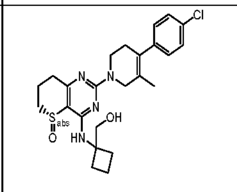 | 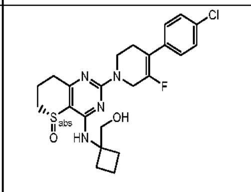 | 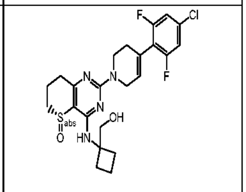 |
| 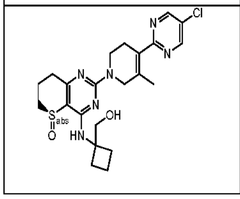 | 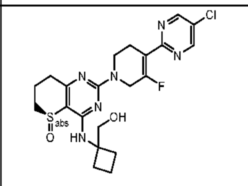 | 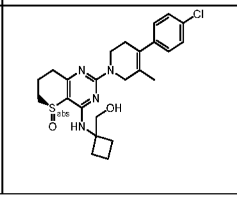 | 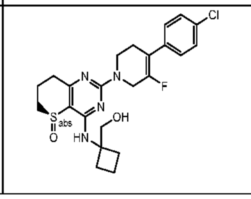 | 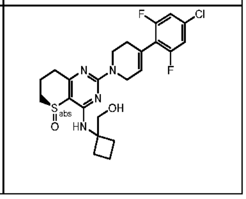 |

| | | | | |
|---|---|---|---|---|
| 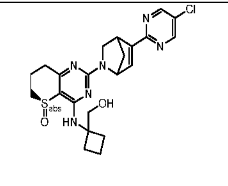 | 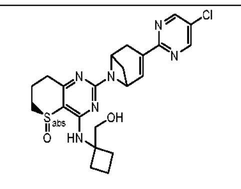 | 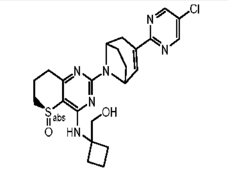 | 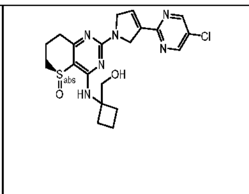 | 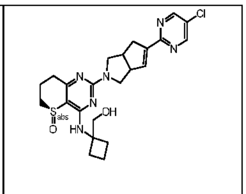 |
| 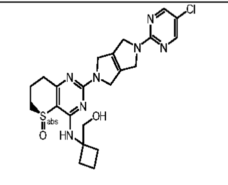 | 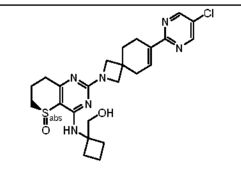 | 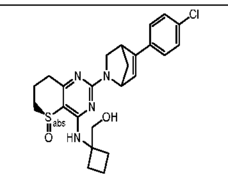 | 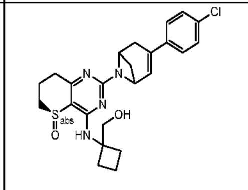 | 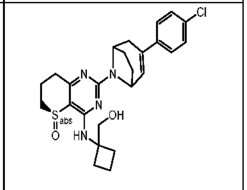 |
| 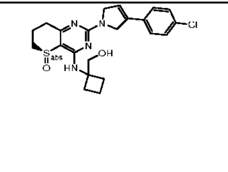 | 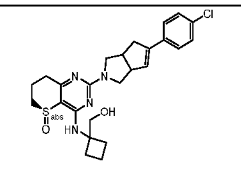 | 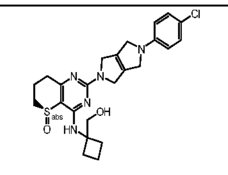 | 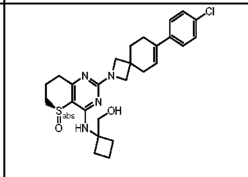 | 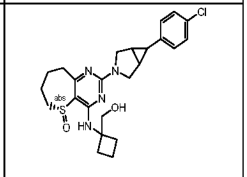 |
| 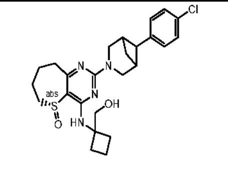 | 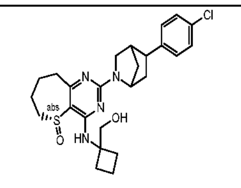 | 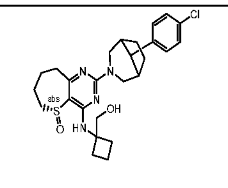 | 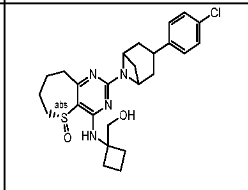 | 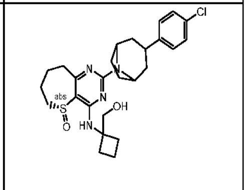 |
| 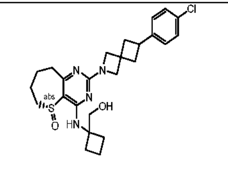 | 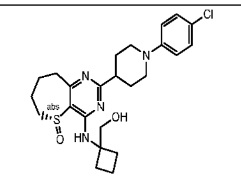 | 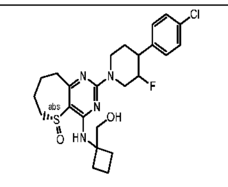 | 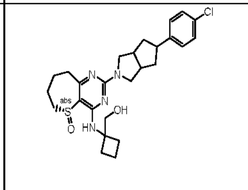 | 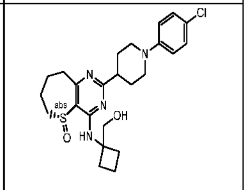 |
| 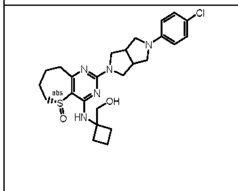 | 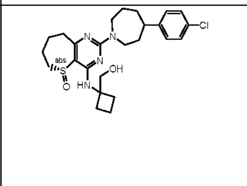 | 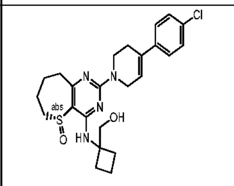 | 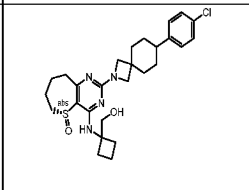 | 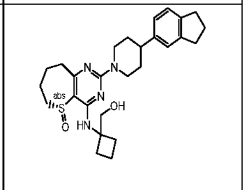 |
| 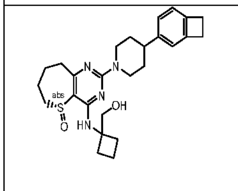 | 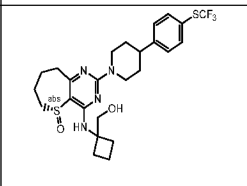 | 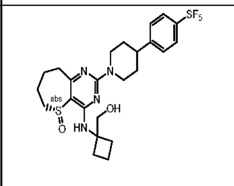 | 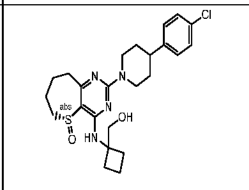 | 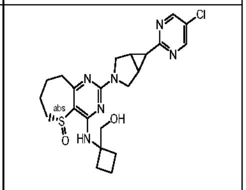 |
| 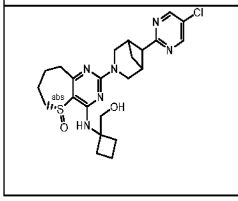 | 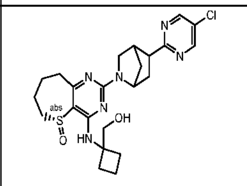 | 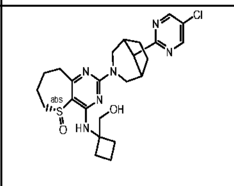 | 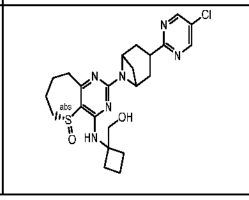 | 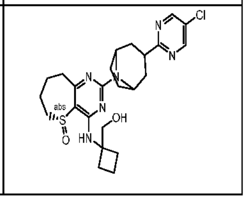 |

| | | | | |
|---|---|---|---|---|
| 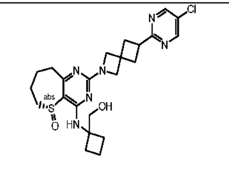 | 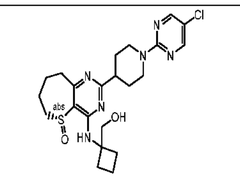 | 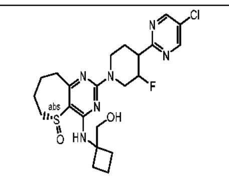 | 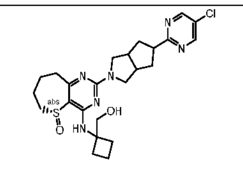 | 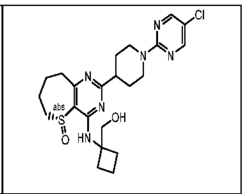 |
| 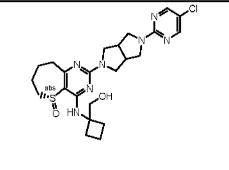 | 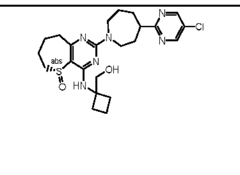 | 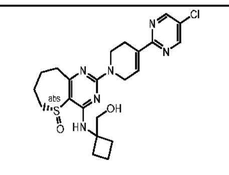 | 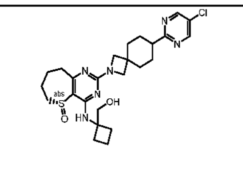 | 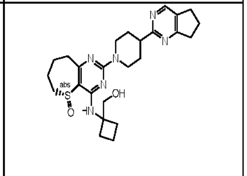 |
| 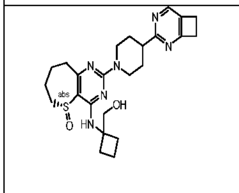 | 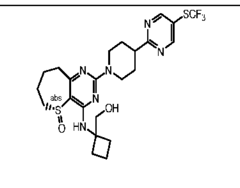 | 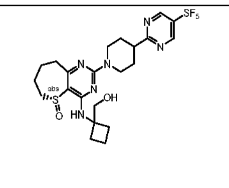 | 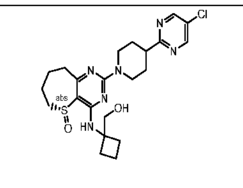 | 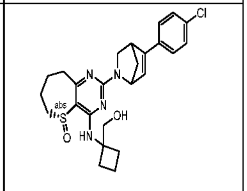 |
| 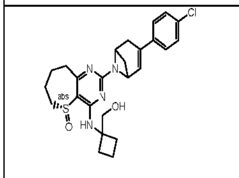 | 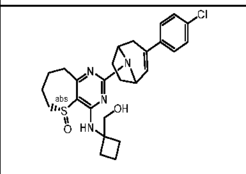 | 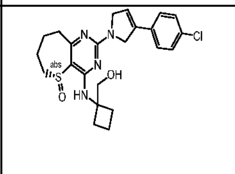 | 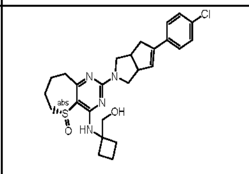 | 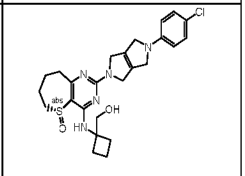 |
| 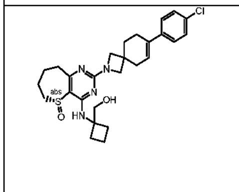 | 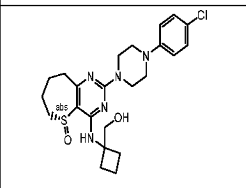 | 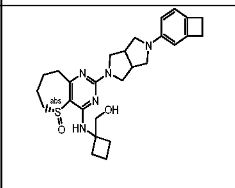 | 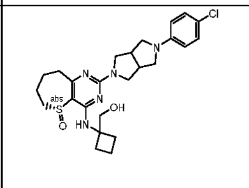 | 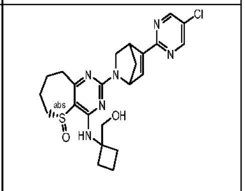 |
| 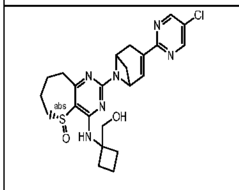 | 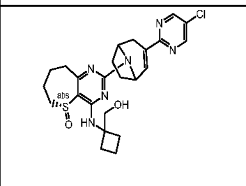 | 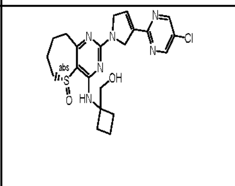 | 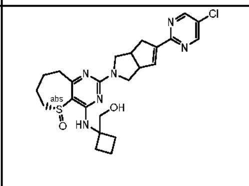 | 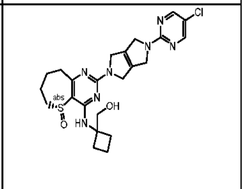 |
| 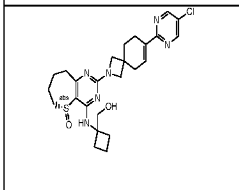 | 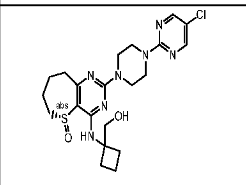 | 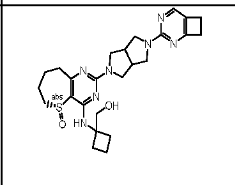 | 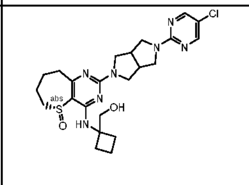 | 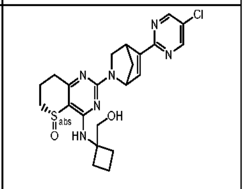 |
| 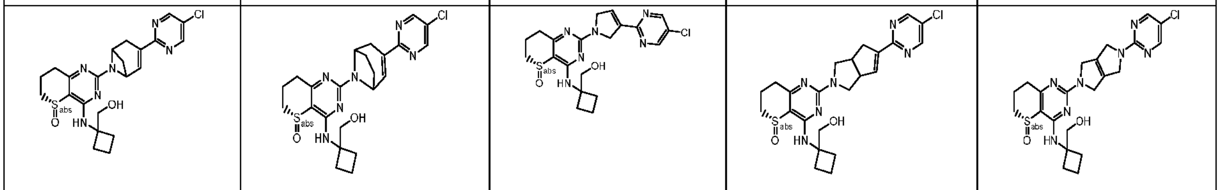 | | | | |
| 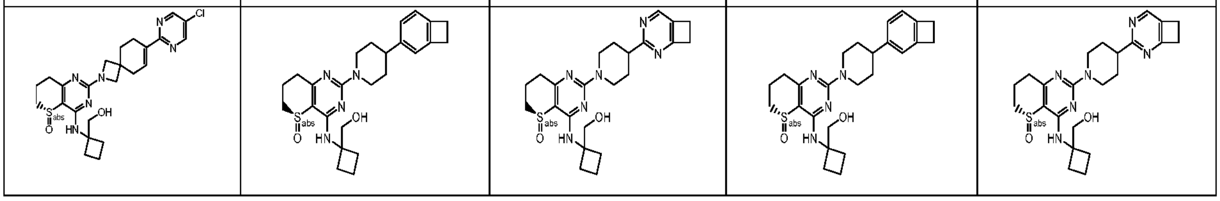 | | | | |

| | | | | |
|---|---|---|---|---|
| 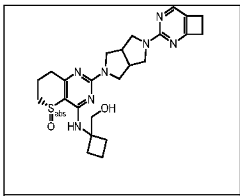 | 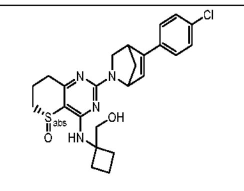 | 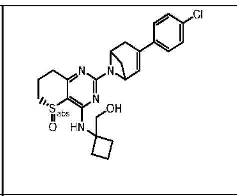 | 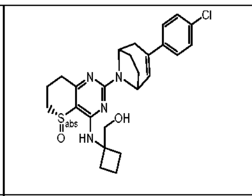 | 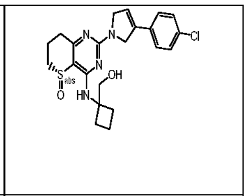 |
| 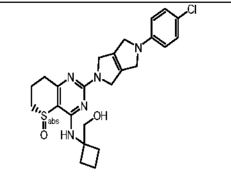 | 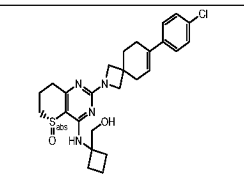 | 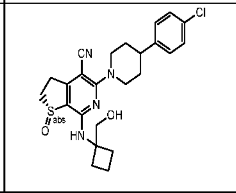 | 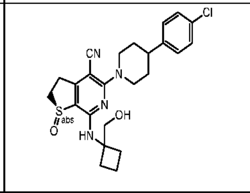 | 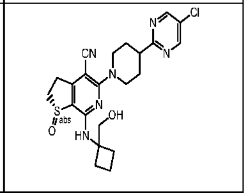 |
| 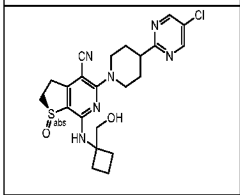 | 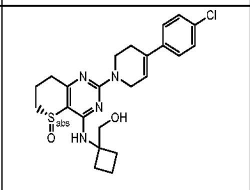 | 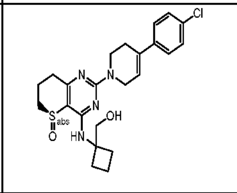 | 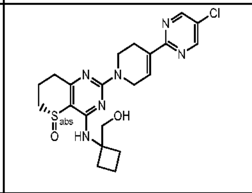 | 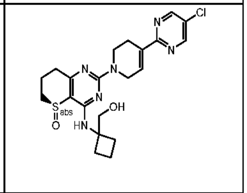 |
| 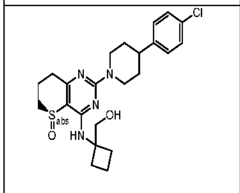 | 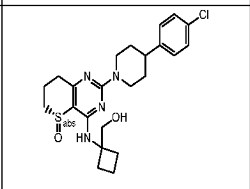 | 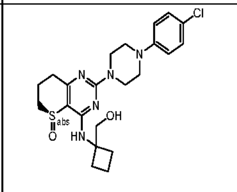 | 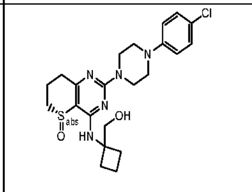 | 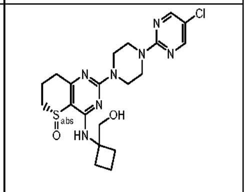 |
| 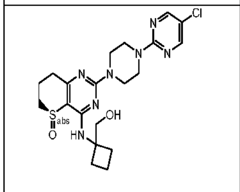 | 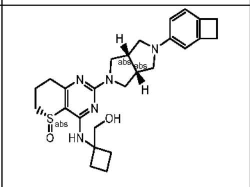 | 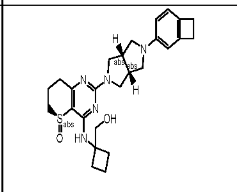 | 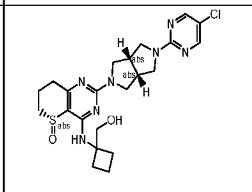 | 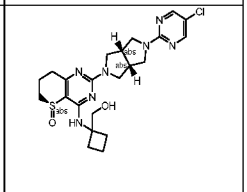 |
| 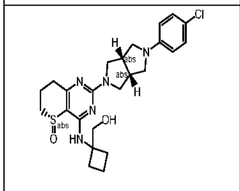 | 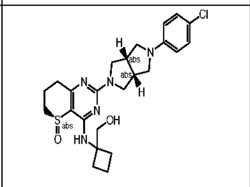 | 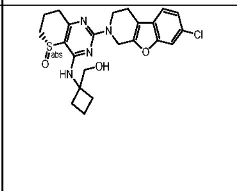 | 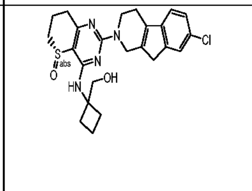 | 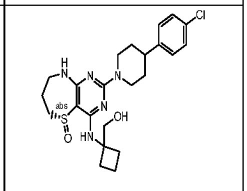 |
| 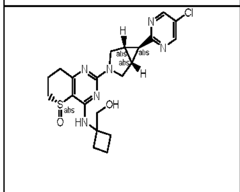 | 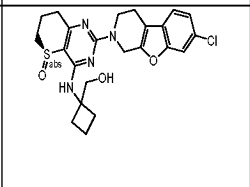 | 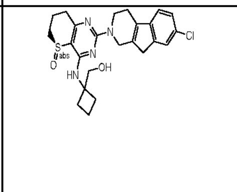 | 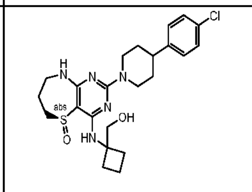 | 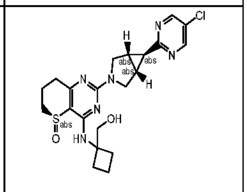 |
| 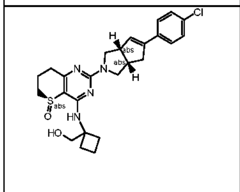 | 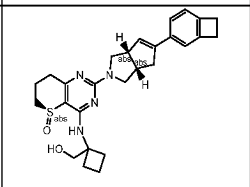 | 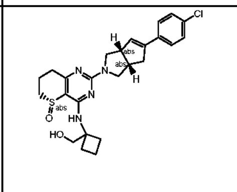 | 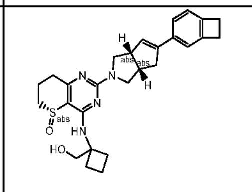 | 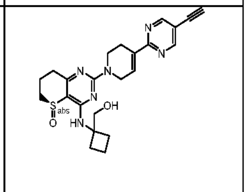 |
| | | | | |

| | | | | |
|---|---|---|---|---|
| 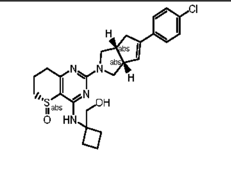 | 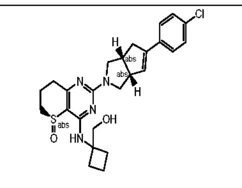 | 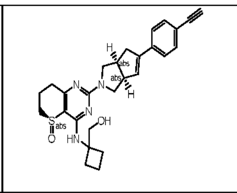 | 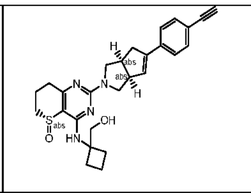 | 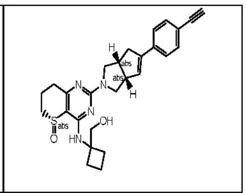 |
| 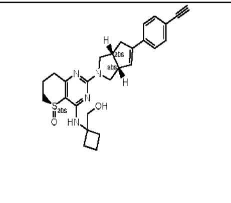 | 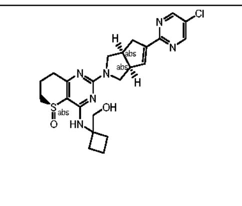 | 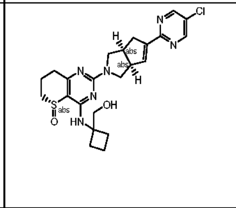 | 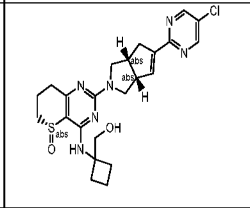 | 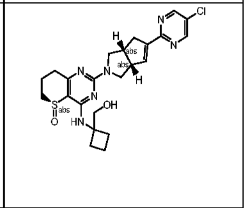 |
| 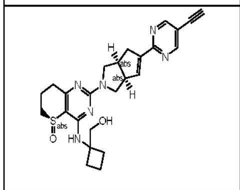 | 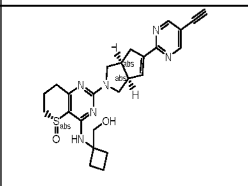 | 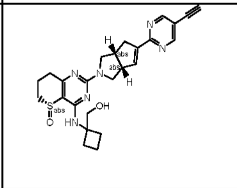 | 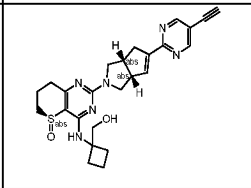 | 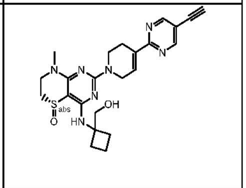 |
| 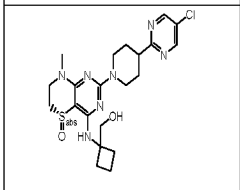 | 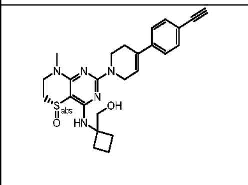 | 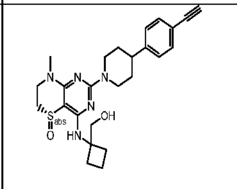 | 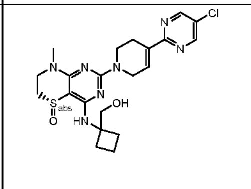 | 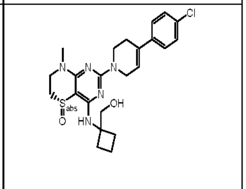 |
| 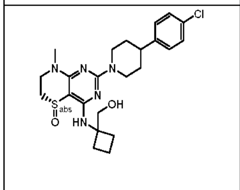 | 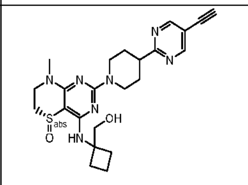 | 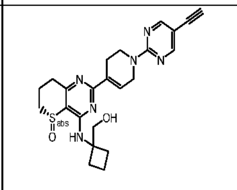 | 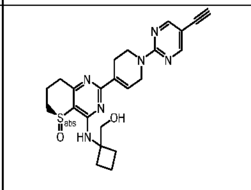 | 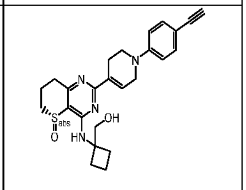 |
| 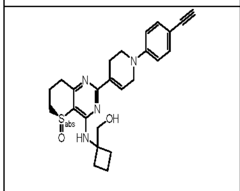 | 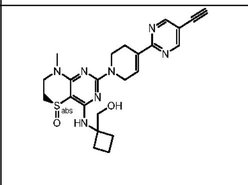 | 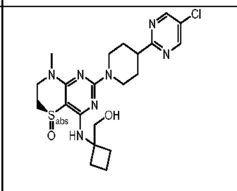 | 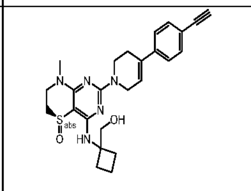 | 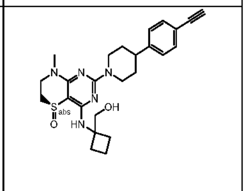 |
| 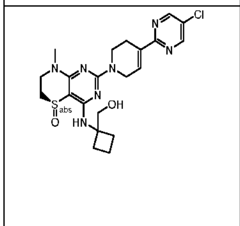 | 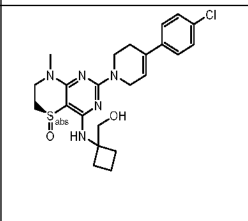 | 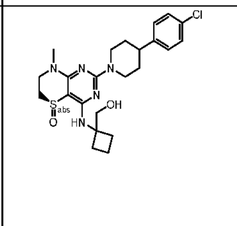 | 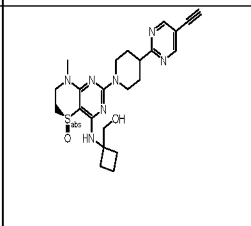 | 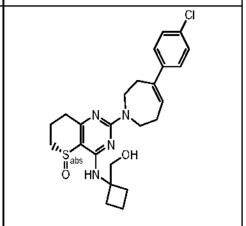 |
| 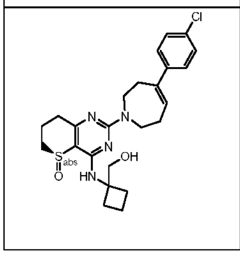 | 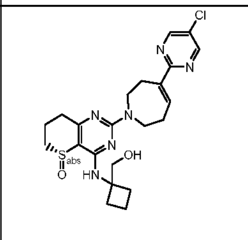 | 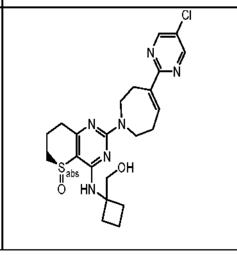 | 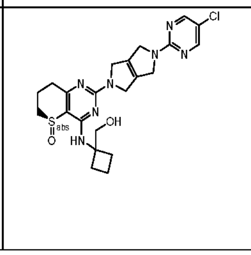 | 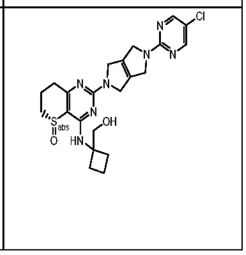 |

| | | | | |
|---|---|---|---|---|
| 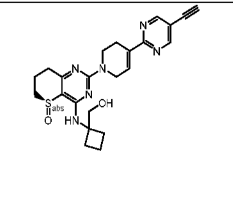 | 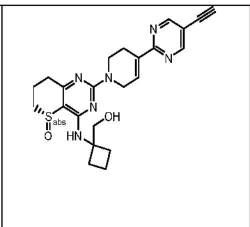 | 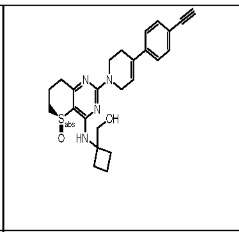 | 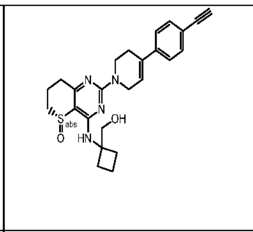 | 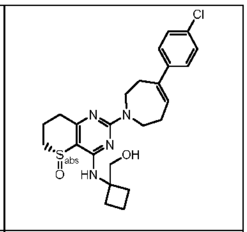 |
| 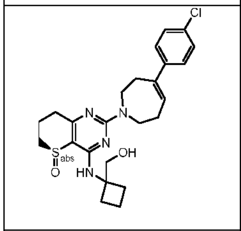 | 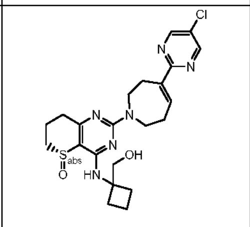 | 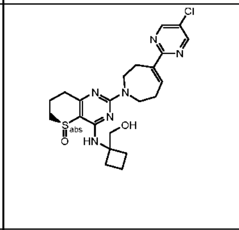 | 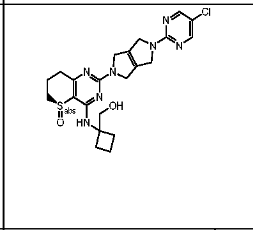 | 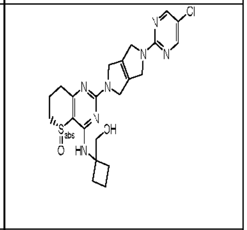 |
| 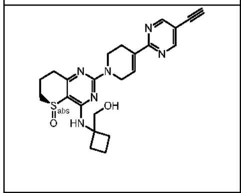 | 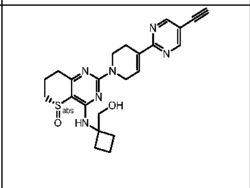 | 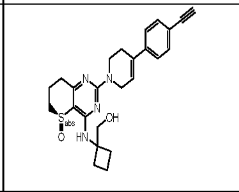 | 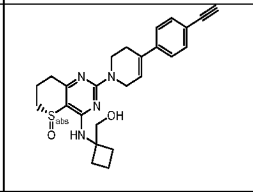 | 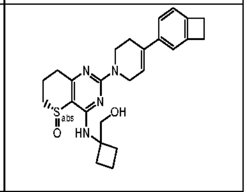 |
| 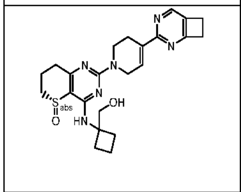 | 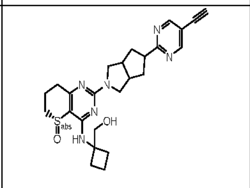 | 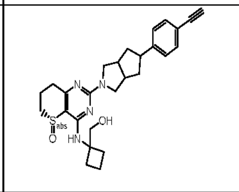 | 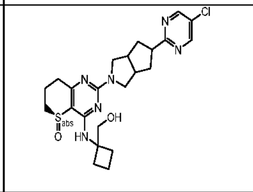 | 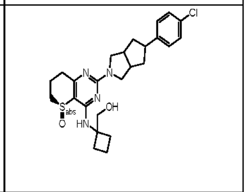 |
| 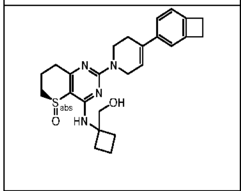 | 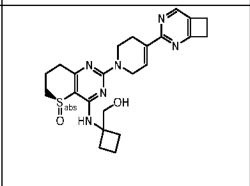 | 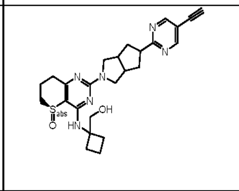 | 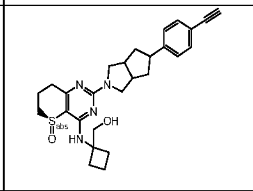 | 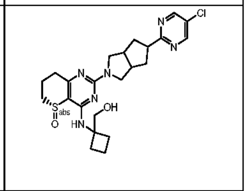 |
| 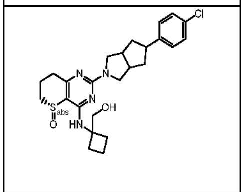 | 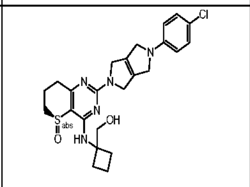 | 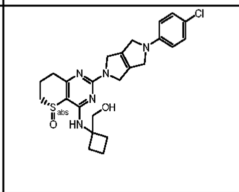 | 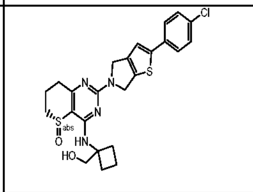 | 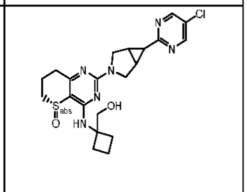 |
| 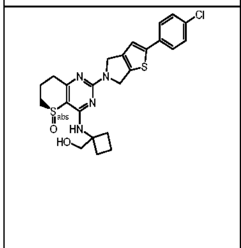 | 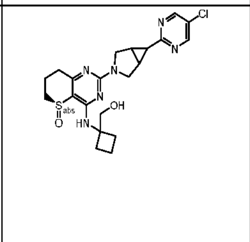 | 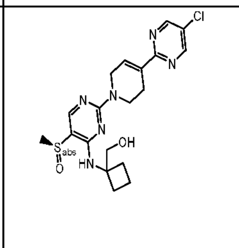 | 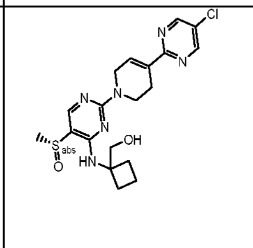 | 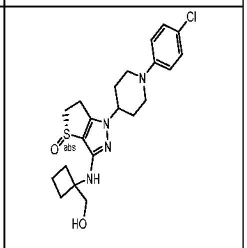 |
| 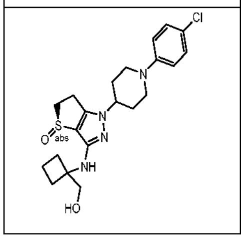 | 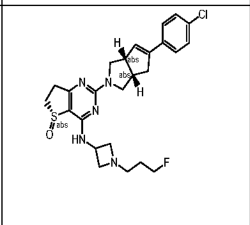 | 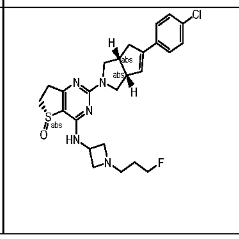 | 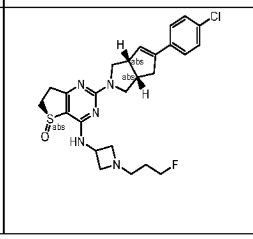 | 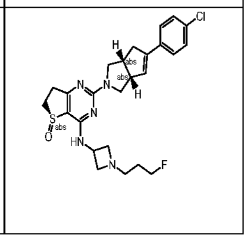 |

| | | | | |
|---|---|---|---|---|
| 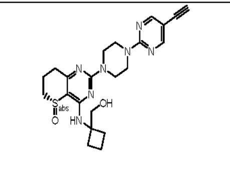 | 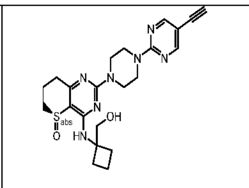 | 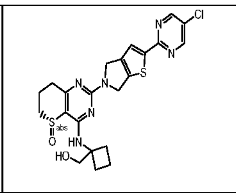 | 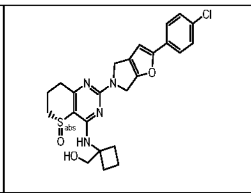 | 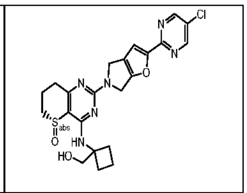 |
| 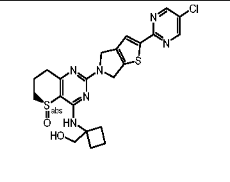 | 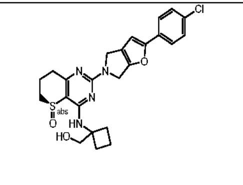 | 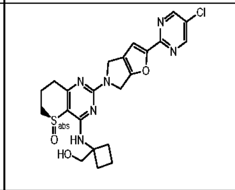 | 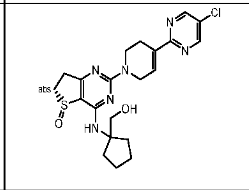 | 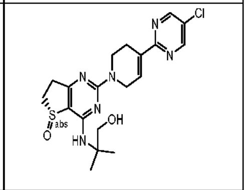 |
| 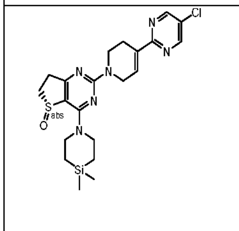 | 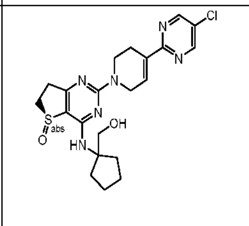 | 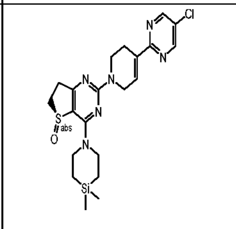 | 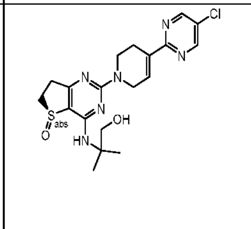 | 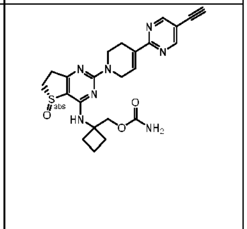 |
| 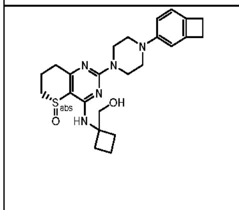 | 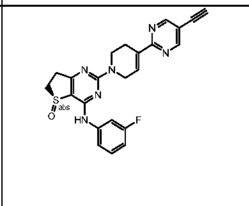 | 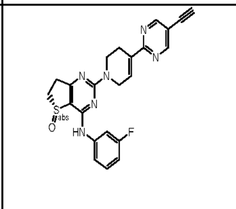 | 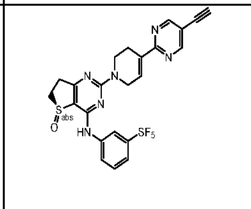 | 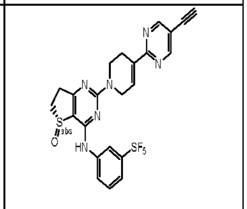 |
| 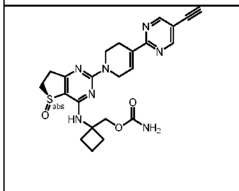 | 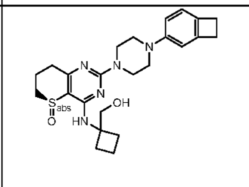 | 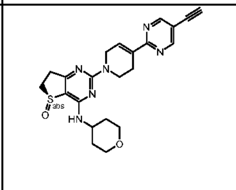 | 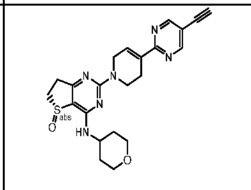 | 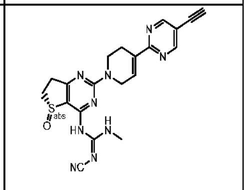 |
| 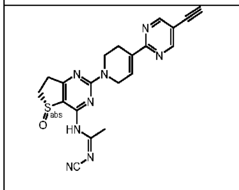 | 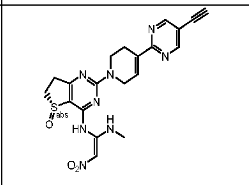 | 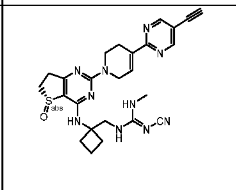 | 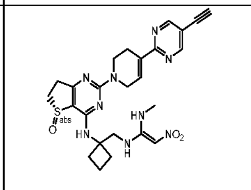 | 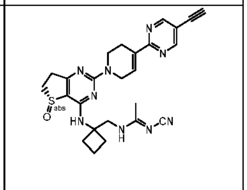 |
| 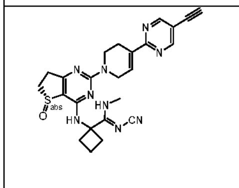 | 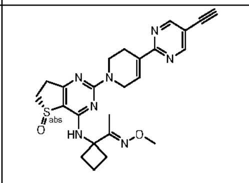 | 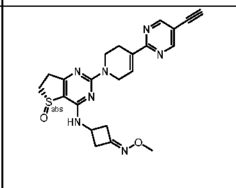 | 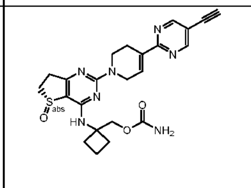 | 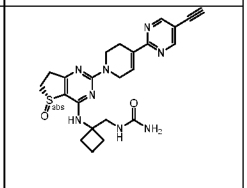 |
| 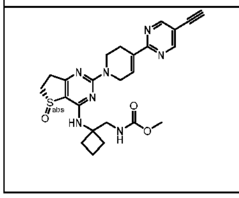 | 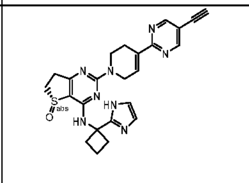 | 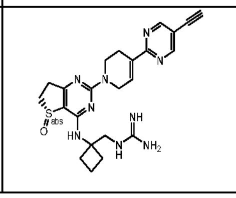 | 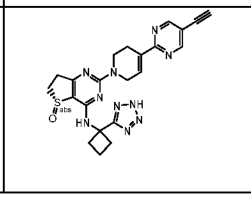 | 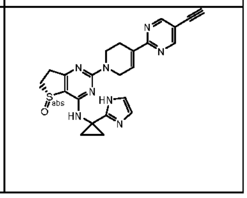 |

| | | | | |
|---|---|---|---|---|
| 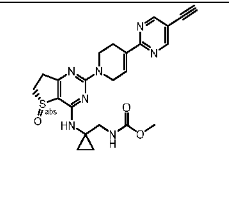 | 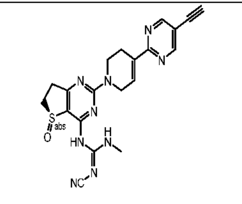 | 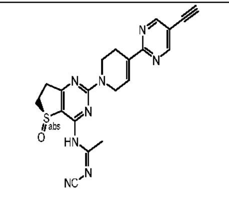 | 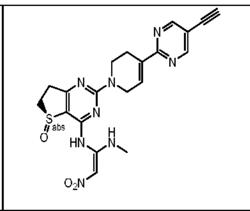 | 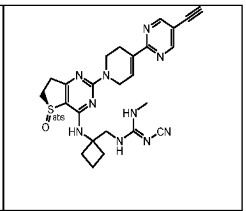 |
| 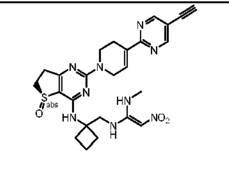 | 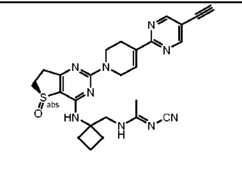 | 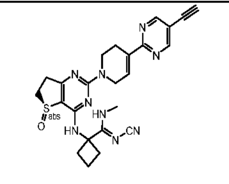 | 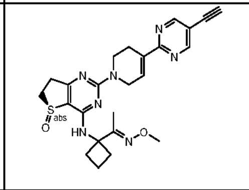 | 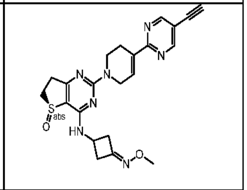 |
| 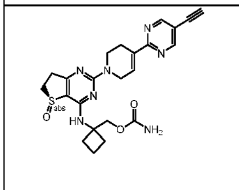 | 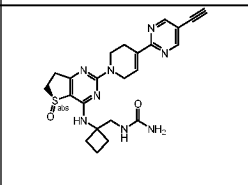 | 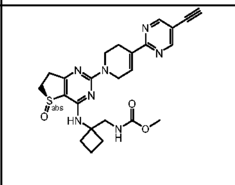 | 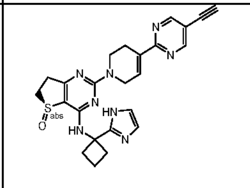 | 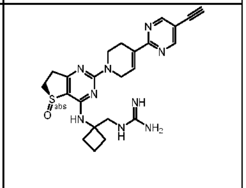 |
| 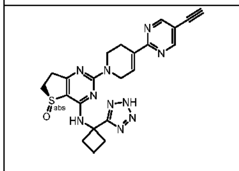 | 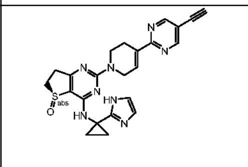 | 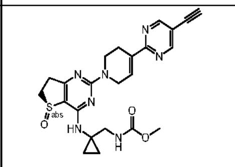 | | |
| 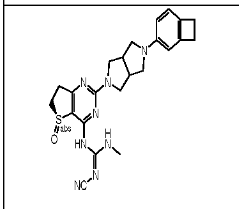 | 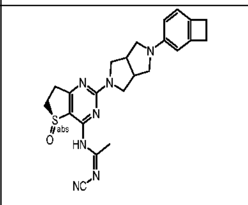 | 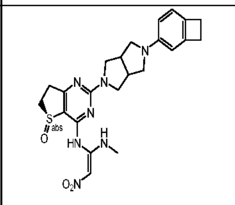 | 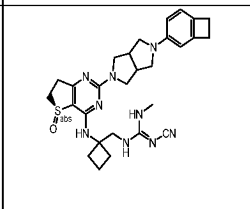 | 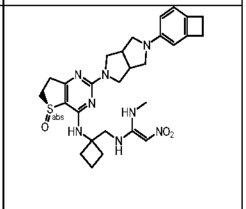 |
| 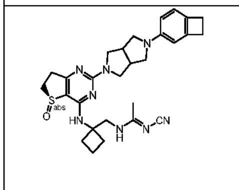 | 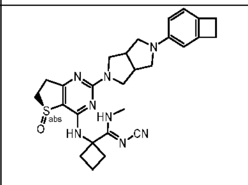 | 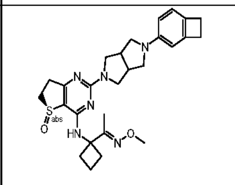 | 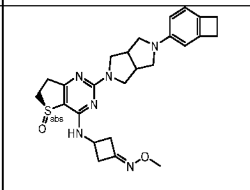 | 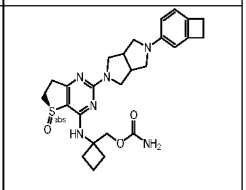 |
| 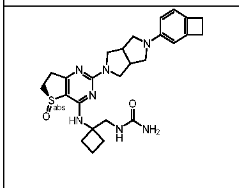 | 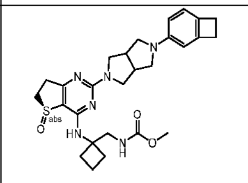 | 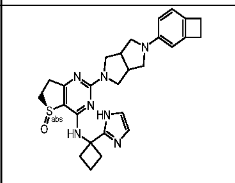 | 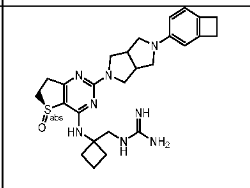 | 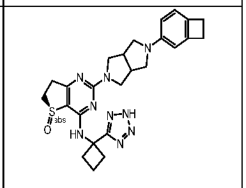 |
| 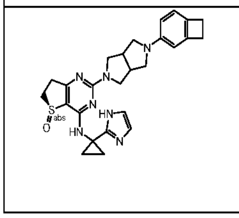 | 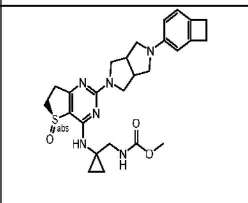 | 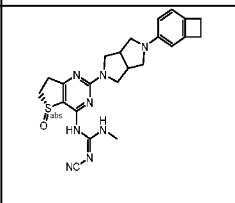 | 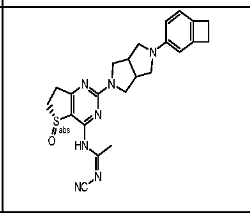 | 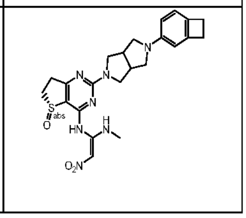 |

| | | | | |
|---|---|---|---|---|
| 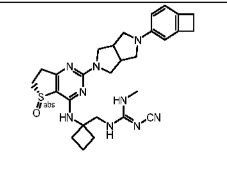 | 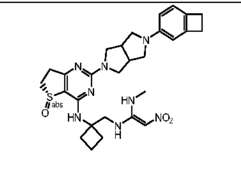 | 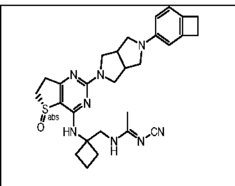 | 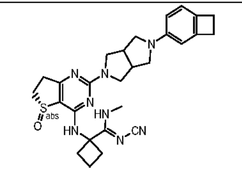 | 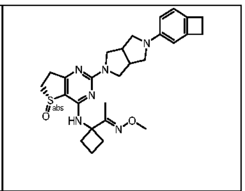 |
| 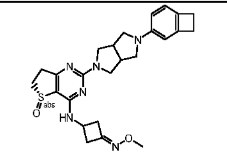 | 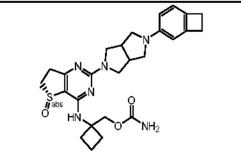 | 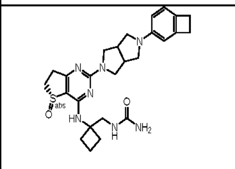 | 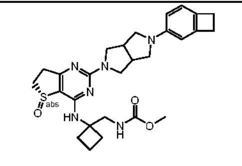 | 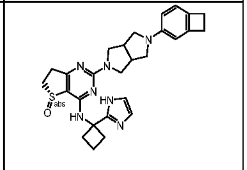 |
| 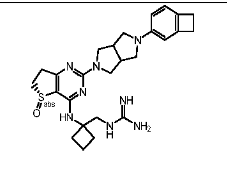 | 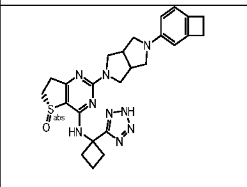 | 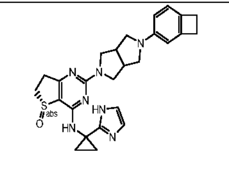 | 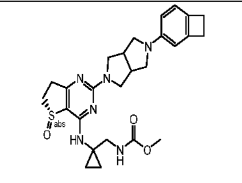 | 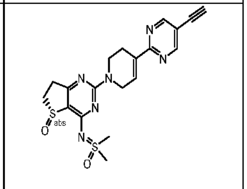 |
| 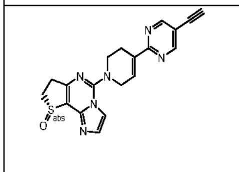 | 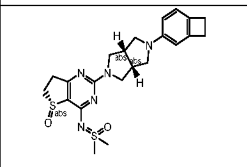 | 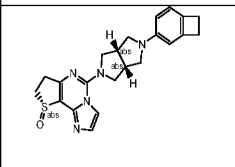 | 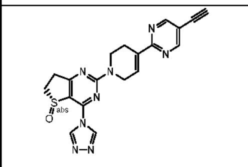 | 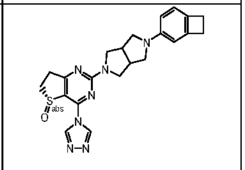 |
| 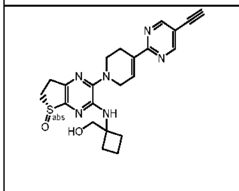 | 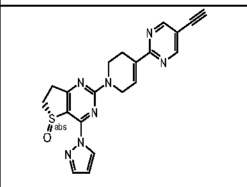 | 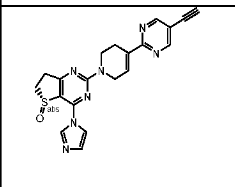 | 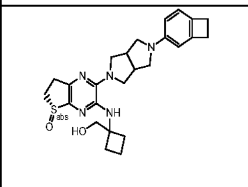 | 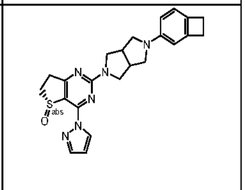 |
| 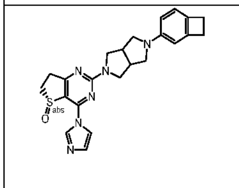 | 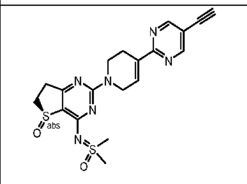 | 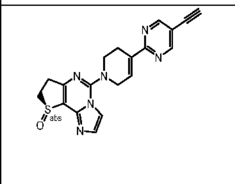 | 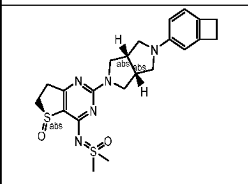 | 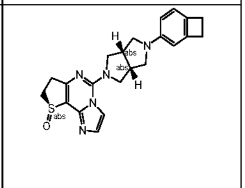 |
| 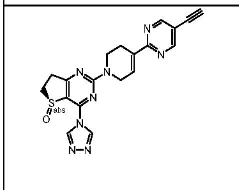 | 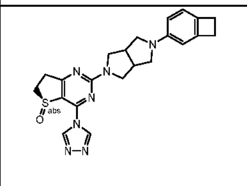 | 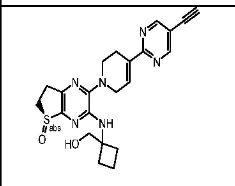 | 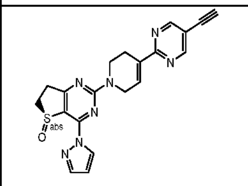 | 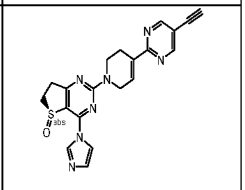 |
| 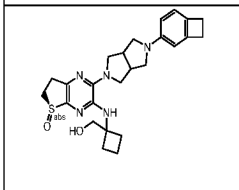 | 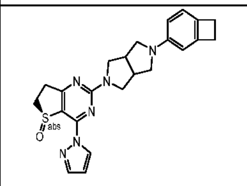 | 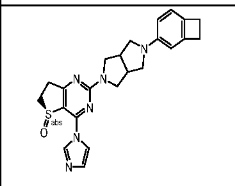 | 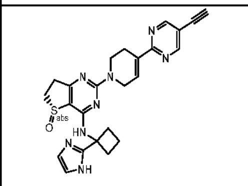 | 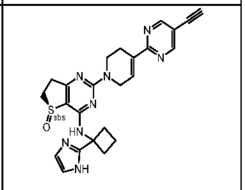 |
| | | | | |

| | | | | |
|---|---|---|---|---|
| 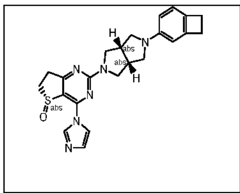 | 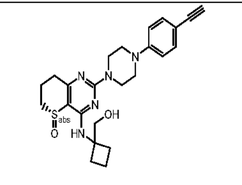 | 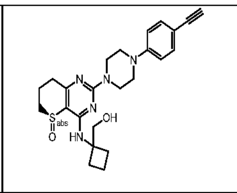 | 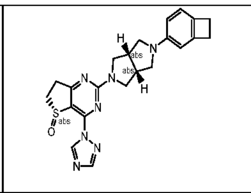 | 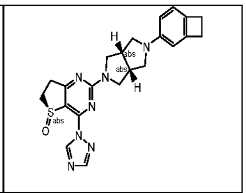 |
| 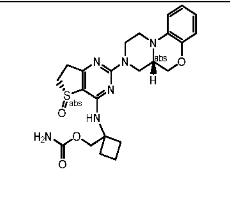 | 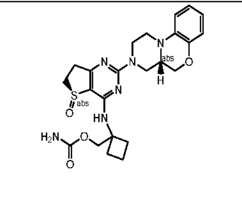 | 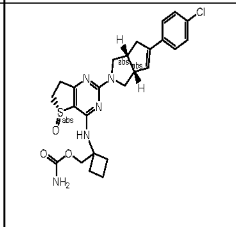 | 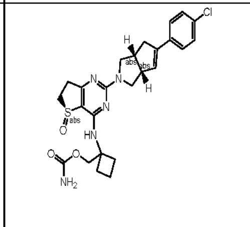 | 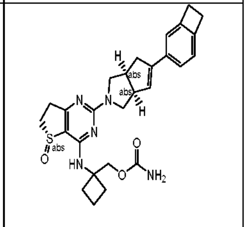 |
| 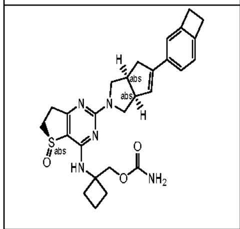 | 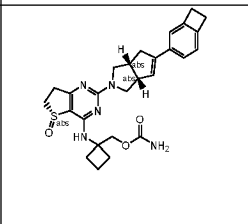 | 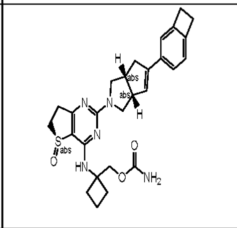 | 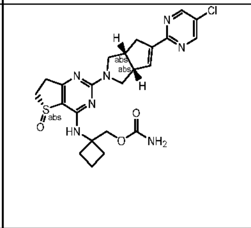 | 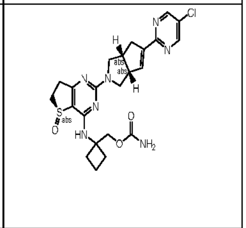 |
| 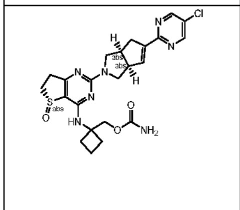 | 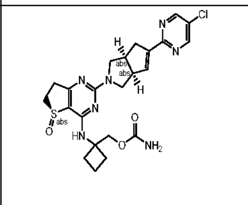 | 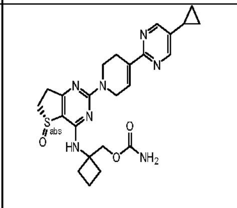 | 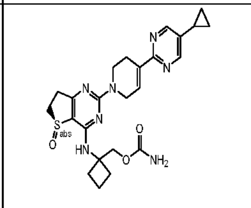 | 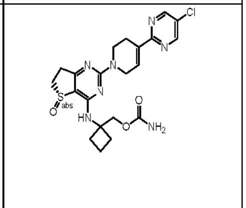 |
| 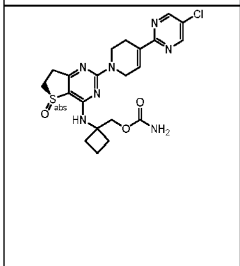 | 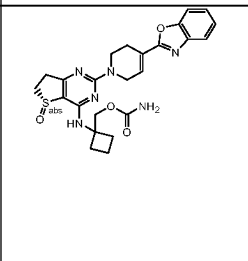 | 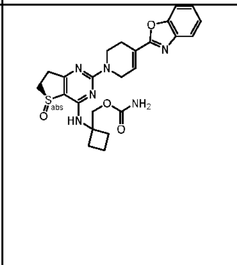 | 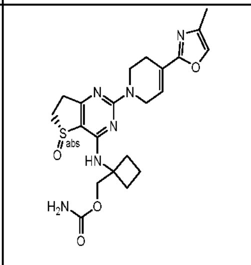 | 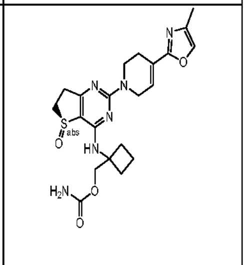 |
| 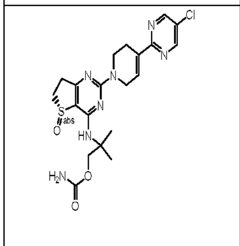 | 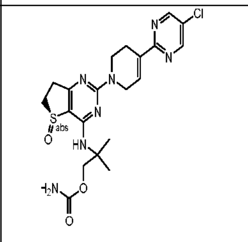 | 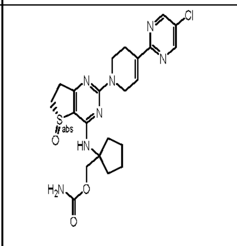 | 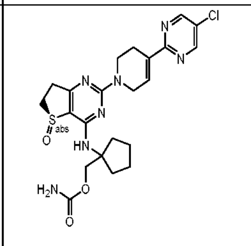 | 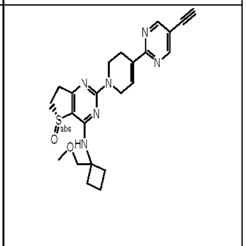 |
| 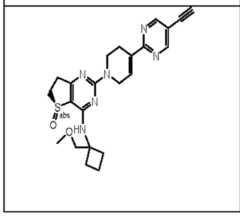 | 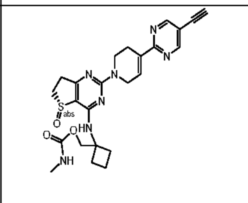 | 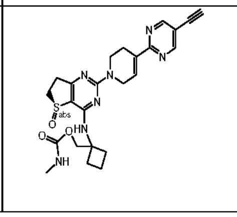 | 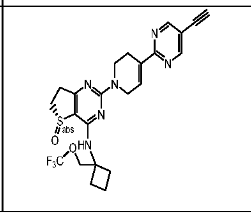 | 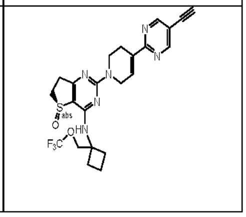 |

| | | | | |
|---|---|---|---|---|
| 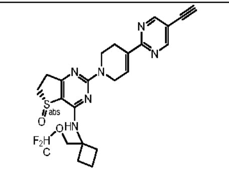 | 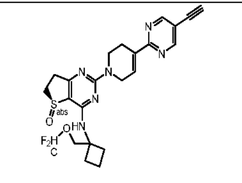 | 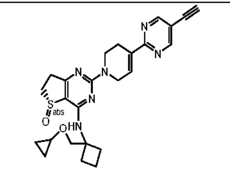 | 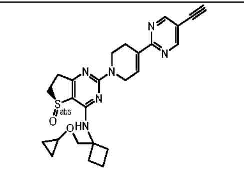 | 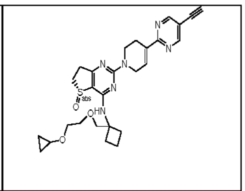 |
| 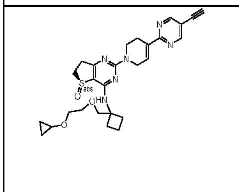 | 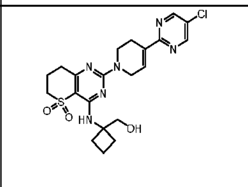 | 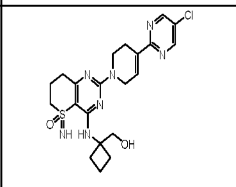 | 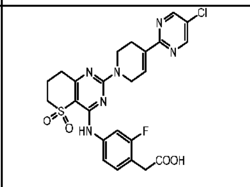 | 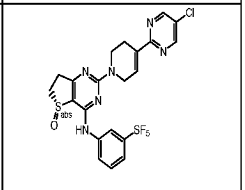 |
| 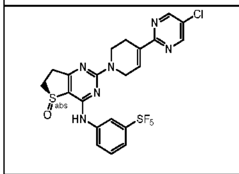 | 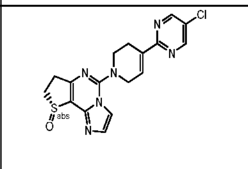 | 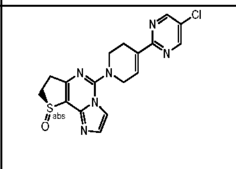 | 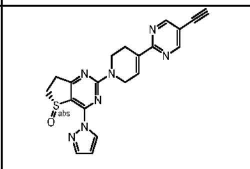 | 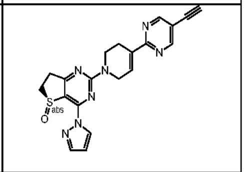 |
| 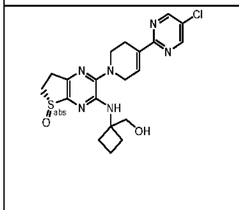 | 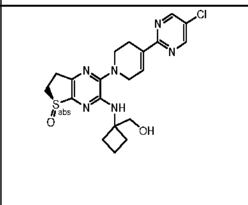 | 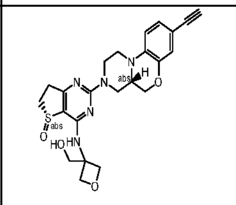 | 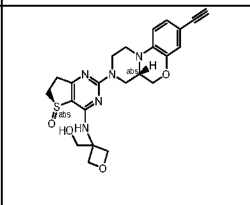 | 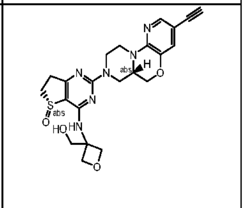 |
| 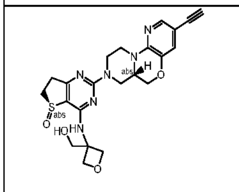 | 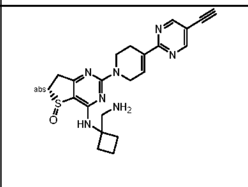 | 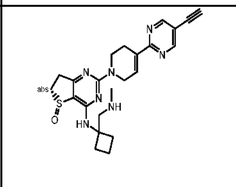 | 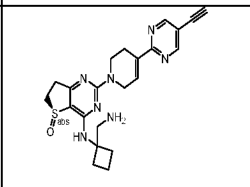 | 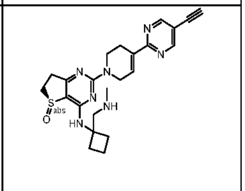 |
| 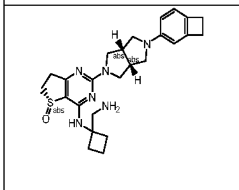 | 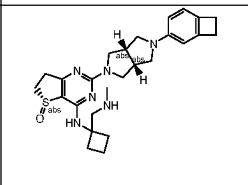 | 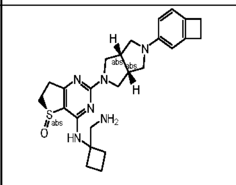 | 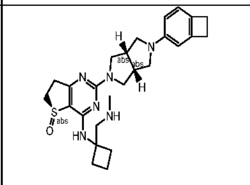 | 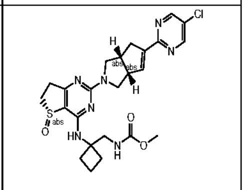 |
| 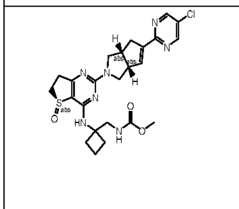 | 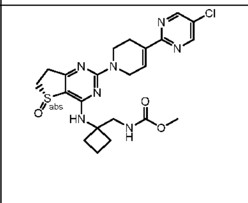 | 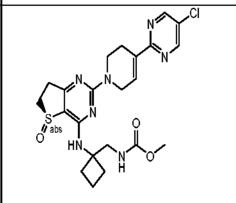 | 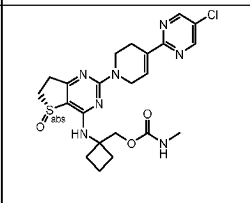 | 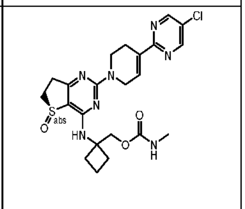 |
| 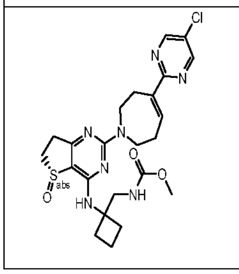 | 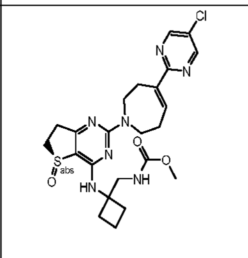 | 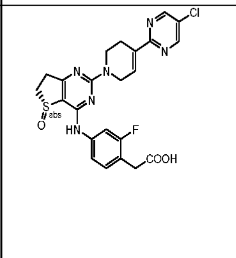 | 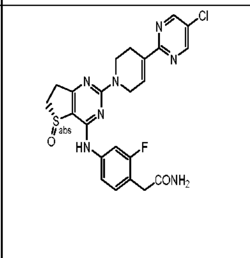 | 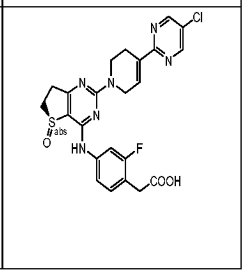 |

| | | | | |
|---|---|---|---|---|
| 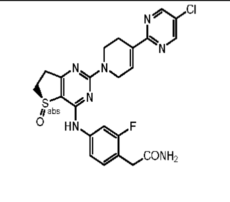 | 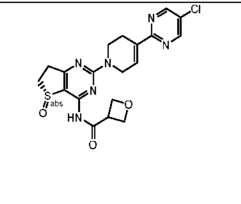 | 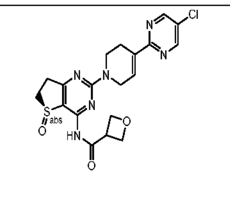 | 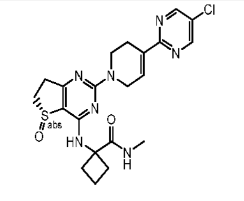 | 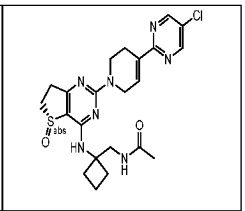 |
| 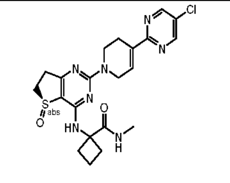 | 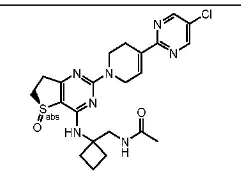 | 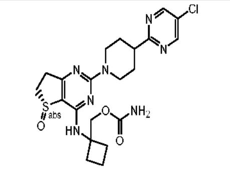 | 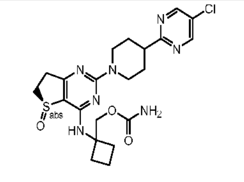 | 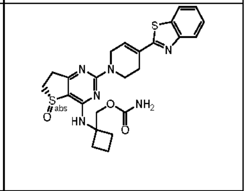 |
| 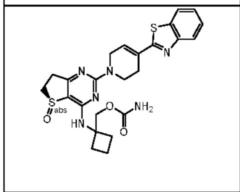 | 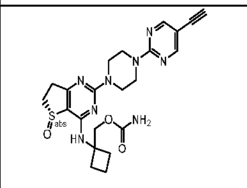 | 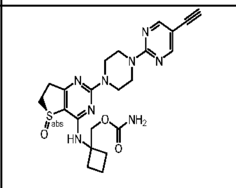 | 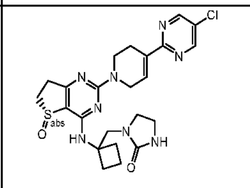 | 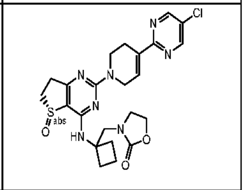 |
| 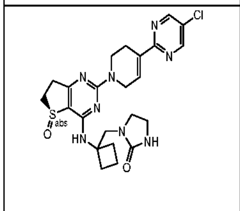 | 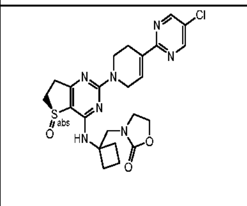 | 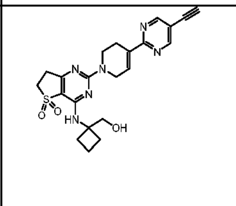 | 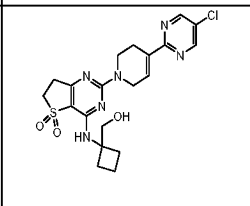 | 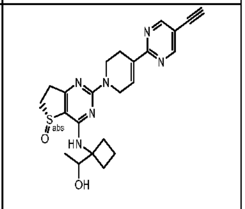 |
| 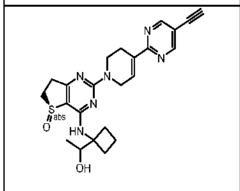 | 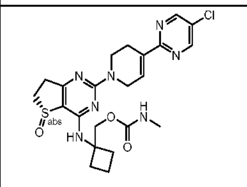 | 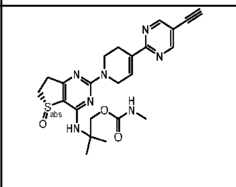 | 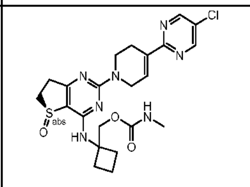 | 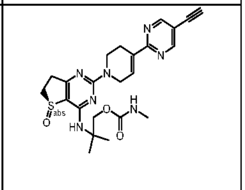 |
| 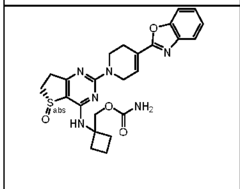 | 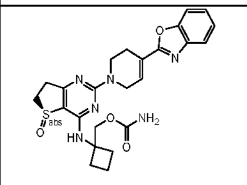 | 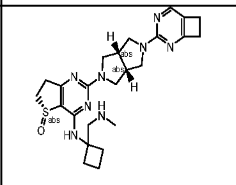 | 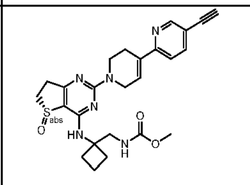 | 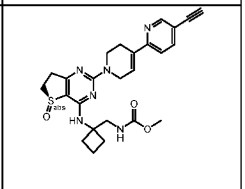 |
| 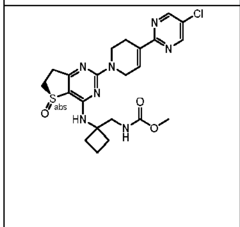 | 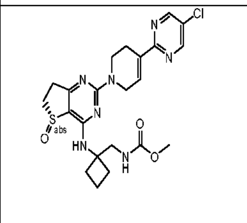 | 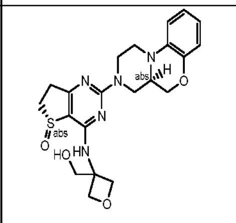 | 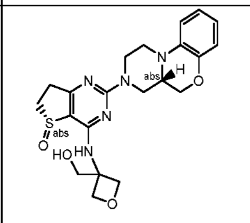 | 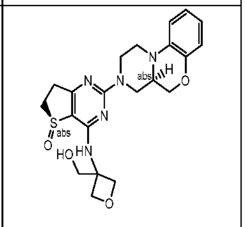 |
| 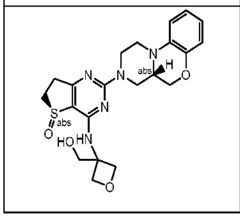 | 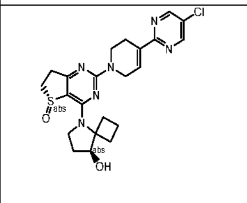 | 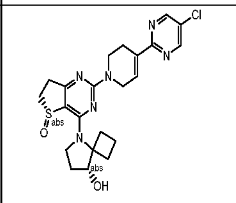 | 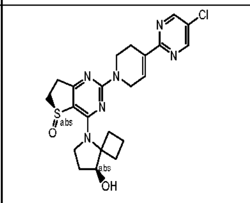 | 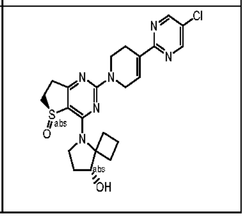 |

| | | | | |
|---|---|---|---|---|
| 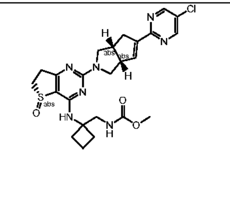 | 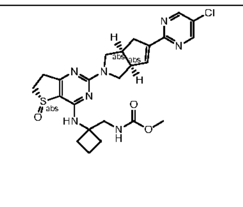 | 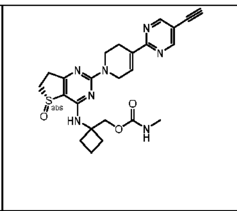 | 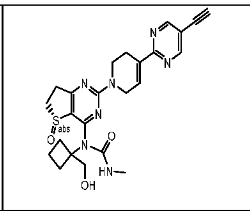 | 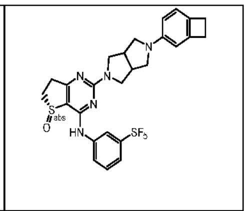 |
| 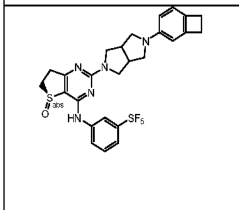 | 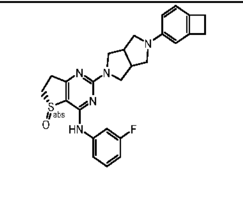 | 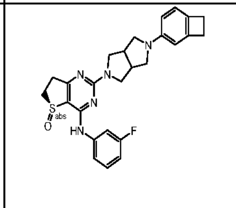 | 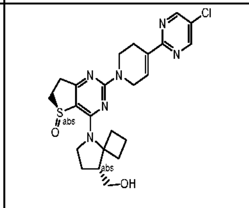 | 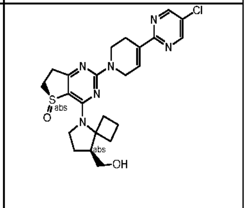 |
| 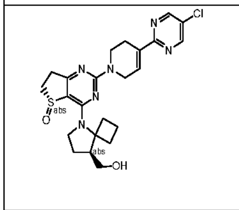 | 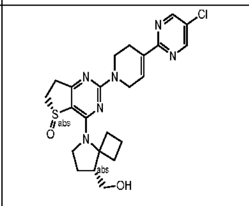 | 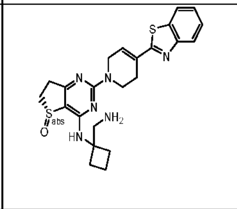 | | 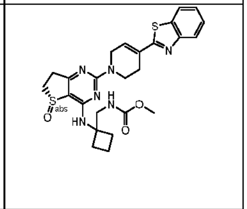 |
| 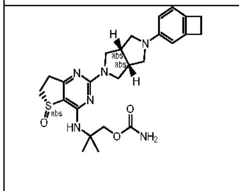 | 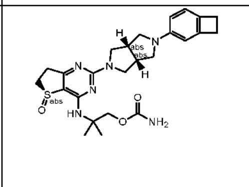 | 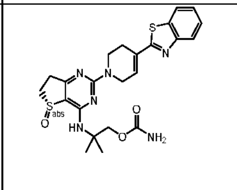 | 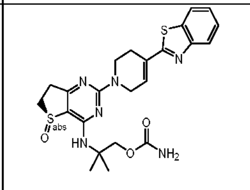 | 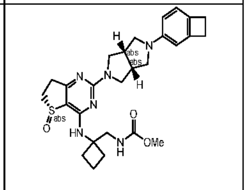 |
| 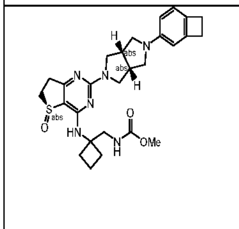 | 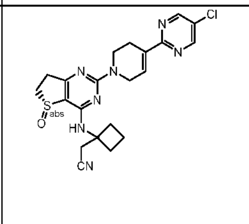 | 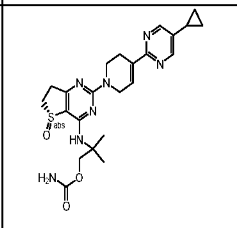 | 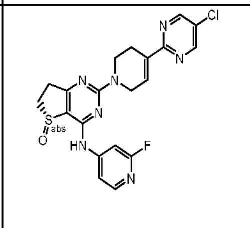 | 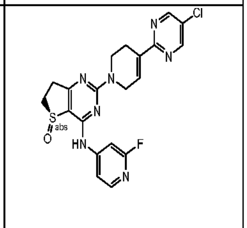 |
| 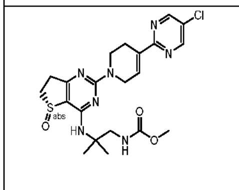 | 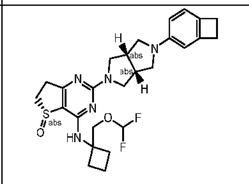 | 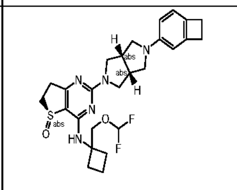 | 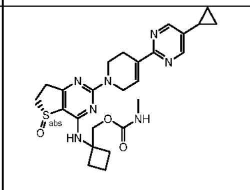 | 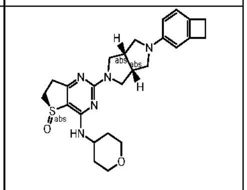 |
| 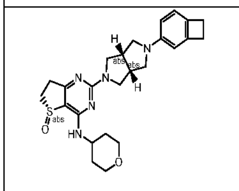 | 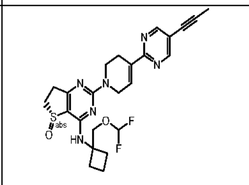 | 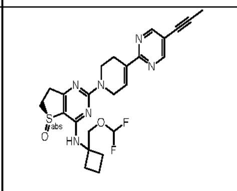 | 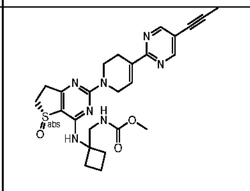 | 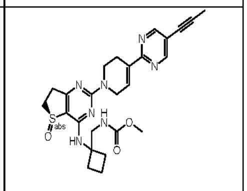 |
| 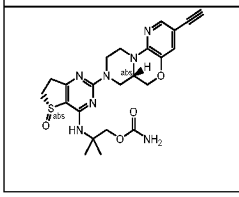 | 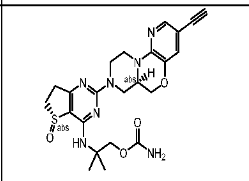 | 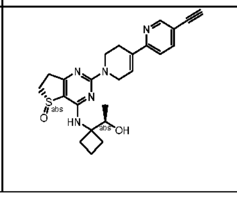 | 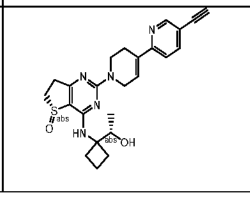 | 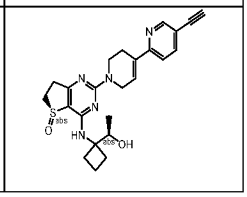 |

| | | | | |
|---|---|---|---|---|
| 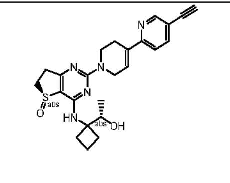 | 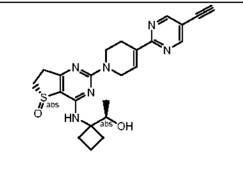 | 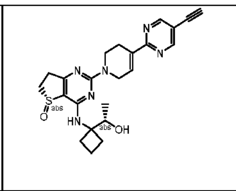 | 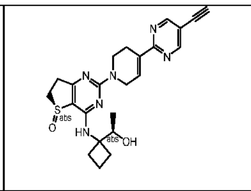 | 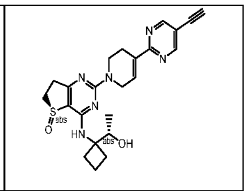 |
| 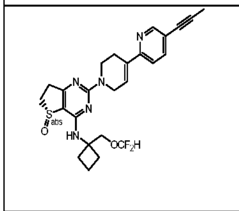 | 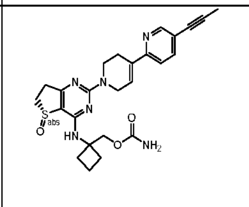 | 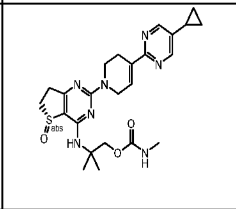 | 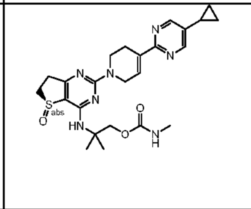 | 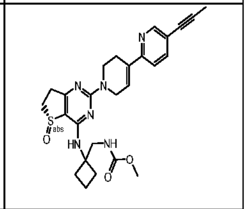 |
| 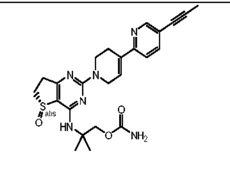 | 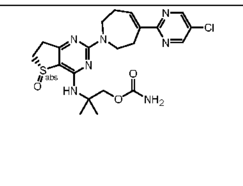 | 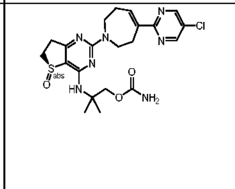 | 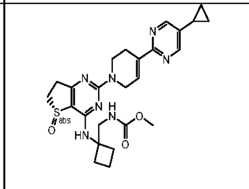 | 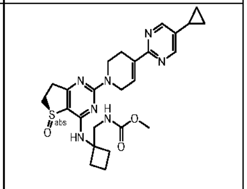 |
| 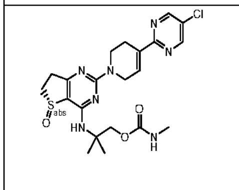 | 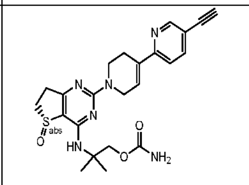 | 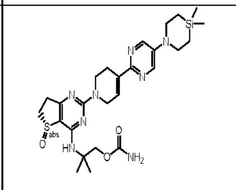 | 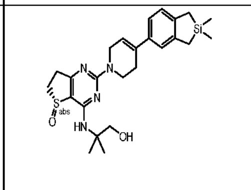 | 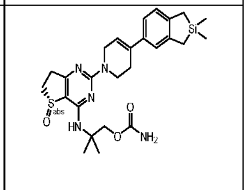 |
| 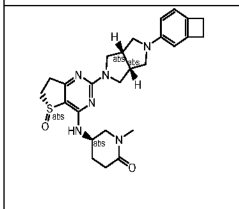 | 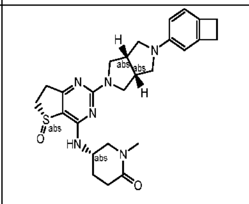 | 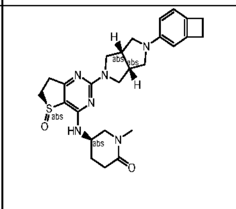 | 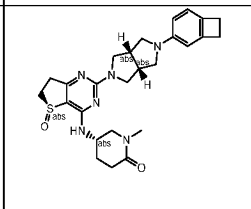 | 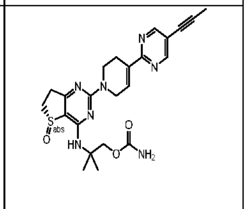 |
| 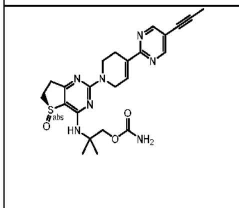 | 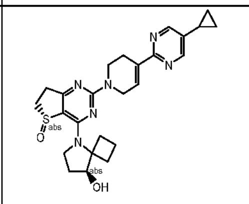 | 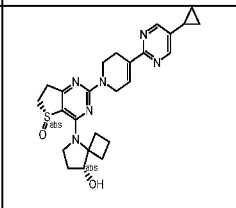 | 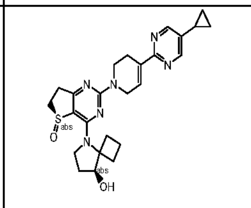 | 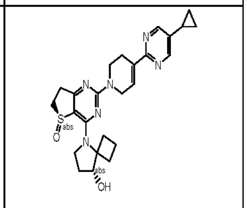 |
| 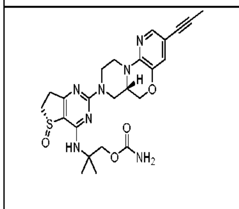 | 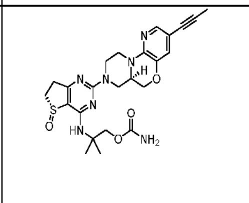 | 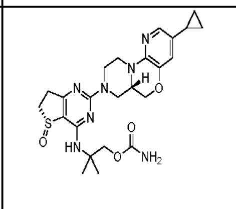 | 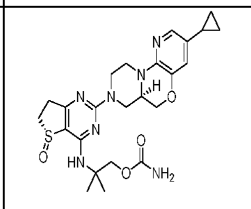 | 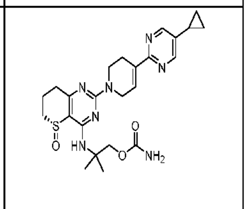 |
| 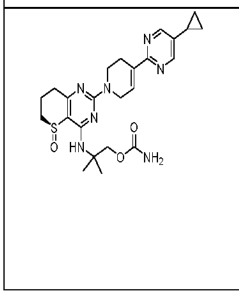 | 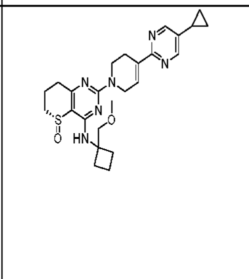 | 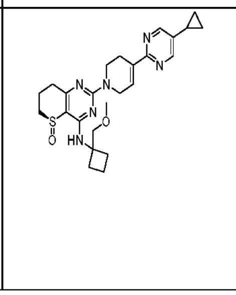 | 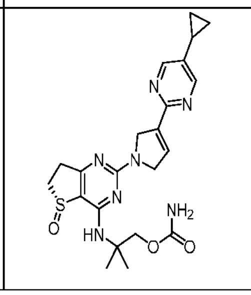 | 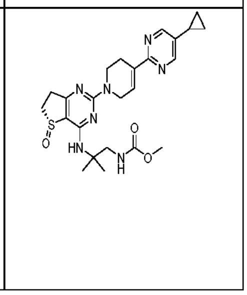 |

| | | | | |
|---|---|---|---|---|
| 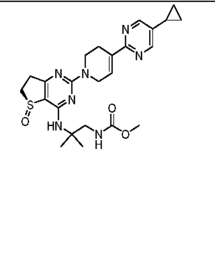 | 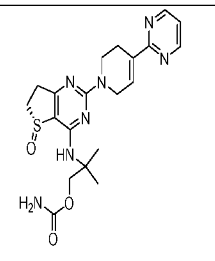 | 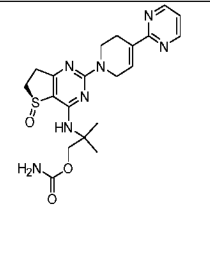 | 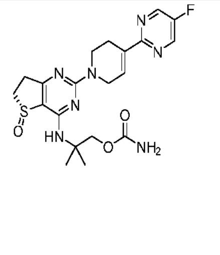 | 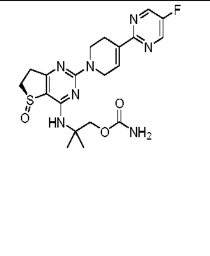 |
| 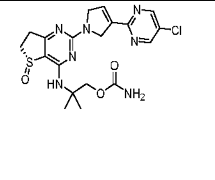 | 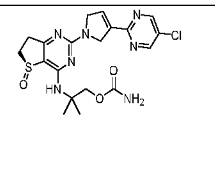 | 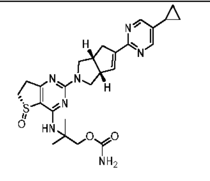 | 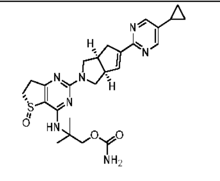 | 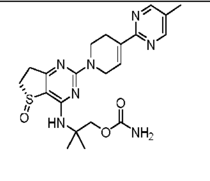 |
| 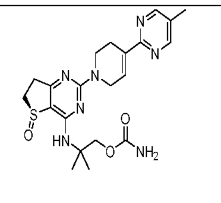 | 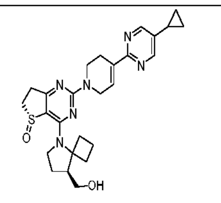 | 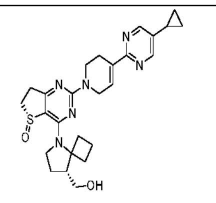 | 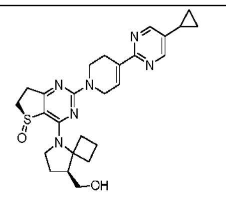 | 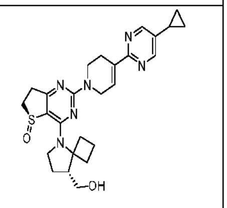 |
| 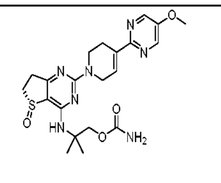 | 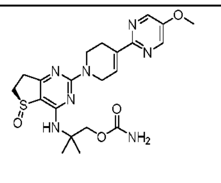 | 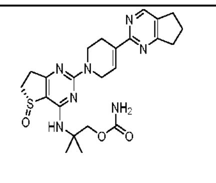 | 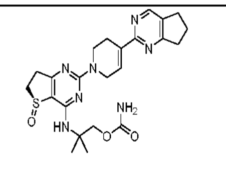 | 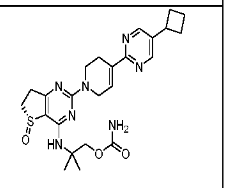 |
| 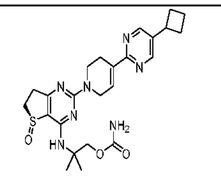 | 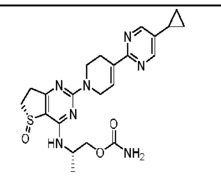 | 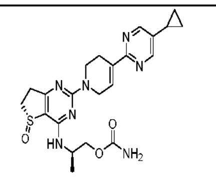 | 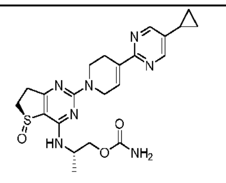 | 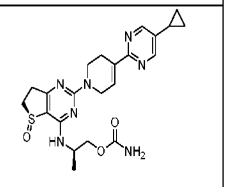 |
| 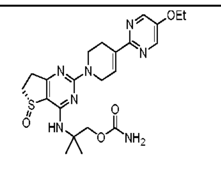 | 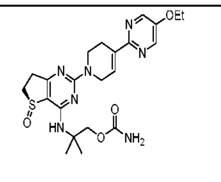 | 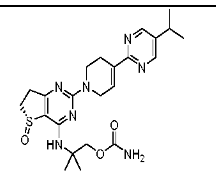 | 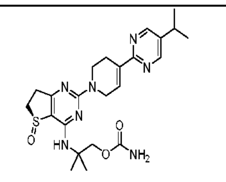 | 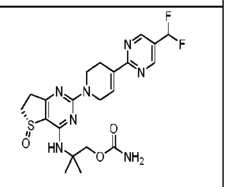 |
| 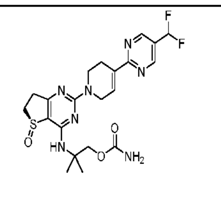 | 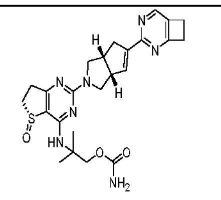 | 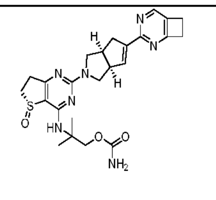 | 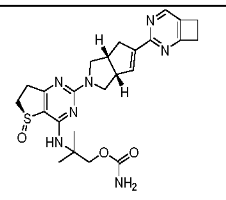 | 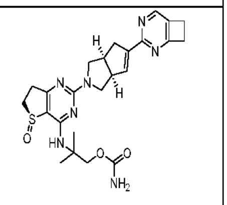 |
| 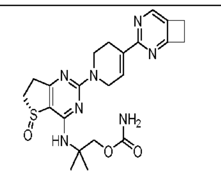 | 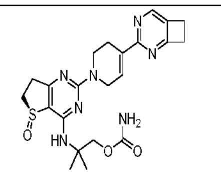 | 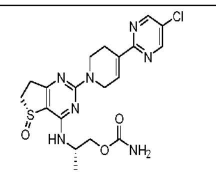 | 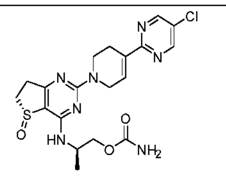 | 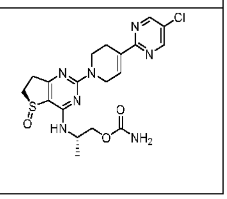 |

| | | | | |
|---|---|---|---|---|
| 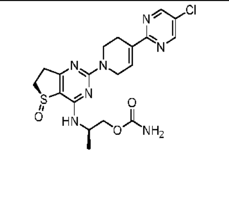 | 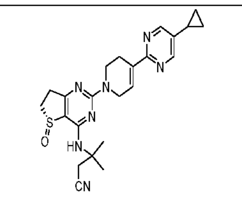 | 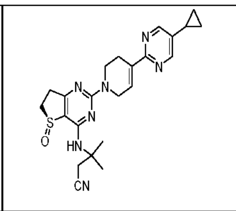 | 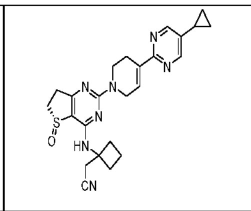 | 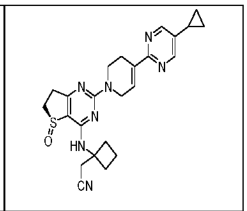 |
| 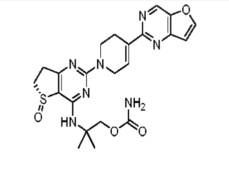 | 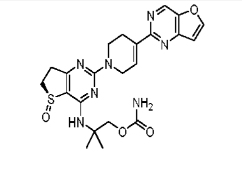 | 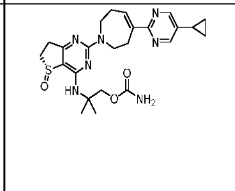 | 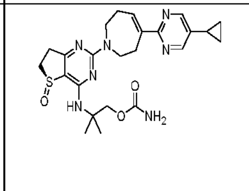 | 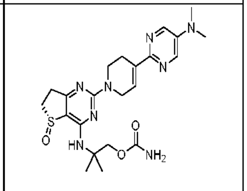 |
| 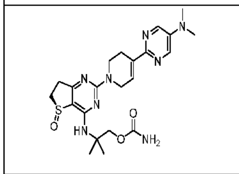 | 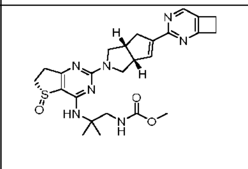 | 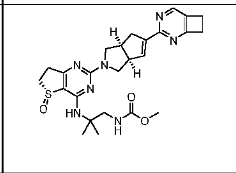 | 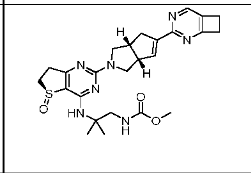 | 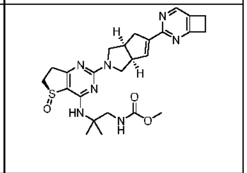 |
| 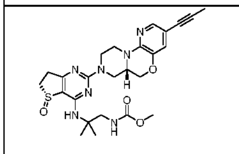 | 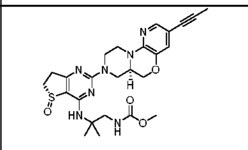 | 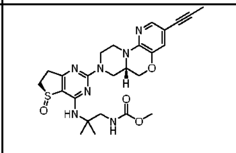 | 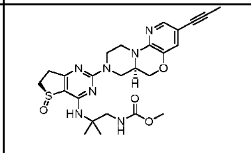 | 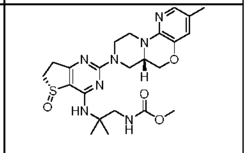 |
| 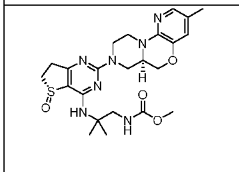 | 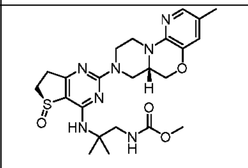 | 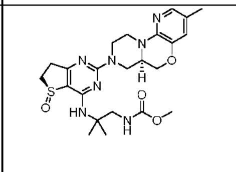 | 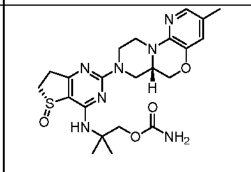 | 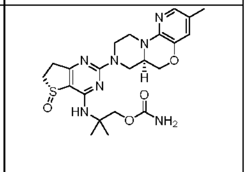 |
| 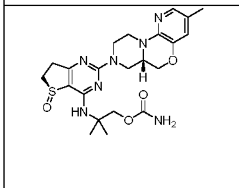 | 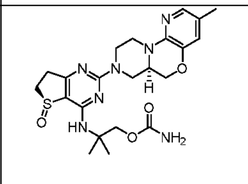 | 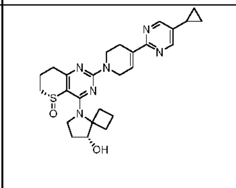 | 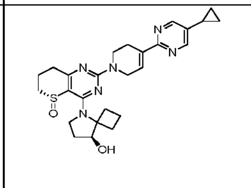 | 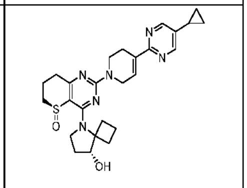 |
| 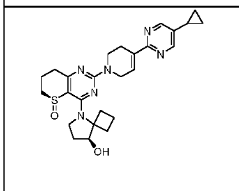 | 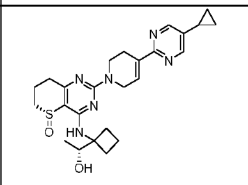 | 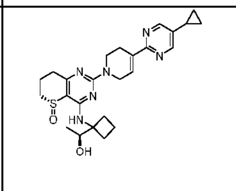 | 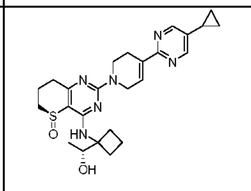 | 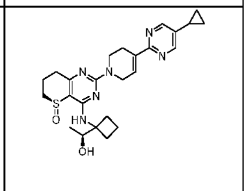 |
| 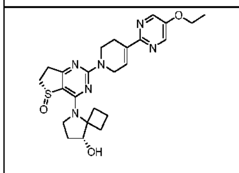 | 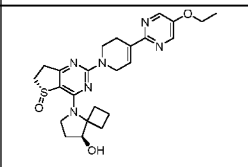 | 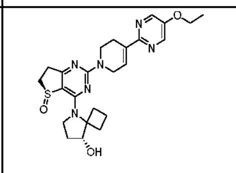 | 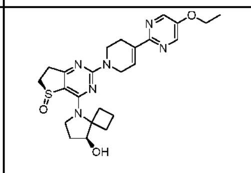 | 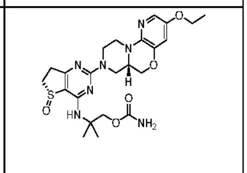 |
| | | | | |

"abs" denotes absolute configuration.

The present disclosure further provides a composition or pharmaceutical preparation comprising the compound or the stereoisomer or pharmaceutically acceptable salt thereof according to any one of the preceding solutions, and a pharmaceutically acceptable carrier and/or auxiliary material. The pharmaceutical composition can be in a unit preparation form (the unit preparation is also referred to as "preparation specification").

Furthermore, the composition or pharmaceutical preparation of the present disclosure comprises 1-1500 mg of

the compound or the stereoisomer or pharmaceutically acceptable salt thereof according to any one of the preceding solutions, and a pharmaceutically acceptable carrier and/or auxiliary material.

The present disclosure further provides the use of the compound or the stereoisomer or pharmaceutically acceptable salt thereof according to any one of the preceding solutions in the preparation of a drug for treating/preventing a PDE4B-mediated disease. Furthermore, the PDE4B-mediated disease is a cancer, COPD, idiopathic pulmonary fibrosis, or an interstitial lung disease.

The present disclosure further provides a method for treating a disease in a mammal or human, comprising administering to a subject a therapeutically effective amount of the compound or the stereoisomer or pharmaceutically acceptable salt thereof as shown in any one of the preceding solutions, wherein the disease is preferably a cancer, COPD, idiopathic pulmonary fibrosis, or an interstitial lung disease, and the therapeutically effective amount is preferably 1-1500 mg. In some embodiments, the mammal described in the present disclosure does not include human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms, or causes of the disease, or any other desired change in the biological system. For example, an "effective amount" in terms of the therapeutic use is an amount comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; and 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg.

In some embodiments, the pharmaceutical composition or preparation of the present disclosure contains the above-mentioned therapeutically effective amount of the compound, or the stereoisomer, solvate, or pharmaceutically acceptable salt thereof according to the present disclosure.

The present disclosure relates to a pharmaceutical composition or pharmaceutical preparation comprising a therapeutically effective amount of the compound or the stereoisomer or pharmaceutically acceptable salt thereof according to the present disclosure, and a carrier and/or auxiliary material. The pharmaceutical composition can be in a unit preparation form (the amount of the active drug in the unit preparation is also referred to as "preparation specification"). In some embodiments, the pharmaceutical composition comprises, without limitation, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1500 mg of the compound or the stereoisomer or pharmaceutically acceptable salt thereof according to the present disclosure.

A method for treating a disease in a mammal is provided, which comprises administering to a subject a therapeutically effective amount of the compound or the stereoisomer or pharmaceutically acceptable salt thereof according to the present disclosure, and a pharmaceutically acceptable carrier and/or auxiliary material, wherein the therapeutically effective amount is preferably 1-1500 mg, and the disease is preferably a cancer, COPD, idiopathic pulmonary fibrosis, or an interstitial lung disease.

A method for treating a disease in a mammal or human is provided, which comprises administering to a subject the compound or the stereoisomer or pharmaceutically acceptable salt thereof according to the present disclosure, as a drug, and a pharmaceutically acceptable carrier and/or auxiliary material at a daily dose of 1-1500 mg/day, wherein the daily dose may be a single dose or divided doses. In some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day. In some embodiments, the daily dose includes but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500 mg/day.

The present disclosure relates to a kit, which may comprise a composition in a single-dose or multi-dose form, wherein the kit comprises the compound or the stereoisomer or pharmaceutically acceptable salt thereof according to the present disclosure, and the amount of the compound or the stereoisomer or pharmaceutically acceptable salt thereof according to the present disclosure is the same as that in the above-mentioned pharmaceutical composition.
In the present disclosure, the amount of the compound or the stereoisomer or pharmaceutically acceptable salt thereof according to the present disclosure is calculated in the form of a free base in each case.
The term "preparation specification" refers to the weight of the active drug contained in each vial, tablet, or other unit preparation.

## Synthesis route

Methods for preparing PDE4B inhibitors are introduced in patent documents such as WO 2013026797 A1. Those skilled in the art would prepare the compound of the present disclosure in conjunction with this document with a known organic synthesis technique using commercially available chemicals and (or) compounds described in chemical documents as starting materials. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.
Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalog are optionally prepared by custom chemical synthesis plants, wherein many of standard chemical supply plants (for example, those listed above) provide custom synthesis services.

## Terminology

Unless otherwise specially specified, the terms in the present disclosure have the following meanings.
The term "halogen" herein refers to F, Cl, Br, I, or isotopes thereof.
The term "halo" or "substituted with halogen" means that a hydrogen atom is replaced with one or more groups selected from F, Cl, Br, I, or isotopes thereof, wherein the upper limit of the number of halogen substituents is equal to the sum of the number of hydrogens that can be replaced in the group to be substituted. Without particular limitation, the number of halogen substituents is any integer between 1 and the upper limit, and when the number of halogen substituents is greater than 1, the group to be substituted can be substituted with the same or different halogen.
The term "deuterated" or "deuterated product" refers to the case where a hydrogen atom on alkyl, cycloalkyl, alkylene, aryl, heteroaryl, mercapto, heterocycloalkyl, alkenyl, alkynyl and other groups is replaced with at least one isotope deuterium, wherein the upper limit of the number of deuterium substituents is equal to the sum of the number of hydrogens that can be replaced in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, and further preferably 1-3 deuterium atoms.
The term "alkyl" refers to a monovalent linear or branched saturated aliphatic hydrocarbon group. Unless otherwise specially specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.
The term "alkylene" refers to a divalent linear or branched chain divalent saturated alkyl, and examples of alkylene include, but are not limited to, methylene, ethylene, propylene, butylene, etc.
The term "cycloalkyl" refers to a monovalent non-aromatic, partially unsaturated or fully saturated, substituted or unsubstituted carbocyclic hydrocarbon group, which generally has 3 to 12 carbon atoms, preferably 3-10 carbon atoms, more preferably 3-6 carbon atoms, and further preferably 3-4 carbon atoms, unless otherwise specially specified. Non-limiting examples include cyclopropyl, cyclobutyl, cyclopentyl, cyclohexyl,

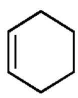
,

cycloheptyl, etc.
The term "cycloalkylene" refers to a divalent group of "cycloalkyl", and non-limiting examples include cyclopropylene, cyclobutylene, etc.

The term "heterocycle" or "heterocyclyl" refers to a substituted or unsubstituted, saturated or unsaturated, aromatic or non-aromatic ring comprising 1 to 3 heteroatoms selected from N, O, or S unless otherwise specifically defined, including monocyclic heterocycles, bridged bicyclic heterocycles, fused bicyclic heterocycles, spiro bicyclic heterocycles, etc., which are 3- to 12-membered heterocycles, more preferably 4- to 12-membered heterocycles, more preferably 4-to 10-membered heterocycles, and further preferably 4- to 7-membered heterocycles unless otherwise specifically defined. The definition thereof comprises heterocycloalkyl and heteroaryl. The N and S in the heterocyclyl ring may be oxidized into various oxidation states. Heterocyclyl can be connected to a heteroatom or a carbon atom, and non-limiting examples of heterocyclyl include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidinyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

, 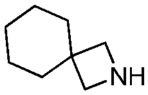, 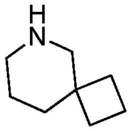, 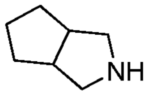, 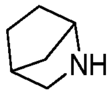, 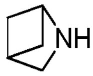, 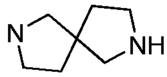,

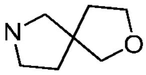, 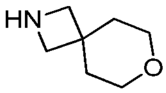, 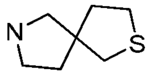, 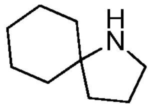, 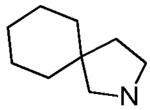,

etc.

The term "heterocyclylene" is a divalent group corresponding to "heterocyclyl", and non-limiting examples include imidazolylene, piperidinylene, aziridinylene, etc.

The term "carbocyclic" or "carbocyclyl" refers to a substituted or unsubstituted, saturated or unsaturated, aromatic or non-aromatic carbocyclic group, including monocyclic carbocyclic rings, bridged bicyclic rings, fused bicyclic rings, spiro bicyclic rings, etc., which have 3 to 12 carbon atoms, preferably 3-10 carbon atoms, and further preferably 3-6 carbon atoms unless otherwise specially specified. The definition thereof comprises cycloalkyl and aryl. In non-limiting embodiments, monocyclic carbocyclic rings include cyclopropyl, cyclobutyl, cyclopentyl, cyclohexyl, cycloheptyl or phenyl,

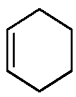,

etc., bridged bicyclic rings include

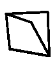, 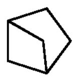, 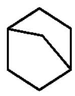,

etc., fused bicyclic rings include

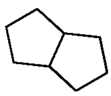, 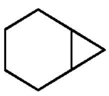, 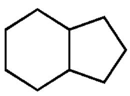, 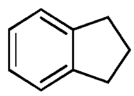, 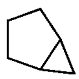,

etc., and spiro bicyclic rings include

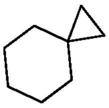, 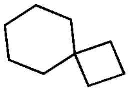, 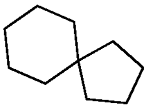, 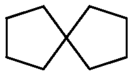, 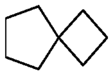, 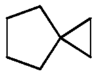,

etc.

The term "aryl" refers to an aromatic carbocyclic ring. Non-limiting examples include phenyl, naphthyl, etc.

The term "alkynyl" refers to a linear or branched monovalent unsaturated hydrocarbon group containing one or more carbon-carbon triple bonds. Unless otherwise specially specified, the alkynyl contains 2-6 carbon atoms, preferably 2-4 carbon atoms, and non-limiting examples include ethynyl, propynyl, propargyl, etc.

The term "alkenyl" refers to a linear or branched monovalent unsaturated hydrocarbon group containing one or more carbon-carbon double bonds. Unless otherwise specially specified, the alkynyl contains 2-6 carbon atoms, preferably contains 2-4 carbon atoms, and non-limiting examples are ethenyl, propenyl, allyl, 2-butenyl, 1-butenyl, etc.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, the haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl. Non-limiting examples of haloalkoxy include monofluoromethoxy, difluoromethoxy, trifluoromethoxy, difluoroethyloxy, *etc.*

The term "$C_{1-4}$ alkylacyl" refers to $C_{1-4}$ alkyl-C(O)-. Non-limiting examples include formyl, acetyl, and propionyl.

The term "$C_{1-4}$ alkylsulfonyl" refers to $C_{1-4}$ alkyl-$S(O)_2$-. Non-limiting examples include methylsulfonyl, ethylsulfonyl, and propylsulfonyl.

The term "heteroaromatic ring" or "heteroaryl" refers to an aromatic heterocycle. Non-limiting examples include pyrazolyl, pyrimidinyl, thiazolyl, pyridinyl, furyl, etc.

The term "heterocycloalkyl" refers to a non-aromatic, partially unsaturated or fully saturated heterocycle, which generally has 4 to 12 ring members, preferably 4 to 10 ring members, more preferably 4 to 7 ring members, and further preferably 5 or 6 ring members. In addition to carbon atoms, heterocycloalkyl further comprises 1-3 heteroatoms selected from N, S, O, Si, and P as ring members. Non-limiting examples include azetidinyl, morpholinyl, piperazinyl, piperidinyl, tetrahydropyranyl, oxetanyl, etc.

The term "alkylamino" or "alkamino" refers to amino substituted with one or two alkyl, and is also written as -N-$(alkyl)_2$ or -NH-alkyl, wherein the latter is also known as monoalkylamino. Non-limiting examples include dimethylamino, monomethylamino, diethylamino, monoethylamino, etc.

The term "optional" or "optionally" means that the events or circumstances described subsequently may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present disclosure, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, auxiliary material, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colorant, a flavoring agent, and a sweetening agent. Examples of pharmaceutically acceptable excipients include, but are not limited to: (1) sugars, such as lactose, glucose, and sucrose; (2) starch, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatin; (7) talc; (8)

excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffer solution; (21) polyester, polycarbonate and/or polyanhydride; and (22) other non-toxic compatible substances used in a pharmaceutical preparation.

The **term** "stereoisomer" refers to an isomer produced as a result of different spatial arrangement of atoms in molecules, including cis-trans isomers, enantiomers and conformational isomers.

The term "solvate" refers to a substance formed by the compound of the present disclosure or the salt thereof and a stoichiometric or non-stoichiometric solvent bound via an intermolecular non-covalent force. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multielement co-crystal formed between a neutral solid and a salt or solvate.

Brief Description of the Drawings

Figure 1 shows that compound 51 inhibits LPS-induced expression of TNF-α in the lung tissue of mice in a dose-dependent manner.

Figure 2 shows that compound 84 inhibits LPS-induced expression of TNF-α in the lung tissue of mice in a dose-dependent manner.

Detailed Description of Embodiments

The content of the present disclosure is described in detail by means of the following examples. In examples in which no specific conditions are indicated, experimental methods are carried out under conventional conditions. The listed examples are intended to better illustrate the content of the present disclosure but should not be construed as limiting the content of the present disclosure. Non-essential improvements and adjustments made to the embodiments by those skilled in the art according to the above Summary of the Invention still fall within the scope of protection of the present disclosure.

Dess-Martin reagent: 1,1,1-Triacetoxy-1,1-dihydro-1,2-benziodoxol-3-(1H)-one
DIPEA: N,N-diisopropylethylamine
NMP: N-methylpyrrolidone
TBDMSCI: Tert-butyl dimethylsilyl chloride
DMAP: 4-Dimethylaminopyridine
HATU: 2-(7-azabenzotriazole)-N,N,N',N'-tetramethyluronium hexafluorophosphate
$T_3P$: Propylphosphonic cyclic anhydride

Example 1

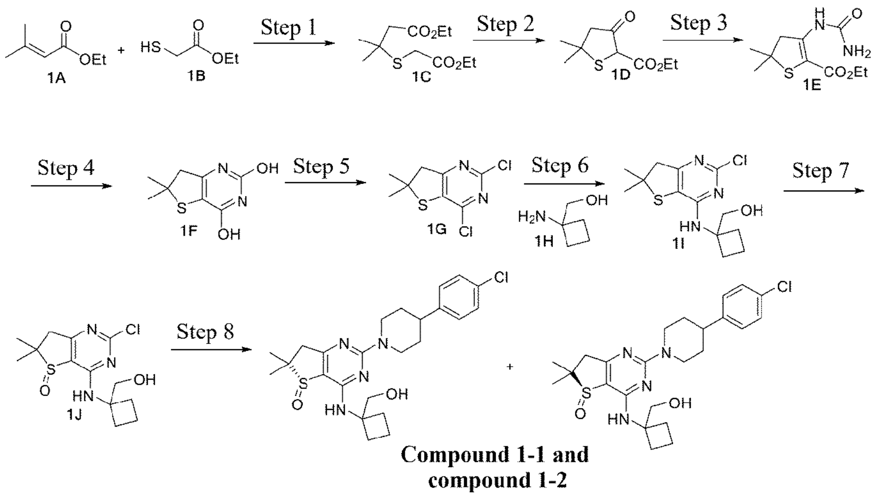


**Compound 1-1 and compound 1-2**

Step 1: In a nitrogen atmosphere, compound **1A** (21.6 mL, 155 mmol), compound **1B** (15.6 mL, 142 mmol), and 2 mL of piperidine were added to a three-mouth flask, heated to 45°C, and reacted for 48 h. The system was adjusted to acidity by adding 1N hydrochloric acid and extracted three times by adding 200 mL of ethyl acetate. The organic phases were dried over anhydrous sodium sulfate and then concentrated to obtain 36 g of crude compound 1C, which was directly used in the next reaction.

LCMS m/z = 249.1 $[M+H]^+$.

Step 2: In a nitrogen atmosphere, 80 mL of dichloromethane was added to a three-mouth flask, and the system was cooled to -10°C. Titanium tetrachloride (4.9 mL, 44.4 mmol) was added, followed by isopropanol (3.4 mL, 44.4 mmol) slowly, and the mixture was reacted for 30 min. Crude compound **1C** (10 g) was dissolved in 10 mL of DCM and then slowly added to the system, and triethylamine (17 mL, 121 mmol) was then added and reacted at -10°C for 2 h. After the raw material disappeared, the reaction was quenched by adding 42 mL of 3N hydrochloric acid, and the system was then heated to room temperature and extracted three times by adding 200 mL of ethyl acetate. The organic phase was dried over anhydrous sodium sulfate and then concentrated to obtain 8 g of crude compound 1D, which was directly used in the next reaction.

$^1H$ NMR (400 MHz, $CDCl_3$) δ 4.36 (s, 1H), 4.25 - 4.19 (m, 2H), 2.80 - 2.73 (m, 1H), 2.66 - 2.58 (m, 1H), 1.58 (s, 3H), 1.49 (s, 3H), 1.32 - 1.27 (m, 3H).

Step 3: 8 g of crude compound **1D** was dissolved in a mixed solvent of methanol:concentrated hydrochloric acid = 150 mL:15 mL, and urea (24 g, 400 mmol) was added. After the system was heated to 90°C and then reacted for 24 h, a solid generated in the system was collected, washed twice with water, and dried to obtain 6.4 g of crude compound **1E,** which was directly used in the next reaction.

LCMS m/z = 245.1 $[M+H]^+$.

Step 4: 6.4 g of crude compound **1E** was added to a round-bottom flask, 100 mL of water and sodium hydroxide (8 g, 200 mmol) were added, and the mixture was heated to 85°C and reacted for 24 h. The system was adjusted to acidity by adding hydrochloric acid, and a solid generated in the system was collected and dried to obtain 3 g of crude compound **1F.** The compound was directly used for the next reaction.

LCMS m/z = 196.9 $[M-H]^-$.

Step 5: In a nitrogen atmosphere, 3 g of crude compound **1F** was dissolved in 60 mL of acetonitrile, phosphorus oxychloride (5.7 mL, 61 mmol) was added, and the mixture was heated to 90°C and reacted for 48 h. After cooling, the system was poured into 300 mL of water, and the generated solid was collected and dried to obtain 1 g of crude compound **1G.**

LCMS m/z = 235.0 and 237.0 $[M+H]^+$.

Step 6: In a nitrogen atmosphere, 1 g of crude compound **1G** was dissolved in 30 mL of acetonitrile, 1.5 g of a crude salt of compound **1H** (see patent WO 2013/026797 A1 for the method) and triethylamine (5 mL, 36 mmol) were added, and the mixture was heated to 80°C and then reacted for 18 h. The reaction system was cooled to room temperature and concentrated. Then, the concentrated product was extracted three times by adding 50 mL of ethyl acetate. The organic phases were dried over anhydrous sodium sulfate and then concentrated to obtain 740 mg of crude compound **1I**, which was directly used in the next reaction.

LCMS m/z = 300.1 $[M+H]^+$.

Step 7: 740 mg of crude compound 1I was dissolved in 30 mL of acetic acid, 2 mL of 30% hydrogen peroxide was added, and the mixture was reacted at room temperature for 2 h. After the raw material disappeared, the system was extracted three times by adding 50 mL of ethyl acetate. The organic phases were dried over anhydrous sodium sulfate and

then concentrated to obtain 540 mg of crude compound **1J,** which was directly used in the next reaction.

LCMS m/z = 316.1 $[M+H]^+$.

Step 8: In a nitrogen atmosphere, 270 mg of crude compound **1J,** 4-(4-chlorophenyl)piperidine (220 mg, 1.12 mmol), and DIPEA (0.86 mL, 5.2 mmol) were dissolved in 30 mL of 1,4-dioxane, and the mixture was heated to 100°C and reacted for 18 h. The reaction system was concentrated and then separated by thin-layer chromatography to obtain 246 mg of crude racemic compound **1,** which was subjected to chiral resolution to obtain **compound 1-1** (77 mg, 19%) and **compound 1-2** (70 mg, 17%).

LCMS m/z = 475.2 $[M+H]^+$.

$^1H$ NMR (400 MHz, DMSO-d6) δ 7.35 - 7.25 (m, 5H), 4.85 - 4.75 (m, 3H), 3.75 - 3.70 (m, 2H), 3.14 - 3.07 (m, 1H), 2.96 - 2.78 (m, 3H), 2.72 - 2.65(m, 1H), 2.42 - 2.29 (m, 2H), 2.19 - 2.10 (m, 2H), 1.85 1.68 (m, 4H), 1.57 - 1.44 (m, 2H), 1.36 (s, 3H), 1.18 (s, 3H).

Chiral resolution method: instrument: Waters 150 SFC; column: Chiralpak Column (250 × 30 mm, I.D 30 mm, 10 um particle size); mobile phase: A for $CO_2$ and B for MeOH; gradient: 30% phase B isocratic elution; flow rate: 80 mL/min; back pressure: 100 bar; column temperature: 25°C; wavelength: 220 nm; retention time: **compound 1-1:** 1.160 min and **compound 1-1:** 1.701 min.

**Example** 2

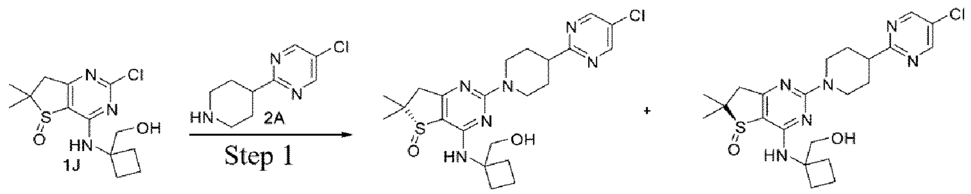


**Compound 2-1 and compound 2-2**

Step 1: In a nitrogen atmosphere, 270 mg of crude compound **1J,** crude compound **2A** (320 mg, see US 20140228286 A1), and DIPEA (0.86 mL, 5.2 mmol) were dissolved in 30 mL of 1,4-dioxane, and the mixture was heated to 100°C and reacted for 18 h. The reaction system was concentrated and then separated by thin-layer chromatography to obtain 226 mg of crude racemic compound **2,** which was subjected to chiral resolution to obtain **compound 2-1** (54 mg, 13%) and **compound 2-2** (40 mg, 10%).

LCMS m/z = 477.2 $[M+H]^+$.

$^1H$ NMR (400 MHz, DMSO-d6) δ 8.86 (s, 2H), 7.31 (s, 1H), 4.86 - 4.80 (m, 1H), 4.73 - 4.64 (m, 2H), 3.76 - 3.69 (m, 2H), 3.23 - 3.00 (m, 4H), 2.73 - 2.65(m, 1H), 2.41 - 2.28 (m, 2H), 2.19 - 2.11 (m, 2H), 2.02 - 1.92 (m, 2H), 1.84 - 1.58 (m, 4H), 1.35 (s, 3H), 1.18 (s, 3H).

Chiral resolution method: instrument: Waters 150 SFC; column: Chiralpak Column (250 × 30 mm, I.D 30 mm, 10 um particle size); mobile phase: A for $CO_2$ and B for MeOH; gradient: 30% phase B isocratic elution; flow rate: 70 mL/min; back pressure: 100 bar; column temperature: 25°C; wavelength: 220 nm; retention time: **compound 2-1:** 1.063 min and **compound 2-2:** 1.546 min.

**Example 3**

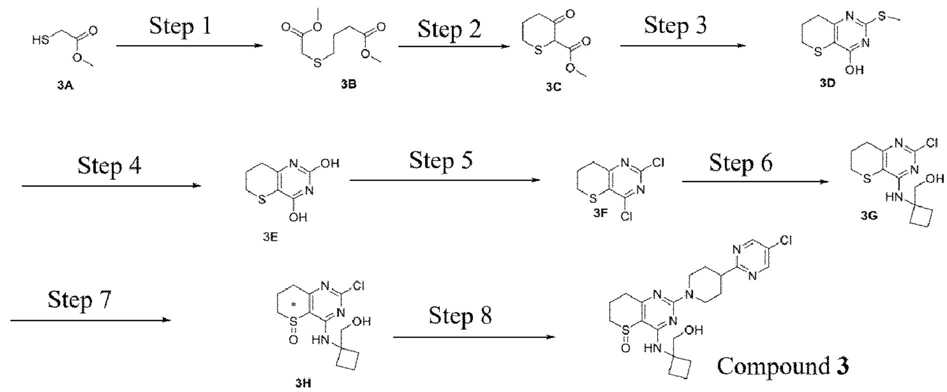

3A 3B 3C 3D 3E 3F 3G 3H Compound **3**

Using compound 3A and methyl 4-chlorobutyrate as raw materials, compound 3 (35 mg, 22%) was obtained according to the operation of Example 1.

$^1H$ NMR (400 MHZ, DMSO-d6) δ 8.86 (s, 2H), 6.48 (s, 1H), 4.88 (t, 1H), 4.67 (d, 2H), 3.70 (m, 2H), 3.17 (s, 1H), 3.00-3.06 (m, 3H), 2.92 (m, 1H), 2.38 (d, 4H), 2.13 (s, 2H), 1.96 (d, 3H), 1.87 - 1.71 (m, 2H), 1.58-1.67 (m, 2H).

LCMS (ESI): = 463.2 $[M+H]^+$.

**Example 4**

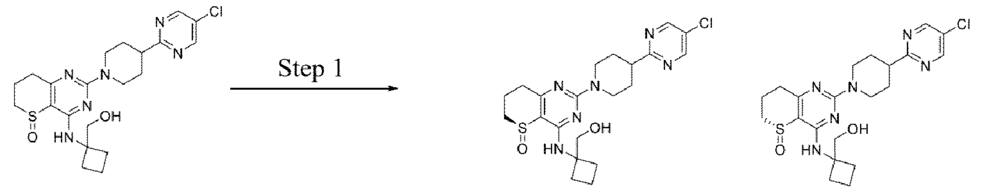

Compound **3**

Compounds **3-1 and 3-2**

Step 1: Compound **3** was subjected to chiral resolution to prepare **compounds 3-1** (26.1 mg) **and 3-2** (21.1 mg, 22%).

Chiral resolution method: instrument: Waters 150 SFC; column: Chiralpak Column (250 × 30 mm, I.D 30 mm, 10 um particle size); mobile phase: A for $CO_2$ and B for IPA + CAN (0.1% $NH_3$•$H_2O$); gradient: 70% phase B isocratic elution; flow rate: 100 mL/min; back pressure: 100 bar; column temperature: 25°C; wavelength: 220 nm.

**Compound** 3-1, retention time: 1.328 min. $^1H$ NMR (400 MHZ, DMSO-d6) δ 8.86 (s, 2H), 6.48 (s, 1H), 4.88 (t, 1H), 4.67 (d, 2H), 3.70 (m, 2H), 3.17 (s, 1H), 3.00-3.06 (m, 3H), 2.92 (m, 1H), 2.38 (d, 4H), 2.13 (s, 2H), 1.96 (d, 3H), 1.87 - 1.71 (m, 2H), 1.58-1.67 (m, 2H).

LCMS (ESI): = 463.2 $[M+H]^+$.

**Compound** 3-2, retention time: 2.125 min. $^1H$ NMR (400 MHZ, DMSO-d6) δ 8.86 (s, 2H), 6.48 (s, 1H), 4.88 (t, 1H), 4.67 (d, 2H), 3.70 (m, 2H), 3.17 (s, 1H), 3.00-3.06 (m, 3H), 2.92 (m, 1H), 2.38 (d, 4H), 2.13 (s, 2H), 1.96 (d, 3H), 1.87 - 1.71 (m, 2H), 1.58-1.67 (m, 2H).

LCMS (ESI): = 463.2 $[M+H]^+$.

**Example** 5

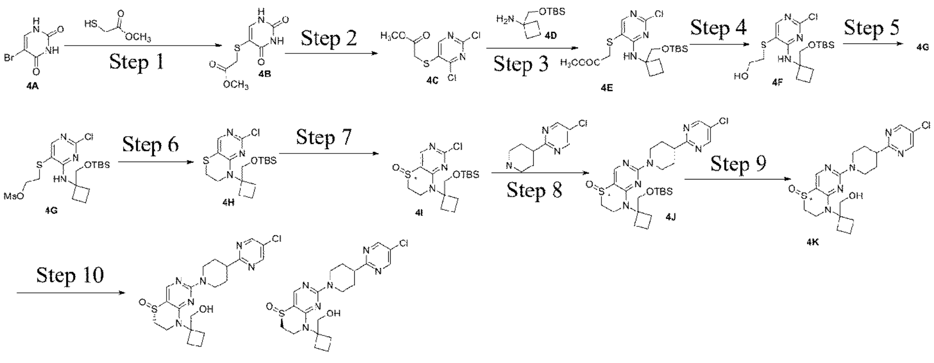


**Compounds 4-1 and 4-2**

Step 1: The raw material 4A (15 g, 78.50 mmol), methyl thioacetate (8.33 g, 78.50 mmol), potassium carbonate (27.12 g, 196.25 mmol), and tetrabutylammonium sulfate (8 g, 23.55 mmol) were separately added to 400 mL of an N,N-dimethylformamide solvent, stirred at room temperature overnight, and then filtered. The filtrate was concentrated under reduced pressure to obtain the target compound, and the crude product was directly used in the next reaction.

LCMS m/z = 217.1 $[M+1]^+$

Step 2: The raw material 4B (2.9 g, 13.41 mmol) was added to thionyl chloride (30 mL). The mixture was heated to 90°C, stirred for 4 hours, then concentrated to remove excess thionyl chloride, dissolved with ethyl acetate, and washed with a saturated sodium bicarbonate solution. The filtrate was dried, and after filtration and concentration, the target compound **4C** (3.1 g, yield 91%) was separated by silica gel column chromatography (PE:EA (v/v) = 1:0-6:1).

LCMS m/z = 253.0 $[M +1]^+$

$^1H$ NMR (400 MHz, $CDCl_3$) δ8.58(s, 1H), 3.72 (s, 3H), 3.59 (s, 2H).

Step 3: The raw material 4C (3.9 g, 15.41 mmol), the raw material 4D (3.32 g, 15.41 mmol), and triethylamine (3.12 g, 30.82 mmol) were added to the solvent acetonitrile (70 mL), then heated to 50°C, stirred for 16 hours, cooled, and concentrated, and the concentrated product was then diluted by adding water, extracted with ethyl acetate, and dried over anhydrous sodium sulfate. After filtration, the filtrate was concentrated and then separated by silica gel column chromatography (PE:EA (v/v) = 1:0-4:1) to obtain the target compound **4E** (2.6 g, yield 39%).

LCMS m/z = 432.1 $[M +1]^+$

Step 4: **4E** (1.3 g, 3.01 mmol) was added to dry tetrahydrofuran (20 mL), and the reagent sodium borohydride (0.68 g, 18.06 mmol) was added in an ice bath. The mixture was then slowly heated to room temperature and stirred for 16 hours. The reaction was quenched by adding water, and the system was extracted with ethyl acetate, and dried over anhydrous sodium sulfate. After filtration, the filtrate was concentrated and then separated by silica gel column chromatography (PE:EA (v/v) = 1:0-2:3) to obtain the target compound **4F** (0.7 g, yield 57%).

LCMS m/z = 404.2 $[M +1]^+$

Step 5: The raw material **4F** (0.6 g, 1.49 mmol) was added to dry dichloromethane (10 mL), and the reagents triethylamine (0.45 g, 4.44 mmol) and methanesulfonyl chloride (0.34 g, 2.97 mmol) were added in an ice bath. The mixture was then slowly heated to room temperature and stirred for 1 hour, and the reaction was quenched by adding water. The system was extracted with dichloromethane, washed with a saturated sodium bicarbonate solution and a sodium chloride aqueous solution, respectively, and dried over and anhydrous sodium sulfate. After filtration, the filtrate was concentrated to obtain the target compound **4F** (0.85 g, yield 100%).

LCMS m/z = 482.1 $[M +1]^+$

Step 6: The raw material **4G** (0.85 g, 1.76 mmol) was added to the solvent N,N-dimethylformamide (10 mL), and the reagent sodium hydride (0.28 g, 7.04 mmol, 60% in oil) was added in an ice bath. The mixture was then slowly heated to room temperature and stirred for 1 hour. The reaction was quenched by adding water, and the system was extracted with ethyl acetate, and dried over anhydrous sodium sulfate. After filtration, the filtrate was concentrated and then separated by silica gel column chromatography (PE:EA (v/v) = 1:0-8:1) to obtain the target compound **4H** (0.45 g, yield 66%).

LCMS m/z = 386.1 $[M +1]^+$

$^1H$ NMR (400 MHz, $CDCl_3$) δ7.77(s, 1H), 3.99 (s, 2H), 3.77-3.80 (m, 2H), 2.83-2.85 (m, 2H), 2.23-2.28 (m, 4H), 1.67-1.74 (m, 2H), 0.85 (s, 9H), 0.0 (s, 6H).

Step 7: The raw material **4H** (400 mg, 1.04 mmol), 1,1'-binaphthyl-2,2'-bisphenylphosphine (29 mg, 0.1 mmol), water (19 mg, 1.04 mmol), and tetraisopropyl titanate (14 mg, 0.051 mmol) were successively added to the solvent dichloromethane (10 mL) and stirred at room temperature for half an hour. Tert-butyl hydroperoxide (120 mg, 1.35 mmol) was then added, and the mixture was further stirred for 3 hours, then diluted by adding water, and then extracted with dichloromethane. After washing with saturated sodium bicarbonate followed by drying and filtration, the filtrate was concentrated and then purified and separated by silica gel column chromatography (PE:EA (v/v) = 1:0-2:3) to obtain the

title compound **4I** (0.40 g, yield 96%).

LCMS m/z = 402.1 [M +1]+

Step 8: The raw material **4I** (0.4 g, 0.99 mmol), the raw material **2A** (0.29 g, 1.48 mmol), and DIPEA (0.38 g, 2.94 mmol) were added to the solvent 1,4-dioxane (20 mL), then heated to 95°C, stirred for 16 hours, cooled, and concentrated, and the concentrated product was then diluted by adding water, extracted with ethyl acetate, and dried over anhydrous sodium sulfate. After filtration, the filtrate was concentrated and then separated by silica gel column chromatography (PE:EA (v/v) = 1:0-4:1) to obtain the target compound **4J** (0.5 g, yield 90%).

LCMS m/z = 563.2 [M +1]+

Step 9: The raw material **4J** (0.6 g, 1.07 mmol) was dissolved in the solvent tetrahydrofuran (20 mL), and tetrabutylammonium fluoride solution (1.6 mL, 1.60 mmol, 1 M) was then added. The mixture was stirred at room temperature for 2 hours, diluted by adding water, then extracted with ethyl acetate, and dried over anhydrous sodium sulfate. After filtration, the filtrate was concentrated and then purified and separated by silica gel column chromatography (DCM:dihydrous methanol (v/v) = 1:0-10:1) to obtain the title compound **4K** (0.3 g, yield 62%).

LCMS m/z = 449.2 [M +1]+

$^1$H NMR (400 MHz, DMSO-$d_6$) δ8.85(s, 1H), 8.16 (s, 1H), 4.93(t, 1H), 4.62-4.65 (m, 2H), 3.84-3.92 (m, 2H), 3.74-3.79 (m, 1H), 3.56-3.62 (m, 1H), 3.16-3.22 (m, 1H), 2.98-3.08 (m, 3H), 2.62-2.68 (m, 1H), 2.38-2.41 (m, 1H), 2.24-2.30 (m, 3H), 1.95 (d, 2H), 1.59-1.73 (m, 4H).

Step 10: The raw material **4K** (300 mg, 0.67 mmol) was purified by chiral HPLC resolution to obtain the target compound **4-1** (100 mg) and the target compound **4-2** (100 mg).

Instrument name: Waters 150 SFC; chromatographic column: Chiralcel OD Column (250 × 30 mm, I.D 30 mm, 10 um particle size); mobile phase: A for $CO_2$ and B for EtOH + ACN (0.1% $NH_3$•$H_2O$); gradient: 40% B phase isocratic elution; flow rate: 100 mL/min; column pressure: 100 bar; column temperature: 25°C; absorption wavelength: 220 nm; cycle time: about 2.9 min; sample preparation: dissolution with acetonitrile.

Peaking time of compound **4-1:** 0.578 min

LCMS m/z = 449.2 [M +1]+

$^1$H NMR (400 MHz, DMSO-$d_6$) δ8.85(s, 1H), 8.16 (s, 1H), 4.93(t, 1H), 4.62-4.65 (m, 2H), 3.84-3.92 (m, 2H), 3.74-3.79 (m, 1H), 3.56-3.62 (m, 1H), 3.16-3.22 (m, 1H), 2.98-3.08 (m, 3H), 2.62-2.68 (m, 1H), 2.38-2.41 (m, 1H), 2.24-2.30 (m, 3H), 1.95 (d, 2H), 1.59-1.73 (m, 4H).

Peaking time of compound **4-2:** 0.812 min

LCMS m/z = 449.2 [M +1]+

$^1$H NMR (400 MHz, DMSO-$d_6$) δ8.85(s, 1H), 8.16 (s, 1H), 4.93(t, 1H), 4.62-4.65 (m, 2H), 3.84-3.92 (m, 2H), 3.74-3.79 (m, 1H), 3.56-3.62 (m, 1H), 3.16-3.22 (m, 1H), 2.98-3.08 (m, 3H), 2.62-2.68 (m, 1H), 2.38-2.41 (m, 1H), 2.24-2.30 (m, 3H), 1.95 (d, 2H), 1.59-1.73 (m, 4H).

**Example 6**

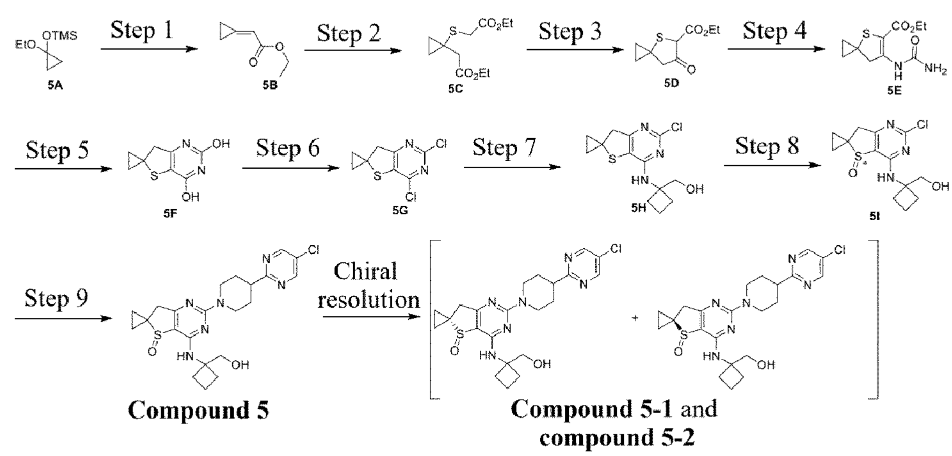

5A 5B 5C 5D 5E
5F 5G 5H 5I
**Compound 5**
**Compound 5-1** and **compound 5-2**

Step 1: Ethoxyformylmethylene triphenylphosphine (14 g, 40.23 mol) dissolved in dichloromethane (6 ml) was added to (1-ethoxycyclopropyloxy)trimethylsilane (7 g, 40.23 mmol) and acetic acid (1.21 g, 20.12 mol) dissolved in tetraethylene glycol dimethyl ether (30 ml) in a 150 ml sealed tube and reacted for 3 h in an oil bath at 100°C. After the reaction was complete, the system was cooled to room temperature. After the reaction was complete, dichloromethane was removed by direct concentration, and the residue was quickly separated and purified by column chromatography

(eluent ratio: EA/PE = 0-10%) to obtain the target compound **5B** (4 g, 79%).

Using compound **5B** and ethyl thioacetate as raw materials, **compound 5** (50 mg, 33%) was obtained according to the operation of Example 1.

Chiral resolution of compound 5:

**Compound 5** (50 mg) was taken for resolution, and after separation, **compound 5-1** (retention time: 1.257 s, 15 mg, ee% = 100%) and **compound 5-2** (retention time: 1.553 s, 17 mg, ee% = 100%) were obtained.

Resolution conditions:

instrument: Waters 150 MGM; column: DAICEL CHIRALPAK AD;
mobile phases: A for CO2 and B for EtOH (0.1% NH3$_8$•$H_2O$)); gradient: B 40%; flow: 120 mL/min;
back pressure: 100 bar;
column temperature: 35°C; wavelength: 220 nm; period: 13 min;

**Compound 5-1:**

$^1H$ NMR (400 MHz, $CDCl_3$) δ = 8.62 (s, 2H), 5.85 (s, 1H), 4.79 (d, 2H), 3.87 (s, 2H), 3.67 (d, 1H), 3.17 (tt, 1H), 3.07 (t, 2H), 2.73 (d, 1H), 2.29 (t, 4H), 2.06 (d, 2H), 1.92 - 1.80 (m, 3H), 1.61 - 1.57 (m, 1H), 1.39 - 1.30 (m, 2H), 1.11 (t, 1H), 1.08 - 1.04 (m, 1H), 0.84 (d, 1H).

**Compound 5-2:**

$^1H$ NMR (400 MHz, $CDCl_3$) δ = 8.62 (s, 2H), 6.00 (s, 1H), 4.80 (d, 2H), 3.86 (d, 2H), 3.68 (d, 1H), 3.17 (tt, 3.8, 1H), 3.07 (t, 2H), 2.72 (d, 1H), 2.35 - 2.24 (m, 4H), 2.06 (d, 2H), 1.88 - 1.80 (m, 3H), 1.62 - 1.56 (m, 1H), 1.39 - 1.30 (m, 1H), 1.17 - 1.09 (m, 1H), 1.07 - 1.00 (m, 1H), 0.92 - 0.79 (m, 1H).

**Example 7**

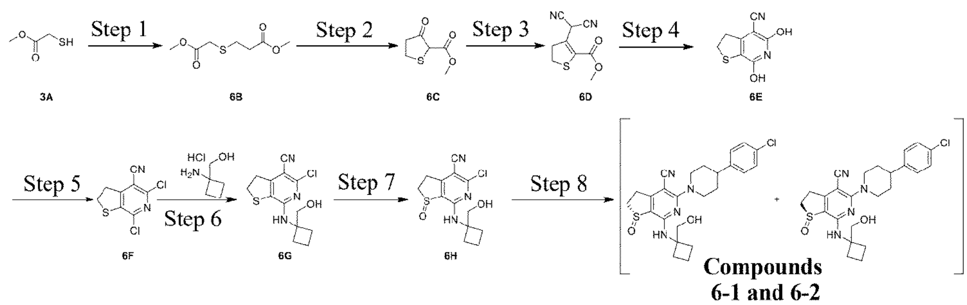


Using compound **3A** and methyl acrylate as raw materials, **compound 6C** (35 g, 97%) was obtained according to the operation of Example 1 (the reactions of the first and second steps).

LC-MS (ESI): m/z = 161.1 [M+H]$^+$

Step 3: At room temperature, compound **6C** (30 g, 187.5 mmol) was dissolved in methanol (300 mL), and malononitrile (18.56 g, 281.2 mmol) and imidazole (38.25 g, 562.5 mmol) were added at room temperature and stirred for 3 h. After the reaction was complete, most of the methanol was spun off, and water (50 mL) was added. After extraction twice with dichloromethane (100 mL × 2), the organic phases were combined, washed with a saturated sodium chloride aqueous solution, and dried over anhydrous sodium sulfate. After filtration, the filtrate was concentrated, and the residue was then separated and purified by silica gel column chromatography (eluent ratio: EA:PE = 0-50%) to obtain the title compound **6D** (26.7 g, 69%).

LC-MS (ESI): m/z =209.1 [M+H]$^+$

Step 4: In a nitrogen atmosphere, compound **6D** (12 g, 61.86 mmol) was dissolved in a hydrogen chloride-dioxane solution (4M, 100 mL) at room temperature, the mixture was then heated to 100°C and reacted overnight,

whereupon a large amount of a solid precipitated. After the reaction was complete, filtration was carried out, and the obtained solid was spin-dried to obtain crude compound **6E** (9 g, 80%). The obtained compound was directly used in the next reaction without further purification.

LC-MS (ESI): m/z =195.1 $[M+H]^+$

Step 5: At room temperature, compound **6E** (2 g, 10.31 mmol) was added to a 120 mL sealed tube charged with phosphorus oxychloride (20 mL), benzyltrimethylammonium chloride (3.83 g, 20.62 mmol) was added at room temperature, and the mixture was then heated to 150°C and reacted for 7 h. After the reaction was complete, the reaction liquid was slowly added to a saturated sodium bicarbonate solution (100 mL) in an ice bath and extracted twice with ethyl acetate (150 mL × 2). The organic phases were combined, washed with a saturated sodium chloride aqueous solution, and dried over anhydrous sodium sulfate. After filtration, the filtrate was concentrated, and the residue was then separated and purified by silica gel column chromatography (eluent ratio: EA:PE = 0-50%) to obtain the title compound **6F** (1.1 g, 46%).

Step 6: At room temperature, compound **6F** (1.1 g, 4.78 mmol) was dissolved in a 1,4-dioxane solution (12 mL), N,N-diisopropylethylamine (1.23 g, 9.56 mmol) and (1-aminocyclobutyl)methanol hydrochloride (0.65 g, 4.78 mmol) were added at room temperature, and the mixture was then heated to 100°C and reacted overnight. After the reaction was complete, the reaction liquid was concentrated, and the residue was then separated and purified by silica gel column chromatography (eluent ratio: MeOH:DCM = 0-10%) to obtain the title compound **6G** (600 mg, 43%).

LC-MS (ESI): m/z = 296.3 $[M+H]^+$

Step 7: At room temperature, compound **6G** (600 mg, 2.03 mmol) was dissolved in acetic acid (30 mL), and 30% hydrogen peroxide (1.0 mL) was added room temperature and reacted for 2 h. After the reaction was complete, a saturated sodium bicarbonate solution (30 mL) was added, and the system was extracted twice with dichloromethane (50 ml × 2). The organic phases were combined, washed with a saturated sodium chloride aqueous solution, and dried over anhydrous sodium sulfate. After filtration, the filtrate was concentrated, and the residue was then separated and purified by silica gel column chromatography (eluent ratio: MeOH:DCM = 0-10%) to obtain the title compound **6H** (300 mg, 47%).

LC-MS (ESI): m/z = 312.3 $[M+H]^+$

Step 8: At room temperature, compound **6H** (150 mg, 0.48 mmol) was dissolved in a 1,4-dioxane solution (2.0 mL), N,N-diisopropylethylamine (124 mg, 0.96 mmol) and 4-(4-chlorophenyl)piperidine (94 mg, 0.48 mmol) were added at room temperature, and the mixture was then heated to 100°C and reacted overnight. After the reaction was complete, the crude product resulting from concentration under reduced pressure was separated by preparative HPLC to obtain racemate 6 (70 mg, 31%). Separation method: 1. instrument: waters 2767 preparative liquid chromatographic instrument; chromatographic column: SunFire@ Prep C18 (19 mm × 250 mm); 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: with a content of 10-55%; c. flow: 12 mL/min.

Chiral preparative resolution of racemate, preparative chromatographic conditions: 1. instrument: Waters 150 SFC; 2. chromatographic column: Chiralpak IC-Column (250 × 30 mm, I.D 30 mm, 10 um particle size); 3. mobile phase system: A for $CO_2$ and B for MeOH (0.1% $NH_3•H_2O$); 4. gradient: B 35%; and 5. flow rate: 100 mL/min. After preparation, the title compound **6-1** (30 mg, 13.3%, retention time: 1.092 min, absolute configuration undetermined) and the title compound **6-2** (31 mg, 13.7%, retention time: 1.761 min, absolute configuration undetermined) were obtained.

**Compound 6-1** $^1H$ NMR (400 MHz, Methanol-$d_4$) δ 7.22 - 7.16 (m, 2H), 7.16 - 7.10 (m, 2H), 4.66 - 4.57 (m, 2H), 3.82 (q, 2H), 3.71 - 3.61 (m, 1H), 3.40 - 3.26 (m, 2H), 3.17 - 3.02 (m, 3H), 2.79 (tt, 1H), 2.35 - 2.08 (m, 4H), 1.90 - 1.70 (m, 4H), 1.70 - 1.57 (m, 2H).

LC-MS (ESI): m/z = 471.1 $[M+H]^+$

**Compound 6-2** $^1H$ NMR (400 MHz, Methanol-$d_4$) δ 7.33 - 7.27 (m, 2H), 7.26 - 7.21 (m, 2H), 4.77 - 4.67 (m, 2H), 3.92 (q, 2H), 3.81 - 3.70 (m, 1H), 3.49 - 3.36 (m, 2H), 3.27 - 3.12 (m, 3H), 2.89 (tt, 1H), 2.43 - 2.24 (m, 4H), 1.98 1.82 (m, 4H), 1.80 - 1.68 (m, 2H).

LC-MS (ESI): m/z = 471.1 $[M+H]^+$

**Example 8**

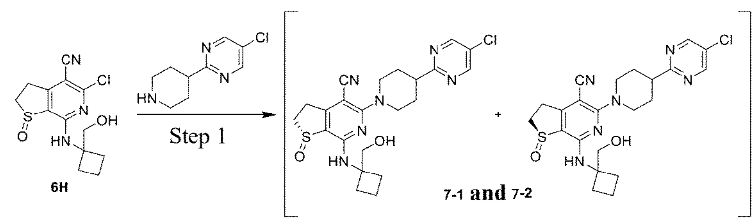


6H

7-1 and 7-2

Using compounds **6H** and **2A** as raw materials, racemate 7 (80 mg, 35%) was obtained according to the operation of Example 2. Separation method: 1. instrument: waters 2767 preparative liquid chromatographic instrument; chromatographic column: SunFire@ Prep C18 (19 mm × 250 mm); 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: with a content of 10-55%; c. flow: 12 mL/min.

Chiral preparative resolution of racemate, preparative chromatographic conditions: 1. instrument: Waters 150 SFC; 2. chromatographic column: Chiralpak IC-Column (250 × 30 mm, I.D 30 mm, 10 um particle size); 3. mobile phase system: A for $CO_2$ and B for MeOH (0.1% $NH_3$•$H_2O$); 4. gradient: B 35%; and 5. flow rate: 100 mL/min. After preparation, the title compound **7-1** (31 mg, 13.6%, retention time: 1.400 min, absolute configuration undetermined) and the title compound **7-2** (33 mg, 14.5%, retention time: 1.681 min, absolute configuration undetermined) were obtained.

Compound **7-1** $^1$H NMR (400 MHz, Methanol-*d*$_4$) δ 8.75 (s, 2H), 4.66 (dt, 2H), 3.92 (q, 2H), 3.81 - 3.71 (m, 1H), 3.50 - 3.36 (m, 2H), 3.30 - 3.19 (m, 4H), 2.42 - 2.24 (m, 4H), 2.10 (dd, 2H), 1.99 - 1.83 (m, 4H).

LC-MS (ESI): m/z = 473.2 $[M+H]^+$

Compound **7-2** $^1$H NMR (400 MHz, Methanol-*d*$_4$) δ 8.75 (s, 2H), 4.67 (dd, 2H), 4.55 (s, 1H), 3.92 (q, 2H), 3.82 - 3.70 (m, 1H), 3.50 - 3.36 (m, 2H), 3.27 - 3.21 (m, 3H), 2.42 - 2.24 (m, 4H), 2.10 (dd, 2H), 2.01 - 1.79 (m, 4H).

LC-MS (ESI): m/z = 473.2 $[M+H]^+$

**Example 9**

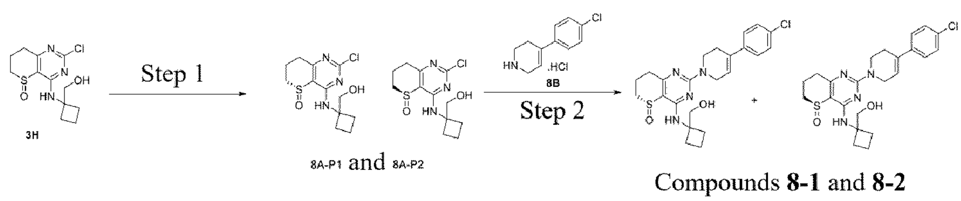


3H

8A-P1 and 8A-P2

Compounds **8-1** and **8-2**

Step 1: Compound **3H** (3.7 g, 12.3 mmol) was separated by a chiral column to obtain compounds **8A-P1** (1.1 g, 30%) and **8A-P2** (0.9 g, 24%). Chiral resolution method: instrument: Waters 150 SFC; column: Chiralpak IC Column (250 × 30 mm, I.D 30 mm, 10 um particle size); mobile phase: A for CO2 and B for IPA + ACN (0.1% $NH_3$•$H_2O$); gradient: 45% phase B isocratic elution; flow rate: 100 mL/min; back pressure: 100 bar; column temperature: 25°C; wavelength: 220 nm.

**Compound 8A-P1,** retention time: 0.614 min. $^1$H NMR (400 MHZ, DMSO-d6) δ 7.58 (s, 1H), 4.91 (t, 1H), 3.68 (d, 2H), 3.25-3.18 (m, 1H), 3.11-3.03(m, 1H), 2.86 - 2.76 (m, 1H), 2.73 - 2.61 (m, 1H), 2.37 - 2.23 (m, 3H), 2.17 (m, 2H), 2.09 - 1.93 (m, 1H), 1.87 - 1.68 (m, 2H).

LCMS (ESI): = 302.1 $[M+H]^+$.

**Compound 8A-P2,** retention time: 1.148 min. $^1$H NMR (400 MHZ, DMSO-d6) δ 7.58 (s, 1H), 4.91 (t, 1H), 3.68 (d, 2H), 3.25-3.18 (m, 1H), 3.11-3.03(m, 1H), 2.86 - 2.76 (m, 1H), 2.73 - 2.61 (m, 1H), 2.37 - 2.23 (m, 3H), 2.17 (m, 2H), 2.09 - 1.93 (m, 1H), 1.87 - 1.68 (m, 2H).

LCMS (ESI): = 302.1 $[M+H]^+$.

Step 2: Compound **8A-P1** (0.10 g, 0.33 mmol), **8B** (77 mg, 0.40 mmol, see CN 103145607 for the synthesis method), and DIPEA (0.13 mg, 0.99 mmol) were dissolved in dioxane (10 mL) and stirred at 85°C for 16 h. After the reaction was complete, the system was concentrated under reduced pressure and subjected to HPLC preparation to obtain **compound 8-1** (60 mg, 40%).

Preparation method: instrument: Abilene 1290 infinity II preparative liquid chromatogram; chromatographic column: XSelect@ Prep C18 (19 mm × 250 mm); the sample was dissolved in DMF and filtered with a 0.45 μm filter to prepare a sample solution. Preparative chromatography conditions: composition of mobile phases A and B: mobile phase A: 5 mmol ammonium acetate, mobile phase B: methanol; gradient elution, mobile phase B with content from 50% to 85%; flow: 15 ml/min; elution time: 18 min; retention time: 16.8.

**Compound 8-1,** $^1$H NMR (400 MHZ, DMSO-d6) δ 7.48 (d, 2H), 7.40 (d, 2H), 6.53 (s, 1H), 6.31 (s, 1H), 4.91 (t, 1H), 4.33 (s, 2H), 3.94 (t, 2H), 3.73 (d, 2H), 3.10-3.05 (m, 1H), 2.95-2.88 (m, 1H), 2.74 - 2.51 (m, 4H), 2.46-2.33 (m, 3H), 2.17 (brs, 2H), 2.02-1.91 (m, 1H), 1.87-1.77 (m, 2H).

The synthesis, preparation, and separation of compound **8-2** (45 mg, 36%) were the same as those of compound **8-1.**

**Compound 8-2,** $^1$H NMR (400 MHZ, DMSO-d6) δ 7.48 (d, 2H), 7.40 (d, 2H), 6.53 (s, 1H), 6.31 (s, 1H), 4.91 (t, 1H), 4.33 (s, 2H), 3.94 (t, 2H), 3.73 (d, 2H), 3.10-3.05 (m, 1H), 2.95-2.88 (m, 1H), 2.74 - 2.51 (m, 4H), 2.46-2.33 (m, 3H), 2.17 (brs, 2H), 2.02-1.91 (m, 1H), 1.87-1.77 (m, 2H).

LCMS (ESI): = 459.50 [M+H] +.

**Example 10**

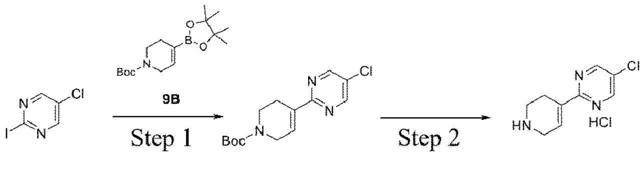


9A 9C 9D

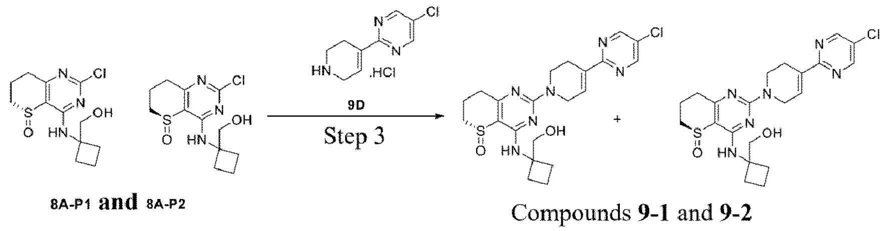


8A-P1 and 8A-P2

Compounds **9-1** and **9-2**

Step 1: Substrate **9A** (5.0 g, 20.77 mmol), dioxane (100 mL), and water (20 mL) were added to a 250 mL single-mouth flask and dissolved, sodium carbonate (6.6 g, 62.31 mmol), **9B** (7.1 g, 22.85 mmol), and [1,1'-bis(diphenylphosphine)ferrocene]palladium dichloride (1.5 g, 2.08 mmol) were added, and the mixture was stirred at 100 degrees Celsius overnight under nitrogen protection. The reaction liquid was diluted by adding water (50 mL) and suction-filtered with diatomite. The filtrate was extracted with ethyl acetate (2 × 100 mL), and the organic phases were combined, dried over anhydrous sodium sulfate, concentrated, and subjected to column chromatography (petroleum ether:ethyl acetate (v/v) = 10:1) to obtain the title compound **9C** (5.0 g, 81%).

LC-MS (ESI): m/z = 240.1 [M-56+H]+.

Step 2: Substrate **9C** (1.0 g, 3.38 mmol), methanol (5 mL), and hydrogen chloride dioxane (4 M, 5 mL) were added to a 50 mL single-mouth flask, dissolved, and stirred at room temperature for 3 h, and the reaction liquid was concentrated to obtain the title compound **9D** (0.79 g, 100%).

LC-MS (ESI): m/z = 196.2 $[M+H]^+$.

Step 3: Compound **8A-P1** (0.10 g, 0.33 mmol), **9D** (77 mg, 0.40 mmol), and DIPEA (0.13 mg, 0.99 mmol) were dissolved in dioxane (10 mL) and stirred at 85°C for 16 h. After the reaction was complete, the solvent was spun off, and HPLC preparation was carried out obtain **compound 9-1** (70 mg, 46%).

Preparation method: instrument: Abilene 1290 infinity II preparative liquid chromatogram; chromatographic column: XSelect@ Prep C18 (19 mm × 250 mm); the sample was dissolved in DMF and filtered with a 0.45 μm filter to prepare a sample solution. Preparative chromatography conditions: composition of mobile phases A and B: mobile phase A: 5 mmol ammonium acetate, mobile phase B: methanol; gradient elution, mobile phase B with content from 50% to 85%; flow: 15 ml/min; elution time: 18 min; retention time: 16.5 min.

The synthesis, preparation, and separation conditions of compound **9-2** (60 mg, 48%) were the same as those of compound **9-1.**

**Compound 9-1,** $^1$H NMR (400 MHZ, DMSO-d6) δ 8.89 (s, 2H), 7.32 - 7.24 (m, 1H), 6.54 (s, 1H), 4.90 (t, 1H), 4.43 (s, 2H), 3.94 (t, 2H), 3.73 (d, 2H), 3.10-3.04 (m, 1H), 2.95-2.88 (m, 1H), 2.71 - 2.53 (m, 4H), 2.45 - 2.27 (m, 3H), 2.22 - 2.09 (m, 2H), 2.02 - 1.91 (m, 1H), 1.88 - 1.69 (m, 2H).

**Compound 9-2,** $^1$H NMR (400 MHZ, DMSO-d6) δ 8.89 (s, 2H), 7.32 - 7.24 (m, 1H), 6.54 (s, 1H), 4.90 (t, 1H), 4.43 (s, 2H), 3.94 (t, 2H), 3.73 (d, 2H), 3.10-3.04 (m, 1H), 2.95-2.88 (m, 1H), 2.71 - 2.53 (m, 4H), 2.45 - 2.27 (m, 3H), 2.22 - 2.09 (m, 2H), 2.02 - 1.91 (m, 1H), 1.88 - 1.69 (m, 2H).

LCMS (ESI): = 461.50 [M+H] +.

**Example 11**

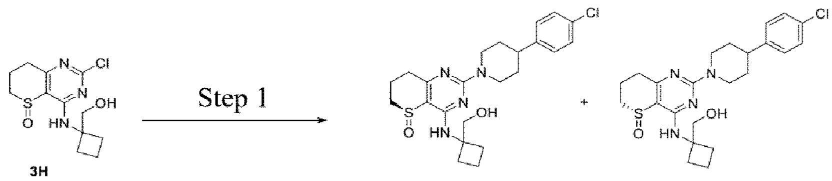

3H

Compounds **10-1** and **10-2**

Step 1: Compound **3H** (150 mg, 0.50 mmol), 4-(4-chlorophenyl)piperidine hydrochloride (131 mg, 0.55 mmol), and DIPEA (260 mg, 2.00 mmol) were dissolved in dioxane and reacted at 90°C for 16 h. After the reaction was complete, the solvent was spun off, and SFC preparation was carried out to obtain **compounds 10-1** (44.3 mg, 19%) **and 10-2** (26.5 mg, 11%).

Chiral resolution method: instrument: Waters 150 SFC; column: Chiralcel OX Column (250 × 30 mm, I.D 30 mm, 10 um particle size); mobile phase: A for $CO_2$ and B for EtOH + ACN; gradient: 45% phase B isocratic elution; flow rate: 120 mL/min; back pressure: 100 bar; column temperature: 25°C; wavelength: 220 nm.

**Compound 10-1,** retention time: 1.128 min. $^1$H NMR (400 MHZ, DMSO-d6) δ 7.38 - 7.29 (m, 2H), 7.30 - 7.20 (m, 2H), 6.47 (s, 1H), 4.90 (t, 1H), 4.77 (d, 2H), 3.70 (d, 2H), 3.10-3.03 (m, 1H), 2.54-2.51 (m, 1H), 2.98 - 2.79 (m, 4H), 2.72 - 2.61 (m, 1H), 2.45-2.30 (m, 3H), 2.18 - 2.08 (m, 2H), 2.03 - 1.89 (m, 1H), 1.83-1.71 (m, 4H), 1.55 - 1.41 (m, 2H).

LCMS (ESI): = 461.2 [M+H] $^+$.

**Compound 10-2,** retention time: 1.609 min. $^1$H NMR (400 MHZ, DMSO-d6) δ 7.38 - 7.29 (m, 2H), 7.30 - 7.20 (m, 2H), 6.47 (s, 1H), 4.90 (t, 1H), 4.77 (d, 2H), 3.70 (d, 2H), 3.10-3.03 (m, 1H), 2.54-2.51 (m, 1H), 2.98 - 2.79 (m, 4H), 2.72 - 2.61 (m, 1H), 2.45-2.30 (m, 3H), 2.18 - 2.08 (m, 2H), 2.03 - 1.89 (m, 1H), 1.83-1.71 (m, 4H), 1.55 - 1.41 (m, 2H).

LCMS (ESI): = 461.2 [M+H] $^+$.

## Example 12

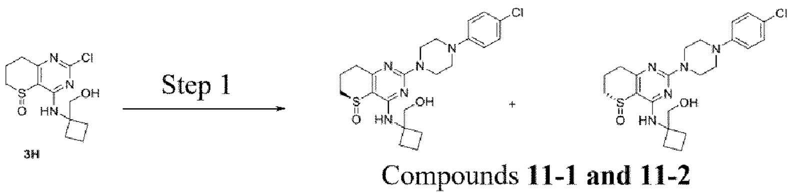

3H

Compounds **11-1 and 11-2**

Step 1: Compound **3H** (150 mg, 0.50 mmol), 4-(4-chlorophenyl)piperazine hydrochloride (131 mg, 0.55 mmol), and DIPEA (260 mg, 2.00 mmol) were dissolved in dioxane and reacted at 90°C for 16 h. After the reaction was complete, the system was concentrated under reduced pressure and subjected to SFC preparation to obtain **compounds 11-1** (29.2 mg, 13%) **and 11-2** (39.9 mg, 17%).

Chiral resolution method: instrument: Waters 150 SFC; column: Chiralcel OX Column (250 × 30 mm, I.D 30 mm, 10 um particle size); mobile phase: A for $CO_2$ and B for EtOH + ACN (0.1% $NH_3$•$H_2O$); gradient: 45% phase B isocratic elution; flow rate: 120 mL/min; back pressure: 100 bar; column temperature: 25°C; wavelength: 220 nm.

**Compound 11-1,** retention time: 1.192 min. $^1$H NMR (400 MHZ, DMSO-d6) δ 7.42 - 7.17 (m, 2H), 7.12 -6.84 (m, 2H), 6.55 (s, 1H), 4.89 (t, 1H), 3.84 (t, 4H), 3.71 (d, 2H), 3.18-3.13 (m, 4H), 3.10-3.04 (m, 1H), 2.95-2.87 (m, 1H), 2.74 - 2.63 (m, 1H), 2.61-2.51 (m, 1H), 2.46 - 2.23 (m, 3H), 2.23 - 2.10 (m, 2H), 2.00-1.94 (m, 1H), 1.91 - 1.67 (m, 2H).

LCMS (ESI): = 462.2 [M+H] $^+$.

**Compound 11-2,** retention time: 1.766 min. $^1$H NMR (400 MHZ, DMSO-d6) δ 7.42 - 7.17 (m, 2H), 7.12 -6.84 (m, 2H), 6.55 (s, 1H), 4.89 (t, 1H), 3.84 (t, 4H), 3.71 (d, 2H), 3.18-3.13 (m, 4H), 3.10-3.04 (m, 1H), 2.95-2.87 (m, 1H), 2.74 - 2.63 (m, 1H), 2.61-2.51 (m, 1H), 2.46 - 2.23 (m, 3H), 2.23 - 2.10 (m, 2H), 2.00-1.94 (m, 1H), 1.91 - 1.67 (m, 2H).

LCMS (ESI): = 462.2 [M+H] $^+$.

## Example 13

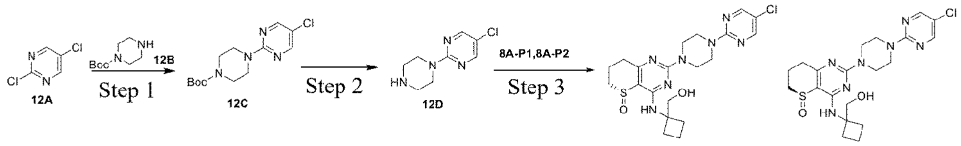


Compounds 12-1 and 12-2

Step 1: Compound **12A** (3.00 g, 20.10 mmol) and compound **12B** (3.74 g, 20.10 mmol) were weighed into a 100 mL single-mouth flask and then dissolved by adding 1,4-dioxane (30 mL), and N,N-diisopropylethylamine (7.79 g, 60.30 mmol) was added to the reaction liquid. After the addition was complete, the mixture was stirred at 80 degrees Celsius for 16 h. After the reaction was complete as determined by a TLC spotting plate (petroleum ether:ethyl acetate = 5:1), water (20 mL) was added to the reaction liquid and stirred for 5 min. The liquid was extracted by adding ethyl acetate (20 mL), and the organic phase was separated. The organic phase was dried over anhydrous sodium sulfate, and the crude product was concentrated under reduced pressure and purified by column chromatography (eluents: petroleum ether:ethyl acetate = 5:1) to obtain the target compound **12C** (3.0 g, yield: 50%).

LCMS m/z = 299.1 $[M +1]^+$

Step 2: Compound **12C** (1.0 g, 3.35 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **12D** (0.80 g, crude).

LCMS m/z = 199.1 $[M +1]^+$

Step 3: Compound **8A-P1** (80 mg, 0.27 mmol), compound **12D** (65 mg, 0.33 mmol), and N,N-diisopropylethylamine (0.10 g, 0.77 mmol) were added to a 100 mL single-mouth flask. After the addition was complete, the system was placed under nitrogen protection and stirred at 90°C for 16 h, and the reaction liquid was concentrated under reduced pressure to obtain the crude target compound, which was subjected to preparative HPLC to obtain the title compound 12-1 (60 mg, 48%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 35-80%; c. flow: 15 mL/min; d. elution time: 20 min; retention time: 13.50 min.

The synthesis, preparation, and separation of compound 12-2 (58 mg, 47%) were the same as those of compound 12-1.

LCMS m/z = 464.2 $[M +1]^+$

**Compound 12-1,** $^1$H NMR (400 MHZ, DMSO-d6) δ 8.44 (d, 2H), 6.55 (s, 1H), 4.88 (t, 1H), 3.73-3.82 (m, 8H), 3.70 (d, 2H), 3.04-3.10 (m, 1H), 2.88-2.95 (m, 1H), 2.54-2.70 (m, 2H), 2.29-2.44 (m, 3H), 2.10-2.19 (m, 2H), 1.90-2.00 (m, 1H), 1.72-1.85 (m, 2H).

**Compound 12-2,** $^1$H NMR (400 MHZ, DMSO-d6) δ 8.44 (d, 2H), 6.55 (s, 1H), 4.88 (t, 1H), 3.73-3.82 (m, 8H), 3.70 (d, 2H), 3.04-3.10 (m, 1H), 2.88-2.95 (m, 1H), 2.54-2.70 (m, 2H), 2.29-2.44 (m, 3H), 2.10-2.19 (m, 2H), 1.90-2.00 (m, 1H), 1.73-1.87 (m, 2H).

**Example 14**

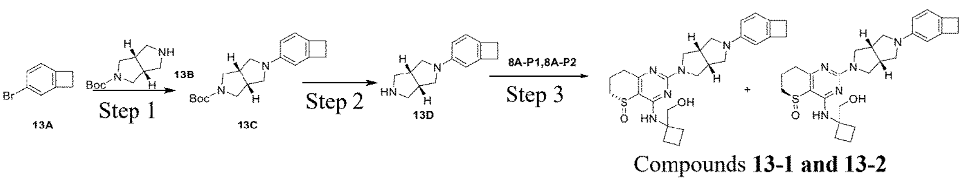


Compounds 13-1 and 13-2

Step 1: Compound **13A** (1.00 g, 5.45 mmol) and compound **13B** (1.16 g, 5.45 mmol) were weighed into a 100 mL single-mouth flask and then dissolved by adding 1,4-dioxane (30 mL), and sodium tert-butoxide (1.57 g, 16.34 mmol), tris(dibenzylideneacetone)dipalladium (0.15 g, 0.27 mmol), and 1,1'-binaphthyl-2,2'-bisdiphenylphosphine (0.34 g, 0.55 mmol) were added to the reaction liquid. After the addition was complete, the mixture was stirred at 90 degrees Celsius for 16 h. After the reaction was complete as determined by a TLC spotting plate (petroleum ether:ethyl acetate = 5:1), water (20 mL) was added to the reaction liquid and stirred for 5 min. The liquid was extracted by adding ethyl acetate (20 mL), and

the organic phase was separated. The organic phase was dried over anhydrous sodium sulfate, and the crude product was concentrated under reduced pressure and purified by column chromatography (eluents: petroleum ether:ethyl acetate = 5:1) to obtain the target compound **13C** (1.0 g, yield: 58%).

LCMS m/z = 315.1 [M +1]$^+$

Step 2: Compound **13C** (0.30 g, 0.95 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **13D** (0.20 g, crude).

LCMS m/z = 215.1 [M +1]$^+$

Step 3: Compound **8A-P1** (80 mg, 0.27 mmol), compound **13D** (69 mg, 0.32 mmol), and N,N-diisopropylethylamine (0.10 g, 0.77 mmol) were added to a 100 mL single-mouth flask. After the addition was complete, the system was placed under nitrogen protection and stirred at 90°C for 16 h, and the reaction liquid was concentrated under reduced pressure to obtain the crude target compound, which was subjected to preparative HPLC to obtain the title compound **13-1** (12 mg, 9%).

Preparation method: instrument: waters 2767 preparative liquid chromatographic instrument; chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM aqueous ammonia); b. gradient elution, mobile phase A with a content of 45-85%; c. flow: 15 mL/min; d. elution time: 20 min; retention time: 12.80 min.

The synthesis, preparation, and separation of compound **13-2** (40 mg, 30%) were the same as those of compound **13-1.**

LCMS m/z = 480.2 [M +1]$^+$

**Compound** 13-1, $^1$H NMR (400 MHz, DMSO-$d_6$) 6 6.84 (d, 1H), 6.45 (s, 1H), 6.33-6.37(m, 2H), 4.88 (t, 1H), 3.67-3.79(m, 4H), 3.36-3.46 (m, 4H), 3.11 (d, 2H), 2.97-3.01 (m, 7H), 2.85-2.92 (m, 1H), 2.53-2.67 (m, 2H), 2.25 -2.46 (m, 3H), 2.05 -2.15 (m, 2H), 1.89-1.98 (m, 1H), 1.69 -1.85 (m, 2H).

**Compound** 13-2, $^1$H NMR (400 MHz, DMSO-$d_6$) 6 6.84 (d, 1H), 6.45 (s, 1H), 6.33-6.37(m, 2H), 4.88 (t, 1H), 3.67-3.79(m, 4H), 3.36-3.46 (m, 4H), 3.11 (d, 2H), 2.97-3.01 (m, 7H), 2.85-2.91 (m, 1H), 2.53-2.67 (m, 2H), 2.25 -2.46 (m, 3H), 2.05 -2.15 (m, 2H), 1.89-1.98 (m, 1H), 1.69 -1.84 (m, 2H).

**Example 15**

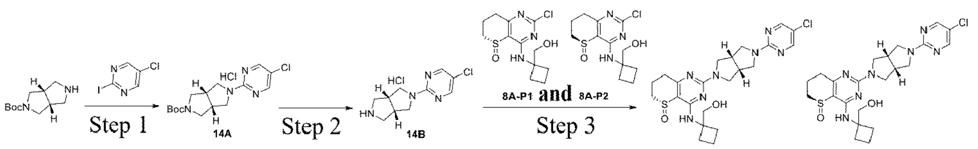


**Compound 14-1 and compound 14-2**

Using the compound tert-butyl (3aR,6aS)-hexahydropyrrolo[3,4-c]pyrrole-2(1H)-carboxylate and **9A** as raw materials, **compound 14-1** (76 mg, 59.6%) was obtained according to the operation of Example 13.

The synthesis and purification of **compound 14-2** (83 mg, 65.1%) were the same as those of **compound 14-1.**

**Compound 14-1:** $^1$H NMR (400 MHz, DMSO-$d_6$) δ 8.39 (s, 2H), 6.46 (s, 1H), 4.95 - 4.81 (m, 1H), 3.81 - 3.64 (m, 6H), 3.46 - 3.34 (m, 4H), 3.12 - 3.00 (m, 3H), 2.95 - 2.82 (m, 1H), 2.71 - 2.54 (m, 2H), 2.45 - 2.25 (m, 3H), 2.18 - 2.05 (m, 2H), 2.00 - 1.88 (m, 1H), 1.88 - 1.64 (m, 2H).

**Compound 14-2:** $^1$H NMR (400 MHz, DMSO-$d_6$) δ 8.39 (s, 2H), 6.46 (s, 1H), 4.91 - 4.83 (m, 1H), 3.82 - 3.62 (m, 6H), 3.47 - 3.36 (m, 4H), 3.15 - 3.02 (m, 3H), 2.94 - 2.81 (m, 1H), 2.70 - 2.52 (m, 2H), 2.47 - 2.24 (m, 3H), 2.18 - 2.05 (m, 2H), 2.03 - 1.89 (m, 1H), 1.88 - 1.63 (m, 2H).

LC-MS (ESI): m/z = 490.6 [M+H]$^+$.

**Example 16**

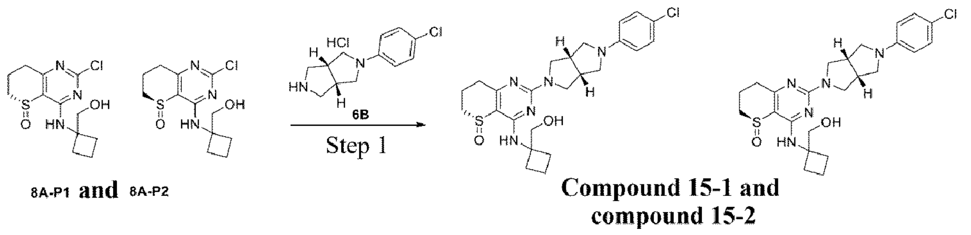


8A-P1 and 8A-P2

Compound 15-1 and compound 15-2

Step 1: **8A-P1** (80 mg, 0.26 mmol) and **6B** (120 mg, see WO 2020099886 for the synthesis method) were dissolved in 1,4-dioxane (10 mL), DIPEA (0.5 mL) was added, and the mixture was left at 90°C overnight. After the raw material was completely reacted as detected by LCMS, the system was concentrated, water (100 mL) was added, and the mixture was extracted with a mixed solution of DCM:MeOH = 20:1 (10 mL × 3). The organic phases were combined, dried over anhydrous sodium sulfate, concentrated, and then passed through a column (DCM:MeOH = 1:0-0:1) to obtain **compound 15-1** (84 mg, 66.2%).

The synthesis and purification of **compound 15-2** (88 mg, 69.8%) were the same as those of **compound 15-1.**

**Compound 15-1:** $^1$H NMR (400 MHz, DMSO-$d_6$) δ 7.21 - 7.10 (m, 2H), 6.58 - 6.50 (m, 2H), 6.46 (s, 1H), 4.92 - 4.85 (m, 1H), 3.84 - 3.73 (m, 2H), 3.73 - 3.65 (m, 2H), 3.51 - 3.43 (m, 2H), 3.43 - 3.35 (m, 2H), 3.20 - 3.12 (m, 2H), 3.11 - 3.00 (m, 3H), 2.93 - 2.81 (m, 1H), 2.69 - 2.59 (m, 1H), 2.58 - 2.51 (m, 1H), 2.47 - 2.25 (m, 3H), 2.16 - 2.04 (m, 2H), 1.99 - 1.88 (m, 1H), 1.87 - 1.64 (m, 2H).

**Compound 15-2:** $^1$H NMR (400 MHz, DMSO-$d_6$) δ 7.20 - 7.11 ,mnbcx (m, 2H), 6.58 - 6.50 (m, 2H), 6.46 (s, 1H), 4.93 - 4.84 (m, 1H), 3.81 - 3.72 (m, 2H), 3.72 - 3.64 (m, 2H), 3.52 - 3.43 (m, 2H), 3.43 - 3.34 (m, 2H), 3.22 - 3.11(m, 2H), 3.11 - 3.02 (m, 3H), 2.91 - 2.80 (m, 1H), 2.68 - 2.59 (m, 1H), 2.58 - 2.52 (m, 1H), 2.48 - 2.29 (m, 3H), 2.16 - 2.03 (m, 2H), 1.99 - 1.87 (m, 1H), 1.87 - 1.64 (m, 2H).

LC-MS (ESI): m/z = 488.6 [M+H]$^+$.

**Example 17**

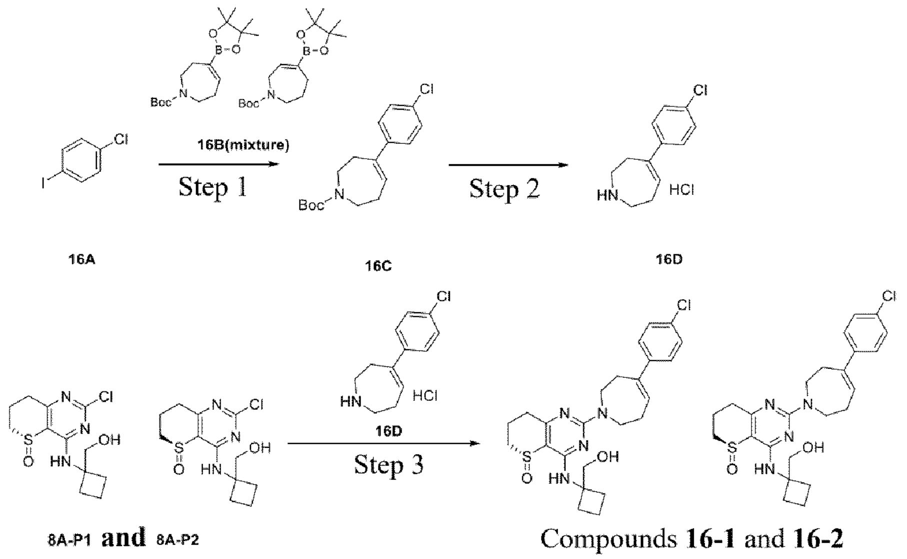


16A 16C 16D

8A-P1 and 8A-P2

Compounds 16-1 and 16-2

Using compounds **16A** and **16B** as raw materials, **compound 16-1** (50 mg, 20%) was obtained according to the operation of Example 10.

The synthesis (with **8A-P2** as a raw material), preparation, and separation of compound **16-2** (50 mg, 20%) were the same as those of compound **16-1.**

Preparation method: instrument: SHIMADZU LC-20AP; chromatographic column: Phenomenex C18; the sample was dissolved with acetonitrile and water and filtered with a 0.45 μm filter to prepare a sample liquid. Preparative chromatography conditions: composition of mobile phases A and B: mobile phase A: 10 mmol ammonium bicarbonate, mobile phase B: acetonitrile; gradient elution, mobile phase B with content from 30% to 60%; flow: 25 ml/min; elution time: 15 min; retention time: 3.3 min.

**Compound 16-1,** $^1$H NMR (400 MHz, DMSO-$d_6$) δ 7.34 (s, 4H), 6.45 (s, 1H), 6.03 (t, 1H), 4.88 (t, 1H), 3.92 (brs,

2H), 3.83 (brs, 2H), 3.70 (d, 2H), 3.09-3.03 (m, 1H), 2.94-2.87 (m, 1H), 2.72-2.63 (m, 3H), 2.59-2.52 (m, 1H), 2.51-2.45 (m, 2H), 2.43 - 2.26 (m, 3H), 2.14-2.10 (m, 2H), 1.97-1.92 (m, 1H), 1.83-1.74 (m, 2H).

**Compound 16-2,** $^1$H NMR (400 MHz, DMSO-$d_6$) δ 7.34 (s, 4H), 6.45 (s, 1H), 6.03 (t, 1H), 4.88 (t, 1H), 3.92 (brs, 2H), 3.83 (brs, 2H), 3.70 (d, 2H), 3.09-3.03 (m, 1H), 2.94-2.87 (m, 1H), 2.72-2.63 (m, 3H), 2.59-2.52 (m, 1H), 2.51-2.45 (m, 2H), 2.43 - 2.26 (m, 3H), 2.14-2.10 (m, 2H), 1.97-1.92 (m, 1H), 1.83-1.74 (m, 2H).

LCMS (ESI): = 473.2 [M+H] $^+$.

**Example 18**

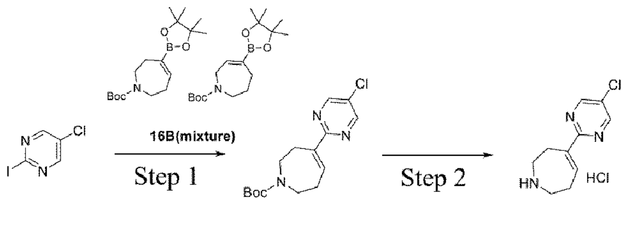


17A 17B 17C

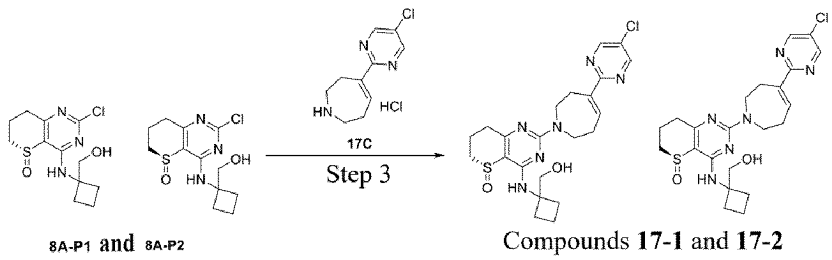


8A-P1 and 8A-P2

Compounds **17-1** and **17-2**

Using **17A** and **16B** as raw materials, **compound 17-1** (30 mg, 12%) was obtained according to the operation of Example 10.

The synthesis (with **8A-P2** as a raw material), preparation, and separation of compound **17-2** (48 mg, 19%) were the same as those of compound **17-1.**

Preparation method: instrument: Waters 150 SFC; chromatographic column: Chiralpak IG Column (250 × 30 mm, I.D 30 mm, 10 um particle size); preparative chromatography conditions: a. composition of mobile phases A and B: mobile phase A: CO2, mobile phase B: EtOH + ACN (0.1% $NH_3$•$H_2O$); b. isocratic elution, mobile phase B with a content of 65%; c. flow: 100 mL/min; d. elution time: 15 min; e. back pressure: 100 bar; f. column temperature: 25 degrees Celsius.

**Compound 17-1,** $^1$H NMR (400 MHz, DMSO-$d_6$) δ 8.84 (s, 2H), 7.38 (s, 1H), 6.45 (s, 1H), 4.91 (t, 1H), 3.88-3.86 (m, 4H), 3.71 (d, 2H), 3.09-3.00 (m, 3H), 2.91-2.87 (m, 1H), 2.71 - 2.52 (m, 4H), 2.46 - 2.24 (m, 3H), 2.16-2.07 (m, 2H), 2.02 - 1.91 (m, 1H), 1.86 - 1.68 (m, 2H).

**Compound 17-2,** $^1$H NMR (400 MHz, DMSO-$d_6$) δ 8.84 (s, 2H), 7.38 (s, 1H), 6.45 (s, 1H), 4.91 (t, 1H), 3.88-3.86 (m, 4H), 3.71 (d, 2H), 3.09-3.00 (m, 3H), 2.91-2.87 (m, 1H), 2.71 - 2.52 (m, 4H), 2.46 - 2.24 (m, 3H), 2.16-2.07 (m, 2H), 2.02 - 1.91 (m, 1H), 1.86 - 1.68 (m, 2H).

LCMS (ESI): = 475.1 [M+H] $^+$.

**Example 19**

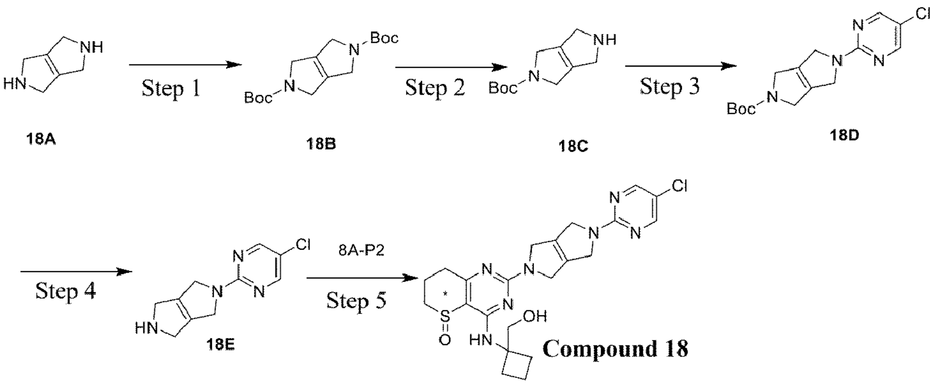


18A 18B 18C 18D 18E Compound 18

Step 1: Compound **18A** (1.50 g, 13.62 mmol) and di-tert-butyl dicarbonate (5.95 g, 27.24 mmol) were weighed into a 100 mL single-mouth flask and then dissolved by adding water (30 mL), and sodium bicarbonate (3.43 g, 40.86 mmol) was added to the reaction liquid. After the addition was complete, the mixture was stirred for 16 h. After the reaction was complete as determined by a TLC spotting plate (petroleum ether:ethyl acetate = 5:1), a solid precipitated from the reaction liquid and is directly filtered to obtain the target compound **18B** (1.5 g, yield: 35%).

$^1$H NMR (400 MHZ, DMSO-d6) δ 3.96-4.06 (m, 8H), 1.42 (s, 18H).

Step 2: Compound **18B** (1.50 g, 4.83 mmol) and p-toluenesulfonic acid (0.83 g, 4.83 mmol) were weighed into a 100 mL single-mouth flask, then dissolved by adding isopropyl acetate (30 mL), and stirred at 60°C for 4 h. After the reaction was complete as determined by a TLC spotting plate (petroleum ether:ethyl acetate = 3:1), a solid precipitated from the reaction liquid and is directly filtered to obtain the target compound **18C** (1.5 g, yield: 100%).

$^1$H NMR (400 MHZ, DMSO-$d_6$) δ 3.96-4.03 (m, 8H), 1.42 (s, 9H).

Step 3: Compound **18C** (0.30 g, 1.43 mmol) and 2-chloro-5-chloropyrimidine (0.21 g, 1.43 mmol) were weighed into a 100 mL single-mouth flask and then dissolved by adding 1,4-dioxane (20 mL), and N,N-diisopropylethylamine (0.55 g, 4.26 mmol) was added to the reaction liquid. After the addition was complete, the mixture was stirred at 80 degrees Celsius for 16 h. After the reaction was complete as determined by a TLC spotting plate (petroleum ether:ethyl acetate = 5:1), water (20 mL) was added to the reaction liquid and stirred for 5 min. The liquid was extracted by adding ethyl acetate (20 mL), and the organic phase was separated. The organic phase was dried over anhydrous sodium sulfate, and the crude product was concentrated under reduced pressure and purified by column chromatography (eluents: petroleum ether:ethyl acetate = 5:1) to obtain the target compound **18D** (0.2 g, yield: 43%).

LCMS m/z = 323.1 [M +1]$^+$

Step 4: Compound **18D** (0.2g, 0.62mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **18E** (0.15g, crude).

LCMS m/z = 223.1 [M +1]$^+$

Step 5: Compound **8A-P2 (single configuration, with the configuration being undetermined)** (150 mg, 0.50 mmol), compound **18E** (110 mg, 0.50 mmol), and N,N-diisopropylethylamine (0.20 g, 1.51 mmol) were added to a 100 mL single-mouth flask. After the addition was complete, the system was placed under nitrogen protection and stirred at 90°C for 16 h, and the reaction liquid was concentrated under reduced pressure to obtain the crude target compound, which was subjected to preparative HPLC to obtain the title compound **18 (single configuration, with the configuration being undetermined)** (30 mg, 12%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 30-70%; c. flow: 15 mL/min; d. elution time: 20 min; retention time: 10.80 min.

LCMS m/z = 488.1 [M +1]$^+$

$^1$H NMR (400 MHZ, DMSO-*d6*) δ 8.45 (d, 2H), 6.54 (s, 1H), 4.90(t, 1H), 4.30 (m, 8H), 3.73 (d, 2H), 3.04-3.11 (m, 1H), 2.88-2.95 (m, 1H), 2.58-2.72 (m, 2H), 2.32-2.44 (m, 3H), 2.15 (s, 2H), 1.91-2.00 (m, 1H), 1.71-1.88 (m, 2H).

**Example 20**

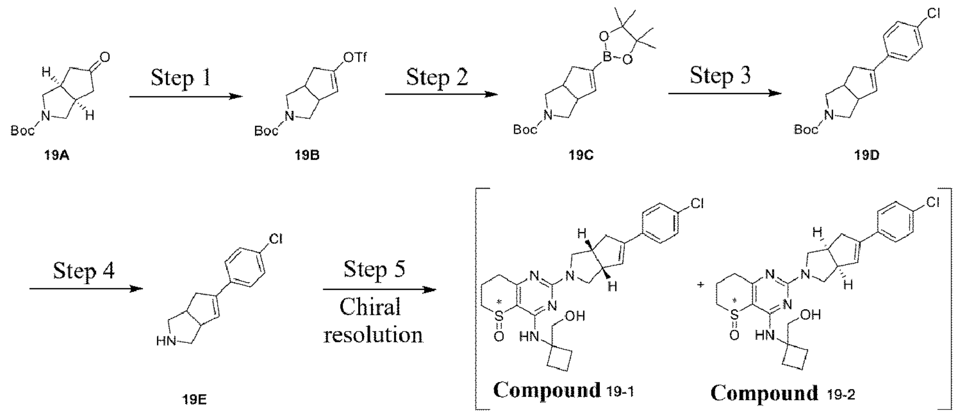


Step 1: Compound 19A (1 g, 4.44 mmol) was dissolved in anhydrous tetrahydrofuran (10 ml). After nitrogen displacement three times, the reaction system was cooled to -78°C, and lithium bis(trimethylsilyl)amide (8.88 mmol) was dropwise added. After the system was maintained at -78°C for 30 min, N-phenyl-bis(trifluoromethanesulfonimide) (2.59 g, 6.66 mmol) dissolved in anhydrous tetrahydrofuran (5 ml) was dropwise added. The system was further maintained at -78°C for 30 min, then transferred to room temperature, and reacted for 2 h. After the reaction was complete, the reaction was quenched by adding a saturated ammonium chloride solution (30 ml), the organic phase was extracted with ethyl acetate (3 × 40 ml), and the organic phases were combined, and dried over anhydrous sodium sulfate. After filtration and concentration, the concentrated product was subjected to quick separation and purification by column chromatography (eluent ratio: EA/PE = 0-10%) to obtain compound 19B (1.3 g, 82%).

LCMS (ESI): m/z = 302.2 $[M+H-56]^+$

Step 2: Under nitrogen, bis(triphenylphosphine)palladium dichloride (260 mg, 0.37 mmol), pinacol diboronate (1.11 g, 4.37 mmol), and potassium acetate (1.07 g, 10.92 mmol) were added to compound 19B (1.3 g, 3.64 mmol) dissolved in 1,4-dioxane (20 ml) and reacted in an oil bath at 90°C for 18 h. After the reaction was complete, the system was cooled to room temperature, filtered with diatomite, and washed with ethyl acetate, and the filtrate was concentrated and directly subjected to quick separation and purification by column chromatography (eluent ratio: EA/PE = 0-10%) to obtain the target compound 19C (0.89 g, 73%).

LCMS (ESI): m/z = 280.2 $[M+H-56]^+$

Step 3: Under nitrogen, 5-chloro-2-iodobenzene (574 mg, 2.41 mmol), 1,1-bis(diphenylphosphine)ferrocene-palladium dichloride (176 mg, 0.24 mmol), and sodium carbonate (766 mg, 7.32 mmol) were added to compound 19C (0.89 g, 2.65 mmol) dissolved in 1,4-dioxane/water (10 ml/2 ml) and reacted in an oil bath at 90°C for 18 h. After the reaction was complete, the system was cooled to room temperature, filtered with diatomite, and washed with ethyl acetate, and the filtrate was concentrated and directly subjected to quick separation and purification by column chromatography (eluent ratio: EA/PE = 0-10%) to obtain the target compound 19D (0.44 g, 52%).

LCMS (ESI): m/z = 264.2 $[M+H-56]^+$

Step 4: Compound 19D (220 mg, 0.69 mmol) was dissolved in hydrogen chloride 1,4-dioxane (2 ml), and the mixture was stirred at room temperature for 1 h and directly spin-dried to obtain compound 19E hydrochloride (170 mg).

LCMS (ESI): m/z = 220.1 $[M+H]^+$

Step 5: Intermediate **8A-P2 (single configuration, with the configuration being undetermined)** (100 mg, 0.33 mmol) was dissolved in 1,4-dioxane (5 ml), compound 19E hydrochloride (85 mg, 0.33 mmol) and diisopropylethylamine (128 mg, 0.99 mmol) were added, and the reaction system was placed in an oil bath at 100°C and reacted for 15 h. After the reaction was complete, the system was cooled to room temperature. The reaction was monitored by LCMS. The system was spin-dried and then subjected to preparation. Preparative HPLC separation method: 1. instrument: waters 2767 preparative liquid chromatographic instrument; chromatographic column: SunFire@ Prep C18 (19 mm × 250 mm); 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: with a content of 10-55%; c. flow: 12 mL/min. With retention time 7.0 min, **compound 19** (130 mg, 81%) was obtained.

LCMS (ESI): m/z = 485.3 $[M+H]^+$

Resolution of compound **19:**

**Compound 19** (130 mg) was taken for resolution, and after separation, **compound 19-1** (retention time: 1.134 min, 37 mg, ee% = 100%) and **compound 19-2** (retention time: 1.421 min, 32 mg, ee% = 100%) were obtained.

Resolution conditions:

instrument: Waters 150 MGM; column: DAICEL CHIRALPAK AD;
mobile phase: A for $CO_2$ and B for EtOH (0.1% $NH_3$•$H_2O$)); gradient: B 40%; flow: 120 mL/min;
back pressure: 100 bar;
column temperature: 35°C; wavelength: 220 nm; period: 13 min;

**Compound 19-1:**

$^1$H NMR (400 MHz, $CDCl_3$) δ = 7.33 (d, 2H), 7.27 (d, 2H), 6.19 (s, 1H), 6.03 (s, 1H), 3.94 (s, 1H), 3.87 (s, 2H), 3.80 - 3.65 (m, 2H), 3.60 (s, 1H), 3.21-3.16 (m, 2H), 3.11 (s, 1H), 3.00 - 2.74 (m, 3H), 2.67 - 2.46 (m, 3H), 2.45 - 2.26 (m, 2H), 2.24 - 2.11 (m, 2H), 2.10 - 2.02 (m, 1H), 2.01 - 1.87 (m, 2H).

**Compound 19-2:**

$^1$H NMR (400 MHz, $CDCl_3$) δ = 7.32 (d, 2H), 7.27 (d, 2H), 6.18 (s, 1H), 6.03 (s, 1H), 4.00 (s, 1H), 3.87 (s, 2H), 3.74-3.66 (m, 2H), 3.60 (s, 1H), 3.29 - 3.15 (m, 2H), 3.15 - 3.03 (m, 1H), 3.02 - 2.73 (m, 3H), 2.68 - 2.47 (m, 3H), 2.40 (s, 1H), 2.36 - 2.26 (m, 1H), 2.23 - 2.10 (m, 2H), 2.10 - 2.01 (m, 1H), 2.02 - 1.84 (m, 2H).

**Example 21**

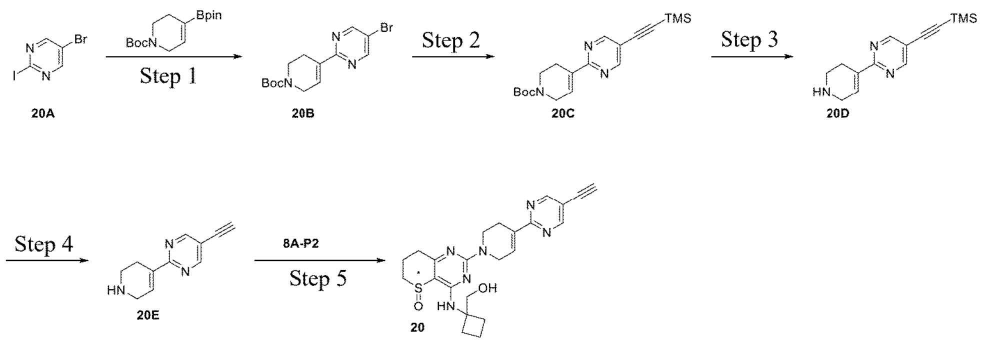

20A 20B 20C 20D 20E 20

Step 1: Compound **20A** (1.4 g, 5.0 mmol) was dissolved in 1,4-dioxane (30 mL), and 1,1-bis(diphenylphosphine) ferrocenepalladium dichloride (360.0 mg, 0.5 mmol), potassium carbonate (1.4 g, 10.0 mmol), N-Boc-1,2,5,6-tetrahydropyridine-4-boronic acid pinacol ester (1.8 g, 6.0 mmol), and water (6.0 mL) were then added. After nitrogen displacement three times, the system was heated to 45°C and reacted for 14 h. The system passed through a short silica gel column, eluted with ethyl acetate, concentrated, and subjected to column chromatography (dichloromethane/methanol = 20/1) to obtain compound **20B** (1.7 g, 99%).
LC-MS (ESI): m/z = 284.1 $[M-56]^+$
Step 2: Compound **20B** (340.0 mg, 1.0 mmol) was dissolved in tetrahydrofuran (10 mL), and bis(triphenylphosphine)palladium dichloride (70.0 mg, 0.1 mmol), triethylamine (202.0 mg, 2.0 mmol), trimethylsilylacetylene (130.0 mg, 1.3 mmol), and cuprous iodide (8.0 mg, 0.05 mmol) were then added. After nitrogen displacement three times, the system was heated to 50°C and reacted for 14 h. The system passed through a short silica gel column, eluted with ethyl acetate, concentrated, and subjected to column chromatography (dichloromethane/methanol = 20/1) to obtain compound **20C** (240.0 mg, 72%).
LC-MS (ESI): m/z = 302.1 $[M-56]^+$
Step 3: Compound **20C** (240.0 mg, 0.67 mmol) was dissolved in dichloromethane (10 mL), trifluoroacetic acid (1.0 mL) was then added, and the mixture was reacted at room temperature for half an hour and then concentrated to obtain compound **20D** (150.0 mg, 87%), which was directly used for the next step.
LC-MS (ESI): m/z = 258.1 $[M+H]^+$
Step 4: Compound **20D** (150.0 mg, 0.58 mmol) was dissolved in methanol (10.0 mL), potassium carbonate (150.0 mg, 1.1 mmol) was then added, and the mixture was reacted at room temperature for 2 h, then concentrated, and subjected to column chromatography (dichloromethane/methanol = 15/1) to obtain compound **20E** (70.0 mg, 65%).

LC-MS (ESI): m/z = 186.1 [M+H]+

Step 5: Compound **20E** (140.0 mg, 0.74 mmol) was dissolved in 1,4-dioxane (15.0 mL), compound **8A-P2 (single configuration, with the configuration being undetermined)** (230.0 mg, 0.8 mmol) was then added, and the mixture was heated back to 90°C, reacted for 12 h, then cooled to room temperature, concentrated, and subjected to column chromatography (dichloromethane/methanol = 10/1) to obtain compound **20** (120.0 mg, 62%).

LC-MS (ESI): m/z = 451.1 [M+H]+

$^1$H NMR (400 MHz, DMSO-d6) δ 8.90 (s, 2H), 7.34 (s, 1H), 6.53 (s, 1H), 4.89 (s, 1H), 4.68 (s, 1H), 4.45 (s, 2H), 3.95 - 3.92 (m, 2H), 3.73 (d, 2H), 3.09 - 3.05 (m, 1H), 2.95 - 2.88 (m, 1H), 2.66 (d, 4H), 2.44 - 2.31 (m, 3H), 2.20 - 2.14 (m, 2H), 1.98 - 1.93 (m, 1H), 1.87 - 1.77 (m, 2H).

**Example 22**

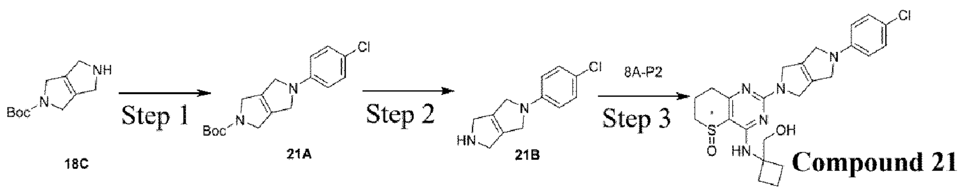


18C 21A 21B Compound 21

Step 1: Compound **18C** (0.50 g, 2.38 mmol) and 1-chloro-4-iodobenzene (0.68 g, 2.68 mmol) were weighed into a 100 mL single-mouth flask and then dissolved by adding 1,4-dioxane (30 mL). 1,1'-Binaphthyl-2,2'-bisphenylphosphine (0.15 g, 0.24 mmol), sodium tert-butoxide (0.69 g, 7.14 mmol), and tris(dibenzylideneacetone)dipalladium (0.14 g, 0.24 mmol) were added to the reaction liquid. After the addition was complete, the mixture was stirred at 85 degrees Celsius for 16 h. After the reaction was complete as determined by a TLC spotting plate (petroleum ether:ethyl acetate = 5:1), water (20 mL) was added to the reaction liquid and stirred for 5 min. The liquid was extracted by adding ethyl acetate (20 mL), and the organic phase was separated. The organic phase was dried over anhydrous sodium sulfate, and the crude product was concentrated under reduced pressure and purified by column chromatography (eluents: petroleum ether:ethyl acetate = 5:1) to obtain the target compound **21A** (0.28 g, yield: 37%).

LCMS m/z = 321.1 [M +1]+

Step 2: Compound **21A** (0.2 g, 0.62 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure to obtain the target compound **21B** (0.15 g, crude).

LCMS m/z = 221.1 [M +1]+

Step 3: Compound **8A-P2** (150 mg, 0.50 mmol), compound **21B** (110 mg, 0.50 mmol), and N,N-diisopropylethylamine (0.20 g, 1.51 mmol) were added to a 100 mL single-mouth flask. After the addition was complete, the system was placed under nitrogen protection and stirred at 90°C for 16 h, and the reaction liquid was concentrated under reduced pressure to obtain the crude target compound, which was subjected to preparative HPLC to obtain the title compound **21** (50 mg, 20%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min; retention time: 12.20 min.

LCMS (ESI): m/z = 486.1 [M +1]+

$^1$H NMR (400 MHZ, DMSO-d6) δ 7.21 (d, 2H), 6.53 (d, 2H), 4.90(t, 1H), 4.31 (s, 4H), 4.10 (s, 4H), 3.73 (d, 2H), 3.05-3.10 (m, 1H), 2.89-2.95 (m, 1H), 2.55-2.72 (m, 2H), 2.32-2.46 (m, 4H), 2.16 (s, 2H), 1.92-2.00 (m, 1H), 1.71-1.87 (m, 2H).

**Example 23**

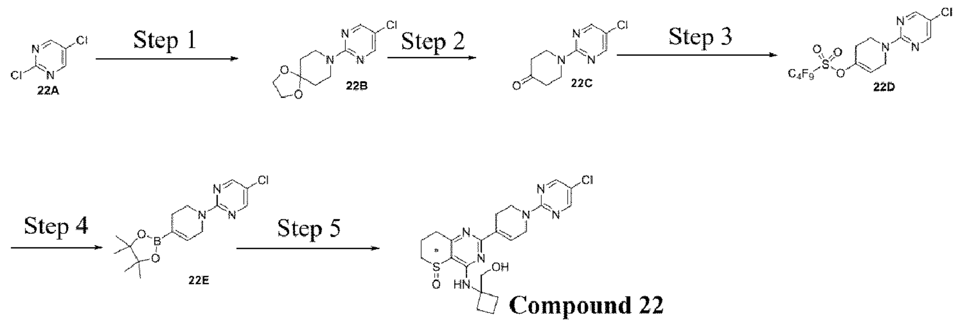


Compound 22

Step 1: **22A** (5.0 g,33.6 mmol), 1,4-dioxa-8-azaspiro[4.5]decane (5.3 g, 36.9 mmol), and DIPEA (13.0 g, 100.8 mmol) were dissolved in 100 mL of dioxane, and the system was heated to 80°C and reacted for 2 h. The reaction system was poured into ice water and extracted twice with ethyl acetate, the organic phases were combined and dried, and the solvent was removed by rotary evaporation. Purification was carried out by silica gel column chromatography (PE:EA = 20:1) to obtain the target compound **22B** (8.5 g, yield 99%).

LCMS (ESI): m/z = 256.2 $[M+H]^+$

Step 2: The raw material **22B** (8.5 g, 33.2 mmol) was dissolved in 30 mL of tetrahydrofuran, 120 mL of 10% sulfuric acid was added, and the mixture was heated to 90°C and reacted under reflux for 12 h. After the reaction was confirmed to be complete by a TLC plotting plate, the system was cooled to room temperature and adjusted to neutral pH with saturated sodium bicarbonate. After washing with ethyl acetate (100 mL × 3), the organic phases were washed with a saturated sodium chloride aqueous solution and dried over anhydrous sodium sulfate, and the solvent was removed by rotary evaporation to obtain the target compound **22C** (6.2 g, yield 88%), which was directly used in the next reaction.

LCMS (ESI): m/z = 212.3 $[M+H]^+$

Step 3: The raw material **22C** (6.2 g, 29.2 mmol) and 1,1,2,2,3,3,4,4,4-nonafluorobutane-1-sulfonyl fluoride (10.6 g,35.0 mmol) were dissolved in 62 mL of tetrahydrofuran, 1,8-diazabicyclo[5.4.0]undec-7-ene (5.3 g, 35.0 mmol) was added, and the mixture was stirred at room temperature for 4 h. After the reaction was complete as monitored by TLC, the system was concentrated and then purified by silica gel column chromatography (PE:EA = 50:1) to obtain the target compound **22D** (10.6 g, yield 73%).

LCMS (ESI): m/z = 494.0 $[M+H]^+$

Step 4: The raw material **22D** (6.50 g, 13.2 mmol), pinacol diboronate (4.02 g, 15.8 mmol), [1,1'-bis(diphenylphosphine)ferrocene]palladium dichloride (190 mg, 0.26 mmol), and 1,1'-bis(diphenylphosphine)ferrocene (144 mg, 0.26 mmol) were suspended in dioxane (65 mL) and reacted at room temperature under stirring for 15 min, and potassium acetate (2.58 g, 26.4 mmol) was added and reacted at 90°C for 16 h. After the reaction was complete as monitored by TLC and LCMS, purification was carried out by silica gel column chromatography (PE:EA=20:1) to obtain the title compound **22E** (3.37 g, yield 79%).

LCMS (ESI): m/z = 322.4 $[M+H]^+$

Step 5: Compound **22E** (500 mg, 1.55 mmol), **8A-P2** (422 mg, 1.40 mmol), tetrakis(triphenylphosphine) palladium (33 mg, 0.03 mmol), and sodium carbonate (493 mg, 4.65 mmol) were suspended in 10 mL of a mixed solvent of dioxane:water = 4:1 and reacted at 90°C for 4 h. After the reaction was complete, the reaction liquid was dropwise added to ice water and filtered. The filtrate was extracted three times with dichloromethane, and the organic phases were combined with the filter residue, concentrated, and purified by silica gel column chromatography (DCM:MeOH = 15:1) to obtain the target compound **22** (142 mg, 23%).

$^1H$ NMR (400 MHz, DMSO-d6) δ 8.45 (s, 2H), 7.18 (t, 1H), 6.93 (s, 1H), 4.92 (s, 1H), 4.41-4.35 (m, 2H), 3.97-3.90 (2H), 3.73 (s, 2H), 3.23 - 3.03 (m, 2H), 2.88 - 2.77 (m, 1H), 2.75 - 2.64 (m, 1H), 2.61 (t, 2H), 2.46 - 2.26 (m, 3H), 2.25-2.15 (m, 2H), 2.11 - 1.96 (m, 1H), 1.94 - 1.73 (m, 2H).

LCMS (ESI): m/z = 461.19 $[M+H]^+$

## Example 24

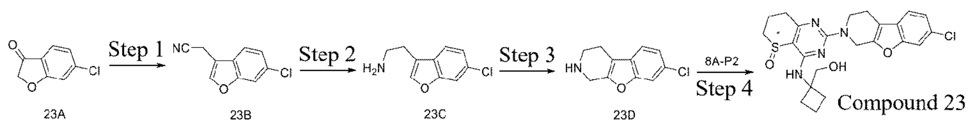


Compound 23

Step 1: Under nitrogen protection, sodium hydride (3.56 g, 88.98 mmol) was slowly added to a solution containing

diethyl cyanomethylphosphate (12.61 g, 71.18 mmol) in tetrahydrofuran (100 mL). After the addition was complete, the reaction liquid was stirred at room temperature for 1 h. After the reaction liquid was cooled to -78°C, **23A** (10.00 g, 59.32 mmol) was added. After the reaction liquid was reacted under stirring for 2 h, the reaction was quenched by adding water (200 mL), and the system was extracted three times with ethyl acetate (100 mL). The organic phases were combined, dried over anhydrous sodium sulfate, and then concentrated in vacuum to obtain a crude product, and the crude product was purified by column chromatography (eluents: PE:EA = 100:1 to 20:1) to obtain the target compound **23B** (6.50 g, yield: 57.18%).

$^1H$ NMR (400 MHz, $CDCl_3$) δ 7.66 - 7.65 (m, 1H), 7.53 - 7.52 (m, 1H), 7.51 - 7.48 (m, 1H), 7.31- 7.28 (m, 1H), 3.74 (s, 2H).

Step 2: Compound **23B** (6.00 g, 31.31 mmol) was dissolved in methanol (120 mL), raney nickel (1 g) was then added, and the reaction liquid was protected by hydrogen displacement and then stirred at room temperature for 16 h. The reaction liquid was filtered, and the filtrate was concentrated under reduced pressure to obtain a crude product. The crude product was purified by column chromatography (eluents: DCM:MEOH = 10:1) to obtain the target compound **23C** (1.60 g, yield: 26.12%).

$^1H$ NMR (400 MHz, $CDCl_3$) δ 7.47 - 7.46 (m, 1H), 7.42 (s, 1H), 7.40 - 7.38 (m, 1H), 7.20 - 7.18 (m, 1H), 2.98 - 2.92 (m, 2H), 2.85 - 2.81 (m, 2H).

Step 3: Compound **23C** (1.60 g, 8.18 mmol) was dissolved in 30 mL of formic acid, paraformaldehyde (3.20 g) was added, and the mixture was then heated to 70°C and stirred for 3 h. The reaction liquid was cooled to room temperature, then concentrated, then washed with a sodium hydroxide aqueous solution, and extracted with ethyl acetate. The organic phase was dried and concentrated to obtain a crude product, and the crude product was purified by column chromatography (eluents: DCM:MEOH = 20:1-10:1) to obtain the target compound **23D** (250 mg, yield: 14.72%).

LCMS m/z = 208.1 $[M+H]^+$

Step 4: Compound **8A-P2** (200 mg, 0.66 mmol) and compound **23D** (250 mg, 1.20 mmol) were dissolved in 1,4-dioxane (6mL), and DIPEA (235 mg, 1.82 mmol) was then added. After the addition was complete, the mixture was heated to 85°C and stirred for 16 h under nitrogen protection. After the reaction was complete, the reaction liquid was concentrated under reduced pressure to obtain a crude product, which was purified by column chromatography (eluents: DCM:MeOH = 100:1 to 9:1) and then further purified by prep.HPLC (preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 25-85%; c. flow: 15 mL/min; d. elution time: 15 min; retention time: 7.0 min) to obtain the target **compound 23** (82.5 mg, 26.32%).

LCMS m/z = 473.2 $[M+H]^+$

$^1H$ NMR (400 MHz, $CDCl_3$) δ 7.45 - 7.44 (m, 1H), 7.34 - 7.32 (m, 1H), 7.22 - 7.19 (m, 1H), 6.23 (br s, 1H), 4.90 (s, 2H), 4.11 (s, 2H), 3.94 (s, 2H), 3.23 - 3.18 (m, 1H), 2.93 - 2.85 (m, 2H), 2.75 (s, 2H), 2.64 - 2.49 (m, 2H), 2.42 - 2.33 (m, 2H), 2.30 - 2.22 (m, 2H), 2.11 - 1.90 (m, 4H).

**Example 25**

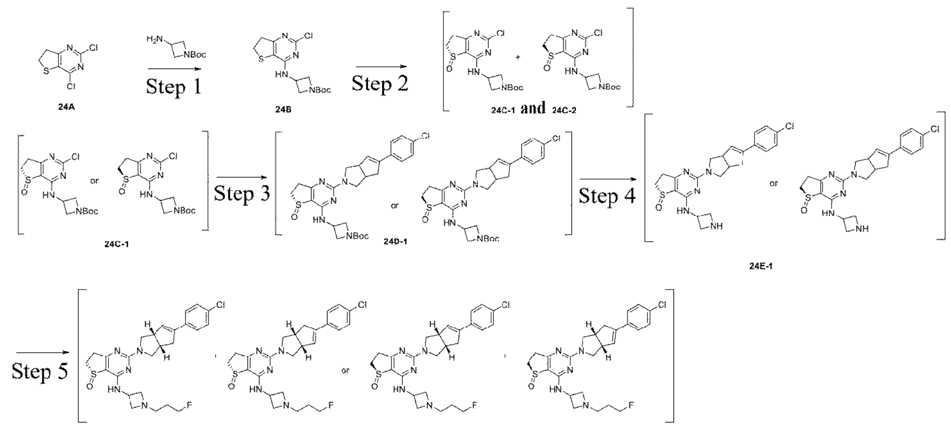


24-1 and 24-2

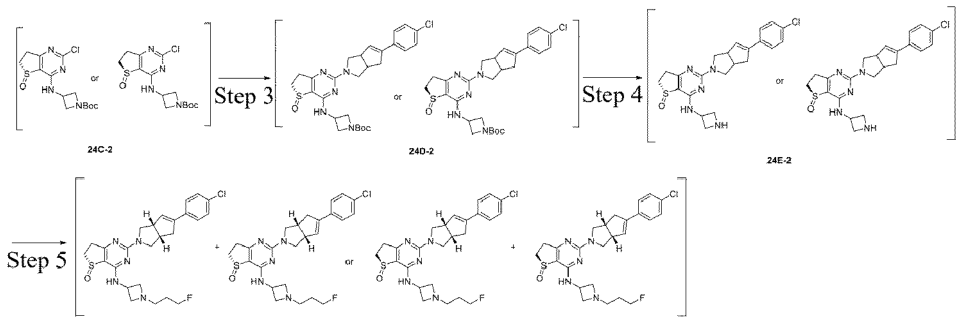


24-3 and 24-4

Step 1: **24A** (5g, 24.18 mmol), 1-tert-butoxycarbonyl-3-aminocyclobutylamine (5 g, 29.02 mmol), and N,N-diisopropylethylamine (9.38 g, 72.54 mmol) were added to acetonitrile (50 mL), heated to 60°C, and reacted for 16 h. After the reaction was complete, the system was cooled to room temperature, the reaction liquid was concentrated under reduced pressure to remove most acetonitrile, and water (50 ml) and ethyl acetate (50 ml) were then added. After liquid separation, the aqueous phase was extracted with ethyl acetate (50 ml × 2), the organic phases were combined, washed with a saturated sodium chloride solution, and dried over anhydrous sodium sulfate. After filtration, the filtrate was concentrated under reduced pressure and then separated and purified by silica gel column chromatography (eluents EA/PE = 0-60%) to obtain the title compound **24B** (7 g, 84.44%).

LC-MS (ESI): m/z = 343.1 $[M+H]^+$.

Step 2: **24B** (7 g, 20.42 mmol), tetraisopropyl titanate (0.58 g, 2.04 mmol), and water (0.37 g, 20.42 mmol) were successively added to dichloromethane (100 mL) and stirred for 10 min, tert-butyl hydroperoxide (7.89 g, 61.26 mmol, purity 70%) was then added, and the mixture was reacted at room temperature for 5 h. As monitored by TLC, the reaction was complete. Water (50 ml) was added to the reaction liquid. After liquid separation, the organic phase was washed with a sodium thiosulfate aqueous solution (50 ml), then washed with a saturated sodium chloride solution, and dried over anhydrous sodium sulfate. After filtration, the filtrate was concentrated under reduced pressure and then separated and purified by silica gel column chromatography (eluents EA/PE = 0-100%) to obtain compound **24C** (6 g, 82.6%).

**24C** (5 g) was taken and subjected to chiral resolution. Chiral preparation was carried out to obtain the title compound **24C-1** (2.2 g, 44%, retention time: 0.853 min) and the title compound **24C-2** (1.6 g, 32%, retention time: 1.070 min). Chiral preparation method: instrument: Waters 150 SFC; chromatographic column: Chiralpak AS Column; mobile phase: A: carbon dioxide, and B: methanol (0.1% aqueous ammonia); isocratic elution: 30% mobile phase B; flow rate: 120 mL/min; back pressure: 100 bar; column temperature: 25°C; wavelength: 220 nm; elution time: 2.5 min.

LC-MS (ESI): m/z = 359.1 $[M+H]^+$.

Step 3: **24C-1** (1 g, 2.79 mmol), **13C** (1.12 g, 3.35 mmol), and N,N-diisopropylethylamine (1.80 g, 13.92 mmol) were added to 1,4-dioxane (15 mL), heated to 100°C, and reacted for 4 h. After the reaction was complete as monitored by LC-MS, the system was cooled to room temperature, directly concentrated under reduced pressure, and then separated and purified by silica gel column chromatography (eluents MeOH/DCM = 0-10%) to obtain the title compound **24D-1** (1.2 g, 79.34%).

According to the above operation, **24D-2** (1.2 g, 79.34%) was obtained from **24C-2** (1 g, 2.79 mmol) as a raw material.

LC-MS (ESI): m/z = 542.2 $[M+H]^+$.

Step 4: **24D-1** (1.2 g, 2.21 mmol) was added to dichloromethane (15 mL), trifluoroacetic acid (4 ml) was then added, and the mixture was reacted at room temperature for 1 h. The reaction liquid was directly concentrated under reduced pressure to obtain crude **24E-1** (1.7 g).

According to the above operation, **24E-2** (1.7 g) was obtained from **24D-2** (1.2 g, 2.21 mmol) as a raw material.

LC-MS (ESI): m/z = 442.2 $[M+H]^+$.

Step 5: Crude product **24E-1** (1.7 g) and 3-fluoro-1-iodopropane (0.61 g, 3.25 mmol) were added to tetrahydrofuran (20 mL), a sodium hydroxide (0.52 g, 13 mmol) aqueous solution (5 mol/L) was then added, and the mixture was reacted at room temperature for 16 h. The reaction liquid was concentrated under reduced pressure to remove tetrahydrofuran, water (20 ml) was added, and the mixture was extracted with (MeOH/DCM = 1/10) (30 ml × 3). The organic phases were combined, washed with a saturated sodium chloride solution, and dried over anhydrous sodium sulfate. After filtration, the filtrate was concentrated under reduced pressure and then separated and purified by silica gel column chromatography (MeOH/DCM = 0-15%) to obtain a product (0.6 g, 55%).

Chiral preparation was carried out to obtain the title compound **24-1** (200 mg, 18.36%, retention time: 1.525 min) and the title compound **24-2** (230 mg, 21.11%, retention time: 2.022 min). Chiral preparation method: instrument: Waters 150 preparative SFC (SFC-26); chromatographic column: ChiralPak AD, 250 × 30 mm I.D., 10 μm; mobile phase: A: carbon dioxide, and B: isopropanol (0.1% aqueous ammonia); isocratic elution: 30% mobile phase B; flow rate: 150 mL/min; back pressure: 100 bar; column temperature: 38°C; wavelength: 220 nm; elution time: 15 min.

According to the above operation, using **24E-2** (1.7 g) as a raw material, a compound (0.7 g, 64%) was obtained, which was subjected to chiral separation to obtain **24-3** and **24-4.**

Chiral preparation was carried out to obtain the title compound **24-3** (310 mg, 28.45%, retention time: 0.698 min) and the title compound **24-4** (280 mg, 25.7%, retention time: 1.514 min). Chiral preparation method: instrument: Waters 150 preparative SFC (SFC-26); chromatographic column: ChiralPak AD, 250 × 30 mm I.D., 10 μm; mobile phase: A: carbon dioxide, and B: ethanol (0.1% aqueous ammonia); isocratic elution: 65% mobile phase B; flow rate: 100 mL/min; back pressure: 100 bar; column temperature: 38°C; wavelength: 220 nm; elution time: 5 min.

**(Compound 24-1)**

$^1$H NMR (400 MHz, DMSO-d6) δ 8.01 (s, 1H), 7.51-7.47 (m, 2H), 7.41-7.37(m, 2H), 6.26 (s, 1H), 4.57-4.50(m, 2H), 4.42-4.38 (m, 1H), 3.88 (dd, 1H), 3.80-3.72 (m, 1H), 3.69 - 3.52 (m, 4H), 3.46-3.38 (m, 1H), 3.25 - 3.15 (m, 2H), 3.12-3.06 (m, 1H), 3.02-2.82(m, 5H), 2.69-2.62 (m, 1H), 2.56-2.50 (m, 2H), 1.72-1.60 (m, 2H).

LC-MS (ESI): m/z = 502.2 $[M+H]^+$.

**(Compound 24-2)**

$^1$H NMR (400 MHz, DMSO-d6) δ 8.00 (s, 1H), 7.51-7.47 (m, 2H), 7.41-7.37(m, 2H), 6.26 (s, 1H), 4.56-4.49 (m, 2H), 4.42-4.37 (m, 1H), 3.88 (dd, 1H), 3.80-3.71 (m, 1H), 3.68-3.52 (m, 4H), 3.47 - 3.35 (m, 1H), 3.24-3.14 (m, 2H), 3.12-3.04 (m, 1H), 3.00 - 2.81 (m, 5H), 2.71-2.59 (m, 1H), 2.49-2.43 (m, 2H), 1.72-1.58 (m, 2H).

LC-MS (ESI): m/z = 502.2 $[M+H]^+$.

**(Compound 24-3)**

$^1$H NMR (400 MHz, $CDCl_3$) δ 7.38-7.26 (m, 4H), 6.58-6.52 (m, 1H), 6.05 (s, 1H), 4.77-4.68 (m, 1H), 4.55-4.47(m, 1H), 4.43-4.36 (m, 1H), 4.03-3.93 (m, 1H), 3.83-3.69 (m, 3H), 3.68 - 3.52 (m, 3H), 3.38-3.21(m, 2H), 3.19-3.08 (m, 1H), 3.08 - 2.94 (m, 3H), 2.93-2.87 (m, 1H), 2.82-2.69 (m, 1H), 2.66-2.57 (m, 1H), 2.56-2.40 (m, 2H), 1.78-1.62 (m, 2H).

LC-MS (ESI): m/z = 502.2 $[M+H]^+$.

**(Compound 24-4)**

$^1$H NMR (400 MHz, $CDCl_3$) δ 7.38-7.26 (m, 4H), 6.65-6.32(m, 1H), 6.05 (s, 1H), 4.83-4.70 (m, 1H), 4.55-4.46(m, 1H), 4.43-4.34 (m, 1H), 4.03-3.92 (m, 1H), 3.87-3.66 (m,4H), 3.66 - 3.52 (m, 2H), 3.39-3.23(m, 2H), 3.18-3.08 (m, 1H), 3.07 - 2.74 (m, 5H), 2.68-2.59 (m, 1H), 2.58-2.44 (m, 2H), 1.84-1.71 (m, 2H).

LC-MS (ESI): m/z = 502.2 $[M+H]^+$.

## Example 26

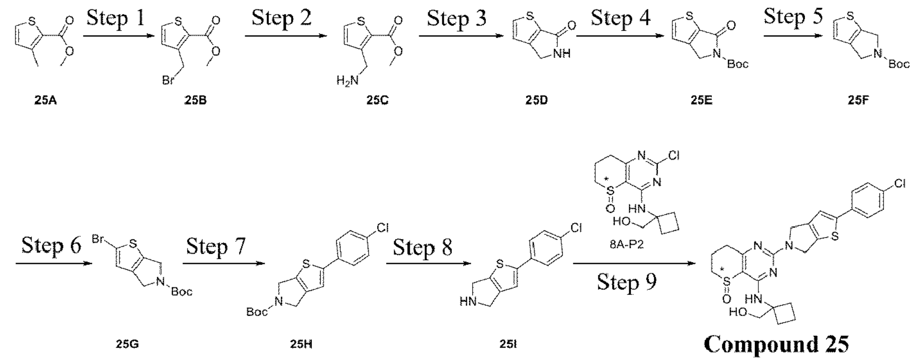


Step 1: Compound **25A** (15 g, 96.03 mmol) was dissolved in carbon tetrachloride (600 mL), and N-bromosuccinimide (17.09 g, 96.03 mmol) and azodiisobutyronitrile (1.58 g, 9.60 mmol) were added thereto. Under nitrogen protection, the mixture was heated to 70°C and reacted under stirring overnight, and the system was cooled to room temperature, washed with water and a saturated sodium chloride aqueous solution, and dried over anhydrous sodium sulfate. Then, the filtrate was spin-dried to obtain compound **25B** (24 g, crude), which was directly used in the next reaction without separation and purification.

LCMS (ESI): = 235.1, 237.1 $[M+H]^+$.

Step 2: Crude **25B** (17 g, 63.8 mmol) was dissolved in an ammonia methanol solution (7.0 M, 20 mL) and methanol (20 mL) and stirred at room temperature for 2 hours. The solvent was evaporated under reduced pressure, and the remaining material was then separated by a silica gel chromatographic column (DCM:MeOH, v/v = 10:1) to obtain compound **25C** (8 g, two-step yield 48.7%).

LCMS (ESI): = 172.1 $[M+H]^+$.

Step 3: Compound **25C** (8 g, 46.73 mmol) was dissolved in a mixed solvent of methanol and ethanol (120 mL, v/v = 1:1), potassium carbonate (12.92 g, 93.45 mmol) was added thereto, and the mixture was stirred, heated to 90°C, and reacted for 3 hours. The system was cooled to room temperature, the solvent was evaporated under reduced pressure, and 150 ml of ethyl acetate was then added for dissolution. Then, a solid was filtered off, and the solvent was spin-dried to obtain compound **25D** (3.25 g, 36%).

LCMS (ESI): = 140.0 $[M+H]^+$.

Step 4: **25D** (3.25 g, 23.35 mmol) was dissolved in dichloromethane (50 mL), triethylamine (7.09 g, 70.06 mmol) and a catalytic amount of 4-dimethylaminopyridine (285 mg, 2.34 mmol) were added thereto, and the mixture was cooled in an ice bath. Di-tert-butyl dicarbonate (10.2 g, 46.71 mmol) was dropwise added, and the mixture was stirred at room temperature for 3 hours, directly spin-dried, and separated by a silica gel chromatographic column (EA:PE = 1:3) to obtain compound **25E** (4.45 g, 79.6%).

LCMS (ESI): = 184.1 $[M+H]^+$.

Step 5: **25E** (4.45 g, 18.60 mmol) was dissolved in tetrahydrofuran (50 mL). Under nitrogen protection and cooling with ice water, borane tetrahydrofuran complex (55.8 mL, 1.0 mol/L) was dropwise added thereto. After the dropwise addition was complete, the mixture was reacted at room temperature for 3 hours. After cooling with ice water, the reaction was quenched with a small amount of methanol, and the solvent was evaporated under reduced pressure to obtain a crude product, which was separated by a silica gel chromatographic column (EA:PE = 1:3) to obtain **compound 25F** (2.26 g, 53.9%).

LCMS (ESI): = 170.1 $[M+H]^+$.

Step 6: **25F** (2.26 g, 10.03 mmol) was dissolved in chloroform (25 mL), and a catalytic amount of glacial acetic acid was added. Liquid bromine (1.6 g, 10.03 mmol) was added dropwise under cooling with ice water, the mixture was stirred at room temperature overnight, and triethylamine (3.04 g, 30.09 mmol) and a catalytic amount of DMAP (123 mg, 1.00 mmol) were added thereto. The mixture was cooled in an ice bath, di-tert-butyl dicarbonate (4.38 g, 20.06 mmol) was dropwise added, and the mixture was stirred at room temperature for 3 hours, directly spin-dried, and separated by a silica gel chromatographic column (EA:PE = 1:3) to obtain **compound 25G** (2.26 g, 74.1%).

LCMS (ESI): = 248.1, 250.1 $[M+H]^+$.

Step 7: **25G** (1 g, 3.29 mmol), 4-chlorophenylboronic acid (616.8 mg, 3.94 mmol), sodium carbonate (697 mg, 6.57 mmol), and tetrakis(triphenylphosphine)palladium (380 mg, 0.33 mmol) were added to a mixed solution of 1,4-dioxane and water (27.5 mL, v/v = 10:1). After nitrogen displacement, the mixture was reacted at 80°C for 3 hours. The reaction was quenched by adding water, the system was extracted with ethyl acetate, washed with a saturated sodium chloride aqueous solution, dried over anhydrous sodium sulfate, and separated by a silica gel chromatographic column to obtain **compound 25H** (640 mg, 58.0%).

LCMS (ESI): = 280.1, 282.1 $[M+H]^+$.

Step 8: **25H** (300 mg) was added to hydrogen chloride dioxane (5 mL, 4N), and the mixture was reacted at room temperature for 2 hours. Then, the system was spin-dried to obtain compound **25I** (300 mg, crude), which was directly used in the next reaction.

LCMS (ESI): = 236.1, 238.1 $[M+H]^+$.

Step 9: **8A-P2** (150 mg, 0.50 mmol), **251** (200 mg, crude), and DIPEA (321 mg, 2.49 mmol) were added to 1,4-dioxane (5 mL), and the mixture was reacted at 80°C for 3 hours. The solvent was spin-dried, and the remaining material was subjected to HPLC preparation. The prepared liquid was adjusted to pH 7-8 by adding sodium bicarbonate, extracted with dichloromethane, and then concentrated to obtain **compound 25** (65 mg, 26.1%).

Preparative HPLC separation method: 1. instrument: waters 2767 preparative liquid chromatographic instrument; chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: with a content of 5-50%; c. flow: 12 mL/min; d. elution time: 20 min.

LCMS (ESI): = 502.1 $[M+H]^+$.

$^1$H NMR (400 MHz, Chloroform-d) δ 7.52 - 7.45 (m, 2H), 7.37 - 7.32 (m, 2H), 7.07 (s, 1H), 6.27 (s, 1H), 5.18 (s, 1H), 4.95 - 4.61 (m, 4H), 4.00 - 3.87 (m, 2H), 3.28 - 3.17 (m, 1H), 3.02 - 2.83 (m, 2H), 2.72 - 2.49 (m, 2H), 2.47 - 2.31 (m, 2H), 2.30 - 2.16 (m, 2H), 2.16 - 2.06 (m, 1H), 2.05 - 1.88 (m, 2H).

**Example 27**

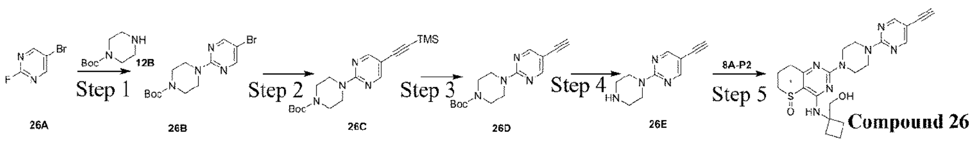


Step 1: Compound **26A** (3.00 g, 16.92 mmol) and compound **12B** (3.78 g, 20.30 mmol) were weighed into a 100 mL single-mouth flask and then dissolved by adding 1,4-dioxane (30 mL), and N,N-diisopropylethylamine (6.56 g, 50.76 mmol) was added to the reaction liquid. After the addition was complete, the mixture was stirred at 80 degrees Celsius for 16 h. After the reaction was complete as determined by a TLC spotting plate (petroleum ether:ethyl acetate = 5:1), water (20 mL) was added to the reaction liquid and stirred for 5 min. The liquid was extracted by adding ethyl acetate (20 mL), and the organic phase was separated. The organic phase was dried over anhydrous sodium sulfate, and the crude product was concentrated under reduced pressure and purified by column chromatography (eluents: petroleum ether:ethyl acetate = 5:1) to obtain the target compound **26B** (2.8 g, yield: 48%).

LCMS m/z = 343.1 $[M+1]^+$

Using compound **26B** and trimethylsilylacetylene as raw materials, compound **26** (80 mg, 27%) was obtained according to the operation of Example 21.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 35-80%; c. flow: 15 mL/min; d. elution time: 20 min; retention time: 12.50 min.

LCMS m/z = 454.2 $[M+1]^+$

$^1$H NMR (400 MHZ, DMSO-d6) δ 8.50 (s, 2H), 6.56 (s, 1H), 4.89 (t, 1H), 4.28 (s, 1H), 3.81 (s, 8H), 3.70 (d, 2H), 3.03-3.11 (m, 1H), 2.88-2.95 (m, 1H), 2.53-2.70 (m, 2H), 2.31-2.45 (m, 3H), 2.15 (s, 2H), 1.91-2.00 (m, 1H), 1.73-1.87 (m, 2H).

**Example 28**

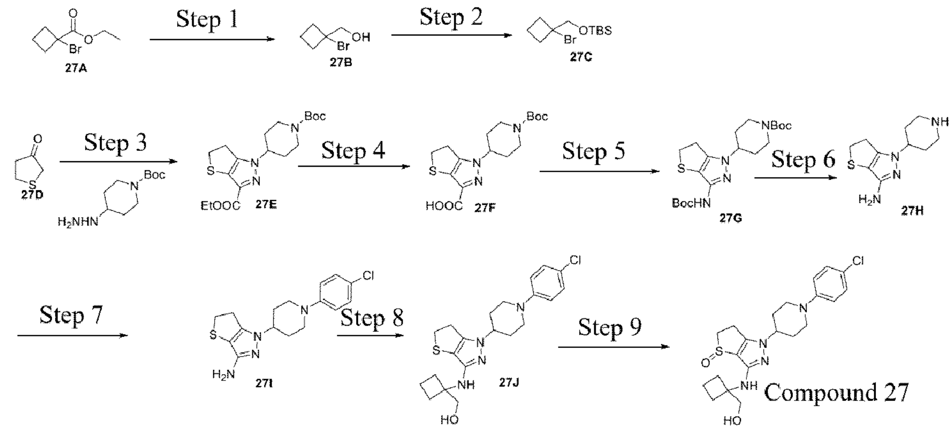

27A 27B 27C 27D 27E 27F 27G 27H 27I 27J Compound 27

Step 1: The raw material **27A** (3 g, 14.49 mmol) was added to 60 mL of a dry tetrahydrofuran solvent, and the reagent lithium tetrahydroaluminate (270 mg, 7.25 mmol) was added in an ice bath. After stirring in the ice bath for half an hour, the mixture was heated to room temperature and stirred for 3 hours. After quenching by adding water, a small amount of dilute hydrochloric acid was added, and the system was then was extracted three times with ethyl acetate, washed with a saturated sodium bicarbonate solution, and dried over anhydrous sodium sulfate. After filtration, the filtrate was concentrated and then directly used for the next reaction.

Step 2: The raw material **27B** (2 g, 12.12 mmol) was dissolved in the solvent dichloromethane (50 mL), and the reagents triethylamine (2.45 g, 24.24 mmol), imidazole (1.65 g, 24.24 mmol), and tert-butyldimethylsilyl chloride (2.74 g, 18.18 mmol) were then added. The mixture was stirred at room temperature overnight, diluted by adding water, then extracted with dichloromethane, washed with a saturated sodium bicarbonate solution, and dried over anhydrous sodium sulfate. After filtration, the filtrate was concentrated and then purified and separated by silica gel column chromatography (PE:EA (v/v) = 1:0-20:1) to obtain **27C** (1.8 g, yield 53%).

$^1$H NMR (400 MHz, $CDCl_3$) δ3.68 (s, 2H), 2.45-2.50 (m, 4H), 2.01-2.06 (m, 1H), 1.72-1.79 (m, 1H), 0.83 (s, 9H), 0.0 (s, 6H).

Step 3: The raw material 3-thienone (8 g, 78.28 mmol) and diethyl oxalate (11.44 g, 78.28 mmol) were added to an anhydrous ethanol solvent (70 mL), and the reagent sodium tert-butoxide (7.9 g, 82.21 mmol) was added in an ice bath and further stirred for 2 hours. Glacial acetic acid (5.17 g, 86.11 mmol) was then added, followed by tert-butyl 4-hydrazinopiperidine-1-carboxylate hydrochloride (16.85 g, 78.28 mmol), and the mixture was further stirred for 20 hours. After quenching by adding water, the system was extracted with EA, washed with a saturated sodium bicarbonate solution, and dried over anhydrous sodium sulfate. After filtration, the filtrate was concentrated and then purified and separated by silica gel column chromatography (PE:EA (v/v) = 1:0-10:1) to obtain **27E** (8 g, yield 27%).

LCMS m/z = 382.2 [M +1]$^+$

Step 4: The raw material **27E** (6 g, 15.73 mmol) was added to a mixed solvent of tetrahydrofuran (30 mL) and methanol (30 mL), and the reagent lithium hydroxide (0.76 g, 31.65 mmol) dissolved in 15 mL of a water solvent was then added and stirred at room temperature for 2 hours. After the raw material was completely reacted as monitored by TLC, the system was concentrated, then diluted by adding water, adjusted to pH 2-3 with 2M hydrochloric acid, extracted with ethyl acetate, and dried over anhydrous sodium sulfate. After filtration, the filtrate was concentrated and directly used for the next reaction.

LCMS m/z = 354.1 [M +1]$^+$

Step 5: The raw material **27F** (4 g, 11.32 mmol) and diphenyl phosphorazidate (5.67 g, 14.72 mmol) were added to tert-butyl alcohol (100 mL), followed by triethylamine (1.72 g, 16.98 mmol). Under nitrogen protection, the mixture was heated to 85°C, stirred for 4 hours, cooled, concentrated, then diluted by adding water, and extracted with ethyl acetate. After drying over anhydrous sodium sulfate and filtration, the filtrate was concentrated and then purified and separated by silica gel column chromatography (PE:EA (v/v) = 1:0-4: 1) to obtain the target compound **27G** (4 g, yield 83%).

LCMS m/z = 425.2 [M +1]$^+$

$^1$H NMR (400 MHz, $CDCl_3$) δ5.99 (s, 1H), 4.24-4.26 (m, 2H), 4.04-4.06 (m, 1H), 3.94-3.95(m, 2H), 3.82-3.83 (m, 2H), 2.77-2.81 (m, 2H), 2.07-2.08 (m, 1H), 2.01-2.02 (m, 1H), 1.86-1.89 (m, 2H), 1.54 (s, 9H), 1.48 (s, 9H).

Step 6: The raw material **27G** (4 g, 9.42 mmol) was added to anhydrous methanol (5 mL), followed by 20 mL of a hydrogen chloride 1,4-dioxane solution (4M), and the mixture was stirred at room temperature for 4 hours. After the raw material was completely reacted as monitored by LCMS, the system was concentrated to dryness and used for the next reaction.

LCMS m/z = 225.1 [M +1]$^+$

Step 7: The raw material **27H** (2.2 g, 9.81 mmol), 4-chloroiodobenzene (2.34 g, 9.81 mmol), tris(dibenzylideneacetone)palladium (0.45 g, 0.49 mmol), 4,5-bis(diphenylphosphino)-9,9-dimethylxanthene (0.57 g, 0.98 mmol), and sodium tert-butoxide (1.89 g, 19.62 mmol) were separately added to 1,4-dioxane (100 mL). Under nitrogen protection, the mixture was heated to 85°C, stirred for 16 hours, cooled, and filtered, and the filtrate was diluted by adding water, extracted with ethyl acetate, and dried over anhydrous sodium sulfate. After filtration, the filtrate was concentrated and then purified and separated by silica gel column chromatography (PE:EA (v/v) = 1:0-4: 1) to obtain the target compound **27I** (0.8 g, yield 24%).

LCMS m/z = 335.1 [M +1]$^+$

Step 8: The raw material **27I** (0.7 g, 2.09 mmol) was dissolved in N,N-dimethylacetamide (20 mL), and sodium hydride (0.42 g, 10.45 mmol, 40% in oil) was added in an ice bath and stirred in the ice bath for 1 hour. The reagent **27C** (1.17 g, 4.18 mmol) was then added and stirred in the ice bath for half an hour, and the mixture was then heated to room temperature and stirred for 3 hours. After the raw material was completely reacted as monitored by LCMS, the reaction was quenched by adding water, and the system was concentrated under reduced pressure by an oil pump to remove the solvent and then separated by silica gel column chromatography (DCM:MeOH (v/v) = 1:0-5:1) to obtain the target compound **27J** (0.8 g, yield 91%).

LCMS m/z = 419.2 [M +1]$^+$

Step 9: The raw material **27J** (200 mg, 0.48 mmol), 1,1'-bi-2-naphthol (69 mg, 0.24 mmol), water (9 mg, 0.48 mmol), and tetraisopropyl titanate(68 mg, 0.24 mmol) were successively added to the solvent dichloromethane (10 mL) and stirred at room temperature for half an hour. Tert-butyl hydroperoxide (65 mg, 0.72 mmol) was then added, and the mixture was further stirred for 3 hours, then diluted by adding water, and then extracted with dichloromethane. After washing with saturated sodium bicarbonate followed by drying and filtration, the filtrate was concentrated and then purified and separated by TLC (DCM:MeOH = 5:1) to obtain the title **compound 27** (0.01 g, yield 5%).

LCMS m/z = 435.2 [M +1]$^+$

$^1H$ NMR (400 MHz, $CD_3OD$-$d_4$) δ 7.14-7.17 (m, 2H), 6.76-6.80 (m, 2H), 4.29-4.39 (m, 2H), 4.15-4.18 (m, 1H), 3.65-3.78 (m, 4H), 3.12-3.15 (m, 2H), 2.52-2.61 (m, 2H), 1.91-2.03 (m, 6H), 1.74-1.81 (m, 3H), 1.60-1.66 (m, 1H).

**Example 29**

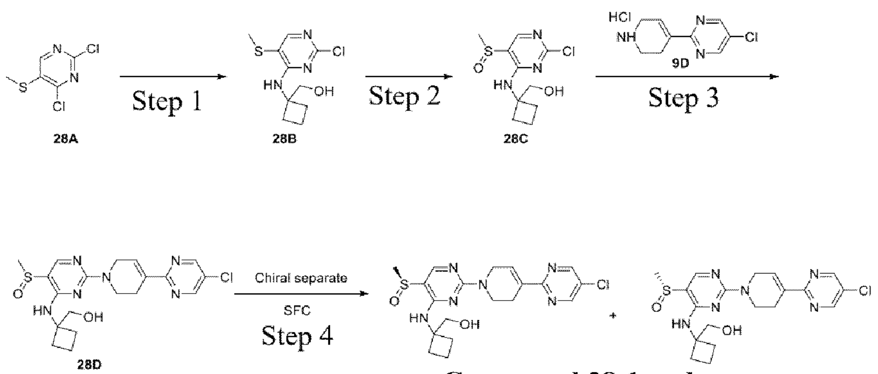

28A
28B
28C
9D
28D

**Compound 28-1 and compound 28-2**

Step 1: Compound **28A** (3.9 g, 20.0 mmol) and 1-aminocyclobutylmethanol hydrochloride (3.5 g, 25.0 mmol) were dissolved in acetonitrile (50.0 mL), triethylamine (6.1 g, 60.0 mmol) was then added, and the mixture was heated to 70°C, and reacted for 14 h. The system was then concentrated and subjected to column chromatography (dichloromethane/methanol = 10/1) to obtain compound **28B** (5.2 g, 98%).

LC-MS (ESI): m/z = 260.0 [M+H]$^+$

Step 2: Compound **28B** (2.0 g, 7.7 mmol) was dissolved in dichloromethane (40 mL), m-chloroperoxybenzoic acid (1.5 g, 8.5 mmol) was added in an ice bath, and the mixture was slowly heated to room temperature and reacted overnight. The system was concentrated to obtain compound **28C** (1.7 g, 80%), which was directly used for the next step.

LC-MS (ESI): m/z = 276.0 [M+H]$^+$

Step 3: Compound **28C** (0.7 g, 2.4 mmol) was dissolved in 1,4-dioxane (40.0 mL), compound **9D** (461.0 mg, 2.4 mmol) and diisopropylethylamine (1.3 g, 9.4 mmol) were then added, and the mixture was heated back to 90°C and reacted for 12 h. The system was then cooled to room temperature, concentrated, and subjected to column chromatography (dichloromethane/methanol = 9/1) to obtain compound **28D** (380.0 mg, 37%).

LC-MS (ESI): m/z = 435.1 [M+H]$^+$

Step 4: Compound **28D** (380 mg) was subjected to chiral resolution to obtain compound **28-1** (100 mg) and compound **28-2** (101 mg).

Preparation method: instrument: MG II preparative SFC (SFC-14), column: ChiralPak AD, 250 × 30 mm I.D., 5 μm, mobile phase: (A for $CO_2$ and B for MeOH (0.1% $NH_3$•$H_2O$); gradient: 50% phase B elution; flow rate: 100 mL/min, back pressure: 100 bar, column temperature: 25°C; wavelength: 220 nm; cycle time: 3.3 min; sample preparation: sample concentration 10 mg/mL, acetonitrile solution injection: 3.5 mL each time. After separation, the fraction was dried on a rotary evaporator at a bath temperature of 35°C to obtain P1 (retention time: 1.334 minutes, assigned as compound **28-1)** and P2 (retention time: 1.840 minutes, assigned as compound **28-2).**

LC-MS (ESI): m/z = 435.6 $[M+H]^+$

$^1H$ NMR (400 MHz, DMSO-d6) δ 8.90 (s, 2H), 7.96 (s, 1H), 7.37 (s, 1H), 7.29 (s, 1H), 4.92 - 4.89 (m, 1H), 4.43 (s, 2H), 3.95 (s, 2H), 3.83 - 3.79 (m, 1H), 3.74 - 3.70 (m, 1H), 2.85 (s, 3H), 2.65 (s, 2H), 2.37 - 2.11 (m, 4H), 1.96 - 1.69 (m, 2H).

LC-MS (ESI): m/z = 435.6 $[M+H]^+$

$^1H$ NMR (400 MHz, DMSO-d6) δ 8.90 (s, 2H), 7.96 (s, 1H), 7.37 (s, 1H), 7.29 (s, 1H), 4.92 - 4.89 (m, 1H), 4.43 (s, 2H), 3.95 (s, 2H), 3.83 - 3.79 (m, 1H), 3.74 - 3.70 (m, 1H), 2.85 (s, 3H), 2.65 (s, 2H), 2.34 - 2.17 (m, 4H), 1.87 - 1.80 (m, 2H).

**Example 30**

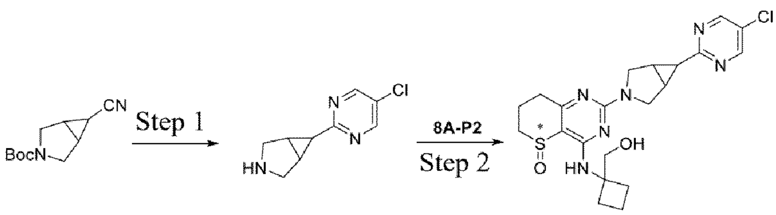


29A 29B Compound 29

Step 1: In an ice bath, under nitrogen protection, methanol (1 mL, 23.00 mmol) was slowly dropwise added to a system containing **29A** (1.50 g, 7.70 mmol, see patent WO 2010078348 A1 for the synthesis) and a 4M hydrogen chloride dioxane solution (6 mL, 23.00 mmol). After the addition was complete, the reaction liquid was stirred at room temperature for 6 h. After the reaction liquid was cooled to 5°C, sodium methoxide (830 mg, 15.4 mmol) and a 7N ammonia methanol solution (1.6 mL) were added and stirred at room temperature for 16 h. At room temperature, a solution of sodium methoxide in methanol (1.10 g dissolved in 4.5 mL of methanol) was added to the reaction liquid and stirred for 30 min. (2-Chloro-3-dimethylamino-prop-2-enylidene)-dimethyl-ammonium hexafluorophosphate (2.00 g) was then added and stirred at room temperature for 3 h. The reaction liquid was concentrated under reduced pressure to a small volume, 2-methyltetrahydrofuran (100 mL) was then added and washed twice with water (80 mL). The organic phases were dried over anhydrous sodium sulfate, and then concentrated in vacuum to obtain a crude product, and the crude product was purified three times by column chromatography (eluents: DCM:MeOH = 100:1 to 10:1) to obtain the target compound **29B** (120 mg, yield: 8.52%).

LC-MS (ESI): m/z = 196.2 $[M+H]^+$.

Step 2: Compound **8A-P2** (100 mg, 0.33 mmol) and compound **29B** (100 mg, 0.51 mmol) were dissolved in 1,4-dioxane (6 mL), DIPEA (130 mg, 1.00 mmol) was then added, and the mixture was heated to 85°C and stirred for 16 h under nitrogen protection. After the reaction was complete, the reaction liquid was concentrated under reduced pressure to obtain a crude product, which was purified by column chromatography (eluents: dichloromethane:methanol = 100:1 to 10:1) and then further purified by prep.HPLC preparation (preparation method: instrument: waters 2767 preparative liquid chromatographic instrument; chromatographic column: SunFire@ Prep C18 (19 mm × 250 mm); the sample was dissolved in N,N-dimethylformamide and filtered with a 0.45 μm filter to prepare a sample solution; preparative chromatography conditions: a. composition of mobile phases A and B: mobile phase A: acetonitrile, and mobile phase B: water (containing 5 mM aqueous ammonia); b. gradient elution, mobile phase A with a content of 20-80%; c. flow: 15 mL/min; d. elution time: 15 min; retention time: 7.0 min) to obtain the target compound **29** (3.5 mg, 2.29%).

LCMS m/z = 462.1 $[M+H]^+$

$^1H$ NMR (400 MHz, $CDCl_3$) δ 8.61 (s, 2H), 5.86 (s, 1H), 4.88 (s, 1H), 4.11 - 4.04 (m, 1H), 3.97 - 3.91 (m, 1H), 3.86 (s, 2H), 3.68 - 3.59 (m, 3H), 3.45 - 3.38 (m, 1H), 3.07 - 2.98 (m, 2H), 2.64 - 2.49 (m, 2H), 2.37 - 2.25 (m, 4H), 1.99 - 1.85 (m, 4H), 1.69 - 1.62 (m, 1H).

**Example 31**

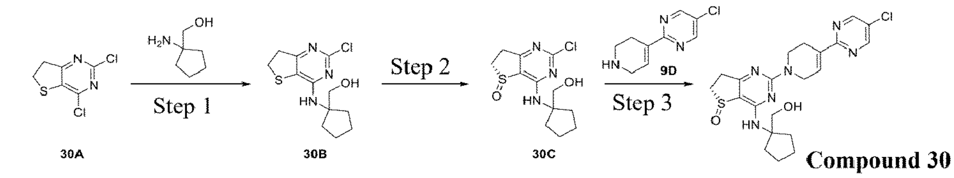


30A 30B 30C Compound 30

Step 1: Compound **30A** (1.0 g, 4.83 mmol) was dissolved in acetonitrile (10 mL), (1-aminocyclopentyl)methanol (610.0 mg, 5.31 mmol) and triethylamine (1.47 g, 14.49 mmol) were then added, and the mixture was heated to 70°C and reacted for 14 h. The system was then concentrated and subjected to column chromatography (petroleum ether/ethyl acetate = 1/1) to obtain compound **30B** (500 mg, 36%).
LC-MS (ESI): m/z = 286.1 $[M+1]^+$
Step 2: Compound **30B** (600.0 mg, 1.75 mmol), (S)-(-)-1,1'-bi-2-naphthol (50.0 mg, 0.18 mmol), titanium tetraisopropoxide (25.0 mg, 0.088 mmol), and water (32.0 mg, 1.75 mmol) were dissolved in dichloromethane (10 mL). After nitrogen displacement three times, the system was stirred at room temperature for one hour, and tert-butyl hydroperoxide (170.0 mg, 1.93 mmol) was added and stirred for 1.5 hours. After quenching with a saturated sodium thiosulfate aqueous solution, the aqueous phase was extracted with dichloromethane, dried, concentrated, and subjected to column chromatography (dichloromethane/methanol = 10/1) to obtain compound **30C** (430.0 mg, 81%).
LC-MS (ESI): m/z = 302.1 $[M+H]^+$.
Step 3: Compound **30C** (430.0 mg, 1.42 mmol) was dissolved in 1,4-dioxane (10.0 mL), compound **9D** (280.0 mg, 1.42 mmol) and triethylamine (550 mg, 4.26 mmol) were then added, and the mixture was heated to 90°C and reacted for 12 h. The system was then cooled to room temperature, concentrated, and subjected to column chromatography (dichloromethane/methanol = 10/1) to obtain compound **30** (290.0 mg, 44%).
LC-MS (ESI): m/z = 461.1 $[M+H]^+$.
$^1H$ NMR (400 MHz, DMSO-$d_6$) δ 8.90 (s, 2H), 7.35 - 7.21 (m, 1H), 6.58 (s, 1H), 4.91 (t, 1H), 4.48 (s, 2H), 3.99 (t, 2H), 3.74 - 3.60 (m, 2H), 3.48 - 3.36 (m, 1H), 3.32 - 3.22 (m, 1H), 3.00 - 2.91 (m, 1H), 2.90 - 2.82 (m, 1H), 2.66 (s, 2H), 2.20 - 2.05 (m, 2H), 1.88 - 1.78 (m, 2H), 1.74 - 1.63 (m, 2H), 1.62 - 1.52 (m, 2H).

**Example 32**

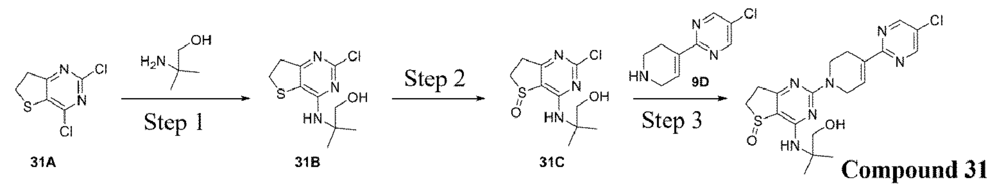


31A 31B 31C Compound 31

Step 1: Compound **31A** (2.0 g, 9.64 mmol) was dissolved in acetonitrile (20 mL), 2-amino-2-methylpropan-1-ol (950.0 mg, 10.64 mmol) and triethylamine (2.93 g, 28.92 mmol) were then added, and the mixture was heated to 70°C and reacted for 14 h. The system was then concentrated and subjected to column chromatography (petroleum ether/ethyl acetate = 1/1) to obtain compound **31B** (600 mg, 24%).
LC-MS (ESI): m/z = 260.1 $[M+1]^+$. Step 2: Compound **31B** (600.0 mg, 2.31 mmol) was dissolved in dichloromethane (10 mL), and m-chloroperoxybenzoic acid (600.0 mg, 3.49 mmol) was added and stirred for 16 hours. After quenching with a saturated sodium thiosulfate aqueous solution, the aqueous phase was extracted with dichloromethane, dried, concentrated, and subjected to column chromatography (dichloromethane/methanol = 10/1) to obtain compound **31C** (632.0 mg, 99%).
LC-MS (ESI): m/z = 276.1 $[M+H]^+$.
Step 3: Compound **31C** (632.0 mg, 2.29 mmol) was dissolved in 1,4-dioxane (10.0 mL), compound **9D** (540.0 mg, 2.75 mmol) and N,N-diisopropylethylamine (890 mg, 6.87 mmol) were then added, and the mixture was heated to 90°C and reacted for 12 h. The system was then cooled to room temperature, concentrated, and subjected to column chromatography (dichloromethane/methanol = 10/1) to obtain compound **31** (440.0 mg, 44%).
LC-MS (ESI): m/z = 435.1 $[M+H]^+$

$^1$H NMR (400 MHz, DMSO-$d_6$) δ 8.90 (s, 2H), 7.38 - 7.16 (m, 1H), 6.23 (s, 1H), 5.15 (t, 1H), 4.49 (s, 2H), 4.00 (t, 2H), 3.51 (d, 2H), 3.48 - 3.33 (m, 2H), 3.01 - 2.91 (m, 1H), 2.91 - 2.82 (m, 1H), 2.67 (s, 2H), 1.43 (s, 6H).

**Example 33**

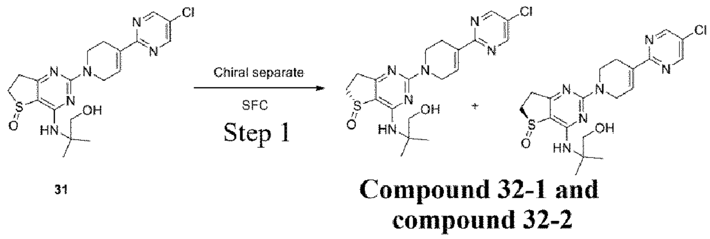


31

**Compound 32-1 and compound 32-2**

Step 1: Compound **31** (380 mg) was subjected to chiral resolution to obtain compound **32-1** (165 mg) and compound **32-2** (180 mg).

Preparation method: instrument: Waters 150 SFC, column: Chiralpak AS, mobile phase: (A for $CO_2$ and B for MeOH (0.1% $NH_3$•$H_2O$); gradient: 40% phase B elution; flow rate: 120 mL/min, back pressure: 100 bar, column temperature: 25°C; wavelength: 220 nm; cycle time: 2.8 min; sample preparation: sample concentration 5 mg/mL, acetonitrile solution injection: 1.0 mL each time. After separation, the fraction was dried on a rotary evaporator at a bath temperature of 35°C to obtain P1 (retention time: 1.730 minutes, assigned as compound **32-1)** and P2 (retention time: 1.948 minutes, assigned as compound **32-2).**

LC-MS (ESI): m/z = 435.1 [M+H]$^+$

$^1$H NMR (400 MHz, DMSO-$d_6$) δ 8.89 (s, 2H), 7.34 - 7.26 (m, 1H), 6.21 (s, 1H), 5.13 (t, 1H), 4.49 (d, 2H), 4.00 (t, 2H), 3.51 (d, 2H), 3.46 - 3.33 (m, 2H), 3.01 - 2.81 (m, 2H), 2.68 (d, 2H), 1.43 (s, 6H).

LC-MS (ESI): m/z = 435.1 [M+H]$^+$

$^1$H NMR (400 MHz, DMSO-$d_6$) δ 8.89 (s, 2H), 7.34-7.26 (m, 1H), 6.21 (s, 1H), 5.13 (t, 1H), 4.49 (d, 2H), 4.00 (t, 2H), 3.51 (d, 2H), 3.47 - 3.32 (m, 2H), 3.02 - 2.81 (m, 2H), 2.67 (d, 2H), 1.43 (s, 6H).

**Example 34**

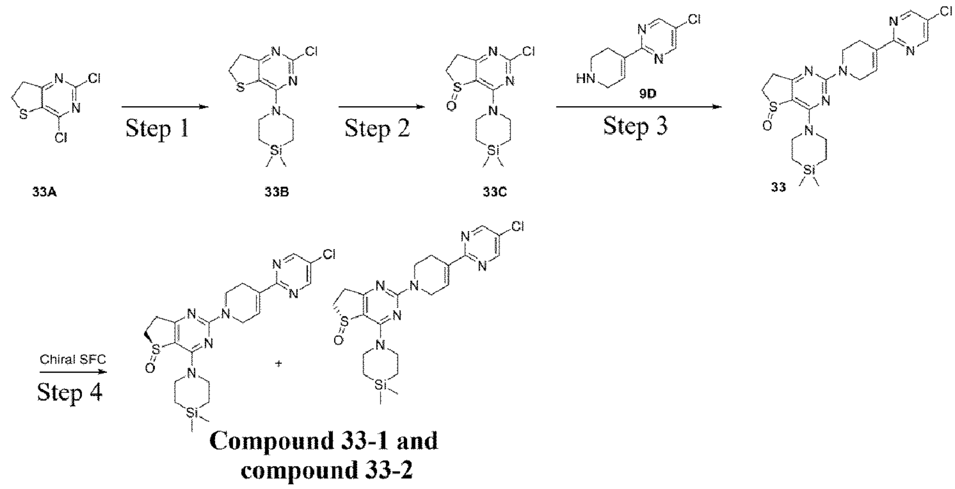


33A 33B 33C 33

**Compound 33-1 and compound 33-2**

Using compound **33A** and 4,4-dimethyl-[1,4]silapiperidine hydrochloride as raw materials, compound **33** (0.6 g, 45%) was obtained according to the operation of Example 31.

LC-MS (ESI): m/z = 474.1 [M+H]$^+$.

Compound **33** (600 mg) was subjected to chiral resolution to obtain compound **33-1** (200 mg) and compound **33-2** (202 mg).

Preparation method: instrument: Waters 150 SFC, column: Chiralcel column (250 × 30 mm × 10 um), mobile phase: (A for $CO_2$; B for 0.1% $NH_3$•$H_2O$ in MEOH); gradient: 35% phase B elution; flow rate: 100 mL/min, back pressure:

100 bar, column temperature: 25°C; wavelength: 220 nm; cycle time: 3.0 min; sample preparation: sample concentration 10 mg/mL, acetonitrile solution injection: 3.0 mL each time. After separation, the fraction was dried on a rotary evaporator at a bath temperature of 35°C to obtain P1 (retention time: 1.592 minutes, assigned as compound **33-1)** and P2 (retention time: 2.079 minutes, assigned as compound **33-2).**

LC-MS (ESI): m/z = 474.1 $[M+H]^+$.

$^1$H NMR (400 MHz, DMSO-d6) δ 8.78 (s, 2H), 7.18 (s, 1H), 4.37 (s, 2H), 4.05 (d, 2H), 3.90 - 3.87 (m, 4H), 3.41 - 3.36 (m, 1H), 3.13 - 3.10 (m, 1H), 2.98 - 2.79 (m, 2H), 2.56 (s, 2H), 0.90 - 0.64 (m, 4H), 0.00 (s, 6H).

LC-MS (ESI): m/z = 474.1 $[M+H]^+$.

$^1$H NMR (400 MHz, DMSO-d6) δ 8.78 (s, 2H), 7.19 (s, 1H), 4.37 (s, 2H), 4.05 (d, 2H), 3.90 - 3.87 (m, 4H), 3.46 - 3.31 (m, 1H), 3.16 - 3.08 (m, 1H), 2.97 - 2.78 (m, 2H), 2.56 (s, 2H), 0.88 - 0.63 (m, 4H), 0.00 (s, 6H).

**Example 35**

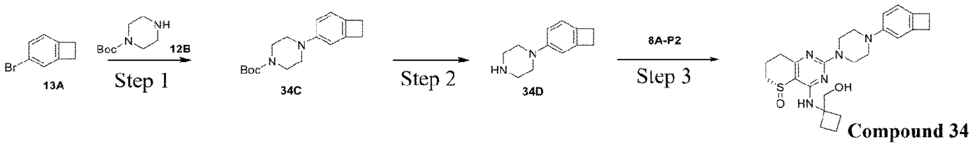


Using compounds **13A** and **12B** as raw materials, compound **34** (160 mg, 53%) was obtained according to the operation of Example 14.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min; retention time: 12.60 min.

LCMS m/z = 454.2 $[M+1]^+$

$^1$H NMR (400 MHZ, DMSO-d6) δ 6.92 (d, 1H), 6.78-6.81 (m, 1H), 6.75 (s, 1H), 6.53 (s, 1H), 4.88 (t, 1H), 3.82 (t, 4H), 3.71 (d, 2H), 3.01-3.09 (m, 9H), 2.88-2.94 (m, 1H), 2.52-2.70 (m, 2H), 2.29-2.43 (m, 3H), 2.09-2.19 (m, 2H), 1.90-2.00 (m, 1H), 1.72-1.86 (m, 2H).

**Example 36**

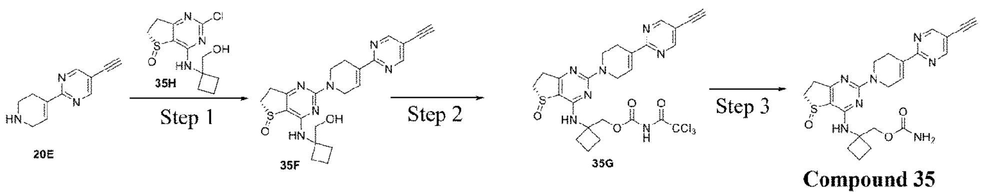


Step 1: Compound **20E** (210.0 mg, 1.14 mmol) was dissolved in 1,4-dioxane (20.0 mL), compound **35H** (345.0 mg, 1.2 mmol, see patent WO 2014124860 A1 for the synthesis) was then added, and the mixture was heated back to 90°C and reacted for 12 h. The system was then cooled to room temperature, concentrated, and subjected to column chromatography (dichloromethane/methanol = 10/1) to obtain compound **35F** (360.0 mg, 72%).

LC-MS (ESI): m/z = 437.1 $[M+H]^+$.

$^1$H NMR (400 MHz, DMSO-d6) δ 8.90 (s, 2H), 7.36 (s, 1H), 7.34 (s, 1H), 4.84 (t, 1H), 4.68 (s, 1H), 4.48 (s, 2H), 3.97 (t, 2H), 3.75 (d, 2H), 3.51 - 3.36 (m, 1H), 3.28 - 3.18 (m, 1H), 2.98 - 2.84 (m, 2H), 2.66 (s, 2H), 2.43 - 2.30 (m, 2H), 2.24 - 2.17 (m, 2H), 1.86 - 1.71 (m, 2H).

Step 2: Compound **35F** (0.8 g, 2.0 mmol) was dissolved in dichloromethane (15 mL), trichloroacetyl isocyanate (452.0 mg, 2.4 mmol) was added in an ice bath, and the mixture was further reacted for 1 h. The system was concentrated to obtain **35G** (1.2 g, 99%), which was directly used for the next step.

LC-MS (ESI): m/z = 624.0 $[M+1]^+$

Step 3: Compound **35G** (1.2 g, 2.0 mmol) was dissolved in methanol (15 mL), water (15 mL) and potassium carbonate (1.0 g, 8.0 mmol) were then added, and the mixture reacted at room temperature for 2.5 hours. After

concentration, the system was extracted by adding dichloromethane, dried, and concentrated to obtain compound 35 (0.6 g, 62%).

LC-MS (ESI): m/z = 480.2 [M+1]$^+$

$^1$H NMR (400 MHz, DMSO-d6) δ 8.90 (s, 2H), 7.61 (s, 1H), 7.34 (s, 1H), 6.48 (s, 2H), 4.68 (s, 1H), 4.48 (s, 2H), 4.44 - 4.32 (m, 2H), 3.99 - 3.96 (m, 2H), 3.52 - 3.36 (m, 1H), 3.27 - 3.11 (m, 1H), 2.99 - 2.85 (m, 2H), 2.66 (s, 2H), 2.41 - 2.24 (m, 4H), 1.95 1.74 (m, 2H).

**Example 37**

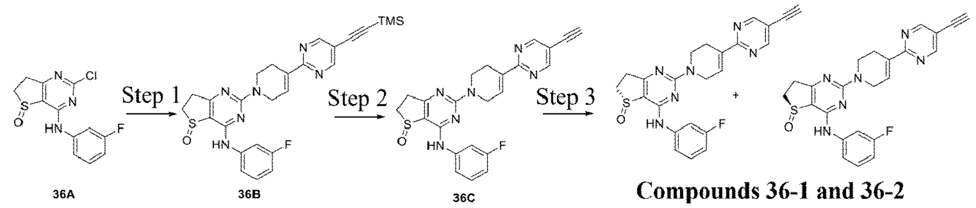


36A 36B 36C **Compounds 36-1 and 36-2**

Step 1: **36A** (360 mg, 1.21 mmol, see patent WO 2011124524 A1 for the synthesis) was dissolved in 1,4-dioxane (8 mL), **20D** (515 mg, 1.45 mmol) and N,N-diisopropylethylamine (780 mg, 6.05 mmol) were successively added, and the mixture was heated to 85°C and stirred for 16 h under nitrogen protection. After the reaction liquid naturally returned to room temperature, the reaction was diluted by adding water (50 mL) and extracted with ethyl acetate (50 mL) three times. The organic phases were combined, dried over anhydrous sodium sulfate, and then concentrated in vacuum to obtain a crude product, which was purified by column chromatography (eluents: DCM:MeOH = 100:1 to 15:1) to obtain the target compound **36B** (590 mg, yield: 94.07%).

LC-MS (ESI): m/z = 519.2 [M+H]$^+$.

Step 2: Compound **36B** (550 mg, 1.06 mmol) was dissolved in methanol (15 mL), potassium carbonate (150 mg, 1.06 mmol) was added, and the mixture was stirred at room temperature for 2 h. After the raw material disappeared as detected by a TLC plotting plate (developing agent: DCM:MeOH = 15:1), the mixture was diluted by adding water (50 mL) and extracted three times with dichloromethane (50 mL). The organic phases were combined, dried over anhydrous sodium sulfate, and then concentrated in vacuum to obtain a crude product, which was purified by column chromatography (eluents: DCM:MeOH = 100:1 to 10:1) to obtain the target compound **36C** (350 mg, yield: 73.92%).

LC-MS (ESI): m/z = 447.2 [M+H]$^+$.

Step 3: Compound **36C** (350 mg, 0.78 mmol) was subjected to chiral resolution to obtain compound 36-1 (13.0 mg) and compound **36-2** (3.2 mg).

Analysis method: instrument: SHIMADZU LC-20AD, column: Chiralpak IG-3 50 × 4.6 mm I.D., 3 μm; mobile phase: A: heptane (0.05%, N,N-diethylaniline), B: ethanol (0.05% N,N-diethylaniline); gradient: 20% B in A; flow rate: 1 mL/min, column temperature: 35°C, wavelength: 254 nm.

**Preparation method:** instrument: SHIMADZU LC-20AP, column: DAICEL CHIRALPAK IG (250 mm * 30 mm, 10 μm), mobile phase: A n-hexane, B ethanol (0.1% $NH_3$•H2O); gradient: 8% B gradient elution, flow rate: 110 mL/min, column temperature: 25°C, wavelength: 254 nm, cycle time: 16 min, sample preparation: sample concentration 1.5 mg/ml, ethanol solution injection: 2 ml each time. After separation, the fraction was dried by a rotary evaporator at a bath temperature of 40°C to obtain compound **36-1** (retention time: 5.716 minutes) and compound **36-2** (retention time: 6.890 minutes).

**Compound 36-1:** LCMS m/z = 447.2 [M+H]$^+$

$^1$H NMR (400 MHz, $CDCl_3$) δ 8.77 (s, 2H), 8.09 (s, 1H), 7.64 - 7.61 (m, 1H), 7.37 (s, 1H), 7.23 - 7.18 (m, 2H), 6.76 - 6.72 (m, 1H), 4.56 - 4.47 (m, 2H), 4.10 - 4.00 (m, 2H), 3.67 - 3.59 (m, 1H), 3.45 - 3.39 (m, 2H), 3.17 - 3.03 (m, 2H), 2.86 - 2.77 (m, 2H).

**Compound 36-2:** LCMS m/z = 447.2 [M+H]$^+$

$^1$H NMR (400 MHz, $CDCl_3$) δ 8.77 (s, 2H), 8.09 (s, 1H), 7.64 - 7.61 (m, 1H), 7.37 (s, 1H), 7.23 - 7.17 (m, 2H), 6.76 - 6.72 (m, 1H), 4.54 - 4.49 (m, 2H), 4.10 - 4.01 (m, 2H), 3.67 - 3.59 (m, 1H), 3.45 - 3.39 (m, 2H), 3.17 - 3.03 (m, 2H), 2.86 - 2.77 (m, 2H).

**Example 38**

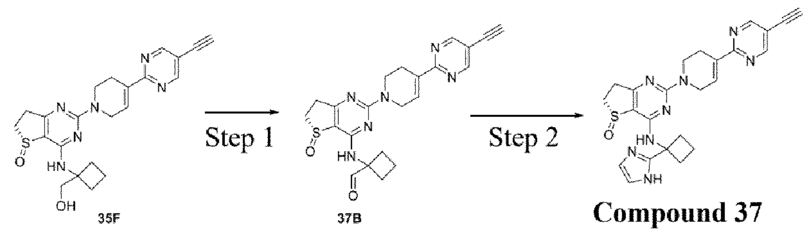


35F 37B Compound 37

Step 1: Compound **35F** (2.0 g, 4.58 mmol) was dissolved in dichloromethane (100 mL), Dess-Martin periodinane (2.92 g, 6.88 mmol) was added, and the mixture was stirred at room temperature overnight. The reaction liquid was quenched with a saturated sodium thiosulfate aqueous solution, and the aqueous phase was extracted with dichloromethane. The organic phase was dried, filtered, concentrated, and subjected to column chromatography (dichloromethane/methanol = 10/1) to obtain compound **37B** (1.2 g, 60%).
LC-MS (ESI): m/z = 435.2 $[M+H]^+$
Step 2: Compound **37B** (220 mg, 0.51 mmol) was dissolved in an ammonia methanol solution (5 mL, 7.0 M) and stirred at room temperature for half an hour, and glyoxal (1 mL, 40 wt.% in water) was then added and stirred at room temperature overnight. The reaction liquid was concentrated and subjected to silica gel column chromatography (dichloromethane:methanol = 10:1) to obtain 130 mg of a pale yellow solid, and after reverse phase preparation (acetonitrile:water = 1/99-99/1), compound **37** (100 mg, 40%) was obtained.
LC-MS (ESI): m/z = 473.2 $[M+H]^+$.
$^1$H NMR (400 MHz, DMSO-$d_6$) δ 11.38 (s, 1H), 8.89 (s, 2H), 8.20 (s, 1H), 7.24 (s, 1H), 6.90 (s, 1H), 6.79 (s, 1H), 4.68 (s, 1H), 4.60-3.95 (m, 2H), 3.94 - 3.58 (m, 2H), 3.49-3.37 (m, 1H), 3.25-3.15(m, 1H), 3.01 - 2.76 (m, 3H), 2.65-2.51 (m, 5H), 2.02 - 1.88 (m, 2H).

**Example 39**

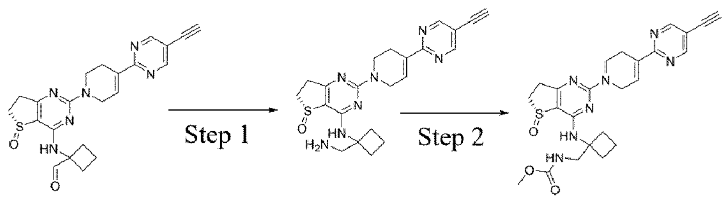


37B 38B Compound 38

Step 1: Compound **37B** (1.2 g, 2.76 mmol) was dissolved in 1,2-dichloroethane (100 mL), ammonium acetate (21.3 g, 275.94 mmol) was added and stirred at room temperature overnight, and sodium triacetoxyborohydride (5.85 g, 27.60 mmol) was then added. The mixture was reacted at room temperature for 2 h. The reaction was quenched by adding a saturated sodium bicarbonate solution. The aqueous phase was extracted with dichloromethane, and the organic phases were combined, dried, filtered, concentrated, and subjected to silica gel column chromatography (dichloromethane:methanol:aqueous ammonia = 10:1:0.1) to obtain compound **38B** (180 mg, 15%).
LC-MS (ESI): m/z = 436.2 $[M+H]^+$.
Step 2: Compound **38B** (100 mg, 0.23 mmol) was dissolved in dichloromethane (10 mL), triethylamine (35 mg, 0.35 mmol) was added in an ice bath and stirred for five minutes, and methyl chloroformate (24 mg, 0.25 mmol) was then added. The mixture was reacted at 0°C for 1 h. The reaction was quenched by adding water, the aqueous phase was extracted with dichloromethane, and the organic phases were combined, dried, filtered, concentrated, and subjected to reverse phase preparation (acetonitrile:water = 1/99-99/1) to obtain compound **38** (50 mg, 44%).
LC-MS (ESI): m/z = 494.2 $[M+H]^+$
$^1$H NMR (400 MHz, DMSO-$d_6$) δ 8.90 (s, 2H), 7.49 (s, 1H), 7.40 - 7.31 (m, 1H), 7.18 (t, 1H), 4.68 (s, 1H), 4.49 (s, 2H), 3.99 (t, 2H), 3.57 - 3.38 (m, 6H), 3.27 - 3.16 (m, 1H), 3.01 - 2.82 (m, 2H), 2.67 (s, 2H), 2.42 - 2.18 (m, 4H), 1.88 - 1.68 (m, 2H).

**Example 40**

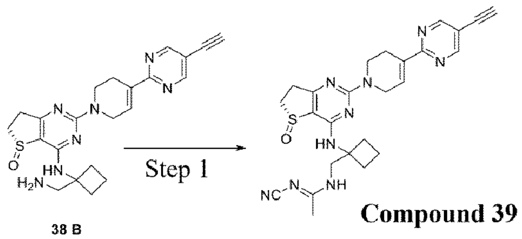


38 B

Compound 39

Step 1: Compound **38B** (100 mg, 0.23 mmol) was dissolved in methanol (5 mL), methyl N-cyanoethanimideate (25 mg, 0.26 mmol) was added, and the mixture was stirred at room temperature overnight. The reaction liquid was concentrated and subjected to reverse phase preparation (acetonitrile:water = 1/99-99/1) to obtain compound **39** (50 mg, 43%).

LC-MS (ESI): m/z = 502.2 $[M+H]^+$

$^1$H NMR (400 MHz, DMSO-$d_6$) δ 8.90 (s, 2H), 8.72 (s, 1H), 7.53 (s, 1H), 7.35 (s, 1H), 4.69 (s, 1H), 4.49 (s, 2H), 3.98 (t, 2H), 3.89-3.81 (m, 1H), 3.78-3.68 (m, 1H), 3.50-3.39 (m, 1H), 3.25-3.18 (m, 1H), 3.03-2.79 (m, 2H), 2.72-2.64 (m, 2H), 2.41-2.23 (m, 4H), 2.21 (s, 3H), 1.86-1.74(m, 2H).

**Example 41**

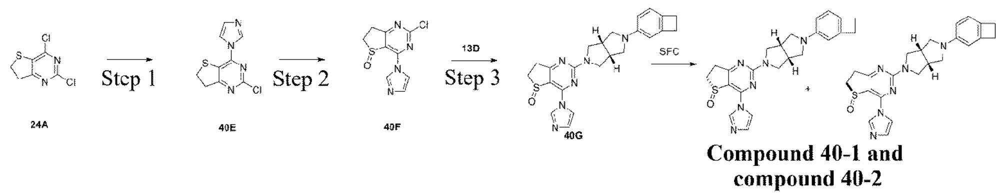


24A 40E 40F 40G

Compound 40-1 and compound 40-2

Step 1: Compound **24A** (3.00 g, 14.46 mmol) and imidazole (1.18 g, 17.35 mmol) were weighed into a 100 mL single-mouth flask and dissolved with acetonitrile (30 mL), and N,N-diisopropylethylamine (5.61 g, 43.38 mmol) was added. After the addition was complete, the system was stirred at 70 degrees Celsius for 16 h. After the reaction was complete as monitored by a TLC spotting plate (petroleum ether:ethyl acetate = 2:1), water (50 mL) was added and stirred for 5 min, and the mixture was extracted with ethyl acetate (30 mL). The organic phase was separated, dried over anhydrous sodium sulfate, and concentrated under reduced pressure, and the crude product was purified by column chromatography (eluents: petroleum ether: ethyl acetate = 2:1) to obtain the target compound **40E** (1.0 g, yield: 29%).

LCMS m/z = 239.1 $[M+1]^+$

Step 2: Compound **40E** (1.0 g, 4.19 mmol) was weighed into a 100 mL single-mouth flask and dissolved with dichloromethane (10 mL), and tert-butyl hydroperoxide (0.65 g, 5.03 mmol) was added and stirred at room temperature for 12 h. After the reaction was complete as monitored by a TLC spotting plate (petroleum ether:ethyl acetate = 2:1), water (50 mL) was added and stirred for 5 min, and the mixture was extracted with dichloromethane (30 mL). The organic phase was separated, dried over anhydrous sodium sulfate, and concentrated under reduced pressure, and the crude product was purified by column chromatography (eluents: dichloromethane: methanol = 20:1) to obtain the target compound **40F** (0.63 g, yield: 59%).

LCMS m/z = 255.1 $[M+1]^+$

Step 3: Compound **40F** (300 mg, 1.18 mmol) was added to a 100 mL single-mouth flask and dissolved with dioxane (10 mL), and compound **40C** (300 mg, 1.42 mmol) and N,N-diisopropylethylamine (0.46 g, 3.54 mmol) were then added. After the addition was complete, the system was placed under nitrogen protection and stirred at 85°C for 16 h. After the reaction was complete as monitored by TLC (dichloromethane:methanol = 10:1), water (50 mL) was added and stirred for 5 min, and the mixture was extracted with dichloromethane (30 mL). The organic phase was separated, dried over anhydrous sodium sulfate, and concentrated under reduced pressure, and the crude product was purified by column chromatography (eluents:dichloromethane: methanol = 10:1) to obtain the target compound **40G,** which was subjected to chiral resolution to obtain the target compound **40-1** (200 mg) and compound **40-2** (120 mg).

Chiral resolution method: instrument: Waters 150 SFC; column: Chiralpak IC Column (250 × 30 mm, I.D 30 mm,

10 um particle size); mobile phase: A for CO2 and B for IPA + ACN (0.1% $NH_3$•$H_2O$); gradient: 45% phase B isocratic elution; flow rate: 100 mL/min; back pressure: 100 bar; column temperature: 25°C; wavelength: 220 nm.

LCMS m/z = 433.2 [M +1]$^+$

**Compound 40-1,** $^1$H NMR (400 MHZ, DMSO-d6) δ 8.59 (s, 1H), 8.00 (s, 1H), 7.23 (s, 1H), 6.85 (d, 1H), 6.34-6.37 (m, 2H), 3.88-3.98 (m, 2H), 3.58-3.77 (m, 3H), 3.40-3.48 (m, 3H), 3.12-3.37 (m, 6H), 2.98-3.05 (m, 4H).

**Compound 40-2,** $^1$H NMR (400 MHZ, DMSO-d6) δ 8.59 (s, 1H), 8.00 (s, 1H), 7.23 (s, 1H), 6.85 (d, 1H), 6.34-6.37 (m, 2H), 3.88-3.99 (m, 2H), 3.58-3.77 (m, 3H), 3.40-3.48 (m, 3H), 3.12-3.37 (m, 6H), 2.97-3.05 (m, 4H).

**Example 42**

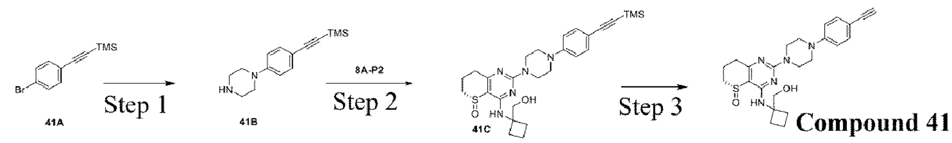


Step 1: Compound **41A**(1.0 g, 3.95 mmol) and piperazine (0.41 g, 4.7 mmol) were weighed into a 100 mL flask and then dissolved by adding dioxane (20 mL), and sodium tert-butoxide (0.76 g, 7.9 mmol), tris(dibenzylideneacetone) dipalladium (0.11 g, 0.20 mmol), and 4,5-bis(diphenylphosphino)-9,9-dimethylxanthene (250 mg, 0.40 mmol) were successively added. After the addition was complete, the mixtuer was stirred at 80°C for 12 h. After the reaction was complete as monitored by TLC (petroleum ether:ethyl acetate = 5:1), water (20 mL) was added and stirred for 5 min, and the mixture was extracted by adding ethyl acetate (20 mL). The organic phase was separated, the organic phase was dried over anhydrous sodium sulfate and concentrated under reduced pressure, and the crude product was purified by column chromatography (eluents: petroleum ether : ethyl acetate = 5:1) to obtain the target compound **41B** (0.50 g, 47%).

LCMS m/z = 259.1 [M +1]$^+$

Step 2: Compound **8A-P2** (350 mg, 1.16 mmol) and compound **41B** (300 mg, 1.16 mmol) were added to a 100 mL single-mouth flask and dissolved with dioxane (10 mL), and N,N-diisopropylethylamine (0.45 g, 3.48 mmol) was finally added. After the addition was complete, the mixture was stirred at 90°C for 16 h under nitrogen protection. After the reaction was complete as monitored by TLC (dichloromethane:methanol = 10:1), water (20 mL) was added and stirred for 5 min, and the mixture was extracted by adding ethyl acetate (20 mL). The organic phase was separated, the organic phase was dried over anhydrous sodium sulfate and concentrated under reduced pressure, and the crude product was purified by column chromatography (eluents:dichloromethane: methanol = 10:1) to obtain the target compound **41C** (0.40 g, 66%).

LCMS m/z = 524.2 [M +1]$^+$

Step 3: Compound **41C** (0.35 g, 0.67 mmol) was weighed into a 100 mL single-mouth flask and dissolved with methanol (10 mL), and potassium carbonate (0.27 g, 1.94 mmol) was finally added. After the addition was complete, the mixture was stirred for 2 h. After the reaction was complete as monitored by TLC (dichloromethane:methanol = 10:1), the reaction liquid was filtered under reduced pressure and then concentrated to obtain the target compound. The crude product was purified by preparative HPLC to obtain the title compound **41** (200 mg, 66%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min; retention time: 12.50 min.

LCMS m/z = 452.2 [M +1]$^+$

$^1$H NMR (400 MHZ, DMSO-d6) δ 7.31 (d, 2H), 6.94 (d, 2H), 6.55 (s, 1H), 4.89 (s, 1H), 3.92 (s, 1H), 3.83 (t, 4H), 3.71 (s, 2H), 3.25 (t, 4H), 3.05-3.09 (m, 1H), 2.88-2.95 (m, 1H), 2.53 -2.71 (m, 2H), 2.28-2.45 (m, 3H), 2.09-2.21 (m, 2H), 1.91-2.00 (m,1H), 1.73-1.88 (m, 2H).

**Example 43**

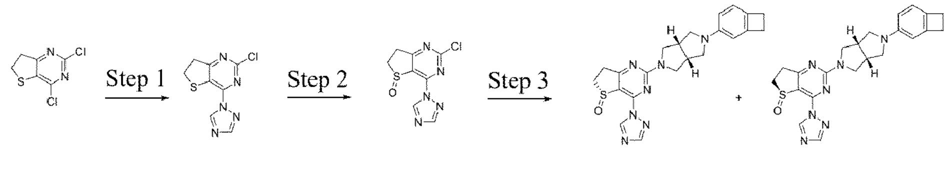


24A 42B 42C **Compounds 42-1 and 42-2**

Using compound **24A** and 1,2,4-triazole as raw materials, compounds **42-1** and **42-2** were obtained according to the operation of Example 41.

Chiral resolution method: instrument: Waters 150 Prep-SFC F; SFC column: Chiralcel OJ-Column ; mobile phase: A for CO2; B for 0.1% $NH_3$•$H_2O$ in IPA and ACN; gradient: B 40% isocratic elution; flow rate: 120 mL/min; back pressure: 100 bar; column temperature: 25°C; wavelength: 220 nm.

**Compound 42-1:** retention time: 1.217 min. $^1$H NMR (400 MHz, DMSO-d6) δ 9.60 (s, 1H), 8.48 (s, 1H), 6.85 (d, 1H), 6.41 - 6.34 (m, 2H), 4.03 - 3.87 (m, 2H), 3.74 - 3.56 (m, 2H), 3.53 - 3.41 (m, 3H), 3.28 - 3.13 (m, 5H), 3.11 - 2.96 (m, 6H).

LCMS (ESI): m/z = 434.2 [M+H] $^+$

**Compound 42-2:** retention time: 1.570 min. $^1$H NMR (400 MHz, DMSO-d6) δ 9.60 (s, 1H), 8.48 (s, 1H), 6.85 (d, 1H), 6.41 - 6.34 (m, 2H), 4.03 - 3.87 (m, 2H), 3.74 - 3.56 (m, 2H), 3.53 - 3.41 (m, 3H), 3.28 - 3.13 (m, 5H), 3.11 - 2.96 (m, 6H).

LCMS (ESI): m/z = 434.2 [M+H] $^+$

## Example 44

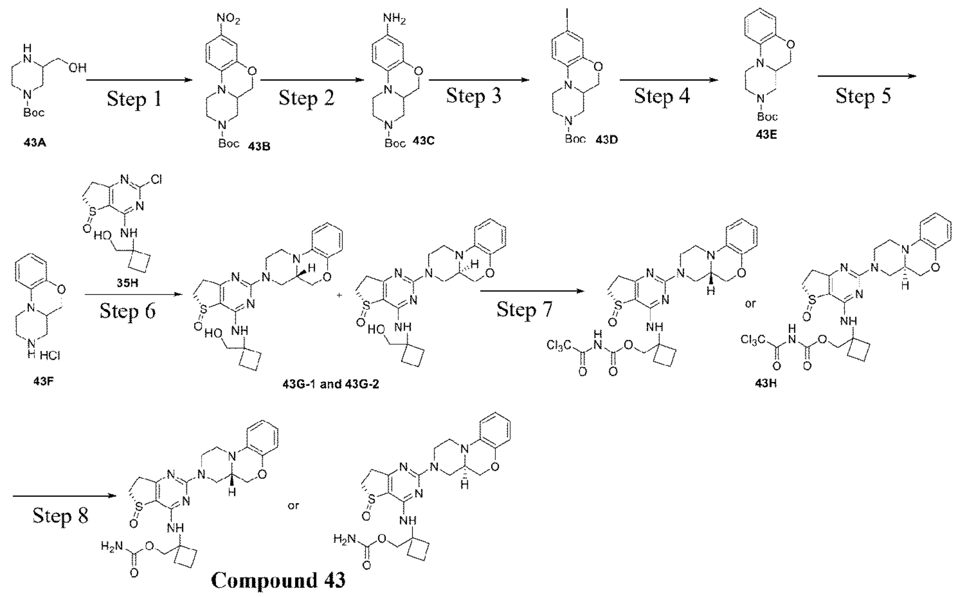


43A 43B 43C 43D 43E 35H 43F 43G-1 and 43G-2 or 43H or **Compound 43**

Step 1: To a 250 mL single-mouth flask, **43A** (10.0 g, 46.3 mmol), 1,2-difluoro-4-nitrobenzene (7.3 g, 46.3 mmol), potassium hydroxide (7.8 g, 138.9 mmol), and N,N-dimethylformamide (100 mL) were successively added. After reaction at room temperature for 6 hours, the system was then heated to 60°C and reacted for 24 hours. After filtration, the filtrate was added to ethyl acetate (500 mL) and washed with water (100 mL × 3). The organic layers were dried over anhydrous sodium sulfate. After filtration, the filtrate was concentrated under reduced pressure and then separated and purified by silica gel column chromatography (eluents: ethyl acetate/petroleum ether (v/v) = 20/100) to obtain **43B** (10.0 g, yield 64.5%).

LC-MS (ESI): m/z = 336.2 [M+H]$^+$.

Step 2: To a 250 mL single-mouth flask, **43B** (10.0 g, 29.9 mmol), ethyl acetate (100 mL), and palladium on carbon (2 g) were successively added, and the mixture was hydrogenated and reacted at room temperature for 12 hours. After filtration, the filtrate was concentrated under reduced pressure to obtain **43C** (7.0 g, yield 76.9%).

LC-MS (ESI): m/z = 306.2 [M+H]$^+$.

Step 3: To a 100 mL single-mouth flask, acetonitrile (30mL), tert-butyl nitrite (1.5 g, 14.7 mmol), and cuprous iodide (2.24 g, 11.8 mmol) were successively added and heated to 65°C, and a solution of **43C** (3.0 g, 9.8 mmol) in

acetonitrile (10 mL) was dropwise added. After the dropwise addition was complete, the system was reacted at 65°C for 4 hours, and after heating was turned off, the system was further reacted for 12 hours, concentrated under reduced pressure, and then separated and purified by silica gel column chromatography (eluents: ethyl acetate/petroleum ether (v/v) = 20/100) to obtain **43D** (0.4 g, yield 12.6%).

LC-MS (ESI): m/z = 361.2 $[M-56+H]^+$.

Step 4: To a 50 mL single-mouth flask, methanol (10 mL), **43D** (0.5 g, 1.20 mmol), and palladium on carbon (100 mg) were successively added, and the mixture was hydrogenated and reacted at room temperature for 16 hours. After filtration, the filtrate was concentrated under reduced pressure to obtain **43E** (0.3 g, yield 86.1%).

LC-MS (ESI): m/z = 235.2 $[M-56+H]^+$.

Step 5: To a 50 mL single-mouth flask, **43E** (0.3 g, 1.03 mmol) and a hydrogen chloride-dioxane solution (4N, 15 mL) were successively added and reacted at room temperature for 1 hour, and the system was concentrated under reduced pressure to obtain **43F** (0.27 g, yield 99.6%).

LC-MS (ESI): m/z = 191.2 $[M+H]^+$.

Step 6: To a 50 mL single-mouth flask, **35H** (287 mg, 1.0 mmol), **43F** (270 mg, 1.03 mmol), dioxane ( 10 mL), and DIPEA (774 mg, 6.0 mmol) were successively added and reacted at 100°C for 12 hours. After cooling to room temperature, the reaction liquid was directly concentrated under reduced pressure and then separated and purified by silica gel column chromatography (eluents: dichloromethane/methanol (v/v) = 100/8) to obtain 400 mg of a mixture.

Chiral preparation was carried out to obtain the title compound **43G-1** (62 mg, 14.1%, retention time: 1.733 min) and the title compound **43G-2** (51 mg, 11.5%, retention time: 1.884 min). Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 3.0 min.

**(Compound 43G-1)**

$^1$H NMR (400 MHz, DMSO-$d_6$) δ 7.43 (s, 1H), 6.98 - 6.86 (m, 1H), 6.86 - 6.75 (m, 1H), 6.76 - 6.59 (m, 2H), 4.90 - 4.79 (m, 1H), 4.78 - 4.59 (m, 2H), 4.37 - 4.28 (m, 1H), 4.00 - 3.92 (m, 1H), 3.90 - 3.78 (m, 1H), 3.78 - 3.68 (m, 2H), 3.49 - 3.36 (m, 1H), 3.27 - 2.81 (m, 5H), 2.76 - 2.55 (m, 2H), 2.41 - 2.12 (m, 4H), 1.87 - 1.70 (m, 2H).

LC-MS (ESI): m/z = 442.2 $[M+H]^+$.

**(Compound 43G-2)**

$^1$H NMR (400 MHz, DMSO-$d_6$) δ 7.44 (s, 1H), 6.95 - 6.89 (m, 1H), 6.84 - 6.77 (m, 1H), 6.75 - 6.62 (m, 2H), 4.89 - 4.78 (m, 1H), 4.79 - 4.59 (m, 2H), 4.39 - 4.28 (m, 1H), 4.02 - 3.81 (m, 2H), 3.79 - 3.68 (m, 2H), 3.51 - 3.39 (m, 1H), 3.26 - 3.16 (m, 1H), 3.14 - 2.83 (m, 4H), 2.76 - 2.55 (m, 2H), 2.43 - 2.25 (m, 2H), 2.26 - 2.13 (m, 2H), 1.89 - 1.69 (m, 2H).

LC-MS (ESI): m/z = 442.2 $[M+H]^+$.

Step 7: To a 50 mL single-mouth flask, dichloromethane (5 mL) and **43G-1** (60 mg, 0.14 mmol) were successively added, trichloroacetyl isocyanate (52 mg, 0.28 mmol) was dropwise added at 0°C and reacted at room temperature for 1 hour, and the system was concentrated under reduced pressure to obtain crude **43H,** which was directly used in the next step.

LC-MS (ESI): m/z = 629.1 $[M+H]^+$.

Step 8: To a 50 mL single-mouth flask, crude **43H,** methanol (2 mL), water (2 mL), and potassium carbonate (38 mg, 0.28 mmol) were successively added and reacted at room temperature overnight, and the reaction liquid was directly concentrated under reduced pressure and then separated and purified by silica gel column chromatography (eluents: dichloromethane/methanol (v/v) = 100/8) to obtain the title **compound 43** (40 mg, two-step yield 60.6%).

$^1$H NMR (400 MHz, DMSO-$d_6$) δ 7.69 (s, 1H), 7.00 - 6.86 (m, 1H), 6.88 - 6.75 (m, 1H), 6.74 - 6.59 (m, 2H), 6.49 (s, 2H), 4.79 - 4.61 (m, 2H), 4.49 - 4.14 (m, 3H), 4.03 - 3.76 (m, 2H), 3.50 - 3.37 (m, 1H), 3.27 - 3.15 (m, 1H), 3.14 - 2.84 (m, 4H), 2.76 - 2.54 (m, 2H), 2.43 - 2.18 (m, 4H), 1.90 - 1.73 (m, 2H).

LC-MS (ESI): m/z = 485.2 $[M+H]^+$.

**Example 45**

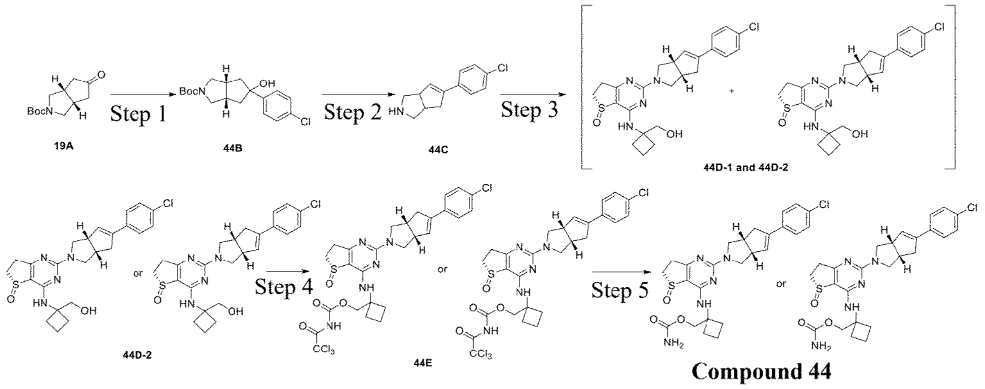

19A 44B 44C 44D-1 and 44D-2
44D-2 44E
Compound 44

Step 1: p-Chloroiodobenzene (5.82 g, 24.41 mmol) and anhydrous tetrahydrofuran (50 mL) were added to a 250 ml three-mouth flask. After nitrogen displacement, n-butyllithium (39 mmol, 11.5 ml) was dropwise added at -78°C and reacted at -78°C for 2 h. A solution of **19A** (5 g, 22.19 mmol) in tetrahydrofuran (20 ml) was then dropwise added, and the mixture was maintained at -78°C and reacted for 1 h. Water (100 ml) was added, and the mixture was extracted wtih ethyl acetate (50 ml × 2). The organic phases were combined, washed with a saturated sodium chloride solution, and dried over anhydrous sodium sulfate. After filtration, the filtrate was concentrated under reduced pressure and then separated and purified by silica gel column chromatography (eluents EA/PE = 0-35%) to obtain the target compound **44B** (4 g, 53.36%).
LC-MS (ESI): m/z = 338.1 $[M+H]^+$.
Step 2: **44B** (4 g, 11.84 mmol) was added to a mixed system of dichloromethane (45 mL) and trifluoroacetic acid (15 mL) and reacted at room temperature for 3 h. The reaction liquid was directly concentrated under reduced pressure and then brought to the next reaction.
LC-MS (ESI): m/z = 220.1 $[M+H]^+$.
Step 3: To a 100 mL single-mouth flask, **35H** (0.5 g, 1.74 mmol), **44C** (0.7 g, 2.09 mmol), dioxane (20 mL), and DIPEA (1.12 g, 8.67 mmol) were successively added and reacted at 100°C for 5 h. After cooling to room temperature, the reaction liquid was directly concentrated under reduced pressure and then separated and purified by silica gel column chromatography (eluents: MeOH/DCM = 0-10%) to obtain 700 mg of a mixture.
Chiral preparation was carried out to obtain the title compound **44D-1** (300 mg, 36.6%, retention time: 0.786 min) and the title compound **44D-2** (240mg, 29.3%, retention time: 0.993 min). Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 120 mL/min; back pressure: 100 bar; column temperature: 25°C; wavelength: 220 nm; elution time: 5.5 min.
Step 4: **44D-2** (0.1 g, 0.21 mmol) was added to dichloromethane (5 mL), the mixture was cooled to 0°C, and trichloroacetyl isocyanate (0.06 g, 0.32 mmol) was dropwise added. After reaction at 0°C for 1 h, the reaction liquid was directly concentrated under reduced pressure to obtain a crude product (0.14 g), which was directly brought to the next reaction.
LC-MS (ESI): m/z = 660.0 $[M+H]^+$.
Step 5: Methanol (3 mL) and water (3 ml) were added to crude **44E** (0.14 g), followed by potassium carbonate (0.09 g, 0.64 mmol). After reaction at room temperature for 16 h, water (10 ml) was added to the reaction liquid, and the mixture was extracted with dichloromethane (10 ml × 2). The organic phases were concentrated under reduced pressure and then separated and purified by silica gel column chromatography (eluents: MeOH/DCM = 0-10%) to obtain **compound 44** (80 mg, 74%).

**(Compound 44)**

$^1$H NMR (400 MHz, DMSO-d6) δ 7.53 - 7.35 (m, 5H), 6.47 (s, 2H), 6.26 (s, 1H), 4.47 - 4.30 (m, 2H), 3.95 - 3.91 (m, 1H), 3.77 - 3.53 (m, 3H), 3.46-3.34 (m, 1H), 3.23 - 3.01 (m, 3H), 2.97 - 2.78 (m, 3H), 2.67 - 2.57(m, 1H), 2.45 - 2.09 (m, 4H), 1.91 - 1.72(m, 2H).
LC-MS (ESI): m/z = 514.1 $[M+H]^+$.

**Example 46**

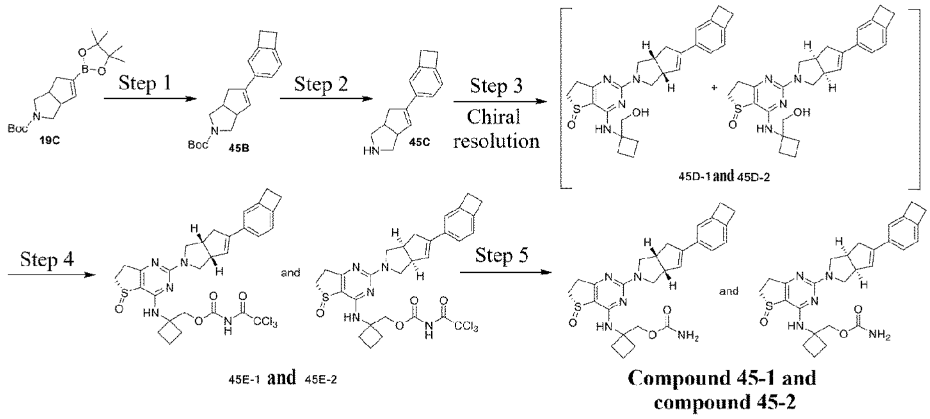

19C, 45B, 45C, 45D-1 and 45D-2, 45E-1 and 45E-2, Compound 45-1 and compound 45-2

Using compound **19C** and 4-bromophenylpropylcyclobutane as raw materials, compound **45-1** (42 mg, 96%) and compound **45-2** (25 mg, 93%) were obtained according to the operation of Examples 20 and 36.

**Compound 45-1**

LCMS (ESI): m/z = 505.2 $[M+H]^+$

$^1H$ NMR (400 MHz, $CDCl_3$) $\delta$ = 7.26 - 7.23 (m, 1H), 7.13 (s, 1H), 6.99 (d, 1H), 5.97 (s, 1H), 5.64 (s, 1H), 4.79 (s, 1H), 4.53 (s, 1H), 4.07 - 3.91 (m, 1H), 3.89 - 3.66 (m, 3H), 3.59 (s, 2H), 3.42 - 3.37 (m, 1H), 3.15 (s, 4H), 3.12-3.08 (d, 1H), 3.06 - 2.91 (m, 3H), 2.63 (d, 1H), 2.50 - 2.13 (m, 4H), 2.04 - 1.83 (m, 2H).

**Compound 45-2**

LCMS (ESI): m/z = 505.2 $[M+H]^+$

$^1H$ NMR (400 MHz, $CDCl_3$) $\delta$ = 7.26 - 7.25 (m, 1H), 7.13 (s, 1H), 6.99 (d, 1H), 5.97 (s, 1H), 5.59 (s, 1H), 4.75 (s, 2H), 4.50 (t, 1H), 3.96 (s, 1H), 3.89 - 3.69 (m, 2H), 3.60 (s, 2H), 3.42-3.35 (m, 1H), 3.16 (s, 4H), 3.12 - 3.07 (m, 1H), 3.06 - 2.92 (m, 3H), 2.63 (d, 1H), 2.38 (s, 2H), 2.23 (s, 2H), 1.95 - 1.88 (m, 2H).

**Example 47**

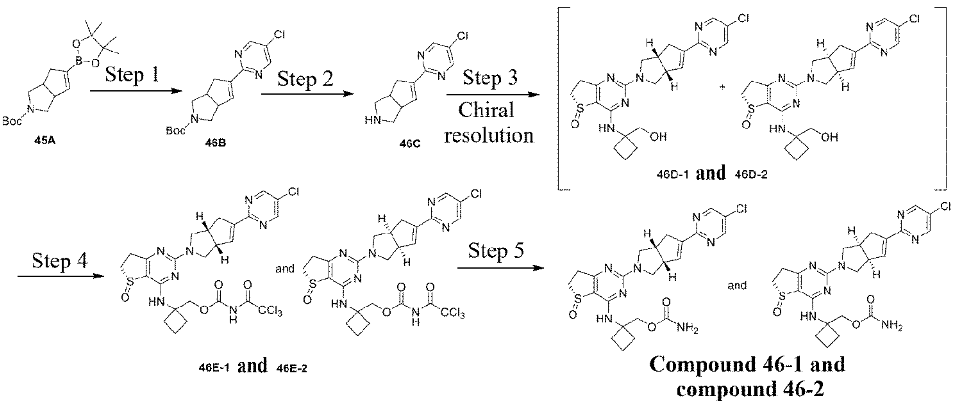

45A, 46B, 46C, 46D-1 and 46D-2, 46E-1 and 46E-2, Compound 46-1 and compound 46-2

Using compound **19C** and 5-chloro-2-iodopyrimidine as raw materials, compounds **46-1** (42 mg, 91%) and **46-2** (41 mg, 94%) were obtained according to the operation of Examples 20 and 36.

LCMS (ESI): m/z = 515.2 $[M+H]^+$

$^1H$ NMR (400 MHz, $CDCl_3$) $\delta$ = 8.62 (s, 2H), 6.86 (s, 1H), 5.56 (s, 1H), 4.76 (s, 2H), 4.52 - 4.45 (m, 2H), 4.12 - 3.73 (m, 3H), 3.68 (s, 1H), 3.62-3.54 (m, 1H), 3.45 - 3.23 (m, 2H), 3.22 - 3.11 (m, 1H), 3.10 - 2.89 (m, 3H), 2.84 (d, 1H), 2.46 - 2.30 (m, 2H), 2.31 - 2.16 (m, 2H), 2.06 - 1.80 (m, 2H), 1.67 (s, 2H).

LCMS (ESI): m/z = 515.2 $[M+H]^+$

$^1H$ NMR (400 MHz, $CDCl_3$) $\delta$ = 8.62 (s, 2H), 6.86 (s, 1H), 5.58 (s, 1H), 4.72 (s, 2H), 4.56-4.47 (m, 2H), 4.13 - 3.53

(m, 5H), 3.45-3.36 (m, 1H), 3.32 - 3.13 (m, 2H), 3.10-2.97 (m, 3H), 2.86-2.82 (m, 1H), 2.42-2.35 (m, 2H), 2.24 (s, 2H), 1.98-1.86 (m, 2H), 1.62 (s, 2H).

**Example 48**

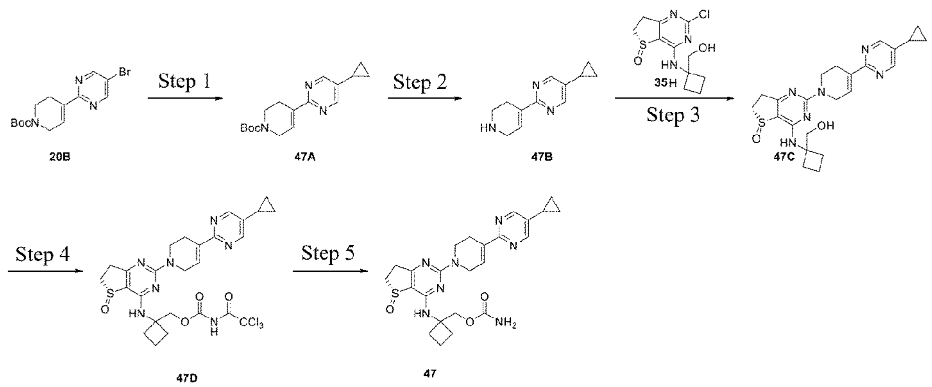


Step 1: Compound **20B** (1.0 g, 3.0 mmol), cyclopropylboronic acid (356.0 mg, 4.0 mmol), palladium acetate (25.0 mg, 0.15 mmol), tricyclohexylphosphine (84.2 mg, 0.3 mmol), and potassium phosphate (2.3 g, 10.5 mmol) were added, and toluene (14 mL) and water (0.7 mL) were finally added. After nitrogen displacement three times, the mixture was heated to 100°C and reacted for 3 h, and the system was then passed through a short silica gel column, eluted with ethyl acetate, concentrated, and subjected to column chromatography (petroleum ether/ethyl acetate = 10/1) to obtain compound **47A** (820.0 mg, 91%).
LC-MS (ESI): m/z = 302.1 $[M+H]^+$
Step 2: Compound **47A** (820.0 mg, 2.7 mmol) was dissolved in dichloromethane (20 mL), trifluoroacetic acid (5.0 mL) was then added, and the mixture was reacted at room temperature for half an hour and then concentrated to obtain compound **47B** (1.0 g, 99%), which was directly used for the next step.
LC-MS (ESI): m/z = 202.1 $[M+H]^+$.
Step 3: Compound **47B** (400.0 mg, 2.0 mmol) was dissolved in 1,4-dioxane (10.0 mL), compound **35H** (540.0 mg, 2.0 mmol) and diisopropylethylamine (1.0 g, 8.0 mmol) were then added, and the mixture was heated back to 90°C and reacted for 12 h. The system was cooled to room temperature, concentrated, and subjected to column chromatography (dichloromethane/methanol = 10/1) to obtain compound **47C** (180.0 mg, 20%).
LC-MS (ESI): m/z = 453.1 $[M+H]^+$.
Step 4: Compound **47C** (0.13 g, 0.28 mmol) was dissolved in dichloromethane (5 mL), trichloroacetyl isocyanate (64.0 mg, 0.34 mmol) was added in an ice bath, and the mixture was further reacted for 1 h. The system was concentrated to obtain **47D** (0.18 g, 99%), which was directly used for the next step.
Step 5: Compound **47D** (0.18 g, 0.28 mmol) was dissolved in methanol (2 mL), water (2 mL) and potassium carbonate (0.1 g, 0.8 mmol) were then added, and the mixture reacted at room temperature for 2.5 hours. After concentration, the system was extracted by adding dichloromethane, dried, and concentrated to obtain compound **47** (0.07 g, 51%).
LC-MS (ESI): m/z = 496.2 $[M+1]^+$
$^1$H NMR (400 MHz, DMSO-d6) δ 8.54 (s, 2H), 7.62 (s, 1H), 7.18 (s, 1H), 6.49 (s, 2H), 4.43 - 4.40 (m, 3H), 3.97 (s, 2H), 3.54 - 3.39 (m, 1H), 3.25 - 3.17 (m, 1H), 2.99 - 2.85 (m, 2H), 2.65 (s, 2H), 2.46 - 2.11 (m, 4H), 2.00 - 1.70 (m, 4H), 1.08 - 0.97 (m, 2H), 0.89 - 0.78 (m, 2H).

**Example 49**

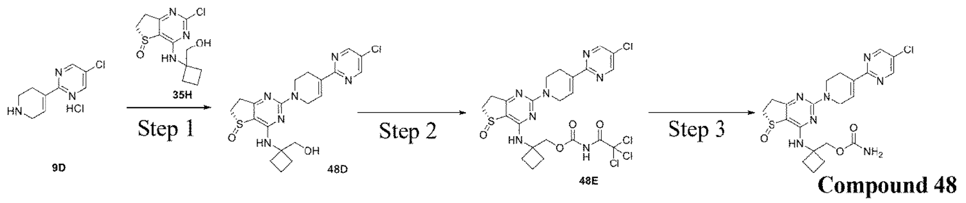


9D 48D 48E Compound 48

Step 1: To a 50 mL single-mouth flask, **35H** (0.20 g, 0.69 mmol) was added and dissolved with dioxane (10 mL), **9D** (0.21 g, 0.92 mmol) and diisopropylethylamine (0.26 g, 2.05 mmol) were added, and the mixture was reacted at 90 degrees Celsius overnight. The reaction liquid was concentrated, purified by HPLC, and freeze-dried to obtain the title compound **48D** (130 mg, 42%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 30-70%; c. flow: 15 mL/min; d. elution time: 15 min; retention time: 8.0 min.

$^1$H NMR (400 MHz, DMSO-d6) δ 8.89 (s, 2H), 7.34 (s, 1H), 7.31 - 7.26 (m, 1H), 4.83 (t, 1H), 4.46 (s, 2H), 3.97 (t, 2H), 3.75 (d, 2H), 3.45-3.41 (m, 1H), 3.24-3.21 (m, 1H), 2.98-2.83 (m, 2H), 2.65 (s, 2H), 2.43 - 2.28 (m, 2H), 2.25 - 2.14 (m, 2H), 1.90 - 1.73 (m, 2H).

LC-MS (ESI): m/z = 447.1 [M+H]$^+$.

Step 2: To a 50 mL single-mouth flask, **48D** (60 mg, 0.13 mmol) was added and dissolved with anhydrous dichloromethane (15 mL), trichloroacetyl isocyanate (30 mg, 0.16 mmol) was added under ice bath condition, and the system was maintained under the ice bath condition and stirred for 4 h. After the reaction was complete as monitored by TLC (DCM:MeOH = 10:1), the reaction liquid was concentrated to obtain the title compound **48E** (85 mg, crude), which was directly used for the next reaction.

Step 3: To a 50 mL single-mouth flask, **48E** (85 mg, 0.13 mmol) was added and dissolved with methanol (6 mL), and potassium carbonate (54 mg, 0.39 mmol) was added and stirred at room temperature for 6 h. The reaction liquid was concentrated and separated by SFC to obtain the target compound **48** (30 mg, 47%).

Preparation method: instrument: SFC Prep 150; chromatographic column: torus AP (19 mm × 250 mm); the sample was dissolved with MeOH and filtered with a 0.45 μm filter to prepare a sample liquid; preparative chromatography conditions: a. composition of mobile phases A and B: mobile phase A: $CO_2$, mobile phase B: MeOH; b. isocratic elution, mobile phase B with a content of 20%; c. flow: 40 mL/min; d. elution time: 20 min; retention time: 8.5 min.

LCMS m/z = 490.2 [M +1]$^+$

$^1$H NMR (400 MHz, DMSO-d6) δ 8.89 (s, 2H), 7.64 (s, 1H), 7.29 (s, 1H), 6.50 (s, 2H), 4.57 - 4.32 (m, 4H), 3.98 (t, 2H), 3.51 - 3.38 (m, 1H), 3.26 - 3.15 (m, 1H), 3.03 - 2.81 (m, 2H), 2.65 (s, 2H), 2.41-2.23 (m, 4H), 1.90 - 1.74 (m, 2H).

**Example 50**

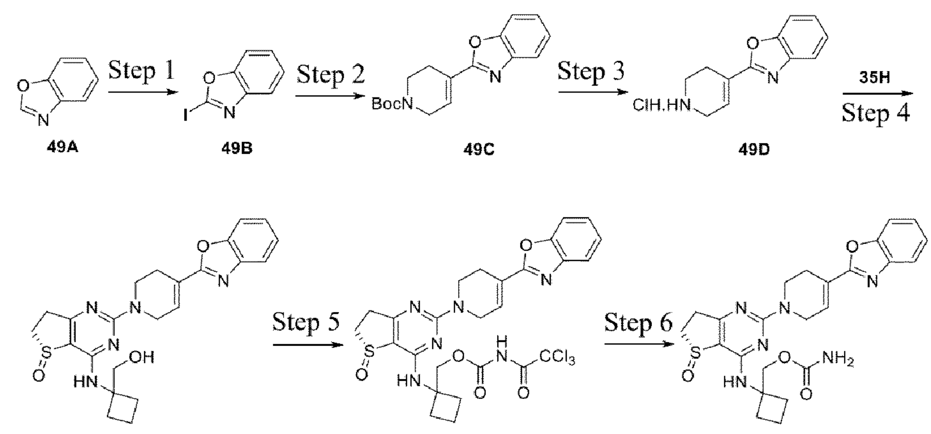


49A 49B 49C 49D 49E 49F Compound 49

Step 1: Under nitrogen protection, **49A** (1.00 g, 8.39 mmol) was dissolved in tetrahydrofuran (30 mL), and after the solution was cooled to -78°C, n-butyllithium (810 mg, 12.59 mmol) was slowly added. After the addition was complete,

the reaction liquid was heated to -40°C, stirred for 1 h, and then cooled to -78°C, and a solution of iodine (2.56 g, 10.07 mmol) in tetrahydrofuran (10 mL) was added. The system then naturally returned to room temperature and was stirred for 1 h, the reaction was quenched by adding water (100 mL), and the system was extracted three times with ethyl acetate (100 mL). The organic phases were combined, dried over anhydrous sodium sulfate, and then concentrated in vacuum to obtain a crude product, and the crude product was purified by column chromatography (eluents: PE:EA = 100:1 to 20:1) to obtain the target compound **49B** (700 mg, yield: 34.03%).

$^1$H NMR (400 MHz, DMSO-d6) δ 7.76 - 7.73 (m, 2H), 7.38 - 7.36 (m, 2H).

Using compounds **49B** and **9B** as raw materials, compound **49** (15 mg, 46.21%) was obtained according to the operation of Examples 10 and 36.

LCMS m/z = 495.10 [M+H]$^+$

$^1$H NMR (400 MHz, DMSO-d6) δ 7.73 - 7.69 (m, 2H), 7.67 (s, 1H), 7.41 - 7.34 (m, 2H), 7.10 (s, 1H), 6.49 (s, 2H), 4.51 (s, 2H), 4.44 - 4.38 (m, 2H), 4.03 - 4.00 (m, 2H), 3.49 - 3.41 (m, 1H), 3.26 - 3.19 (m, 1H), 3.01 - 2.94 (m, 1H), 2.91 - 2.86 (m, 1H), 2.69 (s, 2H), 2.44 - 2.25 (m, 4H), 1.88 - 1.82 (m, 2H).

**Example 51**

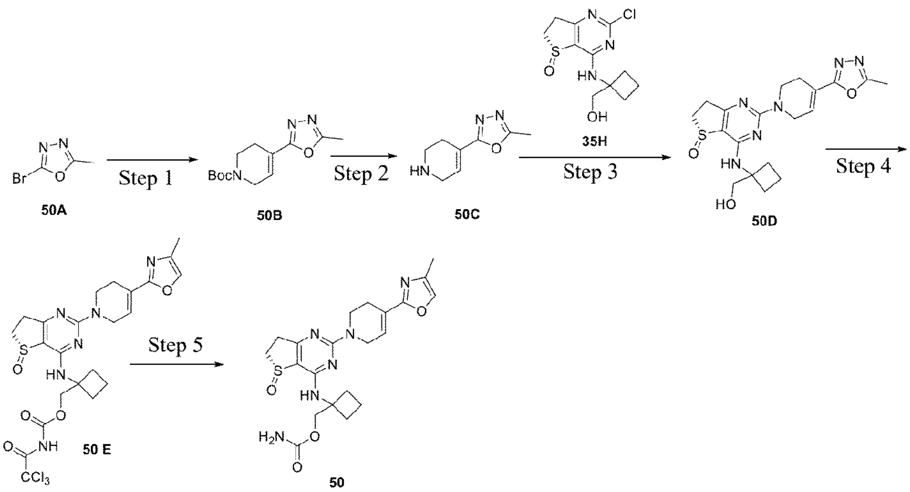


35H

50A 50B 50C 50D

50 E 50

Using compounds **50A** and **9B** as raw materials, compound **50** (20 mg, 26%) was obtained according to the operation of Example 50.

LC-MS (ESI): m/z = 459.2 [M+H]$^+$.

$^1$H NMR (400 MHz, DMSO-$d_6$) δ 7.75 (d, 1H), 7.63 (s, 1H), 6.76 - 6.63 (m, 1H), 6.48 (s, 2H), 4.42 - 4.34 (m, 4H), 3.94 (t, 2H), 3.49 - 3.38(m, 1H), 3.26 - 3.16 (m, 1H), 3.03 - 2.82 (m, 2H), 2.54 (s, 2H), 2.45 - 2.18 (m, 4H), 2.09 (d, 3H), 1.91 - 1.77 (m, 2H).

**Example 52**

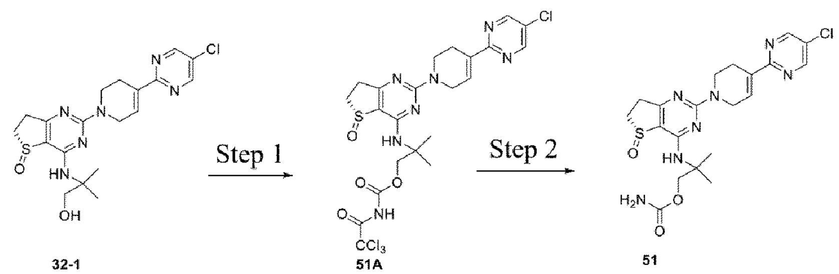


32-1 51A 51

Step 1: Compound **32-1** (100 mg, 0.23 mmol) was dissolved in dichloromethane (5 mL), and trichloroacetyl isocyanate (52 mg, 0.28 mmol) was added in an ice bath and stirred in the ice bath for one hour. The reaction liquid was concentrated to obtain compound **51A** (110 mg, 70%).

Step 2: Compound **51A** (110 mg, 0.18 mmol) was dissolved in methanol (5 mL), and potassium carbonate (73 mg, 0.53 mmol) and water (5 mL) were added in an ice bath and stirred at room temperature 2.5 hours. The reaction liquid was diluted by adding water and extracted with dichloromethane, and the organic phases were combined, dried, filtered, concentrated, and subjected to silica gel column chromatography (dichloromethane:methanol = 10:1) to obtain compound **51** (40 mg, 46%).

LC-MS (ESI): m/z = 478.1 $[M+H]^+$.

$^1$H NMR (400 MHz, DMSO-$d_6$) δ 8.89 (s, 2H), 7.29 (s, 1H), 6.70 - 6.40 (m, 3H), 4.50 (s, 2H), 4.30 (s, 2H), 4.01 (t, 2H), 3.49 - 3.37 (m, 1H), 3.30 - 3.23 (m, 1H), 3.02 -2.92(m, 1H), 2.91 -2.82 (m, 1H), 2.67 (s, 2H), 1.46 (d, 6H).

## Example 53

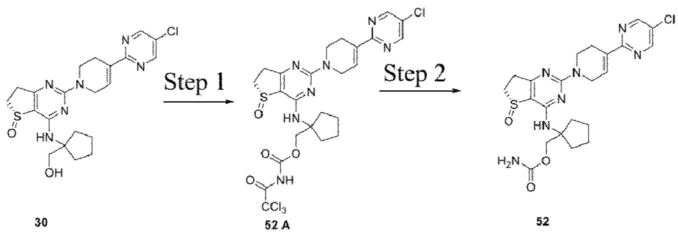


30 52 A 52

Step 1: Compound **30** (100 mg, 0.22 mmol) was dissolved in dichloromethane (5 mL), and trichloroacetyl isocyanate (52 mg, 0.28 mmol) was added in an ice bath and stirred in the ice bath for one hour. The reaction liquid was concentrated to obtain compound **52A** (110 mg, 77%).

Step 2: Compound **52A** (110 mg, 0.18 mmol) was dissolved in methanol (5 mL), and potassium carbonate (73 mg, 0.53 mmol) and water (5 mL) were added in an ice bath and stirred at room temperature 2.5 hours. The reaction liquid was diluted by adding water and extracted with dichloromethane, and the organic phases were combined, dried, filtered, concentrated, and subjected to silica gel column chromatography (dichloromethane:methanol = 10:1) to obtain compound **52** (40 mg, 44%).

LC-MS (ESI): m/z = 504.1 $[M+H]^+$.

$^1$H NMR (400 MHz, DMSO-$d_6$) δ 8.90 (s, 2H), 7.34 - 7.20 (m, 1H), 6.84 (s, 1H), 6.47 (s, 2H), 4.49 (d, 2H), 4.46 - 4.31 (m, 2H), 4.00 (t, 2H), 3.48 - 3.38 (m, 1H), 3.29 - 3.20 (m, 1H), 3.02 - 2.84 (m, 2H), 2.66 (s, 2H), 2.25 - 2.10 (m, 2H), 1.95 - 1.79 (m, 2H), 1.70 - 1.56 (m, 4H).

## Example 54

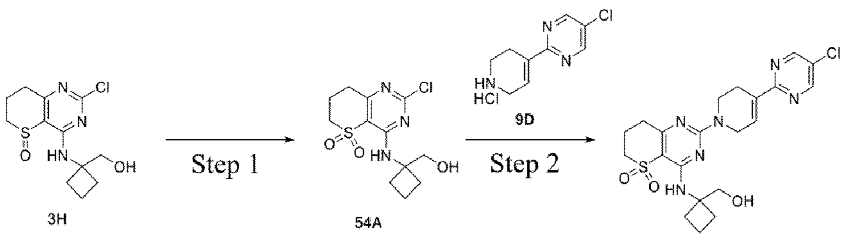


3H 54A 9D

**Compound 54**

Step 1: To a 50 mL single-mouth flask, compound **3H** (500 mg, 1.66 mmol), dichloromethane (10 mL), m-chloroperoxybenzoic acid (342 mg, 1.99 mmol), and N,N-dimethylformamide (100 mL) were successively added. After reaction at room temperature for 2 hours, dichloromethane (20 mL) was added, and the mixture was washed with water (30 mL × 2) and with a saturated sodium bicarbonate aqueous solution (30 mL × 1). The organic layer was dried over anhydrous sodium sulfate. After filtration, the filtrate was concentrated under reduced pressure to obtain **54B** (480 mg, yield 91.2%).

LC-MS (ESI): m/z = 318.1 $[M+H]^+$.

Step 2: To a 50 mL single-mouth flask, **54A** (240 mg, 0.75 mmol), **9D** (173 mg, 0.75 mmol), dioxane ( 10 mL), and DIPEA (484 mg, 3.75 mmol) were successively added and reacted at 100°C for 12 hours. After cooling to room temperature, the reaction liquid was directly concentrated under reduced pressure and then separated and purified

by silica gel column chromatography (eluents: dichloromethane/methanol (v/v) = 100/8) to obtain the title compound **54** (42 mg, yield 11.7%).

$^1$H NMR (400 MHz, DMSO-$d_6$) δ 8.89 (s, 2H), 7.28 (s, 1H), 6.73 (s, 1H), 5.01 - 4.90 (m, 1H), 4.44 (s, 2H), 4.02 - 3.86 (m, 2H), 3.77 - 3.66 (m, 2H), 3.47 - 3.38 (m, 2H), 2.77 - 2.69 (m, 2H), 2.64 (s, 2H), 2.41 - 2.29 (m, 2H), 2.26 - 2.11 (m, 4H), 1.94 - 1.72 (m, 2H).

LC-MS (ESI): m/z = 477.1 $[M+H]^+$.

**Example 55**

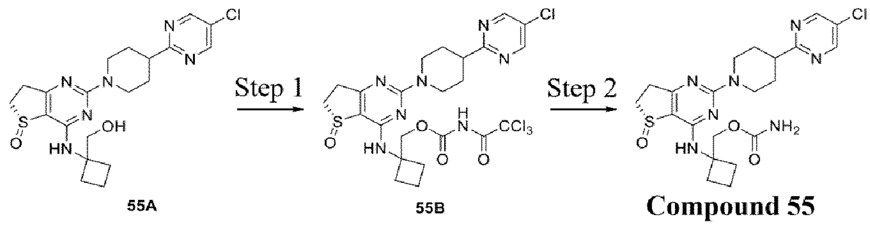


55A 55B Compound 55

Step 1: Under nitrogen protection, compound **55A** (100 mg, 0.22 mmol, see patent WO 2014124860 A1 for the synthesis) was dissolved in dichloromethane (10 mL), and trichloroacetyl isocyanate (50 mg, 0.27 mmol) was slowly dropwise added in an ice bath under stirring. After the addition was complete, the reaction liquid was stirred at 0°C for 1 h. After the raw material disappeared as detected by a spotting plate (developing agent: DCM/MeOH = 10:1), the reaction liquid was concentrated under reduced pressure to obtain crude product **55B** (160 mg), which was directly used in the next reaction.

Step 2: Crude compound **55B** (140 mg, 0.22 mmol) was dissolved in methanol (6 mL), and potassium carbonate (90 mg, 0.66 mmol) was then added at room temperature. After the addition was complete, the mixture was stirred at room temperature 1.5 h under nitrogen protection. After the reaction was complete, the reaction liquid was concentrated under reduced pressure to obtain a crude product, and the crude product was purified by column chromatography (eluents: DCM:MeOH = 100:1 to 9:1), followed by further purification through a reverse phase column (eluents: water:acetonitrile = 100:1 to 1:9) to obtain the target compound **55** (95 mg, 87.91%).

LCMS m/z = 492.10 $[M+H]^+$

$^1$H NMR (400 MHz, DMSO-d6) δ 8.86 (s, 2H), 7.59 (s, 1H), 6.48 (s, 2H), 4.72 - 4.69 (m, 2H), 4.38 - 4.32 (m, 2H), 3.46 - 3.40 (m, 1H), 3.25 - 3.16 (m, 2H), 3.10 - 3.04 (m, 2H), 2.97 - 2.85 (m, 2H), 2.40 - 2.27 (m, 2H), 2.22 - 2.19 (m, 2H), 1.98 - 1.95 (m, 2H), 1.89 - 1.80 (m, 2H), 1.68 - 1.60 (m, 2H).

**Example 56**

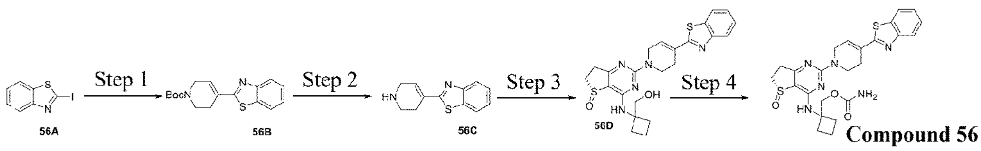


56A 56B 56C 56D Compound 56

Using **56A** and **9B** as raw materials, compound **56** (5.2 mg, 6%) was obtained according to the operation of Example 50.

LCMS (ESI): m/z = 511.0 $[M+H]^+$

$^1$H NMR (400 MHz, DMSO-d6) δ 8.10 - 8.03 (m, 1H), 7.99 - 7.92 (m, 1H), 7.65 (s, 1H), 7.53 - 7.38 (m, 2H), 6.90 (s, 1H), 6.49 (s, 2H), 4.48 - 4.40 (m, 4H), 4.10 - 3.98 (m, 2H), 3.45-3.40 (m, 1H), 3.28 - 3.18 (m, 1H), 3.03 - 2.93 (m, 1H), 2.91 - 2.85 (m, 1H), 2.76 (s, 2H), 2.39 - 2.29 (m, 4H), 1.88-1.82 (m, 2H).

**Example 57**

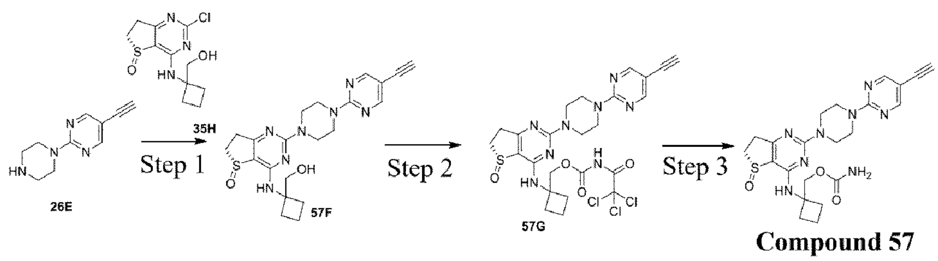

Compound 57

Using compounds **26E** and **35H** as raw materials, compound **57** (20 mg, 43%) was obtained according to the operation of Example 49.
LCMS m/z = 483.20 $[M+H]^+$
H NMR (400 MHZ, DMSO-d6) δ 8.50 (s, 2H), 7.66 (s, 1H), 6.49 (s, 2H), 4.32 - 4.40 (m, 2H), 4.28 (s, 1H), 3.83 (s, 8H), 3.40 - 3.48 (m, 1H), 3.18 - 3.26 (m, 1H), 2.84 - 2.99 (m, 2H), 2.21 - 2.41 (m, 4H), 1.76 - 1.88 (m, 2H).

**Example 58**

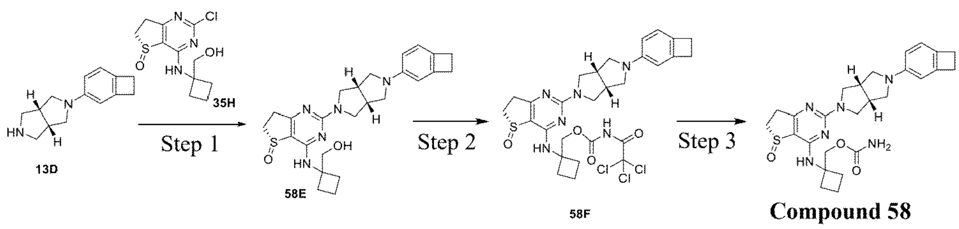

Compound 58

Using compounds **13D** and **35H** as raw materials, compound **58** (20 mg, 17%) was obtained according to the operation of Example 49.
LCMS m/z = 509.20 $[M+H]^+$
H NMR (400 MHZ, DMSO-d6) δ 7.50 (s, 1H), 6.85 (d, 1H), 6.47 (s, 2H), 6.34 - 6.36 (m, 2H), 4.38 (s, 2H), 3.74 - 3.82 (m, 2H), 3.40 - 3.42 (m, 4H), 3.01 - 3.21 (m, 9H), 2.83 - 2.95 (m, 2H), 2.27 - 2.47 (m, 2H), 2.16 - 2.23 (m, 2H), 1.76 - 1.84 (m, 3H).

**Example 59**

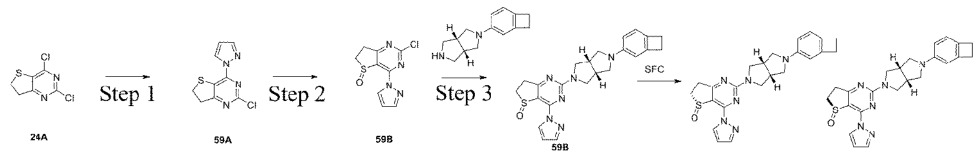

Compound 59-1 and compound 59-2

Using compound **24A** and parazole as raw materials, compound **59-1** (30 mg) and compound **59-2**(50 mg) were obtained according to the operation of Example 41.
Chiral resolution method: instrument: Waters 150 SFC; column: Chiralpak IC Column (250 * 30 mm, I.D 30 mm, 10 um particle size); mobile phase: A for CO2 and B for IPA + ACN (0.1% $NH_3{\cdot}H_2O$); gradient: 45% phase B isocratic elution; flow rate: 100 mL/min; back pressure: 100 bar; column temperature: 25°C; wavelength: 220 nm; elution time: 9.3 min.
LCMS m/z = 433.2 $[M +1]^+$
**Compound 59-1,** retention time 2.01 min, $^1H$ NMR (400 MHZ, DMSO-d6) δ 8.75 (s, 1H), 8.02 (s, 1H), 6.85 (d, 1H), 6.68 (d, 1H), 6.36 - 6.38 (m, 2H), 3.86 - 4.01 (m, 2H), 3.55 - 3.71 (m, 3H), 3.40 - 3.50 (m, 2H), 3.11 - 3.26 (m, 6H), 2.97 - 3.05 (m, 5H).
**Compound 59-2,** retention time 2.48 min, $^1H$ NMR (400 MHZ, DMSO-d6) δ 8.76 (s, 1H), 8.02 (s, 1H), 6.85 (d, 1H), 6.67 (d, 1H), 6.36 - 6.38 (m, 2H), 3.86 - 4.01 (m, 2H), 3.55 - 3.71 (m, 3H), 3.41 - 3.49 (m, 2H), 3.11 - 3.24 (m, 6H), 2.97 -

3.05 (m, 5H).

**Example 60**

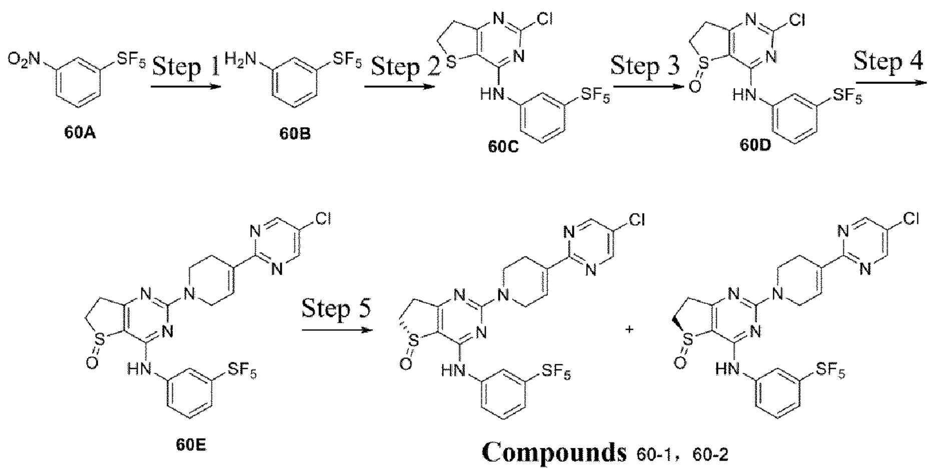


60A 60B 60C 60D 60E

**Compounds** 60-1, 60-2

Step 1: At room temperature, **60A** (5.00 g, 20.07 mmol) was dissolved in ethanol (100 mL), iron powder (6.72 g, 120.41 mmol) was added in portions, and concentrated hydrochloric acid (2.12 g, 20.37 mmol) was then slowly dropwise added. After the addition was complete, the reaction liquid was stirred at room temperature for 4 h under nitrogen protection. After the reaction was complete as monitored by TLC, the reaction was quenched by adding aqueous ammonia, and the system was filtered to obtain a filtrate. Water (100 mL) was added, and the system was diluted and extracted three times with dichloromethane (100 mL). The organic phases were combined, dried over anhydrous sodium sulfate, and then concentrated in vacuum to obtain a crude product, and the crude product was purified by column chromatography (eluents: PE:EA = 100:1 to 4:1) to obtain the target compound **60**B (3.00 g, yield: 68.21%).
LC-MS (ESI): m/z = 220.20 $[M+H]^+$.
$^1$H NMR (400 MHz, $CDCl_3$) δ 7.22 - 7.18 (m, 1H), 7.12 - 7.09 (m, 1H), 7.04 - 7.03 (m, 1H), 6.78 - 6.75 (m, 1H), 3.86 (br s, 2H).
Using compounds **60B** and **24A** as raw materials, compound **60-1** (100 mg) and compound 60-2(160 mg) were obtained according to the operation of Example 37.
Analysis method: instrument: SHIMADZU LC-20AD, column: Chiralpak IG-3 50 × 4.6 mm I.D., 3 μm; mobile phase: A: heptane (0.05%, N,N-diethylaniline), B: ethanol (0.05% N,N-diethylaniline); gradient: 20% B in A; flow rate: 1 mL/min, column temperature: 35°C, wavelength: 254 nm.
**Preparation method:** instrument: SHIMADZU LC-20AP, column: DAICEL CHIRALPAK IG (250 mm × 30 mm, 10 μm), mobile phase: A n-hexane, B ethanol (0.1% $NH_3$•$H_2O$); gradient: 8% B gradient elution, flow rate: 110 mL/min, column temperature: 25°C, wavelength: 254 nm, cycle time: 16 min, sample preparation: sample concentration 1.5 mg/ml, ethanol solution injection: 2 ml each time. After separation, the fraction was dried by a rotary evaporator at a bath temperature of 40°C to obtain compound **60-1** (retention time: 5.716 minutes) and compound **60-2** (retention time: 6.890 minutes).

**Compound 60-1:**

LCMS m/z = 565.5 $[M+H]^+$
$^1$H NMR (400 MHz, DMSO-d6) δ 9.98 (s, 1H), 8.90 (s, 2H), 8.60 (s, 1H), 7.98 - 7.90 (m, 1H), 7.63 - 7.60 (m, 2H), 7.31 - 7.24 (m, 1H), 4.54 - 4.42 (m, 2H), 4.08 - 3.96 (m, 2H), 3.62 - 3.54 (m, 1H), 3.36 - 3.28 (m, 1H), 3.15 - 3.09 (m, 1H), 3.04 - 2.99 (m, 1H), 2.74 - 2.65 (m, 2H).

**Compound 60-2:**

LCMS m/z = 565.5 $[M+H]^+$
$^1$H NMR (400 MHz, DMSO-d6) δ 9.98 (s, 1H), 8.90 (s, 2H), 8.60 (s, 1H), 7.98 - 7.90 (m, 1H), 7.63 - 7.59 (m, 2H), 7.31 - 7.24 (m, 1H), 4.55 - 4.41 (m, 2H), 4.08 - 3.96 (m, 2H), 3.62 - 3.54 (m, 1H), 3.36 - 3.28 (m, 1H), 3.15 - 3.09 (m, 1H), 3.04 - 2.99 (m, 1H), 2.75 - 2.66 (m, 2H).

**Example 61**

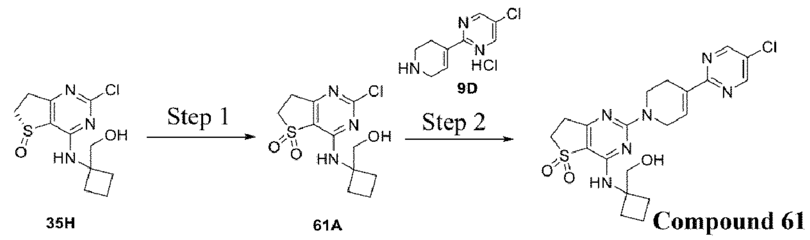


Step 1: To a 100 mL single-mouth flask, **35H** (1.0 g, 3.47 mmol) was added and dissolved with dichloromethane (20 mL), and m-chloroperoxybenzoic acid (1.2 g, 6.94 mmol) was added and stirred at room temperature overnight. The reaction liquid was washed with a sodium hydroxide aqueous solution (5%, 20 mL × 2), dried over anhydrous sodium sulfate, and concentrated to obtain the title compound **61A** (1.0 g, 95%).
LC-MS (ESI): m/z = 304.1 $[M+H]^+$.
Step 2: To a 50 mL single-mouth flask, **61A** (0.2 g, 0.66 mmol) was added and dissolved with dioxane (10 mL), and **9D** (0.18 g, 0.79mmol) and N,N-diisopropylethylamine (0.26 g, 1.98 mmol) were successively added and stirred at 95 degrees Celsius overnight. The reaction liquid was concentrated, slurried with methanol (10 mL) for 0.5 h, suction-filtrated, and dried to obtain the target compound **61** (0.25 g, 82%).
LC-MS (ESI): m/z = 463.1 $[M+H]^+$.
$^1$H NMR (400 MHz, DMSO-d6) $\delta$ 8.90 (s, 2H), 7.30 - 7.28 (m, 1H), 6.12 (s, 1H), 5.02 (t, 1H), 4.49 (s, 2H), 4.00 (t, 2H), 3.71 (d, 2H), 3.49 - 3.46 (m, 2H), 3.10 - 3.06 (m, 2H), 2.67 (s, 2H), 2.53 - 2.51 (m, 2H), 2.20 - 2.06 (m, 2H), 1.89 - 1.74 (m, 2H).

**Example 62**

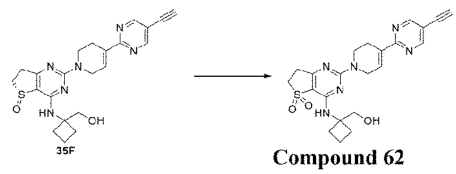

Step 1: Compound **35F** (860.0 mg, 2.0 mmol) was dissolved in dichloromethane (10 mL), m-chloroperoxybenzoic acid (346.0 mg, 2.0 mmol) was then added and reacted at room temperature for 4 h, and the system was quenched by adding a small amount of sodium bicarbonate, concentrated, and subjected to column chromatography (dichloromethane/methanol = 10/1) to obtain compound **62** (120.0 mg, 12%).
LC-MS (ESI): m/z = 453.1 $[M+H]^+$.
$^1$H NMR (400 MHz, DMSO-*d6*) $\delta$ 8.91 (s, 2H), 7.34 (s, 1H), 6.12 (s, 1H), 5.02 (s, 1H), 4.70 (s, 1H), 4.51 (s, 2H), 4.00 (d, 2H), 3.71 (d, 2H), 3.48 (t, 2H), 3.08 (t, 2H), 2.68 (s, 2H), 2.53 (s, 2H), 2.14 (t, 2H), 1.97 - 1.68 (m, 2H).

**Example 63**

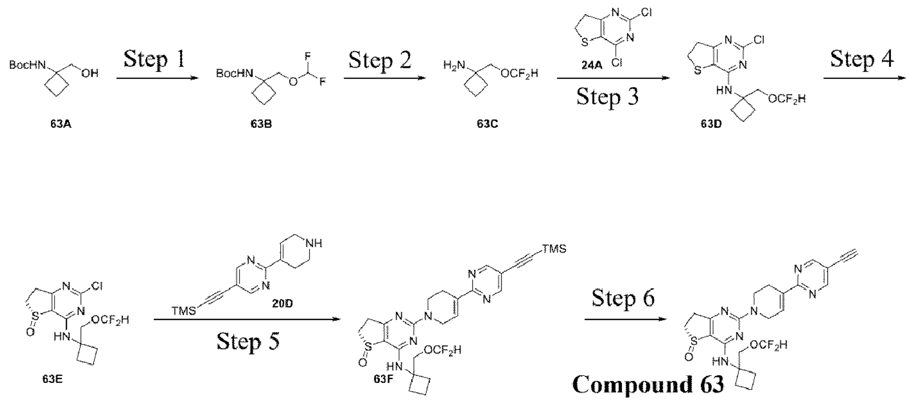


Step 1: Compound **63A** (2.0 g, 10.0 mmol) was dissolved in dichloromethane (5 mL) and water (5 mL), potassium bifluoride (1.6 g, 20.0 mmol) was then added, and difluorobromomethyl trimethylsilane (2.0 g, 10.0 mmol) was finally added. The mixture was reacted at room temperature overnight, extracted with dichloromethane, dried, filtered, concentrated, and subjected to column chromatography (petroleum ether/ethyl acetate = 10/1) to obtain compound **63B** (2.0 g, 81%).

LC-MS (ESI): m/z = 252.1 $[M+H]^+$.

Step 2: Compound **63B** (0.8 g, 3.1 mmol) was dissolved in dichloromethane (10 mL), trifluoroacetic acid (5.0 mL) was then added, and the mixture was reacted at room temperature for half an hour and then concentrated to obtain compound **63C** (1.0 g, 99%), which was directly used for the next step.

LC-MS (ESI): m/z = 152.1 $[M+H]^+$.

Using compounds **63C** and **24A** as raw materials, compound **63** (35.0 mg, 28%) was obtained according to the operation of Example 34.

LC-MS (ESI): m/z = 487.2 $[M+1]^+$

$^1$H NMR (400 MHz, DMSO-*d6*) δ 8.90 (s, 2H), 7.82 (s, 1H), 7.34 (s, 1H), 6.70 (t, 1H), 4.69 (s, 1H), 4.46 (s, 2H), 4.27 (q, 2H), 3.96 (s, 2H), 3.54 - 3.36 (m, 1H), 3.24 - 3.17 (m, 1H), 3.00 - 2.85 (m, 2H), 2.66 (s, 2H), 2.45 - 2.18 (m, 4H), 1.90 - 1.84 (m, 2H).

$^{19}$F NMR (377 MHz, DMSO-*d6*) δ -80.12 (s).

**Example 64**

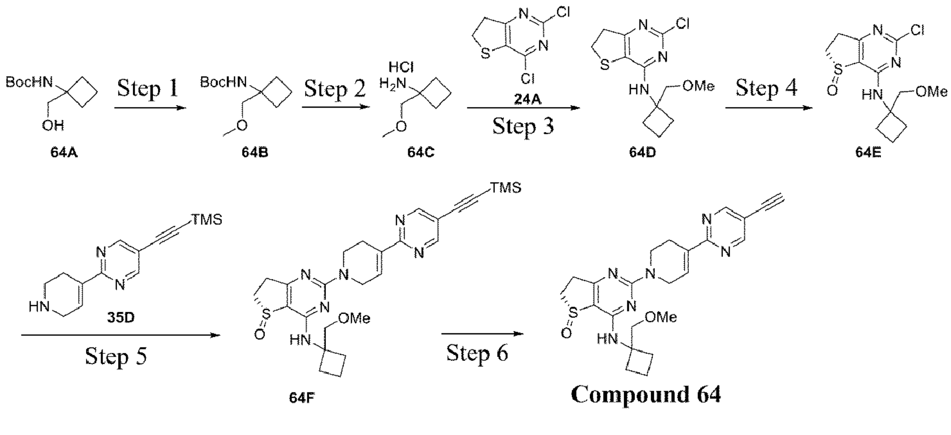


Step 1: Compound **64A** (5.0 g, 24.8 mmol) was dissolved in tetrahydrofuran (50 ml), sodium hydride (1.1 g, 27.5 mmol) was then added and stirred at room temperature for 1 h, and iodomethane (3.9 g, 27.5 mmol) was added and reacted for 12 h. The system was passed through a short silica gel column, eluted with ethyl acetate, concentrated, and subjected to column chromatography (ethyl acetate/petroleum ether = 1/4) to obtain compound **64B** (2.0 g, 38%).

Step 2: Compound **64B** (2.0 g, 9.3 mmol) was dissolved in dichloromethane (30 mL), a hydrogen chloride dioxane solution (6.9 mL, 27.9 mmol) was then added and reacted at room temperature for half an hour, and the system was concentrated to obtain compound **64C** (1.0 g, 92%), which was directly used for the next step.

LC-MS (ESI): m/z = 116.2 $[M+H]^+$

Using compounds **64C** and **24A** as raw materials, compound **64** (10.0 mg, 5.3%) was obtained according to the operation of Example 34.

LC-MS (ESI): m/z = 451.2 [M+1]$^+$

$^1$H NMR (400 MHz, DMSO-*d6*) δ 8.90 (s, 2H), 7.64 (s, 1H), 7.34 (s, 1H), 4.68 (s, 1H), 4.46 (s, 2H), 3.97 (q, 2H), 3.78 - 3.71 (m, 2H), 3.49 - 3.39 (m, 1H), 3.29 (s, 3H), 3.25 - 3.16 (m, 1H), 3.04 - 2.85 (m, 2H), 2.66 (s, 2H), 2.36 - 2.20 (m, 4H), 1.88 - 1.82 (m, 2H).

**Example 65**

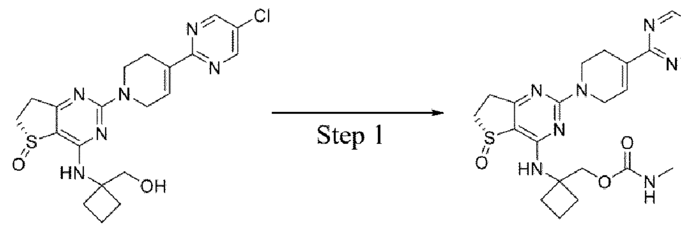


48D

Compound 65

Step 1: To a 100 mL single-mouth flask, compound **48D** (300 mg, 0.67 mmol) was added and dissolved with N,N-dimethylformamide (10 mL), and triethylamine (0.20 g, 2.02 mmol) and methylaminoformyl chloride (94 mg, 1.01 mmol) were added. After the addition was complete, the system was placed under nitrogen protection and stirred at 50°C for 16 h. After the reaction was complete as monitored by TLC (dichloromethane:methanol = 10:1), the reaction liquid was concentrated under reduced pressure, and the crude product was subjected to preparative HPLC to obtain the title compound **65** (200 mg, 66%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min; retention time: 10.50 min.

LCMS m/z = 504.2 [M +1]$^+$

$^1$H NMR (400 MHZ, DMSO-*d6*) δ 8.89 (s, 2H), 7.60 (s, 1H), 7.29 (s, 1H), 6.95 (d, 1H), 4.46 (s, 2H), 4.43 (s, 2H), 3.97 (t, 2H), 3.40-3.48 (m, 1H), 3.18-3.25 (m, 1H), 2.85-2.96 (m, 2H), 2.65 (s, 2H), 2.56 (d, 3H), 2.20 - 2.45 (m, 3H), 1.90 - 1.80 (m, 3H).

**Example 66**

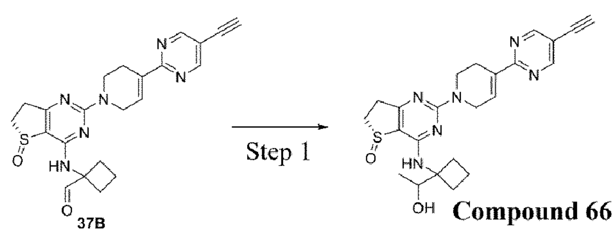


37B

Compound 66

Step 1: Compound **37B** (200 mg, 0.46 mmol) was dissolved in anhydrous tetrahydrofuran (10 mL), and methylmagnesium bromide (0.46 mL, 1.38 mmol, 3.0 mol/L in diethyl ether) was dropwise added in an ice bath under nitrogen protection. After the addition was complete, the mixture was reacted in the ice bath for one hour. After quenching by saturated ammonium chloride, the system was extracted with ethyl acetate, the organic phase was dried, filtered, and concentrated, and the crude product was subjected to reverse phase preparation (acetonitrile:water = 1/99-99/1) to obtain compound **66** (6 mg, 3%).

LC-MS (ESI): m/z = 451.2 [M+H]$^+$。

$^1$H NMR (400 MHz, DMSO-$d_6$) δ 8.90 (s, 2H), 7.38 - 7.29 (m, 2H), 4.92 (d, 1H), 4.69 (s, 1H), 4.53 - 4.41 (m, 2H), 4.30 - 4.16 (m, 1H), 4.07 - 3.97 (m, 1H), 3.97 - 3.87 (m, 1H), 3.48 - 3.37 (m, 1H), 3.27 - 3.22 (m, 1H), 3.00 - 2.91 (m, 1H), 2.90

- 2.82 (m, 1H), 2.69 - 2.62 (m, 2H), 2.43 - 2.32 (m, 3H), 2.28 - 2.21 (m, 1H), 1.84 - 1.69 (m, 2H), 1.00 (d, 3H).

**Example 67**

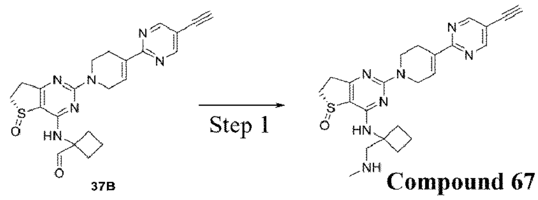


37B

Compound 67

Step 1: Compound **37B** (300 mg, 0.69 mmol) was dissolved in 1,2-dichloroethane (10 mL) and acetic acid (10 mL), a methylamine solution (6.9 mL, 6.9 mmol, 3.0 mol/L in THF) was added in an ice bath, and after the addition was complete, the mixture was stirred at room temperature for 1 hour. Sodium triacetoxyborohydride (731 mg, 3.45 mmol) was then added and reacted at room temperature for 1 hour. After the reaction was complete, the system was quenched by saturated ammonium chloride and extracted with dichloromethane, the organic phase was dried, filtered, and concentrated, and the crude product was subjected to reverse phase preparation (acetonitrile:water = 1/99-99/1) to obtain compound **67** (25 mg, 8%).

LC-MS (ESI): m/z = 450.1 $[M+H]^+$.

$^1H$ NMR (400 MHz, DMSO-$d_6$) δ 8.91 (s, 2H), 8.43 (s, 1H), 7.65 (s, 1H), 7.42 - 7.28 (m, 1H), 4.69 (s, 1H), 4.60 - 4.35 (s, 2H), 4.04 - 3.88 (s, 2H), 3.64 - 3.40 (m, 3H), 3.23 - 3.14 (m, 1H), 3.05 - 2.85 (m, 2H), 2.68 (s, 2H), 2.56 (s, 3H), 2.47 - 2.25 (m, 4H), 1.93 - 1.79 (m, 2H).

**Example 68**

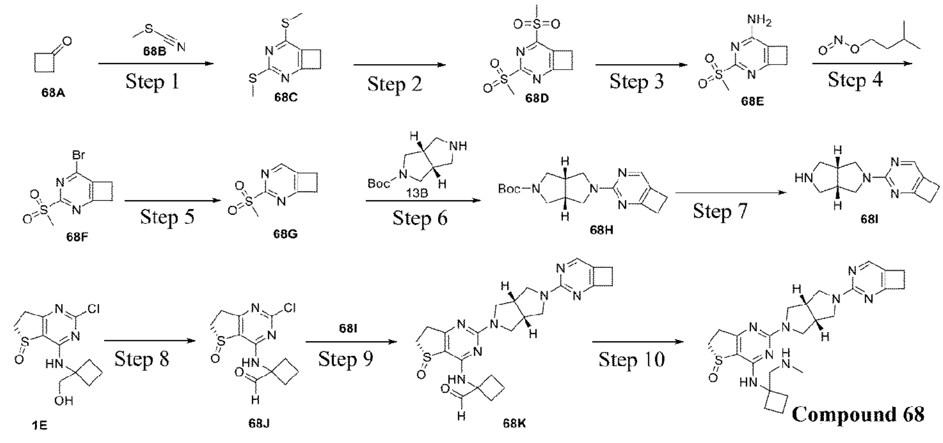


68A, 68C, 68D, 68E, 68F, 68G, 68H, 68I, 1E, 68J, 68K, Compound 68

Step 1: To a 3 L three-mouth flask, methyl thiocyanate **(68B,** 132 g, 1.80 mol) was added and dissolved with 1,2-dichloroethane (1.20 L), trifluoromethanesulfonic anhydride (266 g, 0.940 mol) was added, and cyclobutanone **(68A,** 60.0 g, 0.860 mol) was slowly dropwise added at room temperature. After the addition was complete, the mixture was stirred at room temperature overnight. The reaction liquid was adjusted to pH 8-9 by adding a saturated sodium bicarbonate solution and left to stand for layering. An aqueous phase and an organic phase were separated. The organic phase was dried over anhydrous sodium sulfate, filtered, and concentrated, and the crude product was purified by silica gel column chromatography (dichloromethane/ethyl acetate (v/v) = 99/1-95/5), followed by recrystallization (ethyl acetate/petroleum ether (v/v) = 1/2) to obtain compound **68C** (15.0 g, 9%).

LC-MS (ESI): m/z = 199.1 $[M+H]^+$

Step 2: To a 500 mL single-mouth flask, **68C** (15.0 g, 75.6 mmol) was added and dissolved with dichloromethane (300 mL), and m-chloroperoxybenzoic acid (78.3 g, 454 mmol) was added and stirred at room temperature overnight. The reaction liquid was washed with a 10% sodium hydroxide aqueous solution (100 mL), and the organic phase was dried over anhydrous sodium sulfate, filtered, concentrated, and purified by column chromatography (petroleum ether/ethyl

acetate (v/v) = 50/50-0/100) to obtain compound **68D** (3.8 g, 19%).

LC-MS (ESI): m/z = 263.1 $[M+H]^+$

Step 3: To a 100 mL single-mouth flask, **68D** (3.8 g, 14.5 mmol) was added and dissolved with dichloromethane (40 mL), and aqueous ammonia (20 mL) was added and stirred at room temperature for 4 h. The reaction liquid was left to stand for direct phase separation. The aqueous phase was extracted once with dichloromethane (20 mL). The organic phases were combined, dried over anhydrous sodium sulfate, filtered, and concentrated, and the crude product was purified by column chromatography (dichloromethane/ethyl acetate (v/v) = 67/33-0/100) to obtain the target compound **68E** (2.2 g, 76%).

LC-MS (ESI): m/z = 200.1 $[M+H]^+$

Step 4: To a 100 mL single-mouth flask, **68E** (2.2 g,11.0 mmol) was added and dissolved with acetonitrile (40 ml), isoamyl nitrite (3.9 g, 33.1 mmol) and copper bromide (4.9 g, 22.1 mmol) were added, and the mixture was heated to 75°C and stirred for 6 h under nitrogen protection. After cooling to room temperature, the reaction liquid was directly concentrated, and the crude product was added to water (100 mL) and extracted with dichloromethane (2 × 100 mL). The organic phases were combined, dried over anhydrous sodium sulfate, filtered, and concentrated, and the crude product was purified by column chromatography (petroleum ether/ethyl acetate (v/v) = 75/25-50/50) to obtain the target compound **68F** (2.0 g, 69%).

LC-MS (ESI): m/z = 263.0 $[M+H]^+$

Step 5: To a 100 mL single-mouth flask, **68F** (2.0 g, 7.6 mmol) was added and dissolved with ethyl acetate (40 mL), palladium on carbon (10%, 6.0 g) was added, and the mixture was heated to 60°C and stirred for 72 h. After cooling to room temperature, the reaction liquid was filtered through diatomite and washed clean with ethyl acetate, and the filtrate was concentrated and purified by column chromatography (dichloromethane/ethyl acetate (v/v) = 67/33-0/100) to obtain the target compound **68G** (0.48 g, 34%).

LC-MS (ESI): m/z = 185.1 $[M+H]^+$

Step 6: To a 50 mL single-mouth flask, **68G** (0.18 g, 0.98 mmol) was added and dissolved with dioxane (8 mL), **13B** (0.42 g, 1.96 mmol) and N,N-diisopropylethylamine (0.38 g, 2.94 mmol) were added, and the mixture was heated to 95°C and stirred overnight. After cooling to room temperature, the reaction liquid was concentrated and purified by column chromatography (dichloromethane/ethyl acetate (v/v) = 80/20-50/50) to obtain the target compound **68H** (60 mg, 19%).

LC-MS (ESI): m/z = 317.2 $[M+H]^+$

Step 7: To a 50 mL single-mouth flask, **68H** (60 mg, 0.19 mmol) was added and dissolved with dioxane (3 mL), and a hydrogen chloride-dioxane solution (3 mL, 4 N) was added and stirred at room temperature for 4 h. The reaction liquid was concentrated to obtain a crude product of the target compound **68I** hydrochloride (48 mg), which was directly used in the next reaction.

LC-MS (ESI): m/z = 217.2 $[M+H]^+$

Step 8: Compound **35H** (1.0 g, 3.48 mmol) was dissolved in ethyl acetate (15 ml), 2-iodoxybenzoic acid (2.93 g, 10.4 mmol) was added, and the mixture was stirred in an oil bath at 80°C overnight. After the reaction was complete, the system was cooled to room temperature, filtered, concentrated, and quickly separated and purified by column chromatography (petroleum ether/ethyl acetate (v/v) = 100/0-80/20) to obtain compound **68J** (0.9 g, 82%).

LC-MS (ESI): m/z = 286.1 $[M+H]^+$

Step 9: To a 50 mL single-mouth flask, **68J** (50 mg, 0.17 mmol) was added and dissolved with dioxane (6 mL), and crude **68I** hydrochloride (44 mg, 0.20 mmol) and N,N-diisopropylethylamine (66 mg, 0.51 mmol) were successively added, heated to 95°C, and stirred overnight. After cooling to room temperature, the reaction liquid was concentrated and purified by column chromatography (dichloromethane/ethyl acetate (v/v) = 95/5-90/10) to obtain the target compound **68K** (70 mg, 88%).

LC-MS (ESI): m/z = 466.1$[M+H]^+$

Step 10: To a 50 mL single-mouth flask, **68K** (50 mg, 0.11 mmol) was added and dissolved with tetrahydrofuran (5 mL) and methanol (2 mL), methylamine (0.5 mL, 2 N in THF) was added, and a drop of acetic acid was dripped. The mixture was stirred at room temperature for 1 h, and sodium triacetylborohydride (70 mg, 0.33 mmol) was added and stirred at room temperature overnight. The reaction liquid was concentrated and purified by HPLC to obtain the title compound **68** (3 mg, 6%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM ammonium acetate); b. gradient elution, mobile phase A with a content of 10-60%; c. flow: 15 mL/min; d. elution time: 20 min; retention time: 11.5 min.

$^1$H NMR (400 MHz, DMSO-*d*6) δ 7.90 (s, 1H), 7.30 (s, 1H), 3.80 - 3.70 (m, 6H), 3.43 - 3.35 (m, 4H), 3.25-3.19 (m, 4H), 3.22 - 2.94 (m, 5H), 2.92 - 2.80 (m, 2H), 2.45 - 2.41 (m, 2H), 2.31 (s, 3H), 2.15 - 2.09 (m, 2H), 1.84 - 1.68 (m, 2H).

LC-MS (ESI): m/z = 481.5 $[M+H]^+$

## Example 69

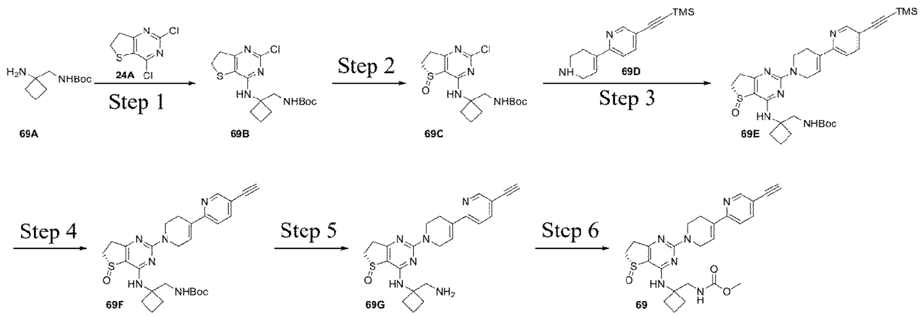


69A 24A 69B 69C 69D 69E 69F 69G 69

Step 1: Compound **69A** (7.7 g, 38.6 mmol) was dissolved in acetonitrile (20 mL), compound **24A** (8.0 g, 38.6 mmol) and triethylamine (19.5 g, 193.0 mmol) were then added, and the mixture was heated to 75°C, reacted for 12 h, then cooled to room temperature, concentrated, and subjected to column chromatography (dichloromethane/methanol (v/v) = 93/7) to obtain compound **69B** (5.5 g, 38%).

LC-MS (ESI) m/z = 371.1 $[M+H]^+$

Step 2: Compound **69B** (2.7 g, 7.28 mmol), (S)-(-)-1,1'-bi-2-naphthol (0.21 g, 0.73 mmol), titanium tetraisopropoxide (0.1 g, 0.36 mmol), and water (0.13 g, 7.28 mmol) were dissolved in dichloromethane (20 mL). After nitrogen displacement three times, the system was stirred at room temperature for one hour, and tert-butyl hydroperoxide (0.72 mg, 8.01 mmol) was added and stirred for 1.5 hours. After quenching by adding a saturated sodium thiosulfate aqueous solution, the aqueous phase was extracted with dichloromethane, dried, concentrated, and subjected to column chromatography (dichloromethane/methanol (v/v) = 90/10) to obtain compound **69C** (3.0 g, 100%).

LC-MS (ESI): m/z = 387.1 $[M+H]^+$.

Step 3: Compound **69C** (1.6 g, 4.14 mmol) was dissolved in 1,4-dioxane (15.0 mL), compound **69D** (1.27 g, 4.97 mmol) and diisopropylethylamine (2.68 g, 20.7 mmol) were then added, and the mixture was heated to 90°C and reacted for 12 h. The system was then cooled to room temperature, concentrated, and subjected to column chromatography (dichloromethane/methanol (v/v) = 90/10) to obtain compound **69E** (2.2 g, 87%).

LC-MS (ESI): m/z = 607.1 $[M+H]^+$.

Step 4: Compound **69E** (2.2 g, 3.63 mmol) was dissolved in methanol (10 mL), and potassium carbonate (0.5 g, 3.63 mmol) was then added and reacted at room temperature for 2.5 hours. The system was then concentrated and subjected to column chromatography (dichloromethane/methanol (v/v) = 90/10) to obtain compound **69F** (1.9 g, 98%).

LC-MS (ESI): m/z = 535.1 $[M+H]^+$.

Step 5: Compound **69F** (1.0 g, 1.87 mmol) was dissolved in dichloromethane (10 mL), and trifluoroacetic acid (3.0 mL) was then added and reacted at room temperature for 2.5 hours. The system was then concentrated and subjected to column chromatography (dichloromethane/methanol (v/v) = 93/7) to obtain compound **69G** (1.0 g, 98%).

LC-MS (ESI): m/z = 435.1 $[M+H]^+$.

Step 6: Compound **69G** (1.0 g, 1.85 mmol) was dissolved in dichloromethane (10 mL), and triethylamine (0.37 g, 3.7 mmol) was added, followed by methyl chloroformate (0.188 g, 2.0 mmol). The mixture was reacted at room temperature for 1.5 hours. The system was concentrated and subjected to column chromatography (dichloromethane/-methanol (v/v) = 90/10) to obtain compound **69** (0.5 g, 55%).

$^1H$ NMR (400 MHz, $CDCl_3$) δ 8.69 (d, 1H), 7.79 - 7.76 (m, 1H), 7.39 (d, 1H), 6.74 (s, 1H), 6.42 (s, 1H), 5.38 (s, 1H), 4.52 (s, 2H), 4.09 (s, 2H), 3.73 - 3.67 (m, 6H), 3.53 - 3.38 (m, 1H), 3.25 (s, 1H), 3.22 - 3.12 (m, 2H), 2.75 (s, 2H), 2.39 (s, 2H), 2.27 - 2.22 (m, 2H), 1.98 - 1.92 (m, 2H).

LC-MS (ESI): m/z = 493.1 $[M+H]^+$.

## Example 70

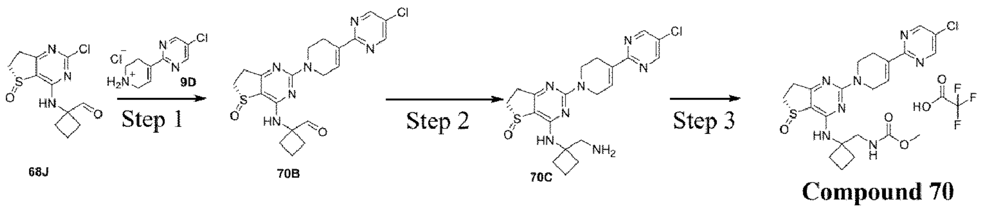


68J 70B 70C Compound 70

Step 1: Compound **68J** (0.50 g, 1.75 mmol) and compound **9D** (0.41 g, 2.10 mmol) were dissolved in 1,4-dioxane (20 mL), and N,N-diisopropylethylamine (0.90 g, 7.00 mmol) was added. After the addition was complete, the mixture was stirred at 90°C for 16 h. After the reaction was complete as detected by TLC (dichloromethane:methanol = 10:1), water (20 mL) was added and stirred for 5 min, and the mixture was extracted with ethyl acetate (20 mL). The organic phase was separated, dried over anhydrous sodium sulfate, and concentrated under reduced pressure, and the crude product was purified by column chromatography (dichloromethane/methanol (v/v) = 90/10) to obtain the target compound **70B** (0.5 g, 64%).

LC-MS (ESI): m/z = 445.2 $[M+H]^+$

Step 2: Compound **70B** (0.16 g, 0.36 mmol) was weighed and dissolved with methanol (20 mL), and ammonium acetate (0.28 g, 3.63 mmol) was added. After the addition was complete, the mixture was stirred at room temperature for 12 h, sodium cyanoborohydride (34 mg, 0.54 mmol) was added, and the mixture was heated to 50°C and stirred for 4 h. After the reaction was complete as detected by TLC (dichloromethane:methanol = 5:1), the reaction liquid was directly concentrated under reduced pressure to obtain the target compound **70C** (0.20 g, crude).

LC-MS (ESI): m/z = 446.1 $[M+H]^+$

Step 3: Crude compound **70C** (200 mg) was dissolved in dichloromethane (10 mL), and triethylamine (0.14 g, 1.35 mmol) and methyl chloroformate (430 mg, 4.55 mmol) were added at 0°C. After the addition was complete, the system was placed under nitrogen protection and stirred at 10°C for 4 h. After the reaction was complete as monitored by TLC (dichloromethane:methanol = 5:1), the reaction liquid was concentrated under reduced pressure to obtain a crude product of the target compound, which was purified by preparative HPLC to obtain the title compound **70**(2.9 mg, 1.8%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM trifluoroacetic acid); b. gradient elution, mobile phase A with a content of 40-80%; c. flow: 15 mL/min; d. elution time: 20 min; retention time: 12.50 min.

$^1H$ NMR (400 MHZ, DMSO-*d*6) δ 8.90 (s, 2H), 7.79 (s, 1H), 7.29 (s, 1H), 7.19 (t, 1H), 4.48 (s, 2H), 4.43 (s, 2H), 3.99 (t, 2H), 3.53-3.55 (m, 1H), 3.52 (s, 3H), 3.41-3.48 (m, 1H), 3.21 - 3.28 (m, 1H), 2.99 - 3.06 (m, 1H), 2.88 - 2.93 (m, 1H), 2.67 (s, 2H), 2.20 - 2.45 (m, 3H), 1.72 - 1.85 (m, 2H).

LC-MS (ESI): m/z = 504.2 $[M+H]^+$

**Example 71**

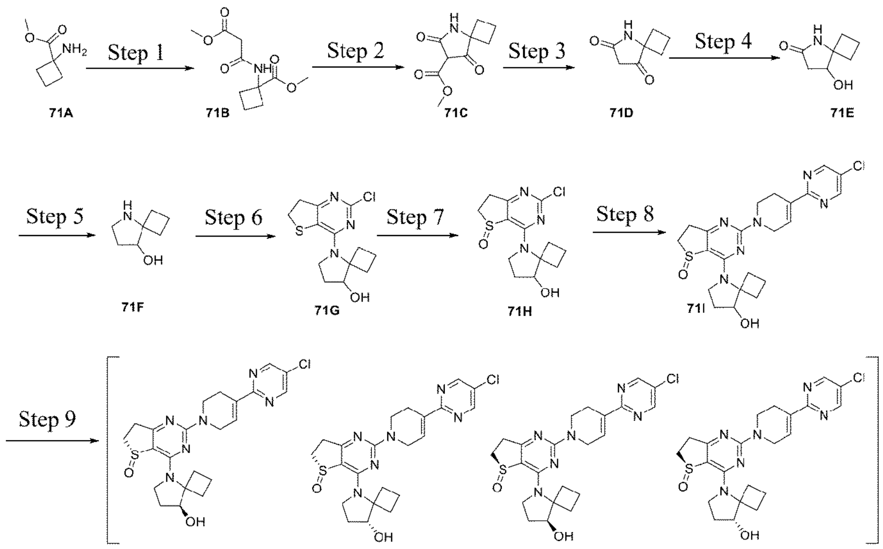


71A 71B 71C 71D 71E

71F 71G 71H 71I

**Compounds** 71-1,2,3,4

Step 1: **71A** (10 g, 77.4 mmol) and triethylamine (9.4 g, 92.9 mmol) were dissolved in dichloromethane (100 mL), and methyl malonyl chloride (14.8 g, 108 mmol) was added in an ice bath and stirred in the ice bath for half an hour. The mixture was heated to room temperature, stirred for 3 hours, diluted by adding water, then extracted three times with dichloromethane, and washed with a saturated sodium bicarbonate solution. The organic phase was dried over anhydrous sodium sulfate. After filtration, the filtrate was concentrated to obtain crude **71B,** which was directly used for the next reaction.

Step 2: The raw material **71B** (15 g) was dissolved in anhydrous methanol (150 mL), sodium methoxide (7.78 g, 144 mmol) was added, and the mixture was heated to 65°C and stirred for 10 hours. After cooling to room temperature, the system was directly filtered, and the filtrate was diluted by adding water, adjusted to pH 5-6 with 1N hydrochloric acid, extracted with dichloromethane, and dried over anhydrous sodium sulfate. After filtration, the filtrate was concentrated to obtain crude compound **71C,** which was directly used for the next reaction.

Step 3: The raw material **71C** (25 g) was dissolved in acetonitrile (250 mL), water (9 mL) was added, and the mixture was heated to 85°C and stirred for 2 hours. The reaction liquid was cooled, concentrated, and then slurried with methyl tert-butyl ether to obtain compound **71D** (6 g, three-step yield 34%).

$^1$H NMR (400 MHz, DMSO-*d6*) δ 8.76 (s, 1H), 2.99(s, 2H), 2.15-2.19 (m, 2H), 1.79-1.82 (m, 2H), 1.66-1.77 (m, 2H).

LC-MS (ESI): m/z = 140.1 $[M+H]^+$.

Step 4: The raw material **71D** (2.5 g, 17.97 mmol) was added to anhydrous methanol (50 mL), and sodium borohydride (1.36 g, 35.9 mmol) was then added in portions and stirred at room temperature for 10 hours. The reaction liquid was concentrated and then purified and separated by silica gel column chromatography (dichloromethane/methanol (v/v) = 100/0-90/10) to obtain compound **71E** (1.8 g, 71%).

LC-MS (ESI): m/z = 142.1 $[M+H]^+$.

Step 5: Lithium tetrahydroaluminate (1.45 g, 38.3 mmol) was suspended in anhydrous tetrahydrofuran (30 mL), and trimethylchlorosilane (3.46 g, 31.9 mmol) was added in an ice bath and stirred for half an hour. **71E** (1.8 g, 12.8 mmol) was then added, and the mixture was heated to 65°C, stirred for 16 hours, and cooled. The reaction was quenched with a 30% potassium hydroxide aqueous solution, and the system was filtered. The filtrate was concentrated and then purified and separated by silica gel column chromatography (dichloromethane/methanol (v/v) = 100/0-80/20) to obtain compound **71F** (0.4 g, 25%).

LC-MS (ESI): m/z = 128.1 $[M+H]^+$

Step 6: Under nitrogen protection, **71F** (0.45 g, 3.54 mmol), **24A** (0.73 g, 3.54 mmol), and N,N-diisopropylethylamine (1.37 g, 10.6 mmol) were successively dissolved in acetonitrile (20 mL), heated to 45°C, stirred for 10 hours, cooled, then diluted by adding water, and extracted with ethyl acetate, and the organic phase was dried over anhydrous sodium sulfate. After filtration, the filtrate was concentrated and then purified and separated by silica gel column chromatography (petroleum ether/ethyl acetate (v/v) = 100/0-50/50) to obtain compound **71G** (0.4 g, 38%).

LC-MS (ESI): m/z = 298.1 $[M+H]^+$

Step 7: The raw material **71G** (400 mg, 1.34 mmol), S-binaphthol (77 mg, 0.27 mmol), water (24 mg, 1.34 mmol), and tetraisopropyl titanate (38 mg, 0.13 mmol) were successively added to dichloromethane (15 mL) and stirred at room temperature for half an hour, and tert-butyl hydroperoxide (180 mg, 2.00 mmol) was added and further stirred for 3 hours.

The mixture was diluted by adding water and extracted with dichloromethane, and the organic phase was washed with a saturated sodium bicarbonate aqueous solution and dried over anhydrous sodium sulfate. After filtration, the filtrate was concentrated and then purified and separated by a preparative silica gel plate (dichloromethane/methanol (v/v) = 90/10) to obtain compound **71H** (250 mg, 60%).

LC-MS (ESI): m/z = 314.1 $[M+H]^+$

Step 8: Under nitrogen protection, **71H** (0.40 g, 1.27 mmol), intermediate **9D** (0.25 g, 1.27 mmol), and N,N-diisopropylethylamine (0.82 g, 6.35 mmol) were successively dissolved in 1,4-dioxane (20 mL), heated to 95°C, stirred for 10 hours, cooled, diluted by adding water, and extracted with ethyl acetate, and the organic phases were combined and dried over anhydrous sodium sulfate. After filtration, the filtrate was concentrated and then purified and separated by silica gel column chromatography (dichloromethane/methanol (v/v) = 100/0-90/10) to obtain two groups of isomers **71-1** and **2** and **71-3** and **4** of compound **71.**

Compound **71-1** and compound **71-2** separation conditions: instrument name: Waters 150 Prep-SFC A; chromatographic column: Chiralcel OX column; mobile phase: A for $CO_2$ and B for isopropanol (0.1% $NH_3$•$H_2O$); gradient: 60%; flow rate: 100 mL/min; column pressure: 100 bar; column temperature: 25°C; absorption wavelength: 220 nm; cycle time: about 8 min.

Compound **71-1,** retention time: 1.592 min; $^1$H NMR (400 MHz, DMSO-*d6*) δ 8.89 (s, 2H), 7.31- 7.35 (m, 1H), 5.31 (d, 1H), 4.57 - 4.59 (m, 2H), 4.05 - -4.17 (m, 3H), 3.86-3.92 (m, 1H), 3.70 - 3.74 (m, 1H), 3.42 - 3.54 (m, 2H), 3.32 - 3.35 (m, 1H), 3.14 - 3.21 (m, 1H), 3.04 - 3.10 (m, 1H), 2.91 - 2.96 (m, 1H), 2.70 - 2.72 (m, 2H), 2.26 - 2.30 (m, 1H), 1.87 - 1.99 (m, 3H), 1.65 - 1.79 (m, 2H).

LC-MS (ESI): m/z = 473.40 $[M+H]^+$

Compound **71-2,** retention time: 2.034 min; $^1$H NMR (400 MHz, DMSO-*d6*) δ 8.89 (s, 2H), 7.31- 7.35 (m, 1H), 5.31 (d, 1H), 4.57 - 4.59 (m, 2H), 4.05 - 4.17 (m, 3H), 3.86-3.92 (m, 1H), 3.70 - 3.74 (m, 1H), 3.42 - 3.54 (m, 2H), 3.32 - 3.35 (m, 1H), 3.14 - 3.21 (m, 1H), 3.04 - 3.10 (m, 1H), 2.91 - 2.96 (m, 1H), 2.70 - 2.72 (m, 2H), 2.26 - 2.30 (m, 1H), 1.87 - 1.99 (m, 3H), 1.65 - 1.79 (m, 2H).

LC-MS (ESI): m/z = 473.40 $[M+H]^+$

Compound **71-3** and compound **71-4** separation conditions: instrument name: Waters 150 Prep-SFC A; chromatographic column: Chiralcel Whelk column; mobile phase: A for CO2 and B for 0.1% $NH_3$•$H_2O$ in ETOH and ACN; gradient: 55%; flow rate: 100 mL/min; column pressure: 100 bar; column temperature: 25°C; absorption wavelength: 220 nm; cycle time: about 7.5 min.

Compound **71-3,** retention time: 1.577 min; $^1$H NMR (400 MHz, DMSO-*d6*) δ 8.89 (s, 2H), 7.31- 7.35 (m, 1H), 5.32 (d, 1H), 4.56 - 4.58 (m, 2H), 4.06 - 4.13 (m, 3H), 3.86 - 3.91 (m, 2H), 3.41 - 3.56 (m, 2H), 3.29 - 3.37 (m, 1H), 3.11 - 3.17 (m, 1H), 2.96 - 3.03 (m, 2H), 2.70 - 2.72 (m, 2H), 2.24 - 2.32 (m, 1H), 1.93 - 1.99 (m, 1H), 1.80 - 1.90 (m, 2H), 1.70 - 1.79 (m, 2H).

LC-MS (ESI): m/z = 473.40 $[M+H]^+$

Compound **71-4,** retention time: 2.246 min; $^1$H NMR (400 MHz, DMSO-*d6*) δ 8.89 (s, 2H), 7.31- 7.35 (m, 1H), 5.32 (d, 1H), 4.56 - 4.58 (m, 2H), 4.06 - 4.13 (m, 3H), 3.86 - 3.91 (m, 2H), 3.41 - 3.56 (m, 2H), 3.29 - 3.37 (m, 1H), 3.11 - 3.17 (m, 1H), 2.96 - 3.03 (m, 2H), 2.70 - 2.72 (m, 2H), 2.24 - 2.32 (m, 1H), 1.93 - 1.99 (m, 1H), 1.80-1.90 (m, 2H), 1.70 - 1.79 (m, 2H).

LC-MS (ESI): m/z = 473.40 $[M+H]^+$

**Example 72**

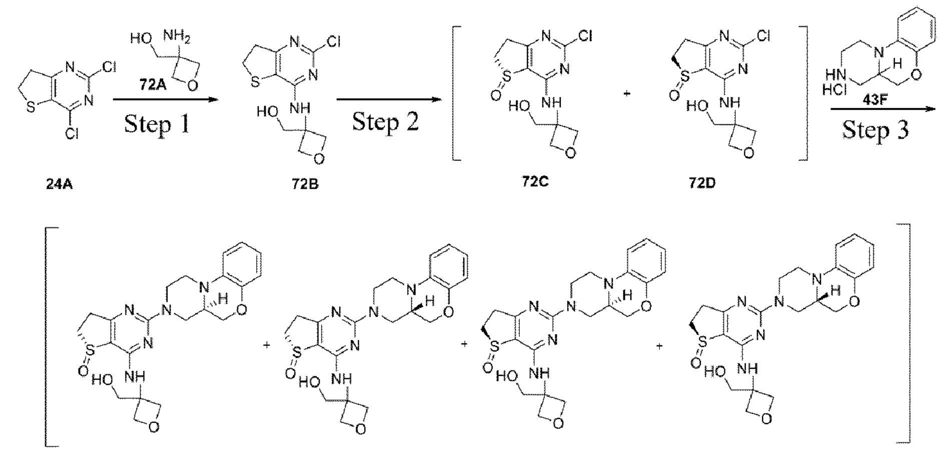


24A 72B 72C 72D

Compounds 72-1,72-2,72-3,72-4

Using compounds **72A** and **24A** as raw materials, a mixture of compounds **72-1** and **72-2** (0.25 g, 84%) was obtained according to the operation of Example 34.

Chiral preparation was carried out to obtain the title compound **72-1** (20 mg, retention time: 1.535 min) and the title compound **72-2** (30 mg, retention time: 2.012 min).

Chiral preparation method: instrument: Waters 150 preparative SFC (SFC-26); chromatographic column: ChiralPak AD, 250 × 30 mm I.D., 10 μm; mobile phase: A: carbon dioxide, and B: isopropanol (0.1% aqueous ammonia); isocratic elution: 30% mobile phase B; flow rate: 150 mL/min; back pressure: 100 bar; column temperature: 38°C; wavelength: 220 nm; elution time: 15 min.

According to the above operation, using **72D** (195 mg) as a raw material, a mixture of **72-3** and **72-4** (0.26 g, 87%) was obtained.

Chiral preparation was carried out to obtain the title compound **72-3** (50 mg, retention time: 0.698 min) and the title compound **72-4** (50 mg, retention time: 1.514 min).

Chiral preparation method: instrument: Waters 150 preparative SFC (SFC-26); chromatographic column: ChiralPak AD, 250 × 30 mm I.D., 10 μm; mobile phase: A: carbon dioxide, and B: ethanol (0.1% aqueous ammonia); isocratic elution: 65% mobile phase B; flow rate: 100 mL/min; back pressure: 100 bar; column temperature: 38°C; wavelength: 220 nm; elution time: 5 min.

**(Compound 72-1)**

$^1$H NMR (400 MHz, DMSO-*d6*) δ 8.21 (s, 1H), 6.94-6.88 (m, 1H), 6.84 - 6.74 (m, 1H), 6.73 - 6.62 (m, 2H), 5.18 - 5.12 (m, 1H), 4.74 - 4.57 (m, 6H), 4.39 - 4.32 (m, 1H), 3.99 - 3.92 (m, 1H), 3.88-3.77 (m, 3H), 3.49 - 3.40 (m, 1H), 3.26 - 3.18 (m, 1H), 3.11 - 2.86 (m, 4H), 2.76 - 2.65 (m, 1H), 2.63 - 2.54 (m, 1H).

LC-MS (ESI): m/z = 444.2 $[M+H]^+$.

**(Compound 72-2)**

$^1$H NMR (400 MHz, DMSO-*d6*) δ 8.21 (s, 1H), 6.94 - 6.88 (m, 1H), 6.83 - 6.76 (m, 1H), 6.73 - 6.62 (m, 2H), 5.18 - 5.12 (m, 1H), 4.74 - 4.57 (m, 6H), 4.39 - 4.32 (m, 1H), 3.99 - 3.92 (m, 1H), 3.88 - 3.76 (m, 3H), 3.49 - 3.40 (m, 1H), 3.26 - 3.19 (m, 1H), 3.12 - 2.86 (m, 4H), 2.76 - 2.66 (m, 1H), 2.64 - 2.54 (m, 1H).

LC-MS (ESI): m/z = 444.2 $[M+H]^+$.

**(Compound 72-3)**

$^1$H NMR (400 MHz, DMSO-*d6*) δ 8.21 (s, 1H), 6.94 - 6.88 (m, 1H), 6.83 - 6.77(m, 1H), 6.73 - 6.62 (m, 2H), 5.19 - 5.12 (m, 1H), 4.74 - 4.57 (m, 6H), 4.39 - 4.32 (m, 1H), 3.99 - 3.91 (m, 1H), 3.87 - 3.76 (m, 3H), 3.49 - 3.41 (m, 1H), 3.28 - 3.18 (m, 1H), 3.12 - 2.86 (m, 4H), 2.76 - 2.66 (m, 1H), 2.64-2.55 (m, 1H).

LC-MS (ESI): m/z = 444.2 $[M+H]^+$.

**(Compound 72-4)**

$^1$H NMR (400 MHz, DMSO-*d6*) δ 8.21 (s, 1H), 6.94 - 6.88 (m, 1H), 6.82 - 6.78(m, 1H), 6.72 - 6.63 (m, 2H), 5.19 - 5.12 (m, 1H), 4.74 - 4.57 (m, 6H), 4.39 - 4.32 (m, 1H), 3.99 - 3.91 (m, 1H), 3.87 - 3.76 (m, 3H), 3.49 - 3.41 (m, 1H), 3.28 - 3.18 (m, 1H), 3.12 - 2.86 (m, 4H), 2.76 - 2.66 (m, 1H), 2.64 - 2.55 (m, 1H).

LC-MS (ESI): m/z = 444.2 $[M+H]^+$.

**Example 73**

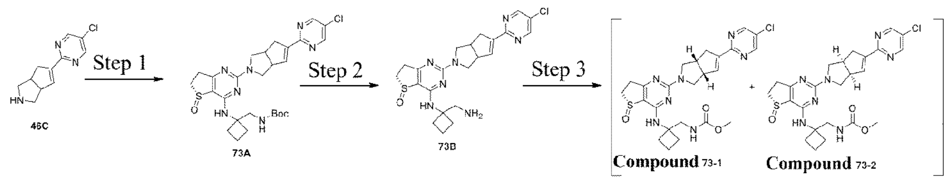


Using compounds **46C** and **69C** as raw materials, compound **73-1** (130 mg, retention time: 2.192 min) and the title compound **73-2** (138.2 mg, retention time: 2.573 min) were obtained according to the operation of Example 69.

Chiral preparation method: instrument: Waters 150 preparative SFC (SFC-26); chromatographic column: Chiralcel Cellulose-2 column; mobile phase: A: carbon dioxide, and B: methanol and acetonitrile (0.1% aqueous ammonia); isocratic elution: 40% mobile phase B; flow rate: 100 mL/min; back pressure: 100 bar; column temperature: 25°C; wavelength: 220 nm; elution time: 7.0 min.

**(Compound 73-1)**

$^1$H NMR (400 MHz, DMSO-*d6*) δ 8.87 (s, 2H), 7.36 (s, 1H), 7.13 (s, 1H), 6.85 (s, 1H), 3.94 - 3.84 (m, 1H), 3.78 (s, 1H), 3.66 (s, 2H), 3.51 (s, 3H), 3.45 - 3.33 (m, 2H), 3.24 - 3.10 (m, 3H), 2.86-2.81 (m, 1H), 2.75 (d, 1H), 2.37 - 2.13 (m, 4H), 1.76 (s, 2H), 1.39 (d, 1H), 1.17 (d, 2H).
LCMS (ESI): m/z = 530.2 $[M+H]^+$

**Compound 73-2:**

$^1$H NMR (400 MHz, DMSO-*d6*) δ 8.87 (s, 2H), 7.35 (s, 1H), 7.11 (s, 1H), 6.85 (s, 1H), 3.80-3.76 (m, 1H), 3.77 (s, 1H), 3.66 (s, 2H), 3.50 (s, 3H), 3.40 (s, 1H), 3.25-3.12 (m, 3H), 3.04 - 2.79 (m, 4H), 2.31- 2.13 (m, 3H), 1.77 (s, 2H), 1.39 (d, 1H), 1.17 (d, 2H).
LCMS (ESI): m/z = 530.2 $[M+H]^+$

**Example 74**

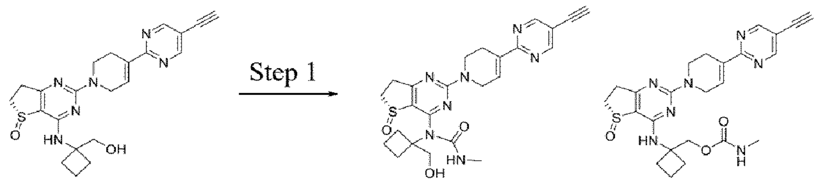


35F

Compound 74-1 and compound 74-2

Step 1: Compound **35F** (0.85 g, 2.0 mmol) was dissolved in tetrahydrofuran (10 mL), and sodium hydride (80.0 mg, 2.0 mmol) was added. After the addition was complete, the mixture was reacted at room temperature for 1 hour, and methylaminoformyl chloride (186.0 mg, 2.0 mmol) was further added and further reacted at room temperature overnight. After the reaction was complete as monitored by TLC, the reaction was quenched by adding a small amount of methanol. The reaction liquid was concentrated, and the crude product was purified by silica gel column chromatography (dichloromethane/methanol (v/v) = 95/5) to obtain compound **74-1** (0.11 g, 11%) and **74-2** (0.25 g, 23%).
LC-MS (ESI): m/z = 494.1 $[M+H]^{+1}$H NMR (400 MHz, DMSO-*d6*) δ 8.90 (s, 2H), 7.58 (s, 1H), 7.34 (s, 1H), 6.93 (s, 1H), 4.68 (s, 1H), 4.45 (d, 3H), 3.97 (s, 2H), 3.52 - 3.36 (m, 1H), 3.23 (d, 2H), 2.92 (m, 1H), 2.67 (s, 3H), 2.56 (d, 3H), 2.35 (d, 2H), 2.25 (s, 2H), 1.82 - 1.85 (m, 2H).
LC-MS (ESI): m/z = 494.1 $[M+H]^+$.
$^1$H NMR (400 MHz, DMSO-*d6*) δ 8.90 (s, 2H), 7.80 (s, 1H), 7.54 (s, 1H), 6.92 (d, 1H), 5.18 - 5.15 (m, 1H), 4.64 (s, 1H), 4.39 (s, 2H), 4.11 (s, 1H), 3.83 (d, 2H), 3.56 - 3.40 (m, 1H), 3.28 - 3.15 (m, 1H), 3.03 - 2.88 (m, 2H), 2.55 (d, 3H), 2.40 - 2.31 (m, 2H), 2.23 (s, 4H), 1.90 - 1.75 (m, 2H).

**Example 75**

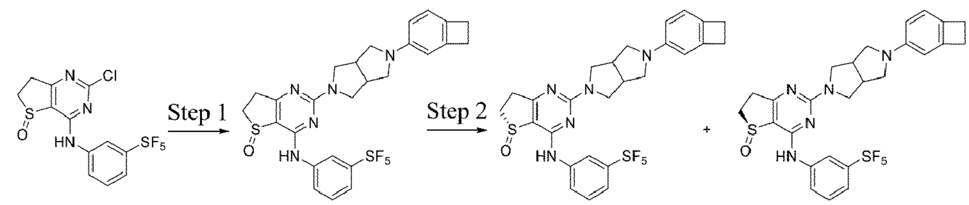


60D 75A Compounds 75-1, 2

Using compounds **60D** and **13D** as raw materials, **75-1** (71.1 mg) and compound **75-2** (36.8 mg) were obtained according to the operation of Example 60.
Analysis method: instrument: SHIMADZU LC-20AD, column: Chiralpak IG-3 50 × 4.6 mm I.D., 3 μm; mobile phase: A: water (0.1% TFA), B: acetonitrile; gradient: 10-80% B in A; flow rate: 1.2 mL/min, column temperature: 45°C, wavelength: 210 & 254 nm.
Preparation method: instrument: SHIMADZU LC-20AP, column: DAICEL CHIRALPAK IG (250 mm * 30 mm, 10 μm) mobile phase: A: water (0.01% NH4HCO3 in H2O), B: acetonitrile; gradient: 70-100% B in A; flow rate: 25 mL /min, column temperature: 25°C, wavelength: 254 nm, cycle time: 16 min, sample preparation: sample concentration 1.5 mg/ml, ethanol solution injection: 2 ml each time. After separation, the fraction was dried by a rotary evaporator at a bath temperature of 40°C to obtain compound **75-1** (retention time: 1.340 min) and compound **75-2** (retention time: 1.935 min).

**(Compound 75-1)**

$^{1}$H NMR (400 MHz, $CDCl_3$) δ 8.79 - 8.50 (m, 2H), 7.49 - 7.47 (m, 1H), 7.37 - 7.36 (m, 1H), 7.31 - 7.29 (m, 1H), 6.93 - 6.87 (m, 1H), 6.44 - 6.33 (m, 2H), 3.89 - 3.81 (m, 2H), 3.66 - 3.33 (m, 6H), 3.25 - 3.07 (m, 10H).
LCMS m/z = 584.1 $[M+H]^+$

**(Compound 75-2)**

$^{1}$H NMR (400 MHz, $CDCl_3$) δ 8.80 - 8.50 (m, 2H), 7.49 - 7.47 (m, 1H), 7.37 - 7.36 (m, 1H), 7.31 - 7.29 (m, 1H), 6.93 - 6.87 (m, 1H), 6.44 - 6.33 (m, 2H), 3.89 - 3.81 (m, 2H), 3.66 - 3.19 (m, 6H), 3.25 - 3.07 (m, 10H).
LCMS m/z = 584.1 $[M+H]^+$

**Example 76**

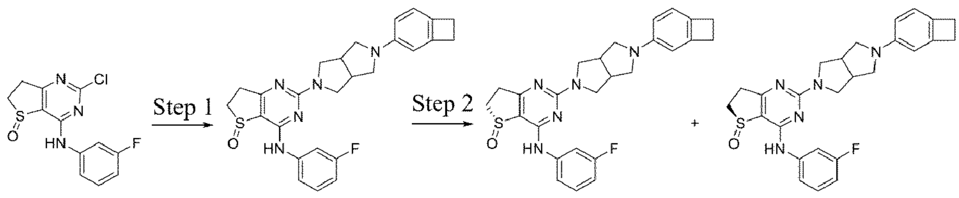


36A 76A Compounds 76-1, 2

Using compounds **36A** and **13D** as raw materials, compound **76-1** (342 mg) and compound **76-2** (50 mg) were obtained according to the operation of Example 60. Analysis method: instrument: SHIMADZU LC-20AD, column: Chiralpak IG-3 50 × 4.6 mm I.D., 3 μm; mobile phase: A: water (0.1% TFA), B: acetonitrile; gradient: 10-80% B in A; flow rate: 1.2 mL/min, column temperature: 45°C, wavelength: 210 & 254 nm.
Preparation method: instrument: SHIMADZU LC-20AP, column: DAICEL CHIRALPAK IG (250mm × 30 mm, 10 μm) mobile phase: A: water (0.01% $NH_4HCO_3$ in $H_2O$), B: acetonitrile; gradient: 60% B in A; flow rate: 25 mL/min, column temperature: 25°C, wavelength: 254 nm, cycle time: 7 min, sample preparation: sample concentration 1.5 mg/mL, ethanol solution injection: 2 mL each time. After separation, the fraction was dried by a rotary evaporator at a bath temperature of 40°C to obtain compound **76-1** (retention time: 1.542 min) and compound **76-2** (retention time: 2.226 min).

**(Compound 76-1)**

$^{1}$H NMR (400 MHz, $CDCl_3$) δ 8.30 - 8.21 (m, 1H), 7.82 - 7.76 (m, 1H), 7.21 - 7.14 (m, 2H), 6.95 - 6.87 (m, 1H), 6.72 - 6.68 (m, 1H), 6.47 - 6.33 (m, 2H), 3.91 - 3.83 (m, 2H), 3.64 - 3.55 (m, 5H), 3.39 - 3.20 (m, 3H), 3.16 - 3.02 (m, 8H).
LCMS m/z = 476.1 $[M+H]^+$

**(Compound 76-2)**

$^{1}$H NMR (400 MHz, $CDCl_3$) δ 8.30 - 8.21 (m, 1H), 7.82 - 7.76 (m, 1H), 7.21 - 7.14 (m, 2H), 6.95 - 6.87 (m, 1H), 6.72 - 6.68 (m, 1H), 6.47 - 6.33 (m, 2H), 3.91 - 3.83 (m, 2H), 3.64 - 3.52 (m, 5H), 3.39 - 3.20 (m, 3H), 3.16 - 3.02 (m, 8H).
LCMS m/z = 476.1 $[M+H]^+$

**Example 77**

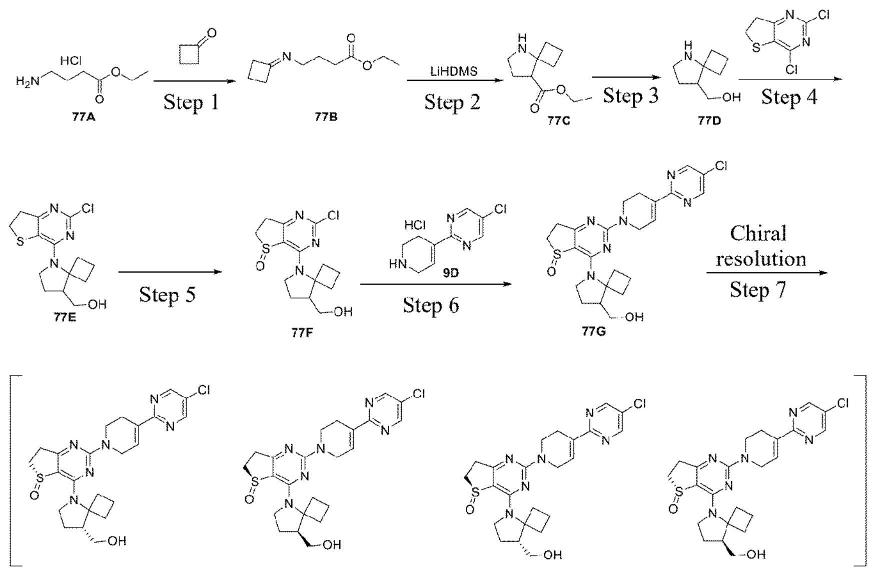


Compounds 77-1,77-2,77-3,77-4

Step 1: Compound **77A** (11.5 g, 68 mmol), cyclobutanone (4.8 g, 68 mmol), and potassium carbonate (28.1 g, 204 mmol) were weighed into a three-mouth flask, dissolved by adding toluene (200 mL), and reacted at 60°C for 24 h. After the reaction was complete as monitored by LCMS, the system was cooled to room temperature and filtered. The filter cake was washed with ethyl acetate (50 mL), and the filtrate was concentrated to obtain a crude product of the target compound **77B** (15.8 g), which did not require further purification.

Step 2: Crude compound **77B** (15.8 g) was weighed into a three-mouth flask and dissolved by adding anhydrous tetrahydrofuran (200 mL). After nitrogen displacement, the mixture was cooled to -78°C, and a lithium bis(trimethylsilyl) amide solution (70 mL, 1M in THF) was slowly dropwise added. After the dropwise addition was complete, the mixture was naturally heated to room temperature and adjusted to weak acidity with a saturated ammonium chloride solution. The organic phase was separated, and the aqueous phase was extracted with ethyl acetate (100 mL × 1). The organic phases were combined, dried over anhydrous sodium sulfate, and filtered. After concentration, the residue was separated by column chromatography (petroleum ether/ethyl acetate (v/v) = 100/0-83/17) to obtain the title compound **77C** (4.9 g, 39%).

LC-MS (ESI): m/z = 184.2 $[M+H]^+$.

Step 3: **77C** (4.9 g, 26.7 mmol) was dissolved in tetrahydrofuran (50 mL), and lithium tetrahydroaluminate (1.9 g, 52 mmol) was slowly added. After the raw material completely disappeared as monitored by TLC, water (2 mL) was slowly added first, followed by a 15% sodium hydroxide aqueous solution (2 mL), and the mixture was fully stirred and then filtered. The filtrate was concentrated to obtain the title compound **77D** (1.23 g, 32.6%).

LC-MS (ESI): m/z = 142.2 $[M+H]^+$.

Step 4: Compound **77D** (1 g, 7 mmol) was dissolved in anhydrous acetonitrile, and 2,4-dichloro-6H,7H-thieno [3,2-d]pyrimidine (1.45 g, 7 mmol) and triethylamine (1.4 g, 14 mmol) were added. The mixture was heated to 55°C and reacted overnight, and the system was cooled to room temperature and then directly concentrated. The residue was separated by column chromatography (petroleum ether/ethyl acetate (v/v) = 100/0-50/50) to obtain the title compound **77E** (668 mg, 30.3%).

LC-MS (ESI): m/z = 312.2 $[M+H]^+$.

Step 5: Compound **77E** (668 mg, 2.14 mmol) was dissolved in dichloromethane, TIPT (1.21 g, 4.28 mmol) and tert-butyl hydroperoxide (231 mg, 2.56 mmol) were successively added, and the mixture was reacted at room temperature for 4 h. The system was concentrated, and the residue was separated by silica gel column chromatography (petroleum ether/ethyl acetate (v/v) = 100/0-0/100) to obtain the title compound **77F** (551 mg, 78.5%).

LC-MS (ESI): m/z = 328.2 $[M+H]^+$ .

Step 6: Compound **77F** (551 mg, 1.68 mmol) was dissolved in 1,4-dioxane, and **9D** (268 mg, 1.68 mmol) and N,N-diisopropylethylamine (650 mg, 5.04 mmol) were successively added and reacted at 85°C for 4 h. They system was cooled to room temperature and then directly concentrated, and the residue was purified and separated by silica gel column chromatography (dichloromethane/methanol (v/v) = 91/9) to obtain two crude compounds **77G-1** (142 mg) and **77G-2** (136 mg).

LC-MS (ESI): m/z = 487.7 $[M+H]^+$

Step 7: **77G-1** was purified and separated by chiral HPLC to obtain compound **77-1** (44 mg, 1.126 min) and **77-2** (32 mg, 1.586 min). Separation conditions: instrument name: Waters 150 Prep-SFC F; chromatographic column: Chiralcel AS column; mobile phase: A for $CO_2$; B for 0.1% $NH_3$•$H_2O$ in IPA and ACN; gradient: 45%; flow rate: 100 mL/min; column pressure: 100 bar; column temperature: 25°C; absorption wavelength: 220 nm; cycle time: about 5 min).

**(Compound 77-1)**

$^1$H NMR (400 MHz, DMSO-$d_6$) δ 8.89 (s, 2H), 7.35 - 7.29 (m, 1H), 4.72 (s, 1H), 4.61 - 4.51 (m, 2H), 4.10 - 4.03 (m, 2H), 3.94 - 3.78 (m, 2H), 3.75 - 3.64 (m, 1H), 3.62 - 3.38 (m, 4H), 3.20 - 3.10 (m, 1H), 3.10 - 2.91 (m, 2H), 2.70 (s, 2H), 2.34 - 2.22 (m, 1H), 2.14 - 1.69 (m, 6H).

LC-MS (ESI): m/z = 487.7 $[M+H]^+$

**(Compound 77-2)**

$^1$H NMR (400 MHz, DMSO-$d_6$) δ 8.89 (s, 2H), 7.35 - 7.29 (m, 1H), 4.72 (s, 1H), 4.61 - 4.51 (m, 2H), 4.10 - 4.03 (m, 2H), 3.94 - 3.78 (m, 2H), 3.75 - 3.64 (m, 1H), 3.62 - 3.38 (m, 4H), 3.20 - 3.10 (m, 1H), 3.10 - 2.91 (m, 2H), 2.70 (s, 2H), 2.34 - 2.22 (m, 1H), 2.14 - 1.69 (m, 6H).

LC-MS (ESI): m/z = 487.7 $[M+H]^+$

**77G-2** was purified and separated by chiral HPLC to obtain compound **77-3** (31 mg, 1.300 min) and **77-4** (35 mg, 2.304 min). Separation conditions: instrument name: Waters 150 Prep-SFC F; chromatographic column: Chiralcel AS column; mobile phase: A for $CO_2$; B for 0.1% $NH_3$•$H_2O$ in IPA and ACN; gradient: 55%; flow rate: 100 mL/min; column pressure: 100 bar; column temperature: 25°C; absorption wavelength: 220 nm; cycle time: about 6.3 min.

**(Compound 77-3)**

$^1$H NMR (400 MHz, DMSO-$d_6$) δ 8.89 (s, 2H), 7.35 - 7.27 (m, 1H), 4.73 - 4.68 (m, 1H), 4.59 - 4.54 (m, 2H), 4.13 - 4.01 (m, 2H), 3.92 - 3.85 (m, 1H), 3.82 - 3.71 (m, 2H), 3.70 - 3.54 (m, 2H), 3.54 - 3.42 (m, 1H), 3.41 - 3.32 (m, 1H), 3.22 - 3.11 (m, 1H), 3.09 - 3.00 (m, 1H), 3.00 - 2.90 (m, 1H), 2.70 (s, 2H), 2.23 - 2.15 (m, 1H), 2.10 - 2.00 (m, 2H), 1.98 - 1.86 (m, 2H), 1.84 - 1.66 (m, 2H).

LC-MS (ESI): m/z = 487.7 $[M+H]^+$

**(Compound 77-4)**

$^1$H NMR (400 MHz, DMSO-$d_6$) δ 8.89 (s, 2H), 7.35 - 7.27 (m, 1H), 4.73 - 4.68 (m, 1H), 4.59 - 4.54 (m, 2H), 4.13 - 4.01 (m, 2H), 3.92 - 3.85 (m, 1H), 3.82 - 3.71 (m, 2H), 3.70 - 3.54 (m, 2H), 3.54 - 3.42 (m, 1H), 3.41 - 3.32 (m, 1H), 3.22 - 3.11 (m, 1H), 3.09 - 3.00 (m, 1H), 3.00 - 2.90 (m, 1H), 2.70 (s, 2H), 2.23 - 2.15 (m, 1H), 2.10 - 2.00 (m, 2H), 1.98 - 1.86 (m, 2H), 1.84 - 1.66 (m, 2H).

LC-MS (ESI): m/z = 487.7 $[M+H]^+$

**Example 78**

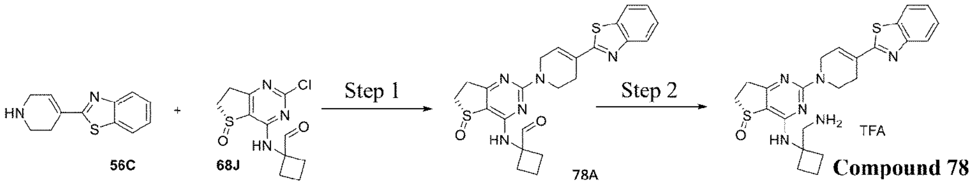


Step 1: Crude intermediate **56C** hydrochloride (800 mg), intermediate **68J** (755 mg, 2.64 mmol), and N,N-diisopropylethylamine (1.02 g, 7.92 mmol) were suspended in 1,4-dioxane (5 mL) and reacted at 80°C for 16 h under nitrogen protection. After the reaction was complete, the system was cooled to room temperature, and the reaction liquid was dropwise added into water (10 mL) and filtered. The filter cake was slurried with ethyl acetate (5 mL), stirred at room temperature for 0.5 h, and filtered, and the solid was dried to obtain compound **78A** (680 mg, 55%).

LC-MS (ESI): m/z = 466.1 $[M+H]^+$

Step 2: Compound **78A** (370 mg, 0.79 mmol) was dissolved in methanol (2 mL), ammonium acetate (61 mg, 1.58

mmol) was added and stirred at 60°C for 1 h, and sodium cyanoborohydride (76 mg, 1.19 mmol) was added and further reacted at 60°C for 1 h. After the reaction was complete, the reaction liquid was concentrated, and the crude product was purified by HPLC to obtain the target compound **78** (66 mg, 1.998 min, 15%). Analysis method: instrument: Shimadzu LCMS-2020; column: Phenomenex C18; mobile phase: A was 0.1% TFA in $H_2O$; B was acetonitrile; gradient: Phase B from 5% to 95% in 4 min; flow rate: 1.5 mL/min; column temperature: 45°C; detection wavelength: 220 & 254 nm;

separation method: instrument: SHIMADZU LC-20AP; preparative column: Phenomenex C18; mobile phase: A was 0.1% TFA in $H_2O$; B was acetonitrile; gradient: Phase B from 15 to 45 in 17 min; flow rate: 25 mL/min; column temperature: room temperature; detection wavelength: 254 nm;

$^1H$ NMR (400 MHz, DMSO-*d6*) δ 8.07 (d, 1H), 7.96 (d, 1H), 7.68 (s, 3H), 7.58 - 7.36 (m, 3H), 6.91-6.88 (m, 1H), 4.47 (s, 2H), 4.02-4.00 (m, 1H), 3.55 - 3.38 (m, 3H), 3.31-3.22 (m, 1H), 3.07 - 2.87 (m, 2H), 2.77 (s, 2H), 2.41-2.28 (m, 4H), 1.98 - 1.79 (m, 2H).

$^{19}F$ NMR (376 MHz, DMSO-*d6*) δ -71.96 (s).

LC-MS (ESI): m/z = 467.0 $[M+H]^+$

**Example 79**

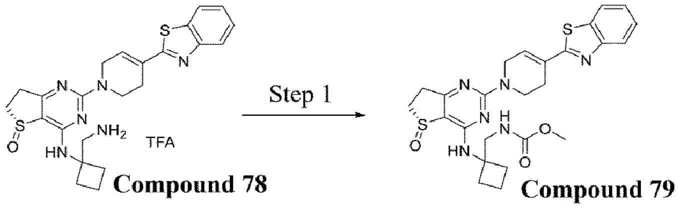


Compound 78 Compound 79

Step 1: Compound **78** (33 mg, 0.058 mmol) and N,N-diisopropylethylamine (9 mg, 0.087 mol) were suspended in dichloromethane (2 mL), methyl chloroformate (6 mg, 0.064 mmol) was added in an ice bath, and the mixture was heated to room temperature and reacted for 1 h. After the reaction was complete as monitored by TLC, the reaction liquid was directly concentrated, and the crude product was purified by HPLC to obtain compound **79** (25 mg, 2.434 min, 82%).

Analysis method: instrument: Shimadzu LCMS-2020; column: Phenomenex C18; mobile phase: A was 0.1% $NH_4CO_3$ in $H_2O$; B was acetonitrile; gradient: Phase B from 5% to 95% in 4 min; flow rate: 1.5 mL/min; column temperature: 45 °C; detection wavelength: 220 & 254 nm; separation method: instrument: SHIMADZU LC-20AP, preparative column: Phenomenex C18; mobile phase: A was 0.1% $NH_4CO_3$ in $H_2O$; B was acetonitrile; gradient: Phase B from 26 to 56 in 17 min; flow rate: 25 mL/min; column temperature: 45°C; detetion wavelength: 254 nm;

$^1H$ NMR (400 MHz, DMSO-d6) δ 8.07 (d, 1H), 7.96 (d, 1H), 7.56 - 7.36 (m, 3H), 7.19-7.15 (m, 1H), 6.90 (s, 1H), 4.49 (s, 2H), 4.03 (t, 2H), 3.59 - 3.38 (m, 5H), 3.31 - 3.17 (m, 2H), 3.01 - 2.82 (m, 2H), 2.77 (s, 2H), 2.43 - 2.16 (m, 4H), 1.91 - 1.69 (m, 2H).

LC-MS (ESI): m/z = 525.1 $[M+H]^+$

**Example 80**

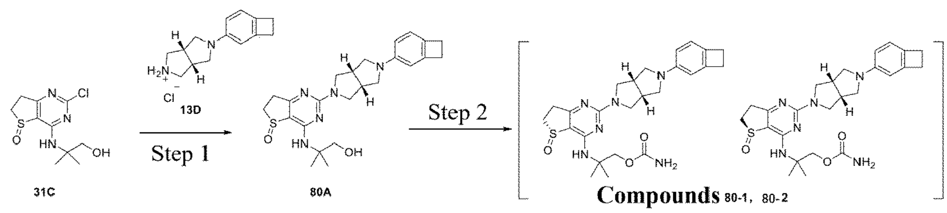


31C 80A Compounds 80-1, 80-2

Using compounds **13D** and **31C** as raw materials, compounds **80-1** (5.6 mg, 2%, 2.257 min) and **80-2** (32.2 mg, 12%, 2.700 min) were obtained according to the operation of Example 49.

Chiral separation method: instrument: Waters 150 Prep-SFC E, chiral column: Chiralcel OJ columnmobile phase: A for $CO_2$; B for 0.1% $NH_3$•$H_2O$ in MEOH and CAN, gradient: B 40%, flow rate: 120 mL/min, column pressure: 100 bar, column temperature: 25°C, detection wavelength: 220 nm, cycle time: 2.3 min.

**(Compound 80-1)**

$^1$H NMR (400 MHz, DMSO-*d6*) δ 6.87-6.83 (m, 1H), 6.42 -6.32 (m, 2H), 6.10 (s, 1H), 5.15 (s, 1H), 3.82 (s, 2H), 3.55 - 3.38 (m, 6H), 3.38 - 3.25 (m, 3H), 3.20 - 2.96 (m, 8H), 2.98 - 2.77 (m, 2H), 1.40 (s, 6H).

LC-MS (ESI): m/z = 497.1 $[M+H]^+$

**(Compound 80-2)**

$^1$H NMR (400 MHz, DMSO-*d6*) δ 6.87-6.83 (m, 1H), 6.42 - 6.32 (m, 2H), 6.10 (s, 1H), 5.15 (s, 1H), 3.82 (s, 2H), 3.55 - 3.38 (m, 6H), 3.38 - 3.25 (m, 3H), 3.20 - 2.96 (m, 8H), 2.98 - 2.77 (m, 2H), 1.40 (s, 6H).

LC-MS (ESI): m/z = 497.1 $[M+H]^+$

**Example 81**

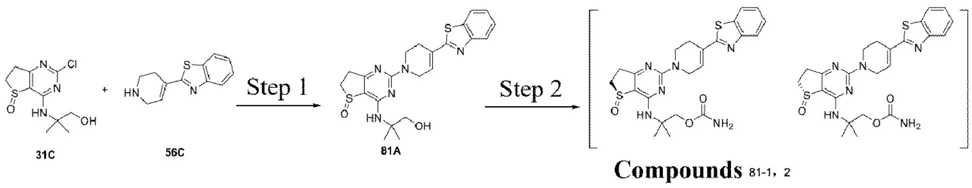


Compounds 81-1, 2

Using compounds **56C** and **31C** as raw materials, compounds **81-1** (3.6 mg, 2%) and **81-2** (21.2 mg, 10%) were obtained according to the operation of Example 49.

**Chiral separation method:**

instrument: Waters 150 Prep-SFC F, chiral column: Chiralcel Cellulose-2 Column; mobile phase: A for CO2; B for 0.1% $NH_3$•$H_2O$ in MeOH and CAN, gradient: B 60%, flow rate: 100 mL/min, column pressure: 100 bar, column temperature: 25°C, detection wavelength: 220 nm, cycle time: 5.0 min.

**Compound 81-1:**

retention time: 0.856 min, $^1$H NMR (400 MHz, DMSO-d6) δ 8.07 (d, 1H), 7.97 (d, 1H), 7.54 - 7.37 (m, 2H), 6.91 (s, 1H), 6.67 (s, 1H), 6.56 (s, 2H), 4.51 (s, 2H), 4.31 (s, 2H), 4.07 - 4.03 (m, 2H), 3.43 - 3.28 (m, 2H), 3.04 - 2.84 (m, 2H), 2.79 (s, 2H), 1.47 (d, 6H).

LC-MS (ESI): m/z = 499.1 $[M+H]^+$

**Compound 81-2:**

retention time: 1.058 min. $^1$H NMR (400 MHz, DMSO-d6) δ 8.07 (d, 1H), 7.97 (d, 1H), 7.54 - 7.37 (m, 2H), 6.91 (s, 1H), 6.67 (s, 1H), 6.56 (s, 2H), 4.51 (s, 2H), 4.31 (s, 2H), 4.07 - 4.03 (m, 2H), 3.43 - 3.28 (m, 2H), 3.04 - 2.84 (m, 2H), 2.79 (s, 2H), 1.47 (d, 6H).

LC-MS (ESI): m/z = 499.1 $[M+H]^+$

**Example 82**

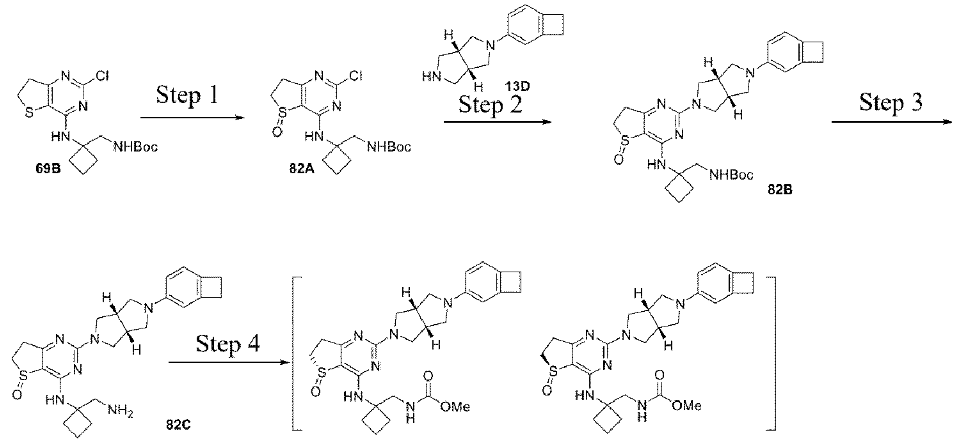


**Compounds** 82-1, 2

Step 1: Compound **69B** (766.9 mg, 2.07 mmol), 1,1'-bi-2-naphthol (58.7 mg, 0.021 mmol), titanium tetraisopropoxide (29.4 mg, 0.011 mmol), and water (32.0 mg, 1.78 mmol) were dissolved in dichloromethane (20 mL). After nitrogen displacement three times, the system was stirred at room temperature for one hour, and tert-butyl hydroperoxide (293 mg, 2.28 mmol, 70% in water) was then added and stirred overnight. After quenching with a saturated sodium thiosulfate aqueous solution, the aqueous phase was extracted with dichloromethane, dried, concentrated, and subjected to column chromatography (dichloromethane/methanol (v/v) = 91/9) to obtain compound **82A** (480 mg, 60%).

Using compounds **82A** and **13D** as raw materials, compounds **82-1** (23.1 mg, 8%, with absolute configuration unknown) and **82-2** (20.2 mg, 7%, with absolute configuration unknown) were obtained according to the operation of Example 69.

**Chiral separation method:** instrument: Instrument: Waters 150 Prep-SFC F, chiral column: Chiralcel Whelk-Column mobile phase: A for CO2; B for 0.1% $NH_3 \cdot H_2O$ in MEOH and CAN, gradient: B 45%; flow rate: 100 mL/min, column pressure: 100 bar, column temperature: 25°C, detection wavelength: 220 nm, cycle time: 8.0 min.

**Compound 82-1:**

retention time: 1.173 min, $^1$H NMR (400 MHz, DMSO-*d6*) δ 7.36 (s, 1H), 7.12 (s, 1H), 6.84 (m, 1H), 6.38-6.33 (m, 2H), 3.79 (s, 2H), 3.58 - 3.32 (m, 10H), 3.19 - 2.97 (m, 8H), 2.97 - 2.80 (m, 2H), 2.44 - 2.22 (m, 3H), 2.17 (s, 2H), 1.85 - 1.65 (m, 2H).

LC-MS (ESI): m/z = 523.2 $[M+H]^+$

**Compound 82-2:**

retention time: 1.360 min. $^1$H NMR (400 MHz, DMSO-*d6*) δ 7.36 (s, 1H), 7.12 (s, 1H), 6.84 (m, 1H), 6.38-6.33 (m, 2H), 3.79 (s, 2H), 3.58 - 3.32 (m, 10H), 3.19 - 2.97 (m, 8H), 2.97 - 2.80 (m, 2H), 2.44 - 2.22 (m, 3H), 2.17 (s, 2H), 1.85 - 1.65 (m, 2H).

LC-MS (ESI): m/z = 523.2 $[M+H]^+$

**Example 83**

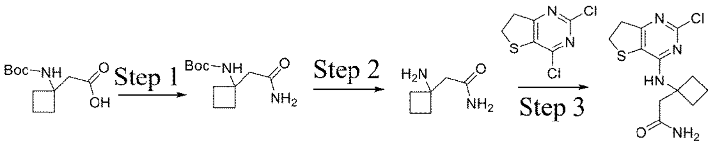


83A 83 B 83 C 83D

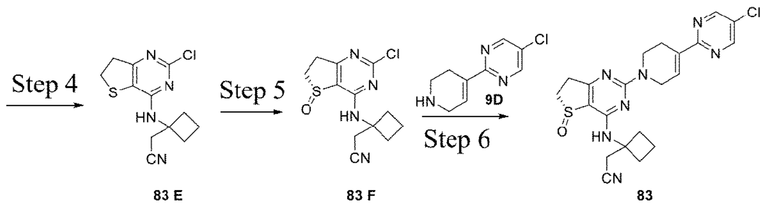


83 E 83 F 83

Step 1: Compound **83A** (5.0 g, 21.81 mmol) was dissolved in acetonitrile (50 mL), pyridine (5.18 g, 65.42 mmol) and di-tert-butyl dicarbonate (7.14 g, 32.71 mmol) were then added and stirred at room temperature for 4 hours, and aqueous ammonia (10 mL) was dropwise added. After the addition was complete, the system was reacted at room temperature for 4 hours. After the reaction was complete, the system was concentrated, a solid was filtered off, and after washing with acetonitrile, compound **83B** (4.5 g, 90%) was obtained.

LC-MS (ESI): m/z = 173.1 $[M+H-56]^+$

Step 2: Compound **83B** (4.5 g, 19.71 mmol) was dissolved in dichloromethane (40 mL), and trifluoroacetic acid (10 mL) was added and stirred at room temperature for 1 hour. The reaction liquid was concentrated to obtain crude compound **83C** trifluoroacetate, which was directly used in the next reaction without purification.

LC-MS (ESI): m/z = 129.1 $[M+H]^+$.

Step 3: The crude compound **83C** trifluoroacetate in the previous step was dissolved in N,N-dimethylformamide (50 mL), N,N-diisopropylethylamine (6.12 g, 29.57 mmol) and 2,4-dichloro-6,7-dihydrothieno[3,2-d]pyrimidine (12.74 g, 6.87 mmol) were added, and the mixture was heated to 80°C and reacted for 16 h. The reaction liquid was cooled to room temperature, diluted with ethyl acetate, and washed with water, and the organic phase was dried, filtered, concentrated, and subjected to column chromatography (dichloromethane/methanol (v/v) = 91/9) to obtain compound **83D** (720 mg, 12%).

LC-MS (ESI): m/z = 299.2 $[M+H]^+$

Step 4: Compound **83D** in the previous step was dissolved in dichloromethane (30 mL), triethylamine (490 mg, 4.82 mmol) was added in an ice bath and stirred for five minutes, and trifluoroacetic anhydride (760 mg, 3.62 mmol) was then dropwise added. After the addition was complete, the mixture was stirred in the ice bath for 2 hours. The reaction liquid was quenched by adding water and extracted with dichloromethane, and the organic phase was dried, filtered, concentrated, and subjected to column chromatography (dichloromethane/methanol (v/v) = 91/9) to obtain compound **83E** (440 mg, 65%).

LC-MS (ESI): m/z = 281.2 $[M+H]^+$

Step 5: Compound **83E** (500 mg, 1.78 mmol), (S)-(-)-1,1'-bi-2-naphthol (51.0 mg, 0.18 mmol), titanium tetraisopropoxide (25.0 mg, 0.088 mmol), and water (32.0 mg, 1.78 mmol) were dissolved in dichloromethane (20 mL). After nitrogen displacement three times, the system was stirred at room temperature for one hour, and tert-butyl hydroperoxide (256 mg, 1.99 mmol, 70% in water) was then added and stirred overnight. After quenching with a saturated sodium thiosulfate aqueous solution, the aqueous phase was extracted with dichloromethane, dried, concentrated, and subjected to column chromatography (dichloromethane/methanol (v/v) = 91/9) to obtain compound **83F** (520 mg, 98%).

LC-MS (ESI): m/z = 297.1 $[M+H]^+$

Step 6: Compound **83F** (520 mg, 1.75 mmol) was dissolved in 1,4-dioxane (20 mL), and compound **9D** (410 mg, 2.10 mmol) and N,N-diisopropylethylamine (680 mg, 5.27 mmol) were then added and stirred at 90°C overnight under nitrogen protection. After the reaction was complete, the reaction liquid was concentrated and subjected to reverse phase preparation (acetonitrile:water = 1/99-99/1) to obtain compound **83** (470 mg, 59%).

$^1$H NMR (400 MHz, DMSO-$d_6$) δ 8.89 (s, 2H), 8.17 (s, 1H), 7.39 - 7.21 (m, 1H), 4.45 (s, 2H), 3.97 (s, 2H), 3.53 - 3.41 (m, 1H), 3.37 (d, 2H), 3.27 - 3.16 (m, 1H), 3.04 - 2.85 (m, 2H), 2.66 (s, 2H), 2.48 - 2.32 (m, 2H), 2.30 - 2.20 (m, 2H), 2.02 - 1.75 (m, 2H).

LC-MS (ESI): m/z = 456.5 $[M+H]^+$

**Example 84**

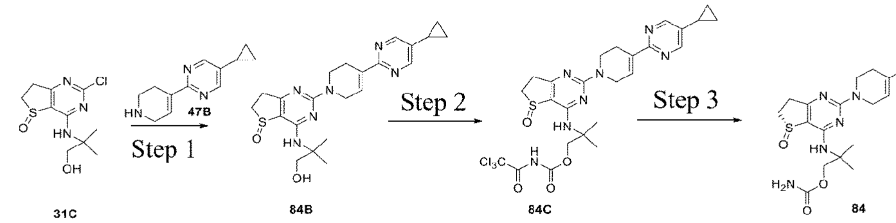


31C 84B 84C 84

Step 1: Compound **31C** (1.0 g, 3.63 mmol) was dissolved in 1,4-dioxane (30 mL), and compound **47B** (880 mg, 4.36 mmol) and N,N-diisopropylethylamine (1.40 g, 10.86 mmol) were added and stirred at 90°C overnight under nitrogen protection. After the reaction was complete, the reaction liquid was concentrated and subjected to silica gel column chromatography (dichloromethane/methanol (v/v) = 95/5) to obtain compound **84B** (1.6 g, 94%).

LC-MS (ESI): m/z = 441.6 $[M+H]^+$

Step 2: Compound **84B** (1.6 g, 3.63 mmol) was dissolved in dichloromethane (5 mL), and trichloroacetyl isocyanate (820 mg, 4.36 mmol) was added in an ice bath and stirred in the ice bath for one hour. The reaction liquid was concentrated to obtain compound **84C** (2.28 g, 100%).

Step 3: Compound **84C** (2.28 g, 3.63 mmol) was dissolved in methanol (20 mL), and potassium carbonate (1.51 g, 10.89 mmol) and water (20 mL) were added in an ice bath and stirred at room temperature 2.5 hours. The reaction liquid was diluted by adding water and extracted with dichloromethane, and the organic phases were combined, dried, filtered, concentrated, and subjected to SFC chiral resolution to obtain compound **84** (1.3 g, 74%).

Preparation method: instrument: Waters 150 Prep-SFC E, column: Chiralcel OX column (250mm × 30 mm, 10 μm), mobile phase: (phase A: $CO_2$, phase B: EtOH (0.1% $NH_3$•$H_2O$)); gradient: 50% mobile phase B, isocratic elution; flow rate: 100 mL/min, back pressure: 100 bar, column temperature: 25°C, wavelength: 220 nm, cycle time: 7.0 min, sample preparation: sample concentration 10 mg/ml, acetonitrile solution injection: 8 ml each time. Retention time: 1.499 minutes. After separation, the fraction was dried in a water bath at 35°C by a rotary evaporator to obtain a product. The solvent was dried by a lyophilizer at -80°C to obtain the final product **84.**

$^1$H NMR (400 MHz, DMSO-$d_6$) δ 8.54 (s, 2H), 7.19 (d, 1H), 6.70 - 6.40 (m, 3H), 4.46 (s, 2H), 4.30 (s, 2H), 4.00 (t, 2H), 3.48 - 3.38 (m, 1H), 3.28 - 3.23 (m, 1H), 3.01 - 2.92 (m, 1H), 2.92 - 2.82 (m, 1H), 2.67 (s, 2H), 1.98 - 1.88 (m, 1H), 1.46 (d, 6H), 1.10 - 0.96 (m, 2H), 0.91 - 0.77 (m, 2H).

LC-MS (ESI): m/z = 484.2 $[M+H]^+$

**Example 85**

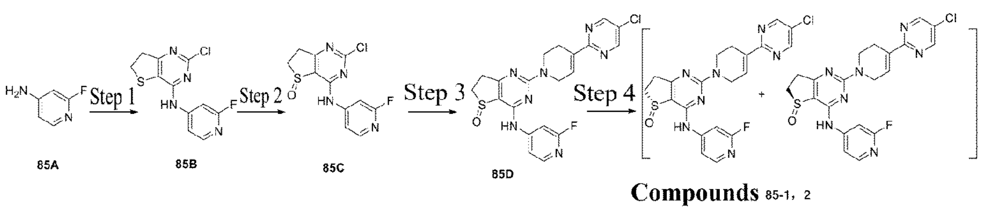


85A 85B 85C 85D

**Compounds** 85-1, 2

Using compounds **85A** and **24A** as raw materials, compound **85-1** (34.4 mg) and compound **85-2** (16.8 mg) were obtained according to the operation of Example 60.

**Analysis method:** instrument: SHIMADZU LC-20AD, column: Chiralpak IG-3 50 × 4.6 mm I.D., 3 μm; mobile phase: A: heptane (0.05%, N,N-diethylaniline), B: ethanol (0.05% N,N-diethylaniline); gradient: 20% B in A; flow rate: 1 mL/min, column temperature: 35°C, wavelength: 254 nm.

**Preparation method:** instrument: SHIMADZU LC-20AP, column: DAICEL CHIRALPAK IG (250 mm x 30 mm, 10 μm), mobile phase: A n-hexane, B ethanol (0.1% $NH_3$•$H_2O$); gradient: 20-60% B gradient elution, flow rate: 110 mL/min, column temperature: 25°C, wavelength: 254 nm, cycle time: 16 min, sample preparation: sample concentration 1.5 mg/ml, ethanol solution injection: 2 ml each time. After separation, the fraction was dried by a rotary evaporator at a bath temperature of 40°C to obtain compound **85-1** and compound **85-2**.

**Compound 85-1:**

$^1$H NMR (400 MHz, DMSO-*d6*) δ 8.91 (s, 2H), 8.14 - 8.12 (m, 1H), 7.80 - 7.75 (m, 1H), 7.63 (s, 1H), 7.33 - 7.32 (m, 1H), 4.66 (br s, 1H), 4.57 - 4.50 (m, 2H), 4.09 - 4.03 (m, 2H), 3.62 - 3.38 (m, 1H), 3.36 - 3.34 (m, 1H), 3.18 - 3.12 (m, 1H), 3.06

- 3.01 (m, 1H), 2.76 - 2.71 (m, 2H).

LCMS m/z = 458.1 [M+H]$^+$

**Compound 85-2:**

$^1$H NMR (400 MHz, DMSO-*d6*) δ 8.91 (s, 2H), 8.14 - 8.12 (m, 1H), 7.78 (s, 1H), 7.63 (s, 1H), 7.33 - 7.32 (m, 1H), 4.66 (br s, 1H), 4.54 (s, 2H), 4.06 (br s, 2H), 3.62 - 3.38 (m, 1H), 3.36 - 3.34 (m, 1H), 3.18 - 3.12 (m, 1H), 3.06 - 3.01 (m, 1H), 2.74 - 2.73 (m, 2H).

LCMS m/z = 458.1 [M+H]$^+$

**Example 88**

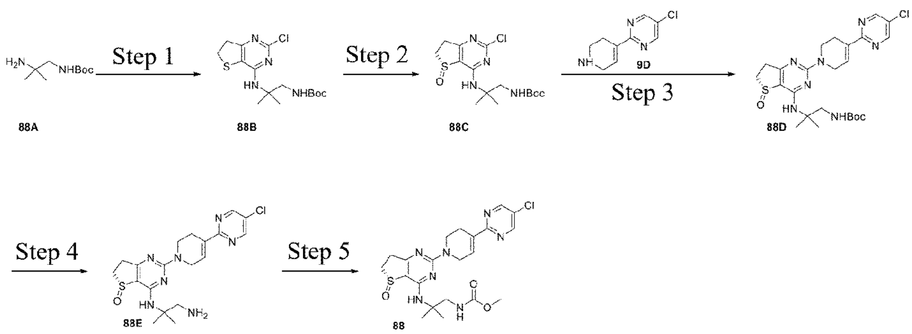


Step 1: Compound **88A** (10.0 g, 53.1 mmol) was dissolved in acetonitrile (100 mL), the compound 2,4-dichloro-6,7-dihydrothieno[3,2-D]pyrimidine (11.0 g, 53.1 mmol) and triethylamine (26.6 g, 265.0 mmol) were added, heated to 75°C, and reacted for 12 h, and the system was then cooled to room temperature, concentrated, and subjected to column chromatography (dichloromethane/methanol (v/v) = 92.5/7.5) to obtain compound **88B** (3.5 g, 18%).

LC-MS (ESI): m/z = 359.1 [M+H]$^+$

Step 2: Compound **88B** (3.5 g, 9.77 mmol), (S)-(-)-1,1'-bi-2-naphthol (0.28 g, 0.98 mmol), titanium tetraisopropoxide (0.13 g, 0.48 mmol), and water (0.17 g, 9.75 mmol) were dissolved in dichloromethane (25 mL). After nitrogen displacement three times, the system was stirred at room temperature for one hour, and tert-butyl hydroperoxide (0.96 mg, 10.7 mmol) was then added and stirred for 1.5 hours. After quenching with a saturated sodium thiosulfate aqueous solution, the aqueous phase was extracted with dichloromethane, dried, concentrated, and subjected to column chromatography (dichloromethane/methanol (v/v) = 91/9) to obtain compound **88C** (3.1 g, 95%).

LC-MS (ESI): m/z = 375.1 [M+H]$^+$

Step 3: Compound **88C** (2.0 g, 5.3 mmol) was dissolved in 1,4-dioxane (15 mL), compound **9D** (1.34 g, 5.8 mmol) and N,N-diisopropylethylamine (2.75 g, 21.2 mmol) were added, and the mixture was heated to 90°C and reacted for 12 h. The system was then cooled to room temperature, concentrated, and subjected to column chromatography (dichloromethane/methanol (v/v) = 91/9) to obtain compound **88D** (2.5 g, 88%).

LC-MS (ESI): m/z = 534.1 [M+H]$^+$

Step 4: Compound **88D** (2.5 g, 4.7 mmol) was dissolved in dichloromethane (10 mL), and trifluoroacetic acid (5.0 mL) was then added and reacted at room temperature for 2.5 hours. The system was then concentrated and subjected to column chromatography (dichloromethane/methanol (v/v) = 92.5/7.5) to obtain compound **88E** (1.9 g, 91%).

LC-MS (ESI): m/z = 434.1 [M+H]$^+$

Step 5: Compound **88E** (0.9 g, 2.0 mmol) was dissolved in dichloromethane (10 mL), and triethylamine (0.4 g, 4.0 mmol) was added, followed by methyl chloroformate (0.188 g, 2.0 mmol). The mixtue was reacted at room temperature for 1.5 hours, concentrated, and subjected to column chromatography (dichloromethane/methanol (v/v) = 91/9), followed by chiral resolution to obtain compound **88** (0.3 g, 30%).

Preparation method: instrument: Waters 150 Prep-SFC, column: Chiralcel OX Colum, mobile phase: (phase A: $CO_2$, phase B: 0.1% $NH_3$•$H_2O$ in MEOH and ACN); gradient: 40% mobile phase B, isocratic elution; flow rate: 120 mL/min, back pressure: 100 bar, column temperature: 25°C, wavelength: 220 nm, cycle time: 4.9 min, sample preparation: sample concentration 50 mg/ml, acetonitrile solution injection: 2 ml each time. After separation, the fraction was dried on a rotary evaporator at a bath temperature of 35°C to obtain **88** (retention time: 1.062 minutes).

$^1$H NMR (400 MHz, DMSO-*d6*) δ 8.89 (s, 2H), 7.45 (d, 1H), 7.30 (s, 1H), 6.70 (s, 1H), 4.50 (d, 2H), 4.01 (t, 2H), 3.57 (s, 3H), 3.46 - 3.38 (m, 2H), 3.34 (s, 1H), 3.25 - 3.20 (m, 1H), 2.99 - 2.93 (m, 1H), 2.89 - 2.84 (m, 1H), 2.67 (s, 2H), 1.45 (s,

3H), 1.42 (s, 3H).

LC-MS (ESI): m/z = 492.1 $[M+H]^+$

**Example 89**

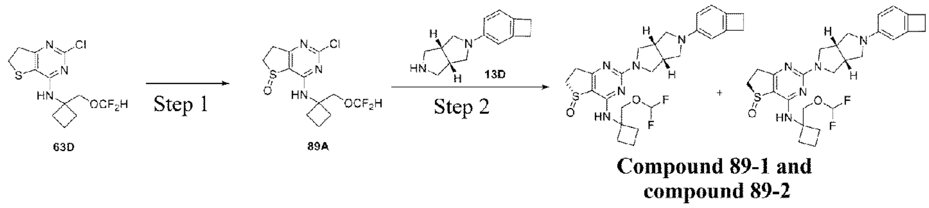


63D

89A

**Compound 89-1 and compound 89-2**

Step 1: Compound **63D** (0.8 g, 2.5 mmol) was dissolved in dichloromethane (5.0 mL), and m-chloroperoxybenzoic acid (0.43 g, 2.5 mmol) was then added and reacted at room temperature for 4 h. The system was concentrated and subjected to column chromatography (dichloromethane/methanol = 15/1) to obtain **89A** (0.67 g, 79%).

LC-MS (ESI): m/z = 338.1 $[M+1]^+$

Step 2: Compound **89A** (0.67 g, 2.0 mmol) was dissolved in 1,4-dioxane (15 mL), **13D** (0.45 g, 2.1 mmol) and N,N-diisopropylethylamine (0.78 g, 6.0 mmol) were then added, and the mixture was heated to 90°C and reacted for 12 h. The system was then cooled to room temperature, concentrated, and subjected to column chromatography (dichloromethane/methanol (v/v) = 91/9) to obtain the target compound, which was further subjected to chiral resolution to obtain compounds **89-1** (20 mg, 2%) and **89-2** (15 mg, 1%).

Preparation method: instrument: Waters 150 Prep-SFC, column: Chiralcel OX Colum, mobile phase: (phase A: CO2, phase B: 0.1% $NH_3$•$H_2O$ in MEOH and ACN); gradient: 40% mobile phase B, isocratic elution; flow rate: 120 mL/min, back pressure: 100 bar, column temperature: 25°C, wavelength: 220 nm, cycle time: 4.9 min, sample preparation: sample concentration 50 mg/ml, acetonitrile solution injection: 2 ml each time. After separation, the fraction was dried by a rotary evaporator at a bath temperature of 35°C to obtain compounds **89-1** (retention time: 0.952 minutes) and **89-2** (retention time: 1.062 minutes).

**Compound 89-1**

$^1$H NMR (400 MHz, DMSO-*d6*) δ 7.51 (s, 1H), 6.85 - 6.32 (m, 4H), 4.20 (s, 2H), 3.99 (s, 2H), 3.61 (s, 2H), 3.52 - 3.33 (m, 3H), 3.16 (s, 5H), 3.06 - 2.88 (m, 6H), 2.18 (d, 4H), 1.82 (d, 2H).

$^{19}$F NMR (377 MHz, DMSO-d6) δ -79.70 (s).

LC-MS (ESI): m/z = 516.1 $[M+H]^+$

**Compound 89-2:**

$^1$H NMR (400 MHz, DMSO-*d6*) δ 7.70 (s, 1H), 6.89 - 6.30 (m, 4H), 4.25 (s, 2H), 3.77 (s, 2H), 3.53 - 3.33 (m, 5H), 3.23 - 2.98 (m, 9H), 2.95 - 2.82 (m, 2H), 2.43 - 2.10 (m, 4H), 1.86 - 1.80 (m, 2H).

$^{19}$F NMR (377 MHz, DMSO-*d6*) δ -79.93 (s).

LC-MS (ESI): m/z = 516.1 $[M+H]^+$

**Example 90**

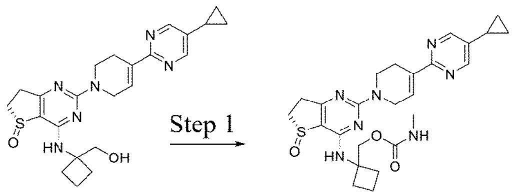


47C

90

Step 1: Compound **47C** (1.80 g, 4.0 mmol) was dissolved in N,N-dimethylformamide (20 mL), and triethylamine

(2.4 g, 24.0 mmol) and methylaminoformyl chloride (0.5 g, 5.2 mmol) were added. After nitrogen displacement three times, the mixture was heated to 70°C and reacted for 14 h. The system was then cooled to room temperature, concentrated, and subjected to column chromatography (dichloromethane/methanol (v/v) = 95/5) to obtain compound **90** (0.3 g, 15%).

$^1$H NMR (400 MHz, DMSO-d6) δ 8.54 (s, 2H), 7.58 (s, 1H), 7.18 (s, 1H), 6.96 - 6.93 (m, 1H), 4.43 (s, 4H), 3.98 - 3.95 (m, 2H), 3.48 - 3.39 (m, 1H), 3.25 - 3.17 (m, 1H), 2.99 - 2.93 (m, 1H), 2.89 - 2.84 (m, 1H), 2.64 (s, 2H), 2.56 (d, 3H), 2.43 - 2.32 (m, 2H), 2.29 - 2.21 (m, 2H), 1.97 - 1.90 (m, 1H), 1.86 - 1.82 (m, 2H), 1.05 - 1.01 (m, 2H), 0.86 - 0.81 (m, 2H).

LC-MS (ESI): m/z = 510.6 $[M+H]^+$

**Example 91**

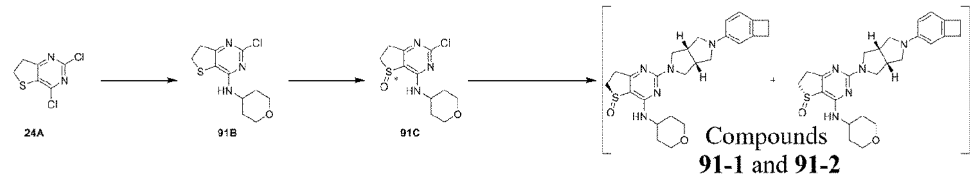


Compounds **91-1** and **91-2**

Step 1: At room temperature, compound **24A** (1.0 g, 4.83 mmol), 4-aminotetrahydropyran (0.59 g, 5.80 mmol), and acetonitrile (15 mL) were added to a 50 mL round-bottom flask, followed by triethylamine (1.22 g, 12.1 mmol), and the mixture was heated to 70°C and reacted for 14 h. After the reaction was complete, the system reaction liquid was cooled to room temperature, concentrated under reduced pressure, and then separated and purified by silica gel column chromatography (petroleum ether/ethyl acetate (v/v) = 100/0-65/35) to obtain the target compound **91B** (1.05 g, 80%).

LC-MS (ESI): m/z = 272.1 $[M+H]^+$

Step 2: At room temperature, **91B** (1.05 g, 3.86 mmol) and dichloromethane (10 mL) were added to a 50 mL round-bottom flask, followed by m-chloroperoxybenzoic acid (1.0 g, 5.81 mmol) slowly, and the mixture was continuously stirred at this temperature for 16 h. After the reaction was complete, the reaction was quenched with a saturated sodium thiosulfate aqueous solution. The aqueous phase was extracted twice with dichloromethane (20 mL), and the organic phases were combined, washed with a saturated sodium chloride aqueous solution, and dried over anhydrous sodium sulfate. After filtration, the reaction liquid was concentrated under reduced pressure and then separated and purified by silica gel column chromatography (petroleum ether/ethyl acetate (v/v) = 100/0-50/50) to obtain the target compound **91C** (0.95 g, 85%).

LC-MS (ESI): m/z = 288.3 $[M+H]^+$

Step 3: At room temperature, compound **91C** (260 mg, 0.90 mmol), compound **13D** (213 mg, 0.99 mmol), and 1,4-dioxane (5 mL) were added to a 25 mL round-bottom flask, and N,N-diisopropylethylamine (347 mg, 2.71 mmol) were added at room temperature, heated to 90°C and reacted for 12 h. After the reaction was complete, the reaction liquid was concentrated under reduced pressure and then separated and purified by silica gel column chromatography (dichloromethane/methanol (v/v) = 91/9) to obtain 230 mg of a racemate, which was separated by SFC to obtain two isomers, i.e., compound **91-1** (100 mg, retention time 1.15 min, 24%) and compound **91-2** (100 mg, retention time 1.91 min, 24%).

Separation conditions of preparative chromatography: 1. instrument: Waters 150 Prep-SFC F; 2. chromatographic column: Chiralcel OX Column; 3. mobile phase system: A for $CO_2$; B for 0.1% $NH_3$•$H_2O$ in MEOH and ACN; 4. gradient: B 70%; 5. flow rate: 100 mL/min.

**Compound 91-1:**

$^1$H NMR (400 MHz, Chloroform-d) δ 6.90 (d, 1H), 6.42 - 6.38 (m, 1H), 6.37 - 6.33 (m, 1H), 5.64 - 5.45 (m, 1H), 4.25 - 4.14 (m, 1H), 4.02 - 3.94 (m, 2H), 3.92 - 3.84 (m, 2H), 3.65 - 3.44 (m, 7H), 3.41 - 3.32 (m, 1H), 3.27 - 3.16 (m, 2H), 3.12 - 3.06 (m, 6H), 3.05 - 2.95 (m, 2H), 2.03 - 1.93 (m, 2H), 1.65 - 1.51 (m, 2H).

LC-MS (ESI): m/z = 466.7 $[M+H]^+$

**Compound 91-2:**

$^1$H NMR (400 MHz, Chloroform-*d*) δ 6.90 (d, 1H), 6.43 - 6.38 (m, 1H), 6.37 - 6.33 (m, 1H), 5.56 - 5.31 (m, 1H), 4.26 - 4.15 (m, 1H), 4.03 - 3.94 (m, 2H), 3.93 - 3.83 (m, 2H), 3.67 - 3.44 (m, 7H), 3.42 - 3.32 (m, 1H), 3.28 - 3.17 (m, 2H), 3.13 - 3.06 (m, 6H), 3.05 - 2.95 (m, 2H), 2.04 - 1.94 (m, 2H), 1.65 - 1.52 (m, 2H).

LC-MS (ESI): m/z = 466.7 $[M+H]^+$

**Example 92**

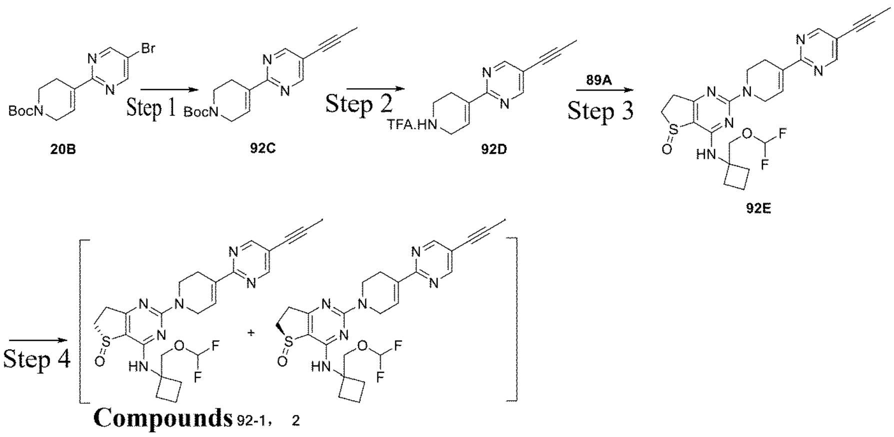


20B 92C 92D 92E

Compounds 92-1, 2

Step 1: Compound **20B** (1.00 g, 2.94 mmol), 1-(trimethylsilyl)propyne (660 mg, 5.88 mmol), potassium carbonate (1.02 g, 7.35 mmol), cuprous iodide (112 mg, 0.59 mmol), and [1,1'-bis(diphenylphosphine)ferrocene]palladium dichloride (160 mg, 0.44 mmol) were added to a reaction flask, followed by 20 mL of a 1,4-dioxane solution, and after nitrogen displacement, the mixture was heated to 85°C and reacted for 16 h. After the raw material disappeared as detected by TLC, the mixture was diluted by adding water (100 mL) and extracted three times with ethyl acetate (100 mL). The organic phases were combined, dried over anhydrous sodium sulfate, and then concentrated in vacuum to obtain a crude product, and the crude product was purified three times by column chromatography (eluents: PE:EA = 100:1 to 20:1) to obtain the target compound **92C** (200 mg, yield: 22.73%).

LC-MS (ESI): m/z = 244.10 $[M-56+H]^+$

$^1H$ NMR (400 MHz, $CDCl_3$) δ 8.65 (s, 2H), 7.20 - 7.19 (m, 1H), 4.18 - 4.17 (m, 2H), 3.64 - 3.61 (m, 2H), 2.69 - 2.68 (m, 2H), 1.49 (s, 9H).

Step 2: Compound **92C** (200 mg, 0.67 mmol) was dissolved in 8 mL of dichloromethane, trifluoroacetic acid (2 mL) was added, and the mixture was then stirred at 0°C for 30 min. After the raw material disappeared as detected by TLC, the reaction liquid was concentrated at room temperature to obtain crude **92D** (230 mg), which was directly used for the next reaction.

LC-MS (ESI): m/z = 200.20 $[M+H]^+$

Step 3: **89A** (200 mg, 0.59 mmol) was dissolved in 1,4-dioxane (8 mL), and **92D** (260 mg, 0.88 mmol) and N,N-diisopropylethylamine (310 mg, 2.38 mmol) were successively added. After the addition was complete, the reaction liquid was heated to 85°C and stirred for 16 h under nitrogen protection. After the reaction liquid naturally returned to room temperature, the reaction was diluted by adding water (40 mL) and extracted three times with ethyl acetate (50 mL), and the organic phases were combined, dried over anhydrous sodium sulfate, and then concentrated in vaccum to obtain a crude product, which was purified by column chromatography (dichloromethane/methanol (v/v) = 99/1-91/9) to obtain the target compound **92E** (250 mg, 84.35%).

LC-MS (ESI): m/z = 501.2 $[M+H]^+$

Step 4: Compound **92E** (250 mg, 0.50 mmol) was subjected to chiral resolution to obtain compound **92-1** (57.7 mg) and compound **92-2** (2.0 mg).

Analysis method: instrument: SHIMADZU LC-30AD, column: Chiralcel Whelk Column; mobile phase: A: $CO_2$, B: 0.05% DEA in IPA and ACN; gradient: 50% B in A; flow rate: 3 mL/min, column temperature: 35°C, wavelength: 220 nm.

Preparation method: instrument: Waters 150 Prep-SFC F, column: Chiralcel Whelk Column (250mm × 30 mm, 10 μm) mobile phase: A: $CO_2$, B: 0.1% $NH_3 \cdot H_2O$ in IPA and ACN; gradient: 45% B gradient elution, flow rate: 100 mL /min, column temperature: 25°C, wavelength: 220 nm, cycle time: 2.7 min, sample preparation: sample concentration 5 mg/ml, ethanol solution injection: 1 ml each time. After separation, the fraction was dried by a rotary evaporator at a bath temperature of 35°C to obtain compound **92-1** and compound **92-2**.

**Compound 92-1:**

$^1H$ NMR (400 MHz, $CDCl_3$) δ 8.66 (s, 2H), 7.29 - 7.27 (m, 1H), 6.42 - 6.04 (m, 1H), 5.54 (s, 1H), 4.49 (s, 2H), 4.36 - 4.33 (m, 1H), 4.19 - 4.17 (m, 1H), 4.07 - 4.01 (m, 2H), 3.65 - 3.57 (m, 1H), 3.44 - 3.37 (m, 1H), 3.06 - 2.98 (m, 2H), 2.77 (s, 2H), 2.45 - 2.27 (m, 4H), 2.11 (s, 3H), 2.02 - 1.87 (m, 2H).

LC-MS (ESI): m/z = 501.2 [M+H]$^+$

**Compound 92-2:**

$^1$H NMR (400 MHz, $CDCl_3$) δ 8.66 (s, 2H), 7.29 - 7.27 (m, 1H), 6.42 - 6.04 (m, 1H), 5.54 (s, 1H), 4.54 - 4.45 (m, 2H), 4.36 - 4.33 (m, 1H), 4.19 - 4.17 (m, 1H), 4.07 - 4.01 (m, 2H), 3.65 - 3.57 (m, 1H), 3.44 - 3.37 (m, 1H), 3.06 - 2.98 (m, 2H), 2.77 (s, 2H), 2.45 - 2.27 (m, 4H), 2.11 (s, 3H), 2.02 - 1.87 (m, 2H).
LC-MS (ESI): m/z = 501.2 [M+H]$^+$

**Example 93**

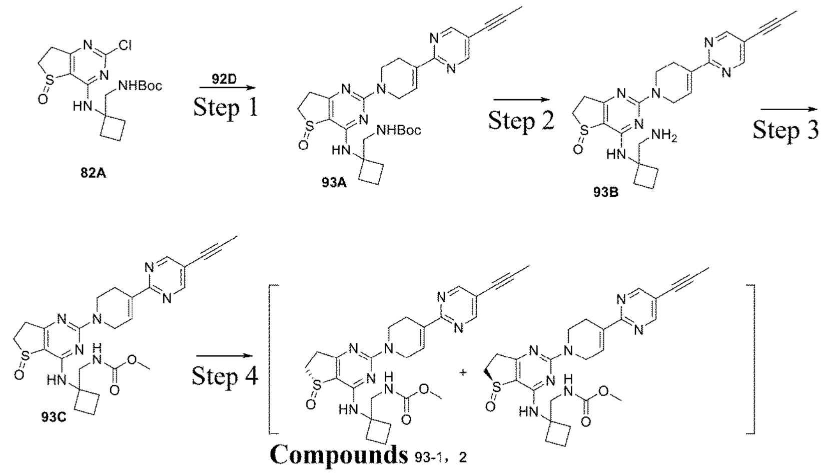


Compounds 93-1, 2

Using compounds **82A** and **92D** as raw materials, compound **93-1** and compound **93-2** were obtained according to the operation of Example 69.

**Compound 93-1:**

$^1$H NMR (400 MHz, $CDCl_3$) δ 8.66 (s, 2H), 7.29 - 7.28 (m, 1H), 5.76 - 5.71 (m, 2H), 4.51 (s, 2H), 4.05 (s, 2H), 3.75 - 3.68 (m, 2H), 3.64 (s, 3H), 3.63 - 3.57 (m, 1H), 3.43 - 3.39 (m, 1H), 3.07 - 2.99 (m, 2H), 2.78 (s, 2H), 2.35 - 2.25 (m, 4H), 2.11 (s, 3H), 1.97 - 1.92 (m, 2H).
LC-MS (ESI): m/z = 508.6 [M+H]$^+$

**Compound 93-2:**

$^1$H NMR (400 MHz, $CDCl_3$) δ 8.66 (s, 2H), 7.29 - 7.28 (m, 1H), 5.76 - 5.71 (m, 2H), 4.51 (s, 2H), 4.09 - 4.04 (m, 2H), 3.75 - 3.68 (m, 2H), 3.64 (s, 3H), 3.63 - 3.57 (m, 1H), 3.43 - 3.39 (m, 1H), 3.07 - 2.99 (m, 2H), 2.78 (s, 2H), 2.35 - 2.25 (m, 4H), 2.11 (s, 3H), 1.97 - 1.92 (m, 2H).
LC-MS (ESI): m/z = 508.6 [M+H]$^+$

Example **94**

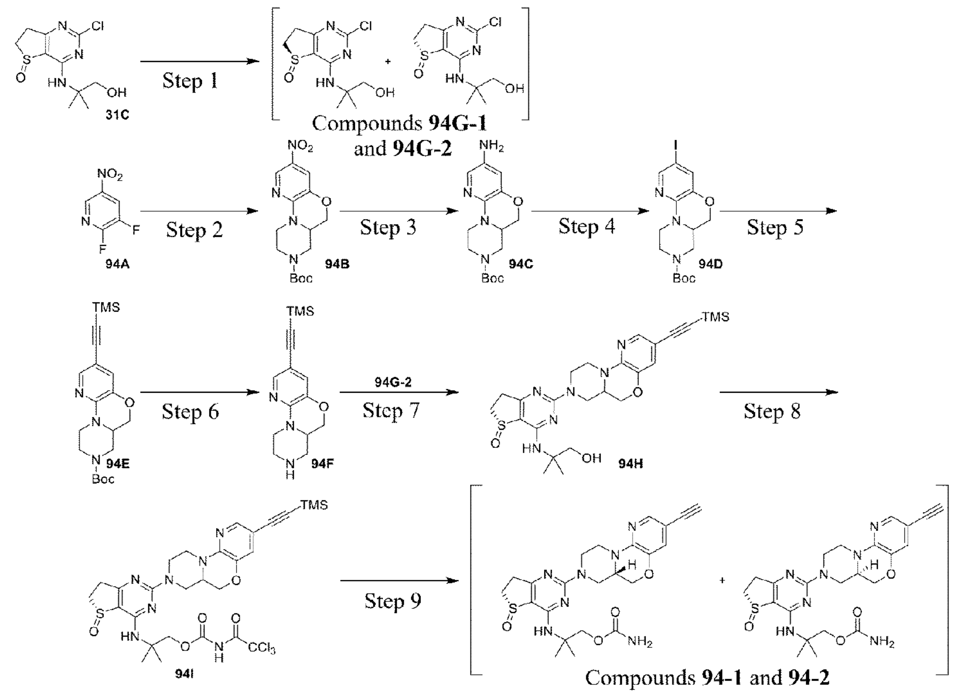


Step 1: Compound **31C** (3.0 g) was separated by SFC to obtain two isomers, i.e., compound **94G-1** (1.35 g, retention time 3.50 min, 45%) and compound **94G-2** (1.45 g, retention time 4.20 min, 48%).

Separation conditions of preparative chromatography: 1. instrument: Waters 150 Prep-SFC E; 2. chromatographic column: Chiralcel IC column

; 3. mobile phase system: A for $CO_2$; B for 0.1% $NH_3 \cdot H_2O$ in MEOH; 4. gradient: B 40%; and 5. flow rate: 180 mL/min.

LC-MS (ESI): m/z = 276.1 $[M+H]^+$.

Step 2: At room temperature, compound **91A** (5.0 g, 31.3 mmol), tert-butyl 3-(hydroxymethyl)piperazine-1-carboxylate (6.8 g, 31.3mmol), potassium hydroxide (5.3 g, 93.9 mmol), and N,N-dimethylformamide (100 mL) were successively added. The mixture was heated to 100°C and reacted for 15 h. After the reaction was complete, the reaction liquid was filtered, and the filtrate was extracted with ethyl acetate (500 mL). The organic phase was washed with water (100 mL × 3) and with a saturated sodium chloride aqueous solution (100 mL), and the organic phase was dried over anhydrous sodium sulfate. After filtration, the filtrate was concentrated under reduced pressure to obtain **94B** (10 g, 95%).

LC-MS (ESI): m/z = 281.1 $[M-^tBu+H]^+$

Step 3: At room temperature in a hydrogen atmosphere, compound **94B** (10 g, 29.76 mmol), palladium on carbon (2 g), and ethyl acetate (300 mL) were added to a 1 L round-bottom flask and reacted at room temperature for 12 hours. After the reaction was complete, the reaction liquid was filtered, and the filtrate was concentrated under reduced pressure to obtain **94C** (8.0 g, 88%).

LC-MS (ESI): m/z = 307.1 $[M+H]^+$

Step 4: At room temperature, tert-butyl nitrite (3.5 g,33.83 mmol), cuprous iodide (5.2 g, 27.06 mmol), and acetonitrile (30 mL) were successively added to a 100 mL round-bottom flask and heated to 65°C, and a solution of **94C** (6.9 g, 22.55 mmol) in acetonitrile (40 mL) was dropwise added. After the dropwise addition was complete, the mixture was reacted at 65°C for 6 h. After the reaction was complete, the reaction liquid was cooled to room temperature, concentrated under reduced pressure, and then separated and purified by silica gel column chromatography (eluents EA/PE (v/v) = 0-20%) to obtain **94D** (1.5 g, 16%).

LC-MS (ESI): m/z = 418.4 $[M+H]^+$

Step 5: At room temperature in a nitrogen atmosphere, compound **94D** (600 mg, 1.44 mmol), trimethylsilylacetylene (160 mg, 1.58 mmol), bis(triphenylphosphine)palladium dichloride (98 mg, 0.14 mmol), cuprous iodide (55 mg, 0.29 mmol), and tetrahydrofuran (10 mL) were added to a 50 mL round-bottom flask, followed by triethylamine (436 mg, 4.32 mmol), and the mixture was reacted at this temperature for 12 h. After the reaction was complete, water (5 mL) was added, and the mixture was extracted twice with ethyl acetate (10 mL). The organic phases were combined, washed with a saturated sodium chloride aqueous solution, and dried over anhydrous sodium sulfate. After filtration, the reaction liquid was concentrated under reduced pressure and then separated and purified by silica gel column chromatography (eluents: EA/PE (v/v) = 0-30%) to obtain the target compound **94E** (450 mg, 81%).

LC-MS (ESI): m/z = 388.4 $[M+H]^+$

Step 6: At room temperature, **94E** (450 mg, 1.16 mmol), dichloromethane (3 mL), and trifluoroacetic acid (1 mL)

were added to a 25 mL round-bottom flask, and the mixture was continuously stirred at this temperature for 2 h. After the reaction was complete, the reaction liquid was concentrated under reduced pressure to obtain the target compound **94F** (325 mg, 97%).

LC-MS (ESI): m/z = 288.3 $[M+H]^+$

Step 7: At room temperature, compound **94F** (200 mg, 0.70 mmol), compound **94G-2** (211 mg, 0.76 mmol), and 1,4-dioxane (5 mL) were added to a 25 mL round-bottom flask, and N,N-diisopropylethylamine (270 mg, 2.09 mmol) were added at room temperature, heated to 100°C and reacted for 12 h. After the reaction was complete, the reaction liquid was concentrated under reduced pressure and then separated and purified by silica gel column chromatography (eluents: MeOH/DCM (v/v) = 0-10%) to obtain the target compound **94H** (185 mg, 50%).

LC-MS (ESI): m/z = 527.7 $[M+H]^+$

Step 8: At room temperature, compound **94H** (185 mg, 0.35 mmol) and dichloromethane (5 mL) were added to a 25 mL round-bottom flask, and trichloroacetyl isocyanate (79 mg, 0.42 mmol) was added in an ice bath and stirred in the ice bath for 1 h. After the reaction was complete, the reaction liquid was concentrated under reduced pressure and then separated and purified by silica gel column chromatography (eluents: MeOH/DCM (v/v) = 0-10%) to obtain the target compound **94I** (160 mg, 64%).

LC-MS (ESI): m/z = 714.2 $[M+H]^+$

Step 9: At room temperature, compound **94I** (160 mg, 0.22 mmol) and methanol (2 mL) were added to a 25 mL round-bottom flask, and potassium carbonate (92 mg, 0.66 mmol) and water (2 mL) were added in an ice bath and reacted at room temperature for 2.5 h. After the reaction was complete, the reaction liquid was concentrated under reduced pressure and then separated and purified by silica gel column chromatography (eluents: MeOH/DCM (v/v) = 0-10%) to obtain 90 mg of a racemate, which was separated by SFC to obtain two isomers, i.e., compound **94-1** (30 mg, retention time 1.80 min, 27%) and compound **94-2** (30 mg, retention time 1.97 min, 27%).

Separation conditions of preparative chromatography: 1. instrument: Waters 150 Prep-SFC E; 2. chromatographic column: Chiralcel OJ column; 3. mobile phase system: A for $CO_2$; B for 0.1% $NH_3{\bullet}H_2O$ in MEOH; 4. gradient: B 35%; and 5. flow rate: 70 mL/min.

Compound **94-1:**

$^1H$ NMR (400 MHz, Chloroform-*d*) δ 7.95 (d, 1H), 7.03 (d, 1H), 5.51 (s, 1H), 4.90 - 4.80 (m, 2H), 4.66 - 4.60 (m, 1H), 4.38 - 4.32 (m, 3H), 4.03 - 3.98 (m, 1H), 3.63 - 3.53 (m, 1H), 3.49 - 3.36 (m, 2H), 3.17 - 3.08 (m, 1H), 3.07 - 2.95 (m, 3H), 2.92 - 2.85 (m, 1H), 2.83 - 2.76 (m, 1H), 1.50 (d, 6H).

LC-MS (ESI): m/z = 498.6$[M+H]^+$

Compound **94-2:**

$^1H$ NMR (400 MHz, Chloroform-*d*) δ 7.95 (d, 1H), 7.03 (d, 1H), 5.49 (s, 1H), 4.89 - 4.79 (m, 2H), 4.66 - 4.59 (m, 1H), 4.41 - 4.35 (m, 3H), 4.04 - 3.97 (m, 1H), 3.63 - 3.53 (m, 1H), 3.49 - 3.37 (m, 2H), 3.17 - 3.09 (m, 1H), 3.06 - 2.95 (m, 3H), 2.92 - 2.84 (m, 1H), 2.83 - 2.76 (m, 1H), 1.50 (d, 6H).

LC-MS (ESI): m/z = 498.6$[M+H]^+$

**Example 95**

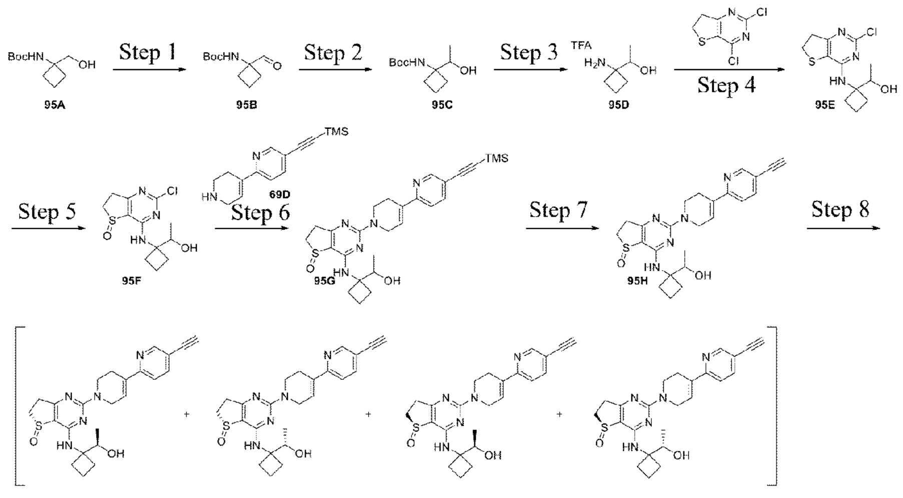


**Compounds** 95-1, 95-2, 95-3, 95-4

Step 1: Compound **95A** (10.0 g, 50.0 mmol) was dissolved in ethyl acetate (50.0 mL), 2-iodoxybenzoic acid (21.0 g, 75.0 mmol) was then added, and the mixture was heated to reflux and reacted overnight. The system was cooled to room temperature, filtered, and concentrated to obtain compound **95B** (10.0 g, 95%).

LC-MS (ESI): m/z = 200.1 $[M+H]^+$

Step 2: Compound **95B** (10.0 g, 50.0 mmol) was dissolved in tetrahydrofuran (50.0 mL), methylmagnesium bromide (3.0 M in THF, 100.0 mmol) was then added in an ice bath, and the mixture was slowly heated to room temperature and reacted for 4 hours. After quenching by adding saturated ammonium chloride, the system was extracted with ethyl acetate, dried, and concentrated to obtain compound **95C** (10.0 g, 92%).

LC-MS (ESI): m/z = 160.1 $[M+H-56]^+$

Step 3: Compound **95C** (10.0 g, 46.0 mmol) was dissolved in dichloromethane (50.0 mL), and trifluoroacetic acid (20.0 mL) was then added and reacted at room temperature for 2 hours. The system was then concentrated and subjected to column chromatography (dichloromethane/methanol = 10/1) to obtain compound **95D** (5.6 g, 56%).

LC-MS (ESI): m/z = 116.1 $[M+H]^+$

Step 4: Compound **95D** (5.6 g, 26.0 mmol) was dissolved in acetonitrile (40.0 mL), 2,4-dichloro-6,7-dihydrothieno[3,2-D]pyrimidine (5.1 g, 25.0 mmol), and triethylamine (10.4 g, 104.0 mmol) were then added, heated to 75°C, and reacted for 12 h. The system was then cooled to room temperature, concentrated, and subjected to column chromatography (dichloromethane/methanol = 10/1) to obtain compound **95E** (3.6 g, 48%).

LC-MS (ESI): m/z = 286.1 $[M+H]^+$

Step 5: Compound **95E** (3.6 g, 12.6 mmol), (S)-(-)-1,1'-bi-2-naphthol (360.0 mg, 1.3 mmol), titanium tetraisopropoxide (180.0 mg, 0.62 mmol), and water (229.0 mg, 12.1 mmol) were dissolved in dichloromethane (80.0 mL). After nitrogen displacement three times, the mixture was stirred at room temperature for 1 hour, tert-butyl hydroperoxide (1.2 g, 13.9 mmol) was added, stirred for 1.5 hours, then concentrated, and subjected to column chromatography (dichloromethane/methanol = 10/1) to obtain compound **95F** (1.2 g, 31%).

LC-MS (ESI): m/z = 302.1 $[M+H]^+$

Step 6: Compound **95F** (0.7 g, 2.3 mmol) was dissolved in 1,4-dioxane (10.0 mL), and **69D** (640.0 mg, 2.5 mmol) and diisopropylethylamine (0.93 g, 7.0 mmol) were then added, heated to 90°C and reacted for 12 h. The system was then cooled to room temperature, concentrated, and subjected to column chromatography (dichloromethane/methanol = 10/1) to obtain compound **95G** (1.0 g, 81%).

LC-MS (ESI): m/z = 522.1 $[M+H]^+$

Step 7: Compound **95G** (1.0 g, 1.9 mmol) was dissolved in methanol (10.0 mL), potassium carbonate (276.0 mg, 2.0 mmol) was then added, and the mixture was reacted at room temperature for 2 h, then concentrated, and subjected to column chromatography (dichloromethane/methanol = 10/1) to obtain compound **95H** (0.7 g, 70%).

LC-MS (ESI): m/z = 450.1 $[M+H]^+$

Step 8: Compound **95H** (1.1 g, 2.3 mmol) was subjected to chiral resolution.

Preparation method: instrument: SHIMADZU LC-20AP, column: Phenomenex C18, mobile phase: (A was 10 mmol $NH_4HCO_3$ in $H_2O$; B was acetonitrile); gradient: 28-58% mobile phase B, isocratic elution; flow rate: 25 mL/min, column temperature: 25°C, wavelength: 254 nm, sample preparation: sample concentration 60 mg/ml, acetonitrile/aqueous solution injection: 1.5 ml each time. After separation, the fraction was dried by a rotary evaporator at a bath temperature of 35°C to obtain compounds **95-1** (retention time: 1.328 minutes), **95-2** (retention time: 1.120 minutes), **95-3** (retention time: 1.388 minutes), and **95-4** (retention time: 1.421 minutes).

Compound **95-1**:

$^1$H NMR (400 MHz, DMSO-*d6*) δ 8.64 (d, 1H), 7.89 - 7.86 (m, 1H), 7.61 (d, 1H), 7.26 (d, 1H), 6.88 (s, 1H), 4.61 - 4.36 (m, 5H), 4.02 (s, 2H), 3.52 - 3.35 (m, 1H), 3.29 - 3.19 (m, 1H), 3.00 - 2.94 (m, 1H), 2.90 - 2.85 (m, 1H), 2.63 (s, 2H), 2.21 - 2.02 (m, 1H), 1.74 - 1.57 (m, 5H), 1.09 (s, 3H).
LC-MS (ESI): m/z = 450.1 $[M+H]^+$.

Compound **95-2:**

$^1$H NMR (400 MHz, DMSO-d6) δ 8.63 (d, 1H), 7.88 - 7.86 (m, 1H), 7.61 (d, 1H), 7.33 (s, 1H), 6.87 (s, 1H), 4.92 (d, 1H), 4.43 (s, 3H), 4.25 - 4.16 (m, 1H), 4.04 -3.89 (m, 2H), 3.53 - 3.35 (m, 1H), 2.98 - 2.84 (m, 2H), 2.64 (d, 2H), 2.47 - 2.16 (m, 5H), 1.88 - 1.65 (m, 2H), 1.01 (d, 3H).
LC-MS (ESI): m/z = 450.1 $[M+H]^+$

Compound 95-3:

$^1$H NMR (400 MHz, DMSO-d6) δ 8.64 (d, 1H), 7.89 - 7.86 (m, 1H), 7.61 (d, 1H), 6.89 (s, 1H), 6.43 (d, 1H), 4.83 (s, 1H), 4.44 (d, 3H), 4.13 - 4.07 (m, 1H), 4.01 (t, 2H), 3.48 - 3.33 (m, 2H), 3.04 - 2.81 (m, 2H), 2.65 (d, 2H), 2.20 - 2.04 (m, 1H), 1.82 - 1.66 (m, 3H), 1.66 - 1.49 (m, 2H), 1.21 (s, 3H).
LC-MS (ESI): m/z = 450.1 $[M+H]^+$

Compound **95-4:**

$^1$H NMR (400 MHz, DMSO-*d6*) δ 8.64 (d, 1H), 7.89 - 7.87 (m, 1H), 7.76 (s, 1H), 7.62 (d, 1H), 6.88 (s, 1H), 4.51 - 4.35 (m, 3H), 4.23 - 4.19 (m, 2H), 4.06 - 3.88 (m, 3H), 3.56 - 3.37 (m, 1H), 3.37 - 3.21 (m, 1H), 3.08 - 3.02 (m, 1H), 2.94 - 2.89 (m, 1H), 2.65 (s, 2H), 2.42 - 2.30 (m, 3H), 1.87 - 1.64 (m, 2H), 1.01 (d, 3H).
LC-MS (ESI): m/z = 450.1 $[M+H]^+$

**Example 96**

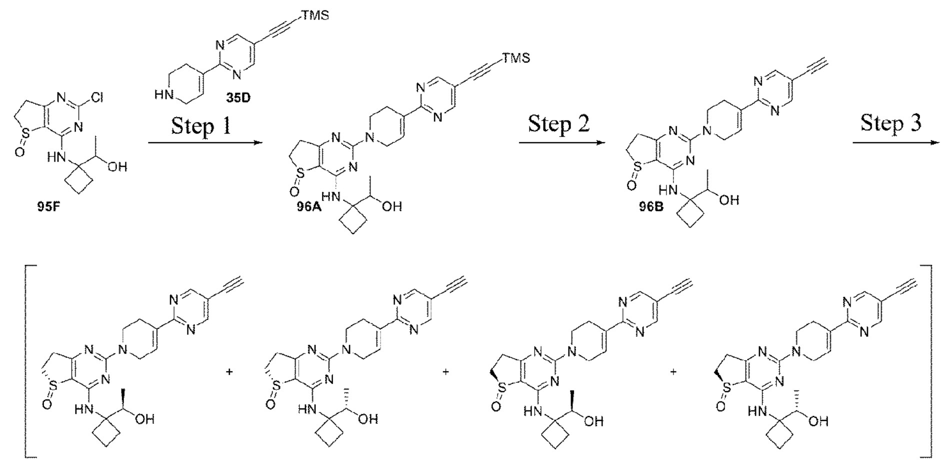


**Compounds** 96-1, 96-2, 96-3, 96-4

Step 1: Compound **95F** (1.2 g, 4.0 mmol) was dissolved in 1,4-dioxane (20.0 mL), and 35D (1.2 g, 4.5 mmol) and diisopropylethylamine (1.6 g, 12.0 mmol) were then added, heated to 90°C and reacted for 12 h. The system was then cooled to room temperature, concentrated, and subjected to column chromatography (dichloromethane/methanol = 10/1) to obtain compound 96A (1.2 g, 57%).
LC-MS (ESI): m/z = 523.1 $[M+H]^+$
Step 2: Compound **96A** (1.2 g, 2.3 mmol) was dissolved in methanol (10.0 mL), potassium carbonate (276.0 mg, 2.0 mmol) was then added, and the mixture was reacted at room temperature for 2 h, then concentrated, and subjected to column chromatography (dichloromethane/methanol = 10/1) to obtain compound **96B** (1.1 g, 100%).
LC-MS (ESI): m/z = 451.1 $[M+H]^+$

Step 3: Compound **96B** (1.1 g, 2.3 mmol) was subjected to chiral resolution.

Preparation method: instrument: SHIMADZU LC-20AP, column: Phenomenex C18, mobile phase: (phase A: 0.1% TFA in $H_2O$, phase B: ACN); gradient: 15-45% mobile phase B, isocratic elution; flow rate: 25 mL /min, column temperature: 25°C, wavelength: 254 nm, sample preparation: sample concentration 50 mg/ml, acetonitrile/aqueous solution injection: 1.5 ml each time. After separation, the fraction was dried by a rotary evaporator at a bath temperature of 35°C to obtain compounds **96-1** (retention time: 1.055 minutes), **96-2** (retention time: 1.040 minutes), **96-3** (retention time: 1.107 minutes), and **96-4** (retention time: 2.457 minutes).

Compound **96-1:**

$^1$H NMR (400 MHz, DMSO-*d6*) δ 8.91 (s, 2H), 7.60 (s, 1H), 7.33 (s, 1H), 4.69 (s, 1H), 4.50 (t, 2H), 4.24 - 4.20 (m, 1H), 4.07 - 3.99 (m, 2H), 3.96 - 3.91 (m, 2H), 3.47 - 3.41 (m, 1H), 3.32 - 3.19 (m, 1H), 3.04 - 2.98 (m, 1H), 2.92 - 2.87 (m, 1H), 2.67 (s, 2H), 2.42 - 2.29 (m, 3H), 1.79 -1.72 (m, 2H), 1.00 (d, 3H).

LC-MS (ESI): m/z = 451.1 $[M+H]^+$

Compound **96-2:**

$^1$H NMR (400 MHz, DMSO-*d6*) δ 8.91 (s, 2H), 7.57 (s, 1H), 7.33 (s, 1H), 4.69 (s, 1H), 4.50 (t, 2H), 4.24 - 4.20 (m, 1H), 4.07 - 3.99 (m, 2H), 3.97 - 3.92 (m, 2H), 3.47 - 3.41 (m, 1H), 3.32 - 3.19 (m, 2H), 3.04 - 2.98 (m, 1H), 2.91 - 2.86 (m, 1H), 2.67 (s, 2H), 2.42 - 2.29 (m, 3H), 1.79 -1.72 (m, 2H), 1.01 (d, 3H).

LC-MS (ESI): m/z = 451.1 $[M+H]^+$

Compound **96-3:**

$^1$H NMR (400 MHz, DMSO-*d6*) δ 8.91 (s, 2H), 7.34 (s, 1H), 6.55 (s, 1H), 4.69 (s, 1H), 4.51 (d, 2H), 4.16 (d, 1H), 4.01 (t, 2H), 3.50 - 3.41 (m, 3H), 3.12 - 2.83 (m, 2H), 2.69 (s, 2H), 2.22 - 2.01 (m, 1H), 1.75 - 1.69 (m, 3H), 1.57 (t, 2H), 1.20 (s, 3H).

LC-MS (ESI): m/z = 451.1 $[M+H]^+$

Compound **96-4:**

$^1$H NMR (400 MHz, DMSO-*d6*) δ 8.91 (s, 2H), 7.34 (s, 1H), 6.55 (s, 1H), 4.69 (s, 1H), 4.51 (d, 3H), 4.16 (d, 1H), 4.01 (t, 2H), 3.51 - 3.42 (m, 2H), 3.12 - 2.83 (m, 2H), 2.69 (s, 2H), 2.21 - 2.00 (m, 1H), 1.75 - 1.69 (m, 3H), 1.57 (t, 2H), 1.21 (s, 3H).

LC-MS (ESI): m/z = 451.1 $[M+H]^+$

**Example 97**

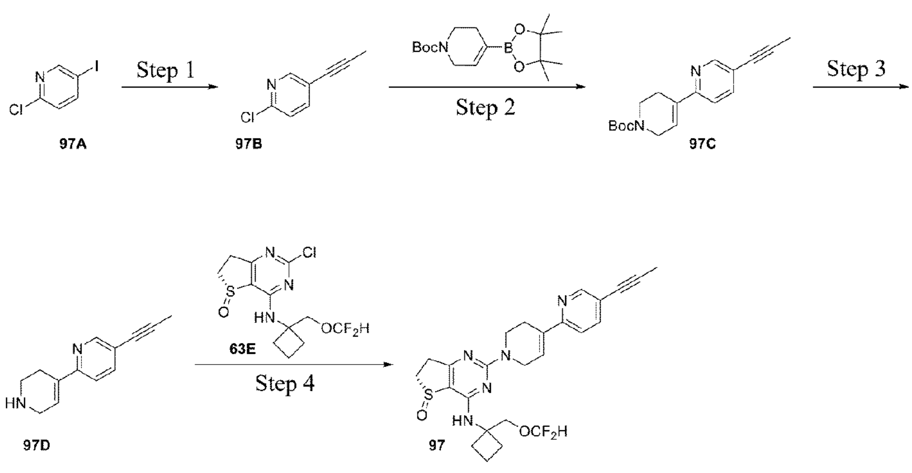


Step 1: Compound **97A** (2.0 g, 8.4 mmol) was dissolved in toluene (50 mL), and tributylpropynylstannane (3.1 g, 9.2 mmol) and tetrakis(triphenylphosphine)palladium (0.1 g, 0.8 mmol) were successively added. After nitrogen displacement three times, the system was heated to 100°C and reacted for 14 h. After the reaction was complete as monitored by a TLC plate, the system was concentrated and purified by a silica gel column (PE/EA = 10/1) to obtain the product **97B** (1.2 g, 94.4%).

$^1$H NMR (400 MHz, $CDCl_3$) δ 8.37 (s, 1H), 7.62 (d, 1H), 7.25 (d, 1H), 2.07 (s, 3H).

Step 2: Compound **97B** (1.2 g, 7.9 mmol) was dissolved in 1,4-dioxane (20 mL), and 1,1-bis(diphenylphosphine) ferrocenepalladium dichloride (0.6 g, 0.8 mmol), cesium carbonate (5.2 g, 15.8 mmol), N-Boc-1,2,5,6-tetrahydropyridine-4-boronic acid pinacol ester (2.9 g, 9.5 mmol), and water (4 mL) were then added. After nitrogen displacement three times, the mixture was heated to 90°C and reacted for 4 h, and the system was then passed through a short silica gel column, eluted with ethyl acetate, concentrated, and subjected to column chromatography (petroleum ether/ethyl acetate = 9/1) to obtain compound **97C** (2.0 g, 84.6%).

LC-MS (ESI): m/z = 299.4 [M+H]$^+$

Step 3: Compound **97C** (1.5 g, 5.0 mmol) was dissolved in dichloromethane (10 mL), trifluoroacetic acid (3.8 mL) was then added, and the mixture was reacted at room temperature for half an hour and then concentrated to obtain compound **97D** (0.9 g, 90.3%), which was directly used for the next step.

LC-MS (ESI): m/z = 199.4 [M+H]$^+$

Step 4: Compound **63E** (1.1 g, 3.2 mmol) was dissolved in 1,4-dioxane (20.0 mL), and compound **97D** (0.7 g, 3.5 mmol) and diisopropylethylamine (2.1 g, 16.0 mmol) were then added, heated to 90°C, and reacted for 12 h. The system was then cooled to room temperature, concentrated, and subjected to column chromatography (dichloromethane/methanol = 10/1) to obtain the target product, which was further subjected to chiral resolution to obtain compound **97** (20 mg, 2%).

Preparation method: instrument: Waters 150 Prep-SFC, column: Chiralcel OX Colum, mobile phase: (phase A: CO2, phase B: 0.1% $NH_3$•$H_2O$ in MEOH and ACN); gradient: 40% mobile phase B, isocratic elution; flow rate: 120 mL/min, back pressure: 100 bar, column temperature: 25°C, wavelength: 220 nm, cycle time: 4.9 min, sample preparation: sample concentration 50 mg/ml, acetonitrile solution injection: 2 ml each time. After separation, the fraction was dried on a rotary evaporator at a bath temperature of 35°C to obtain compound **97** (retention time: 0.952 minutes).

$^1$H NMR (400 MHz, DMSO-*d6*) δ 8.55 (d, 1H), 7.88 (s, 1H), 7.78 - 7.76 (m, 1H), 7.56 (d, 1H), 6.90 - 6.52 (m, 2H), 4.39 (s, 2H), 4.32 - 4.20 (m, 2H), 3.96 (s, 2H), 3.52 - 3.39 (m, 1H), 3.24 - 3.16 (m, 1H), 3.00 - 2.85 (m, 2H), 2.61 (s, 2H), 2.44 - 2.15 (m, 4H), 2.08 (s, 3H), 1.97 - 1.77 (m, 2H).

LC-MS (ESI): m/z = 500.1 [M+H]$^+$.

## Example 98

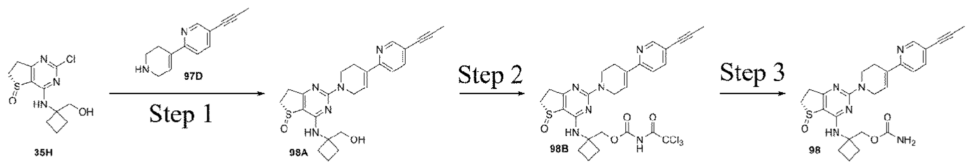


Step 1: Compound **35H** (1.68 g, 5.8 mmol) was dissolved in 1,4-dioxane (20.0 mL), and compound **97D** (1.4 g, 7.0 mmol) and diisopropylethylamine (3.0 g, 23.3 mmol) were then added, heated back to 90°C, and reacted for 12 h. The system was then cooled to room temperature, concentrated, and subjected to column chromatography (dichloromethane/methanol = 10/1) to obtain the target product, which was further subjected to chiral resolution to obtain compound 98A (2.5 g, 95%).

LC-MS (ESI): m/z = 450.1 [M+H]$^+$

Step 2: Compound **98A** (2.4 g, 5.3 mmol) was dissolved in dichloromethane (10.0 mL), and trichloroacetyl isocyanate (1.3 g, 6.9 mmol) was added in an ice bath and stirred in the ice bath for one hour. The reaction liquid was concentrated to obtain compound **98B** (2.5 g, 73%).

LC-MS (ESI): m/z = 637.1 [M+H]$^+$

Step 3: Compound **98B** (2.5 g, 3.9 mmol) was dissolved in methanol (20 mL), and potassium carbonate (2.2 g, 15.7 mmol) and water (20 mL) were added in an ice bath and stirred at room temperature 2.5 hours. The reaction liquid was diluted by adding water and extracted with dichloromethane, and the organic phases were combined, dried, filtered, and concentrated to obtain compound **98** (0.4 g, 20%).

$^1$H NMR (400 MHz, DMSO-*d6*) δ 8.55 (d, 1H), 7.78 - 7. 75 (m, 1H), 7.66 (s, 1H), 7.56 (d, 1H), 6.84 (s, 1H), 6.51 (s, 2H), 4.38 (d, 4H), 3.97 (t, 2H), 3.46 - 3.40 (m, 1H), 3.24 - 3.17 (m, 1H), 2.99 - 2.85 (m, 2H), 2.61 (s, 2H), 2.43 - 2.21 (m, 4H), 2.08 (s, 3H), 1.87 - 1.81 (m, 2H).

LC-MS (ESI): m/z = 493.1 [M+H]$^+$

**Example 99**

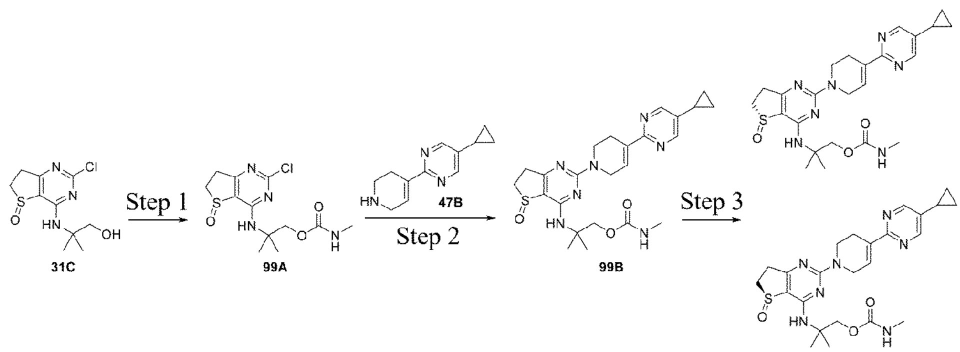


**Compound 99-1, Compound 99-2**

Step 1: Compound **31C** (2.5 g, 9.1 mmol) was dissolved in N,N-dimethylformamide (30 mL), and methylaminoformyl chloride (1.1 g, 11.0 mmol) and triethylamine (1.5 g, 13.6 mmol) were added and stirred at 65°C overnight under nitrogen protection. The reaction liquid was concentrated and subjected to silica gel column chromatography (dichloromethane/methanol (v/v) = 95/5) to obtain compound **99A** (1.1 g, 36.4%).

LC-MS (ESI): m/z = 333.8 $[M+H]^+$

Step 2: Compound **99A** (1.0 g, 3.0mmol) was dissolved in 1,4-dioxane (30 mL), and compound **47B** (660 mg, 3.3 mmol) and N,N-diisopropylethylamine (1.2 g, 9.0 mmol) were added and stirred at 90°C overnight under nitrogen protection. After the reaction was complete, the reaction liquid was concentrated and subjected to silica gel column chromatography (dichloromethane/methanol (v/v) = 95/5) to obtain compound **99B** (1.2 g, 78.5%).

LC-MS (ESI): m/z = 498.6 $[M+H]^+$

Step 3: Compound **101B** (1.3 mg) was subjected to chiral resolution to obtain compound **101** (208 mg).

Preparation method: instrument: Waters 150 Prep-SFC E, column: Chiralcel OJ column, mobile phase: (phase A: CO2, phase B: 0.1% $NH_3 \bullet H_2O$ in MEOH; gradient: 30% mobile phase B, isocratic elution; flow rate: 120 mL/min, back pressure: 100 bar, column temperature: 25°C, wavelength: 220 nm, cycle time: 4.2min, sample preparation: sample concentration 10 mg/ml, acetonitrile solution injection: 1.5 ml each time. After separation, the fraction was dried in a water bath at 35°C by a rotary evaporator to obtain a product. The solvent was dried with a freeze-dryer at -80°C to obtain a final product of compound **99-1** (retention time: 1.789 minutes) and compound **99-2** (retention time: 2.042 minutes).

$^1H$ NMR (400 MHz, DMSO-$d_6$) δ 8.54 (s, 2H), 7.19 (s, 1H), 7.09 - 7.04 (m, 1H), 6.61 (s, 1H), 4.46 (s, 2H), 4.35 - 4.28 (m, 2H), 3.99 (t, 2H), 3.47 - 3.37 (m, 1H), 3.28 - 3.21 (m, 1H), 2.99 - 2.96 (m, 1H), 2.90 - 2.84 (m, 1H), 2.67 (s, 2H), 2.56 (d, 3H), 1.97 1.90 (m, 1H), 1.45 (d, 6H), 1.06 - 1.01 (m, 2H), 0.86 - 0.82 (m, 2H).

$^1H$ NMR (400 MHz, DMSO-$d_6$) δ 8.54 (s, 2H), 7.19 (s, 1H), 7.09 - 7.04 (m, 1H), 6.62 (s, 1H), 4.46 (s, 2H), 4.35 - 4.28 (m, 2H), 3.99 (t, 2H), 3.46 - 3.38 (m, 1H), 3.28 - 3.23 (m, 1H), 2.99 - 2.93 (m, 1H), 2.89 - 2.84 (m, 1H), 2.67 (s, 2H), 2.56 (d, 3H), 1.97 1.90 (m, 1H), 1.45 (d, 6H), 1.04 - 1.01 (m, 2H), 0.86 - 0.82 (m, 2H).

**Example 100**

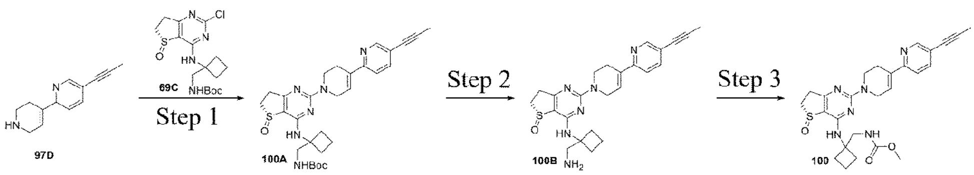


Step 1: Compound **97D** (0.9 g, 4.5 mmol) was dissolved in 1,4-dioxane (30.0 mL), compound **69C** (1.5 g, 4.0 mmol) and N,N-diisopropylethylamine (1.2 g, 9 mmol) were then added, and the mixture was heated to 90°C and reacted for 12 h. The system was then cooled to room temperature, concentrated, and subjected to column chromatography (dichloromethane/methanol = 10/1) to obtain compound **100A** (2.1 g, 84.2%).

LC-MS (ESI): m/z = 549.5 $[M+H]^+$

Step 2: Compound **100A** (2.1 g, 3.8 mmol) was dissolved in dichloromethane (15 mL), and trifluoroacetic acid (5 mL) was then added and reacted at room temperature for half an hour. The system was then concentrated and subjected to column chromatography (dichloromethane/methanol = 10/1) to obtain compound **100B** (1.5 g, 88.2%).

LC-MS (ESI): m/z = 449.5 $[M+H]^+$

Step 3: Compound **100B** (1.2 g, 2.7 mmol) was dissolved in dichloromethane (20.0 mL), and methyl chloroformate (0.3 g, 3.0 mmol) and triethylamine (0.8 g, 8.0 mmol) were then added and reacted at room temperature for half an hour. The system was concentrated, and subjected to column chromatography (dichloromethane/methanol = 10/1) to obtain compound **100** (1.5 g, 88.2%).

$^1H$ NMR (400 MHz, DMSO-$d_6$) δ 8.54 (d, 1H), 7.78 - 7.75 (m, 1H), 7.56 (d, 1H), 7.47 (s, 1H), 7.16 (t, 1H), 6.84 (s, 1H), 4.43 (s, 2H), 3.98 (t, 2H), 3.59 - 3.45 (m, 5H), 3.43 - 3.39 (m, 1H), 3.25 - 3.17 (m, 1H), 2.96 - 2.84 (m, 2H), 2.61 (s, 2H), 2.39 - 2.18 (m, 4H), 2.08 (s, 3H), 1.83 - 1.72 (m, 2H).

LC-MS (ESI): m/z = 507.1 $[M+H]^+$

**Example 101**

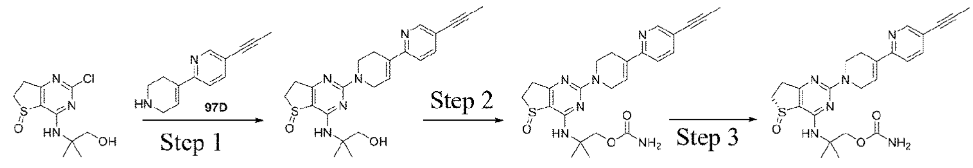


31C 101A 101B 101

Step 1: Compound **31C** (1.1 g, 4.0 mmol) was dissolved in 1,4-dioxane (30 mL), and compound **97D** (870 mg, 4.4 mmol) and N,N-diisopropylethylamine (1.6 g, 12 mmol) were added and stirred at 90°C overnight under nitrogen protection. After the reaction was complete, the reaction liquid was cooled to room temperature, then concentrated, and subjected to silica gel column chromatography (dichloromethane/methanol (v/v) = 95/5) to obtain compound **101A** (1.4 g, 80%).

LC-MS (ESI): m/z = 438.6 $[M+H]^+$

Step 2: Compound **101A** (1.6 g, 3.63 mmol) was dissolved in dichloromethane (5 mL), and trichloroacetyl isocyanate (820 mg, 4.36 mmol) was added in an ice bath and stirred in the ice bath for one hour. After the reaction liquid was concentrated to remove dichloromethane, methanol (20 mL) was added, and potassium carbonate (1.51 g, 10.89 mmol) and water (20 mL) were added in an ice bath and stirred at room temperature for 2.5 hours. The reaction liquid was diluted by adding water and extracted with dichloromethane, and the organic phases were combined, dried, filtered, concentrated, and subjected to silica gel column chromatography (dichloromethane/methanol (v/v) = 95/5) to obtain compound **101B** (1.3 g, 74%).

LC-MS (ESI): m/z = 481.6 $[M+H]^+$

Step 3: Compound **101B** (1.3 g) was subjected to chiral resolution to obtain compound **101** (208 mg).

Preparation method: instrument: Waters 150 Prep-SFC F, column: Chiralcel OJ Column, mobile phase: (phase A: CO2, phase B: 0.1% $NH_3$•$H_2O$ in MEOH; gradient: 35% mobile phase B, isocratic elution; flow rate: 100 mL/min, back pressure: 100 bar, column temperature: 25°C, wavelength: 220 nm, cycle time: 6min, sample preparation: sample concentration 50 mg/ml, acetonitrile solution injection: 4 ml each time. Retention time: 2.485 minutes. After separation, the fraction was dried in a water bath at 35°C by a rotary evaporator to obtain a product. The solvent was dried by a lyophilizer at -80°C to obtain the final product **101.**

$^1H$ NMR (400 MHz, DMSO-$d_6$) δ 8.55 (d, 1H), 7.78 - 7.76 (m, 1H), 7.57 (d, 1H), 6.85 (d, 1H), 6.69 (t, 1H), 6.55 (s, 2H), 4.44 (s, 2H), 4.29 (s, 2H), 4.00 (t, 2H), 3.41- 3.38 (m, 1H), 3.29 - 3.22 (m, 1H), 2.99 - 2.96 (m, 1H), 2.90 - 2.84 (m, 1H), 2.64 (s, 2H), 2.08 (s, 3H), 1.46 (d, 6H).

LC-MS (ESI): m/z = 481.6 $[M+H]^+$

**Example 102**

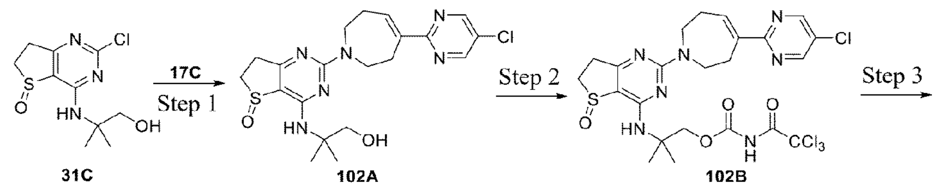


31C 102A 102B

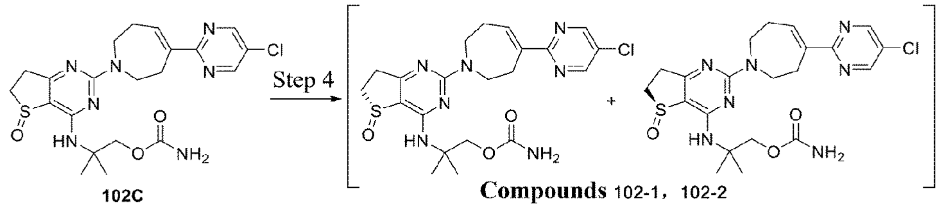


102C **Compounds** 102-1, 102-2

Step 1: **31C** (300 mg, 1.09 mmol) was dissolved in 1,4-dioxane (10 mL), and **17C** (320 mg, 1.31 mmol) and N,N-diisopropylethylamine (420 mg, 3.26 mmol) were successively added. After the addition was complete, the reaction liquid was heated to 85°C and stirred for 16 h under nitrogen protection. After the reaction liquid naturally returned to room temperature, the reaction was diluted by adding water (50 mL) and extracted three times with ethyl acetate (40 mL), and the organic phases were combined, dried over anhydrous sodium sulfate, and then concentrated in vaccum to obtain a crude product, which was purified by column chromatography (eluents: DCM:MeOH = 100:1 to 10:1) to obtain the target compound **102A** (350 mg, 71.66%).

LC-MS (ESI): m/z = 449.2 $[M+H]^+$

Step 2: Under nitrogen protection, compound **102A** (120 mg, 0.27 mmol) was dissolved in dichloromethane (12 mL), and trichloroacetyl isocyanate (60 mg, 0.32 mmol) was slowly dropwise added under stirring in an ice bath. After the addition was complete, the reaction liquid was stirred at 0°C for 1 h. After the raw material disappeared as monitored by a TLC spotting plate (developing agent: DCM/MeOH = 10:1), the reaction liquid was concentrated under reduced pressure to obtain crude product **102B** (170 mg), which was directly used in the next reaction.

LC-MS (ESI): m/z = 636.0 $[M+H]^+$

Step 3: Crude compound **102B** (170 mg, 0.27 mmol) was dissolved in methanol (8 mL), and potassium carbonate (110 mg, 0.80 mmol) was added at room temperature. After the addition was complete, the mixture was stirred at room temperature 1.5 h under nitrogen protection. After the reaction was complete, the reaction liquid was concentrated under reduced pressure to obtain a crude product, and the crude product was purified by column chromatography (eluents: DCM:MeOH = 100:1 to 9:1), followed by further purification through a reverse phase column (eluents: water:acetonitrile = 100:1 to 1:9) to obtain the target compound **102C** (120 mg, 91.44%).

LC-MS (ESI): m/z = 492.1 $[M+H]^+$

Step 4: Compound **102C** (120 mg, 0.24 mmol) was subjected to chiral resolution to obtain compound **102-1** (18.4 mg) and compound **102-2** (14.2 mg).

Analysis method: instrument: SHIMADZU LC-30AD, column: Chiralcel Whelk Column; mobile phase: A: $CO_2$, B: 0.05% DEA in IPA and ACN; gradient: 50% B in A; flow rate: 3 mL/min, column temperature: 35°C, wavelength: 220 nm.

Preparation method: instrument: Waters 150 Prep-SFC F, column: Chiralcel Whelk Column (250mm $\times$ 30 mm, 10 $\mu$m) mobile phase: A: $CO_2$, B: 0.1% $NH_3{\bullet}H_2O$ in IPA and ACN; gradient: 55% B gradient elution, flow rate: 60 mL /min, column temperature: 25°C, wavelength: 220 nm, cycle time: 4.2 min, sample preparation: sample concentration 5 mg/ml, ethanol solution injection: 1 ml each time. After separation, the fraction was dried by a rotary evaporator at a bath temperature of 35°C to obtain compound **102-1** and compound **102-2.**

Compound **102-1:**

$^1H$ NMR (400 MHz, $CDCl_3$) $\delta$ 8.53 (s, 2H), 7.33 - 7.29 (m, 1H), 5.30 (s, 1H), 4.32 - 4.23 (m, 2H), 3.99 - 3.88 (m, 4H), 3.56 - 3.48 (m, 1H), 3.33 - 3.26 (m, 1H), 3.08 - 3.02 (m, 2H), 2.96 - 2.83 (m, 3H), 2.61 2.56 (m, 1H), 1.67 - 1.59 (m, 2H), 1.45 - 1.43 (m, 6H).

LC-MS (ESI): m/z = 492.1 $[M+H]^+$

Compound **102-2:**

$^1H$ NMR (400 MHz, $CDCl_3$) $\delta$ 8.53 (s, 2H), 7.33 - 7.29 (m, 1H), 5.30 (s, 1H), 4.32 - 4.23 (m, 2H), 4.03 - 3.88 (m, 4H), 3.56 - 3.48 (m, 1H), 3.33 - 3.26 (m, 1H), 3.08 - 3.02 (m, 2H), 2.96 - 2.83 (m, 3H), 2.63 - 2.56 (m, 1H), 1.67 - 1.59 (m, 2H), 1.45 - 1.43 (m, 6H).

LC-MS (ESI): m/z = 492.1 $[M+H]^+$

**Example 103**

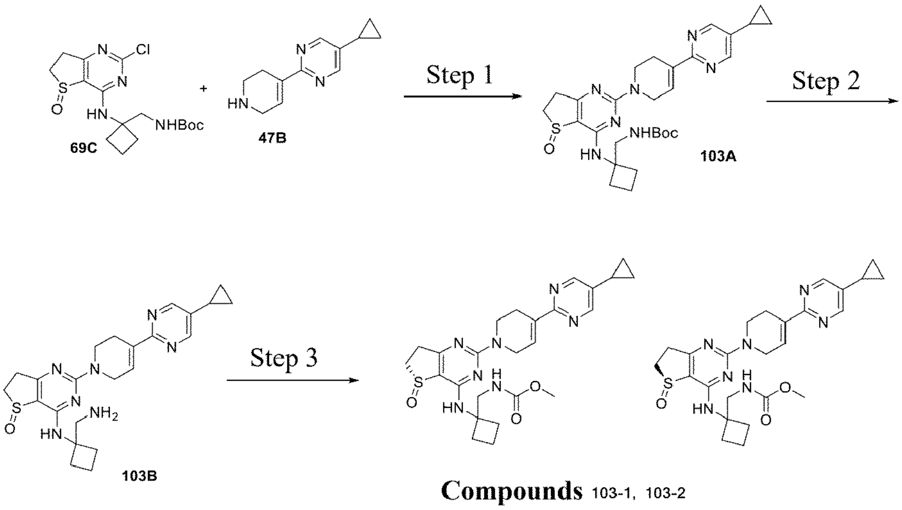

69C
47B
103A
103B

**Compounds** 103-1, 103-2

Step 1: Intermediate **69C** (450 mg, 1.16 mmol), intermediate **47B** (280 mg, 1.39 mmol), and N,N-diisopropylethylamine (748 mg, 5.80 mmol) were suspended in 1,4-dioxane (5 mL) and reacted at 80°C for 16 h under nitrogen protection, and the reaction liquid was cooled to room temperature and then dripped into 10 mL of water. The system was extracted twice with 10 mL of dichloromethane, and the organic phases were combined, then dried, concentrated, and separated by silica gel column chromatography (DCM:MeOH = 15:1) to obtain compound **103A** (600 mg, 94%).
LCMS(ESI): m/z = 552.2 $[M+H]^+$
Step 2: Compound **103A** (600 mg, 1.08 mmol) was dissolved in 4 mL of a solution of hydrogen chloride dioxane (4 M) and stirred at room temperature for 3 h. After the reaction was complete as monitored by LCMS, the system was concentrated under reduced pressure and dried, and crude **103B** (580 mg) was directly used for the next step.
LCMS (ESI): m/z = 452.3 $[M+H]^+$
Step 3: Crude **103B** (580 mg, 1.08 mmol calculated based on the 100% yield of the second step) was dissolved in 20 mL of dichloromethane, and triethylamine (218 mg, 2.16 mmol) and methyl chloroformate (124 mg, 1.30 mmol) were added at 0°C under stirring and reacted in an ice bath for 1 h. After the reaction was complete as monitored by LCMS, 5 mL of water was added, and the mixture was extracted with dichloromethane (10 mL x 2). The organic phases were combined, dried over anhydrous sodium sulfate, and concentrated under reduced pressure. After concentration, the system was separated by silica gel column chromatography (DCM:MeOH = 20:1), followed by SFC separation to obtain two optically pure isomers **103-1** (17.2 mg, 3%, with absolute configuration unknown) and **103-2** (108.2 mg, 20%, with absolute configuration unknown).

Chiral separation method:

instrument: Instrument: Waters 150 Prep-SFC F, chiral column: Chiralcel Whelk-Column mobile phase: A for CO2; B for 0.1% $NH_3$•$H_2O$ in MEOH and CAN, gradient: B 50%;
flow rate: 100 mL/min, column pressure: 100 bar, column temperature: 25°C, detection wavelength: 220 nm, cycle time: 3.0 min.

Compound **103-1:**

retention time: 0.832 min, $^1H$ NMR (400 MHz, DMSO-d6) δ 8.54 (s, 2H), 7.49 (s, 1H), 7.19 (s, 2H), 4.44 (s, 2H), 3.97 (t, 2H), 3.61 - 3.35 (m, 6H), 3.25-3.17 (m, 1H), 3.01 - 2.83 (m, 2H), 2.65 (s, 2H), 2.42 - 2.16 (m, 4H), 1.98-1.88 (m, 1H), 1.85-1.75 (m, 2H), 1.06 - 0.96 (m, 2H), 0.89 - 0.75 (m, 2H).
LCMS (ESI): m/z = 510.2 $[M+H]^+$

Compound **103-2:**

retention time: 0.990 min, $^1$H NMR (400 MHz, DMSO-d6) δ 8.54 (s, 2H), 7.49 (s, 1H), 7.19 (s, 2H), 4.44 (s, 2H), 3.97 (t, 2H), 3.61 - 3.35 (m, 6H), 3.25-3.17 (m, 1H), 3.01 - 2.83 (m, 2H), 2.65 (s, 2H), 2.42 - 2.16 (m, 4H), 1.98-1.88 (m, 1H), 1.85-1.75 (m, 2H), 1.06 - 0.96 (m, 2H), 0.89 - 0.75 (m, 2H).

LCMS (ESI): m/z = 510.2 $[M+H]^+$

Example **104**

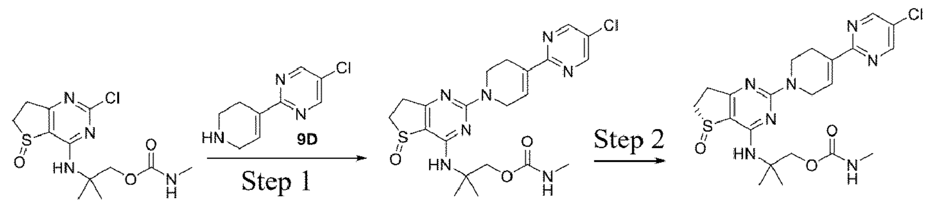


99A 104A **Compound** 104

Step 1: Compound **99A** (600 mg, 1.8 mmol) was dissolved in 1,4-dioxane (20 mL), and compound **9D** (420 mg, 2.16 mmol) and N,N-diisopropylethylamine (1.4 g, 10.8 mmol) were added and stirred at 90°C overnight under nitrogen protection. After the reaction was complete, the reaction liquid was concentrated and subjected to silica gel column chromatography (dichloromethane/methanol (v/v) = 95/5) to obtain compound **104A** (500 mg, 56.5%).

LC-MS (ESI): m/z = 492.1 $[M+H]^+$

Step 2: Compound **104A** (500 mg) was subjected to chiral resolution to obtain compound **104** (282 mg, 56.4%).

Preparation method: instrument: Waters 150 Prep-SFC F, column: Chiralcel Whelk-Column, mobile phase: (phase A: $CO_2$, phase B: 0.1% $NH_3$•$H_2O$ in IPA and ACN; gradient: 45% mobile phase B, isocratic elution; flow rate: 100 mL/min, back pressure: 100 bar, column temperature: 25°C, wavelength: 220 nm, cycle time: 3.1 min, sample preparation: sample concentration 10 mg/ml, acetonitrile solution injection: 5.0 ml each time. After separation, the fraction was dried in a water bath at 35°C by a rotary evaporator to obtain a product. The solvent was dried with a freeze-dryer at -80°C to obtain a final product of compound **104** (retention time: 0.867 minutes).

LC-MS (ESI): m/z = 492.1 $[M+H]^+$

$^1$H NMR (400 MHz, DMSO-$d_6$) δ 8.90 (s, 2H), 7.30 (d, 1H), 7.10 (d, 1H), 6.69 (s, 1H), 4.49 (s, 2H), 4.41 - 4.26 (m, 2H), 4.00 (t, 2H), 3.50 - 3.39 (m, 1H), 3.32 - 3.21 (m, 1H), 3.02 - 2.92 (m, 1H), 2.92 - 2.82 (m, 1H), 2.67 (s, 2H), 2.56 (d, 3H), 1.45 (d, 6H).

Example **105**

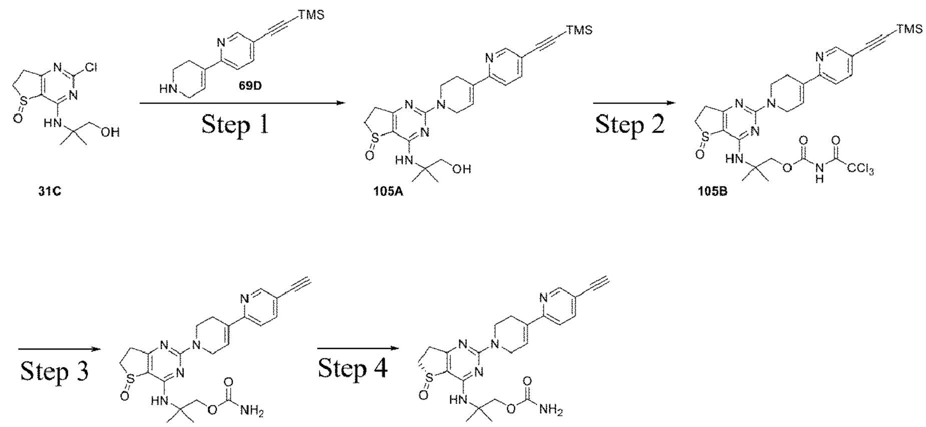


31C 105A 105B 105C 105

Step 1: Compound **31C** (1.6 g, 5.8 mmol) was dissolved in 1,4-dioxane (30 mL), and compound **69D** (1.5 g, 5.8 mmol) and N,N-diisopropylethylamine (2.2 g, 17.4 mmol) were then added and stirred at 90 degrees Celsius overnight

under nitrogen protection. After the reaction was complete, the reaction liquid was concentrated and subjected to silica gel column chromatography (dichloromethane:methanol = 20:1) to obtain compound **105A** (2.4 g, 83%).

LC-MS (ESI): m/z = 496.1 $[M+H]^+$

Step 2: Compound **105A** (2.4 g, 4.8 mmol) was dissolved in dichloromethane (10.0 mL), trichloroacetyl isocyanate (1.2 g, 6.3 mmol) was added in an ice bath and stirred in the ice bath for one hour, and the reaction liquid was concentrated to obtain compound **105B** (2.5 g, 75%), which was directly used for the next step.

Step 3: Compound **105B** (2.5 g, 3.6 mmol) was dissolved in methanol (20 mL), and potassium carbonate (2.0 g, 14.6 mmol) and water (20 mL) were added in an ice bath and stirred at room temperature 2.5 hours. The reaction liquid was diluted by adding water and extracted with dichloromethane, and the organic phases were combined, dried, filtered, and concentrated to obtain compound **105C** (1.5 g, 84%).

LC-MS (ESI): m/z = 467.1 $[M+H]^+$

Step 4: Compound **105C** was subjected to chiral SFC resolution to obtain compound **105** (1.0 g).

Preparation method: instrument: Waters 150 SFC, column: Chiralcel AD column, mobile phase: (A for $CO_2$ and B for 0.1% $NH_3$•$H_2O$ in IPA and ACN; gradient: 55% mobile phase B, isocratic elution; flow rate: 100 mL/min, back pressure: 100 bar, column temperature: 25°C, wavelength: 220 nm, cycle time: 7.8 min, sample preparation: sample concentration 10 mg/ml, acetonitrile methanol mixed solution injection: 3.5 ml each time. retention time: 0.821 minutes. After separation, the fraction was dried in a water bath at 35°C by a rotary evaporator to obtain a product. The solvent was dried by a lyophilizer at -80°C to obtain the final product **105.**

LC-MS (ESI): m/z = 467.1 $[M+H]^+$

$^1H$ NMR (400 MHz, DMSO-$d_6$) δ 8.64 (d, 1H), 7.89 - 7.86 (m, 1H), 7.62 (d, 1H), 6.90 (s, 1H), 6.70 - 6.55 (m, 3H), 4.45 (s, 3H), 4.30 (s, 2H), 4.01 (t, 2H), 3.47 - 3.39 (m, 1H), 3.29 - 3.22 (m, 1H), 3.03 - 2.80 (m, 2H), 2.65 (s, 2H), 1.46 (d, 6H).

Example **106**

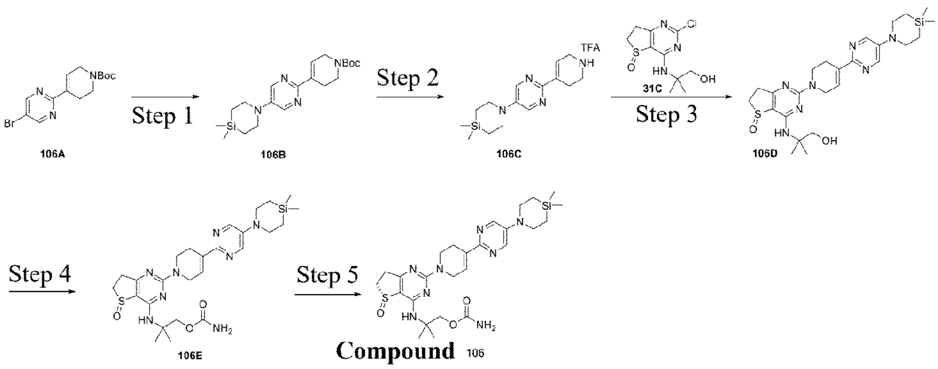


106A 106B 106C 106D 106E Compound 106

Step 1: Compound **106A** (5.0 g, 14.7 mmol), 4,4-dimethyl-[1,4]silapiperidine hydrochloride (2.9 g, 17.6 mmol), bis(dibenzylideneacetone)palladium (0.7 mmol, 0.4 g), 2-(di-tert-butylphosphine)biphenyl (1.5 mmol, 0.4 g), and sodium tert-butoxide (44.1 mmol, 4.2 g) were dissolved in toluene (120 mL) and stirred at 100°C overnight under nitrogen protection. The reaction liquid was concentrated and subjected to silica gel column chromatography (dichloromethane/-methanol (v/v) = 4/1) to obtain compound **106B** (2.8 g, 49.1%).

$^1H$ NMR (400 MHz, DMSO-*d6*) δ 8.29 (s, 2 H), 6.88 (s, 1 H), 3.89 - 3.63 (m, 6 H), 2.70 (s, 2 H), 1.49 (s, 9 H), 0.84 - 0.74 (m, 4 H), 0.10 (s, 6 H).

Step 2: Compound **106B** (2.8 g, 7.2 mmol) was dissolved in dichloromethane (20 mL), trifluoroacetic acid (10 mL) was then added, and the mixture was reacted at room temperature for half an hour and then concentrated to obtain compound **106C** (2.7 g, 96.3%), which was directly used for the next step.

Using compounds **31C** and **106C** as raw materials, compound **106** (21 mg) was obtained according to the operation of Example 105.

Preparation method: instrument: Waters 150 Prep-SFC E, column: Chiralcel OX column, mobile phase: (phase A: CO2, phase B: 0.1% $NH_3$•$H_2O$ in MeOH and ACN; gradient: 60% mobile phase B, isocratic elution; flow rate: 100 mL/min, back pressure: 100 bar, column temperature: 25°C, wavelength: 220 nm, cycle time: 11min, sample preparation: sample concentration 50 mg/ml, acetonitrile solution injection: 2.0ml each time. After separation, the fraction was dried in a water bath at 35°C by a rotary evaporator to obtain a product. The solvent was dried with a freeze-dryer at -80°C to obtain a final product of compound **106** (retention time: 1.960 minutes).

LC-MS (ESI): m/z = 571.3 $[M+H]^+$

$^1H$ NMR (400 MHz, DMSO-$d_6$) δ 8.39 (s, 2H), 6.94 (s, 1H), 6.66 (s, 1H), 6.66 - 6.36 (m, 2H), 4.42 (s, 2H), 4.30 (s, 2H), 3.98 (t, 2H), 3.77 - 3.62 (m, 4H), 3.47 - 3.39(m, 1H), 3.27 - 3.22 (m, 1H), 2.99 - 2.85 (m, 2H), 2.65 (s, 2H), 1.46 (d, 6H), 0.77 - 0.62 (m, 4H), 0.08 (s, 6H).

Example **107**

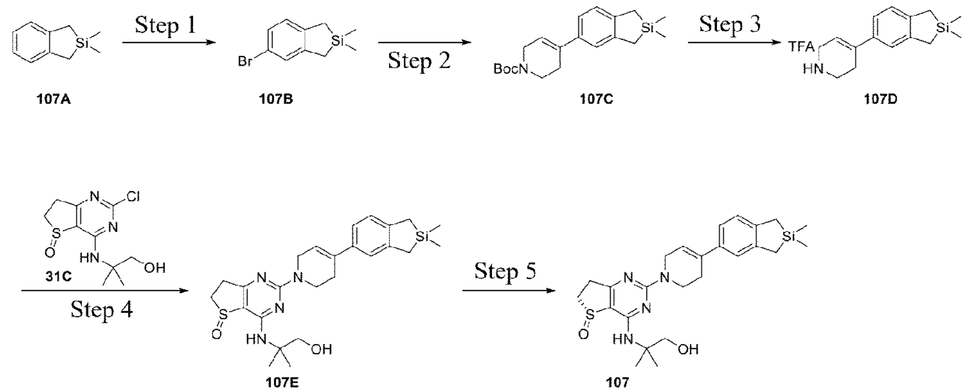


107A 107B 107C 107D 31C 107E 107

Step 1: Compound **107A** (1.6 g, 10 mmol) was dissolved in hexafluoroisopropanol (20 mL), N-bromosuccinimide (2.1 g, 12 mmol) was added and reacted at room temperature for 2 h, and the system was passed through a short silica gel column, eluted with petroleum ether, and concentrated to obtain compound **107B** (2.2 g, 90%).

$^1H$ NMR (400 MHz, DMSO-$d_6$) δ 7.37 (s, 1H), 7.20 (d, 1H), 7.13 (d, 1H), 2.02 (s, 2H), 1.95 (s, 2H), 0.19 (s, 6H).

Step 2: Compound **107B** (1.8 g, 7.6 mmol) was dissolved in 1,4-dioxane (40 mL), and 1,1-bis(diphenylphosphine)ferrocenepalladium dichloride (0.6 mg, 0.8 mmol), cesium carbonate (5.0 g, 15.4 mmol), N-Boc-1,2,5,6-tetrahydropyridine-4-boronic acid pinacol ester (2.6 g, 8.6 mmol), and water (10 mL) were then added. After nitrogen displacement three times, the mixture was heated to 90°C and reacted for 3 h, and the system was then passed through a short silica gel column, eluted with ethyl acetate, concentrated, and subjected to column chromatography (petroleum ether/ethyl acetate = 9/1) to obtain compound **107C** (2.3 g, 89%).

$^1H$ NMR (400 MHz, DMSO-$d_6$) δ 7.24 (s, 1H), 7.15 - 7.09 (m, 2H), 6.04 (s, 1H), 3.96 (s, 2H), 3.51 (t, 2H), 2.41 (s, 2H), 1.99 (s, 2H), 1.97 (s, 2H), 1.42 (s, 9H), 0.19 (s, 6H).

Step 3: Compound **107C** (2.1 g, 6.0 mmol) was dissolved in dichloromethane (20 mL), trifluoroacetic acid (15 mL) was then added, and the mixture was reacted at room temperature for half an hour and then concentrated to obtain compound **107D** (1.9 g, 90.4%), which was directly used for the next step.

Step 4: Compound **31C** (1.0 g, 3.6 mmol) was dissolved in 1,4-dioxane (30.0 mL), compound **107D** (1.5 g, 4.3 mmol) and N,N-diisopropylethylamine (1.4 g, 10.9 mmol) were then added, and the mixture was heated to 90°C and reacted for 12 h. The system was then cooled to room temperature, concentrated, and subjected to column chromatography (dichloromethane/methanol = 10/1) to obtain compound **107E** (1.3 g, 64.1%).

LC-MS (ESI): m/z = 483.2 $[M+H]^+$

Step 5: Compound **107E** (800 mg) was subjected to chiral resolution to obtain compound **107** (97 mg).

Preparation method: instrument: Waters 150 Prep-SFC E, column: Chiralcel OX column, mobile phase: (phase A: CO2, phase B: 0.1% $NH_3$•$H_2O$ in MeOH and ACN; gradient: 45% mobile phase B, isocratic elution; flow rate: 100 mL/min, back pressure: 100 bar, column temperature: 25°C, wavelength: 220 nm, cycle time: 4.8 min, sample preparation: sample concentration 10 mg/ml, acetonitrile solution injection: 2.0 ml each time. After separation, the fraction was dried in a water bath at 35°C by a rotary evaporator to obtain a product. The solvent was dried with a freeze-dryer at -80°C to obtain a final product of compound **107** (retention time: 0.987 minutes).

LC-MS (ESI): m/z = 483.2 $[M+H]^+$

$^1H$ NMR (400 MHz, DMSO-$d_6$) δ 7.28 (s, 1H), 7.15 (s, 2H), 6.21 (s, 1H), 6.19 (s, 1H), 5.15 (t, 1H), 4.35 (s, 2H), 3.99 (t, 2H), 3.50 (d, 2H), 3.46 - 3.35 (m, 1H), 3.35 - 3.27 (m, 4H), 2.99 - 2.77 (m, 2H), 2.01 (s, 2H), 1.98 (s, 2H), 1.43 (s, 6H), 0.20 (s, 6H).

Example **108**

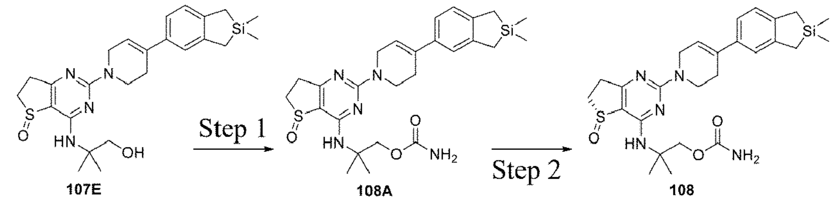

107E 108A 108

Using compound **107E** as raw materials, compound **108** (200 mg) was obtained according to the operation of Example **105.**

Preparation method: instrument: Waters 150 Prep-SFC C, column: Chiralcel AS Column, mobile phase: phase A: CO2, phase B: 0.1% $NH_3$•$H_2O$ in ETOH; gradient: 45% mobile phase B, isocratic elution; flow rate: 70 mL/min, back pressure: 100 bar, column temperature: 25°C, wavelength: 220 nm, cycle time: 7.0 min, sample preparation: sample concentration 10 mg/ml, acetonitrile solution injection: 4.0 ml each time. After separation, the fraction was dried in a water bath at 35°C by a rotary evaporator to obtain a product. The solvent was dried with a freeze-dryer at -80°C to obtain a final product of compound **108** (retention time: 1.779 minutes).

LC-MS (ESI): m/z = 526.7 $[M+H]^+$

$^1H$ NMR (400 MHz, DMSO-$d_6$) δ 7.27 (s, 1H), 7.15 (s, 2H), 6.66 (s, 3H), 6.53 (s, 2H), 6.18 (s, 1H), 4.35 (s, 2H), 4.29 (s, 2H), 3.98 (t, 2H), 3.49 - 3.35 (m, 1H), 3.31 (s, 2H), 3.28 - 3.13 (m, 1H), 3.06 - 2.80 (m, 2H), 2.00 (s, 2H), 1.98 (s, 2H), 1.46 (d, 3H), 1.45 (d, 3H), 0.20 (s, 6H).

**Example 109**

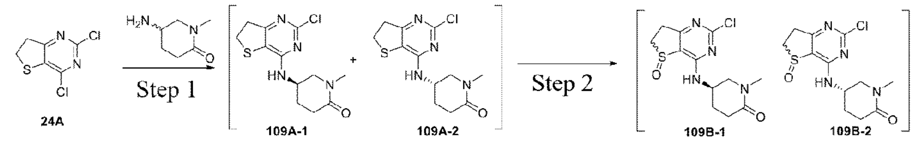

24A 109A-1 109A-2 109B-1 109B-2

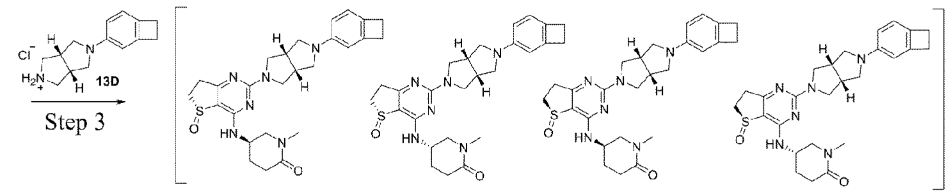

109-1, -2, -3, -4

Using compound **24A** and 1-methyl-5-amino-2-piperidone as raw materials, compound **109-1** (67 mg, retention time 0.98 min, 11.9%) and compound **109-2** (110 mg, retention time 1.45 min, 19.5%) were obtained according to the operation of Example **41.**

Separation conditions of preparative chromatography: 1. instrument: Waters 150 Prep-SFC F; 2. chromatographic column: Chiralcel OX Column; 3. mobile phase system: A for $CO_2$; B for 0.1% $NH_3$•$H_2O$ in MEOH and ACN; 4. gradient: B 70%; 5. flow rate: 100 mL/min.

**compound 109-1:**

$^1H$ NMR (400 MHz, DMSO-$d_6$) δ 7.73 (d, 1H), 6.85 (d, 1H), 6.35 (d, 2H), 4.49 (s, 1H), 3.80 (s, 2H), 3.55 - 3.35 (m, 6H), 3.19 (d, 4H), 3.07 (s, 2H), 3.04 - 2.83 (m, 6H), 2.79 (d, 3H), 2.30 (t, 2H), 2.02 - 1.82 (m, 2H).

LC-MS (ESI): m/z = 493.3 $[M+H]^+$

**Compound 109-2:**

$^1H$ NMR (400 MHz, DMSO-$d_6$) δ 7.74 (d, 1H), 6.85 (d, 1H), 6.40 - 6.28 (m, 2H), 4.49 (s, 1H), 3.80 (s, 2H), 3.55 - 3.36 (m, 6H), 3.16 (d, 4H), 3.10 - 2.96 (m, 6H), 2.97 - 2.82 (m, 2H), 2.78 (d, 3H), 2.31 (t, 2H), 1.96 (td, 2H).

LC-MS (ESI): m/z = 493.3 $[M+H]^+$

Using compound **109B-2** (370 mg, 1.18 mmol) as a raw material, compound **109-3** (95 mg, retention time 1.32 min, 16.4%) and compound **109-4** (110 mg, retention time 1.67 min, 21.8%) were obtained according to the above synthesis route.

**Compound 109-3:**

$^1H$ NMR (400 MHz, DMSO-$d_6$) δ 7.90 - 7.57 (m, 1H), 6.84 (d, 1H), 6.34 (d, 2H), 4.60 - 4.39 (m, 1H), 3.95 - 3.69 (m, 2H), 3.59 - 2.94 (m, 16H, overlapped), 2.88 (d, 2H), 2.78 (s, 3H), 2.39 - 2.17 (m, 2H), 1.93 (d, 2H).
LC-MS (ESI): m/z = 493.3 $[M+H]^+$

**Compound 109-4:**

$^1H$ NMR (400 MHz, DMSO-$d_6$) δ 7.73 (d, 1H), 6.85 (d, 1H), 6.35 (d, 2H), 4.49 (s, 1H), 3.80 (s, 2H), 3.56 - 3.35 (m, 6H), 3.19 (d, 4H), 3.07 (s, 2H), 3.01 (d, 4H), 2.97 - 2.84 (m, 2H), 2.79 (s, 3H), 2.30 (t, 2H), 2.03 - 1.82 (m, 2H).
LC-MS (ESI): m/z = 493.3 $[M+H]^+$

**Example 110**

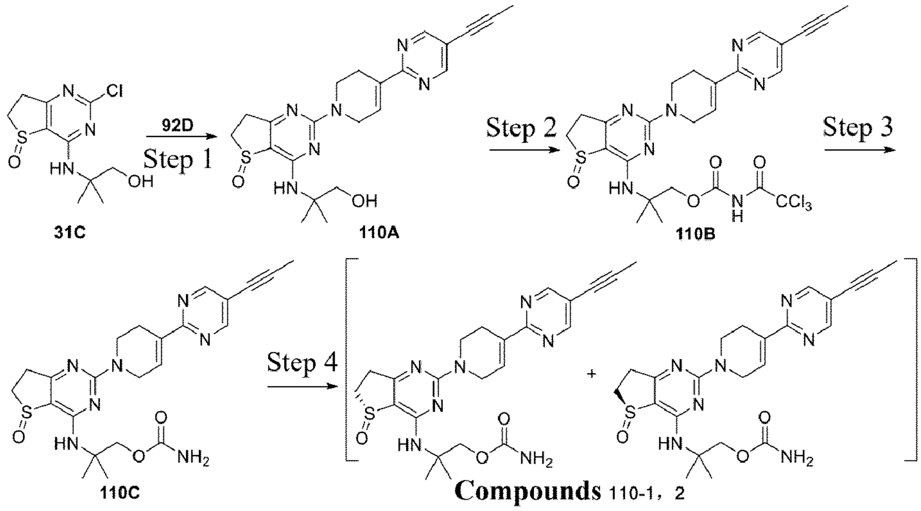


31C 110A 110B

110C

Compounds 110-1，2

Using compounds **31C** and **92D** as raw materials, compound **110C** (400 mg, 0.83 mmol) was obtained according to the operation of Example **84,** and after chiral resolution, compound **110-1** (221.9 mg) and compound **110-2** (13.6 mg) were obtained.
Analysis method: instrument: SHIMADZU LC-30AD, column: Chiralcel IK Column; mobile phase: A: $CO_2$, B: 0.05% DEA in IPA and ACN; gradient: 40% B in A; flow rate: 3 mL/min, column temperature: 35°C, wavelength: 220 nm.
Preparation method: instrument: Waters 150 Prep-SFC E, column: Chiralcel IK Column (250 mm × 30 mm, 10 μm) mobile phase: A: $CO_2$, B: 0.1% $NH_3$•$H_2O$ in IPA and ACN; gradient: 50% B gradient elution, flow rate: 100 mL/min, column temperature: 25°C, wavelength: 220 nm, cycle time: 5.0 min, sample preparation: sample concentration 10 mg/ml, methanol solution injection: 2.5 ml each time. After separation, the fraction was dried by a rotary evaporator at a bath temperature of 35°C to obtain compound **110-1** and compound **110-2.**

**Compound 110-1:**

$^1H$ NMR (400 MHz, $CDCl_3$) δ 8.66 (s, 2H), 7.31 - 7.29 (m, 1H), 5.47 (s, 1H), 4.90 (br s, 2H), 4.54 (s, 2H), 4.40 - 4.32 (m, 2H), 4.12 - 4.05 (m, 2H), 3.65 - 3.57 (m, 1H), 3.42 - 3.36 (m, 1H), 3.05 - 2.99 (m, 2H), 2.81- 2.77 (m, 2H), 2.11 (s, 3H), 1.54 - 1.52 (m, 6H).
LC-MS (ESI): m/z = 482.2 $[M+H]^+$

**Compound 110-2:**

$^1H$ NMR (400 MHz, $CDCl_3$) δ 8.66 (s, 2H), 7.31 - 7.29 (m, 1H), 5.47 (s, 1H), 4.90 (brs, 2H), 4.54 (s, 2H), 4.40 - 4.32

(m, 2H), 4.12 - 4.05 (m, 2H), 3.65 - 3.57 (m, 1H), 3.42 - 3.36 (m, 1H), 3.05 - 2.99 (m, 2H), 2.82 - 2.76 (m, 2H), 2.11 (s, 3H), 1.54 - 1.52 (m, 6H).

LC-MS (ESI): m/z = 482.2 $[M+H]^+$

**Example 111**

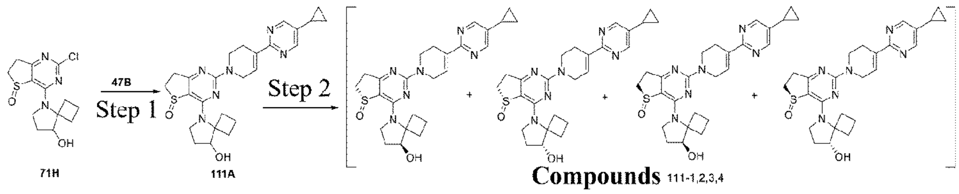


71H

111A

Using compounds **47B** and **71H** as raw materials, compound **111-1** (180.8 mg), compound **111-2** (92.7 mg), compound **111-3** (11.1 mg), and compound **111-4** (61.6 mg) were obtained according to the operation of Example **71** (steps 8 and 9).

HPLC analysis method: instrument: SHIMADZU LC-2020AD, column: C18 Column; mobile phase: A: 0.1% TFA in $H_2O$, B: ACN; gradient: 10-80% B in A; flow rate: 1.2 mL/min, column temperature: 45°C, wavelength: 210 nm & 254 nm.

HPLC preparation method: instrument: SHIMADZU LC-20AP, column: C18 Column; mobile phase: A: 0.225% FA in $H_2O$, B: ACN; gradient: 55-85% B gradient elution; flow rate: 75 mL/min, column temperature: 25°C; wavelength: 220 nm; cycle time: 5.0 min, sample preparation: sample concentration 50 mg/ml, methanol solution injection: 5.0 ml each time. After separation, the fraction was dried by a rotary evaporator at a bath temperature of 35°C to obtain compound **P1** and compound **P2.**

SFC analysis method: instrument: SHIMADZU LC-30AD, column: Chiralcel IC Column; mobile phase: A:$CO_2$, B: 0.05% DEA in EtOH and ACN; gradient: 60% B in A; flow rate: 3 mL/min, column temperature: 35°C, wavelength: 220 nm.

SFC preparation method: instrument: Waters 150 Prep-SFC F, column: Chiralcel Cellulose-2 Column; mobile phase: A: $CO_2$, B: 0.1% $NH_3$•H2O in IPA and ACN; gradient: 60% B gradient elution; flow rate: 100 mL/min, column temperature: 25°C; wavelength: 220 nm; cycle time: 8.6 min; sample preparation: sample concentration 10 mg/ml, acetonitrile solution injection: 1.5 ml each time. After separation, the fraction was dried by a rotary evaporator at a bath temperature of 35°C to obtain compound **111-1** (180.8 mg), compound **111-2** (92.7 mg), compound **111-3** (11.1 mg), and compound **111-4** (61.6 mg).

Compound **111-1,** retention time: 1.059 min; $^1H$ NMR (400 MHz, $CDCl_3$) δ 8.43 (s, 2H), 7.24 - 7.22 (m, 1H), 4.67 - 4.53 (m, 2H), 4.27 (s, 1H), 4.22 - 4.06 (m, 4H), 3.83 - 3.71 (m, 2H), 3.51 - 3.43 (m, 1H), 3.17 - 3.12 (m, 1H), 3.07 - 3.01 (m, 2H), 2.82 (s, 2H), 2.25 - 2.18 (m, 2H), 2.06 - 1.98 (m, 2H), 1.89 - 1.76 (m, 2H), 1.66 - 1.61 (m, 2H), 1.11 - 1.06 (m, 2H), 0.79 - 0.75 (m, 2H).

LC-MS (ESI): m/z =479.3 $[M+H]^+$

Compound **111-2,** retention time: 1.245 min; $^1H$ NMR (400 MHz, $CDCl_3$) δ 8.43 (s, 2H), 7.24 - 7.22 (m, 1H), 4.67 - 4.53 (m, 2H), 4.27 (s, 1H), 4.22 - 4.06 (m, 4H), 3.83 - 3.71 (m, 2H), 3.51 - 3.43 (m, 1H), 3.17 - 3.12 (m, 1H), 3.07 - 3.01 (m, 2H), 2.82 (s, 2H), 2.46 - 2.18 (m, 2H), 2.08 - 1.98 (m, 2H), 1.89 - 1.76 (m, 2H), 1.66 - 1.61 (m, 2H), 1.11 - 1.06 (m, 2H), 0.79 - 0.75 (m, 2H).

LC-MS (ESI): m/z =479.3 $[M+H]^+$

Compound **111-3,** retention time: 1.493 min; $^1H$ NMR (400 MHz, $CDCl_3$) δ 8.43 (s, 2H), 7.24 - 7.22 (m, 1H), 4.61 (s, 2H), 4.23 - 4.22 (s, 1H), 4.19 - 4.12 (m, 3H), 3.78 - 3.63 (m, 3H), 3.49 - 3.41 (m, 1H), 3.16 - 3.10 (m, 3H), 2.83 (s, 2H), 2.26 - 2.21 (m, 1H), 2.09 - 2.06 (m, 2H), 2.00 - 1.92 (m, 1H), 1.89 - 1.83 (m, 2H), 1.72 - 1.67 (m, 2H), 1.11 - 1.06 (m, 2H), 0.80 - 0.75 (m, 2H).

LC-MS (ESI): m/z =479.3 $[M+H]^+$

Compound **111-4,** retention time: 2.368 min; $^1H$ NMR (400 MHz, $CDCl_3$) δ 8.43 (s, 2H), 7.24 - 7.22 (m, 1H), 4.62 - 4.60 (s, 2H), 4.23 - 4.22 (s, 1H), 4.19 - 4.12 (m, 3H), 3.78 - 3.63 (m, 3H), 3.49 - 3.41 (m, 1H), 3.16 - 3.10 (m, 3H), 2.83 (s, 2H), 2.26 - 2.21 (m, 1H), 2.09 - 2.06 (m, 2H), 2.00 - 1.92 (m, 1H), 1.89 - 1.83 (m, 2H), 1.72 - 1.67 (m, 2H), 1.11 - 1.06 (m, 2H), 0.80 - 0.75 (m, 2H).

LC-MS (ESI): m/z =479.3 $[M+H]^+$

Example **112**

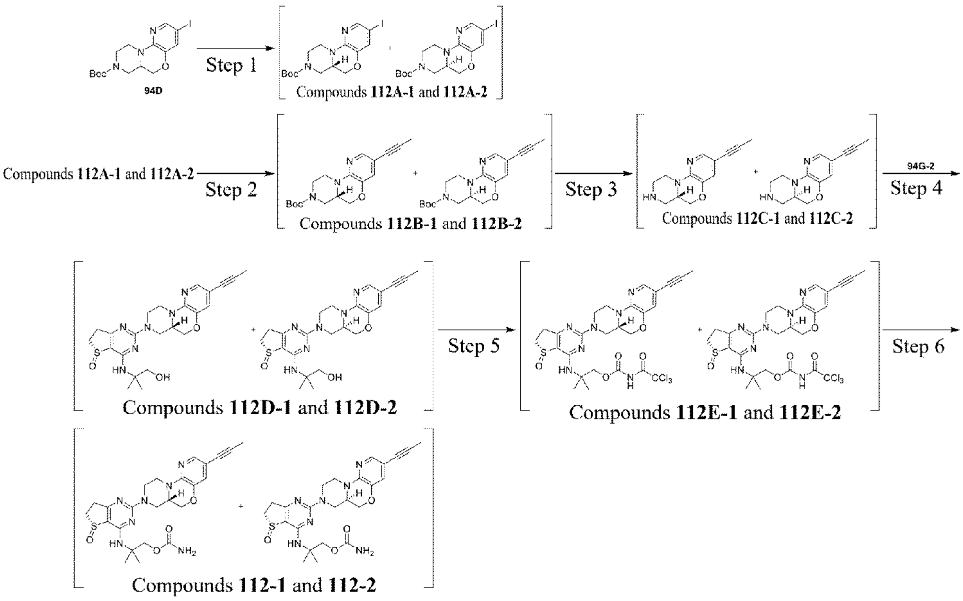


Step 1: Compound **94D** (1.5 g) was separated by SFC to obtain two isomers, i.e., compound **112A-1** (700 mg, retention time 5.86 min, 47%) and compound **112A-2** (700 mg, retention time 7.27 min, 47%).

Separation conditions of preparative chromatography: 1. instrument: Waters 150 AP-SFC; 2. chromatographic column: AD; 3. mobile phase system: A for CO2; B for MEOH; 4. gradient: B 28%; and 5. flow rate: 40 mL/min

LC-MS (ESI): m/z = 418.0 $[M+H]^+$

Step 2: At room temperature in a nitrogen atmosphere, compound **112A-1** (700 mg, 1.68 mmol), propyne (1 M/L, 33.6 mL, 33.6 mmol), bis(triphenylphosphine)palladium dichloride (119.3 mg, 0.17 mmol), cuprous iodide (64.6 mg, 0.34 mmol), and tetrahydrofuran (10 mL) were added to a 50 mL round-bottom flask, followed by triethylamine (509.1 mg, 5.04 mmol), and the mixture was reacted at this temperature for 12 h. After the reaction was complete, water (5 mL) was added, and the mixture was extracted twice with ethyl acetate (10 mL). The organic phases were combined, washed with a saturated sodium chloride aqueous solution, and dried over anhydrous sodium sulfate. After filtration, the reaction liquid was concentrated under reduced pressure and then separated and purified by silica gel column chromatography (eluents: EA/PE (v/v) = 0-30%) to obtain the target compound **112B-1** (500 mg, 90%).

LC-MS (ESI): m/z = 330.2 $[M+H]^+$

According to the above operation, using compound **112A-2** (700 mg, 1.68 mmol) as a raw material, the target compound **112B-2** (500 mg, 90%) was obtained.

LC-MS (ESI): m/z = 330.2 $[M+H]^+$

Step 3: At room temperature, **112B-1** (500 mg, 1.52 mmol), dichloromethane (4 mL), and trifluoroacetic acid (1 mL) were added to a 25 mL round-bottom flask, and the mixture was continuously stirred at this temperature for 2 h. After the reaction was complete, the reaction liquid was concentrated under reduced pressure to obtain the target compound **112C-1** (325 mg, 93%).

LC-MS (ESI): m/z = 230.2 $[M+H]^+$

According to the above operation, using compound **112B-2** (500 mg, 1.52 mmol) as a raw material, the target compound **112C-2** (330 mg, 95%) was obtained.

LC-MS (ESI): m/z = 230.2 $[M+H]^+$

Step 4: At room temperature, compound **112C-1** (325 mg, 1.42 mmol), compound **94G-2** (429.9 mg, 1.56 mmol), and 1,4-dioxane (5 mL) were added to a 25 mL round-bottom flask, and N,N-diisopropylethylamine (545.3 mg, 4.26 mmol) were added at room temperature, heated to 100°C and reacted for 12 h. After the reaction was complete, the reaction liquid was concentrated under reduced pressure and then separated and purified by silica gel column chromatography (eluents: MeOH/DCM (v/v) = 0-10%) to obtain the target compound **112D-1** (600 mg, 90%).

LC-MS (ESI): m/z = 469.2 $[M+H]^+$

According to the above operation, using compound **112C-2** (330 mg, 1.44 mmol) as a raw material, the target compound **112D-2** (620 mg, 92%) was obtained.

LC-MS (ESI): m/z = 469.2 $[M+H]^+$

Step 5: At room temperature, compound **112D-1** (300 mg, 0.64 mmol) and dichloromethane (5 mL) were added to a 25 mL round-bottom flask, and trichloroacetyl isocyanate (144.7 mg, 0.77 mmol) was added in an ice bath and stirred in the ice bath for 1 h. After the reaction was complete, the reaction liquid was concentrated under reduced pressure and then separated and purified by silica gel column chromatography (eluents: MeOH/DCM (v/v) = 0-10%) to obtain the target

compound **112E-1** (300 mg, 71%).

LC-MS (ESI): m/z = 656.2 [M+H]+

According to the above operation, using compound **112D-2** (300 mg, 0.64 mmol) as a raw material, the target compound **112E-2** (300 mg, 71%) was obtained.

LC-MS (ESI): m/z = 656.2 [M+H]+

Step 6: At room temperature, compound **112E-1** (150 mg, 0.23 mmol) and methanol (2 mL) were added to a 25 mL round-bottom flask, and potassium carbonate (95.2 mg, 0.69mmol) and water (2 mL) were added in an ice bath and reacted at room temperature for 2.5 h. After the reaction was complete, the reaction liquid was concentrated under reduced pressure and then separated and purified by silica gel column chromatography (eluents: MeOH/EA (v/v) = 0-10%) to obtain the target compound **112-1** (100 mg, 86%)

According to the above operation, using compound **112E-2** (150 mg, 0.23 mmol) as a raw material, the target compound **112-2** (100 mg, 86%) was obtained.

Compound **112-1:**

$^1$H NMR (400 MHz, Chloroform-d) δ 7.85 (d, 1H), 6.95 (d, 1H), 5.53 (brs, 1H), 5.00 - 4.74 (m, 4H), 4.62 - 4.56 (m, 1H), 4.39 - 4.30 (m, 3H), 4.03 - 3.98 (m, 1H), 3.62 - 3.53 (m, 1H), 3.44 - 3.35 (m, 2H), 3.17 - 3.08 (m, 1H), 3.05 - 2.94 (m, 2H), 2.89 - 2.75 (m, 2H), 2.03 (s, 3H), 1.51 (s, 3H), 1.48 (s, 3H).

LC-MS (ESI): m/z = 512.2 [M+H]+

Compound **112-2:**

$^1$H NMR (400 MHz, DMSO-$d_6$) δ 7.78 (d, 1H), 6.98 (d, 1H), 6.74 (brs, 1H), 6.68 - 6.44 (m, 2H), 4.82 - 4.71 (m, 2H), 4.51 -4.39 (m, 2H), 4.36 - 4.25 (m, 1H), 4.04 - 3.94 (m, 1H), 3.49 - 3.37 (m, 2H), 3.29 - 3.20 (m, 2H), 3.12 - 3.02 (m, 1H), 3.01 - 2.92 (m, 1H), 2.92 - 2.84 (m, 1H), 2.84 - 2.73 (m, 2H), 2.00 (s, 3H), 1.44 (s, 3H), 1.42 (s, 3H).

LC-MS (ESI): m/z = 512.2 [M+H]+

**Example 113**

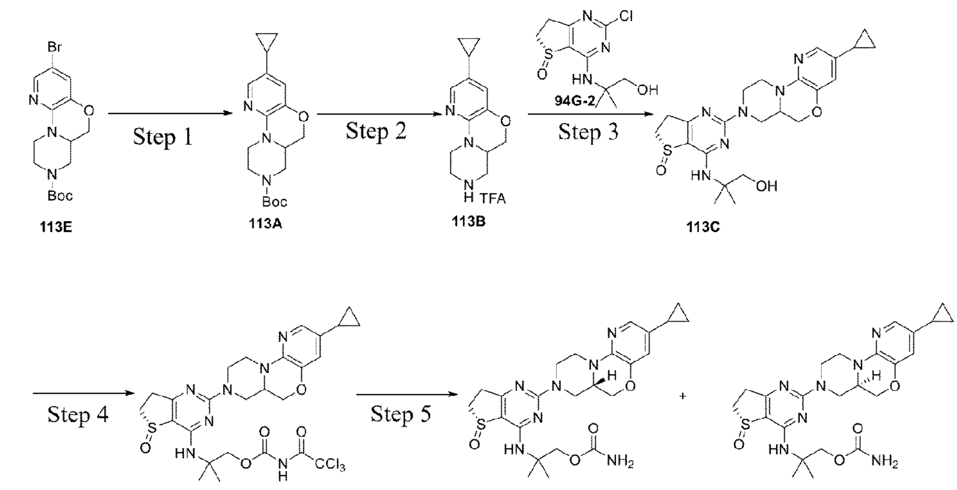


113E 113A 113B 113C 113D

Compounds 113-1, 113-2

Step 1: To a 100 mL single-mouth flask, **113E** (1.2 g, 3.25 mmol, see WO 2022184103 for the synthesis), dioxane (15 mL), water (3 mL), 1,1-bis(diphenylphosphine)ferrocenepalladium dichloride (240 mg, 0.33 mmol), potassium carbonate (1.35 g, 9.75 mmol), and cyclopropylboronic acid (280 mg, 3.25 mmol) were successively added and reacted at 80°C overnight under nitrogen protection. After concentration under reduced pressure, the system was then separated and purified by silica gel column chromatography (eluents: ethyl acetate/petroleum ether (v/v) = 20/100) to obtain **113A** (800 mg, yield 74.4%).

LC-MS (ESI): m/z = 332.2 [M+H]+.

Using **113A** as a raw material, 150 mg of a mixture of compounds **113-1** and **113-2** was synthesized according to the operation of Example 112.

Chiral preparation was carried out to obtain the title compound **113-1** (53 mg, two-step yield 16.2%, retention time: 0.883 min) and the title compound **113-2** (65 mg, 19.8%, retention time: 1.147 min). Chiral preparation method: instrument: Waters 150 MGM; chromatographic column: Chiralpak Column; mobile phase: A: carbon dioxide, and B: ethanol (0.1% aqueous ammonia); isocratic elution: 70% mobile phase B; flow rate: 100 mL/min; back pressure: 100 bar; column temperature: 25°C; wavelength: 220 nm; elution time: 4.0 min.

**(Compound 113-1)**

$^1$H NMR (400 MHz, DMSO-$d_6$) δ 7.63 - 7.58 (m, 1H), 6.71 (s, 1H), 6.67 - 6.66 (m, 1H), 6.56 (s, 2H), 4.85 - 4.66 (m, 2H), 4.46 - 4.21 (m, 4H), 4.04 - 3.90 (m, 1H), 3.52 - 3.35 (m, 1H), 3.29 - 3.22 (m, 2H), 3.09 - 2.63 (m, 5H), 1.85 - 1.72 (m, 1H), 1.47 - 1.39 (m, 6H), 0.89 - 0.81 (m, 2H), 0.60 - 0.55 (m, 2H).
LC-MS (ESI): m/z = 514.2 $[M+H]^+$.

**(Compound 113-2)**

$^1$H NMR (400 MHz, DMSO-$d_6$) δ 7.62 - 7.59 (m, 1H), 6.70 (s, 1H), 6.67 - 6.64 (m, 1H), 6.56 (s, 2H), 4.89 - 4.55 (m, 2H), 4.48 - 4.19 (m, 4H), 4.03 - 3.83 (m, 1H), 3.53 - 3.38 (m, 1H), 3.29 - 3.22 (m, 2H), 3.06 - 2.65 (m, 5H), 1.85 - 1.72 (m, 1H), 1.46 - 1.39 (m, 6H), 0.87 - 0.82 (m, 2H), 0.60 - 0.55 (m, 2H).
LC-MS (ESI): m/z = 514.2 $[M+H]^+$.

**Example 114**

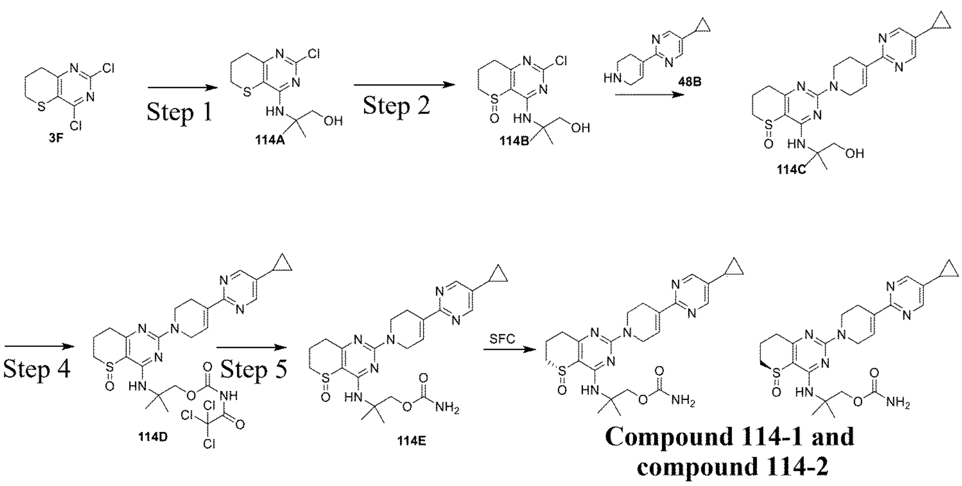

3F
114A
114B
48B
114C
114D
114E

**Compound 114-1 and compound 114-2**

Using **3F** and 2-amino-2-methylpropan-1-ol as raw materials, compound **114-1** (50 mg) and compound **114-2** (45 mg) were synthesized according to the operation of Example 88.
Chiral resolution method: instrument: Waters 150 SFC; column: Chiralpak IC Column (250 × 30 mm, I.D 30 mm, 10 um particle size); mobile phase: A for CO2 and B for IPA + ACN (0.1% $NH_3•H_2O$); gradient: 45% phase B isocratic elution; flow rate: 100 mL/min; back pressure: 100 bar; column temperature: 25°C; wavelength: 220 nm.
LCMS m/z = 498.2 $[M +1]^+$
**Compound 114-1,** $^1$H NMR (400 MHZ, DMSO-$d_6$) δ 8.54 (s, 2H), 7.18 (s, 1H), 6.52 (s, 2H), 6.18 (s, 1H), 4.42 (s, 2H), 4.27 (s, 2H), 3.95 (t, 2H), 2.95-3.10 (m, 2H), 2.56-2.73 (m, 4H), 2.25-2.35 (m, 1H), 1.91-1.98 (m, 2H), 1.46 (s, 6H), 1.00-1.06 (m, 2H), 0.82-0.86 (m, 2H).
**Compound 114-2,** $^1$H NMR (400 MHZ, DMSO-$d_6$) δ 8.54 (s, 2H), 7.18 (s, 1H), 6.52 (s, 2H), 6.18 (s, 1H), 4.42 (s, 2H), 4.27 (s, 2H), 3.95 (t, 2H), 2.95-3.10 (m, 2H), 2.56-2.73 (m, 4H), 2.25-2.35 (m, 1H), 1.91-1.98 (m, 2H), 1.46 (s, 6H), 1.00-1.06 (m, 2H), 0.82-0.86 (m, 2H).

**Example 115**

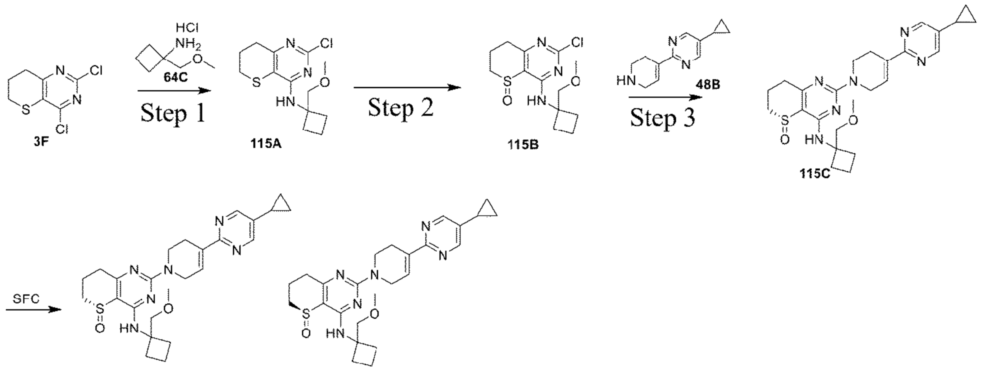


**Compound 115-1 and compound 115-2**

Using **3F** and **64C** as raw materials, compound **115-1** (110 mg) and compound **115-2** (100 mg) were synthesized according to the operation of Example 64.

Chiral resolution method: instrument: Waters 150 SFC; column: Chiralpak IC Column (250 × 30 mm, I.D 30 mm, 10 um particle size); mobile phase: A for CO2 and B for IPA + ACN (0.1% $NH_3$•$H_2O$); gradient: 60% phase B isocratic elution; flow rate: 100 mL/min; back pressure: 100 bar; column temperature: 25°C; wavelength: 220 nm.

LCMS m/z = 481.2 [M +1]$^+$

**Compound 115-1,** $^1$H NMR (400 MHZ, DMSO-$d_6$) δ 8.53 (s, 2H), 7.18 (s, 1H), 6.60 (s, 1H), 4.39 (s, 2H), 3.93 (t, 2H), 3.69-3.75 (m, 2H), 3.30 (s, 3H), 3.04-3.08 (m, 1H), 2.90-2.96 (m, 1H), 2.56-2.72 (m, 4H), 2.30-2.42 (m, 3H), 2.14-2.25 (m, 2H), 1.81-1.98 (m, 4H), 1.00-1.05 (m, 2H), 0.82-0.85 (m, 2H).

**Compound 115-2,** $^1$H NMR (400 MHZ, DMSO-$d_6$) δ 8.53 (s, 2H), 7.18 (s, 1H), 6.60 (s, 1H), 4.39 (s, 2H), 3.93 (t, 2H), 3.69-3.75 (m, 2H), 3.30 (s, 3H), 3.04-3.08 (m, 1H), 2.90-2.96 (m, 1H), 2.56-2.72 (m, 4H), 2.30-2.42 (m, 3H), 2.14-2.25 (m, 2H), 1.81-1.98 (m, 4H), 1.00-1.05 (m, 2H), 0.82-0.85 (m, 2H).

## Example 116

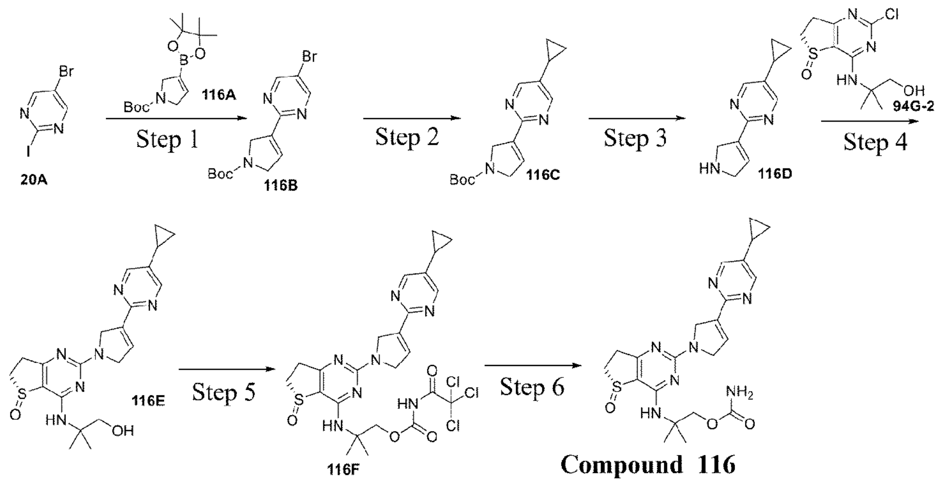


**Compound 116**

Step 1: Compound **20A** (5 g, 17.55 mmol) and compound **116A** (5.18 g, 17.55 mmol) were dissolved in tetrahydrofuran (40 mL), and 1,1-bis(diphenylphosphine)ferrocenepalladium dichloride (1.28 g, 1.76 mmol), potassium carbonate (7.28 g, 52.57 mmol), and water (10 mL) were then added. After nitrogen displacement three times, the system was heated to 80°C and reacted for 14 h. After the reaction was complete as detected by a TLC spotting plate (petroleum ether:ethyl acetate = 5:1), water (50 mL) was added and stirred for 5 min, and the mixture was extracted with ethyl acetate (30 mL). The organic phase was separated, dried over anhydrous sodium sulfate, and concentrated under reduced pressure, and the crude product was purified by column chromatography (eluents: petroleum ether : ethyl acetate = 5:1) to obtain the target compound **116B** (3.5 g, 61.14%).

LC-MS (ESI): m/z = 326.1 [M+1]$^+$.

Using **116B** as a raw material, compound **116** (200 mg, 66%) was synthesized according to the operation of

Example 47.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min; retention time: 12.00 min.

LCMS m/z = 470.2 $[M+1]^+$

$^1H$ NMR (400 MHZ, DMSO-$d_6$) δ 8.62 (s, 1H), 8.58 (s, 1H), 6.94 (s, 1H), 6.47-6.69 (m, 3H), 4.68 (d, 2H), 4.53 (d, 2H), 4.30 (d, 2H), 3.41-3.50 (m, 1H), 3.23-3.28 (m, 1H), 2.86-3.02 (m, 2H), 1.92-2.00 (m, 1H), 1.46-1.51 (m, 6H), 1.01-1.08 (m, 2H), 0.82-0.89 (m, 2H).

**Example 117**

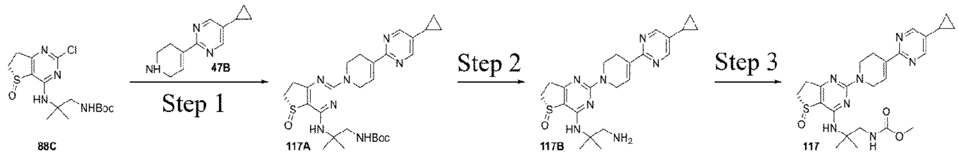

88C 117A 117B 117

Using **47B** and **88C** as raw materials, compound **117** (1.0 g, 50%) was synthesized according to the operation of Example 88.

Preparation method: instrument: Waters 150 Prep-SFC C, column: Chiralcel AS Column, mobile phase: (A for $CO_2$; B for MEOH); gradient: 45% mobile phase B, isocratic elution; flow rate: 70 mL/min, back pressure: 100 bar, column temperature: 25°C, wavelength: 220 nm, cycle time: 6.0 min, sample preparation: sample concentration 10 mg/ml, acetonitrile solution injection: 3 ml each time. After separation, the fraction was dried on a rotary evaporator at a bath temperature of 35°C to obtain **117** (retention time: 1.977 minutes).

LC-MS (ESI): m/z = 498.1 $[M+1]^+$

$^1H$ NMR (400 MHz, DMSO-*d6*) δ 8.54 (s, 2H), 7.45 (t, 1H), 7.19 (s, 1H), 6.69 (s, 1H), 4.47 (s, 2H), 4.00 (t, 2H), 3.57 (s, 3H), 3.48 - 3.37 (m, 2H), 3.28 - 3.19 (m, 2H), 2.99 - 2.83 (m, 2H), 2.67 (s, 2H), 1.93 (m, 1H), 1.43 (d, 6H), 1.12 - 0.99 (m, 2H), 0.91 - 0.76 (m, 2H).

**Example 118**

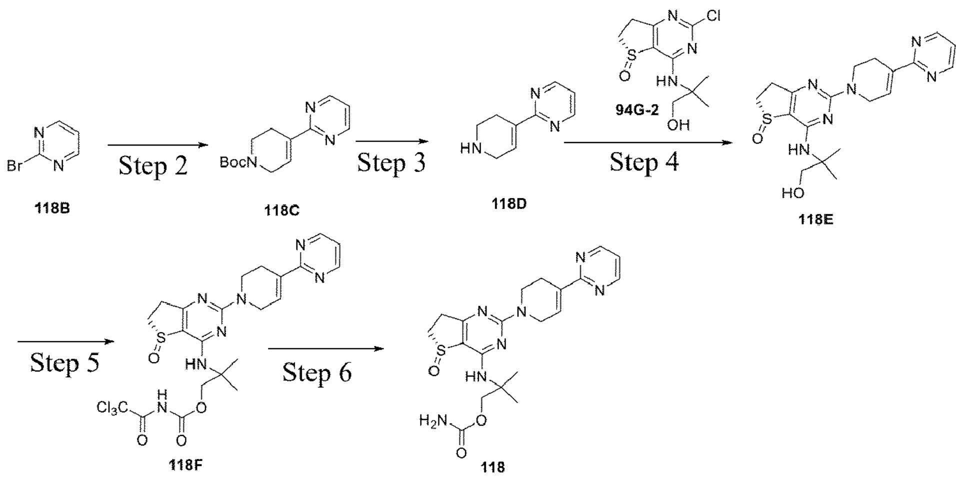

118B 118C 118D 118E 118F 118

Using **118B** and N-Boc-1,2,5,6-tetrahydropyridine-4-boronic acid pinacol ester as raw materials, compound **118** (800 mg, 61%) was synthesized according to the operation of Example 47.

LC-MS (ESI): m/z = 444.2 $[M+H]^+$

$^1H$ NMR (400 MHz, DMSO-$d_6$) δ 8.79 (d, 2H), 7.35 (t, 1H), 7.31 - 7.25 (m, 1H), 6.75 - 6.40 (m, 3H), 4.49 (s, 2H), 4.30 (s, 2H), 4.01 (t, 2H), 3.49 - 3.38 (m, 1H), 3.29 - 3.21 (m, 1H), 3.02 - 2.92 (m, 1H), 2.91 - 2.83 (m, 1H), 2.70 (s, 2H), 1.47

(d, 6H).

**Example 119**

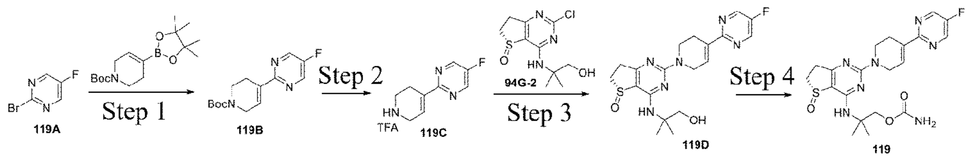


Using **119A** and N-Boc-1,2,5,6-tetrahydropyridine-4-boronic acid pinacol ester as raw materials, compound **119** (470 mg, 51%) was synthesized according to the operation of Example 47.

LC-MS (ESI): m/z = 462.2 $[M+H]^+$

$^1H$ NMR (400 MHz, DMSO-$d_6$) δ 8.86 (s, 2H), 7.22 (s, 1H), 6.64 (s, 1H), 6.56 (s, 2H), 4.48 (s, 2H), 4.30 (s, 2H), 4.01 (t, 2H), 3.55 - 3.37 (m, 1H), 3.28 - 3.22 (m, 1H), 3.03 - 2.92 (m, 1H), 2.92 - 2.86 (m, 1H), 2.69 (s, 2H), 1.46 (d, 6H).

$^{19}F$ NMR (376 MHz, DMSO-$d_6$) δ -138.83 (s, 1F).

Example **120**

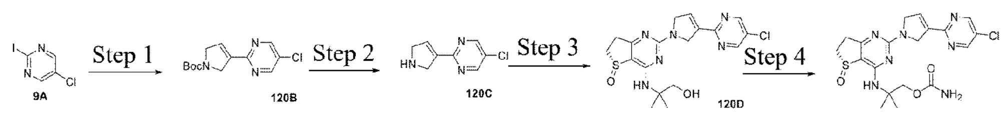


**Compound 120**

Using compound **9A** as a raw material, the target compound **120** (10 mg, 4%) was obtained according to the synthesis step of Example **116.**

LCMS (ESI): m/z = 464.2 $[M+H]^+$

$^1H$ NMR (400 MHz, DMSO-$d_6$) δ 8.96 (d, 2H), 7.09-7.07 (m, 1H), 6.73-6.41 (s, 2H), 4.75-4.65 (m, 2H), 4.61-4.52 (m, 2H), 4.30 (d, 2H), 3.59 - 3.41 (m, 1H), 3.05-2.84(m, 2H), 2.56-2.52(m, 2H)1.54-1.44 (m, 6H).

**Example 121**

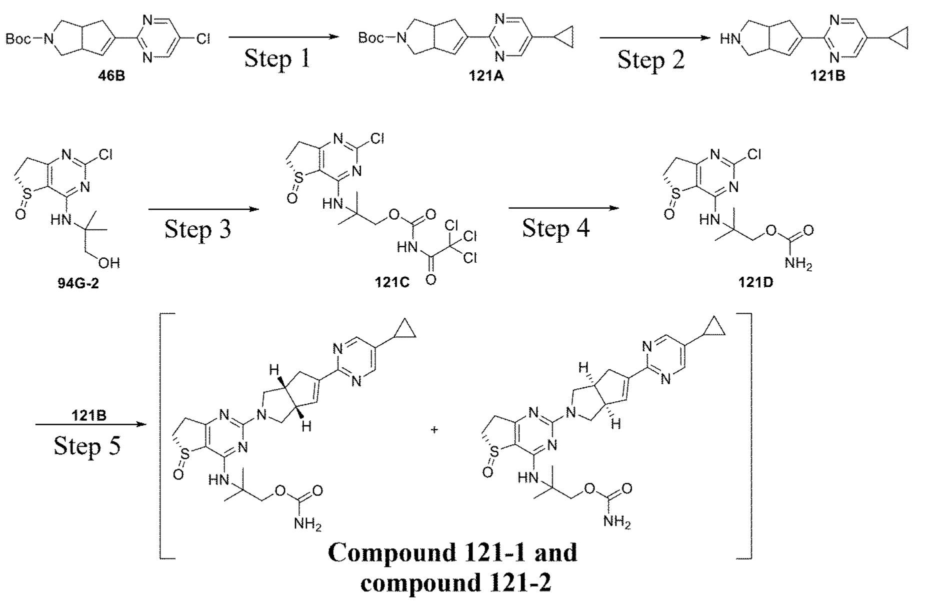


**Compound 121-1 and compound 121-2**

Using **46B** as a raw material, compound **121** (300 mg, 96%) was obtained according to the operation of Example 48.

LC-MS (ESI): m/z = 510.2 $[M+H]^+$.

Resolution of compound **121:**

Compound **121** (300 mg) was taken for resolution, and after separation, compound **121-1** (retention time: 0.568 min, 87.2 mg, ee% = 100%) and compound **121-2** (retention time: 0.763 min, 84.1 mg, ee% = 100%) were obtained.

Resolution conditions:

instrument: Waters 150 Prep-SFC F; column: Chiralcel AD column;
mobile phase: A for $CO_2$; B for 0.1% $NH_3$•$H_2O$ in IPA and ACN; gradient: B 60%; flow: 100 mL/min;
back pressure: 100 bar;
column temperature: 25°C; wavelength: 220 nm; period: 3.2 min;

**Compound 121-1:**

$^1$H NMR (400 MHz, DMSO-*d6*) δ = 8.52 (s, 2H), 6.73 (s, 1H), 6.53 (s, 2H), 6.47 (s, 1H), 4.29 (s, 2H), 3.94-3.89 (m, 1H), 3.81 - 3.59 (m, 3H), 3.45 - 3.33 (m, 1H), 3.29 - 3.18 (m, 2H), 3.17 - 3.05 (m, 1H), 3.02 - 2.87 (m, 2H), 2.87 - 2.79 (m, 1H), 2.75 (d, 1H), 1.97 - 1.88 (m, 1H), 1.42 (s, 6H), 1.07 - 0.98 (m, 2H), 0.85 - 0.78 (m, 2H).

**Compound 121-2:**

$^1$H NMR (400 MHz, DMSO-*d6*) δ = 8.52 (s, 2H), 6.73 (s, 1H), 6.53 (s, 2H), 6.47 (s, 1H), 4.29-4.22 (m, 2H), 3.94-3.89 (m, 1H), 3.84 - 3.57 (m, 3H), 3.46 - 3.34 (m, 1H), 3.28 - 3.17 (m, 2H), 3.12 (s, 1H), 3.03 - 2.87 (m, 2H), 2.87 - 2.79 (m, 1H), 2.75 (d, 1H), 1.96 - 1.88 (m, 1H), 1.42 (s, 6H), 1.07 - 0.99 (m, 2H), 0.88 - 0.78 (m, 2H).

**Example 122**

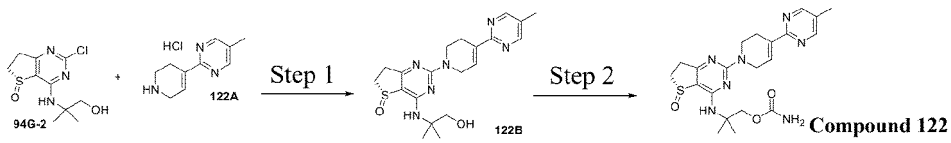


Using **94G-2** and **122A** (synthesized according to WO 2017148518) as raw materials, the target compound **122** (26 mg, 12%) was obtained by preparation and purification by HPLC according to Example 114 (the operations of steps 4, 5, and 6).

LCMS (ESI): m/z = 458.5 $[M+H]^+$

Preparation method: instrument: SHIMADZU LC-20AP; preparative column: Phenomenex C18; mobile phase: A was 10 mM $NH_4HCO_3$ in $H_2O$; B was acetonitrile; gradient: phase B from 33 to 63 in 10 min; flow rate: 25 mL/min; column temperature: room temperature; detection wavelength: 210 nm;

$^1$H NMR (400 MHz, DMSO-d6) δ 8.63 (s, 2H), 7.23-7.19 (m, 1H), 6.70-6.45 (m, 2H), 4.47 (s, 2H), 4.30 (s, 2H), 4.00 (t, 2H), 3.47-3.38 (m, 1H), 3.35-3.22 (m, 2H), 3.01 - 2.83 (m, 2H), 2.68 (s, 2H), 2.26 (s, 3H), 1.46 (d, 6H).

**Example 123**

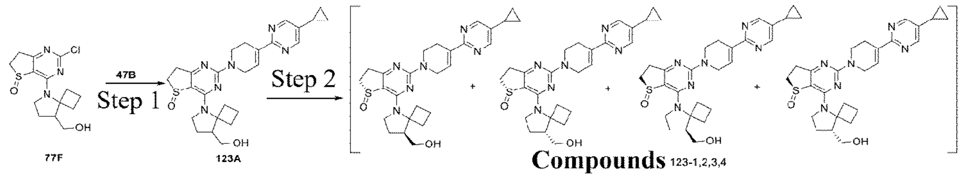


Using **77F** and **47B** as raw materials, compound **123-1** (70.9 mg), compound **123-2** (97.0 mg), compound **123-3** (58.4 mg), and compound **123-4** (35.0 mg) were obtained by chiral resolution according to Example 77 (steps 6 and 7).

HPLC analysis method: instrument: SHIMADZU LC-2020AD, column: C18 Column; mobile phase: A: 0.1% TFA in $H_2O$, B: ACN; gradient: 10-80% B in A; flow rate: 1.2 mL/min, column temperature: 45°C, wavelength: 210 nm & 254 nm.

HPLC preparation method: instrument: SHIMADZU LC-20AP, column: XB-SiOH Column; mobile phase: A: hexane, B: EtOH; gradient: 15-45% B gradient elution; flow rate: 90 mL/min, column temperature: 25°C; wavelength: 220 nm; cycle time: 5.0 min, sample preparation: sample concentration 50 mg/ml, acetonitrile solution injection: 8.0 ml each time.

After separation, the fraction was dried by a rotary evaporator at a bath temperature of 35°C to obtain compound **P1** and compound **P2.**

SFC analysis method: instrument: SHIMADZU LC-30AD, column: Chiralcel AS Column; mobile phase: A:$CO_2$, B: 0.05% DEA in IPA and ACN; gradient: 60% B in A; flow rate: 3 mL/min, column temperature: 35°C, wavelength: 220 nm.

SFC preparation method: instrument: Waters 150 Prep-SFC E, column: Chiralcel Cellulose-2 Column; mobile phase: A: $CO_2$, B: 0.1% $NH_3 \cdot H_2O$ in EtOH and ACN; gradient: 60% B gradient elution; flow rate: 100 mL/min, column temperature: 25°C; wavelength: 220 nm; cycle time: 6.0 min; sample preparation: sample concentration 10 mg/ml, acetonitrile solution injection: 1.5 ml each time.

After separation, the fraction was dried by a rotary evaporator at a bath temperature of 35°C to obtain compound **123-1** (70.9 mg), compound **123-2** (97.0 mg), compound **123-3** (58.4 mg), and compound **123-4** (35.0 mg).

Compound **123-1:** retention time: 2.018 min; $^1H$ NMR (400 MHz, $CDCl_3$) δ 8.43 (s, 2H), 7.24 - 7.22 (m, 1H), 4.61 - 4.60 (m, 2H), 4.16 - 4.10 (m, 3H), 4.03 - 3.98 (m, 1H), 3.89 - 3.83 (m, 1H), 3.81 - 3.71 (m, 2H), 3.58 - 3.50 (m, 1H), 3.15 - 3.03 (m, 3H), 2.82 - 2.81 (m, 2H), 2.36 - 2.30 (m, 1H), 2.19 - 2.12 (m, 1H), 2.04 - 1.95 (m, 2H), 1.91-1.84 (m, 3H), 1.82 - 1.72 (m, 2H), 1.61 (s, 1H), 1.11 - 1.06 (m, 2H), 0.79 - 0.75 (m, 2H).

LC-MS (ESI): m/z = 493.3 $[M+H]^+$

Compound **123-2:** retention time: 2.062 min; $^1H$ NMR (400 MHz, $CDCl_3$) δ 8.43 (s, 2H), 7.24 - 7.22 (m, 1H), 4.61 - 4.60 (m, 2H), 4.16 - 4.10 (m, 3H), 4.03 - 3.98 (m, 1H), 3.89 - 3.83 (m, 1H), 3.81 - 3.71 (m, 2H), 3.58 - 3.50 (m, 1H), 3.12 - 3.05 (m, 3H), 2.82 - 2.81 (m, 2H), 2.36 - 2.30 (m, 1H), 2.19 - 2.12 (m, 1H), 2.04 - 1.95 (m, 2H), 1.91-1.84 (m, 3H), 1.82 - 1.72 (m, 2H), 1.61 (s, 1H), 1.11 - 1.06 (m, 2H), 0.79 - 0.75 (m, 2H).

LC-MS (ESI): m/z = 493.3 $[M+H]^+$

Compound **123-3:** retention time: 2.061 min; $^1H$ NMR (400 MHz, $CDCl_3$) δ 8.43 (s, 2H), 7.23 - 7.22 (m, 1H), 4.61 - 4.60 (m, 2H), 4.16 - 4.10 (m, 3H), 4.03 - 3.97 (m, 1H), 3.89 - 3.83 (m, 1H), 3.81 - 3.71 (m, 3H), 3.58 - 3.50 (m, 1H), 3.15 - 3.05 (m, 3H), 2.82 (s, 2H), 2.36 - 2.30 (m, 1H), 2.19 - 2.12 (m, 1H), 2.04 - 1.95 (m, 2H), 1.92 - 1.82 (m, 3H), 1.80 - 1.73 (m, 2H), 1.11 - 1.06 (m, 2H), 0.79 - 0.75 (m, 2H).

LC-MS (ESI): m/z = 493.3 $[M+H]^+$

Compound **123-4:** retention time: 2.021 min; $^1H$ NMR (400 MHz, $CDCl_3$) δ 8.43 (s, 2H), 7.23 - 7.22 (m, 1H), 4.61 - 4.60 (m, 2H), 4.16 - 4.10 (m, 3H), 4.03 - 3.97 (m, 1H), 3.89 - 3.83 (m, 1H), 3.81 - 3.71 (m, 3H), 3.58 - 3.50 (m, 1H), 3.15 - 3.05 (m, 3H), 2.82 (s, 2H), 2.36 - 2.30 (m, 1H), 2.19 - 2.12 (m, 1H), 2.04 - 1.95 (m, 2H), 1.92 - 1.82 (m, 3H), 1.80 - 1.75 (m, 2H), 1.11 - 1.06 (m, 2H), 0.79 - 0.75 (m, 2H).

LC-MS (ESI): m/z = 493.3 $[M+H]^+$

**Example 124**

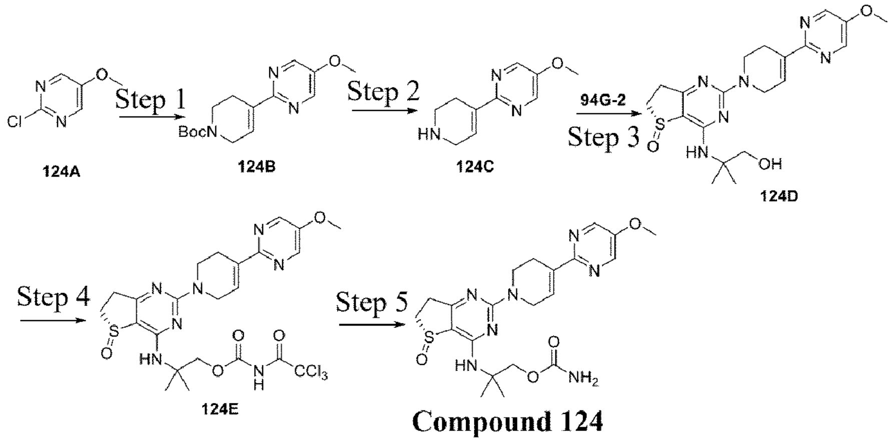

124A 124B 124C 124D 124E
Compound 124

Using **124A** as a raw material, the target compound **124** (80 mg, 18.18%) was obtained according to the operation of Example 119.

$^1$H NMR (400 MHz, $CDCl_3$) δ 8.37 (s, 2H), 7.12 - 7.10 (m, 1H), 5.48 (s, 1H), 4.51 (brs, 2H), 4.41 - 4.30 (m, 2H), 4.08 - 4.07 (m, 2H), 3.92 (s, 3H), 3.64 - 3.56 (m, 1H), 3.47 (s, 2H), 3.42 - 3.35 (m, 1H), 3.04 - 2.97 (m, 2H), 2.79 (s, 2H), 1.55 (s, 3H), 1.52 (s, 3H).

LC-MS (ESI): m/z = 474.8 $[M+H]^+$

**Example 125**

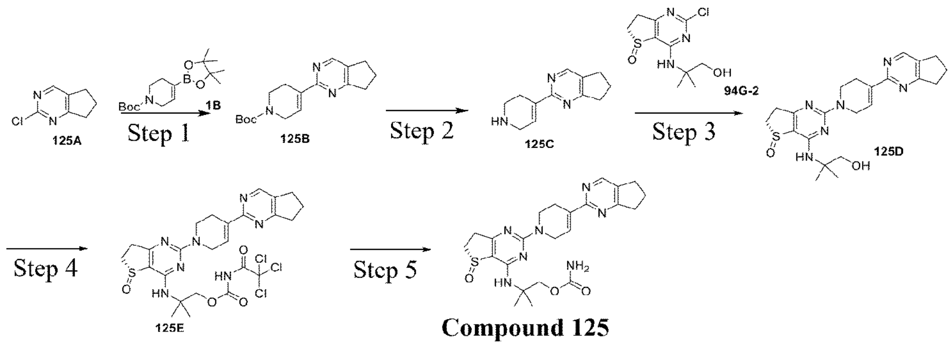

125A 125B 125C 125D 125E
Compound 125

Using **125A** as a raw material, the target compound **125** (52 mg, 17%) was obtained according to the operation of Example 119.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min.

LCMS m/z = 484.2 $[M+1]^+$

$^1$H NMR (400 MHZ, DMSO-d6) δ 8.57 (s, 1H), 7.21 (s, 1H), 6.64 (s, 1H), 6.58 (s, 2H), 4.46 (s, 2H), 4.30 (s, 2H), 4.00 (t, 2H), 3.39-3.51 (m, 1H), 3.23-3.28 (m, 1H), 2.86-3.02 (m, 6H), 2.69 (s, 2H), 2.03-2.10 (m, 2H), 1.46 (d, 6H).

**Example 126**

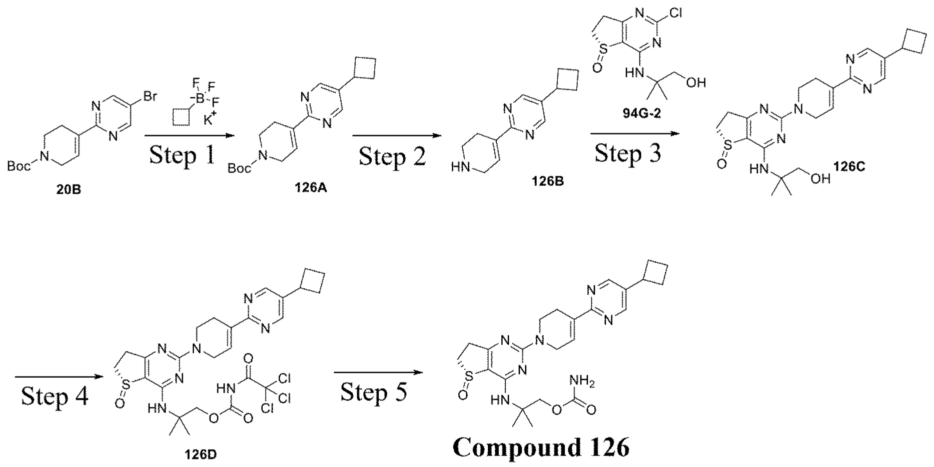


**Compound 126**

Using **20B** and potassium cyclobutyltrifluoroborate as raw materials, the title compound **126** (100 mg, 32%) was obtained according to the operation of Example 116.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 40-80%; c. flow: 15 mL/min; d. elution time: 20 min.

LCMS m/z = 498.2 $[M +1]^+$

**Compound 126,** $^1$H NMR (400 MHZ, DMSO-*d6*) δ 8.69 (s, 2H), 7.23 (s, 1H), 6.64 (s, 1H), 6.59 (s, 2H), 4.47 (s, 2H), 4.30 (s, 2H), 4.00 (t, 2H), 3.50-3.59 (m, 1H), 3.39-3.47 (m, 1H), 3.23-3.28 (m, 1H), 2.84-3.00 (m, 2H), 2.69 (s, 2H), 2.28-2.35 (m, 2H), 2.13-2.23(m, 2H), 1.97-2.06 (m, 1H), 1.83-1.91 (m, 1H), 1.46 (d, 6H).

**Example 127**

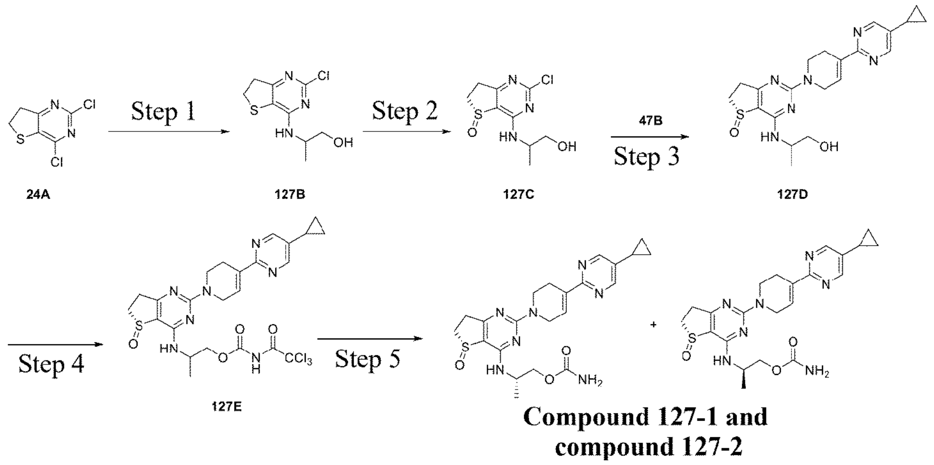


**Compound 127-1 and compound 127-2**

Using **24A** and 2-aminopropanol as raw materials, compounds **127-1** (0.9 g, 20%, Rf 0.6) and **127-2** (1.8 g, 41%, Rf 0.3) were obtained by separation and purification through silica gel column chromatography (dichloromethane/methanol = 10/1) according to the operation of Example 114.

LC-MS (ESI): m/z = 470.2 $[M+H]^+$.

$^1$H NMR (400 MHz, DMSO-$d_6$) δ 8.54 (s, 2H), 7.56 (d, 1H), 7.28 - 7.12 (m, 1H), 6.50 (s, 2H), 4.60 - 4.49 (m, 1H), 4.49 - 4.38 (m, 2H), 4.10 - 3.79 (m, 4H), 3.51 - 3.40 (m, 1H), 3.27 - 3.16 (m, 1H), 3.01 - 2.82 (m, 2H), 2.66 (s, 2H), 1.98 - 1.89 (m, 1H), 1.17 (d, 3H), 1.11 - 1.00 (m, 2H), 0.90 - 0.76 (m, 2H).

LC-MS (ESI): m/z = 470.2 $[M+H]^+$.

$^1$H NMR (400 MHz, DMSO-$d_6$) δ 8.54 (s, 2H), 7.50 (d, 1H), 7.21 - 7.15 (m, 1H), 6.47 (s, 2H), 4.65 - 4.49 (m, 1H), 4.49 - 4.38 (m, 2H), 4.09 - 3.87 (m, 4H), 3.51 - 3.39 (m, 1H), 3.26 - 3.15 (m, 1H), 3.05 - 2.83 (m, 2H), 2.66 (s, 2H), 1.98 - 1.89

(m, 1H), 1.19 (d, 3H), 1.08 - 0.95 (m, 2H), 0.91 - 0.80 (m, 2H).

Example **128**

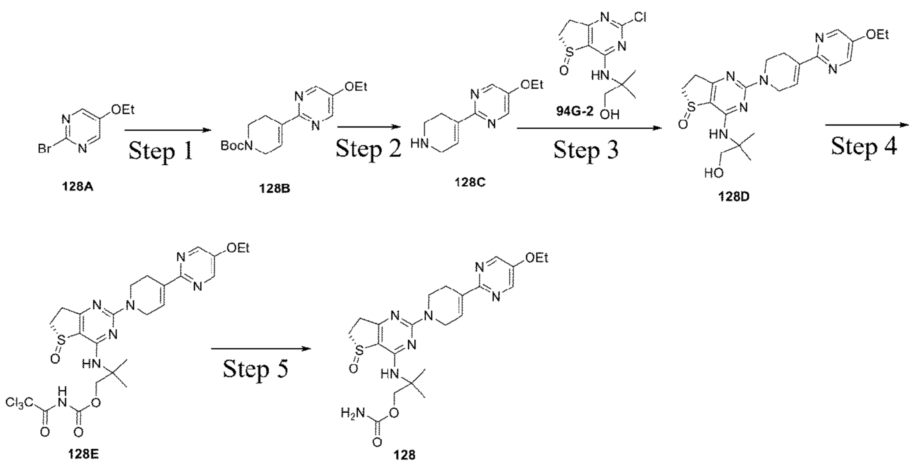


Using **128A** as a raw material, the target compound **128** (490 mg, 41%) was obtained according to the operation of Example 119.
LC-MS (ESI): m/z = 488.2 $[M+H]^+$
$^1H$ NMR (400 MHz, DMSO-$d_6$) δ 8.51 (s, 2H), 7.19 - 7.01 (m, 1H), 6.70 - 6.40 (m, 3H), 4.45 (d, 2H), 4.30 (s, 2H), 4.23 - 4.16 (m, 2H), 4.00 (t, 2H), 3.49 - 3.36 (m, 1H), 3.29 - 3.22 (m, 1H), 3.02 - 2.91 (m, 1H), 2.91 - 2.82 (m, 1H), 2.73 - 2.62 (m, 2H), 1.46 (d, 6H), 1.36 (t, 3H).

Example **129**

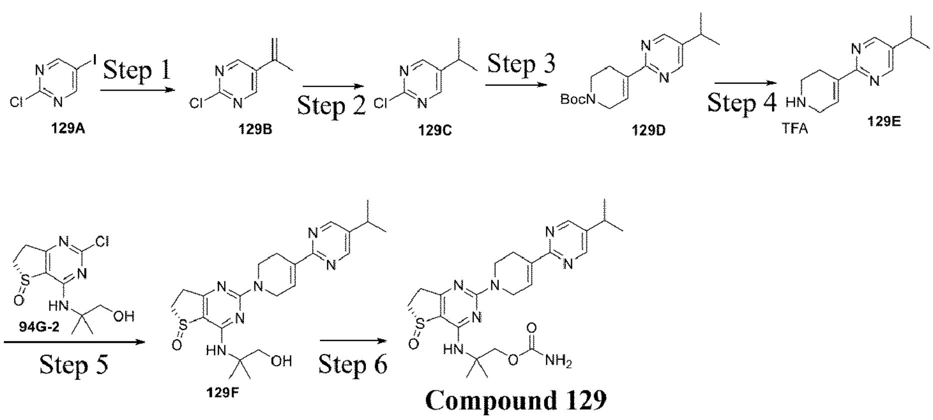


Step 1: **129A** (2.4 g, 10 mmol) was dissolved in 1,4-dioxane (50 mL), and 1,1-bis(diphenylphosphino)ferrocene-palladium dichloride (0.73 g, 1 mmol), cesium carbonate (6.5 g, 20 mmol), isopropenylboronic acid pinacol ester (2.1 g, 12 mmol), and water (10 mL) were then added. After nitrogen displacement three times, the mixture was heated to 90°C and reacted for 3 h. The system was then passed through a short silica gel column, eluted with ethyl acetate, concentrated, and subjected to column chromatography (petroleum ether/ethyl acetate = 9/1) to obtain compound **129B** (1.4 g, 90%).
LC-MS (ESI): m/z = 155.6 $[M+H]^+$。
Step 2: **129B** (1.4 g, 9 mmol) was dissolved in anhydrous ethanol, and platinum dioxide (0.2 g, 0.9 mmol) was then added. After insertion of a hydrogen balloon for purging three times, the mixture was reacted for 12 h, then passed through a short silica gel column, eluted with ethyl acetate, concentrated, and subjected to column chromatography (petroleum ether/ethyl acetate = 9 / 1) to obtain compound **129C** (1.4 g, 98%).
$^1H$ NMR (400 MHz, $CDCl_3$) δ 8.50 (s, 2H), 3.01 - 2.94 (m, 1H), 1.32 (d, 6H).

Using **129C** as a raw material, compound **129** (650 mg, 48%) was obtained according to the operation of Example 49.

LC-MS (ESI): m/z = 486.6 $[M+H]^+$.

$^1H$ NMR (400 MHz, DMSO-*d6*) δ 8.70 (s, 2H), 7.22 (s, 1H), 6.63 (s, 1H), 6.56 (s, 2H), 4.47 (s, 2H), 4.30 (s, 2H), 4.00 (t, 2H), 3.55 - 3.38 (m, 1H), 3.29 - 3.24 (m, 1H), 3.02 - 2.82 (m, 3H), 2.69 (s, 2H), 1.46 (d, 6H), 1.26 (d, 6H).

Example **130**

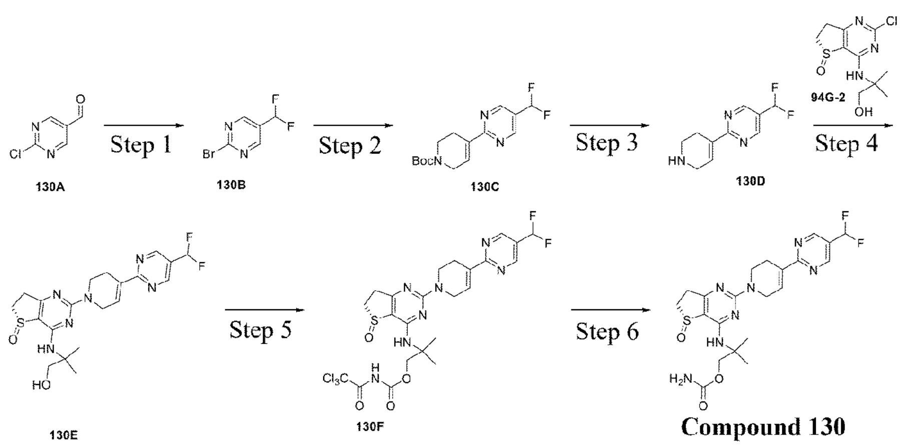


130A 130B 130C 130D 130E 130F Compound 130

Step 1: Compound **130A** (6.2 g, 43.5 mmol) was dissolved in dichloromethane (100 mL), and diethylaminosulfur trifluoride (14.1 g, 87.0 mmol) was then slowly added at -78°C. After the addition was complete, the mixture was slowly heated to room temperature and reacted overnight. The reaction was quenched by adding a sodium bicarbonate solution, and the system was extracted wtih dichloromethane, dried, filtered, concentrated, and subjected to column chromatography (petroleum ether/ethyl acetate = 10/1) to obtain compound **130B** (4.9 g, 68%).

Using **130B** as a raw material, compound **130** (800 mg, 61%) was obtained according to the operation of Example 49.

LC-MS (ESI): m/z = 494.1 $[M+H]^+$.

$^1H$ NMR (400 MHz, DMSO-*d6*) δ 9.01 (s, 2H), 7.41 (s, 1H), 7.19 (t, 1H), 6.60 (d, 3H), 4.52 (s, 2H), 4.31 (s, 2H), 4.02 (t, 2H), 3.52 - 3.36 (m, 1H), 3.28 - 3.21 (m, 1H), 3.07 - 2.78 (m, 2H), 2.72 (s, 2H), 1.47 (d, 6H).

$^{19}F$ NMR (376 MHz, DMSO-*d6*) δ -110.81 (s).

**Example 131**

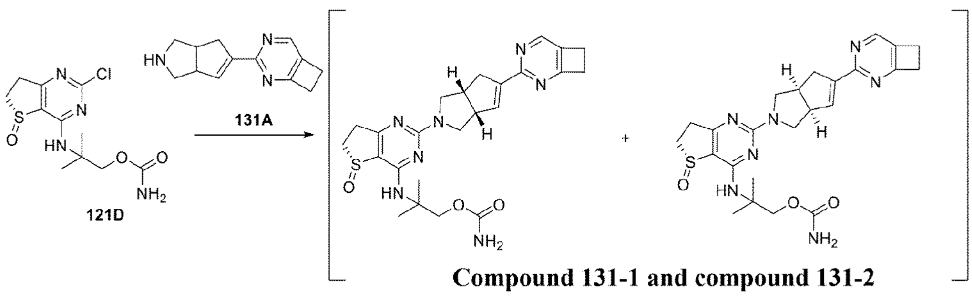


121D

Compound 131-1 and compound 131-2

Compound **121D** (149 mg, 0.47 mmol) was dissolved in 1,4-dioxane (10.0 mL), compound **131A** (100 mg, 0.47 mmol) and diisopropylethylamine (182 mg, 1.41 mmol) were then added, and the mixture was heated back to 100°C and reacted for 5 h. The system was then cooled to room temperature, concentrated, and subjected to column chromatography (dichloromethane/methanol = 10/1) to obtain compound **131** (200 mg, 86%).

LC-MS (ESI): m/z = 496.2 $[M+H]^+$.

Resolution of compound **131:** Compound **131** (200 mg) was taken for resolution, and after separation, compound **131-1** (retention time: 0.972 min, 48.9 mg, ee% = 100%) and compound **131-2** (retention time: 1.852 min, 45.8 mg, ee% =

100%) were obtained. Resolution conditions: instrument: Waters 150 Prep-SFC E; column: Chiralcel Cellulose-2 column; mobile phase: A for $CO_2$; B for 0.1% $NH_3$•$H_2O$ in IPA and ACN; gradient: B 70%; flow: 100 mL/min; back pressure: 100 bar; column temperature: 25°C; wavelength: 220 nm; period: 7.0 min;

**Compound 131-1:**

$^1$H NMR (400 MHz, DMSO-*d6*) δ = 8.32 (s, 1H), 6.75 (s, 1H), 6.52 (s, 2H), 4.36 - 4.18 (m, 2H), 3.94-3.89 (m, 1H), 3.82 - 3.60 (m, 3H), 3.45 - 3.36 (m, 3H), 3.28 - 3.23 (m, 2H), 3.21-3.19 (m, 3H), 3.15 - 3.07 (m, 1H), 3.01 - 2.88 (m, 2H), 2.87-2.81 (m, 1H), 2.74 (d, 1H), 1.44 (s, 6H).

**Compound 131-2:**

$^1$H NMR (400 MHz, DMSO-*d6*) δ = 8.32 (s, 1H), 6.75 (s, 1H), 6.54 (s, 1H), 6.47 (s, 1H), 4.33 - 4.17 (m, 2H), 3.94-3.89 (m, 1H), 3.81 - 3.60 (m, 3H), 3.41-3.35 (m, 3H), 3.28 - 3.23 (m, 2H), 3.23 - 3.16 (m, 3H), 3.16 - 3.05 (m, 1H), 3.02 - 2.88 (m, 2H), 2.87 - 2.79 (m, 1H), 2.74 (d, 1H), 1.43 (s, 6H).

Example 132

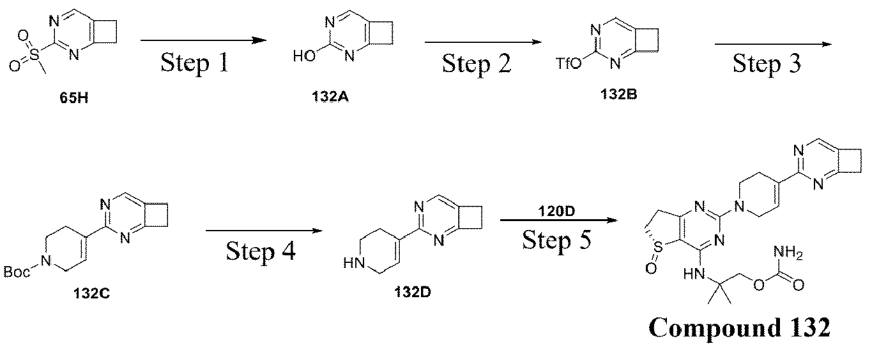


Step 1: **65H** (500 mg, 2.7 mmol) was dissolved in tetrahydrofuran (20 ml), sodium hydroxide (216 mg, 5.4 mmol) dissolved in water (0.5 ml) was dropwise added, and the mixture was heated to 60°C and reacted for 1 h. The system was then concentrated and quickly separated and purified (methanol/dichloromethane = 0-10%) to obtain compound **132A** (298 mg, 90%).

LC-MS (ESI): m/z = 123.1 [M+H]$^+$

Step 2: Compound **132A** (298 mg, 2.44 mmol) was dissolved in N,N-dimethylformamide (5 ml), and N-Phenyl-bis(trifluoromethanesulfonyl)imide (1.74 g, 4.88 mmol) and potassium carbonate (1.01 g, 7.32 mmol) were added and stirred at room temperature 3 h. After the reaction was complete, the reaction system was diluted by adding ethyl acetate (40 ml). The organic phase was washed with water (10 ml * 3), and the organic phases were dried over anhydrous sodium sulfate, concentrated, and quickly separated and purified (ethyl acetate/petroleum ether = 0-20%) to obtain compound **132B** (589 mg, 95%).

LC-MS (ESI): m/z = 255.1 [M+H]$^+$

Step 3: Compound **132B** (100 mg, 0.39 mmol) and tert-butyl 4-(4,4,5,5-tetramethyl-1,3,2-dioxaborolan-2-yl)-3,6-dihydropyridine-1(2H)-carboxylate (183 mg, 0.59 mmol) were dissolved in 1,4-dioxane/water (5 ml/1 ml), and Pd (dppf) $Cl_2$ (29 mg, 0.04 mmol) and cesium carbonate (381 mg, 1.17 mmol) were added. After nitrogen displacement three times, the mixture was heated to 100°C and reacted for 3 h, and the system was then concentrated and quickly separated and purified (petroleum ether/ethyl acetate = 10/1) to obtain compound **132C** (101 mg, 90%).

LC-MS (ESI): m/z = 288. 2 [M+H]$^+$。

Step 4: Compound **132C** (50 mg, 0.18 mmol) was dissolved in dichloromethane/trifluoroacetic acid (6 mL/2 ml) and reacted at room temperature for half an hour, and the system was then concentrated to obtain compound **132D,** which was directly used for the next step.

LC-MS (ESI): m/z = 188.2 [M+H]$^+$.

Step 5: The compound **132D** from the above step was dissolved in 1,4-dioxane (10.0 mL), and diisopropylethylamine (70 mg, 0.54 mmol) and compound **120D** (55 mg, 0.18 mmol) were then added, heated back to 100°C, and reacted for 5 h, and the system was then cooled to room temperature, concentrated, and quickly separated and purified (dichloromethane/methanol = 10/1) to obtain compound **132** (30 mg, 36%).

$^1$H NMR (400 MHz, DMSO-*d6*) δ = 8.35 (s, 1H), 7.21 (s, 1H), 6.64 (s, 1H), 6.56 (s, 1H), 4.46 (s, 2H), 4.30 (s, 2H), 3.99 (t, J=5.6, 2H), 3.49 - 3.36 (m, 3H), 3.29 - 3.24 (m, 1H), 3.24 - 3.18 (m, 2H), 3.02 - 2.92 (m, 1H), 2.88-2.84 (m, 1H), 2.67 (s, 2H), 1.46 (d, 6H).

LC-MS (ESI): m/z = 470.2 [M+H]$^+$.

**Example 133**

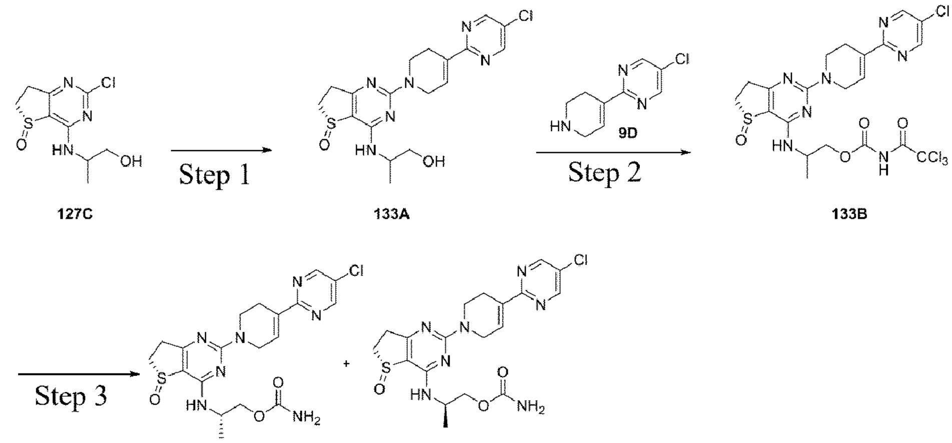


127C 133A 133B

**Compound 133-1 and compound 133-2**

Using **127C** as a raw material, compounds **133-1** (241 mg, 6%) and **133-2** (120 mg, 3%) were obtained by resolution according to the synthesis method in Example 127. Preparation method: instrument: WATYERS 150 preparative SFC (SFC-26), column: ChiralPak AD column (250 mm * 30 mm, 10 μm), mobile phase: (phase A: CO2, phase B: IPA (0.1% $NH_3$•$H_2O$)); gradient: 30% mobile phase B, isocratic elution; flow rate: 150 mL/min, back pressure: 100 bar, column temperature: 38°C, wavelength: 220 nm, cycle time: 30 min, sample preparation: sample concentration 5 mg/ml, methanol/DCM solution injection: 4.0 ml each time. After separation, the fraction was dried by a rotary evaporator in a water bath at 40°C to obtain products **133-1** (retention time: 1.934 min) and **133-2** (retention time: 2.424 min).

**133-1:** LC-MS (ESI): m/z = 464.1 [M+H]$^+$.

$^1$H NMR (400 MHz, DMSO-$d_6$) δ 8.90 (s, 2H), 7.51 (d, 1H), 7.40 - 7.17 (m, 1H), 6.46 (s, 2H), 4.63 - 4.42 (m, 3H), 4.10 - 3.86 (m, 4H), 3.50 - 3.38(m, 1H), 3.26 - 3.20 (m, 1H), 3.05 - 2.93 (m, 1H), 2.92 - 2.83 (m, 1H), 2.73 - 2.61 (m, 2H), 1.19 (d, 3H).

**133-2:** LC-MS (ESI): m/z = 464.1 [M+H]$^+$.

$^1$H NMR (400 MHz, DMSO-$d_6$) δ 8.90 (s, 2H), 7.57 (d, 1H), 7.34 - 7.22 (m, 1H), 6.50 (s, 2H), 4.63 - 4.42 (m, 3H), 4.10 - 3.86 (m, 4H), 3.52 - 3.40 (m, 1H), 3.26 - 3.18 (m, 1H), 3.05 - 2.93 (m, 1H), 2.92 - 2.83 (m, 1H), 2.71 - 2.61 (m, 2H), 1.17 (d, 3H).

Example **134**

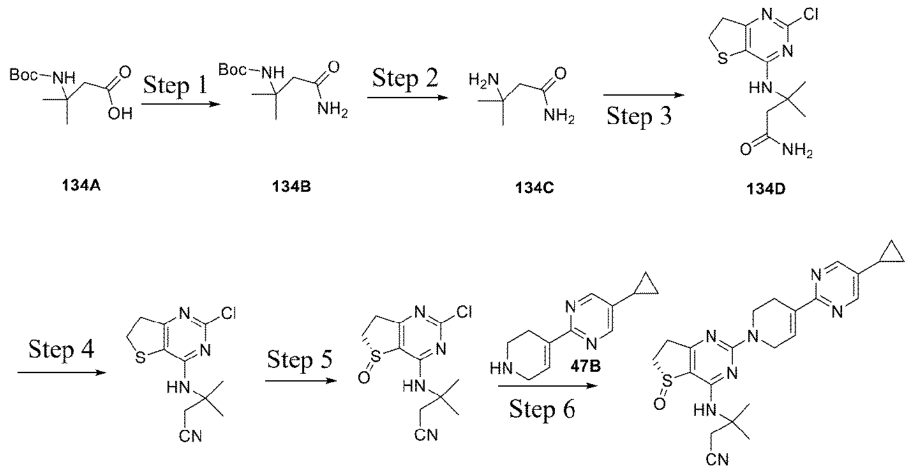


134A 134B 134C 134D

134E 134F 134

Using **134A** as a raw material, compound **134** (315 mg, 50%) was obtained according to the operation of Example 83. Preparation method: instrument: Waters 150 Prep-SFC A, column: Chiralcel AD column (250 mm × 30 mm, 10 μm), mobile phase: (phase A: CO2, phase B: IPA and ACN (0.1% $NH_3$•$H_2O$)); gradient: 70% mobile phase B, isocratic elution; flow rate: 100 mL/min, back pressure: 100 bar, column temperature: 25°C, wavelength: 220 nm, cycle time: 5.3 min, sample preparation: sample concentration 10 mg/ml, acetonitrile solution injection: 2.5 ml each time. After separation, the fraction was dried in a water bath at 35°C by a rotary evaporator to obtain a product. The solvent was then dried with a freeze-dryer at -80°C to obtain the final product **134** (retention time: 0.828 min).

LC-MS (ESI): m/z = 450.3 $[M+H]^+$

$^1H$ NMR (400 MHz, DMSO-$d_6$) δ 8.55 (s, 2H), 7.29 - 7.15 (m, 1H), 7.04 (s, 1H), 4.45 (s, 2H), 3.98 (t, 2H), 3.51 3.40 (m, 1H), 3.35 (d, 1H), 3.28 - 3.21 (m, 2H), 3.06 - 2.94 (m, 1H), 2.94 - 2.84 (m, 1H), 2.68 (s, 2H), 2.02 - 1.90 (m, 1H), 1.54 (d, 6H), 1.09 - 0.98 (m, 2H), 0.92 - 0.78 (m, 2H).

Example **135**

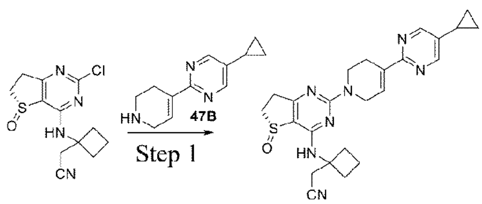


83F 135

Step 1: Compound **83F** (500 mg, 1.68 mmol) was dissolved in 1,4-dioxane (20 mL), and compound **47B** (410 mg, 2.02 mmol) and N,N-diisopropylethylamine (1.08 g, 8.39 mmol) were then added and stirred at 90 degrees Celsius overnight under nitrogen protection. After the reaction was complete, the reaction liquid was concentrated and subjected to silica gel column chromatography (dichloromethane/methanol = 10/1), and the crude product was subjected to SFC chiral resolution to obtain compound **135** (608 mg, 78%).

Preparation method: instrument: Waters 150 Prep-SFC F, column: Chiralcel AD column (250 mm * 30 mm, 10 μm), mobile phase: (phase A: CO2, phase B: IPA and ACN (0.1% $NH_3$•$H_2O$)); gradient: 70% mobile phase B, isocratic elution; flow rate: 100 mL/min, back pressure: 100 bar, column temperature: 25°C, wavelength: 220 nm, cycle time: 4.3 min, sample preparation: sample concentration 5 mg/ml, acetonitrile solution injection: 2.0 ml each time. After separation, the fraction was dried in a water bath at 35°C by a rotary evaporator to obtain a product. The solvent was then dried with a freeze-dryer at -80°C to obtain the final product **135** (retention time: 0.956 min).

LC-MS (ESI): m/z = 462.2 $[M+H]^+$

$^1H$ NMR (400 MHz, DMSO-*d6*) δ 8.54 (s, 2H), 8.14 (s, 1H), 7.20 - 7.16 (m, 1H), 4.42 (s, 2H), 3.97 (d, 2H), 3.52 - 3.40 (m, 1H), 3.36 (d, 2H), 3.28 - 3.17 (m, 1H), 3.03 - 2.94 (m, 1H), 2.93 - 2.85 (m, 1H), 2.75 - 2.61 (m, 2H), 2.48 - 2.33 (m, 2H), 2.32 - 2.20 (m, 2H), 2.02 - 1.79 (m, 3H), 1.09 - 0.93 (m, 2H), 0.88 - 0.76 (m, 2H).

Example **136**

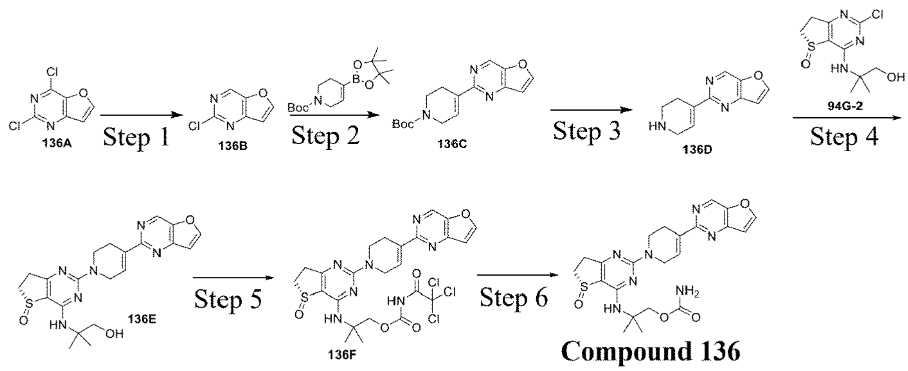


Step 1: Compound **136A** (5.00 g, 26.49 mmol) and acetic acid (9.54 g, 158.94 mmol) were weighed into a 100 mL single-mouth flask and dissolved with methanol (50 mL), and zinc powder (6.93 g, 105.96 mmol) was added in portions. After the addition was complete, the mixture was stirred at 70 degrees Celsius for 0.5 h. After the reaction was complete as detected by a TLC spotting plate (petroleum ether:ethyl acetate = 5:1), water (50 mL) was added and stirred for 5 min, and the mixture was extracted with ethyl acetate (50 mL). The organic phase was separated, dried over anhydrous sodium sulfate, and concentrated under reduced pressure, and the crude product was purified by column chromatography (eluents: petroleum ether:ethyl acetate = 5:1) to obtain the target compound **136B** (1.0 g, yield: 24%).

LCMS m/z = 155.1 $[M+1]^+$

Using compound **136B** as a raw material, the title compound 1**36** (90 mg, 23%) was obtained according to the operation of Example 119.

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A with a content of 30-70%; c. flow: 15 mL/min; d. elution time: 20 min.

LCMS m/z = 484.2 $[M+1]^+$

**Compound 136,** $^1$H NMR (400 MHZ, DMSO-*d6*) δ 9.13 (s, 1H), 8.52 (d, 1H), 7.26 (s, 1H), 7.22 (d, 1H), 6.63 (s, 1H), 6.55 (s, 2H), 4.49 (s, 2H), 4.31 (s, 2H), 4.03 (t, 2H), 3.40-3.51 (m, 1H), 3.24-3.31 (m, 1H), 2.84-3.01 (m, 2H), 2.78 (s, 2H), 1.47 (d, 6H).

Example **137**

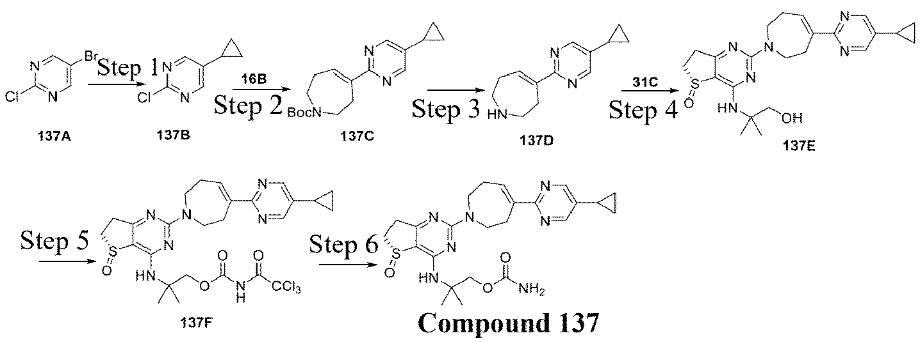


Step 1: Compound **137A** (2.00 g, 10.34 mmol) was dissolved in tetrahydrofuran (40 mL), and potassium carbonate (3.57 g, 25.85 mmol), cyclopropylboronic acid (0.98 g, 11.37 mmol), and water (8 mL) were then successively added. After nitrogen displacement three times, 1,1-bis(diphenylphosphino)ferrocene-palladium dichloride (0.92 g, 1.24 mmol) was added. After the addition was complete, the reaction was heated to 65°C and stirred for 16 h. After the reaction liquid naturally returned to room temperature, the reaction was diluted by adding water (50 mL) and extracted with ethyl

acetate (50 mL) three times. The organic phases were combined, dried over anhydrous sodium sulfate, and then concentrated in vacuum to obtain a crude product, which was purified by column chromatography (eluents: petroleum ether/ethyl acetate = 4/1) to obtain compound **137B** (1.00 g, 62.56%).

$^1$H NMR (400 MHz, $CDCl_3$) δ 8.35 (s, 2H), 1.90 - 1.83 (m, 1H), 1.16 1.11 (m, 2H), 0.81 - 0.77 (m, 2H).

LC-MS (ESI): m/z = 155.1 $[M+H]^+$.

Using **137B** as a raw material, the target compound **137** (48 mg, 13.20%) was obtained according to the operation of Example 119.

$^1$H NMR (400 MHz, $CDCl_3$) δ 8.38 (s, 2H), 7.35 - 7.28 (m, 1H), 5.34 (s, 1H), 4.80 (brs, 2H), 4.63 - 4.51 (m, 2H), 4.38 - 4.31 (m, 2H), 4.05 - 3.95 (m, 2H), 3.60 - 3.52 (m, 1H), 3.38 - 3.31 (m, 1H), 3.01 - 2.93 (m, 4H), 2.04 - 1.98 (m ,2H), 1.86 - 1.79 (m, 1H), 1.51 (s, 3H), 1.49 (s, 3H), 1.08 - 1.03 (m, 2H), 0.76 - 0.72 (m, 2H).

LC-MS (ESI): m/z = 498.2 $[M+H]^+$.

Example **138**

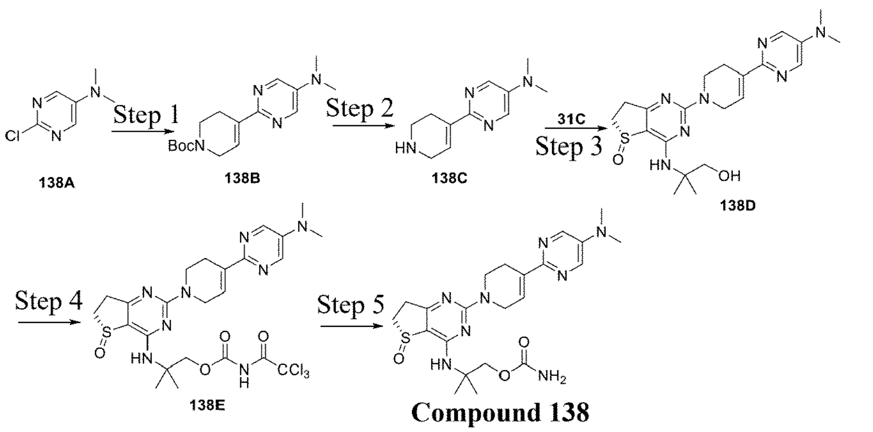


Using **138A** (see document *JACS-142(50)-21197* for the synthesis) as a raw material, the target compound **138** (125 mg, 81.58%) was obtained according to the operation of Example 119.

$^1$H NMR (400 MHz, $CDCl_3$) δ 8.19 (s, 2H), 7.00 - 6.98 (m, 1H), 5.38 (s, 1H), 4.85 - 4.76 (m, 2H), 4.50 - 4.48 (m, 2H), 4.41 - 4.33 (m, 2H), 4.09 - 4.05 (m, 2H), 3.64 - 3.56 (m, 1H), 3.42 - 3.35 (m, 1H), 3.03 - 2.98 (m, 8H), 2.81 - 2.77 (m, 2H), 1.54 (s, 3H), 1.52 (s, 3H).

LC-MS (ESI): m/z = 487.2 $[M+H]^+$

**Example 139**

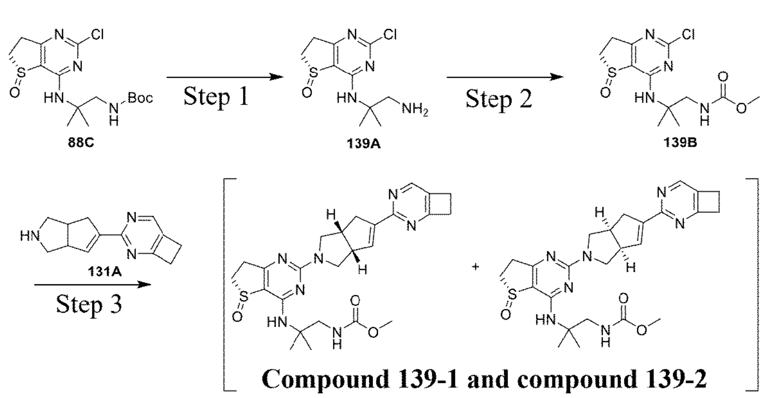


Step 1: Compound **88C** (500 mg, 1.33 mmol) was dissolved in dichloromethane/trifluoroacetic acid (12 mL/4 ml) and reacted at room temperature for half an hour, and the system was then concentrated to obtain compound **139A,** which was directly used for the next step.

LC-MS (ESI): m/z = 275.1 [M+H]+

Step 2: The compound **139A** trifluoroacetate compound from the previous step was dissolved in tetrahydrofuran/water (10 ml/10 ml), sodium bicarbonate (337 mg, 4.01 mmol) was added, and the mixture was placed in an ice bath and cooled. Methyl chloroformate (189 mg, 2.0 mmol) was dropwise added to the reaction system. After the reaction was complete, the system was directly spin-dried and quickly separated and purified (eluent ratio: MeOH/DCM = 0-20%) to obtain compound **139B** (421 mg, 95%).

LC-MS (ESI): m/z = 333.1[M+H]$^+$

Step 3: Using **139B** as a raw material, compounds **139-1** (18.5 mg, 24%) and **139-2** (20.3 mg, 26%) were obtained by resolution according to the synthesis method in Example 131.

Resolution of compound **139:** Compound **139** (60 mg) was taken for resolution, and after separation, compound **139-1** (retention time: 1.574 min, 18.5 mg, ee% = 100%) and compound **139-2** (retention time: 2.485 min, 20.5 mg, ee% = 100%) were obtained. Resolution conditions: instrument: Waters 150 Prep-SFC E; column: Chiralcel Cellulose-2 column; mobile phase: A for CO2; B for 0.1% $NH_3$•$H_2O$ in IPA and ACN; gradient: B 70%; flow: 100 mL/min; back pressure: 100 bar; column temperature: 25°C; wavelength: 220 nm; period: 7.0 min;

Compound **139-1:**

$^1$H NMR (400 MHz, $CDCl_3$) δ = 8.24 (s, 1H), 6.81 (s, 1H), 5.51 (s, 1H), 5.24 (d, 1H), 4.10 - 3.95 (m, 1H), 3.94 - 3.76 (m, 2H), 3.70 - 3.54 (m, 7H), 3.50 - 3.42 (m, 2H), 3.41 - 3.28 (m, 2H), 3.23 (t, 2H), 3.20 - 3.14 (m, 1H), 3.10 - 3.06 (m, 1H), 3.04 - 2.93 (m, 2H), 2.88 (d, 1H), 1.46 (d, 6H).

LC-MS (ESI): m/z = 510.2[M+H]$^+$

Compound **139-2:**

$^1$H NMR (400 MHz, $CDCl_3$) δ = 8.24 (s, 1H), 6.81 (s, 1H), 5.48 (s, 1H), 5.25 (d, 1H), 4.08-4.00 (m, 1H), 3.97 - 3.77 (m, 2H), 3.76 - 3.50 (m, 7H), 3.49 - 3.43 (m, 2H), 3.42 - 3.29 (m, 2H), 3.27 - 3.21 (m, 2H), 3.21 - 3.15 (m, 1H), 3.15 - 2.94 (m, 3H), 2.88 (d, 1H), 1.45 (s, 6H).

LC-MS (ESI): m/z = 510.2 [M+H]$^+$

**Example 140**

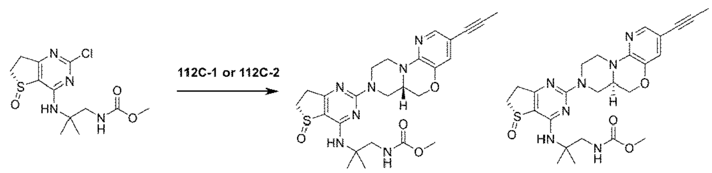


139B

**Compound 140-1**
**or compound 140-2**

Using **139B** and **112C-1** as raw materials, compound **140-1** (10 mg, 32%) was obtained according to the synthesis method in Example **112.**

Using **139B** and **112C-2** as raw materials, compound **140-2** (10 mg, 32%) was obtained by resolution according to the synthesis method in Example **112.**

Compound **140-1:**

$^1$H NMR (400 MHz, $CDCl_3$) δ = 7.86 (d,1H), 6.96 (d, 1H), 5.65 (s, 1H), 5.29 (d, 1H), 4.84 (d, 2H), 4.61 (d, 1H), 4.35 (d, 1H), 4.03-3.99 (m, 1H), 3.69 (s, 3H), 3.68 - 3.48 (m, 3H), 3.47 - 3.31 (m, 2H), 3.13 (t, 1H), 3.08 - 2.95 (m, 2H), 2.92 - 2.74 (m, 2H), 2.03 (s, 3H), 1.47 (d, 6H).

LC-MS (ESI): m/z = 526.2 [M+H]$^+$

Compound **140-2:**

$^1$H NMR (400 MHz, $CDCl_3$) δ = 7.86 (d, 1H), 6.96 (d, 1H), 5.65 (s, 1H), 5.28 (t, 1H), 4.84 (d, 2H), 4.61 (d, 1H), 4.35 (d, 1H), 4.03 - 3.99 (m, 1H), 3.69 (s, 3H), 3.62 - 3.56 (m, 3H), 3.45 - 3.33 (m, 2H), 3.19 - 3.09 (m, 1H), 3.06 - 2.97 (m, 2H), 2.90

- 2.74 (m, 2H), 2.03 (s, 3H), 1.47 (s, 6H).
LC-MS (ESI): m/z = 526.2 [M+H]$^+$

**Example 141:**

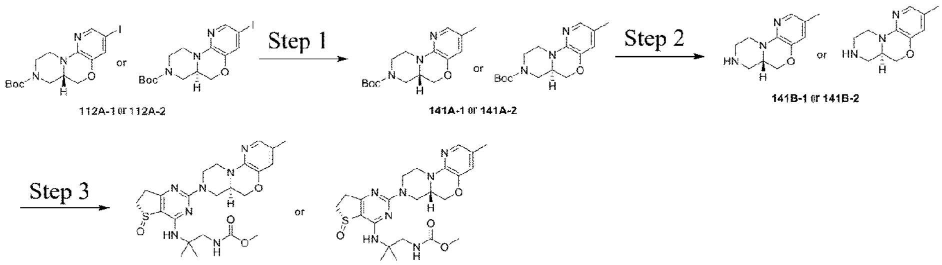


**Compound 141-1 or compound 141-2**

Step 1: Compound **112A-1** (400 mg, 0.96 mmol) and trimethylboroxine (1.92 mmol) were dissolved in N,N-dimethylformamide (10 ml), and Pd(dppf)$Cl_2$ (73 mg, 0.01 mmol) and potassium carbonate (397 mg, 2.88 mmol) were added. After nitrogen displacement three times, the mixture was heated to 110°C and reacted for 3 h, and the system was then concentrated and quickly separated and purified (petroleum ether/ethyl acetate = 10/1) to obtain compound **141A-1** (200 mg, 68%).
LC-MS (ESI): m/z = 306.2 [M+H]$^+$
Using **141A-1** (100 mg, 0.33 mmol) as a raw material, compound **141-1** (76 mg, 46%) was obtained according to the synthesis method in Example **112.**
According to the above method, using **112A-2** as a raw material, the target compound **141-2** (58 mg, 35%) was obtained.

Compound **141-1:**

$^1$H NMR (400 MHz, DMSO-d6) δ = 7.57 (s, 1H), 7.43 (t, 1H), 6.87 (s, 1H), 6.72 (s, 1H), 4.79 (d, 2H), 4.42 (d, 2H), 4.04 - 3.92 (m, 1H), 3.58 (s, 3H), 3.48 - 3.38 (m, 2H), 3.34 (d, 1H), 3.27 - 3.19 (m, 2H), 3.09 - 2.92 (m, 2H), 2.89 - 2.84 (m, 1H), 2.76 (t, 1H), 2.67 (t, 1H), 2.12 (s, 3H), 1.40 (d, 6H).
LC-MS (ESI): m/z = 502.2 [M+H]$^+$

Compound **141-2:**

$^1$H NMR (400 MHz, DMSO-*d6*) δ = 7.57 (s, 1H), 7.43 (t, 1H), 6.87 (s, 1H), 6.73 (s, 1H), 4.80 (d, 2H), 4.41 (d, 2H), 4.05 - 3.93 (m, 1H), 3.58 (s, 3H), 3.48 - 3.38 (m, 3H), 3.29 - 3.19 (m, 2H), 3.08 - 2.92 (m, 2H), 2.89 - 2.84 (m, 1H), 2.76 (t, 1H), 2.67 (t, 1H), 2.12 (s, 3H), 1.40 (d, 6H).
LC-MS (ESI): m/z = 502.2 [M+H]$^+$

**Example 142**

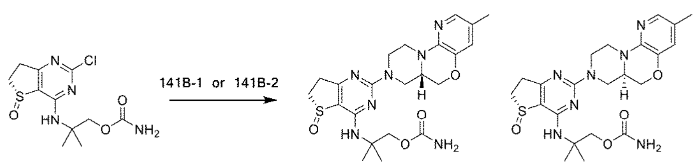


121D

**Compound 142-1 and compound 142-2**

Using **121D** and **141B-1** (67 mg, 0.33 mmol) as raw materials, compound **142-1** (88 mg, 55%) was obtained according to the synthesis method in Example **121.**

According to the above method, using **121D** and **141B-2** (67 mg, 0.33 mmol) as raw materials, the target compound **142-2** (100 mg, 62%) was obtained.

Compound **142-1:**

$^1$H NMR (400 MHz, DMSO-*d6*) δ = 7.57 (s, 1H), 6.87 (s, 1H), 6.70 (s, 1H), 6.56 (s, 2H), 4.76 (d, 2H), 4.48 - 4.21 (m, 4H), 4.05 - 3.90 (m, 1H), 3.53 - 3.37 (m, 1H), 3.29 - 3.20 (m, 2H), 3.08 - 2.92 (m, 2H), 2.90 - 2.85 (m, 1H), 2.77 (t, 1H), 2.70 - 2.65 (m, 1H), 2.12 (s, 3H), 1.43 (d, 6H).
LC-MS (ESI): m/z = 488.2 $[M+H]^+$

Compound **142-2:**

$^1$H NMR (400 MHz, DMSO-*d6*) δ = 7.57 (s, 1H), 6.87 (d, 1H), 6.71 (s, 1H), 6.56 (s, 2H), 4.77 (d, 2H), 4.47 - 4.20 (m, 4H), 4.03 - 3.92 (m, 1H), 3.50 - 3.36 (m, 1H), 3.28 - 3.23 (m, 2H), 3.10 - 2.92 (m, 2H), 2.92 - 2.83 (m, 1H), 2.77 (t, 1H), 2.67 (t, 1H), 2.12 (s, 3H), 1.43 (d, 6H).
LC-MS (ESI): m/z = 488.2 $[M+H]^+$

**Example 143:**

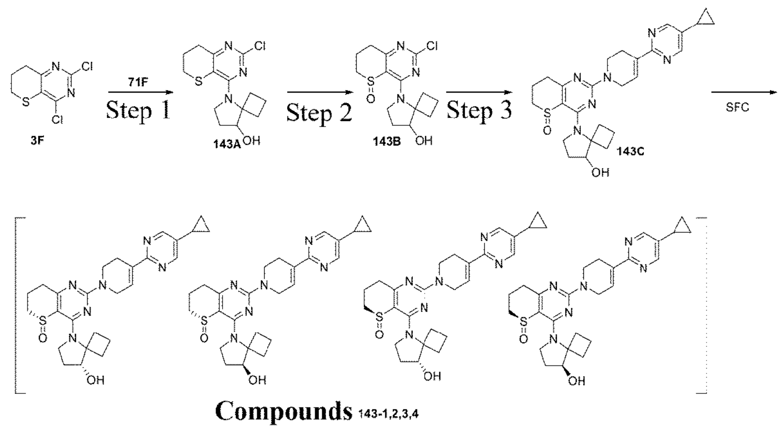


**Compounds** 143-1,2,3,4

Using **3F** and compound **71F** as raw materials, the target compound, racemate **143C,** was synthesized according to the operation of Example **114,** and the racemate was resolved by SFC to obtain compound **143-1** (25 mg), compound **143-2** (13 mg), compound **143-3** (26 mg), and compound **143-4** (12 mg).
SFC preparation method: instrument: Waters 150 Prep-SFC F, column: Chiralcel Cellulose-2 Column; mobile phase: A: CO2, B: 0.1% $NH_3$•$H_2O$ in IPA and ACN; gradient: 60% B gradient elution; flow rate: 100 mL/min, column temperature: 25°C; wavelength: 220 nm; cycle time: 8.6 min; sample preparation: sample concentration 10 mg/ml, acetonitrile solution injection: 1.5 ml each time. After separation, the fraction was dried by a rotary evaporator at a bath temperature of 35°C to obtain compound **143-1** (25.2 mg), compound **143-2** (13.7 mg), compound **143-3** (26.1 mg), and compound **143-4** (12.0 mg).
Compound **143-1,** retention time: 1.638 min; $^1$H NMR (400 MHz, $CDCl_3$) δ 8.54 (s, 2H), 7.21 (s, 1H), 5.27 (d, 1H), 4.49 (s, 2H), 3.97 - 4.18 (m, 5H), 3.45 - 3.52 (m, 1H), 3.28 (s, 2H), 3.04 - 3.11 (m, 1H), 2.83 - 2.91 (m, 1H), 2.65 - 2.75 (m, 4H), 2.19 - 2.28 (m, 1H), 1.69 - 1.95 (m, 6H), 1.55 - 1.64 (m, 1H), 1.01 - 1.06 (m, 2H), 0.81 - 0.85 (m, 2H).
LC-MS (ESI): m/z = 493.2 $[M+H]^+$
Compound **143-2,** retention time: 1.745 min; $^1$H NMR (400 MHz, $CDCl_3$) δ 8.54 (s, 2H), 7.21 (s, 1H), 5.27 (d, 1H), 4.49 (s, 2H), 3.97 - 4.18 (m, 5H), 3.45 - 3.52 (m, 1H), 3.28 (s, 2H), 3.04 - 3.11 (m, 1H), 2.83 - 2.91 (m, 1H), 2.65 - 2.75 (m, 4H), 2.19 - 2.28 (m, 1H), 1.69 - 1.95 (m, 6H), 1.55 - 1.64 (m, 1H), 1.01 - 1.06 (m, 2H), 0.81 - 0.85 (m, 2H).
LC-MS (ESI): m/z = 493.2 $[M+H]^+$
Compound **143-3,** retention time: 1.794 min; $^1$H NMR (400 MHz, $CDCl_3$) δ 8.54 (s, 2H), 7.21 (s, 1H), 5.27 (d, 1H), 4.49 (s, 2H), 3.97 - 4.18 (m, 5H), 3.45 - 3.52 (m, 1H), 3.28 (s, 2H), 3.04 - 3.11 (m, 1H), 2.83 - 2.91 (m, 1H), 2.65 - 2.75 (m, 4H), 2.19 - 2.28 (m, 1H), 1.69 - 1.95 (m, 6H), 1.55 - 1.64 (m, 1H), 1.01 - 1.06 (m, 2H), 0.81 - 0.85 (m, 2H).
LC-MS (ESI): m/z = 493.2 $[M+H]^+$
Compound **143-4,** retention time: 2.558 min; $^1$H NMR (400 MHz, $CDCl_3$) δ 8.54 (s, 2H), 7.21 (s, 1H), 5.27 (d, 1H),

4.49 (s, 2H), 3.97 - 4.18 (m, 5H), 3.45 - 3.52 (m, 1H), 3.28 (s, 2H), 3.04 - 3.11 (m, 1H), 2.83 - 2.91 (m, 1H), 2.65 - 2.75 (m, 4H), 2.19 - 2.28 (m, 1H), 1.69 - 1.95 (m, 6H), 1.55 - 1.64 (m, 1H), 1.01 - 1.06 (m, 2H), 0.81 - 0.85 (m, 2H).

LC-MS (ESI): m/z = 493.2 $[M+H]^+$

**Example 144:**

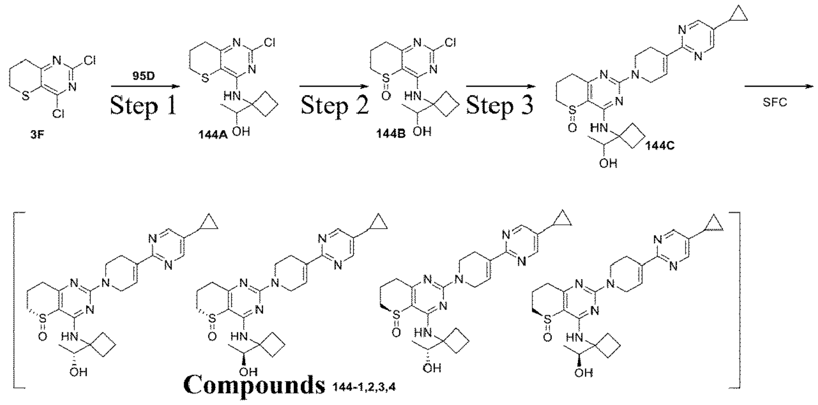


3F 144A 144B 144C

Compounds 144-1,2,3,4

Using **3F** and compound **95D** as raw materials, the target compound, racemate **144C,** was obtained according to the operation of Example **95,** and the racemate was resolved by SFC to obtain compound **144-1** (4.8 mg), compound **144-2** (5.2 mg), compound **144-3** (4.6 mg), and compound **144-4** (2.6 mg).

SFC preparation method: instrument: Waters 150 Prep-SFC F, column: Chiralcel Cellulose-2 Column; mobile phase: A: CO2, B: 0.1% $NH_3$•$H_2O$ in IPA and ACN; gradient: 50% B gradient elution; flow rate: 100 mL/min, column temperature: 25°C; wavelength: 220 nm; cycle time: 8.6 min; sample preparation: sample concentration 10 mg/ml, acetonitrile solution injection: 1.5 ml each time. After separation, the fraction was dried by a rotary evaporator at a bath temperature of 35°C to obtain compound **144-1** (4.8 mg), compound **144-2** (5.2 mg), compound **144-3** (4.6 mg), and compound **144-4** (2.6 mg).

Compound **144-1,** retention time: 1.328 min; $^1$H NMR (400 MHz, $CDCl_3$) δ 8.54 (s, 2H), 7.18 (s, 1H), 6.49 (d, 1H), 4.90 (s, 1H), 4.41 (s, 2H), 3.93 - 4.04 (m, 3H), 3.27 (s, 2H), 3.04 - 3.10 (m, 1H), 2.87 - 2.94 (m, 1H), 2.57 - 2.71 (m, 4H), 2.11 - 2.17 (m, 1H), 1.89 - 2.00 (m, 2H), 1.68 - 1.79 (m, 2H), 1.48 - 1.58 (m, 2H), 1.21 (s, 3H), 1.00 1.05 (m, 2H), 0.81 - 0.85 (m, 2H).

LC-MS (ESI): m/z = 481.2 $[M+H]^+$

Compound **144-2,** retention time: 1.425 min; $^1$H NMR (400 MHz, $CDCl_3$) δ 8.54 (s, 2H), 7.18 (s, 1H), 6.49 (d, 1H), 4.90 (s, 1H), 4.41 (s, 2H), 3.93 - 4.04 (m, 3H), 3.27 (s, 2H), 3.04 - 3.10 (m, 1H), 2.87 - 2.94 (m, 1H), 2.57 - 2.71 (m, 4H), 2.11 - 2.17 (m, 1H), 1.89 - 2.00 (m, 2H), 1.68 - 1.79 (m, 2H), 1.48 - 1.58 (m, 2H), 1.21 (s, 3H), 1.00 1.05 (m, 2H), 0.81 - 0.85 (m, 2H).

LC-MS (ESI): m/z = 481.2 $[M+H]^+$

Compound **144-3,** retention time: 2.012 min; $^1$H NMR (400 MHz, $CDCl_3$) δ 8.54 (s, 2H), 7.18 (s, 1H), 6.49 (d, 1H), 4.90 (s, 1H), 4.41 (s, 2H), 3.93 - 4.04 (m, 3H), 3.27 (s, 2H), 3.04 - 3.10 (m, 1H), 2.87 - 2.94 (m, 1H), 2.57 - 2.71 (m, 4H), 2.11 - 2.17 (m, 1H), 1.89 - 2.00 (m, 2H), 1.68 - 1.79 (m, 2H), 1.48 - 1.58 (m, 2H), 1.21 (s, 3H), 1.00 1.05 (m, 2H), 0.81 - 0.85 (m, 2H).

LC-MS (ESI): m/z = 481.2 $[M+H]^+$

Compound **144-4,** retention time: 2.302 min; $^1$H NMR (400 MHz, $CDCl_3$) δ 8.54 (s, 2H), 7.18 (s, 1H), 6.49 (d, 1H), 4.90 (s, 1H), 4.41 (s, 2H), 3.93 - 4.04 (m, 3H), 3.27 (s, 2H), 3.04 - 3.10 (m, 1H), 2.87 - 2.94 (m, 1H), 2.57 - 2.71 (m, 4H), 2.11 - 2.17 (m, 1H), 1.89 - 2.00 (m, 2H), 1.68 - 1.79 (m, 2H), 1.48 - 1.58 (m, 2H), 1.21 (s, 3H), 1.00 1.05 (m, 2H), 0.81 - 0.85 (m, 2H).

LC-MS (ESI): m/z = 481.2 $[M+H]^+$

**Example 145:**

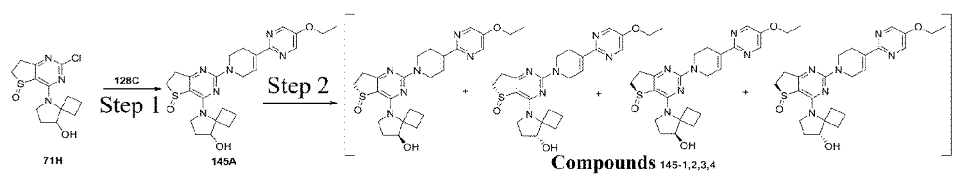


71H 145A

Compounds 145-1,2,3,4

Step 1: Under nitrogen protection, **71H** (0.78 g, 2.49 mmol), intermediate **128C** (0.90 g, 3.73 mmol), and N,N-diisopropylethylamine (0.96 g, 7.46 mmol) were successively dissolved in 1,4-dioxane (20 mL), heated to 85°C, and stirred for 14 hours. The reaction liquid was cooled to room temperature, then diluted by adding water (80 mL), and extracted three times with dichloromethane (50 mL). The organic phases were combined and dried over anhydrous sodium sulfate. After filtration, the filtrate was concentrated and then purified and separated by silica gel column chromatography (dichloromethane/methanol (v/v) = 90/10) to obtain racemic compound **145A** (1.20 g, yield: 96.07%).

LC-MS (ESI): m/z = 483.6 [M+H]+

Step 2: Compound **145A** (1.20 g, 2.49 mmol) was subjected to reverse phsae preparation to obtain P1 (500 mg) and P2 (450 mg), which were then respectively subjected to chiral resolution to obtain compound **145-1** (113.2 mg), compound **145-2** (91.6 mg), compound **145-3** (163.8 mg), and compound **145-4** (176.8 mg).

HPLC analysis method: instrument: SHIMADZU LC-2020AD, column: C18 Column; mobile phase: A: 0.1% TFA in $H_2O$, B: ACN; gradient: 20-80% B in A; flow rate: 1.2 mL/min, column temperature: 45°C, wavelength: 210 nm & 254 nm.

HPLC preparation method: instrument: SHIMADZU LC-20AP, column: XB-SiOH Column; mobile phase: A: hexane, B: EtOH; gradient: 15-55% B gradient elution; flow rate: 80 mL/min, column temperature: 25°C; wavelength: 220 nm; cycle time: 5.0 min, sample preparation: sample concentration 50 mg/ml, acetonitrile solution injection: 8.0 ml each time.

After separation, the fraction was dried by a rotary evaporator at a bath temperature of 35°C to obtain compound P1 and compound P2.

SFC analysis method: instrument: SHIMADZU LC-2020AD, column: Chiralcel IH Column; mobile phase: A: Hexane, B: 0.1% IPAm IPA and ACN; gradient: 30% B in A; flow rate: 1.0 mL/min, column temperature: 35°C, wavelength: 254 nm.

SFC preparation method: instrument: SHIMADZU LC-20AP, column: Chiralcel IH Column; mobile phase: A: hexane, B: 0.1% IPAm IPA and ACN; gradient: 67% B gradient elution; flow rate: 60 mL/min, column temperature: 25°C; wavelength: 220 nm & 254 nm; cycle time: 6.0 min; sample preparation: sample concentration 10 mg/ml, acetonitrile solution injection: 4.0 ml each time.

After separation, the fraction was dried by a rotary evaporator at a bath temperature of 35°C to obtain compound **145-1** (113.2 mg), compound **145-2** (91.6 mg), compound **145-3** (163.8 mg), and compound **145-4** (176.8 mg).

Compound **145-1:** retention time: 2.516 min; $^1$H NMR (400 MHz, $CDCl_3$) δ 8.36 (s, 2H), 7.14 - 7.12 (m, 1H), 4.65 - 4.55 (m, 2H), 4.21 - 4.12 (m, 6H), 3.76 - 3.62 (m, 3H), 3.48 - 3.41 (m, 1H), 3.18 - 3.07 (m, 3H), 2.82 (s, 2H), 2.27 - 2.22 (m, 1H), 2.08 - 2.07 (m, 2H), 1.99 - 1.91 (m, 1H), 1.85 - 1.80 (m, 2H), 1.72 - 1.67 (m, 1H), 1.48 - 1.45 (m, 3H).

LC-MS (ESI): m/z = 483.0 $[M+H]^+$

Compound **145-2:** retention time: 4.322 min; $^1$H NMR (400 MHz, $CDCl_3$) δ 8.36 (s, 2H), 7.14 - 7.12 (m, 1H), 4.65 - 4.55 (m, 2H), 4.21 - 4.11 (m, 6H), 3.76 - 3.62 (m, 3H), 3.48 - 3.41 (m, 1H), 3.18 - 3.07 (m, 3H), 2.82 (s, 2H), 2.27 - 2.22 (m, 1H), 2.08 - 2.07 (m, 2H), 1.99 - 1.91 (m, 1H), 1.85 - 1.80 (m, 2H), 1.72 - 1.67 (m, 1H), 1.48 - 1.45 (m, 3H).

LC-MS (ESI): m/z = 483.0 $[M+H]^+$

Compound **145-3:** retention time: 2.490 min; $^1$H NMR (400 MHz, $CDCl_3$) δ 8.36 (s, 2H), 7.14 - 7.12 (m, 1H), 4.65 - 4.55 (m, 2H), 4.21 - 4.12 (m, 6H), 3.76 - 3.62 (m, 3H), 3.48 - 3.41 (m, 1H), 3.18 - 3.07 (m, 3H), 2.82 (s, 2H), 2.27 - 2.22 (m, 1H), 2.08 - 2.06 (m, 2H), 1.99 - 1.91 (m, 1H), 1.85 - 1.80 (m, 2H), 1.72 - 1.67 (m, 1H), 1.48 - 1.45 (m, 3H).

LC-MS (ESI): m/z = 483.0 $[M+H]^+$

Compound **145-4:** retention time: 3.794 min; $^1$H NMR (400 MHz, $CDCl_3$) δ 8.36 (s, 2H), 7.14 - 7.12 (m, 1H), 4.65 - 4.55 (m, 2H), 4.21 - 4.12 (m, 6H), 3.76 - 3.62 (m, 3H), 3.48 - 3.41 (m, 1H), 3.18 - 3.07 (m, 3H), 2.82 (s, 2H), 2.27 - 2.21 (m, 1H), 2.08 - 2.07 (m, 2H), 1.99 - 1.91 (m, 1H), 1.85 - 1.80 (m, 2H), 1.72 - 1.67 (m, 1H), 1.48 - 1.45 (m, 3H).

LC-MS (ESI): m/z = 483.0 $[M+H]^+$

**Example 146:**

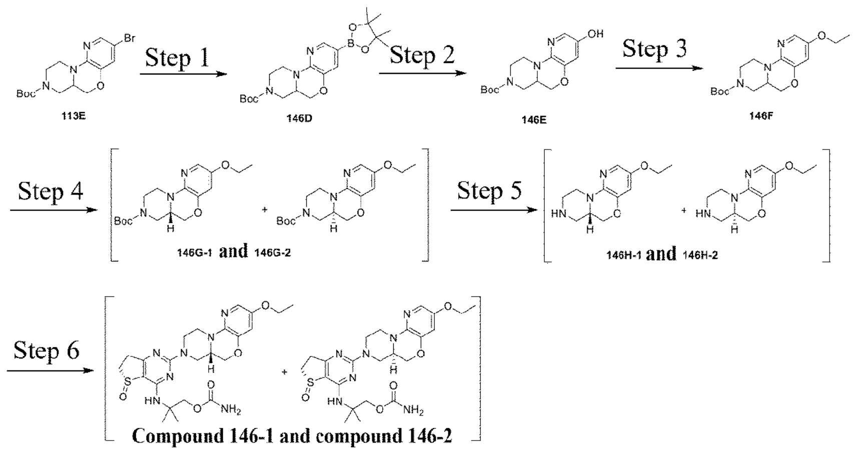


113E 146D 146E 146F

146G-1 and 146G-2

146H-1 and 146H-2

Compound 146-1 and compound 146-2

Step 1: Compound **113E** (5 g, 13.55 mmol) was dissolved in dry toluene (50 ml), and pinacol diboronate (0.88 g, 27.1 mmol), [1,1'-bis(diphenylphosphine)ferrocene]palladium dichloride dichloromethane complex (1.98 g, 2.71 mmol), and sodium acetate (2.22 g, 27.1 mmol) were added. After nitrogen displacement three times, the mixture was heated to 100°C and further reacted under stirring for 4 h. After the reaction was complete, the system was cooled to room temperature, and the reaction was quenched by adding water (50 mL). The system was extracted with ethyl acetate (50 mL × 3), and the organic phases were combined, washed with a saturated sodium chloride aqueous solution, and dried over anhydrous sodium sulfate. After filtration, the reaction liquid was concentrated under reduced pressure and then separated and purified by silica gel column chromatography (petroleum ether/ethyl acetate (v/v) = 100/0-70/30) to obtain the target compound **146D** (4.8 g, 85%).

LC-MS (ESI): m/z = 418.2 $[M+H]^+$。

Step 2: Compound **146D** (1.2 g, 2.87 mmol) was dissolved in acetone (20 ml), and hydrogen peroxide (0.24 ml, 8.61 mmol) and sodium bicarbonate (1.69 g, 20.09 mmol) were added and stirred at room temperature overnight. After the reaction was complete as detected by TLC, the reaction system was diluted by adding water and dichloromethane. The aqueous phase was extracted with dichloromethane. The organic phases were collected, combined, dried over anhydrous sodium sulfate, concentrated, and quickly separated and purified (petroleum ether/ethyl acetate = 5/1) to obtain compound **146E** (0.8 g, 91%).

LC-MS (ESI): m/z = 308.2 $[M+H]^+$.

Step 3: Compound **146E** (0.8 g, 2.61 mmol) was dissolved in acetone (10 ml), and cesium carbonate (2.55 g, 7.83 mmol) and iodoethane (0.82 g, 5.22 mmol) were added and stirred at room temperature for 3 h. After the reaction was complete, the reaction was quenched by adding water and extracted with ethyl acetate. The organic phases were collected, combined, dried over anhydrous sodium sulfate, concentrated, and quickly separated and purified (petroleum ether/ethyl acetate = 10/1) to obtain compound **146F** (0.8 g, 92%).

LC-MS (ESI): m/z = 336.2 $[M+H]^+$.

Step 4: Compound **146F** (0.8 g) was separated by SFC to obtain two isomers, i.e., compound **146G-1** (373 mg, retention time 5.86 min) and compound **146G-2** (340 mg, retention time 7.27 min).

Separation conditions of preparative chromatography: 1. instrument: Waters 150 AP-SFC; 2. chromatographic column: AD; 3. mobile phase system: A for $CO_2$; B for MeOH; 4. gradient: B 28%; and 5. flow rate: 40 mL/min

Using **146G-1** (100 mg, 0.3 mmol) as a raw material, compound **146-1** (105 mg, 68%) was obtained according to the synthesis method in Example **112.**

According to the above method, using **146G-2** as a raw material, the target compound **146-2** (100 mg, 64%) was obtained.

Compound **146-1:**

$^1H$ NMR (400 MHz, DMSO-*d6*) δ = 7.50 (d, 1H), 6.78 (d, 1H), 6.70 (s, 1H), 6.56 (s, 2H), 4.77 (t, 2H), 4.46 - 4.21 (m, 4H), 4.05 - 3.92 (m, 3H), 3.49 - 3.36 (m, 1H), 3.28 - 3.16 (m, 2H), 3.10 - 2.92 (m, 2H), 2.92 - 2.82 (m, 1H), 2.76 (t, 1H), 2.67 - 2.60 (m, 1H), 1.43 (d, 6H), 1.28 (t, 3H).

LC-MS (ESI): m/z = 518.2 $[M+H]^+$

Compound **146-2:**

$^1H$ NMR (400 MHz, DMSO-*d6*) δ = 7.50 (d, 1H), 6.78 (d, 1H), 6.71 (s, 1H), 6.56 (s, 2H), 4.77 (d, 2H), 4.49 - 4.19 (m, 4H), 4.05 - 3.89 (m, 3H), 3.51 - 3.38 (m, 1H), 3.19 (t, 2H), 3.11 - 2.92 (m, 2H), 2.91 - 2.86 (m, 1H), 2.75 (t, 1H), 2.63 (t, 1H),

1.43 (d, 6H), 1.28 (t, 3H).

LC-MS (ESI): m/z = 518.2 $[M+H]^+$

**Example 147:**

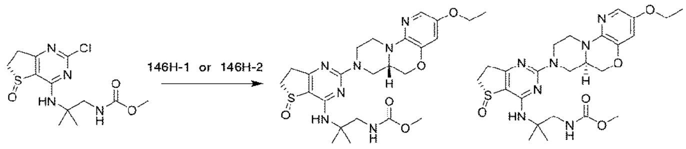


139B

**Compound 147-1 and compound 147-2**

Step 1: Using **139B** (100 mg, 0.3 mmol) and **146H-1** as raw materials, compound **147-1** (100 mg, 63%) was obtained according to the synthesis method in Example **112.**

Using **139B** (100 mg, 0.3 mmol) and **146H-2** as raw materials, the target compound **147-2** (101 mg, 63%) was obtained according to the synthesis method in Example 112.

Compound **147-1:**

$^1$H NMR (400 MHz, DMSO-*d6*) δ = 7.50 (d, 1H), 7.43 (t, 1H), 6.78 (d, 1H), 6.72 (s, 1H), 4.80 (d, 2H), 4.44 (d, 1H), 4.35 (d, 1H), 4.06 - 3.92 (m, 3H), 3.57 (s, 3H), 3.48 - 3.38 (m, 3H), 3.26 - 3.21 (m, 2H), 3.04 (t, 1H), 3.00 - 2.91 (m, 1H), 2.91 - 2.82 (m, 1H), 2.75 (t, 1H), 2.67 - 2.61 (m, 1H), 1.47 - 1.34 (m, 6H), 1.28 (t, 3H).

LC-MS (ESI): m/z = 532.2 $[M+H]^+$

Compound **147-2:**

$^1$H NMR (400 MHz, DMSO-*d6*) δ = 7.50 (d, 1H), 7.43 (t, 1H), 6.78 (d, 1H), 6.72 (s, 1H), 4.80 (d, 2H), 4.45 (d, 1H), 4.34 (d, 1H), 4.06 - 3.90 (m, 3H), 3.57 (s, 3H), 3.44 - 3.38 (m, 3H), 3.27 - 3.15 (m, 2H), 3.12 - 2.92 (m, 2H), 2.91 - 2.82 (m, 1H), 2.75 (t, 1H), 2.64 (t, 1H), 1.40 (d, 6H), 1.28 (t, 3H).

LC-MS (ESI): m/z = 532.2 $[M+H]^+$

**Example 148**

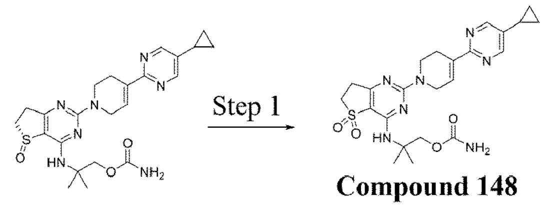


84

**Compound 148**

Step 1: Compound **84** (500 mg, 1.03 mmol) was dissolved in dichloromethane (15 mL), and m-chloroperoxybenzoic acid (210 m g, 1.24 mmol) was added and reacted at room temperature for 16 h. The system was then quenched by adding a saturated sodium thiosulfate solution. The aqueous phase was extracted with dichloromethane (20 mL × 3), and the organic phases were combined and dried over anhydrous sodium sulfate. After filtration, the filtrate was concentrated, and the crude product was purified by column chromatography (dichloromethane:methanol = 10:1 (v/v)) to obtain the target compound **148** (190 mg, yield: 47%).

LC-MS (ESI): m/z = 500.1 $[M+1]^+$.

$^1$H NMR (400 MHz, DMSO-*d6*) δ 8.55 (s, 2H), 7.19 (t, 1H), 6.54 (s, 2H), 5.46 (s, 1H), 4.48 (s, 2H), 4.27 (s, 2H), 4.01 (t, 2H), 3.49 (t, 2H), 3.09 (t, 2H), 2.68 (d, 2H), 2.01 - 1.83 (m, 1H), 1.47 (s, 6H), 1.11 - 0.97 (m, 2H), 0.92 - 0.76 (m, 2H).

**Biological test:**

1. Effect of compounds on PDE4B2 activity

The effect of compounds on PDE4B2 activity was detected using a fluorescence polarization kit (BPS Bioscience, Catalog #60343). According to the instructions of the kit, FAM-Cyclic-3',5'-AMP at a final concentration of 0.1 μM, 1 ng/well of PDE4B2 (for a negative control, a PDE buffer was added), and gradient-diluted compounds (for a positive control well, a PDE buffer containing 10% of DMSO was added) were added to each well, fully mixed, and then reacted at room temperature for 1 h. The Binding Agent was diluted with the Binding Agent Diluent (cAMP) at a ratio of 1:100 for later use, the Binding Agent dilution was taken and added to the test plate at 50 μl/well and incubated at room temperature with slow shaking for 20 minutes. After the incubation was complete, Envision was used for FP detection, with Excitation at 480 nm and Emission at 535 nm. FP was usually represented by mP value.

$$mP = \left(\frac{I_{//} - G(I_\perp)}{I_{//} + G(I_\perp)}\right) x\ 1000$$

$I_{//}$ (S535): fluorescence intensity in parallel direction
$I_\perp$ (P535): fluorescence intensity in perpendicular direction
G: G factor = 1

Calculation of inhibition rate (% Inhibition):

$$\%\ \text{Inhibition} = [1 - (mP_{(sample)} - mP_{(negative\ control)}) / (mP_{(positive\ control)} - mP_{(negative\ control)})] \times 100\%$$

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by GraphPad Prism 8 software.

The compounds of the present disclosure had an $IC_{50}$ value < 300 nM against PDE4B2; preferably, some compounds had an $IC_{50}$ value < 100 nM; more preferably, some compounds had an $IC_{50}$ value < 50 nM; and further preferably, some compounds had an $IC_{50}$ value < 10 nM.

The compounds of the present disclosure had an $IC_{50}$ value of less than 300 nM against PDE4B2, preferably less than 100 nM, more preferably less than 50 nM, and further preferably less than 10 nM. The $IC_{50}$ values of some specific compounds are as shown in Table 1 below, wherein A < 10 nM, 10 nM ≤ B < 50 nM, and 50 nM ≤ C < 100 nM.

Table 1. PDE4B2 activity

| Compound | PDE4B2 $IC_{50}$ (nM) | Compound | PDE4B2 $IC_{50}$ (nM) |
|---|---|---|---|
| Compound 3 | B | Compound 98 | A |
| Compound 12-2 | A | Compound 100 | A |
| Compound 13-2 | A | Compound 101 | A |
| Compound 14-1 | B | Compound 102-1 | B |
| Compound 15-2 | A | Compound 102-2 | A |
| Compound 16-2 | B | Compound 103-2 | A |
| Compound 17-2 | A | Compound 104 | B |
| Compound 19-1 | A | Compound 105 | A |
| Compound 20 | A | Compound 106 | B |
| Compound 23 | A | Compound 107 | B |
| Compound 24-2 | A | Compound 108 | B |
| Compound 26 | B | Compound 109-2 | B |

(continued)

| Compound | PDE4B2 $IC_{50}$ (nM) | Compound | PDE4B2 $IC_{50}$ (nM) |
|---|---|---|---|
| Compound 30 | A | Compound 109-4 | A |
| Compound 31 | B | Compound 110-1 | A |
| Compound 32-2 | A | Compound 110-2 | A |
| Compound 33-2 | B | Compound 111-2 | A |
| Compound 34 | A | Compound 111-4 | A |
| Compound 35 | A | Compound 112-1 | A |
| Compound 36-1 | A | Compound 112-2 | B |
| Compound 36-2 | B | Compound 113-2 | B |
| Compound 39 | A | Compound 114-1 | B |
| Compound 41 | A | Compound 115-1 | B |
| Compound 43 | A | Compound 116 | B |
| Compound 44 | A | Compound 117 | B |
| Compound 45-1 | A | Compound 118 | B |
| Compound 45-2 | A | Compound 119 | B |
| Compound 46-1 | A | Compound 121-1 | A |
| Compound 46-2 | A | Compound 121-2 | B |
| Compound 47 | A | Compound 122 | B |
| Compound 48 | A | Compound 123-1 | A |
| Compound 49 | A | Compound 123-2 | A |
| Compound 50 | A | Compound 124 | B |
| Compound 51 | A | Compound 125 | B |
| Compound 52 | A | Compound 126 | A |
| Compound 54 | C | Compound 127-1 | B |
| Compound 55 | C | Compound 127-2 | B |
| Compound 56 | A | Compound 128 | A |
| Compound 57 | A | Compound 129 | A |
| Compound 58 | A | Compound 130 | B |
| Compound 60-2 | B | Compound 131-1 | B |
| Compound 63 | A | Compound 131-2 | B |
| Compound 64 | A | Compound 132 | B |
| Compound 65 | A | Compound 133-1 | A |
| Compound 66 | A | Compound 133-2 | A |
| Compound 69 | A | Compound 134 | A |
| Compound 70 | A | Compound 135 | A |
| Compound 71-3 | B | Compound 136 | A |
| Compound 71-4 | A | Compound 137 | B |
| Compound 72-3 | A | Compound 138 | B |
| Compound 72-4 | A | Compound 139-1 | A |
| Compound 73-1 | A | Compound 139-2 | B |

(continued)

| Compound | PDE4B2 $IC_{50}$ (nM) | Compound | PDE4B2 $IC_{50}$ (nM) |
|---|---|---|---|
| Compound 73-2 | A | Compound 140-1 | B |
| Compound 74-2 | A | Compound 140-2 | B |
| Compound 75-1 | B | Compound 141-1 | B |
| Compound 76-1 | A | Compound 141-2 | B |
| Compound 77-1 | A | Compound 142-1 | A |
| Compound 77-2 | B | Compound 142-2 | B |
| Compound 77-3 | A | Compound 143-3 | A |
| Compound 78 | B | Compound 143-4 | A |
| Compound 79 | A | Compound 144-1 | B |
| Compound 80-2 | B | Compound 144-2 | A |
| Compound 81-1 | A | Compound 144-4 | A |
| Compound 82-2 | A | Compound 145-1 | A |
| Compound 83 | A | Compound 145-4 | A |
| Compound 84 | A | Compound 146-1 | B |
| Compound 85-1 | A | Compound 146-2 | B |
| Compound 88 | A | Compound 147-1 | B |
| Compound 89-2 | B | Compound 147-2 | B |
| Compound 90 | B | | |
| Compound 91-1 | A | | |
| Compound 92-1 | A | | |
| Compound 92-2 | A | | |
| Compound 93-1 | A | | |
| Compound 94-1 | A | | |
| Compound 94-2 | A | | |
| Compound 95-1 | A | | |
| Compound 95-2 | A | | |
| Compound 95-3 | A | | |
| Compound 95-4 | A | | |
| Compound 96-1 | A | | |
| Compound 96-2 | A | | |
| Compound 96-3 | A | | |
| Compound 96-4 | B | | |
| Compound 97 | A | | |

2. Effect of compounds on PDE4D3 activity

The effect of compounds on PDE4D3 activity was detected using a fluorescence polarization kit (BPS Bioscience, Catalog #60346). According to the instructions of the kit, FAM-Cyclic-3',5'-AMP at a final concentration of 0.1 μM, 0.05 ng/well of PDE4D3 (for a negative control, a PDE buffer was added), and gradient-diluted compounds (for a positive control well, a PDE buffer containing 10% of DMSO was added) were added to each well, fully mixed, and then reacted at room temperature for 1 h. The Binding Agent was diluted with the Binding Agent Diluent (cAMP) at a ratio of 1:100 for later use, the Binding Agent dilution was taken and added to the test plate at 100 μl/well and incubated at room

temperature with slow shaking for 1 h. After the incubation was complete, Envision was used for FP detection, with Excitation at 480 nm and Emission at 535 nm. FP was usually represented by mP value.

$$mP = \left(\frac{I_{//} - G(I_{\perp})}{I_{//} + G(I_{\perp})}\right) x\ 1000$$

$I_{//}$ (S535): fluorescence intensity in parallel direction
$I_{\perp}$ (P535): fluorescence intensity in perpendicular direction
G: G factor = 1

Calculation of inhibition rate (% Inhibition):

$$\% \text{ Inhibition} = [1 - (mP_{(sample)} - mP_{(negative\ control)}) / (mP_{(positive\ control)} - mP_{(negative\ control)})] \times 100\%$$

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by GraphPad Prism 8 software.

3. Effect of compounds on PDE4B1 activity

The effect of compounds on PDE4B1 activity was detected using a fluorescence polarization kit (IMAP FP IPP Explorer KIT, Molecular Device, Cat# R8124). The IMAP Reaction Buffer containing 0.1% BSA (5×) in the kit was diluted into a 1-fold reaction buffer containing 1 mM DTT. 0.12 nM PDE4B1 (BPS, Cat#60041) was added to the 1-fold reaction buffer to form a 2-fold enzyme solution. 10 μL of the 2-fold enzyme solution was added to wells of a 384-well reaction plate. For the negative control well, 10 μL of the 1-fold reaction buffer was used instead of the enzyme solution. Centrifugation at 1000 rpm for 1 minute and incubation at room temperature for 15 minutes were carried out. 0.2 uM FAM-labeled cAMP (FAM-cAMP, Molecular Device, Cat# R7506) was added to the 1-fold reaction buffer to form a 2-fold substrate solution. 10 μL of the 2-fold substrate solution was added to each well of the 384-well reaction plate. Centrifugation at 1000 rpm for 1 minute was carried out. A reaction was carried out at room temperature for 20 minutes. IMAP Progressive Binding Buffer A (5×), IMAP Progressive Binding Buffer B (5×), and IMAP Progressive Binding Reagent (provided by IMAP FP IPP Explorer Kit) were prepared into a reaction stop solution according to the instructions for use. 60 μL of an 80/60-fold reaction stop solution was added to each well of the 384-well reaction plate to stop the reaction, and the reaction product was centrifuged at 1000 rpm for 1 minute. Incubation at room temperature in the dark for 60 minutes was carried out. The mP values (Ex480/Em535 (s), Em535 (p)) were read by a microplate reader (Envision).

FP was usually represented by mP value. mP value for FP measurement = 1000 * (S - G * P)/(S + G * P)

S: fluorescence intensity in parallel direction
P: fluorescence intensity in perpendicular direction
G: G factor = 1

Calculation of inhibition rate (% Inhibition):

$$\% \text{ Inhibition} = (mP_{(positive\ control)} - mP_{(sample)}) / (mP_{(positive\ control)} - mP_{(negative\ control)}) \times 100\%$$

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the IC50 value of each compound was calculated by XLFit excel add-in version 5.4.0.8 software.

$$\text{Fitting formula: } Y = \text{Bottom} + (\text{Top} - \text{Bottom}) / (1 + (IC_{50}/X)^{\wedge}\text{HillSlope})$$

The $IC_{50}$ values of the compounds of the present disclosure against PDE4B1 are less than 300 nM, and the $IC_{50}$ values of the compounds are preferably less than 100 nM, more preferably less than 50 nM, and further preferably less than 10 nM. The $IC_{50}$ values of some specific compounds are as shown in Table 3 below, wherein A < 10 nM, 10 nM ≤ B < 50 nM, and 50 nM ≤ C < 100 nM.

Table 3. PDE4B1 activity

| Compound | PDE4B1 $IC_{50}$ (nM) |
|---|---|
| Compound 3 | A |
| Compound 8-2 | A |
| Compound 9-2 | A |
| Compound 10-2 | B |
| Compound 11-1 | A |

4. Effect of compounds on PDE4D2 activity

The effect of compounds on PDE4D2 activity was detected using a fluorescence polarization kit (BPS Bioscience, Catalog #60345). During the reaction, 12.5 μL of 0.272 ng/well of an enzyme (final concentration: 0.068 ng/well) and 12.5 μL of the compound diluted in a gradient manner (DMSO concentration: 4%) were first pre-incubated at room temperature for 15 minutes, the same volume of the enzyme at the same concentration and 12.5 μL of PDE buffer containing 4% DMSO were added to the positive control well, and 25 μL of PDE buffer containing 2% DMSO was added to the negative control well. After completion, 25 μL of 0.2 μM FAM-Cyclic-3',5'-AMP (final concentration: 0.1 μM) was added to each well, fully mixed, and then incubated at room temperature with slow shaking for 30 minutes. The Binding Agent was diluted with the Binding Agent Diluent (cAMP) at a ratio of 1:100 for later use, the Binding Agent dilution was taken and added to all the wells of the test plate at 100 μl/well and incubated at room temperature with slow shaking for 1 hour. After the incubation was complete, BMG LRBTECH microplate reader was used for FP detection, with Excitation at 485 nm and Emission at 520 nm. FP was usually represented by mP value.

$$mP = \left(\frac{I_{II} - G(I_{\perp})}{I_{II} + G(I_{\perp})}\right) x\ 1000$$

$I_{//}$ (S520): fluorescence intensity in parallel direction
$I_{\perp}$ (P520): fluorescence intensity in perpendicular direction
G: G factor = 1

Calculation of inhibition rate (% Inhibition):

$$\%\ \text{Inhibition} = [1 - (mP_{(sample)} - mP_{(negative\ control)}) / (mP_{(positive\ control)} - mP_{(negative\ control)})] \times 100\%$$

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the IC50 value of each compound was calculated by GraphPad Prism 8 software.

The $IC_{50}$ values of some specific compounds of the present disclosure are as shown in Table 4 below, wherein A <

10 nM, 10 nM ≤ B < 50 nM, and 50 nM ≤ C < 100 nM.

Table 4. PDE4D2 activity

| Compound | PDE4D2 $IC_{50}$ (nM) | Compound | PDE4D2 $IC_{50}$ (nM) |
|---|---|---|---|
| Compound 3 | A | Compound 96-2 | A |
| Compound 8-2 | A | Compound 96-3 | B |
| Compound 9-2 | A | Compound 96-4 | B |
| Compound 11-1 | B | Compound 97 | A |
| Compound 12-2 | B | Compound 98 | A |
| Compound 13-2 | B | Compound 100 | A |
| Compound 14-1 | B | Compound 101 | A |
| Compound 15-2 | B | Compound 102-1 | C |
| Compound 16-2 | B | Compound 102-2 | A |
| Compound 17-2 | B | Compound 103-2 | A |
| Compound 19-1 | B | Compound 104 | B |
| Compound 23 | B | Compound 105 | A |
| Compound 24-2 | B | Compound 106 | B |
| Compound 26 | B | Compound 107 | B |
| Compound 30 | A | Compound 108 | B |
| Compound 31 | B | Compound 109-2 | C |
| Compound 32-2 | A | Compound 109-4 | A |
| Compound 34 | B | Compound 110-1 | A |
| Compound 35 | A | Compound 110-2 | B |
| Compound 36-1 | A | Compound 111-2 | A |
| Compound 36-2 | B | Compound 111-4 | A |
| Compound 39 | A | Compound 112-1 | B |
| Compound 41 | B | Compound 112-2 | C |
| Compound 43 | A | Compound 113-2 | C |
| Compound 44 | B | Compound 114-1 | B |
| Compound 45-1 | A | Compound 115-1 | C |
| Compound 45-2 | B | Compound 116 | C |
| Compound 46-1 | A | Compound 117 | B |
| Compound 46-2 | B | Compound 118 | B |
| Compound 47 | A | Compound 119 | B |
| Compound 48 | A | Compound 121-1 | B |
| Compound 49 | A | Compound 121-2 | B |
| Compound 50 | A | Compound 122 | B |
| Compound 51 | A | Compound 123-1 | A |
| Compound 52 | A | Compound 123-2 | A |
| Compound 56 | A | Compound 124 | B |
| Compound 57 | B | Compound 125 | B |
| Compound 58 | B | Compound 126 | B |

(continued)

| Compound | PDE4D2 $IC_{50}$ (nM) | Compound | PDE4D2 $IC_{50}$ (nM) |
|---|---|---|---|
| Compound 63 | A | Compound 127-1 | B |
| Compound 64 | A | Compound 127-2 | B |
| Compound 65 | A | Compound 128 | A |
| Compound 66 | A | Compound 129 | B |
| Compound 69 | A | Compound 130 | B |
| Compound 70 | A | Compound 131-1 | B |
| Compound 71-3 | B | Compound 131-2 | C |
| Compound 71-4 | A | Compound 132 | B |
| Compound 72-3 | B | Compound 133-1 | B |
| Compound 72-4 | A | Compound 133-2 | A |
| Compound 73-1 | A | Compound 134 | A |
| Compound 73-2 | B | Compound 135 | A |
| Compound 74-2 | A | Compound 136 | A |
| Compound 75-1 | B | Compound 137 | B |
| Compound 76-1 | B | Compound 138 | B |
| Compound 77-1 | A | Compound 139-1 | A |
| Compound 77-3 | A | Compound 139-2 | B |
| Compound 78 | B | Compound 140-1 | B |
| Compound 79 | A | Compound 140-2 | C |
| Compound 80-2 | B | Compound 141-1 | B |
| Compound 81-1 | A | Compound 141-2 | C |
| Compound 82-2 | B | Compound 142-1 | B |
| Compound 83 | B | Compound 142-2 | B |
| Compound 84 | A | Compound 143-3 | A |
| Compound 85-1 | A | Compound 143-4 | A |
| Compound 88 | A | Compound 144-1 | B |
| Compound 89-2 | B | Compound 144-2 | A |
| Compound 90 | B | Compound 144-4 | A |
| Compound 91-1 | A | Compound 145-1 | A |
| Compound 92-1 | A | Compound 145-4 | A |
| Compound 92-2 | B | Compound 146-1 | A |
| Compound 93-1 | A | Compound 146-2 | B |
| Compound 94-1 | A | Compound 147-1 | C |
| Compound 94-2 | B | Compound 147-2 | C |
| Compound 95-1 | A | | |
| Compound 95-2 | A | | |
| Compound 95-3 | A | | |
| Compound 95-4 | A | | |
| Compound 96-1 | B | | |

**5.** Detection of inhibitory activity of compounds on the release of tumor necrosis factor-a (TNF-α) from human peripheral blood mononuclear cells induced by lipopolysaccharide (LPS) *in vitro*

Normal human peripheral blood which was anticoagulated (citric acid anticoagulation) was collected, and hPBMCs were prepared by Ficoll-Paque PLUS (Cytiva, Cat#17144002, density 1.077 g/mL). The concentration of hPBMC cells was adjusted to 0.25 x $10^6$ cells/mL with RPMI1640 medium and inoculated into a 96-well plate in an amount of 50000 cells per well. Subsequently, different concentrations of drugs were added for pre-incubation for 1 h (the DMSO final concentration was 0.1%, and the positive and negative control wells had the same volume of RPMI1640 containing 0.1% DMSO). After the pre-incubation was complete, 100 ng/mL LPS (SIGMA, L2630) was added to the compound and positive control wells, and the negative control well had the same volume of RPMI1640 containing 0.1% DMSO. Incubation was carried out for 4 h in an incubator at 37°C and 5% $CO_2$. The cell supernatant was collected, and the content of TNF-α in the supernatant sample was detected by human TNF-α Elisa quantitative test kit (Sino Biological, Cat#KIT10602). The $IC_{50}$ value was calculated using GraphPad Prism software. The $IC_{50}$ values of some specific compounds of the present disclosure are as shown in Table 5 below, wherein A < 10 nM, 10 nM ≤ B < 50 nM, and 50 nM ≤ C < 100 nM.

Table 5. Inhibitory activity of compounds on TNF-α induced by LPS *in vitro*

| Test compound | **Re** $IC_{50}$ (nM) | **Max inhibition** (%) |
|---|---|---|
| **Compound 9-2** | B | 69.47 |
| **Compound 32-2** | A | / |
| **Compound 35** | A | 85.08 |
| **Compound 36-1** | A | 73.52 |
| **Compound 37** | B | 78.28 |
| **Compound 39** | A | 71.11 |
| **Compound 41** | B | 69.8 |
| **Compound 43** | B | 80.31 |
| **Compound 44** | B | 77.27 |
| **Compound 45-1** | B | 71.28 |
| **Compound 46-1** | B | 75.89 |
| **Compound 46-2** | B | 73.36 |
| **Compound 47** | A | 80.45 |
| **Compound 48** | B | 73.86 |
| **Compound 49** | A | 73.46 |
| **Compound 50** | A | 72.14 |
| **Compound 51** | A | 69.04 |
| **Compound 52** | B | / |
| **Compound 56** | A | / |
| **Compound 60-2** | B | 72.31 |
| **Compound 63** | B | 72.71 |
| **Compound 64** | B | 79.89 |
| **Compound 65** | B | 68.15 |
| **Compound 66** | B | 67.07 |
| **Compound 69** | A | 70.87 |
| **Compound 70** | A | 55.38 |
| **Compound 71-2** | B | 58.51 |
| **Compound 71-3** | A | 61.86 |
| **Compound 71-4** | A | 57.63 |

(continued)

| Test compound | **Re** $IC_{50}$ (nM) | **Max inhibition** (%) |
|---|---|---|
| **Compound 72-3** | A | 65.66 |
| **Compound 72-4** | A | 62.45 |
| **Compound 73-1** | A | 67.49 |
| **Compound 73-2** | A | 67.88 |
| **Compound 74-1** | A | 51.67 |
| **Compound 74-2** | A | 79.64 |
| **Compound 76-1** | B | 74.65 |
| **Compound 77-1** | A | 65.28 |
| **Compound 77-3** | A | 66.35 |
| **Compound 78** | B | 77.39 |
| **Compound 79** | B | 65.52 |
| **Compound 80-2** | A | 75.43 |
| **Compound 81-1** | A | 74.08 |
| **Compound 82-2** | B | 73.50 |
| **Compound 83** | A | 71.03 |
| **Compound 84** | A | 68.04 |
| **Compound 85-1** | A | 71.38 |
| **Compound 88** | A | 74.9 |
| **Compound 90** | B | 69.53 |
| **Compound 91-1** | A | 72.87 |
| **Compound 92-1** | A | 69.72 |
| **Compound 93-1** | A | 71.65 |
| **Compound 94-1** | A | 76.10 |
| **Compound 94-2** | A | 67.98 |
| **Compound 95-2** | A | 70.67 |
| **Compound 95-3** | A | 81.97 |
| **Compound 96-1** | A | 72.83 |
| **Compound 96-2** | A | 81.63 |
| **Compound 96-4** | B | 70.23 |
| **Compound 97** | A | 76.25 |
| **Compound 98** | A | 72.70 |
| **Compound 100** | A | 79.21 |
| **Compound 101** | A | 79.98 |
| **Compound 102-1** | B | 99.73 |
| **Compound 102-2** | A | 87.54 |
| **Compound 104** | B | 78.75 |
| **Compound 105** | A | 83.96 |
| **Compound 106** | B | 98.36 |
| **Compound 109-4** | A | 85.74 |

(continued)

| Test compound | **Re** $IC_{50}$ (nM) | **Max inhibition** (%) |
|---|---|---|
| **Compound 110-1** | A | 88.09 |
| **Compound 110-2** | A | 74.07 |
| **Compound 111-2** | A | 75.39 |
| **Compound 111-4** | A | 78.77 |
| **Compound 112-1** | B | 83.76 |
| **Compound 117** | B | 85.97 |
| **Compound 119** | B | 84.64 |
| **Compound 121-1** | A | 78.80 |
| **Compound 139-1** | A | 92.69 |
| **Compound 140-1** | A | 93.38 |
| **Compound 140-2** | B | 89.58 |
| **Compound 144-4** | A | 94.06 |
| Re $IC_{50}$: relative $IC_{50}$. | | |

6. Pharmacokinetic test in mice

6.1 Experimental animals: Male ICR mice, 20-25 g, 6 mice/compound, purchased from Chengdu Ddossy Experimental Animals Co., Ltd.

6.2 Experiment design: On the day of the experiment, 6 ICR mice were randomly grouped according to their body weights. The animals were fasted with water available for 12 to 14 h one day before the administration and were fed 4 h after the administration.

Table 6. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | **Compound 51** | 2.5 | 0.5 | 5 | Plasma | Intravenous administration |
| G2 | 3 | | 10 | 1 | 10 | Plasma | Intragastric administration |
| Note: Vehicle I for intravenous administration: 5% DMA + 5% Solutol + 90% Saline; vehicle for intragastric administration: 0.5% MC (DMA: dimethylacetamide; Solutol: polyethylene glycol-15-hydroxystearate; Saline: physiological saline; MC: methylcellulose) | | | | | | | |

Before and after administration, 0.06 mL of blood was taken from the orbit under isoflurane anesthesia and placed in an EDTAK2 centrifuge tube for centrifugation at 5000 rpm at 4°C for 10 min, followed by plasma collection. The blood collection time points for the intravenous group and intragastric group were both 0 min, 5 min, 15 min, 30 min, 1 h, 2 h, 4 h, 6 h, 8 h, and 24 h. Before analysis and detection, all samples were stored at -80°C, and the samples were quantitatively analyzed by LC-MS/MS.

Table 7. Pharmacokinetic parameters of test compounds in mouse plasma

| Test compound | Mode of administration | CL (mL/min/kg) | $Vd_{ss}$ (L/kg) | $AUC_{0-t}$ (hr*ng/mL) | T1/2 (h) | F (%) |
|---|---|---|---|---|---|---|
| **Compound 51** | i.v. (2.5 mg/kg) | 7.81 ± 1.4 | 1.29 ± 0.039 | 5423 ± 926 | 2.82 ± 0.23 | - |
| | i.g. (10 mg/kg) | - | - | 30077 ± 5361 | 3.33 ± 0.58 | > 98 |
| -: not applicable. | | | | | | |

Conclusion: The compounds of the present disclosure, such as those in the examples, exhibited excellent pharmacokinetic properties in the PK test in mice.

## 7 Pharmacokinetic test in rats

**7.1 Experimental animals:** Male SD rats, about 220 g, 6-8 weeks old, 6 rats/compound, purchased from Chengdu Ddossy Experimental Animals Co., Ltd.

**7.2 Experiment design:** On the day of the experiment, 6 SD rats were randomly grouped according to their body weights. The animals were fasted with water available for 12 to 14 h one day before the administration and were fed 4 h after the administration.

Table 8. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 84 | 2.5 | 0.5 | 5 | Plasma | Intravenous administration |
| G2 | 3 | | 10 | 1 | 10 | Plasma | Intragastric administration |
| Note: Vehicle for intravenous administration: 10% DMA + 10% Solutol + 80% Saline; vehicle for intragastric administration: 0.5% MC<br>(DMA: dimethylacetamide; Solutol: polyethylene glycol-15-hydroxystearate; Saline: physiological saline; MC: methylcellulose) | | | | | | | |

Before and after the administration, 0.15 ml of blood was taken from the orbit under isoflurane anesthesia and placed in an EDTAK2 centrifuge tube for centrifugation at 5000 rpm at 4°C for 10 min, followed by plasma collection. The blood collection time points for the intravenous administration group and intragastric administration group were: 0, 5 min, 15 min, 30 min, 1 h, 2 h, 4 h, 6 h, 8 h and 24 h. Before analysis and detection, all samples were stored at -80°C, and the samples were quantitatively analyzed by LC-MS/MS.

Table 9. Pharmacokinetic parameters of test compounds in rat plasma

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 84 | i.v. (2.5 mg/kg) | 2.54 ± 0.32 | 0.324 ± 0.012 | 16531 ± 1991 | - |
| | i.g. (10 mg/kg) | - | - | 52179 ± 21323 | 78.9 ± 32 |
| -: not applicable. | | | | | |

Conclusion: The compounds of the present disclosure, such as those in the examples, exhibited excellent pharmacokinetic properties in the PK test in mice.

**8. Pharmacokinetic test in beagle dogs**

**8.1 Experimental animals:** Male beagle dogs, about 8-11 kg, 6 beagle dogs/compound, purchased from Beijing Marshall Biotechnology Co., Ltd.

**8.2 Experimental method:** On the day of the experiment, 6 beagle dogs were randomly grouped according to their body weights. The animals were fasted with water available for 12 to 14 h one day before the administration and were fed 4 h after the administration. Administration was carried out according to Table 10.

Table 10. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | **Compound 51** | 1 | 1 | 1 | Plasma | Intravenous administration |
| G2 | 3 | | 3 | 0.6 | 5 | Plasma | Intragastric administration |
| Note: Vehicle I for intravenous administration: 5% DMA + 5% Solutol + 90% Saline; vehicle for intragastric administration: 0.5% MC (DMA: dimethylacetamide; Solutol: polyethylene glycol-15-hydroxystearate; Saline: physiological saline; MC: Methylcellulose solution) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for the intravenous group and intragastric administration group were: 0 min, 5 min, 15 min, 30 min, 1h, 2 h, 4 h, 6 h, 8 h, 10 h, 12 h, 24 h, 48 h, and 72 h. Before analysis and detection, all samples were stored at -80°C, and the samples were quantitatively analyzed by LC-MS/MS.

Table 11. Pharmacokinetic parameters of test compounds in beagle dog plasma

| Test compound | Mode of administration | CL (mL/min/kg) | $Vd_{ss}$ (L/kg) | $AUC_{0-t}$ (hr*ng/mL) | T1/2 (h) | F (%) |
|---|---|---|---|---|---|---|
| **Compound 51** | i.v. (1 mg/kg) | 3.06 ± 0.18 | 3.08 ± 0.61 | 4991 ± 239 | 12.8 ± 3.8 | - |
| | i.g. (3 mg/kg) | - | - | 11211 ± 1713 | 16.1 ± 5.5 | 74.9 ± 11 |
| -: not applicable. | | | | | | |

**Conclusion:** The compounds of the present disclosure, such as those in the examples, exhibited excellent pharmacokinetic properties in the PK test in beagle dogs.

**9. Detection of inhibitory activity on TNF-α secretion in LPS-induced lung inflammation model of mice**

In mice, intratracheal administration of lipopolysaccharides (LPS, Sigma, L2880) might induce increased TNF-α concentration in lung tissue. In this experiment, a certain number of mice (BABL/c, male, weighing 18-22 g) were randomly divided into groups, with 8 mice in each group, and orally administered with a certain dose of the test compound (the test compound was suspended with 0.5% MC and prepared into a certain concentration, and the suspension was orally administered at a volume of 10 ml/kg body weight). After 0.5 h, based on 2.5 uL/g animal body weight, LPS was intratracheally administered at a dose of 0.8 mg/mL via a lung quantitative atomizing needle. 24 h later, the mice were intraperitoneally anesthetized with 20% urethane (10 mL/kg), and then sacrificed by cervical dislocation. Subsequently, lung tissue was taken and the lower part of the left lobe was fully homogenized, and the content of TNF-α in the supernatant of the homogenate was detected by Mouse TNFα ELISA Kit (RD, SMTA00B).

Inhibition rate of test compound on TNF-α level (% Inh) = (model group - test group)/ model group * 100%.

Results and conclusion: The results are shown in Figures 1 and 2.

## Claims

1. A compound represented by formula I, or a stereoisomer or pharmaceutically acceptable salt thereof,

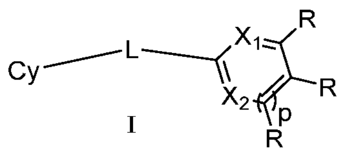

I

wherein when Cy is selected from Cy1, Cy2, Cy3, Cy8, and Cy10, L is $-(L_1)n-(L_2)m-$;

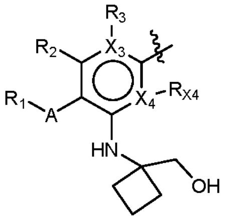
Cy1 ,
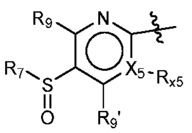
Cy2 ,
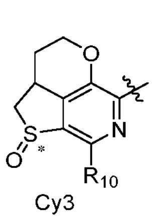
Cy3 ,
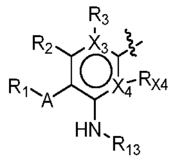
Cy8 , and
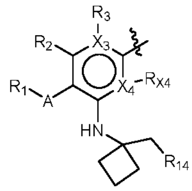
Cy10 ;

when Cy is selected from Cy4, Cy5, and Cy6, L is $-L_3-$;

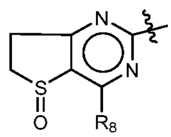
Cy4 ,
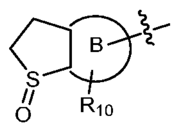
Cy5 ,
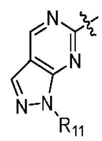
Cy6 , and
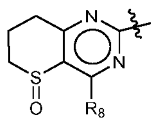
Cy11 ;

and
when Cy is selected from Cy7 and Cy9, L is $-L_4-$;

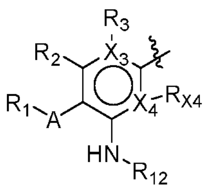
Cy7 and
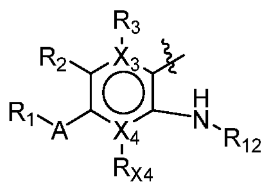
Cy9 ;

$L_1$ is $-NR_4-$, $-CR_5=N-$, $-CR_5=CR_5-$, or $-CR_5R_5-$;

$L_2$ is 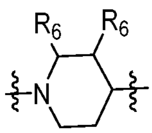 , 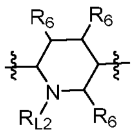 , 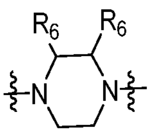 , 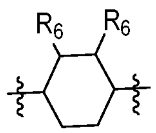 , 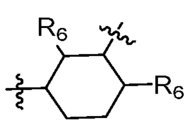 , 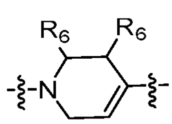 ,

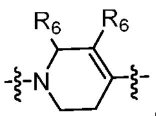, 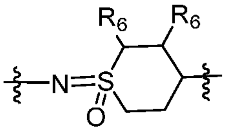, 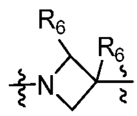,

$C_{1-4}$ alkylene, $C_{2-4}$ alkenylene, $C_{2-4}$ alkynylene, CO, O, $NR_{L2}$, a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 10-membered fused heterocycloalkyl containing 1-3 heteroatoms selected from N, S, and O, a 6- to 10-membered bridged heterocycloalkyl containing 1-3 heteroatoms selected from N, S, and O, or a 7- to 12-membered spiro heterocycloalkyl containing 1-3 heteroatoms selected from N, S, and O, wherein the alkylene, alkenylene, alkynylene, monocyclic heterocycloalkyl, fused heterocycloalkyl, bridged heterocycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$L_3$ is heterocycloalkylene or heterocycloalkylene-$(CH_2)_r$-, wherein the heterocycloalkylene is a 4-to 10-membered heterocycle containing 1-3 heteroatoms selected from N, S, and O and is optionally substituted with 1-3 $R_{L3}$;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 8-membered heterocycloalkyl containing 1-3 heteroatoms selected from N, S, and O;

$L_4$ is

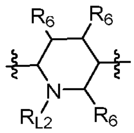, 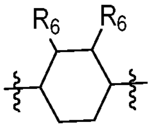, 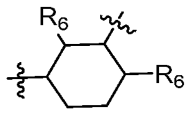, 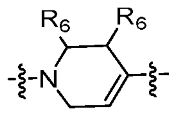, 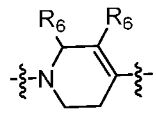,

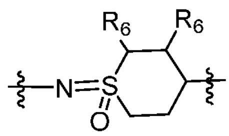, 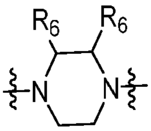,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$; A is -S(O)-, -C(O)-, -B(OH)-, -S-, -S(=N)-CN, or -C(=N)-CN;

$X_1$ and $X_2$ are independently $CR_{X1}$, O, S, or N;

$X_3$, $X_4$, and $X_5$ are independently C or N;

ring B is heteroaryl or heterocycloalkyl fused heteroaryl, wherein the heteroaryl is a 5-membered heteroaryl containing 1-3 heteroatoms selected from N, S, and O, and the heterocycloalkyl is a 5- to 6-membered heterocycloalkyl containing 1-3 heteroatoms selected from N, S, and O;

each $R_{X1}$ and R are independently H, halogen, $C_{1-4}$ alkyl, $C_{1-4}$ alkoxy, $-N(CH_3)_2$, $-NH(CH_3)$, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent, or $R_3$ and $R_2$, $R_3$ and $R_6$, $R_3$ and $R_4$, or $R_3$ and $R_5$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$, $R_{x4}$ and $R_5$, or $R_{x4}$ and $R_6$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_4$ is H, $C_{1-4}$ alkyl, $C_{1-4}$ haloalkyl, or $C_{3-6}$ cycloalkyl;

$R_5$ is H, $C_{1-4}$ alkyl, $C_{1-4}$ haloalkyl, $C_{1-4}$ alkoxy, -$NHC_{1-4}$ alkyl, or $C_{3-6}$ cycloalkyl;

$R_6$ is H, halogen, =O, CN, $C_{1-4}$ alkyl, or $C_{1-4}$ alkoxy;

$R_{x5}$ is $C_{1-4}$ alkyl, CN, OH, or absent;

$R_7$ is $C_{1-4}$ alkyl, $C_{1-4}$ haloalkyl, $C_{3-6}$ cycloalkyl, or a 5- to 8-membered heterocycloalkyl containing 1-3 heteroatoms selected from N, S, and O, or $R_7$ and $R_9$, together with the atom to which they are attached, form a 5- to 6-membered heterocycloalkyl containing 1-3 heteroatoms selected from N, S, and O, wherein the alkyl, haloalkyl, cycloalkyl, and heterocycloalkyl are optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form $C_{3-8}$ cycloalkyl, phenyl, a 5- to 10-membered heterocycloalkyl containing 1-3 heteroatoms selected from N, S, and O, or a 5- to 6-membered heteroaryl containing 1-3 heteroatoms selected from N, S, and O, wherein the cycloalkyl, phenyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;

$R_8$ is $R_{8'}$, -$OR_{8'}$, or -$(CH_2)_rR_{8'}$;

$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, -N=S(=O)($C_{1-4}$ alkyl)$_2$, or -N($C_{3-6}$ cycloalkyl)C(O)NH($C_{1-4}$ alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;

$R_9$ is H, $C_{1-4}$ alkyl, or $C_{1-4}$ haloalkyl;

each $R_{10}$ is independently $R_{8'}$, -$OR_{8'}$, -$NHR_{8'}$, or -$(CH_2)_rR_{8'}$;

$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;

$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, $C_{1-4}$ alkyl-CN, -CRaRb=N-O-$C_{1-4}$ alkyl, -C(NRaRb)=N-CN, -C($C_{1-4}$ alkyl)=N-CN, -C(NRaRb)=CRa-$NO_2$, -$C(CH_3)_2$-$CH_2$-O-C(O)$NH_2$, -$C(CH_3)_2$-$CH_2$-NH-C(O)O($C_{1-4}$ alkyl), -$C(CH_3)_2$-$CH_2$-O-C(O)$NHCH_3$, - $CH(CH_3)$-$CH_2$-O-C(O)$NH_2$, -$CH(CH_3)$-$CH_2$-NH-C(O)O($C_{1-4}$ alkyl), -$CH(CH_3)$-$CH_2$-O-C(O)$NHCH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from halogen, CN, =O, OH, -$SF_5$, $C_{1-4}$ alkyl, $C_{1-4}$ alkyl-CN, hydroxy $C_{1-4}$ alkyl, $C_{1-4}$ haloalkyl, -$(CH_2)_t$-O-C(O)$NH_2$, -$(CH_2)_t$-NRa-C(O)$NH_2$, -$(CH_2)_t$-NRa-C(=NH)$NH_2$, -$(CH_2)_t$-NRa-C(O)-O-($C_{1-4}$ alkyl), -$(CH_2)_t$-NRa-C($C_{1-4}$ alkyl)=N-CN, -$(CH_2)_t$-NRa-C(NRaRb)=N-CN, -$(CH_2)_t$-NRa-C(NRaRb)=CRa-$NO_2$, -$(CH_2)_t$-C(NRaRb)=N-CN, -$(CH_2)_t$-C($C_{1-4}$ alkyl)=N-CN, -$(CH_2)_t$-C($C_{1-4}$ alkyl)=N-O-($C_{1-4}$ alkyl), =N-O-$C_{1-4}$ alkyl, a 5- to 6-membered heteroaryl, -$(CH_2)_t$-O-$C_{1-4}$ alkyl, -$(CH_2)_t$-O-C(O)$NHCH_3$, -$(CH_2)_t$-O-$C_{1-2}$ haloalkyl, - $(CH_2)_t$-O-$C_{3-6}$ cycloalkyl, -$(CH_2)_t$-O-$C_{1-4}$ alkyl-O-$C_{3-6}$ cycloalkyl, -$(CH_2)_t$-COOH, -$(CH_2)_t$-NRaRb, -$(CH_2)_t$-C(O)NRaRb, and -$(CH_2)_t$-NRa-C(O)$CH_3$;

Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O;

or alternatively, Ra and Rb, together with the atom to which they are attached, form $C_{3-6}$ cycloalkyl;

$R_{13}$ is selected from a 7- to 11-membered spiro ring, a 4- to 10-membered fused ring, a 5- to 8-membered bridged heterocycle, a 4- to 6-membered monocyclic heterocycloalkyl containing $S(O)_2$ group, a 4-membered monocyclic

heterocycloalkyl containing 1-3 heteroatoms selected from N, S, and O, and a 7-membered monocyclic heterocycloalkyl containing 1-3 heteroatoms selected from N, S, and O, wherein the spiro ring, fused ring, bridged heterocycle, and monocyclic heterocycloalkyl are optionally substituted with 1-3 groups from halogen, CN, =O, OH, $C_{1-4}$ alkyl, hydroxy $C_{1-4}$ alkyl, and $C_{1-4}$ haloalkyl;

$R_{14}$ is selected from $-(CH_2)_t-O-C(O)NH_2$, $-(CH_2)_t-O-C(O)NHCH_3$, and a 5- to 6-membered monocyclic heterocycloalkyl containing 1-3 heteroatoms selected from N, S, and O, wherein the monocyclic heterocycloalkyl is optionally substituted with 1-3 groups from halogen, CN, =O, OH, $C_{1-4}$ alkyl, and $C_{1-4}$ haloalkyl;

n and m are independently 0, 1, 2, or 3;

r is selected from 1, 2, 3, or 4;

t is selected from 0, 1, 2, 3, or 4; and

p is selected from 0 or 1;

provided that

Cy1 is not

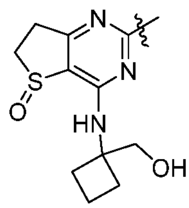

or

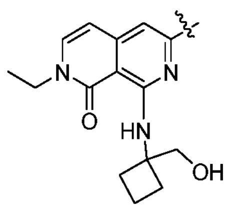

.

**2.** The compound or the stereoisomer or pharmaceutically acceptable salt thereof according to claim 1,

wherein ring B is pyrazolyl, imidazolyl, pyrrolyl, tetrahydropyrrolopyrrolyl, tetrahydropyrroloimidazolyl, or thienopyrrolyl;

$R_1$ is $C_{1-4}$ alkyl, or $C_{1-4}$ haloalkyl, or $R_1$ and $R_2$, together with the atom to which they are attached, form a 5- to 7-membered monocyclic heterocycloalkyl, a 6- to 8-membered fused heterocycloalkyl, a 7- to 9-membered spiro heterocycloalkyl, or a 5- to 6-membered heteroaryl containing 1-3 heteroatoms selected from N, S, B, and O, wherein the monocyclic heterocycloalkyl, fused heterocycloalkyl, spiro heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_2$ is H, $C_{1-4}$ alkyl, or $C_{1-4}$ haloalkyl;

$R_3$ is H, halogen, $C_{1-4}$ alkyl, CN, OH, or absent;

or $R_3$ and $R_2$, together with the atom to which they are attached, form a 5- to 6-membered heterocycloalkyl or a 5- to 6-membered heteroaryl containing 1-3 heteroatoms selected from N, S, and O, wherein the alkyl, cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

or $R_3$ and $R_6$, together with the atom to which they are attached, form $C_{5-7}$ cycloalkyl, a 5- to 6-membered heterocycloalkyl, or a 5- to 6-membered heteroaryl containing 1-3 heteroatoms selected from N, S, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_{x4}$ is H, halogen, $C_{1-4}$ alkyl, CN, OH, or absent, or $R_{x4}$ and $R_4$ or $R_{x4}$ and $R_5$, together with the atom to which they are attached, form a 5- to 6-membered heteroaryl containing 1-3 heteroatoms selected from N, S, and O, wherein the heteroaryl is optionally substituted with 1-3 groups selected from halogen, =O, CN, $C_{1-4}$ alkyl, $C_{1-4}$ alkoxy, OH, and $NH_2$;

$R_7$ is $C_{1-4}$ alkyl, or $C_{1-4}$ haloalkyl, or $R_7$ and $R_9$, together with the atom to which they are attached, form a 5- to 6-membered heterocycloalkyl containing 1-3 heteroatoms selected from N, S, and O, wherein the heterocycloalkyl is optionally substituted with 1-3 groups selected from halogen, CN, $C_{1-4}$ alkyl, $C_{1-4}$ alkoxy, OH, and $NH_2$;

at least one group of $R_{9'}$ and $R_7$ or $R_{9'}$ and $R_{x5}$, together with the atom to which they are attached, form a 5- to 7-membered monocyclic heterocycloalkyl, a 7- to 10-membered spiro heterocycloalkyl, or a 5- to 6-membered heteroaryl containing 1-3 heteroatoms selected from N, S, and O, wherein the monocyclic heterocycloalkyl, spiro heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; and

r is 1 or 2.

**3.** The compound or the stereoisomer or pharmaceutically acceptable salt thereof according to claim 2,

wherein each $L_2$ is independently selected from

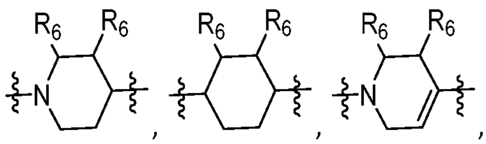

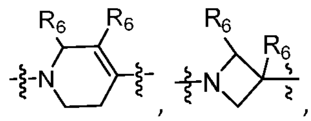

CO, O, $NR_{L2}$, $C_{1-4}$ alkylene, $C_{2-4}$ alkenylene, $C_{2-4}$ alkynylene,

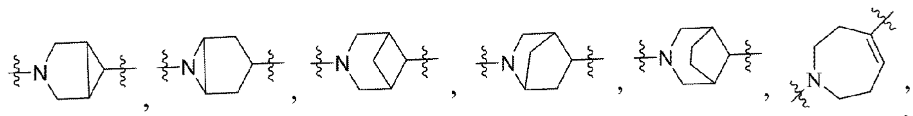

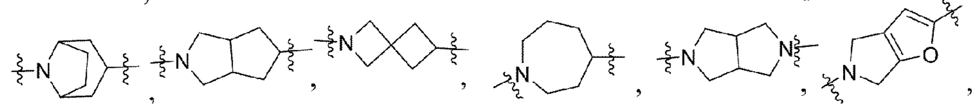

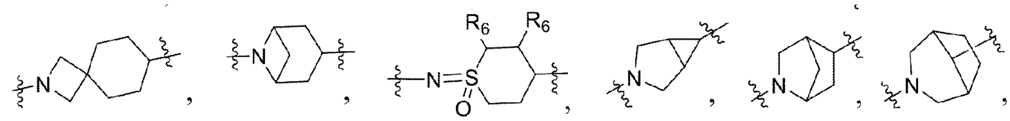

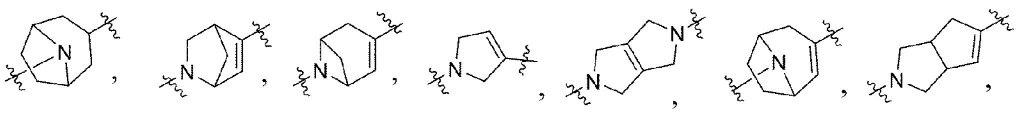

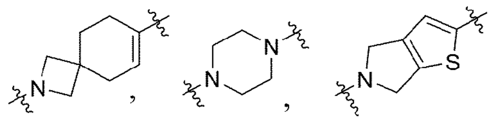

;

$R_{L2}$ is H, $C_{1-4}$ alkyl, or $C_{3-6}$ cycloalkyl;
$L_3$ is selected from

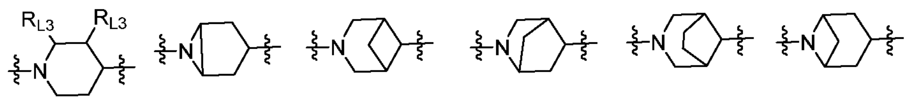

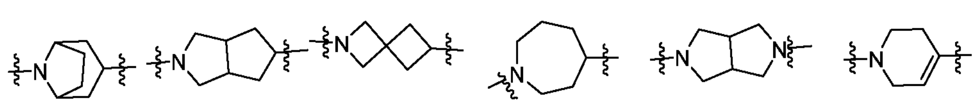

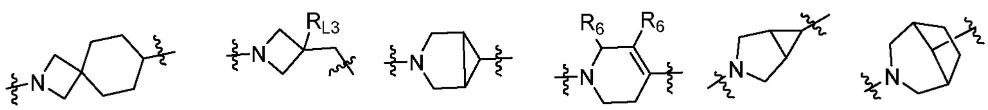

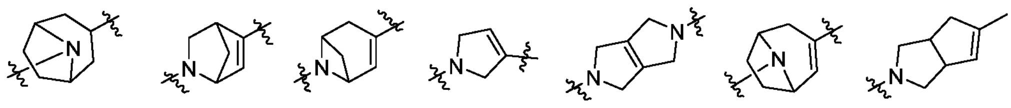

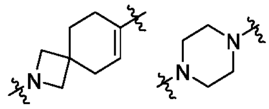

;

each $R_{L3}$ is independently selected from halogen, =O, CN, $C_{1-4}$ alkyl, or $C_{1-4}$ alkoxy, or $R_{L3}$ and $R_{X1}$, together with the atom to which they are attached, form $C_{3-6}$ cycloalkyl or a 5- to 6-membered heterocycloalkyl containing 1-3 heteroatoms selected from N, S, and O;

$L_4$ is selected from

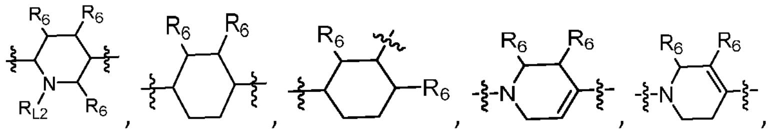

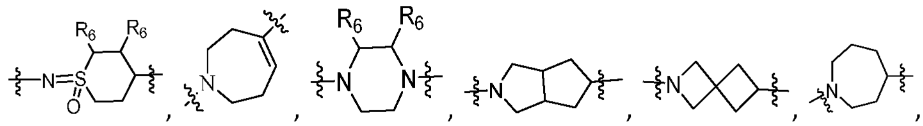

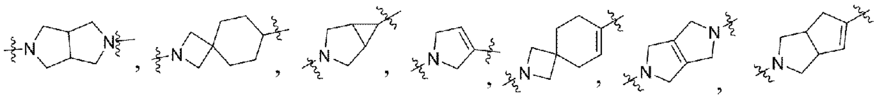

;

A is -S(O)-, -C(O)-, -B(OH)-, -S-, or -S(=N)-CN;

Cy5 is selected from

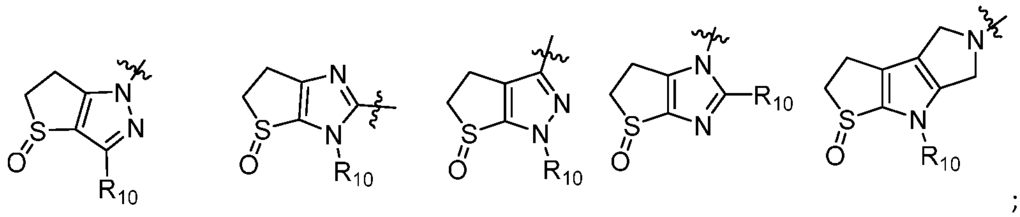

;

each $R_{X1}$ and R are independently H, halogen, $C_{1-4}$ alkyl, $C_{1-4}$ alkoxy, $-N(CH_3)_2$, $-NH(CH_3)$, $C_{2-4}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-6}$ cycloalkyl, or a 5- to 6-membered heterocycloalkyl containing 1-3 heteroatoms selected from N, S, O, and Si, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$;

$R_1$ is $C_{1-4}$ alkyl or $C_{1-4}$ haloalkyl;

or $R_1$ and $R_2$, together with the atom to which they are attached, form a ring selected from:

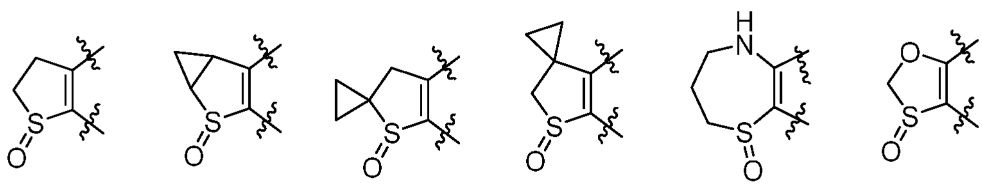

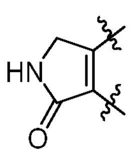 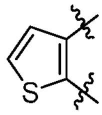 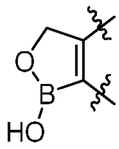 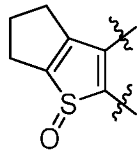 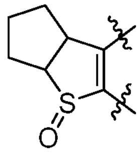 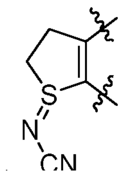

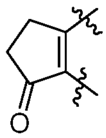 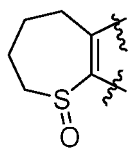 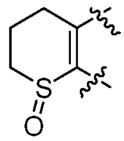 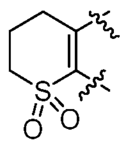 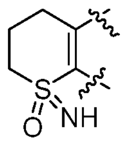 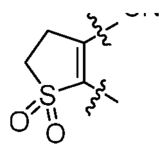 ,

optionally substituted with 1-3 groups selected from halogen, =O, CN, $C_{1-4}$ alkyl, $C_{1-4}$ alkoxy, OH, and $NH_2$;
$R_3$ is H, halogen, $C_{1-4}$ alkyl, CN, OH, or absent;
or $R_3$ and $R_2$, together with the atom to which they are attached, form a ring selected from:

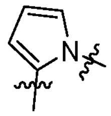 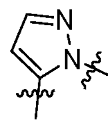 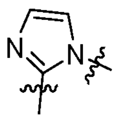 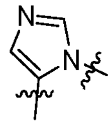 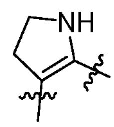 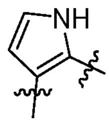 ,

optionally substituted with 1-3 groups selected from halogen, =O, CN, $C_{1-4}$ alkyl, $C_{1-4}$ alkoxy, OH, and $NH_2$;
or $R_3$ and $R_4$ or $R_3$ and $R_5$, together with the atom to which they are attached, form a 5- to 6-membered heterocycloalkyl or a 5- to 6-membered heteroaryl containing 1-3 heteroatoms selected from N, S, and O, or phenyl, wherein the heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;
or $R_3$ and $R_6$, together with the atom to which they are attached, form $C_{5-6}$ cycloalkyl, or a 5- to 6-membered heterocycloalkyl containing 1-3 heteroatoms selected from N, S, and O, optionally substituted with 1-3 groups selected from halogen, =O, CN, $C_{1-4}$ alkyl, $C_{1-4}$ alkoxy, OH, and $NH_2$;
$R_{x4}$ is H, halogen, $C_{1-4}$ alkyl, CN, OH, or absent; or $R_{x4}$ and $R_4$ or $R_{x4}$ and $R_5$, together with the atom to which they are attached, form imidazolyl, pyrazolyl, pyrrolyl, thienyl, or thiazolyl, optionally substituted with 1-3 groups selected from halogen, =O, CN, $C_{1-4}$ alkyl, $C_{1-4}$ alkoxy, OH, and $NH_2$;
$R_4$ is H, $C_{1-4}$ alkyl, or $C_{1-4}$ haloalkyl;
$R_5$ is H, $C_{1-4}$ alkyl, $C_{1-4}$ haloalkyl, $C_{1-4}$ alkoxy, or -NH$C_{1-4}$ alkyl;
$R_6$ is H, halogen, =O, CN, $C_{1-4}$ alkyl, or $C_{1-4}$ alkoxy;
$R_7$ is $C_{1-4}$ alkyl, or $C_{1-4}$ haloalkyl, or $R_7$ and $R_9$, together with the atom to which they are attached, form a ring selected from:

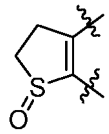 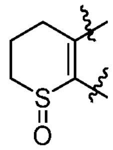 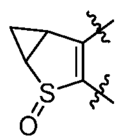 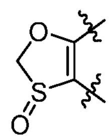 ,

wherein the ring is optionally substituted with 1-3 groups selected from halogen, CN, $C_{1-4}$ alkyl, $C_{1-4}$ alkoxy, OH, and $NH_2$;
or $R_{9'}$ and $R_7$, together with the atom to which they are attached, form a ring selected from:

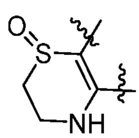 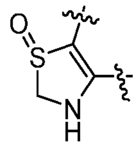 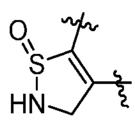 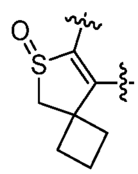 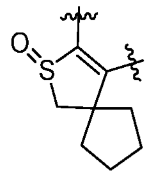 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$;
or $R_{9'}$ and $R_{x5}$, together with the atom to which they are attached, form

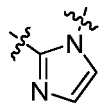 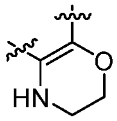 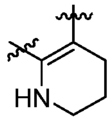 ,

optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, CN, or $R_{8'}$; provided that at least one group of $R_{9'}$ and $R_7$ or $R_{9'}$ and $R_{x5}$ form a ring;
$R_8$ is $R_{8'}$, $-OR_{8'}$, or $-(CH_2)_rR_{8'}$;
$R_{8'}$ is $C_{3-6}$ cycloalkyl, a 4- to 8-membered heterocycloalkyl containing 1-3 heteroatoms selected from N, S, O, Si, and P, a 5- to 6-membered heteroaryl containing 1-3 heteroatoms selected from N, S, and O, $-N=S(=O)(C_{1-4}$ alkyl$)_2$, or $-N(C_{3-6}$ cycloalkyl)C(O)NH($C_{1-4}$ alkyl), wherein the cycloalkyl, heterocycloalkyl, and heteroaryl are optionally substituted with 1-3 groups selected from $C_{1-4}$ alkyl, hydroxy $C_{1-4}$ alkyl, mercapto $C_{1-4}$ alkyl, halogen, halo $C_{1-4}$ alkyl, =O, OH, $NH_2$, and CN;
$R_9$ is H or $C_{1-4}$ alkyl;
each $R_{10}$ is independently $R_{8'}$, $-OR_{8'}$, $-NHR_{8'}$, or $-(CH_2)_rR_{8'}$;
$R_{11}$ is $C_{3-6}$ cycloalkyl optionally substituted with 1-3 groups from halogen, CN, =O, OH, $C_{1-4}$ alkyl, hydroxy $C_{1-4}$ alkyl, and $C_{1-4}$ haloalkyl;
$R_{12}$ is selected from cyclobutyl, cyclopentyl, cyclohexyl, phenyl, cyclopropyl, adamantyl, tetrahydropyranyl, piperidinyl, oxolanyl, oxanyl,

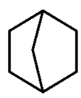 ,

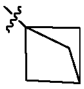 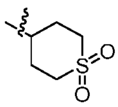 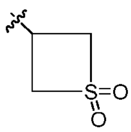 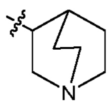 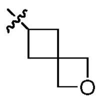 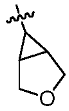 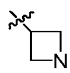 , 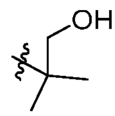 ,

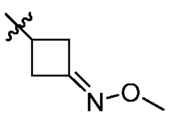 , 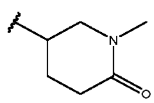 ,

$C_{1-4}$ alkyl-CN, $-C(NHC_{1-2}$ alkyl)=N-CN, $-C(C_{1-2}$ alkyl)=N-CN, $-C(NHC_{1-2}$ alkyl)=$CH-NO_2$, $-CH(CH_3)-CH_2-NH-C(O)O(CH_3)$, $-C(CH_3)_2-CH_2-NH-C(O)O(CH_3)$, $-CH(CH_3)-CH_2-O-C(O)NH_2$, $-C(CH_3)_2-CH_2-O-C(O)NH_2$, -C(O)-Ra, $-CH(CH_3)-CH_2-O-C(O)NHCH_3$, or $-C(CH_3)_2-CH_2-O-C(O)NHCH_3$; wherein the ring is optionally substituted with 1-3 groups selected from halogen, CN, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t-NH-C(O)NH_2$, $-(CH_2)_t-NH-C(=NH)NH_2$, $-(CH_2)_t-NH-C(O)-O-(C_{1-2}$ alkyl), $-(CH_2)_t-NH-C(C_{1-2}$ alkyl)=N-CN, $-(CH_2)_t-NH-C(NHC_{1-2}$ alkyl)=N-CN, $-(CH_2)_t-NH-C(NHC_{1-2}$ alkyl)=$CH-NO_2$, $-(CH_2)_t-C(NHC_{1-2}$ alkyl)=N-CN, $-(CH_2)_t-C(C_{1-2}$ alkyl)=N-CN, $-(CH_2)_t-C(C_{1-2}$ alkyl)=N-O-($C_{1-2}$ alkyl), =N-O-$C_{1-2}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, $-(CH_2)_t-O-C_{3-6}$ cycloalkyl, $-(CH_2)_t-O-C_{1-4}$ alkyl-O-$C_{3-6}$ cycloalkyl, $-(CH_2)_t-COOH$, $-(CH_2)_t-NRaRb$, $-(CH_2)_t-C(O)NRaRb$, and $-(CH_2)_t-NRa-C(O)CH_3$;
$R_{13}$ is selected from

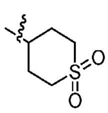 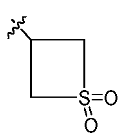 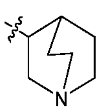 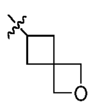 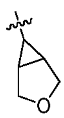 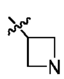 ;

wherein the ring is optionally substituted with 1-3 groups selected from halogen, CN, OH, $C_{1-4}$ alkyl, hydroxy $C_{1-4}$ alkyl, and $C_{1-4}$ haloalkyl;
n is 0, 1, 2, or 3;
m is 0, 1, or 2;
r is 1 or 2; and
t is selected from 0, 1, or 2.

**4.** The compound or the stereoisomer or pharmaceutically acceptable salt thereof according to claim 1 or 2, wherein

Cy1 is selected from

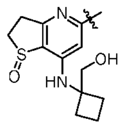 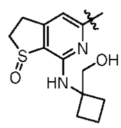 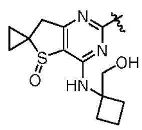 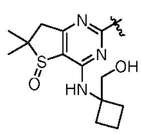 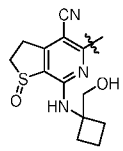 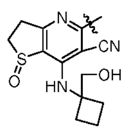 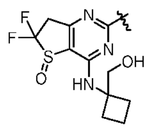

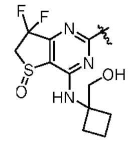 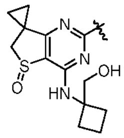 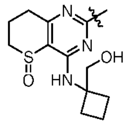 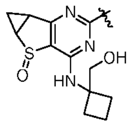 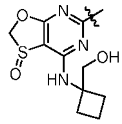 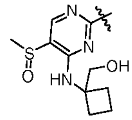

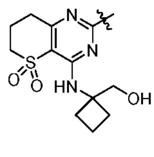 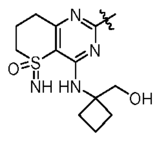 and 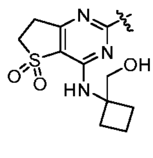 ;

Cy2 is selected from

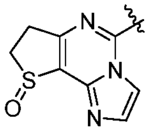 and 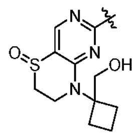 ;

Cy4 is selected from

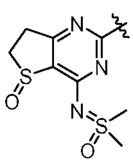 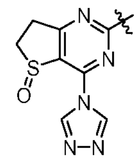 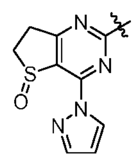 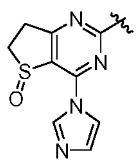 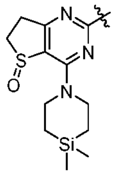

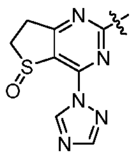 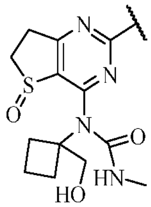 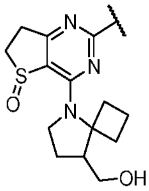 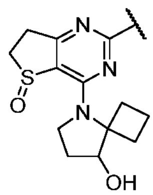 and 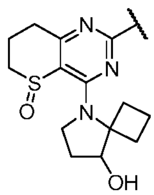 ;

Cy5 is

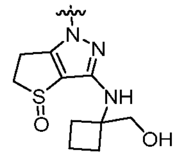 ;

Cy7 is selected from

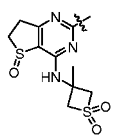 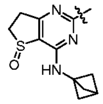 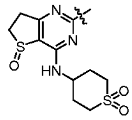 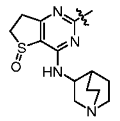 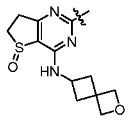 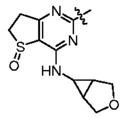 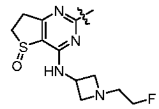

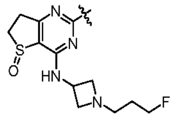

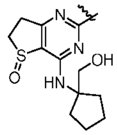 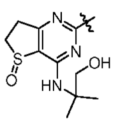 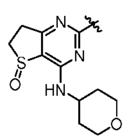 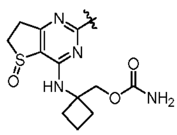 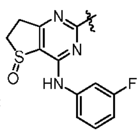 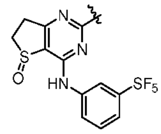

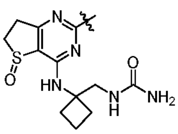 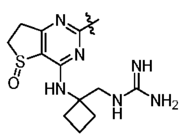 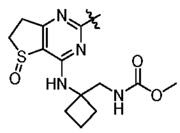 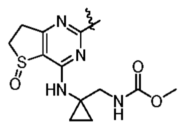

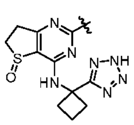 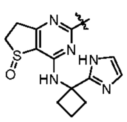 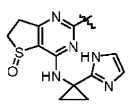 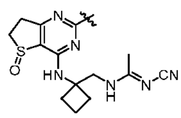 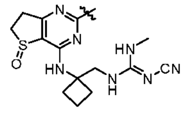 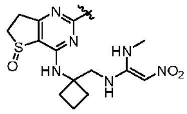

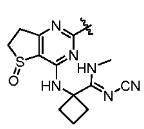 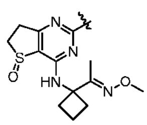 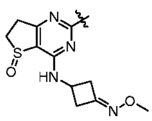 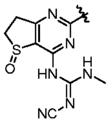 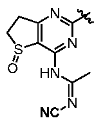 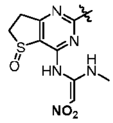 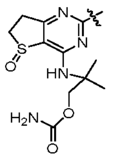

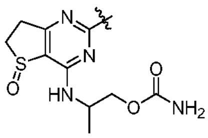 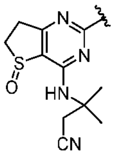 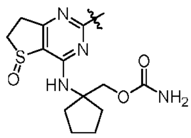 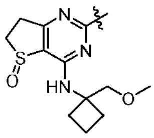 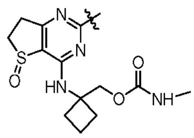 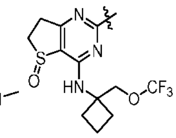

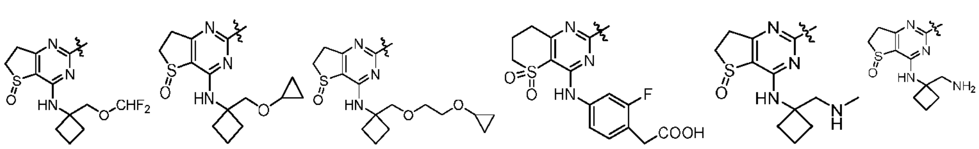

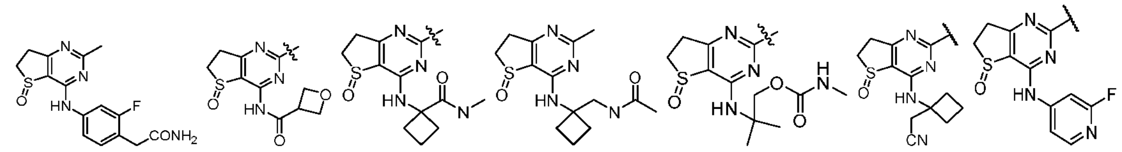

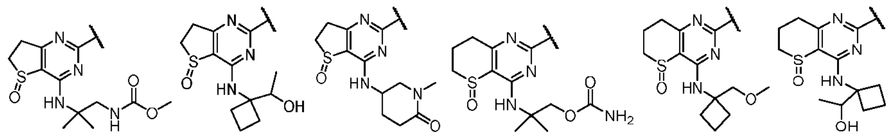

and

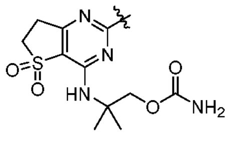;

and

Cy9 is

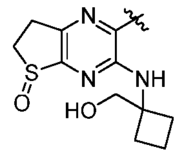.

**5.** The compound or the stereoisomer or pharmaceutically acceptable salt thereof according to any one of claims 1-4, wherein

-$(L_1)$n-$(L_2)$m- is selected from:

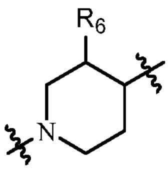, 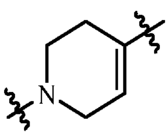, 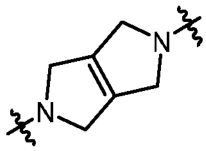, 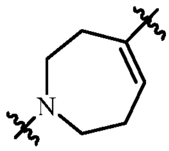,

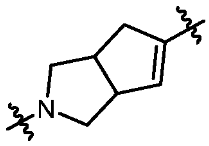, 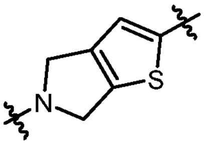, 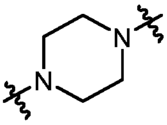, 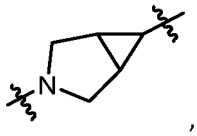, and 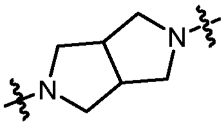,

with $R_6$ being H;

-$L_3$- is selected from:

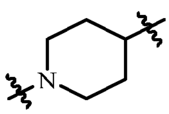, 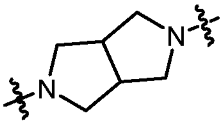, 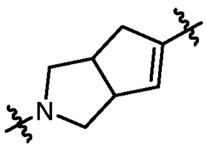, and 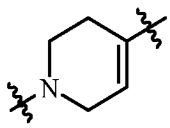;

-$L_4$- is selected from

: 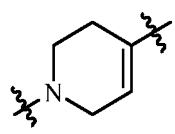 , 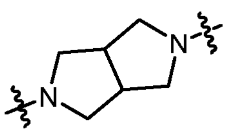 , 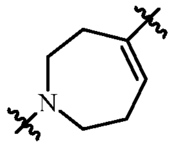 , 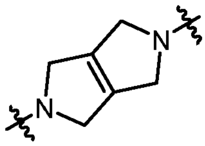 ,

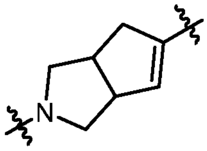 , 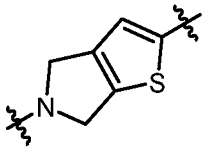 , and 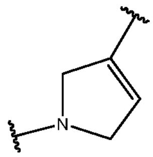 ;

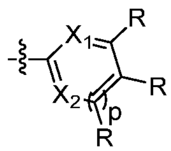

is selected from one of the following structures optionally substituted with 1-3 $R_R$:

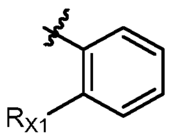 , 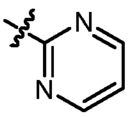 , 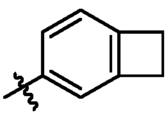 , 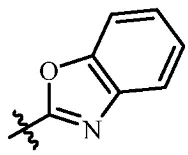 , 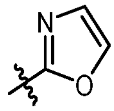 , 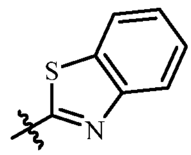 ,

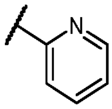 , 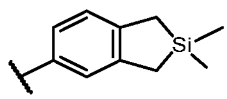 , 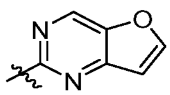 , 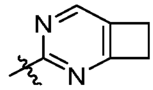 , 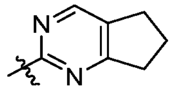 ,

and each $R_R$ is independently selected from F, Cl, Br, methyl, isopropyl, ethoxy, methoxy, ethynyl, propynyl, cyclopropyl, cyclobutyl, dimethylamino, and

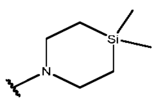 ;

and $R_{X1}$ is H;
or $R_{X1}$ and $R_6$, together with the atom to which they are attached, form morpholinyl, furyl, pyrrolyl, or thienyl.

**6.** The compound or the stereoisomer or pharmaceutically acceptable salt thereof according to claim 1,

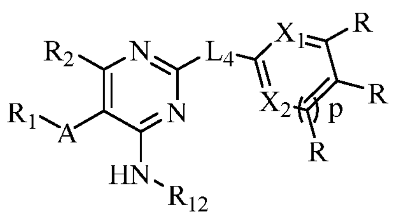

II

,

wherein $L_4$ is

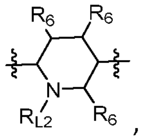, 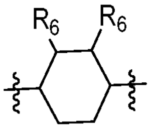, 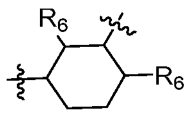, 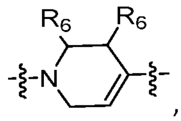, 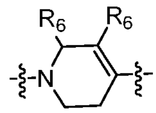,

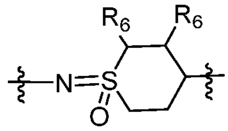, 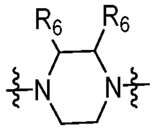,

a 5- to 6-membered heteroaryl containing 1-3 heteroatoms selected from N, S, and O, a 5-membered monocyclic heterocycloalkyl containing 1-3 heteroatoms selected from N, S, and O, a 7- to 8-membered monocyclic heterocycloalkyl containing 1-3 heteroatoms selected from N, S, and O, a 5- to 6-membered heterocycloalkyl fused 5- to 6-membered heterocycloalkyl containing 1-3 heteroatoms selected from N, S, and O, a 5- to 6-membered heterocycloalkyl fused 5- to 6-membered cycloalkyl containing 1-3 heteroatoms selected from N, S, and O, or a 7- to 12-membered spiro heterocycloalkyl containing 1-3 heteroatoms selected from N, S, and O, wherein the monocyclic heterocycloalkyl, 5- to 6-membered heterocycloalkyl fused 5- to 6-membered heterocycloalkyl, 5- to 6-membered heterocycloalkyl fused 5- to 6-membered cycloalkyl, and spiro heterocycloalkyl are optionally substituted with 1-3 groups selected from halogen, =O, CN, $C_{1-4}$ alkyl, $C_{1-4}$ alkoxy, OH, and $NH_2$;

A is -S(O)-, -C(O)-, -B(OH)-, -S-, -S(=N)-CN, or -C(=N)-CN;

$X_1$ and $X_2$ are independently $CR_{X1}$, S, or N;

$R_{L2}$ is H, $C_{1-4}$ alkyl, or $C_{3-6}$ cycloalkyl;

$R_6$ is H, halogen, =O, CN, $C_{1-4}$ alkyl, or $C_{1-4}$ alkoxy;

each $R_{X1}$ and R are independently H, halogen, $C_{1-4}$ alkyl, $C_{1-4}$ alkoxy, $-N(CH_3)_2$, $-NH(CH_3)$, $C_{2-6}$ alkynyl, $NHSO_2NH_2$, $NHCONH_2$, $NHCOC_{1-4}$ alkyl, $CONHC_{1-4}$ alkyl, $NHSO_2C_{1-4}$ alkyl, $SO_2NHC_{1-4}$ alkyl, $SO_2C_{1-4}$ alkyl, $SCF_3$, $SF_5$, a 3- to 5-membered cycloalkyl, $C_{1-4}$ haloalkyl, or a 5- to 10-membered heterocycloalkyl containing 1-3 heteroatoms selected from N, S, O, and Si, or two R on adjacent ring atoms, R and $R_{X1}$, or $R_{X1}$ and $R_6$, together with the atom to which they are attached, form $C_{3-8}$ cycloalkyl, a 5- to 10-membered heterocycloalkyl containing 1-3 heteroatoms selected from N, S, O, and Si, phenyl, or a 5- to 6-membered heteroaryl containing 1-3 heteroatoms selected from N, S, and O, wherein the cycloalkyl, heterocycloalkyl, phenyl, and heteroaryl are optionally substituted with 1-3 groups selected from halogen, =O, CN, $C_{1-4}$ alkyl, $C_{1-4}$ alkoxy, OH, and $NH_2$;

$R_1$ and $R_2$, together with the atom to which they are attached, form a 5- to 10-membered heterocycloalkyl or a 5- to 6-membered heteroaryl containing 1-3 heteroatoms selected from N, S, B, and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

$R_{12}$ is selected from a 4- to 10-membered heterocycloalkylene, a 3- to 10-membered cycloalkylene, a 6- to 10-membered arylene, a 5- to 10-membered heteroarylene, hydroxy-$C_{1-6}$ alkyl, $C_{1-4}$ alkyl-CN, -CRaRb=N-O-$C_{1-4}$ alkyl, -C(NRaRb)=N-CN, -C($C_{1-4}$ alkyl)=N-CN, -C(NRaRb)=CRa-$NO_2$, $-C(CH_3)_2-CH_2-O-C(O)NH_2$, $-C(CH_3)_2-CH_2-NH-C(O)O(C_{1-4}$ alkyl), $-C(CH_3)_2-CH_2-O-C(O)NHCH_3$, - $CH(CH_3)-CH_2-O-C(O)NH_2$, $-CH(CH_3)-CH_2-NH-C(O)O(C_{1-4}$ alkyl), $-CH(CH_3)-CH_2-O-C(O)NHCH_3$, -C(O)-Ra, or -C(O)-(4- to 6-membered heterocycloalkyl), wherein the heterocycloalkylene, cycloalkylene, arylene, and heteroarylene are optionally further substituted with 1-3 groups from halogen, CN, =O, OH, $-SF_5$, $C_{1-4}$ alkyl, $C_{1-4}$ alkyl-CN, hydroxy $C_{1-4}$ alkyl, $C_{1-4}$ haloalkyl, $-(CH_2)_t-O-C(O)NH_2$, $-(CH_2)_t$-NRa-C(O)$NH_2$, $-(CH_2)_t$-NRa-C(=NH)$NH_2$, $-(CH_2)_t$-NRa-C(O)-O-($C_{1-4}$ alkyl), $-(CH_2)_t$-NRa-C($C_{1-4}$ alkyl)=N-CN, $-(CH_2)_t$-NRa-C(NRaRb)=N-CN, $-(CH_2)_t$-NRa-C(NRaRb)=CRa-$NO_2$, $-(CH_2)_t$-C(NRaRb)=N-CN, $-(CH_2)_t$-C($C_{1-4}$ alkyl)=N-CN, $-(CH_2)_t$-C($C_{1-4}$ alkyl)=N-O-($C_{1-4}$ alkyl), =N-O-$C_{1-4}$ alkyl, a 5- to 6-membered heteroaryl, $-(CH_2)_t-O-C_{1-4}$ alkyl, $-(CH_2)_t-O-C(O)NHCH_3$, $-(CH_2)_t-O-C_{1-2}$ haloalkyl, - $(CH_2)_t-O-C_{3-6}$ cycloalkyl, $-(CH_2)_t-O-C_{1-4}$ alkyl-O-$C_{3-6}$ cycloalkyl, $-(CH_2)_t$-COOH, $-(CH_2)_t$-NRaRb, $-(CH_2)_t$-C(O)NRaRb, and $-(CH_2)_t$-NRa-C(O)$CH_3$;

Ra and Rb are each independently selected from H, halogen, $C_{1-4}$ alkyl, $C_{1-4}$ haloalkyl, $C_{1-4}$ alkoxy, halo $C_{1-4}$ alkoxy, or a 4- to 6-membered heterocycloalkyl containing 1-2 heteroatoms selected from N and O; and

p is selected from 0 or 1.

**7.** The compound or the stereoisomer or pharmaceutically acceptable salt thereof according to claim 6,

wherein $L_4$ is

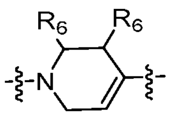, 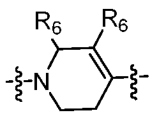, 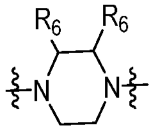, 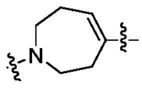 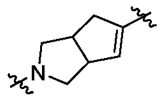;

and
$R_{12}$ is selected from $-C(CH_3)_1-CH_2-O-C(O)NH_2$, $-C(CH_3)_2-CH_2-NH-C(O)O(C_{1-4}$ alkyl), $-C(CH_3)_2-CH_2-O-C(O)NHCH_3$, $-CH(CH_3)-CH_2-O-C(O)NH_2$, $-CH(CH_3)-CH_2-NH-C(O)O(C_{1-4}$ alkyl), and $-CH(CH_3)-CH_2-O-C(O)NHCH_3$.

**8.** The compound or the stereoisomer or pharmaceutically acceptable salt thereof according to claim 1, wherein the compound is selected from one of the structures in Table 1.

**9.** The compound or the stereoisomer or pharmaceutically acceptable salt thereof according to claim 1, wherein the compound is selected from one of the structures in Table 2.

**10.** A pharmaceutical composition or pharmaceutical preparation comprising the compound or the stereoisomer or pharmaceutically acceptable salt thereof according to any one of claims 1-9, and a pharmaceutically acceptable carrier and/or auxiliary material.

**11.** The pharmaceutical composition or pharmaceutical preparation according to claim 10, wherein the pharmaceutical composition or pharmaceutical preparation comprises 1-1500 mg of the compound or the stereoisomer or pharmaceutically acceptable salt thereof according to any one of claims 1-9, and a pharmaceutically acceptable carrier and/or auxiliary material.

**12.** Use of the compound or the stereoisomer or pharmaceutically acceptable salt thereof according to any one of claims 1-9 in preparing a drug for treating/preventing a PDE4B-mediated disease.

**13.** A method for treating a disease in a mammal or human, comprising administering to a subject a therapeutically effective amount of the compound or the stereoisomer or pharmaceutically acceptable salt thereof according to any one of claims 1-9, wherein the therapeutically effective amount is preferably 1-1500 mg, and the disease is preferably a cancer, COPD, idiopathic pulmonary fibrosis, or an interstitial lung disease.

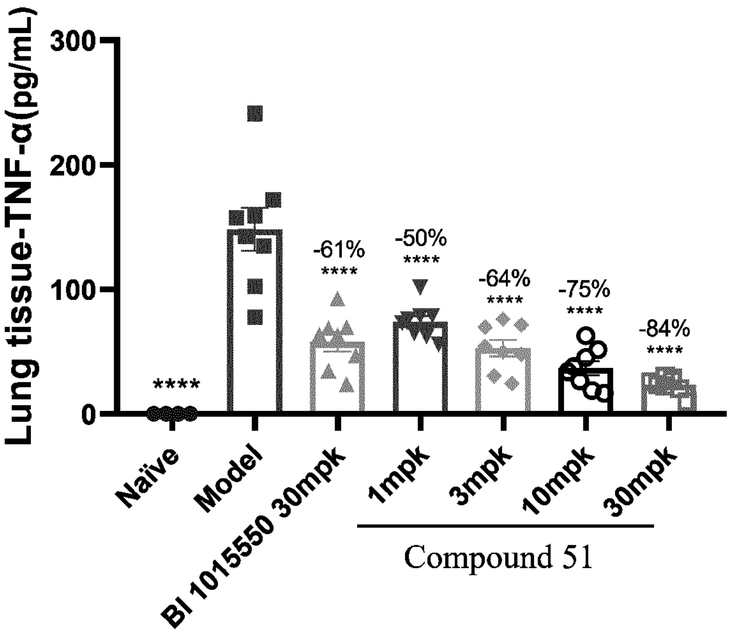


.****p<0.0001 vs Model

Figure 1

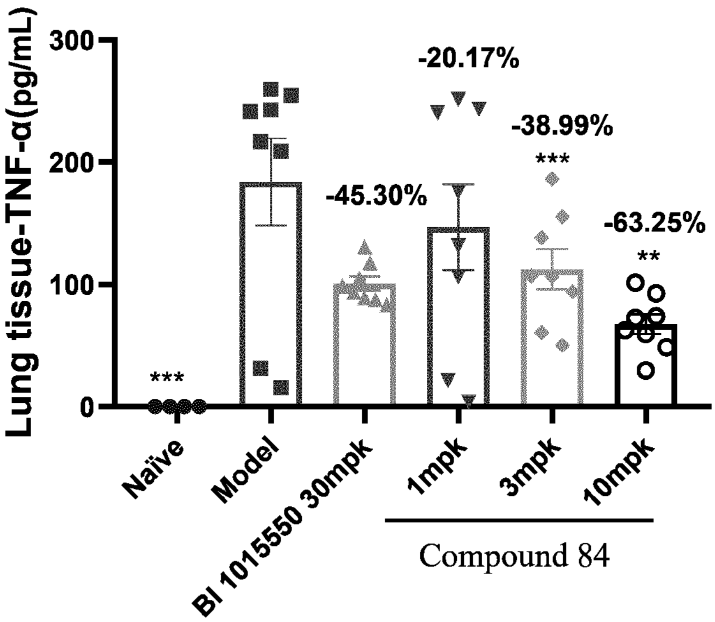


***p<0.001.**p<0.01. vs Model

Figure 2

**INTERNATIONAL SEARCH REPORT**

International application No. **PCT/CN2023/112061**

**A. CLASSIFICATION OF SUBJECT MATTER**

C07D495/04(2006.01)i; C07D519/00(2006.01)i; A61K31/519(2006.01)i; A61K31/498(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, VEN, CNKI, DWPI, STN, 西藏海思科制药, 噻吩并嘧啶, 哌嗪, 哌啶, 苯, PDE4!, thiophene, pyrimidine, piperazine, piperidine, phenyl, 结构式检索, search for structural formula

**C. DOCUMENTS CONSIDERED TO BE RELEVANT**

| Category* | Citation of document, with indication, where appropriate, of the relevant passages | Relevant to claim No. |
|---|---|---|
| X | CN 101426505 A (BOEHRINGER INGELHEIM INTERNATIONAL GMBH) 06 May 2009 (2009-05-06) abstract, claim 1, pages 109-110, tables, compounds | 1-13 |
| X | CN 101827852 A (BOEHRINGER INGELHEIM INTERNATIONAL GMBH) 08 September 2010 (2010-09-08) abstract, the claims, page 14, line 2, the third compound | 1-13 |
| X | CN 101163706 A (BOEHRINGER INGELHEIM INTERNATIONAL GMBH) 16 April 2008 (2008-04-16) abstract, description, pages 74-117, tables, compounds | 1-13 |
| X | CN 101827853 A (BOEHRINGER INGELHEIM INTERNATIONAL GMBH) 08 September 2010 (2010-09-08) abstract, description, pages 45-56, table A | 1-13 |
| X | CN 101903394 A (BOEHRINGER INGELHEIM INTERNATIONAL GMBH) 01 December 2010 (2010-12-01) abstract, description, pages 43-45 embodiments, compounds | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 November 2023** | **20 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/112061

C. DOCUMENTS CONSIDERED TO BE RELEVANT

| Category* | Citation of document, with indication, where appropriate, of the relevant passages | Relevant to claim No. |
|---|---|---|
| X | US 2011028441 A1 (BOEHRINGER INGELHEIM INTERNATIONAL GMBH) 03 February 2011 (2011-02-03) abstract, description, page 15, embodiment 7, pages 32-44, tables A-C | 1-13 |
| X | US 2011046096 A1 (BOEHRINGER INGELHEIM INTERNATIONAL GMBH) 24 February 2011 (2011-02-24) abstract, and description, pages 7-14 | 1-13 |
| X | US 2012028932 A1 (BOEHRINGER INGELHEIM INTERNATIONAL GMBH) 02 February 2012 (2012-02-02) abstract, and description, pages 11-32 | 1-13 |
| X | US 2012035143 A1 (BOEHRINGER INGELHEIM INTERNATIONAL GMBH) 09 February 2012 (2012-02-09) abstract, description, pages 13-43, embodiments, compounds | 1-13 |
| X | US 2013225609 A1 (BOEHRINGER INGELHEIM INTERNATIONAL GMBH) 29 August 2013 (2013-08-29) abstract, description, pages 13-43, embodiments, compounds | 1-13 |
| PA | US 2023190754 A1 (BOEHRINGER INGELHEIM INTERNATIONAL GMBH) 22 June 2023 (2023-06-22) entire document | 1-13 |
| PA | WO 2023104958 A1 (BOEHRINGER INGELHEIM INTERNATIONAL GMBH) 15 June 2023 (2023-06-15) entire document | 1-13 |
| A | US 2014228286 A1 (BOEHRINGER INGELHEIM INTERNATIONAL GMBH) 14 August 2014 (2014-08-14) entire document | 1-13 |
| A | CN 111278442 A (BOEHRINGER INGELHEIM INTERNATIONAL GMBH) 12 June 2020 (2020-06-12) entire document | 1-13 |

Form PCT/ISA/210 (second sheet) (July 2022)

**INTERNATIONAL SEARCH REPORT**

International application No.
**PCT/CN2023/112061**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 relates to a disease diagnosis and treatment method (PCT Rule 39.1(iv)), and the following search is performed on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/112061**

| Patent document cited in search report | Publication date (day/month/year) | Patent family member(s) | Publication date (day/month/year) |
|---|---|---|---|
| CN 101426505 A | 06 May 2009 | EA 200802051 A1 | 28 April 2009 |
| | | JP 2010523467 A | 15 July 2010 |
| | | JP 5214588 B2 | 19 June 2013 |
| | | ECSP 088738 A | 31 October 2008 |
| | | WO 2007118793 A1 | 25 October 2007 |
| | | EP 2010185 A1 | 07 January 2009 |
| | | EP 2010185 B1 | 13 June 2012 |
| | | CO 6140032 A2 | 19 March 2010 |
| | | BRPI 0710459 A2 | 16 August 2011 |
| | | IL 194739 A0 | 03 August 2009 |
| | | TW 200808811 A | 16 February 2008 |
| | | PE 20080319 A1 | 13 May 2008 |
| | | CA 2647243 A1 | 25 October 2007 |
| | | AU 2007239573 A1 | 25 October 2007 |
| | | US 2008096882 A1 | 24 April 2008 |
| | | US 8114878 B2 | 14 February 2012 |
| | | US 2014005154 A1 | 02 January 2014 |
| | | US 8822474 B2 | 02 September 2014 |
| | | UY 30287 A1 | 30 November 2007 |
| | | ZA 200807015 B | 27 January 2010 |
| | | KR 20090009885 A | 23 January 2009 |
| | | AR 060516 A1 | 25 June 2008 |
| | | EP 1847543 A1 | 24 October 2007 |
| | | MX 2008012915 A | 15 October 2008 |
| | | US 2012108534 A1 | 03 May 2012 |
| | | US 8604039 B2 | 10 December 2013 |
| | | NO 20083710 L | 18 November 2008 |
| CN 101827852 A | 08 September 2010 | MX 2010004026 A | 30 April 2010 |
| | | PT 2610258 E | 24 October 2014 |
| | | WO 2009050248 A1 | 23 April 2009 |
| | | UY 31405 A1 | 29 May 2009 |
| | | BRPI 0818006 A2 | 22 December 2015 |
| | | BRPI 0818006 B1 | 22 October 2019 |
| | | BRPI 0818006 B8 | 25 May 2021 |
| | | CL 2008003096 A1 | 12 February 2010 |
| | | CY 1112703 T1 | 10 February 2016 |
| | | CA 2705414 A1 | 23 April 2009 |
| | | CA 2705414 C | 24 May 2016 |
| | | MA 31845 B1 | 01 November 2010 |
| | | TW 200918074 A | 01 May 2009 |
| | | TWI 421077 B | 01 January 2014 |
| | | AU 2008313660 A1 | 23 April 2009 |
| | | AU 2008313660 B2 | 07 November 2013 |
| | | PE 20091386 A1 | 17 October 2009 |
| | | ES 2524910 T3 | 15 December 2014 |
| | | JP 2013064001 A | 11 April 2013 |
| | | JP 5615889 B2 | 29 October 2014 |
| | | ES 2381452 T3 | 28 May 2012 |
| | | EP 2215092 A1 | 11 August 2010 |
| | | EP 2215092 B1 | 25 January 2012 |

Form PCT/ISA/210 (patent family annex) (July 2022)

**INTERNATIONAL SEARCH REPORT**
**Information on patent family members**

International application No.
**PCT/CN2023/112061**

| Patent document cited in search report | Publication date (day/month/year) | Patent family member(s) | Publication date (day/month/year) |
|---|---|---|---|
| | | UA 99309 C2 | 10 August 2012 |
| | | HRP 20141153 T1 | 13 February 2015 |
| | | ECSP 10010156 A | 29 June 2010 |
| | | SI 2610258 T1 | 31 December 2014 |
| | | SI 2215092 T1 | 30 April 2012 |
| | | NZ 585346 A | 30 September 2011 |
| | | DK 2610258 T3 | 10 November 2014 |
| | | PL 2215092 T3 | 31 July 2012 |
| | | PL 2610258 T3 | 27 February 2015 |
| | | TN 2010000175 A1 | 11 November 2011 |
| | | EP 2610258 A1 | 03 July 2013 |
| | | EP 2610258 B1 | 27 August 2014 |
| | | RS 52271 B | 31 October 2012 |
| | | DK 2215092 T3 | 07 May 2012 |
| | | EA 201000609 A1 | 29 October 2010 |
| | | EA 019480 B1 | 30 April 2014 |
| | | HRP 20120334 T1 | 31 May 2012 |
| | | AR 069075 A1 | 30 December 2009 |
| | | CY 1115858 T1 | 25 January 2017 |
| | | ME 01330 B | 20 December 2013 |
| | | EP 2380891 A1 | 26 October 2011 |
| | | EP 2380891 B1 | 11 December 2013 |
| | | ZA 201001683 B | 27 October 2010 |
| | | AT 542825 T | 15 February 2012 |
| | | JP 2011500640 A | 06 January 2011 |
| | | JP 5150728 B2 | 27 February 2013 |
| | | KR 20100100807 A | 15 September 2010 |
| | | KR 101548975 B1 | 01 September 2015 |
| | | PE 20131463 A1 | 23 December 2013 |
| | | US 2011021501 A1 | 27 January 2011 |
| | | US 8754073 B2 | 17 June 2014 |
| | | MY 153979 A | 30 April 2015 |
| | | PT 2215092 E | 10 April 2012 |
| CN 101163706 A | 16 April 2008 | AR 053235 A1 | 25 April 2007 |
| | | NO 20074615 L | 19 November 2007 |
| | | HRP 20090395 T1 | 31 August 2009 |
| | | AT 434620 T | 15 July 2009 |
| | | ES 2328400 T3 | 12 November 2009 |
| | | MX 2007012313 A | 21 November 2007 |
| | | EP 1874781 A1 | 09 January 2008 |
| | | EP 1874781 B1 | 24 June 2009 |
| | | EP 1874781 B9 | 08 September 2010 |
| | | NZ 563488 A | 30 April 2010 |
| | | EP 2060575 A1 | 20 May 2009 |
| | | EA 200702200 A1 | 28 April 2008 |
| | | EA 013108 B1 | 26 February 2010 |
| | | PT 1874781 E | 27 July 2009 |
| | | KR 20080004621 A | 09 January 2008 |
| | | KR 101335964 B1 | 04 December 2013 |
| | | RS 51163 B | 31 October 2010 |

Form PCT/ISA/210 (patent family annex) (July 2022)

**INTERNATIONAL SEARCH REPORT**
**Information on patent family members**

International application No.
**PCT/CN2023/112061**

| Patent document cited in search report | Publication date (day/month/year) | Patent family member(s) | Publication date (day/month/year) |
|---|---|---|---|
| | | DK 1874781 T3 | 02 November 2009 |
| | | UA 93872 C2 | 25 March 2011 |
| | | CA 2605161 A1 | 26 October 2006 |
| | | US 2010197656 A1 | 05 August 2010 |
| | | US 8354531 B2 | 15 January 2013 |
| | | DE 102005019201 A1 | 02 November 2006 |
| | | TWI 380984 B | 01 January 2013 |
| | | DE 502006004066 D1 | 06 August 2009 |
| | | CY 1109348 T1 | 02 July 2014 |
| | | BRPI 0608387 A2 | 29 December 2009 |
| | | JP 2008536890 A | 11 September 2008 |
| | | JP 5021624 B2 | 12 September 2012 |
| | | US 2007259846 A1 | 08 November 2007 |
| | | US 7511045 B2 | 31 March 2009 |
| | | AU 2006237354 A1 | 26 October 2006 |
| | | AU 2006237354 B2 | 01 March 2012 |
| | | SI 1874781 T1 | 31 December 2009 |
| | | US 2009186875 A1 | 23 July 2009 |
| | | US 7723341 B2 | 25 May 2010 |
| | | PL 1874781 T3 | 31 December 2009 |
| | | IL 186749 A0 | 09 February 2008 |
| | | IL 186749 A | 31 August 2014 |
| | | WO 2006111549 A1 | 26 October 2006 |
| | | ZA 200707591 B | 26 August 2009 |
| CN 101827853 A | 08 September 2010 | US 2014107103 A1 | 17 April 2014 |
| | | US 9115142 B2 | 25 August 2015 |
| | | EP 2205609 A2 | 14 July 2010 |
| | | EP 2205609 B1 | 29 March 2017 |
| | | MX 2010003346 A | 09 April 2010 |
| | | AR 069076 A1 | 30 December 2009 |
| | | BRPI 0817781 A2 | 24 September 2019 |
| | | NZ 585348 A | 24 February 2012 |
| | | ZA 201001621 B | 27 October 2010 |
| | | PE 20091081 A1 | 24 August 2009 |
| | | AU 2008313742 A1 | 23 April 2009 |
| | | AU 2008313742 B2 | 28 November 2013 |
| | | RU 2010119645 A | 27 January 2012 |
| | | RU 2500681 C2 | 10 December 2013 |
| | | US 2010305102 A1 | 02 December 2010 |
| | | US 8637519 B2 | 28 January 2014 |
| | | KR 20100075930 A | 05 July 2010 |
| | | CA 2702524 A1 | 23 April 2009 |
| | | TW 200918073 A | 01 May 2009 |
| | | TWI 445535 B | 21 July 2014 |
| | | CL 2008003097 A1 | 22 January 2010 |
| | | UY 31402 A1 | 29 May 2009 |
| | | WO 2009050242 A2 | 23 April 2009 |
| | | WO 2009050242 A3 | 30 July 2009 |
| | | JP 2011500639 A | 06 January 2011 |
| | | JP 5341899 B2 | 13 November 2013 |

Form PCT/ISA/210 (patent family annex) (July 2022)

**INTERNATIONAL SEARCH REPORT**
**Information on patent family members**

International application No.
**PCT/CN2023/112061**

| Patent document cited in search report | | | Publication date (day/month/year) | Patent family member(s) | | | Publication date (day/month/year) |
|---|---|---|---|---|---|---|---|
| CN | 101903394 | A | 01 December 2010 | AU | 2008312624 | A1 | 23 April 2009 |
| | | | | AU | 2008312624 | B2 | 10 July 2014 |
| | | | | KR | 20100095528 | A | 31 August 2010 |
| | | | | ZA | 201002425 | B | 23 February 2011 |
| | | | | NZ | 584962 | A | 29 July 2011 |
| | | | | ES | 2424445 | T3 | 02 October 2013 |
| | | | | CL | 2012000560 | A1 | 14 September 2012 |
| | | | | EP | 2205608 | A1 | 14 July 2010 |
| | | | | EP | 2205608 | B1 | 08 May 2013 |
| | | | | RU | 2010119647 | A | 27 November 2011 |
| | | | | RU | 2528340 | C2 | 10 September 2014 |
| | | | | BRPI | 0818671 | A2 | 15 September 2015 |
| | | | | DK | 2205608 | T3 | 01 July 2013 |
| | | | | PL | 2205608 | T3 | 30 September 2013 |
| | | | | JP | 2011500701 | A | 06 January 2011 |
| | | | | JP | 5538231 | B2 | 02 July 2014 |
| | | | | US | 2010222585 | A1 | 02 September 2010 |
| | | | | US | 8653282 | B2 | 18 February 2014 |
| | | | | IL | 204854 | A0 | 30 November 2010 |
| | | | | CL | 2008003095 | A1 | 22 January 2010 |
| | | | | TW | 200918541 | A | 01 May 2009 |
| | | | | AR | 069077 | A1 | 30 December 2009 |
| | | | | WO | 2009052138 | A1 | 23 April 2009 |
| | | | | WO | 2009052138 | A8 | 15 November 2012 |
| | | | | MX | 2010003826 | A | 21 April 2010 |
| | | | | CA | 2702500 | A1 | 23 April 2009 |
| | | | | NZ | 592534 | A | 28 March 2013 |
| US | 2011028441 | A1 | 03 February 2011 | WO | 2009053268 | A1 | 30 April 2009 |
| | | | | CA | 2702447 | A1 | 30 April 2009 |
| | | | | EP | 2214673 | A1 | 11 August 2010 |
| | | | | EP | 2214673 | B1 | 30 May 2012 |
| | | | | JP | 2011500621 | A | 06 January 2011 |
| US | 2011046096 | A1 | 24 February 2011 | EP | 2215093 | A1 | 11 August 2010 |
| | | | | EP | 2215093 | B1 | 14 December 2011 |
| | | | | AT | 537175 | T | 15 December 2011 |
| | | | | CA | 2702518 | A1 | 23 April 2009 |
| | | | | WO | 2009050236 | A1 | 23 April 2009 |
| | | | | US | 8486948 | B2 | 16 July 2013 |
| | | | | US | 2013274264 | A1 | 17 October 2013 |
| | | | | US | 9090626 | B2 | 28 July 2015 |
| | | | | JP | 2011500638 | A | 06 January 2011 |
| | | | | JP | 5563466 | B2 | 30 July 2014 |
| US | 2012028932 | A1 | 02 February 2012 | CA | 2753597 | A1 | 02 September 2010 |
| | | | | JP | 2012519159 | A | 23 August 2012 |
| | | | | US | 8592400 | B2 | 26 November 2013 |
| | | | | US | 2014031343 | A1 | 30 January 2014 |
| | | | | US | 8759326 | B2 | 24 June 2014 |
| | | | | WO | 2010097332 | A1 | 02 September 2010 |
| | | | | EP | 2400961 | A1 | 04 January 2012 |
| | | | | EP | 2400961 | B1 | 22 November 2017 |

Form PCT/ISA/210 (patent family annex) (July 2022)

**INTERNATIONAL SEARCH REPORT**
**Information on patent family members**

International application No.
**PCT/CN2023/112061**

| Patent document cited in search report | | | Publication date (day/month/year) | Patent family member(s) | | | Publication date (day/month/year) |
|---|---|---|---|---|---|---|---|
| US | 2012035143 | A1 | 09 February 2012 | EP | 2400962 | A1 | 04 January 2012 |
| | | | | EP | 2400962 | B1 | 08 November 2017 |
| | | | | US | 9161927 | B2 | 20 October 2015 |
| | | | | JP | 2012519160 | A | 23 August 2012 |
| | | | | CA | 2753604 | A1 | 02 September 2010 |
| | | | | WO | 2010097334 | A1 | 02 September 2010 |
| US | 2013225609 | A1 | 29 August 2013 | US | 2014350035 | A1 | 27 November 2014 |
| | | | | WO | 2011124525 | A1 | 13 October 2011 |
| | | | | EP | 2555774 | A1 | 13 February 2013 |
| | | | | EP | 2555774 | B1 | 21 October 2015 |
| | | | | JP | 2013523792 | A | 17 June 2013 |
| US | 2023190754 | A1 | 22 June 2023 | WO | 2023104961 | A1 | 15 June 2023 |
| WO | 2023104958 | A1 | 15 June 2023 | US | 2023181590 | A1 | 15 June 2023 |
| US | 2014228286 | A1 | 14 August 2014 | WO | 2014124860 | A1 | 21 August 2014 |
| CN | 111278442 | A | 12 June 2020 | IL | 273169 | A | 30 April 2020 |
| | | | | US | 2019134043 | A1 | 09 May 2019 |
| | | | | US | 11406638 | B2 | 09 August 2022 |
| | | | | MX | 2020004173 | A | 03 August 2020 |
| | | | | CL | 2020000627 | A1 | 21 August 2020 |
| | | | | TW | 201929858 | A | 01 August 2019 |
| | | | | TWI | 700088 | B | 01 August 2020 |
| | | | | AU | 2018357775 | A1 | 19 March 2020 |
| | | | | EP | 3700529 | A1 | 02 September 2020 |
| | | | | BR | 112020003973 | A2 | 01 September 2020 |
| | | | | PH | 12020550452 | A1 | 15 March 2021 |
| | | | | JP | 2021500362 | A | 07 January 2021 |
| | | | | JP | 7196169 | B2 | 26 December 2022 |
| | | | | WO | 2019081235 | A1 | 02 May 2019 |
| | | | | MA | 50441 | A | 02 September 2020 |
| | | | | US | 2022378793 | A1 | 01 December 2022 |
| | | | | CA | 3079299 | A1 | 02 May 2019 |
| | | | | KR | 20200075864 | A | 26 June 2020 |
| | | | | EA | 202090977 | A1 | 04 September 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013026797 A1
- US 20140228286 A1
- CN 103145607
- WO 2020099886 A
- WO 2010078348 A1
- WO 2014124860 A1
- WO 2011124524 A1
- WO 2022184103 A
- WO 2017148518 A